(19) Europäisches Patentamt
European Patent Office
Office européen des brevets

(11) EP 4 023 659 A1

(12) **EUROPEAN PATENT APPLICATION**
published in accordance with Art. 153(4) EPC

(43) Date of publication:
**06.07.2022 Bulletin 2022/27**

(21) Application number: **20856439.3**

(22) Date of filing: **28.08.2020**

(51) International Patent Classification (IPC):
***C07H 15/04*** (2006.01) ***C07H 21/02*** (2006.01)
***C12N 15/113*** (2010.01) ***C12N 15/00*** (2006.01)
***A61K 31/7105*** (2006.01) ***A61K 47/54*** (2017.01)
***A61P 1/16*** (2006.01)

(52) Cooperative Patent Classification (CPC):
**A61K 31/7105; A61K 47/54; A61P 1/16;
C07H 15/04; C07H 21/02; C12N 15/00;
C12N 15/113;** Y02P 20/55

(86) International application number:
**PCT/CN2020/112105**

(87) International publication number:
**WO 2021/037205 (04.03.2021 Gazette 2021/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.08.2019 CN 201910820597
29.08.2019 CN 201910820620**

(71) Applicant: **Suzhou Ribo Life Science Co., Ltd.
Kunshan, Jiangsu 215300 (CN)**

(72) Inventors:
- **ZHANG, Hongyan
Kunshan, Jiangsu 215300 (CN)**
- **YANG, Zhiwei
Kunshan, Jiangsu 215300 (CN)**
- **GAO, Shan
Kunshan, Jiangsu 215300 (CN)**
- **CAO, Liqiang
Kunshan, Jiangsu 215300 (CN)**
- **LIU, Guocheng
Kunshan, Jiangsu 215300 (CN)**

(74) Representative: **SSM Sandmair
Patentanwälte Rechtsanwalt
Partnerschaft mbB
Joseph-Wild-Straße 20
81829 München (DE)**

(54) **COMPOUND AND DRUG CONJUGATE, AND PREPARATION METHOD AND USE THEREOF**

(57) Disclosed is a compound with a structure as shown in Formula (101) and a corresponding drug conjugate, wherein the drug conjugate can be specifically targeted at cells and has a low toxicity and an excellent delivery efficiency.

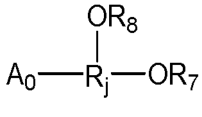

(101)

EP 4 023 659 A1

Sequence Listing

<110> SUZHOU RIBO LIFE SCIENCE CO., LTD.

<120> COMPOUND AND DRUG CONJUGATE, AND PREPARATION METHOD AND USE THEREOF

<130> FP1200617P

<150> 201910820597.7
<151> 2019-08-29

<150> 201910820620.2
<151> 2019-08-29

<160> 724

<170> SIPOSequenceListing 1.0

<210> 1
<211> 19
<212> RNA
<213> artifical sequence

<400> 1
ccuugaggca uacuucaaa 19

<210> 2
<211> 21
<212> RNA
<213> artifical sequence

<400> 2
uuugaaguau gccucaaggu u 21

<210> 3
<211> 21
<212> RNA
<213> artifical sequence

<400> 3
gaccuugagg cauacuucaa a 21

<210> 4
<211> 23
<212> RNA
<213> artifical sequence

<400> 4
uuugaaguau gccucaaggu cgg 23

<210> 5
<211> 19
<212> RNA
<213> artifical sequence

<400> 5
ccuugaggca uacuucaaa 19

<210> 6
<211> 21
<212> RNA
<213> artifical sequence

<400> 6
uuugaaguau gccucaaggu u 21

<210> 7
<211> 19
<212> RNA
<213> artifical sequence

<400> 7
ccuugaggca uacuucaaa 19

<210> 8
<211> 21
<212> RNA
<213> artifical sequence

<400> 8
uuugaaguau gccucaaggu u 21

<210> 9
<211> 21
<212> RNA
<213> artifical sequence

<400> 9
gaccuugagg cauacuucaa a 21

<210> 10
<211> 23
<212> RNA
<213> artifical sequence

<400> 10
uuugaaguau gccucaaggu cgg 23

<210> 11
<211> 21
<212> RNA
<213> artifical sequence

<400> 11
gaccuugagg cauacuucaa a 21

<210> 12
<211> 23
<212> RNA
<213> artifical sequence

<400> 12
uuugaaguau gccucaaggu cgg 23

<210> 13
<211> 19
<212> RNA
<213> artifical sequence

<400> 13
ccuugaggca uacuucaaa 19

<210> 14
<211> 21
<212> RNA

<213> artifical sequence

<400> 14
uuugaaguau gccucaaggu u 21

<210> 15
<211> 19
<212> RNA
<213> artifical sequence

<400> 15
ccuugaggca uacuucaaa 19

<210> 16
<211> 21
<212> RNA
<213> artifical sequence

<400> 16
uuugaaguau gccucaaggu u 21

<210> 17
<211> 21
<212> RNA
<213> artifical sequence

<400> 17
gaccuugagg cauacuucaa a 21

<210> 18
<211> 23
<212> RNA
<213> artifical sequence

<400> 18
uuugaaguau gccucaaggu cgg 23

<210> 19
<211> 21
<212> RNA
<213> artifical sequence

<400> 19
gaccuugagg cauacuucaa a 21

<210> 20
<211> 23
<212> RNA
<213> artifical sequence

<400> 20
uuugaaguau gccucaaggu cgg 23

<210> 31
<211> 19
<212> RNA
<213> artifical sequence

<400> 31
ccuugaggca uacuucaaa 19

<210> 22
<211> 21

<212> RNA
<213> artifical sequence

<400> 22
uuugaaguau gccucaaggu u 21

<210> 23
<211> 19
<212> RNA
<213> artifical sequence

<400> 23
ccuugaggca uacuucaaa 19

<210> 24
<211> 21
<212> RNA
<213> artifical sequence

<400> 24
uuugaaguau gccucaaggu u 21

<210> 25
<211> 21
<212> RNA
<213> artifical sequence

<400> 25
gaccuugagg cauacuucaa a 21

<210> 26
<211> 23
<212> RNA
<213> artifical sequence

<400> 26
uuugaaguau gccucaaggu cgg 23

<210> 27
<211> 21
<212> RNA
<213> artifical sequence

<400> 27
gaccuugagg cauacuucaa a 21

<210> 28
<211> 23
<212> RNA
<213> artifical sequence

<400> 28
uuugaaguau gccucaaggu cgg 23

<210> 29
<211> 19
<212> RNA
<213> artifical sequence

<400> 29
ccuugaggca uacuucaaa 19

<210> 30

<211> 21
<212> RNA
<213> artifical sequence

<400> 30
uuugaaguau gccucaaggu u 21

<210> 31
<211> 19
<212> RNA
<213> artifical sequence

<400> 31
ccuugaggca uacuucaaa 19

<210> 32
<211> 21
<212> RNA
<213> artifical sequence

<400> 32
uuugaaguau gccucaaggu u 21

<210> 33
<211> 21
<212> RNA
<213> artifical sequence

<400> 33
gaccuugagg cauacuucaa a 21

<210> 34
<211> 23
<212> RNA
<213> artifical sequence

<400> 34
uuugaaguau gccucaaggu cgg 23

<210> 35
<211> 21
<212> RNA
<213> artifical sequence

<400> 35
gaccuugagg cauacuucaa a 21

<210> 36
<211> 23
<212> RNA
<213> artifical sequence

<400> 36
uuugaaguau gccucaaggu cgg 23

<210> 37
<211> 19
<212> RNA
<213> artifical sequence

<400> 37
ugcuaugccu caucuucua 19

<210> 38
<211> 21
<212> RNA
<213> artifical sequence

<400> 38
uagaagauga ggcauagcag c 21

<210> 39
<211> 19
<212> RNA
<213> artifical sequence

<400> 39
ugcuaugccu caucuucua 19

<210> 40
<211> 21
<212> RNA
<213> artifical sequence

<400> 40
uagaagauga ggcauagcau u 21

<210> 41
<211> 19
<212> RNA
<213> artifical sequence

<400> 41
ugcuaugccu caucuucua 19

<210> 42
<211> 21
<212> RNA
<213> artifical sequence

<400> 42
uagaagauga ggcauagcag c 21

<210> 43
<211> 19
<212> RNA
<213> artifical sequence

<400> 43
ugcuaugccu caucuucua 19

<210> 44
<211> 21
<212> RNA
<213> artifical sequence

<400> 44
uagaagauga ggcauagcau u 21

<210> 45
<211> 19
<212> RNA
<213> artifical sequence

<400> 45
ugcuaugccu caucuucua 19

<210> 46
<211> 21
<212> RNA
<213> artifical sequence

<400> 46
uagaagauga ggcauagcag c 21

<210> 47
<211> 19
<212> RNA
<213> artifical sequence

<400> 47
ugcuaugccu caucuucua 19

<210> 48
<211> 21
<212> RNA
<213> artifical sequence

<400> 48
uagaagauga ggcauagcau u 21

<210> 49
<211> 19
<212> RNA
<213> artifical sequence

<400> 49
ugcuaugccu caucuucua 19

<210> 50
<211> 21
<212> RNA
<213> artifical sequence

<400> 50
uagaagauga ggcauagcag c 21

<210> 51
<211> 19
<212> RNA
<213> artifical sequence

<400> 51
ugcuaugccu caucuucua 19

<210> 52
<211> 21
<212> RNA
<213> artifical sequence

<400> 52
uagaagauga ggcauagcau u 21

<210> 53
<211> 19
<212> RNA
<213> artifical sequence

<400> 53

ugcuaugccu caucuucua 19

<210> 54
<211> 21
<212> RNA
<213> artifical sequence

<400> 54
uagaagauga ggcauagcag c 21

<210> 55
<211> 19
<212> RNA
<213> artifical sequence

<400> 55
ugcuaugccu caucuucua 19

<210> 56
<211> 21
<212> RNA
<213> artifical sequence

<400> 56
uagaagauga ggcauagcau u 21

<210> 57
<211> 19
<212> RNA
<213> artifical sequence

<400> 57
ugcuaugccu caucuucua 19

<210> 58
<211> 21
<212> RNA
<213> artifical sequence

<400> 58
uagaagauga ggcauagcag c 21

<210> 59
<211> 19
<212> RNA
<213> artifical sequence

<400> 59
ugcuaugccu caucuucua 19

<210> 60
<211> 21
<212> RNA
<213> artifical sequence

<400> 60
uagaagauga ggcauagcau u 21

<210> 61
<211> 19
<212> RNA
<213> artifical sequence

<400> 61
ugcuaugccu caucuucua 19

<210> 62
<211> 21
<212> RNA
<213> artifical sequence

<400> 62
uagaagauga ggcauagcag c 21

<210> 63
<211> 19
<212> RNA
<213> artifical sequence

<400> 63
ugcuaugccu caucuucua 19

<210> 64
<211> 21
<212> RNA
<213> artifical sequence

<400> 64
uagaagauga ggcauagcau u 21

<210> 65
<211> 19
<212> RNA
<213> artifical sequence

<400> 65
ugcuaugccu caucuucua 19

<210> 66
<211> 21
<212> RNA
<213> artifical sequence

<400> 66
uagaagauga ggcauagcag c 21

<210> 67
<211> 19
<212> RNA
<213> artifical sequence

<400> 67
ugcuaugccu caucuucua 19

<210> 68
<211> 21
<212> RNA
<213> artifical sequence

<400> 68
uagaagauga ggcauagcau u 21

<210> 69
<211> 19
<212> RNA
<213> artifical sequence

<400> 69
ugcuaugccu caucuucua 19

<210> 70
<211> 21
<212> RNA
<213> artifical sequence

<400> 70
uagaagauga ggcauagcag c 21

<210> 71
<211> 19
<212> RNA
<213> artifical sequence

<400> 71
ugcuaugccu caucuucua 19

<210> 72
<211> 21
<212> RNA
<213> artifical sequence

<400> 72
uagaagauga ggcauagcau u 21

<210> 73
<211> 19
<212> RNA
<213> artifical sequence

<400> 73
ucugugccuu cucaucuga 19

<210> 74
<211> 21
<212> RNA
<213> artifical sequence

<400> 74
ucagaugaga aggcacagac g 21

<210> 75
<211> 19
<212> RNA
<213> artifical sequence

<400> 75
ucugugccuu cucaucuga 19

<210> 76
<211> 21
<212> RNA
<213> artifical sequence

<400> 76
ucagaugaga aggcacagac g 21

<210> 77
<211> 19
<212> RNA

<213> artifical sequence

<400> 77
ucugugccuu cucaucuga 19

<210> 78
<211> 21
<212> RNA
<213> artifical sequence

<400> 78
ucagaugaga aggcacagac g 21

<210> 79
<211> 19
<212> RNA
<213> artifical sequence

<400> 79
ucugugccuu cucaucuga 19

<210> 80
<211> 21
<212> RNA
<213> artifical sequence

<400> 80
ucagaugaga aggcacagac g 21

<210> 81
<211> 19
<212> RNA
<213> artifical sequence

<400> 81
ucugugccuu cucaucuga 19

<210> 82
<211> 21
<212> RNA
<213> artifical sequence

<400> 82
ucagaugaga aggcacagac g 21

<210> 83
<211> 19
<212> RNA
<213> artifical sequence

<400> 83
ucugugccuu cucaucuga 19

<210> 84
<211> 21
<212> RNA
<213> artifical sequence

<400> 84
ucagaugaga aggcacagac g 21

<210> 85
<211> 19

<212> RNA
<213> artifical sequence

<400> 85
ucugugccuu cucaucuga 19

<210> 86
<211> 21
<212> RNA
<213> artifical sequence

<400> 86
ucagaugaga aggcacagac g 21

<210> 87
<211> 19
<212> RNA
<213> artifical sequence

<400> 87
ucugugccuu cucaucuga 19

<210> 88
<211> 21
<212> RNA
<213> artifical sequence

<400> 88
ucagaugaga aggcacagac g 21

<210> 89
<211> 19
<212> RNA
<213> artifical sequence

<400> 89
ucugugccuu cucaucuga 19

<210> 90
<211> 21
<212> RNA
<213> artifical sequence

<400> 90
ucagaugaga aggcacagac g 21

<210> 91
<211> 19
<212> RNA
<213> artifical sequence

<400> 91
cgugugcacu ucgcuucaa 19

<210> 92
<211> 21
<212> RNA
<213> artifical sequence

<400> 92
uugaagcgaa gugcacacgg u 21

<210> 93

<211> 19
<212> RNA
<213> artifical sequence

<400> 93
cgugugcacu ucgcuucaa 19

<210> 94
<211> 21
<212> RNA
<213> artifical sequence

<400> 94
uugaagcgaa gugcacacgg u 21

<210> 95
<211> 19
<212> RNA
<213> artifical sequence

<400> 95
cgugugcacu ucgcuucaa 19

<210> 96
<211> 21
<212> RNA
<213> artifical sequence

<400> 96
uugaagcgaa gugcacacgg u 21

<210> 97
<211> 19
<212> RNA
<213> artifical sequence

<400> 97
cgugugcacu ucgcuucaa 19

<210> 98
<211> 21
<212> RNA
<213> artifical sequence

<400> 98
uugaagcgaa gugcacacgg u 21

<210> 99
<211> 19
<212> RNA
<213> artifical sequence

<400> 99
cgugugcacu ucgcuucaa 19

<210> 100
<211> 21
<212> RNA
<213> artifical sequence

<400> 100
uugaagcgaa gugcacacgg u 21

<210> 101
<211> 19
<212> RNA
<213> artifical sequence

<400> 101
cgugugcacu ucgcuucaa 19

<210> 102
<211> 21
<212> RNA
<213> artifical sequence

<400> 102
uugaagcgaa gugcacacgg u 21

<210> 103
<211> 19
<212> RNA
<213> artifical sequence

<400> 103
cgugugcacu ucgcuucaa 19

<210> 104
<211> 21
<212> RNA
<213> artifical sequence

<400> 104
uugaagcgaa gugcacacgg u 21

<210> 105
<211> 19
<212> RNA
<213> artifical sequence

<400> 105
cgugugcacu ucgcuucaa 19

<210> 106
<211> 21
<212> RNA
<213> artifical sequence

<400> 106
uugaagcgaa gugcacacgg u 21

<210> 107
<211> 19
<212> RNA
<213> artifical sequence

<400> 107
cgugugcacu ucgcuucaa 19

<210> 108
<211> 21
<212> RNA
<213> artifical sequence

<400> 108
uugaagcgaa gugcacacgg u 21

<210> 109
<211> 19
<212> RNA
<213> artifical sequence

<400> 109
gaaaguaugu caacgaauu 19

<210> 110
<211> 21
<212> RNA
<213> artifical sequence

<400> 110
aauucguuga cauacuuucu u 21

<210> 111
<211> 19
<212> RNA
<213> artifical sequence

<400> 111
gaaaguaugu caacgaauu 19

<210> 112
<211> 21
<212> RNA
<213> artifical sequence

<400> 112
aauucguuga cauacuuucc a 21

<210> 113
<211> 19
<212> RNA
<213> artifical sequence

<400> 113
gaaaguaugu caacgaaua 19

<210> 114
<211> 21
<212> RNA
<213> artifical sequence

<400> 114
uauucguuga cauacuuucu u 21

<210> 115
<211> 19
<212> RNA
<213> artifical sequence

<400> 115
gaaaguaugu caacgaaua 19

<210> 116
<211> 21
<212> RNA
<213> artifical sequence

<400> 116

uauucguuga cauacuuucc a 21

<210> 117
<211> 21
<212> RNA
<213> artifical sequence

<400> 117
uggaaaguau gucaacgaau a 21

<210> 118
<211> 23
<212> RNA
<213> artifical sequence

<400> 118
uauucguuga cauacuuucc auu 23

<210> 119
<211> 21
<212> RNA
<213> artifical sequence

<400> 119
uggaaaguau gucaacgaau a 21

<210> 120
<211> 23
<212> RNA
<213> artifical sequence

<400> 120
uauucguuga cauacuuucc aau 23

<210> 121
<211> 21
<212> RNA
<213> artifical sequence

<400> 121
uggaaaguau gucaacgaau u 21

<210> 122
<211> 23
<212> RNA
<213> artifical sequence

<400> 122
aauucguuga cauacuuucc auu 23

<210> 123
<211> 19
<212> RNA
<213> artifical sequence

<400> 123
gaaaguaugu caacgaauu 19

<210> 124
<211> 21
<212> RNA
<213> artifical sequence

<400> 124
aauucguuga cauacuuucu u 21

<210> 125
<211> 19
<212> RNA
<213> artifical sequence

<400> 125
gaaaguaugu caacgaauu 19

<210> 126
<211> 21
<212> RNA
<213> artifical sequence

<400> 126
aauucguuga cauacuuucc a 21

<210> 127
<211> 19
<212> RNA
<213> artifical sequence

<400> 127
gaaaguaugu caacgaaua 19

<210> 128
<211> 21
<212> RNA
<213> artifical sequence

<400> 128
uauucguuga cauacuuucu u 21

<210> 129
<211> 19
<212> RNA
<213> artifical sequence

<400> 129
gaaaguaugu caacgaaua 19

<210> 130
<211> 21
<212> RNA
<213> artifical sequence

<400> 130
uauucguuga cauacuuucc a 21

<210> 131
<211> 19
<212> RNA
<213> artifical sequence

<400> 131
gaaaguaugu caacgaauu 19

<210> 132
<211> 21
<212> RNA
<213> artifical sequence

<400> 132
aauucguuga cauacuuucu u 21

<210> 133
<211> 19
<212> RNA
<213> artifical sequence

<400> 133
gaaaguaugu caacgaauu 19

<210> 134
<211> 21
<212> RNA
<213> artifical sequence

<400> 134
aauucguuga cauacuuucc a 21

<210> 135
<211> 19
<212> RNA
<213> artifical sequence

<400> 135
gaaaguaugu caacgaaua 19

<210> 136
<211> 21
<212> RNA
<213> artifical sequence

<400> 136
uauucguuga cauacuuucu u 21

<210> 137
<211> 19
<212> RNA
<213> artifical sequence

<400> 137
gaaaguaugu caacgaaua 19

<210> 138
<211> 21
<212> RNA
<213> artifical sequence

<400> 138
uauucguuga cauacuuucc a 21

<210> 139
<211> 19
<212> RNA
<213> artifical sequence

<400> 139
gaaaguaugu caacgaauu 19

<210> 140
<211> 21
<212> RNA

<213> artifical sequence

<400> 140
aauucguuga cauacuuucu u 21

<210> 141
<211> 19
<212> RNA
<213> artifical sequence

<400> 141
gaaaguaugu caacgaauu 19

<210> 142
<211> 21
<212> RNA
<213> artifical sequence

<400> 142
aauucguuga cauacuuucc a 21

<210> 143
<211> 19
<212> RNA
<213> artifical sequence

<400> 143
gaaaguaugu caacgaaua 19

<210> 144
<211> 21
<212> RNA
<213> artifical sequence

<400> 144
uauucguuga cauacuuucu u 21

<210> 145
<211> 19
<212> RNA
<213> artifical sequence

<400> 145
gaaaguaugu caacgaaua 19

<210> 146
<211> 21
<212> RNA
<213> artifical sequence

<400> 146
uauucguuga cauacuuucc a 21

<210> 147
<211> 21
<212> RNA
<213> artifical sequence

<400> 147
uggaaaguau gucaacgaau a 21

<210> 148
<211> 23

<212> RNA
<213> artifical sequence

<400> 148
uauucguuga cauacuuucc auu 23

<210> 149
<211> 21
<212> RNA
<213> artifical sequence

<400> 149
uggaaaguau gucaacgaau a 21

<210> 150
<211> 23
<212> RNA
<213> artifical sequence

<400> 150
uauucguuga cauacuuucc aau 23

<210> 151
<211> 21
<212> RNA
<213> artifical sequence

<400> 151
uggaaaguau gucaacgaau u 21

<210> 152
<211> 23
<212> RNA
<213> artifical sequence

<400> 152
aauucguuga cauacuuucc auu 23

<210> 153
<211> 19
<212> RNA
<213> artifical sequence

<400> 153
gaaaguaugu caacgaauu 19

<210> 154
<211> 21
<212> RNA
<213> artifical sequence

<400> 154
aauucguuga cauacuuucu u 21

<210> 155
<211> 19
<212> RNA
<213> artifical sequence

<400> 155
gaaaguaugu caacgaauu 19

<210> 156

<211> 21
<212> RNA
<213> artifical sequence

<400> 156
aauucguuga cauacuuucc a 21

<210> 157
<211> 19
<212> RNA
<213> artifical sequence

<400> 157
gaaaguaugu caacgaaua 19

<210> 158
<211> 21
<212> RNA
<213> artifical sequence

<400> 158
uauucguuga cauacuuucu u 21

<210> 159
<211> 19
<212> RNA
<213> artifical sequence

<400> 159
gaaaguaugu caacgaaua 19

<210> 160
<211> 21
<212> RNA
<213> artifical sequence

<400> 160
uauucguuga cauacuuucc a 21

<210> 161
<211> 19
<212> RNA
<213> artifical sequence

<400> 161
gaaaguaugu caacgaauu 19

<210> 162
<211> 21
<212> RNA
<213> artifical sequence

<400> 162
aauucguuga cauacuuucu u 21

<210> 163
<211> 19
<212> RNA
<213> artifical sequence

<400> 163
gaaaguaugu caacgaauu 19

<210> 164
<211> 21
<212> RNA
<213> artifical sequence

<400> 164
aauucguuga cauacuuucc a 21

<210> 165
<211> 19
<212> RNA
<213> artifical sequence

<400> 165
gaaaguaugu caacgaaua 19

<210> 166
<211> 21
<212> RNA
<213> artifical sequence

<400> 166
uauucguuga cauacuuucu u 21

<210> 167
<211> 19
<212> RNA
<213> artifical sequence

<400> 167
gaaaguaugu caacgaaua 19

<210> 168
<211> 21
<212> RNA
<213> artifical sequence

<400> 168
uauucguuga cauacuuucc a 21

<210> 169
<211> 19
<212> RNA
<213> artifical sequence

<400> 169
gaaaguaugu caacgaauu 19

<210> 170
<211> 21
<212> RNA
<213> artifical sequence

<400> 170
aauucguuga cauacuuucu u 21

<210> 171
<211> 19
<212> RNA
<213> artifical sequence

<400> 171
gaaaguaugu caacgaauu 19

<210> 172
<211> 21
<212> RNA
<213> artifical sequence

<400> 172
aauucguuga cauacuuucc a 21

<210> 173
<211> 19
<212> RNA
<213> artifical sequence

<400> 173
gaaaguaugu caacgaaua 19

<210> 174
<211> 21
<212> RNA
<213> artifical sequence

<400> 174
uauucguuga cauacuuucu u 21

<210> 175
<211> 19
<212> RNA
<213> artifical sequence

<400> 175
gaaaguaugu caacgaaua 19

<210> 176
<211> 21
<212> RNA
<213> artifical sequence

<400> 176
uauucguuga cauacuuucc a 21

<210> 177
<211> 21
<212> RNA
<213> artifical sequence

<400> 177
uggaaaguau gucaacgaau a 21

<210> 178
<211> 23
<212> RNA
<213> artifical sequence

<400> 178
uauucguuga cauacuuucc auu 23

<210> 179
<211> 21
<212> RNA
<213> artifical sequence

<400> 179

uggaaaguau gucaacgaau a 21

<210> 180
<211> 23
<212> RNA
<213> artifical sequence

<400> 180
uauucguuga cauacuuucc aau 23

<210> 181
<211> 21
<212> RNA
<213> artifical sequence

<400> 181
uggaaaguau gucaacgaau u 21

<210> 182
<211> 23
<212> RNA
<213> artifical sequence

<400> 182
aauucguuga cauacuuucc auu 23

<210> 183
<211> 19
<212> RNA
<213> artifical sequence

<400> 183
gaaaguaugu caacgaauu 19

<210> 184
<211> 21
<212> RNA
<213> artifical sequence

<400> 184
aauucguuga cauacuuucu u 21

<210> 185
<211> 19
<212> RNA
<213> artifical sequence

<400> 185
gaaaguaugu caacgaauu 19

<210> 186
<211> 21
<212> RNA
<213> artifical sequence

<400> 186
aauucguuga cauacuuucc a 21

<210> 187
<211> 19
<212> RNA
<213> artifical sequence

<400> 187
gaaaguaugu caacgaaua 19

<210> 188
<211> 21
<212> RNA
<213> artifical sequence

<400> 188
uauucguuga cauacuuucu u 21

<210> 189
<211> 19
<212> RNA
<213> artifical sequence

<400> 189
gaaaguaugu caacgaaua 19

<210> 190
<211> 21
<212> RNA
<213> artifical sequence

<400> 190
uauucguuga cauacuuucc a 21

<210> 191
<211> 19
<212> RNA
<213> artifical sequence

<400> 191
gaaaguaugu caacgaauu 19

<210> 192
<211> 21
<212> RNA
<213> artifical sequence

<400> 192
aauucguuga cauacuuucu u 21

<210> 193
<211> 19
<212> RNA
<213> artifical sequence

<400> 193
gaaaguaugu caacgaauu 19

<210> 194
<211> 21
<212> RNA
<213> artifical sequence

<400> 194
aauucguuga cauacuuucc a 21

<210> 195
<211> 19
<212> RNA
<213> artifical sequence

<400> 195
gaaaguaugu caacgaaua 19

<210> 196
<211> 21
<212> RNA
<213> artifical sequence

<400> 196
uauucguuga cauacuuucu u 21

<210> 197
<211> 19
<212> RNA
<213> artifical sequence

<400> 197
gaaaguaugu caacgaaua 19

<210> 198
<211> 21
<212> RNA
<213> artifical sequence

<400> 198
uauucguuga cauacuuucc a 21

<210> 199
<211> 19
<212> RNA
<213> artifical sequence

<400> 199
gaaaguaugu caacgaauu 19

<210> 200
<211> 21
<212> RNA
<213> artifical sequence

<400> 200
aauucguuga cauacuuucu u 21

<210> 201
<211> 19
<212> RNA
<213> artifical sequence

<400> 201
gaaaguaugu caacgaauu 19

<210> 202
<211> 21
<212> RNA
<213> artifical sequence

<400> 202
aauucguuga cauacuuucc a 21

<210> 203
<211> 19
<212> RNA

<213> artifical sequence

<400> 203
gaaaguaugu caacgaaua 19

<210> 204
<211> 21
<212> RNA
<213> artifical sequence

<400> 204
uauucguuga cauacuuucu u 21

<210> 205
<211> 19
<212> RNA
<213> artifical sequence

<400> 205
gaaaguaugu caacgaaua 19

<210> 206
<211> 21
<212> RNA
<213> artifical sequence

<400> 206
uauucguuga cauacuuucc a 21

<210> 207
<211> 21
<212> RNA
<213> artifical sequence

<400> 207
uggaaaguau gucaacgaau a 21

<210> 208
<211> 23
<212> RNA
<213> artifical sequence

<400> 208
uauucguuga cauacuuucc auu 23

<210> 209
<211> 21
<212> RNA
<213> artifical sequence

<400> 209
uggaaaguau gucaacgaau a 21

<210> 210
<211> 23
<212> RNA
<213> artifical sequence

<400> 210
uauucguuga cauacuuucc aau 23

<210> 211
<211> 21

<212> RNA
<213> artifical sequence

<400> 211
uggaaaguau gucaacgaau u 21

<210> 212
<211> 23
<212> RNA
<213> artifical sequence

<400> 212
aauucguuga cauacuuucc auu 23

<210> 213
<211> 19
<212> RNA
<213> artifical sequence

<400> 213
gaaaguaugu caacgaauu 19

<210> 214
<211> 21
<212> RNA
<213> artifical sequence

<400> 214
aauucguuga cauacuuucu u 21

<210> 215
<211> 19
<212> RNA
<213> artifical sequence

<400> 215
gaaaguaugu caacgaauu 19

<210> 216
<211> 21
<212> RNA
<213> artifical sequence

<400> 216
aauucguuga cauacuuucc a 21

<210> 217
<211> 19
<212> RNA
<213> artifical sequence

<400> 217
gaaaguaugu caacgaaua 19

<210> 218
<211> 21
<212> RNA
<213> artifical sequence

<400> 218
uauucguuga cauacuuucu u 21

<210> 219

<211> 19
<212> RNA
<213> artifical sequence

<400> 219
gaaaguaugu caacgaaua 19

<210> 220
<211> 21
<212> RNA
<213> artifical sequence

<400> 220
uauucguuga cauacuuucc a 21

<210> 221
<211> 19
<212> RNA
<213> artifical sequence

<400> 221
gaaaguaugu caacgaauu 19

<210> 222
<211> 21
<212> RNA
<213> artifical sequence

<400> 222
aauucguuga cauacuuucu u 21

<210> 223
<211> 19
<212> RNA
<213> artifical sequence

<400> 223
gaaaguaugu caacgaauu 19

<210> 224
<211> 21
<212> RNA
<213> artifical sequence

<400> 224
aauucguuga cauacuuucc a 21

<210> 225
<211> 19
<212> RNA
<213> artifical sequence

<400> 225
gaaaguaugu caacgaaua 19

<210> 226
<211> 21
<212> RNA
<213> artifical sequence

<400> 226
uauucguuga cauacuuucu u 21

<210> 227
<211> 19
<212> RNA
<213> artifical sequence

<400> 227
gaaaguaugu caacgaaua 19

<210> 228
<211> 21
<212> RNA
<213> artifical sequence

<400> 228
uauucguuga cauacuuucc a 21

<210> 229
<211> 19
<212> RNA
<213> artifical sequence

<400> 229
gaaaguaugu caacgaauu 19

<210> 230
<211> 21
<212> RNA
<213> artifical sequence

<400> 230
aauucguuga cauacuuucu u 21

<210> 231
<211> 19
<212> RNA
<213> artifical sequence

<400> 231
gaaaguaugu caacgaauu 19

<210> 232
<211> 21
<212> RNA
<213> artifical sequence

<400> 232
aauucguuga cauacuuucc a 21

<210> 233
<211> 19
<212> RNA
<213> artifical sequence

<400> 233
gaaaguaugu caacgaaua 19

<210> 234
<211> 21
<212> RNA
<213> artifical sequence

<400> 234
uauucguuga cauacuuucu u 21

<210> 235
<211> 19
<212> RNA
<213> artifical sequence

<400> 235
gaaaguaugu caacgaaua 19

<210> 236
<211> 21
<212> RNA
<213> artifical sequence

<400> 236
uauucguuga cauacuuucc a 21

<210> 237
<211> 21
<212> RNA
<213> artifical sequence

<400> 237
uggaaaguau gucaacgaau a 21

<210> 238
<211> 23
<212> RNA
<213> artifical sequence

<400> 238
uauucguuga cauacuuucc auu 23

<210> 239
<211> 21
<212> RNA
<213> artifical sequence

<400> 239
uggaaaguau gucaacgaau a 21

<210> 240
<211> 23
<212> RNA
<213> artifical sequence

<400> 240
uauucguuga cauacuuucc aau 23

<210> 241
<211> 21
<212> RNA
<213> artifical sequence

<400> 241
uggaaaguau gucaacgaau u 21

<210> 242
<211> 23
<212> RNA
<213> artifical sequence

<400> 242

aauucguuga cauacuuucc auu 23

<210> 243
<211> 19
<212> RNA
<213> artifical sequence

<400> 243
ccaagagcac caagaacua 19

<210> 244
<211> 21
<212> RNA
<213> artifical sequence

<400> 244
uaguucuugg ugcucuuggc u 21

<210> 245
<211> 21
<212> RNA
<213> artifical sequence

<400> 245
agccaagagc accaagaacu a 21

<210> 246
<211> 23
<212> RNA
<213> artifical sequence

<400> 246
uaguucuugg ugcucuuggc uug 23

<210> 247
<211> 19
<212> RNA
<213> artifical sequence

<400> 247
ccaagagcac caagaacua 19

<210> 248
<211> 21
<212> RNA
<213> artifical sequence

<400> 248
uaguucuugg ugcucuuggc u 21

<210> 249
<211> 21
<212> RNA
<213> artifical sequence

<400> 249
agccaagagc accaagaacu a 21

<210> 250
<211> 23
<212> RNA
<213> artifical sequence

<400> 250
uaguucuugg ugcucuuggc uug 23

<210> 251
<211> 19
<212> RNA
<213> artifical sequence

<400> 251
ccaagagcac caagaacua 19

<210> 252
<211> 21
<212> RNA
<213> artifical sequence

<400> 252
uaguucuugg ugcucuuggc u 21

<210> 253
<211> 21
<212> RNA
<213> artifical sequence

<400> 253
agccaagagc accaagaacu a 21

<210> 254
<211> 23
<212> RNA
<213> artifical sequence

<400> 254
uaguucuugg ugcucuuggc uug 23

<210> 255
<211> 19
<212> RNA
<213> artifical sequence

<400> 255
ccaagagcac caagaacua 19

<210> 256
<211> 21
<212> RNA
<213> artifical sequence

<400> 256
uaguucuugg ugcucuuggc u 21

<210> 257
<211> 21
<212> RNA
<213> artifical sequence

<400> 257
agccaagagc accaagaacu a 21

<210> 258
<211> 23
<212> RNA
<213> artifical sequence

<400> 258
uaguucuugg ugcucuuggc uug 23

<210> 259
<211> 19
<212> RNA
<213> artifical sequence

<400> 259
ccaagagcac caagaacua 19

<210> 260
<211> 21
<212> RNA
<213> artifical sequence

<400> 260
uaguucuugg ugcucuuggc u 21

<210> 261
<211> 21
<212> RNA
<213> artifical sequence

<400> 261
agccaagagc accaagaacu a 21

<210> 262
<211> 23
<212> RNA
<213> artifical sequence

<400> 262
uaguucuugg ugcucuuggc uug 23

<210> 263
<211> 19
<212> RNA
<213> artifical sequence

<400> 263
ccaagagcac caagaacua 19

<210> 264
<211> 21
<212> RNA
<213> artifical sequence

<400> 264
uaguucuugg ugcucuuggc u 21

<210> 265
<211> 21
<212> RNA
<213> artifical sequence

<400> 265
agccaagagc accaagaacu a 21

<210> 266
<211> 23
<212> RNA

<213> artifical sequence

<400> 266
uaguucuugg ugcucuuggc uug 23

<210> 267
<211> 19
<212> RNA
<213> artifical sequence

<400> 267
ccaagagcac caagaacua 19

<210> 268
<211> 21
<212> RNA
<213> artifical sequence

<400> 268
uaguucuugg ugcucuuggc u 21

<210> 269
<211> 21
<212> RNA
<213> artifical sequence

<400> 269
agccaagagc accaagaacu a 21

<210> 270
<211> 23
<212> RNA
<213> artifical sequence

<400> 270
uaguucuugg ugcucuuggc uug 23

<210> 271
<211> 19
<212> RNA
<213> artifical sequence

<400> 271
ccaagagcac caagaacua 19

<210> 272
<211> 21
<212> RNA
<213> artifical sequence

<400> 272
uaguucuugg ugcucuuggc u 21

<210> 273
<211> 21
<212> RNA
<213> artifical sequence

<400> 273
agccaagagc accaagaacu a 21

<210> 274
<211> 23

<212> RNA
<213> artifical sequence

<400> 274
uaguucuugg ugcucuuggc uug 23

<210> 275
<211> 19
<212> RNA
<213> artifical sequence

<400> 275
ccaagagcac caagaacua 19

<210> 276
<211> 21
<212> RNA
<213> artifical sequence

<400> 276
uaguucuugg ugcucuuggc u 21

<210> 277
<211> 21
<212> RNA
<213> artifical sequence

<400> 277
agccaagagc accaagaacu a 21

<210> 278
<211> 23
<212> RNA
<213> artifical sequence

<400> 278
uaguucuugg ugcucuuggc uug 23

<210> 279
<211> 19
<212> RNA
<213> artifical sequence

<400> 279
ccaagagcac caagaacua 19

<210> 280
<211> 21
<212> RNA
<213> artifical sequence

<400> 280
uaguucuugg ugcucuuggc u 21

<210> 281
<211> 21
<212> RNA
<213> artifical sequence

<400> 281
agccaagagc accaagaacu a 21

<210> 282

<211> 23
<212> RNA
<213> artifical sequence

<400> 282
uaguucuugg ugcucuuggc uug 23

<210> 283
<211> 19
<212> RNA
<213> artifical sequence

<400> 283
ccaagagcac caagaacua 19

<210> 284
<211> 21
<212> RNA
<213> artifical sequence

<400> 284
uaguucuugg ugcucuuggc u 21

<210> 285
<211> 21
<212> RNA
<213> artifical sequence

<400> 285
agccaagagc accaagaacu a 21

<210> 286
<211> 23
<212> RNA
<213> artifical sequence

<400> 286
uaguucuugg ugcucuuggc uug 23

<210> 287
<211> 19
<212> RNA
<213> artifical sequence

<400> 287
ccaagagcac caagaacua 19

<210> 288
<211> 21
<212> RNA
<213> artifical sequence

<400> 288
uaguucuugg ugcucuuggc u 21

<210> 289
<211> 21
<212> RNA
<213> artifical sequence

<400> 289
agccaagagc accaagaacu a 21

<210> 290
<211> 23
<212> RNA
<213> artifical sequence

<400> 290
uaguucuugg ugcucuuggc uug 23

<210> 291
<211> 19
<212> RNA
<213> artifical sequence

<400> 291
ccaagagcac caagaacua 19

<210> 292
<211> 21
<212> RNA
<213> artifical sequence

<400> 292
uaguucuugg ugcucuuggc u 21

<210> 293
<211> 21
<212> RNA
<213> artifical sequence

<400> 293
agccaagagc accaagaacu a 21

<210> 294
<211> 23
<212> RNA
<213> artifical sequence

<400> 294
uaguucuugg ugcucuuggc uug 23

<210> 295
<211> 19
<212> RNA
<213> artifical sequence

<400> 295
caauaaagcu ggacaagaa 19

<210> 296
<211> 21
<212> RNA
<213> artifical sequence

<400> 296
uucuugucca gcuuuauugg g 21

<210> 297
<211> 21
<212> RNA
<213> artifical sequence

<400> 297
cccaauaaag cuggacaaga a 21

<210> 298
<211> 23
<212> RNA
<213> artifical sequence

<400> 298
uucuugucca gcuuuauugg gag 23

<210> 299
<211> 19
<212> RNA
<213> artifical sequence

<400> 299
caauaaagcu ggacaagaa 19

<210> 300
<211> 21
<212> RNA
<213> artifical sequence

<400> 300
uucuugucca gcuuuauugg g 21

<210> 301
<211> 21
<212> RNA
<213> artifical sequence

<400> 301
cccaauaaag cuggacaaga a 21

<210> 302
<211> 23
<212> RNA
<213> artifical sequence

<400> 302
uucuugucca gcuuuauugg gag 23

<210> 303
<211> 19
<212> RNA
<213> artifical sequence

<400> 303
caauaaagcu ggacaagaa 19

<210> 304
<211> 21
<212> RNA
<213> artifical sequence

<400> 304
uucuugucca gcuuuauugg g 21

<210> 305
<211> 21
<212> RNA
<213> artifical sequence

<400> 305

cccaauaaag cuggacaaga a 21

<210> 306
<211> 23
<212> RNA
<213> artifical sequence

<400> 306
uucuugucca gcuuuauugg gag 23

<210> 307
<211> 19
<212> RNA
<213> artifical sequence

<400> 307
caauaaagcu ggacaagaa 19

<210> 308
<211> 21
<212> RNA
<213> artifical sequence

<400> 308
uucuugucca gcuuuauugg g 21

<210> 309
<211> 21
<212> RNA
<213> artifical sequence

<400> 309
cccaauaaag cuggacaaga a 21

<210> 310
<211> 23
<212> RNA
<213> artifical sequence

<400> 310
uucuugucca gcuuuauugg gag 23

<210> 311
<211> 19
<212> RNA
<213> artifical sequence

<400> 311
caauaaagcu ggacaagaa 19

<210> 312
<211> 21
<212> RNA
<213> artifical sequence

<400> 312
uucuugucca gcuuuauugg g 21

<210> 313
<211> 21
<212> RNA
<213> artifical sequence

<400> 313
cccaauaaag cuggacaaga a 21

<210> 314
<211> 23
<212> RNA
<213> artifical sequence

<400> 314
uucuugucca gcuuuauugg gag 23

<210> 315
<211> 19
<212> RNA
<213> artifical sequence

<400> 315
caauaaagcu ggacaagaa 19

<210> 316
<211> 21
<212> RNA
<213> artifical sequence

<400> 316
uucuugucca gcuuuauugg g 21

<210> 317
<211> 21
<212> RNA
<213> artifical sequence

<400> 317
cccaauaaag cuggacaaga a 21

<210> 318
<211> 23
<212> RNA
<213> artifical sequence

<400> 318
uucuugucca gcuuuauugg gag 23

<210> 319
<211> 19
<212> RNA
<213> artifical sequence

<400> 319
caauaaagcu ggacaagaa 19

<210> 320
<211> 21
<212> RNA
<213> artifical sequence

<400> 320
uucuugucca gcuuuauugg g 21

<210> 321
<211> 21
<212> RNA
<213> artifical sequence

<400> 321
cccaauaaag cuggacaaga a 21

<210> 322
<211> 23
<212> RNA
<213> artifical sequence

<400> 322
uucuugucca gcuuuauugg gag 23

<210> 323
<211> 19
<212> RNA
<213> artifical sequence

<400> 323
caauaaagcu ggacaagaa 19

<210> 324
<211> 21
<212> RNA
<213> artifical sequence

<400> 324
uucuugucca gcuuuauugg g 21

<210> 325
<211> 21
<212> RNA
<213> artifical sequence

<400> 325
cccaauaaag cuggacaaga a 21

<210> 326
<211> 23
<212> RNA
<213> artifical sequence

<400> 326
uucuugucca gcuuuauugg gag 23

<210> 327
<211> 19
<212> RNA
<213> artifical sequence

<400> 327
caauaaagcu ggacaagaa 19

<210> 328
<211> 21
<212> RNA
<213> artifical sequence

<400> 328
uucuugucca gcuuuauugg g 21

<210> 329
<211> 21
<212> RNA

<213> artifical sequence

<400> 329
cccaauaaag cuggacaaga a 21

<210> 330
<211> 23
<212> RNA
<213> artifical sequence

<400> 330
uucuugucca gcuuuauugg gag 23

<210> 331
<211> 19
<212> RNA
<213> artifical sequence

<400> 331
ccuugaggca uacuucaaa 19

<210> 332
<211> 21
<212> RNA
<213> artifical sequence

<400> 332
uuugaaguau gccucaaggu u 21

<210> 333
<211> 21
<212> RNA
<213> artifical sequence

<400> 333
gaccuugagg cauacuucaa a 21

<210> 334
<211> 23
<212> RNA
<213> artifical sequence

<400> 334
uuugaaguau gccucaaggu cgg 23

<210> 335
<211> 19
<212> RNA
<213> artifical sequence

<400> 335
ccuugaggca uacuucaaa 19

<210> 336
<211> 21
<212> RNA
<213> artifical sequence

<400> 336
uuugaaguau gccucaaggu u 21

<210> 337
<211> 19

<212> RNA
<213> artifical sequence

<400> 337
ccuugaggca uacuucaaa 19

<210> 338
<211> 21
<212> RNA
<213> artifical sequence

<400> 338
uuugaaguau gccucaaggu u 21

<210> 339
<211> 21
<212> RNA
<213> artifical sequence

<400> 339
gaccuugagg cauacuucaa a 21

<210> 340
<211> 23
<212> RNA
<213> artifical sequence

<400> 340
uuugaaguau gccucaaggu cgg 23

<210> 341
<211> 21
<212> RNA
<213> artifical sequence

<400> 341
gaccuugagg cauacuucaa a 21

<210> 342
<211> 23
<212> RNA
<213> artifical sequence

<400> 342
uuugaaguau gccucaaggu cgg 23

<210> 343
<211> 19
<212> RNA
<213> artifical sequence

<400> 343
ccuugaggca uacuucaaa 19

<210> 344
<211> 21
<212> RNA
<213> artifical sequence

<400> 344
uuugaaguau gccucaaggu u 21

<210> 345

<211> 19
<212> RNA
<213> artifical sequence

<400> 345
ccuugaggca uacuucaaa 19

<210> 346
<211> 21
<212> RNA
<213> artifical sequence

<400> 346
uuugaaguau gccucaaggu u 21

<210> 347
<211> 21
<212> RNA
<213> artifical sequence

<400> 347
gaccuugagg cauacuucaa a 21

<210> 348
<211> 23
<212> RNA
<213> artifical sequence

<400> 348
uuugaaguau gccucaaggu cgg 23

<210> 349
<211> 21
<212> RNA
<213> artifical sequence

<400> 349
gaccuugagg cauacuucaa a 21

<210> 350
<211> 23
<212> RNA
<213> artifical sequence

<400> 350
uuugaaguau gccucaaggu cgg 23

<210> 351
<211> 19
<212> RNA
<213> artifical sequence

<400> 351
ccuugaggca uacuucaaa 19

<210> 352
<211> 21
<212> RNA
<213> artifical sequence

<400> 352
uuugaaguau gccucaaggu u 21

<210> 353
<211> 19
<212> RNA
<213> artifical sequence

<400> 353
ccuugaggca uacuucaaa 19

<210> 354
<211> 21
<212> RNA
<213> artifical sequence

<400> 354
uuugaaguau gccucaaggu u 21

<210> 355
<211> 21
<212> RNA
<213> artifical sequence

<400> 355
gaccuugagg cauacuucaa a 21

<210> 356
<211> 23
<212> RNA
<213> artifical sequence

<400> 356
uuugaaguau gccucaaggu cgg 23

<210> 357
<211> 21
<212> RNA
<213> artifical sequence

<400> 357
gaccuugagg cauacuucaa a 21

<210> 358
<211> 23
<212> RNA
<213> artifical sequence

<400> 358
uuugaaguau gccucaaggu cgg 23

<210> 359
<211> 19
<212> RNA
<213> artifical sequence

<400> 359
ccuugaggca uacuucaaa 19

<210> 360
<211> 21
<212> RNA
<213> artifical sequence

<400> 360
uuugaaguau gccucaaggu u 21

<210> 361
<211> 19
<212> RNA
<213> artifical sequence

<400> 361
ccuugaggca uacuucaaa 19

<210> 362
<211> 21
<212> RNA
<213> artifical sequence

<400> 362
uuugaaguau gccucaaggu u 21

<210> 363
<211> 19
<212> RNA
<213> artifical sequence

<400> 363
ccuugaggca uacuucaaa 19

<210> 364
<211> 21
<212> RNA
<213> artifical sequence

<400> 364
uuugaaguau gccucaaggu u 21

<210> 365
<211> 19
<212> RNA
<213> artifical sequence

<400> 365
ccuugaggca uacuucaau 19

<210> 366
<211> 21
<212> RNA
<213> artifical sequence

<400> 366
auugaaguau gccucaaggu u 21

<210> 367
<211> 19
<212> RNA
<213> artifical sequence

<400> 367
ccuugaggca uacuucaaa 19

<210> 368
<211> 21
<212> RNA
<213> artifical sequence

<400> 368

uuugaaguau gccucaaggu u 21

<210> 369
<211> 21
<212> RNA
<213> artifical sequence

<400> 369
gaccuugagg cauacuucaa a 21

<210> 370
<211> 23
<212> RNA
<213> artifical sequence

<400> 370
uuugaaguau gccucaaggu cgg 23

<210> 371
<211> 21
<212> RNA
<213> artifical sequence

<400> 371
gaccuugagg cauacuucaa a 21

<210> 372
<211> 23
<212> RNA
<213> artifical sequence

<400> 372
uuugaaguau gccucaaggu cgg 23

<210> 373
<211> 19
<212> RNA
<213> artifical sequence

<400> 373
ccuugaggca uacuucaaa 19

<210> 374
<211> 21
<212> RNA
<213> artifical sequence

<400> 374
uuugaaguau gccucaaggu u 21

<210> 375
<211> 19
<212> RNA
<213> artifical sequence

<400> 375
ccuugaggca uacuucaaa 19

<210> 376
<211> 21
<212> RNA
<213> artifical sequence

<400> 376
uuugaaguau gccucaaggu u 21

<210> 377
<211> 19
<212> RNA
<213> artifical sequence

<400> 377
ccuugaggca uacuucaaa 19

<210> 378
<211> 21
<212> RNA
<213> artifical sequence

<400> 378
uuugaaguau gccucaaggu u 21

<210> 379
<211> 19
<212> RNA
<213> artifical sequence

<400> 379
ccuugaggca uacuucaaa 19

<210> 380
<211> 21
<212> RNA
<213> artifical sequence

<400> 380
uuugaaguau gccucaaggu u 21

<210> 381
<211> 19
<212> RNA
<213> artifical sequence

<400> 381
ccuugaggca uacuucaaa 19

<210> 382
<211> 21
<212> RNA
<213> artifical sequence

<400> 382
uuugaaguau gccucaaggu u 21

<210> 383
<211> 19
<212> RNA
<213> artifical sequence

<400> 383
ccuugaggca uacuucaaa 19

<210> 385
<211> 21
<212> RNA
<213> artifical sequence

<400> 385
uuugaaguau gccucaaggu u 21

<210> 385
<211> 19
<212> RNA
<213> artifical sequence

<400> 385
ccuugaggca uacuucaaa 19

<210> 386
<211> 21
<212> RNA
<213> artifical sequence

<400> 386
uuugaaguau gccucaaggu u 21

<210> 387
<211> 19
<212> RNA
<213> artifical sequence

<400> 387
ccuugaggca uacuucaaa 19

<210> 388
<211> 21
<212> RNA
<213> artifical sequence

<400> 388
uuugaaguau gccucaaggu u 21

<210> 389
<211> 19
<212> RNA
<213> artifical sequence

<400> 389
uucuccgaac gugucacgu 19

<210> 390
<211> 21
<212> RNA
<213> artifical sequence

<400> 390
acgugacacg uucggagaau u 21

<210> 391
<211> 19
<212> RNA
<213> artifical sequence

<400> 391
ugcuaugccu caucuucua 19

<210> 392
<211> 21
<212> RNA

<213> artifical sequence

<400> 392
uagaagauga ggcauagcag c 21

<210> 393
<211> 19
<212> RNA
<213> artifical sequence

<400> 393
ugcuaugccu caucuucua 19

<210> 394
<211> 21
<212> RNA
<213> artifical sequence

<400> 394
uagaagauga ggcauagcau u 21

<210> 395
<211> 19
<212> RNA
<213> artifical sequence

<400> 395
ugcuaugccu caucuucua 19

<210> 396
<211> 21
<212> RNA
<213> artifical sequence

<400> 396
uagaagauga ggcauagcag c 21

<210> 397
<211> 19
<212> RNA
<213> artifical sequence

<400> 397
ugcuaugccu caucuucua 19

<210> 398
<211> 21
<212> RNA
<213> artifical sequence

<400> 398
uagaagauga ggcauagcau u 21

<210> 399
<211> 19
<212> RNA
<213> artifical sequence

<400> 399
ugcuaugccu caucuucua 19

<210> 400
<211> 21

<212> RNA
<213> artifical sequence

<400> 400
uagaagauga ggcauagcag c 21

<210> 401
<211> 19
<212> RNA
<213> artifical sequence

<400> 401
ugcuaugccu caucuucua 19

<210> 402
<211> 21
<212> RNA
<213> artifical sequence

<400> 402
uagaagauga ggcauagcag c 21

<210> 403
<211> 19
<212> RNA
<213> artifical sequence

<400> 403
ugcuaugccu caucuucua 19

<210> 404
<211> 21
<212> RNA
<213> artifical sequence

<400> 404
uagaagauga ggcauagcag c 21

<210> 405
<211> 19
<212> RNA
<213> artifical sequence

<400> 405
ugcuaugccu caucuucuu 19

<210> 406
<211> 21
<212> RNA
<213> artifical sequence

<400> 406
aagaagauga ggcauagcag c 21

<210> 407
<211> 19
<212> RNA
<213> artifical sequence

<400> 407
ugcuaugccu caucuucua 19

<210> 408

<211> 21
<212> RNA
<213> artifical sequence

<400> 408
uagaagauga ggcauagcau u 21

<210> 409
<211> 19
<212> RNA
<213> artifical sequence

<400> 409
ugcuaugccu caucuucua 19

<210> 410
<211> 21
<212> RNA
<213> artifical sequence

<400> 410
uagaagauga ggcauagcag c 21

<210> 411
<211> 19
<212> RNA
<213> artifical sequence

<400> 411
ugcuaugccu caucuucua 19

<210> 412
<211> 21
<212> RNA
<213> artifical sequence

<400> 412
uagaagauga ggcauagcau u 21

<210> 413
<211> 19
<212> RNA
<213> artifical sequence

<400> 413
ugcuaugccu caucuucua 19

<210> 414
<211> 21
<212> RNA
<213> artifical sequence

<400> 414
uagaagauga ggcauagcag c 21

<210> 415
<211> 19
<212> RNA
<213> artifical sequence

<400> 415
ugcuaugccu caucuucua 19

<210> 416
<211> 21
<212> RNA
<213> artifical sequence

<400> 416
uagaagauga ggcauagcag c 21

<210> 417
<211> 19
<212> RNA
<213> artifical sequence

<400> 417
ugcuaugccu caucuucua 19

<210> 418
<211> 21
<212> RNA
<213> artifical sequence

<400> 418
uagaagauga ggcauagcag c 21

<210> 419
<211> 21
<212> RNA
<213> artifical sequence

<400> 419
gcugcuaugc cucaucuucu a 21

<210> 420
<211> 23
<212> RNA
<213> artifical sequence

<400> 420
uagaagauga ggcauagcag cgc 23

<210> 421
<211> 19
<212> RNA
<213> artifical sequence

<400> 421
ugcuaugccu caucuucua 19

<210> 422
<211> 21
<212> RNA
<213> artifical sequence

<400> 422
uagaagauga ggcauagcag c 21

<210> 423
<211> 19
<212> RNA
<213> artifical sequence

<400> 423
ugcuaugccu caucuucua 19

<210> 424
<211> 21
<212> RNA
<213> artifical sequence

<400> 424
uagaagauga ggcauagcau u 21

<210> 425
<211> 19
<212> RNA
<213> artifical sequence

<400> 425
ugcuaugccu caucuucua 19

<210> 426
<211> 21
<212> RNA
<213> artifical sequence

<400> 426
uagaagauga ggcauagcau u 21

<210> 427
<211> 19
<212> RNA
<213> artifical sequence

<400> 427
ugcuaugccu caucuucua 19

<210> 428
<211> 21
<212> RNA
<213> artifical sequence

<400> 428
uagaagauga ggcauagcau u 21

<210> 429
<211> 19
<212> RNA
<213> artifical sequence

<400> 429
ugcuaugccu caucuucua 19

<210> 430
<211> 21
<212> RNA
<213> artifical sequence

<400> 430
uagaagauga ggcauagcau u 21

<210> 431
<211> 19
<212> RNA
<213> artifical sequence

<400> 431

ugcuaugccu caucuucua 19

<210> 432
<211> 21
<212> RNA
<213> artifical sequence

<400> 432
uagaagauga ggcauagcau u 21

<210> 433
<211> 19
<212> RNA
<213> artifical sequence

<400> 433
ugcuaugccu caucuucua 19

<210> 434
<211> 21
<212> RNA
<213> artifical sequence

<400> 434
uagaagauga ggcauagcau u 21

<210> 435
<211> 19
<212> RNA
<213> artifical sequence

<400> 435
ucugugccuu cucaucuga 19

<210> 436
<211> 21
<212> RNA
<213> artifical sequence

<400> 436
ucagaugaga aggcacagac g 21

<210> 437
<211> 19
<212> RNA
<213> artifical sequence

<400> 437
ucugugccuu cucaucuga 19

<210> 438
<211> 21
<212> RNA
<213> artifical sequence

<400> 438
ucagaugaga aggcacagac g 21

<210> 439
<211> 19
<212> RNA
<213> artifical sequence

<400> 439
ucugugccuu cucaucuga 19

<210> 440
<211> 21
<212> RNA
<213> artifical sequence

<400> 440
ucagaugaga aggcacagac g 21

<210> 441
<211> 19
<212> RNA
<213> artifical sequence

<400> 441
ucugugccuu cucaucuga 19

<210> 442
<211> 21
<212> RNA
<213> artifical sequence

<400> 442
ucagaugaga aggcacagac g 21

<210> 443
<211> 19
<212> RNA
<213> artifical sequence

<400> 443
ucugugccuu cucaucuga 19

<210> 444
<211> 21
<212> RNA
<213> artifical sequence

<400> 444
ucagaugaga aggcacagac g 21

<210> 445
<211> 19
<212> RNA
<213> artifical sequence

<400> 445
ucugugccuu cucaucuga 19

<210> 446
<211> 21
<212> RNA
<213> artifical sequence

<400> 446
ucagaugaga aggcacagac g 21

<210> 447
<211> 19
<212> RNA
<213> artifical sequence

<400> 447
ucugugccuu cucaucuga 19

<210> 448
<211> 21
<212> RNA
<213> artifical sequence

<400> 448
ucagaugaga aggcacagac g 21

<210> 449
<211> 19
<212> RNA
<213> artifical sequence

<400> 449
ucugugccuu cucaucuga 19

<210> 450
<211> 21
<212> RNA
<213> artifical sequence

<400> 450
ucagaugaga aggcacagac g 21

<210> 451
<211> 21
<212> RNA
<213> artifical sequence

<400> 451
cgucugugcc uucucaucug a 21

<210> 452
<211> 23
<212> RNA
<213> artifical sequence

<400> 452
ucagaugaga aggcacagac ggg 23

<210> 453
<211> 19
<212> RNA
<213> artifical sequence

<400> 453
ucugugccuu cucaucuga 19

<210> 454
<211> 21
<212> RNA
<213> artifical sequence

<400> 454
ucagaugaga aggcacagac g 21

<210> 455
<211> 19
<212> RNA

<213> artifical sequence

<400> 455
ucugugccuu cucaucuga 19

<210> 456
<211> 21
<212> RNA
<213> artifical sequence

<400> 456
ucagaugaga aggcacagac g 21

<210> 457
<211> 19
<212> RNA
<213> artifical sequence

<400> 457
ucugugccuu cucaucugu 19

<210> 458
<211> 21
<212> RNA
<213> artifical sequence

<400> 458
acagaugaga aggcacagac g 21

<210> 459
<211> 19
<212> RNA
<213> artifical sequence

<400> 459
ucugugccuu cucaucuga 19

<210> 460
<211> 21
<212> RNA
<213> artifical sequence

<400> 460
ucagaugaga aggcacagac g 21

<210> 461
<211> 19
<212> RNA
<213> artifical sequence

<400> 461
ucugugccuu cucaucuga 19

<210> 462
<211> 21
<212> RNA
<213> artifical sequence

<400> 462
ucagaugaga aggcacagac g 21

<210> 463
<211> 19

<212> RNA
<213> artifical sequence

<400> 463
ucugugccuu cucaucuga 19

<210> 464
<211> 21
<212> RNA
<213> artifical sequence

<400> 464
ucagaugaga aggcacagac g 21

<210> 465
<211> 19
<212> RNA
<213> artifical sequence

<400> 465
ucugugccuu cucaucuga 19

<210> 466
<211> 21
<212> RNA
<213> artifical sequence

<400> 466
ucagaugaga aggcacagac g 21

<210> 467
<211> 19
<212> RNA
<213> artifical sequence

<400> 467
ucugugccuu cucaucuga 19

<210> 468
<211> 21
<212> RNA
<213> artifical sequence

<400> 468
ucagaugaga aggcacagac g 21

<210> 469
<211> 19
<212> RNA
<213> artifical sequence

<400> 469
cgugugcacu ucgcuucaa 19

<210> 470
<211> 21
<212> RNA
<213> artifical sequence

<400> 470
uugaagcgaa gugcacacgg u 21

<210> 471

<211> 19
<212> RNA
<213> artifical sequence

<400> 471
cgugugcacu ucgcuucaa 19

<210> 472
<211> 21
<212> RNA
<213> artifical sequence

<400> 472
uugaagcgaa gugcacacgg u 21

<210> 473
<211> 19
<212> RNA
<213> artifical sequence

<400> 473
cgugugcacu ucgcuucaa 19

<210> 474
<211> 21
<212> RNA
<213> artifical sequence

<400> 474
uugaagcgaa gugcacacgg u 21

<210> 475
<211> 19
<212> RNA
<213> artifical sequence

<400> 475
cgugugcacu ucgcuucaa 19

<210> 476
<211> 21
<212> RNA
<213> artifical sequence

<400> 476
uugaagcgaa gugcacacgg u 21

<210> 477
<211> 19
<212> RNA
<213> artifical sequence

<400> 477
cgugugcacu ucgcuucaa 19

<210> 478
<211> 21
<212> RNA
<213> artifical sequence

<400> 478
uugaagcgaa gugcacacgg u 21

<210> 479
<211> 19
<212> RNA
<213> artifical sequence

<400> 479
cgugugcacu ucgcuucaa 19

<210> 480
<211> 21
<212> RNA
<213> artifical sequence

<400> 480
uugaagcgaa gugcacacgg u 21

<210> 481
<211> 19
<212> RNA
<213> artifical sequence

<400> 481
cgugugcacu ucgcuucaa 19

<210> 482
<211> 21
<212> RNA
<213> artifical sequence

<400> 482
uugaagcgaa gugcacacgg u 21

<210> 483
<211> 19
<212> RNA
<213> artifical sequence

<400> 483
cgugugcacu ucgcuucaa 19

<210> 484
<211> 21
<212> RNA
<213> artifical sequence

<400> 484
uugaagcgaa gugcacacgg u 21

<210> 485
<211> 21
<212> RNA
<213> artifical sequence

<400> 485
accgugugca cuucgcuuca a 21

<210> 486
<211> 23
<212> RNA
<213> artifical sequence

<400> 486
uugaagcgaa gugcacacgg ucc 23

<210> 487
<211> 19
<212> RNA
<213> artifical sequence

<400> 487
cgugugcacu ucgcuucaa 19

<210> 488
<211> 21
<212> RNA
<213> artifical sequence

<400> 488
uugaagcgaa gugcacacgg u 21

<210> 489
<211> 19
<212> RNA
<213> artifical sequence

<400> 489
cgugugcacu ucgcuucaa 19

<210> 490
<211> 21
<212> RNA
<213> artifical sequence

<400> 490
uugaagcgaa gugcacacgg u 21

<210> 491
<211> 19
<212> RNA
<213> artifical sequence

<400> 491
cgugugcacu ucgcuucau 19

<210> 492
<211> 21
<212> RNA
<213> artifical sequence

<400> 492
augaagcgaa gugcacacgg u 21

<210> 493
<211> 19
<212> RNA
<213> artifical sequence

<400> 493
cgugugcacu ucgcuucaa 19

<210> 494
<211> 21
<212> RNA
<213> artifical sequence

<400> 494

uugaagcgaa gugcacacgg u 21

<210> 495
<211> 19
<212> RNA
<213> artifical sequence

<400> 495
cgugugcacu ucgcuucaa 19

<210> 496
<211> 21
<212> RNA
<213> artifical sequence

<400> 496
uugaagcgaa gugcacacgg u 21

<210> 497
<211> 19
<212> RNA
<213> artifical sequence

<400> 497
cgugugcacu ucgcuucaa 19

<210> 498
<211> 21
<212> RNA
<213> artifical sequence

<400> 498
uugaagcgaa gugcacacgg u 21

<210> 499
<211> 19
<212> RNA
<213> artifical sequence

<400> 499
cgugugcacu ucgcuucaa 19

<210> 500
<211> 21
<212> RNA
<213> artifical sequence

<400> 500
uugaagcgaa gugcacacgg u 21

<210> 501
<211> 19
<212> RNA
<213> artifical sequence

<400> 501
cgugugcacu ucgcuucaa 19

<210> 502
<211> 21
<212> RNA
<213> artifical sequence

<400> 502
uugaagcgaa gugcacacgg u 21

<210> 503
<211> 19
<212> RNA
<213> artifical sequence

<400> 503
gaaaguaugu caacgaaua 19

<210> 504
<211> 21
<212> RNA
<213> artifical sequence

<400> 504
uauucguuga cauacuuucu u 21

<210> 505
<211> 19
<212> RNA
<213> artifical sequence

<400> 505
gaaaguaugu caacgaaua 19

<210> 506
<211> 21
<212> RNA
<213> artifical sequence

<400> 506
uauucguuga cauacuuucc a 21

<210> 507
<211> 19
<212> RNA
<213> artifical sequence

<400> 507
gaaaguaugu caacgaaua 19

<210> 508
<211> 21
<212> RNA
<213> artifical sequence

<400> 508
uauucguuga cauacuuucu u 21

<210> 509
<211> 19
<212> RNA
<213> artifical sequence

<400> 509
gaaaguaugu caacgaaua 19

<210> 510
<211> 21
<212> RNA
<213> artifical sequence

<400> 510
uauucguuga cauacuuucc a 21

<210> 511
<211> 19
<212> RNA
<213> artifical sequence

<400> 511
gaaaguaugu caacgaaua 19

<210> 512
<211> 21
<212> RNA
<213> artifical sequence

<400> 512
uauucguuga cauacuuucu u 21

<210> 513
<211> 19
<212> RNA
<213> artifical sequence

<400> 513
gaaaguaugu caacgaaua 19

<210> 514
<211> 21
<212> RNA
<213> artifical sequence

<400> 514
uauucguuga cauacuuucu u 21

<210> 515
<211> 19
<212> RNA
<213> artifical sequence

<400> 515
gaaaguaugu caacgaaua 19

<210> 516
<211> 21
<212> RNA
<213> artifical sequence

<400> 516
uauucguuga cauacuuucu u 21

<210> 517
<211> 19
<212> RNA
<213> artifical sequence

<400> 517
gaaaguaugu caacgaaua 19

<210> 518
<211> 21
<212> RNA

<213> artifical sequence

<400> 518
uauucguuga cauacuuucu u 21

<210> 519
<211> 19
<212> RNA
<213> artifical sequence

<400> 519
gaaaguaugu caacgaaua 19

<210> 520
<211> 21
<212> RNA
<213> artifical sequence

<400> 520
uauucguuga cauacuuucu u 21

<210> 521
<211> 19
<212> RNA
<213> artifical sequence

<400> 521
gaaaguaugu caacgaaua 19

<210> 522
<211> 22
<212> RNA
<213> artifical sequence

<400> 522
vuauucguug acauacuuuc uu 22

<210> 523
<211> 19
<212> RNA
<213> artifical sequence

<400> 523
gaaaguaugu caacgaaua 19

<210> 524
<211> 21
<212> RNA
<213> artifical sequence

<400> 524
uauucguuga cauacuuucu u 21

<210> 525
<211> 19
<212> RNA
<213> artifical sequence

<400> 525
gaaaguaugu caacgaaua 19

<210> 526
<211> 21

<212> RNA
<213> artifical sequence

<400> 526
uauucguuga cauacuuucu u 21

<210> 527
<211> 19
<212> RNA
<213> artifical sequence

<400> 527
gaaaguaugu caacgaaua 19

<210> 528
<211> 21
<212> RNA
<213> artifical sequence

<400> 528
uauucguuga cauacuuucu u 21

<210> 529
<211> 19
<212> RNA
<213> artifical sequence

<400> 529
gaaaguaugu caacgaauu 19

<210> 530
<211> 21
<212> RNA
<213> artifical sequence

<400> 530
aauucguuga cauacuuucu u 21

<210> 531
<211> 21
<212> RNA
<213> artifical sequence

<400> 531
uggaaaguau gucaacgaau a 21

<210> 532
<211> 23
<212> RNA
<213> artifical sequence

<400> 532
uauucguuga cauacuuucc auu 23

<210> 533
<211> 19
<212> RNA
<213> artifical sequence

<400> 533
gaaaguaugu caacgaauu 19

<210> 534

<211> 19
<212> RNA
<213> artifical sequence

<400> 534
aauucguuga cauacuuuc 19

<210> 535
<211> 19
<212> RNA
<213> artifical sequence

<400> 535
gaaaguaugu caacgaauu 19

<210> 536
<211> 21
<212> RNA
<213> artifical sequence

<400> 536
aauucguuga cauacuuucc a 21

<210> 537
<211> 19
<212> RNA
<213> artifical sequence

<400> 537
gaaaguaugu caacgaaua 19

<210> 538
<211> 21
<212> RNA
<213> artifical sequence

<400> 538
uauucguuga cauacuuucu u 21

<210> 539
<211> 19
<212> RNA
<213> artifical sequence

<400> 539
gaaaguaugu caacgaaua 19

<210> 540
<211> 21
<212> RNA
<213> artifical sequence

<400> 540
uauucguuga cauacuuucu u 21

<210> 541
<211> 19
<212> RNA
<213> artifical sequence

<400> 541
gaaaguaugu caacgaaua 19

<210> 542
<211> 21
<212> RNA
<213> artifical sequence

<400> 542
uauucguuga cauacuuucu u 21

<210> 543
<211> 19
<212> RNA
<213> artifical sequence

<400> 543
gaaaguaugu caacgaaua 19

<210> 544
<211> 21
<212> RNA
<213> artifical sequence

<400> 544
uauucguuga cauacuuucu u 21

<210> 545
<211> 19
<212> RNA
<213> artifical sequence

<400> 545
gaaaguaugu caacgaaua 19

<210> 546
<211> 21
<212> RNA
<213> artifical sequence

<400> 546
uauucguuga cauacuuucu u 21

<210> 547
<211> 19
<212> RNA
<213> artifical sequence

<400> 547
ccaagagcac caagaacua 19

<210> 548
<211> 21
<212> RNA
<213> artifical sequence

<400> 548
uaguucuugg ugcucuuggc u 21

<210> 549
<211> 21
<212> RNA
<213> artifical sequence

<400> 549
agccaagagc accaagaacu a 21

<210> 550
<211> 23
<212> RNA
<213> artifical sequence

<400> 550
uaguucuugg ugcucuuggc uug 23

<210> 551
<211> 19
<212> RNA
<213> artifical sequence

<400> 551
ccaagagcac caagaacua 19

<210> 552
<211> 21
<212> RNA
<213> artifical sequence

<400> 552
uaguucuugg ugcucuuggc u 21

<210> 553
<211> 21
<212> RNA
<213> artifical sequence

<400> 553
agccaagagc accaagaacu a 21

<210> 554
<211> 23
<212> RNA
<213> artifical sequence

<400> 554
uaguucuugg ugcucuuggc uug 23

<210> 555
<211> 19
<212> RNA
<213> artifical sequence

<400> 555
ccaagagcac caagaacua 19

<210> 556
<211> 21
<212> RNA
<213> artifical sequence

<400> 556
uaguucuugg ugcucuuggc u 21

<210> 557
<211> 21
<212> RNA
<213> artifical sequence

<400> 557

agccaagagc accaagaacu a 21

<210> 558
<211> 23
<212> RNA
<213> artifical sequence

<400> 558
uaguucuugg ugcucuuggc uug 23

<210> 559
<211> 19
<212> RNA
<213> artifical sequence

<400> 559
ccaagagcac caagaacua 19

<210> 560
<211> 21
<212> RNA
<213> artifical sequence

<400> 560
uaguucuugg ugcucuuggc u 21

<210> 561
<211> 21
<212> RNA
<213> artifical sequence

<400> 561
agccaagagc accaagaacu a 21

<210> 562
<211> 23
<212> RNA
<213> artifical sequence

<400> 562
uaguucuugg ugcucuuggc uug 23

<210> 563
<211> 19
<212> RNA
<213> artifical sequence

<400> 563
ccaagagcac caagaacua 19

<210> 564
<211> 21
<212> RNA
<213> artifical sequence

<400> 564
uaguucuugg ugcucuuggc u 21

<210> 565
<211> 21
<212> RNA
<213> artifical sequence

<400> 565
agccaagagc accaagaacu a 21

<210> 566
<211> 23
<212> RNA
<213> artifical sequence

<400> 566
uaguucuugg ugcucuuggc uug 23

<210> 567
<211> 19
<212> RNA
<213> artifical sequence

<400> 567
ccaagagcac caagaacua 19

<210> 568
<211> 21
<212> RNA
<213> artifical sequence

<400> 568
uaguucuugg ugcucuuggc u 21

<210> 569
<211> 21
<212> RNA
<213> artifical sequence

<400> 569
agccaagagc accaagaacu a 21

<210> 570
<211> 23
<212> RNA
<213> artifical sequence

<400> 570
uaguucuugg ugcucuuggc uug 23

<210> 571
<211> 19
<212> RNA
<213> artifical sequence

<400> 571
ccaagagcac caagaacua 19

<210> 572
<211> 21
<212> RNA
<213> artifical sequence

<400> 572
uaguucuugg ugcucuuggc u 21

<210> 573
<211> 21
<212> RNA
<213> artifical sequence

<400> 573
agccaagagc accaagaacu a 21

<210> 574
<211> 23
<212> RNA
<213> artifical sequence

<400> 574
uaguucuugg ugcucuuggc uug 23

<210> 575
<211> 19
<212> RNA
<213> artifical sequence

<400> 575
ccaagagcac caagaacua 19

<210> 576
<211> 21
<212> RNA
<213> artifical sequence

<400> 576
uaguucuugg ugcucuuggc u 21

<210> 577
<211> 21
<212> RNA
<213> artifical sequence

<400> 577
agccaagagc accaagaacu a 21

<210> 578
<211> 23
<212> RNA
<213> artifical sequence

<400> 578
uaguucuugg ugcucuuggc uug 23

<210> 579
<211> 19
<212> RNA
<213> artifical sequence

<400> 579
ccaagagcac caagaacua 19

<210> 580
<211> 21
<212> RNA
<213> artifical sequence

<400> 580
uaguucuugg ugcucuuggc u 21

<210> 581
<211> 21
<212> RNA

<213> artifical sequence

<400> 581
agccaagagc accaagaacu a 21

<210> 582
<211> 23
<212> RNA
<213> artifical sequence

<400> 582
uaguucuugg ugcucuuggc uug 23

<210> 583
<211> 19
<212> RNA
<213> artifical sequence

<400> 583
ccaagagcac caagaacua 19

<210> 584
<211> 21
<212> RNA
<213> artifical sequence

<400> 584
uaguucuugg ugcucuuggc u 21

<210> 585
<211> 21
<212> RNA
<213> artifical sequence

<400> 585
agccaagagc accaagaacu a 21

<210> 586
<211> 23
<212> RNA
<213> artifical sequence

<400> 586
uaguucuugg ugcucuuggc uug 23

<210> 587
<211> 19
<212> RNA
<213> artifical sequence

<400> 587
ccaagagcac caagaacua 19

<210> 588
<211> 21
<212> RNA
<213> artifical sequence

<400> 588
uaguucuugg ugcucuuggc u 21

<210> 589
<211> 21

<212> RNA
<213> artifical sequence

<400> 589
agccaagagc accaagaacu a 21

<210> 590
<211> 23
<212> RNA
<213> artifical sequence

<400> 590
uaguucuugg ugcucuuggc uug 23

<210> 591
<211> 19
<212> RNA
<213> artifical sequence

<400> 591
ccaagagcac caagaacua 19

<210> 592
<211> 21
<212> RNA
<213> artifical sequence

<400> 592
uaguucuugg ugcucuuggc u 21

<210> 593
<211> 21
<212> RNA
<213> artifical sequence

<400> 593
agccaagagc accaagaacu a 21

<210> 594
<211> 23
<212> RNA
<213> artifical sequence

<400> 594
uaguucuugg ugcucuuggc uug 23

<210> 595
<211> 19
<212> RNA
<213> artifical sequence

<400> 595
ccaagagcac caagaacua 19

<210> 596
<211> 21
<212> RNA
<213> artifical sequence

<400> 596
uaguucuugg ugcucuuggc u 21

<210> 597

<211> 19
<212> RNA
<213> artifical sequence

<400> 597
ccaagagcac caagaacua 19

<210> 598
<211> 21
<212> RNA
<213> artifical sequence

<400> 598
uaguucuugg ugcucuuggc u 21

<210> 599
<211> 19
<212> RNA
<213> artifical sequence

<400> 599
ccaagagcac caagaacua 19

<210> 600
<211> 21
<212> RNA
<213> artifical sequence

<400> 600
uaguucuugg ugcucuuggc u 21

<210> 601
<211> 19
<212> RNA
<213> artifical sequence

<400> 601
ccaagagcac caagaacuu 19

<210> 602
<211> 21
<212> RNA
<213> artifical sequence

<400> 602
aaguucuugg ugcucuuggc u 21

<210> 603
<211> 21
<212> RNA
<213> artifical sequence

<400> 603
agccaagagc accaagaacu a 21

<210> 604
<211> 23
<212> RNA
<213> artifical sequence

<400> 604
uaguucuugg ugcucuuggc uug 23

<210> 605
<211> 19
<212> RNA
<213> artifical sequence

<400> 605
ccaagagcac caagaacua 19

<210> 606
<211> 21
<212> RNA
<213> artifical sequence

<400> 606
uaguucuugg ugcucuuggc u 21

<210> 607
<211> 19
<212> RNA
<213> artifical sequence

<400> 607
ccaagagcac caagaacua 19

<210> 608
<211> 21
<212> RNA
<213> artifical sequence

<400> 608
uaguucuugg ugcucuuggc u 21

<210> 609
<211> 19
<212> RNA
<213> artifical sequence

<400> 609
ccaagagcac caagaacua 19

<210> 610
<211> 21
<212> RNA
<213> artifical sequence

<400> 610
uaguucuugg ugcucuuggc u 21

<210> 611
<211> 19
<212> RNA
<213> artifical sequence

<400> 611
ccaagagcac caagaacua 19

<210> 612
<211> 21
<212> RNA
<213> artifical sequence

<400> 612
uaguucuugg ugcucuuggc u 21

<210> 613
<211> 19
<212> RNA
<213> artifical sequence

<400> 613
ccaagagcac caagaacua 19

<210> 614
<211> 21
<212> RNA
<213> artifical sequence

<400> 614
uaguucuugg ugcucuuggc u 21

<210> 615
<211> 19
<212> RNA
<213> artifical sequence

<400> 615
ccaagagcac caagaacua 19

<210> 616
<211> 21
<212> RNA
<213> artifical sequence

<400> 616
uaguucuugg ugcucuuggc u 21

<210> 617
<211> 19
<212> RNA
<213> artifical sequence

<400> 617
ccaagagcac caagaacua 19

<210> 618
<211> 21
<212> RNA
<213> artifical sequence

<400> 618
uaguucuugg ugcucuuggc u 21

<210> 619
<211> 19
<212> RNA
<213> artifical sequence

<400> 619
ccaagagcac caagaacua 19

<210> 620
<211> 21
<212> RNA
<213> artifical sequence

<400> 620

uaguucuugg ugcucuuggc u 21

<210> 621
<211> 19
<212> RNA
<213> artifical sequence

<400> 621
ccaagagcac caagaacua 19

<210> 622
<211> 21
<212> RNA
<213> artifical sequence

<400> 622
uaguucuugg ugcucuuggc u 21

<210> 623
<211> 19
<212> RNA
<213> artifical sequence

<400> 623
caauaaagcu ggacaagaa 19

<210> 624
<211> 21
<212> RNA
<213> artifical sequence

<400> 624
uucuugucca gcuuuauugg g 21

<210> 625
<211> 21
<212> RNA
<213> artifical sequence

<400> 625
cccaauaaag cuggacaaga a 21

<210> 626
<211> 23
<212> RNA
<213> artifical sequence

<400> 626
uucuugucca gcuuuauugg gag 23

<210> 627
<211> 19
<212> RNA
<213> artifical sequence

<400> 627
caauaaagcu ggacaagaa 19

<210> 628
<211> 21
<212> RNA
<213> artifical sequence

<400> 628
uucuugucca gcuuuauugg g 21

<210> 629
<211> 21
<212> RNA
<213> artifical sequence

<400> 629
cccaauaaag cuggacaaga a 21

<210> 630
<211> 23
<212> RNA
<213> artifical sequence

<400> 630
uucuugucca gcuuuauugg gag 23

<210> 631
<211> 19
<212> RNA
<213> artifical sequence

<400> 631
caauaaagcu ggacaagaa 19

<210> 632
<211> 21
<212> RNA
<213> artifical sequence

<400> 632
uucuugucca gcuuuauugg g 21

<210> 633
<211> 21
<212> RNA
<213> artifical sequence

<400> 633
cccaauaaag cuggacaaga a 21

<210> 634
<211> 23
<212> RNA
<213> artifical sequence

<400> 634
uucuugucca gcuuuauugg gag 23

<210> 635
<211> 19
<212> RNA
<213> artifical sequence

<400> 635
caauaaagcu ggacaagaa 19

<210> 636
<211> 21
<212> RNA
<213> artifical sequence

<400> 636
uucuugucca gcuuuauugg g 21

<210> 637
<211> 21
<212> RNA
<213> artifical sequence

<400> 637
cccaauaaag cuggacaaga a 21

<210> 638
<211> 23
<212> RNA
<213> artifical sequence

<400> 638
uucuugucca gcuuuauugg gag 23

<210> 639
<211> 19
<212> RNA
<213> artifical sequence

<400> 639
caauaaagcu ggacaagaa 19

<210> 640
<211> 21
<212> RNA
<213> artifical sequence

<400> 640
uucuugucca gcuuuauugg g 21

<210> 641
<211> 21
<212> RNA
<213> artifical sequence

<400> 641
cccaauaaag cuggacaaga a 21

<210> 642
<211> 23
<212> RNA
<213> artifical sequence

<400> 642
uucuugucca gcuuuauugg gag 23

<210> 643
<211> 19
<212> RNA
<213> artifical sequence

<400> 643
caauaaagcu ggacaagaa 19

<210> 644
<211> 21
<212> RNA

<213> artifical sequence

<400> 644
uucuugucca gcuuuauugg g 21

<210> 645
<211> 21
<212> RNA
<213> artifical sequence

<400> 645
cccaauaaag cuggacaaga a 21

<210> 646
<211> 23
<212> RNA
<213> artifical sequence

<400> 646
uucuugucca gcuuuauugg gag 23

<210> 647
<211> 19
<212> RNA
<213> artifical sequence

<400> 647
caauaaagcu ggacaagaa 19

<210> 648
<211> 21
<212> RNA
<213> artifical sequence

<400> 648
uucuugucca gcuuuauugg g 21

<210> 649
<211> 21
<212> RNA
<213> artifical sequence

<400> 649
cccaauaaag cuggacaaga a 21

<210> 650
<211> 23
<212> RNA
<213> artifical sequence

<400> 650
uucuugucca gcuuuauugg gag 23

<210> 651
<211> 19
<212> RNA
<213> artifical sequence

<400> 651
caauaaagcu ggacaagaa 19

<210> 652
<211> 21

<212> RNA
<213> artifical sequence

<400> 652
uucuugucca gcuuuauugg g 21

<210> 653
<211> 21
<212> RNA
<213> artifical sequence

<400> 653
cccaauaaag cuggacaaga a 21

<210> 654
<211> 23
<212> RNA
<213> artifical sequence

<400> 654
uucuugucca gcuuuauugg gag 23

<210> 655
<211> 19
<212> RNA
<213> artifical sequence

<400> 655
caauaaagcu ggacaagaa 19

<210> 656
<211> 21
<212> RNA
<213> artifical sequence

<400> 656
uucuugucca gcuuuauugg g 21

<210> 657
<211> 19
<212> RNA
<213> artifical sequence

<400> 657
caauaaagcu ggacaagaa 19

<210> 658
<211> 21
<212> RNA
<213> artifical sequence

<400> 658
uucuugucca gcuuuauugg g 21

<210> 659
<211> 19
<212> RNA
<213> artifical sequence

<400> 659
caauaaagcu ggacaagaa 19

<210> 660

<211> 21
<212> RNA
<213> artifical sequence

<400> 660
uucuugucca gcuuuauugg g 21

<210> 661
<211> 19
<212> RNA
<213> artifical sequence

<400> 661
caauaaagcu ggacaagau 19

<210> 662
<211> 21
<212> RNA
<213> artifical sequence

<400> 662
aucuugucca gcuuuauugg g 21

<210> 663
<211> 21
<212> RNA
<213> artifical sequence

<400> 663
cccaauaaag cuggacaaga a 21

<210> 664
<211> 23
<212> RNA
<213> artifical sequence

<400> 664
uucuugucca gcuuuauugg gag 23

<210> 665
<211> 19
<212> RNA
<213> artifical sequence

<400> 665
caauaaagcu ggacaagaa 19

<210> 666
<211> 21
<212> RNA
<213> artifical sequence

<400> 666
uucuugucca gcuuuauugg g 21

<210> 667
<211> 19
<212> RNA
<213> artifical sequence

<400> 667
caauaaagcu ggacaagaa 19

<210> 668
<211> 21
<212> RNA
<213> artifical sequence

<400> 668
uucuugucca gcuuuauugg g 21

<210> 669
<211> 19
<212> RNA
<213> artifical sequence

<400> 669
caauaaagcu ggacaagaa 19

<210> 670
<211> 21
<212> RNA
<213> artifical sequence

<400> 670
uucuugucca gcuuuauugg g 21

<210> 671
<211> 19
<212> RNA
<213> artifical sequence

<400> 671
caauaaagcu ggacaagaa 19

<210> 672
<211> 21
<212> RNA
<213> artifical sequence

<400> 672
uucuugucca gcuuuauugg g 21

<210> 673
<211> 21
<212> RNA
<213> artifical sequence

<400> 673
aacaguguuc uugcucuaua a 21

<210> 674
<211> 23
<212> RNA
<213> artifical sequence

<400> 674
uuauagagca agaacacugu uuu 23

<210> 675
<211> 19
<212> RNA
<213> artifical sequence

<400> 675
cuagaaaacu ggauaacgu 19

<210> 676
<211> 21
<212> RNA
<213> artifical sequence

<400> 676
acguuaucca guuuucuagc c 21

<210> 678
<211> 20
<212> DNA
<213> artifical sequence

<400> 678
ccgtctgtgc cttctcatct 20

<210> 678
<211> 20
<212> DNA
<213> artifical sequence

<400> 678
taatctcctc ccccaactcc 20

<210> 679
<211> 24
<212> DNA
<213> artifical sequence

<400> 679
agcttctttg cagctccttc gttg 24

<210> 680
<211> 24
<212> DNA
<213> artifical sequence

<400> 680
ttctgaccca ttcccaccat caca 24

<210> 681
<211> 21
<212> DNA
<213> artifical sequence

<400> 681
cgtttctcct ggctcagttt a 21

<210> 682
<211> 21
<212> DNA
<213> artifical sequence

<400> 682
cagcggtaaa aagggactca a 21

<210> 683
<211> 20
<212> DNA
<213> artifical sequence

<400> 683

agaaggctgg ggctcatttg 20

<210> 684
<211> 20
<212> DNA
<213> artifical sequence

<400> 684
aggggccatc cacagtcttc 20

<210> 685
<211> 21
<212> DNA
<213> artifical sequence

<400> 685
cgtttctcct ggctcagttt a 21

<210> 686
<211> 21
<212> DNA
<213> artifical sequence

<400> 686
cagcggtaaa aagggactca a 21

<210> 687
<211> 24
<212> DNA
<213> artifical sequence

<400> 687
agcttctttg cagctccttc gttg 24

<210> 688
<211> 24
<212> DNA
<213> artifical sequence

<400> 688
ttctgaccca ttcccaccat caca 24

<210> 689
<211> 20
<212> DNA
<213> artifical sequence

<400> 689
gtcttttggg ttttgctgcc 20

<210> 690
<211> 20
<212> DNA
<213> artifical sequence

<400> 690
gcaacggggt aaaggttcag 20

<210> 691
<211> 19
<212> DNA
<213> artifical sequence

<400> 691
ggtcggagtc aacggattt 19

<210> 692
<211> 19
<212> DNA
<213> artifical sequence

<400> 692
ccagcatcgc cccacttga 19

<210> 693
<211> 20
<212> DNA
<213> artifical sequence

<400> 693
gtcttttggg ttttgctgcc 20

<210> 694
<211> 20
<212> DNA
<213> artifical sequence

<400> 694
gcaacggggt aaaggttcag 20

<210> 695
<211> 24
<212> DNA
<213> artifical sequence

<400> 695
agcttctttg cagctccttc gttg 24

<210> 696
<211> 24
<212> DNA
<213> artifical sequence

<400> 696
ttctgaccca ttcccaccat caca 24

<210> 697
<211> 18
<212> DNA
<213> artifical sequence

<400> 697
accaactata cgctacat 18

<210> 698
<211> 18
<212> DNA
<213> artifical sequence

<400> 698
cctcctgaat aaccctct 18

<210> 699
<211> 18
<212> DNA
<213> artifical sequence

<400> 699
ccaaccgcga gaagatga 18

<210> 700
<211> 20
<212> DNA
<213> artifical sequence

<400> 700
ccagaggcgt acagggatag 20

<210> 701
<211> 23
<212> DNA
<213> artifical sequence

<400> 701
gaggagcagc taaccaactt aat 23

<210> 702
<211> 23
<212> DNA
<213> artifical sequence

<400> 702
tctgcatgtg ctgttgactt aat 23

<210> 703
<211> 24
<212> DNA
<213> artifical sequence

<400> 703
agcttctttg cagctccttc gttg 24

<210> 704
<211> 24
<212> DNA
<213> artifical sequence

<400> 704
ttctgaccca ttcccaccat caca 24

<210> 705
<211> 20
<212> DNA
<213> artifical sequence

<400> 705
gtgaccgatg gcttcagttc 20

<210> 706
<211> 20
<212> DNA
<213> artifical sequence

<400> 706
atggataggc aggtggactt 20

<210> 707
<211> 18
<212> DNA

<213> artifical sequence

<400> 707
ccaaccgcga gaagatga 18

<210> 708
<211> 20
<212> DNA
<213> artifical sequence

<400> 708
ccagaggcgt acagggatag 20

<210> 709
<211> 20
<212> DNA
<213> artifical sequence

<400> 709
gtgaccgatg gcttcagttc 20

<210> 710
<211> 20
<212> DNA
<213> artifical sequence

<400> 710
atggataggc aggtggactt 20

<210> 714
<211> 24
<212> DNA
<213> artifical sequence

<400> 714
agcttctttg cagctccttc gttg 24

<210> 712
<211> 24
<212> DNA
<213> artifical sequence

<400> 712
ttctgaccca ttcccaccat caca 24

<210> 713
<211> 20
<212> DNA
<213> artifical sequence

<400> 715
ccgtctgtgc cttctcatct 20

<210> 714
<211> 20
<212> DNA
<213> artifical sequence

<400> 716
taatctcctc ccccaactcc 20

<210> 715
<211> 20

<212> DNA
<213> artifical sequence

<400> 717
agaaggctgg ggctcatttg 20

<210> 716
<211> 20
<212> DNA
<213> artifical sequence

<400> 718
aggggccatc cacagtcttc 20

<210> 717
<211> 19
<212> RNA
<213> artifical sequence

<400> 719
cuagaaaacu ggauaacgu 19

<210> 718
<211> 21
<212> RNA
<213> artifical sequence

<400> 720
acguuaucca guuuucuagc c 21

<210> 719
<211> 19
<212> RNA
<213> artifical sequence

<400> 723
cuagaaaacu ggauaacgu 19

<210> 720
<211> 21
<212> RNA
<213> artifical sequence

<400> 724
acguuaucca guuuucuagc c 21

<210> 721
<211> 21
<212> DNA
<213> artifical sequence

<400> 729
cgtttctcct ggctcagttt a 21

<210> 722
<211> 21
<212> DNA
<213> artifical sequence

<400> 730
cagcggtaaa aagggactca a 21

<210> 723

<211> 20
<212> DNA
<213> artifical sequence

<400> 731
agaaggctgg ggctcatttg 20

<210> 724
<211> 20
<212> DNA
<213> artifical sequence

<400> 732
aggggccatc cacagtcttc 20

## Description

### TECHNICAL FIELD

The present disclosure relates to a compound for conjugation with an active drug, the corresponding drug conjugate, and preparation method and use thereof. The present disclosure also relates to a method for preventing and/or treating a pathological condition or disease by using the drug conjugate.

### BACKGROUND ART

Delivery system is one of the key technolgies in the development of small nucleic acid drugs. At present, the technology of targeted delivery of conjugate is the most intensively studied delivery system among the delivery systems of small nucleic acids around the world. There is still an urgent need to develop a new drug conjugate with higher *in vivo* delivery efficiency of an active drug, lower toxicity, and higher activity in the art.

### SUMMARY OF THE INVENTION

To meet the above need, we have invented a compound for conjugation with an active drug and the corresponding conjugate with the active drug having higher *in vivo* delivery efficiency, lower toxicity and/or better stability, and preparation method and use thereof.

In some embodiments, the present disclosure provides a compound having a structure as shown by Formula (101):

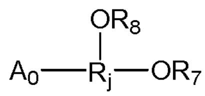

Formula (101)

wherein,

$A_0$ has a structure as shown by Formula (312):

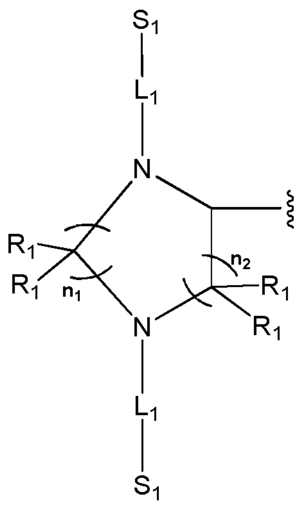

Formula (312)

wherein

- $n_1$ is an integer of 1-4;
- $n_2$ is an integer of 0-3;
- each $L_1$ is a linear alkylene of 1 - 70 carbon atoms in length, wherein one or more carbon atoms are optionally

replaced with one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N(Ci-Cio alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, - $SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -$NHSO_2$($C_1$-$C_{10}$ alkyl), -$NHSO_2$(phenyl), and -$NHSO_2$($C_1$-$C_{10}$ haloalkyl); each $S_1$ is independently a $M_1$, in which any active hydroxyl groups and/or amino groups, if any, are protected with protecting groups;

each $M_1$ is independently selected from a ligand capable of binding to a cell surface receptor; each $R_1$ independently of one another is selected from H, substituted or unsubstituted $C_1$-$C_4$ hydrocarbyl or halogen;

$R_j$ is a linking group;

$R_7$ is any functional group capable of forming a phosphoester linkage, phosphorothioate linkage, phosphoroborate linkage, or carboxylate linkage with a hydroxyl group via reaction, or any functional group capable of forming an amide linkage with an amino group via reaction; $R_8$ is a hydroxyl protecting group.

In some embodiments, the present disclosure provides a compound having a structure as shown by Formula (111):

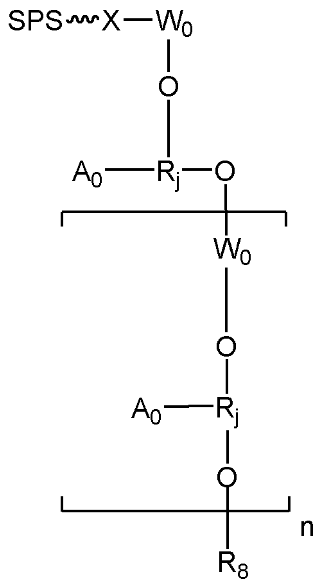

Formula (111)

wherein the definitions and options of each $A_0$, each $R_j$ and $R_8$ are respectively as described above;

Wo is a linking group;

X is O or NH;

SPS represents a solid phase support; and

n is an integer of 0-7.

In some embodiments, X is O; and Wo and X form a phosphoester linkage, phosphorothioate linkage, or phosphoroborate linkage.

In some embodiments, the present disclosure provides a drug conjugate having a structure as shown by Formula (301):

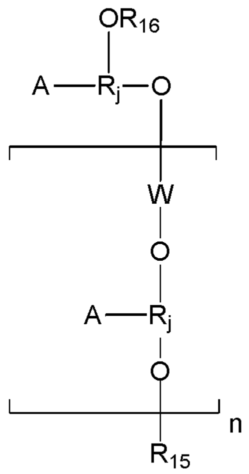

Formula (301)

wherein,

A has a structure as shown by Formula (302), wherein the definitions and options of $R_j$, $R_1$, $L_1$, $M_1$, n, $n_1$, and $n_2$ are respectively as described above;

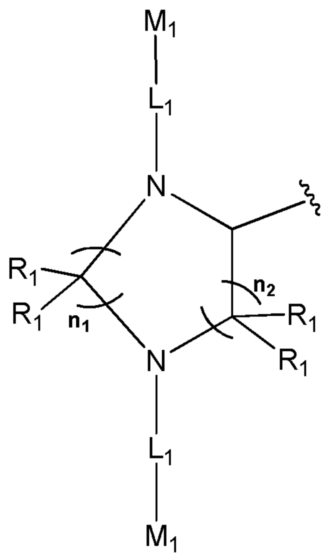

Formula (302)

$R_{16}$ and $R_{15}$ are respectively H or an active drug group, and at least one of $R_{16}$ and $R_{15}$ is an active drug group. In some embodiments, the active drug group has a structure as shown by Formula A60.

W is a linking group. In some embodiments, W has a structure as shown by Formula (A61) or (C1'):

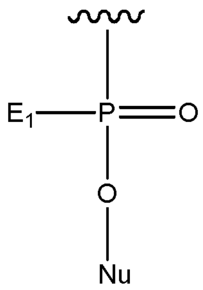, 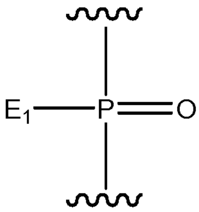, 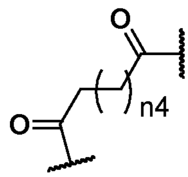;

Formula (A60) Formula (A61) Formula (C1')

wherein,

$E_1$ is OH, SH or $BH_2$;
n4 is an integer of 1-4;

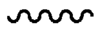 represents the site where a group is linked;
Nu represents a functional oligonucleotide.

In some embodiments, the present disclosure provides use of the drug conjugate of the present disclosure in the manufacture of a medicament for treating and/or preventing a pathological condition or disease caused by the expression of a gene in a target cell.

In some embodiments, the present disclosure provides a method for treating a pathological condition or disease caused by the expression of a gene in a target cell, the method comprising administering the drug conjugate of the present disclosure to a patient suffering from the disease.

In some embodiments, the present disclosure provides a method of regulating the expression of a gene in a cell, the method comprising contacting the drug conjugate of the present disclosure with the cell, wherein the regulation comprises inhibiting or enhancing the expression of the gene.

In some embodiments, the present disclosure provides a kit comprising the drug conjugate of the present disclosure.

**ADVANTAGEOUS EFFECTS**

The compound as shown by Formula (101) or the compound as shown by Formula (111) of the present disclosure can be conjugated to various active drug groups (such as, small molecule drugs, monoclonal antibodies or functional oligonucleotides) to produce the drug conjugates of the present disclosure. The drug conjugate can specifically deliver the active drugs to target organs or tissues, bind to specific targets, regulate the *in vivo* content or function of a protein, inhibit or enhance the expression of the corresponding mRNA of a gene that needs to be inhibited or enhanced, and regulate the expression of cell associated gene, thereby preventing and/or treating a relevant pathological condition or disease. In some embodiments, the active drug contained in the drug conjugate of the present disclosure is an oligonucleotide; the drug conjugate is an oligonucleotide conjugate; and the drug conjugate exhibits higher delivery efficiency *in vivo,* lower toxicity, better stability and/or higher activity.

According to some embodiments of the present disclosure, when the active drug is an siRNA for inhibiting the expression of hepatitis B virus (HBV) gene, the drug conjugate of the present disclosure can effectively target the liver; when administered at a dosage of 1 mg/kg, the drug conjugate could inhibit at least 65.77% of HBV gene expression in the liver of hepatitis B model mouse, and exhibit an inhibition rate of up to 91.96% against HBV gene expression at a dosage of 1 mg/kg. In the meantime, the drug conjugate of the present disclosure can also effectively reduce the expression of HBV surface antigen in hepatitis B model mouse; when administered at a dosage of 3 mg/kg, the drug conjugate could exhibit an inhibition rate of up to 97.80% against the expression of HBV surface antigen and an inhibition rate of 85.7% against HBV DNA. Moreover, when administered at a dosage of 3 mg/kg, the drug conjugate could continuously exhibit excellent inhibitory effect on HBV expression over an experimental period of up to 140 days.

According to some embodiments of the present disclosure, when the active drug is an siRNA for inhibiting the expression of hepatitis B virus (HBV) gene, the drug conjugate of the present disclosure can effectively deliver the siRNA to the liver and exhibit excellent properties of inhibiting HBV gene expression. When administered at a dosage of 1 mg/kg, the drug conjugate could inhibit at least 68.3%, or even 78.7-88.5% of HBV gene expression in the liver of hepatitis B model mouse. In the meantime, the drug conjugate of the present disclosure can also effectively reduce the expression

of HBV surface antigen in hepatitis B model mouse; when administered at a dosage of 3 mg/kg, the drug conjugate could exhibit an inhibition rate of 98.1% against the expression of HBV surface antigen and an inhibition rate of 93.5% against HBV DNA. Moreover, when administered at a dosage of 3 mg/kg, the drug conjugate could continuously exhibit excellent inhibitory effect on HBV expression over an experimental period of up to 84 days.

According to some embodiments of the present disclosure, when the active drug is an siRNA for inhibiting the expression of hepatitis B virus (HBV) gene, the drug conjugate of the present disclosure can effectively deliver the siRNA to the liver and exhibit excellent properties of inhibiting HBV gene expression. When administered at a dosage of 1 mg/kg, the drug conjugate could inhibit at least 50.4% (in some embodiments, 76.2-84.6%) of HBV gene expression in the liver of hepatitis B model mouse. In the meantime, the drug conjugate of the present disclosure can also effectively reduce the expression of HBV surface antigen in hepatitis B model mouse; even when administered at a dosage of 3 mg/kg, the drug conjugate could exhibit an inhibition rate of 82.5% against the expression of HBV surface antigen and an inhibition rate of 83.9% against HBV DNA. Moreover, when administered at a dosage of 3 mg/kg, the drug conjugate could continuously exhibit higher inhibitory effect on HBV expression over an experimental period of 21 days.

According to some embodiments of the present disclosure, when the active drug is an siRNA for inhibiting the expression of hepatitis B virus (HBV) gene, the drug conjugate of the present disclosure can effectively deliver the siRNA to the liver and exhibit excellent properties of inhibiting HBV gene expression. When administered at a dosage of 1 mg/kg, the drug conjugate could inhibit at least 65.8%, or even 76.3-84.1%, of HBV gene expression in the liver of hepatitis B model mouse. In the meantime, the drug conjugate of the present disclosure can also effectively reduce the expression of HBV surface antigen in hepatitis B model mouse; even when administered at a dosage of 3 mg/kg, the drug conjugate could exhibit an inhibition rate of 95.6% against the expression of HBV surface antigen and an inhibition rate of 93.1% against HBV DNA. Moreover, when administered at a dosage of 3 mg/kg, the drug conjugate could continuously exhibit excellent inhibitory effect on HBV expression over an experimental period of up to 56 days.

According to some embodiments of the present disclosure, when the active drug is an siRNA for inhibiting the expression of hepatitis B virus (HBV) gene, the drug conjugate of the present disclosure can effectively deliver the siRNA to the liver and exhibit excellent properties of inhibiting HBV gene expression. When administered at a dosage of 1 mg/kg, the drug conjugate could inhibit 80% or higher of HBV gene expression in the liver of hepatitis B model mouse. In the meantime, the drug conjugate of the present disclosure can also effectively reduce the expression of HBV surface antigen in hepatitis B model mouse; even when administered at a dosage of 3 mg/kg, the drug conjugate could exhibit an inhibition rate of up to 99% or higher against the expression of HBV surface antigen and an inhibition rate of 90% or higher against HBV DNA. Moreover, when administered at a dosage of 3 mg/kg, the drug conjugate could continuously exhibit excellent inhibitory effect on HBV expression over an experimental period of up to 112 days.

According to some embodiments of the present disclosure, when the active drug is an siRNA for inhibiting the expression of angiopoietin-like protein 3 (ANGPTL3) gene, the drug conjugate of the present disclosure can effectively deliver the siRNA to the liver and exhibit excellent properties of inhibiting ANGPTL3 gene expression. When administered at a dosage of 1 mg/kg, the drug conjugate could inhibit at least 53.2% of ANGPTL3 gene expression in the liver of high-fat model mouse; when administered at a dosage of 3 mg/kg, the drug conjugate could exhibit an inhibition rate of up to 86.4% against ANGPTL3 mRNA. Moreover, when administered at a single dosage of 3 mg/kg, the drug conjugate could continuously exhibit excellent inhibitory effect on ANGPTL3 expression and effect of reducing blood lipid level over an experimental period of up to 49 days.

According to some embodiments of the present disclosure, when the active drug is an siRNA for inhibiting the expression of apolipoprotein C3 (APOC3) gene, the drug conjugate of the present disclosure can effectively deliver the siRNA to the liver and exhibit excellent properties of inhibiting APOC3 gene expression. When administered at a dosage of 3 mg/kg, the drug conjugate could inhibit at least 71.4% of APOC3 gene expression in the liver of high-fat model mouse. Moreover, when administered at a single dosage of 3 mg/kg, the drug conjugate could continuously exhibit excellent inhibitory effect on blood lipid over an experimental period of up to 65 days.

In certain embodiments, the drug conjugate of the present disclosure can also exhibit low animal level toxicity and good safety. For example, in some embodiments, even if the conjugate of the present disclosure was administered to C57BL/6J mice at a dosage 100 times higher than the effective concentration (based on the effective concentration of 3 mg/kg), no significant toxic reaction was observed.

The above examples show that the drug conjugate of the present disclosure can effectively deliver a functionally active drug to target organs or tissues and remain active *in vivo* for a prolonged period, thereby effectively treating and/or preventing a pathological condition or disease caused by the expression of genes in cells.

Additional features and advantages of the present disclosure will be detailedly illustrated in the following part "DETAILED DESCRIPTION OF THE INVENTION ".

Incorperation by Reference

All publications, patents, and patent applications mentioned in this description are incorporated herein by

reference to the same extent as if each individual publication, patent, and patent application were specifically and individually incorporated herein by reference.

## BRIEF DESCRIPTION OF THE DRAWINGS

FIG. 1 shows the semiquantitative test result of the stability of the drug conjugate in the *in vitro* human plasma.

FIG. 2 shows the semiquantitative test result of the stability of the drug conjugate in the *in vitro* cynomolgus monkey plasma.

FIG.3 is the time-dependent metabolic curve of PK/TK concentration in rat plasma when the drug conjugate is administered at the dosage of 1 mg/kg or 0.5 mg/kg.

FIG. 4 is the time-dependent metabolic curve of PK/TK concentration in rat liver when the drug conjugate is administered at the dosage of 1 mg/kg or 0.5 mg/kg.

FIG. 5 shows the *in vivo* inhibition rate of the drug conjugate against HBV mRNA expression in C57BL/6J-Tg(Alb1HBV)44Bri/J mice after the drug conjugate is administered to the mice at the dosage of 1 mg/kg or 0.1 mg/kg.

FIG. 6 shows time-dependent curve of the *in vivo* effects of the drug conjugate on serum HBsAg level in M-Tg HBV transgenic mice after the drug conjugate is administered at the dosage of 3 mg/kg or 1 mg/kg.

FIG. 7A shows the *in vivo* inhibition rate of the drug conjugate against HBV mRNA in C57BL/6J-Tg(Alb1HBV)44Bri/J mice after the drug conjugate is administered at the dosage of 1 mg/kg or 0.1 mg/kg.

FIG. 7B shows the *in vivo* inhibition rates of different drug conjugates against HBV mRNA in C57BL/6J-Tg(Alb1HBV)44Bri/J mice after the drug conjugates are administered at the dosage of 1 mg/kg or 0.1 mg/kg.

FIG. 8A shows time-dependent curve of the *in vivo* effects of the drug conjugate on serum HBsAg level in M-Tg HBV transgenic mice after the drug conjugate is administered at the dosage of 3 mg/kg or 1 mg/kg.

FIG. 8B shows time-dependent curve of the *in vivo* effects of the drug conjugate on serum HBV DNA level in M-Tg HBV transgenic mice after the drug conjugate is administered at the dosage of 3 mg/kg or 1 mg/kg.

FIG. 9 shows the inhibition rate of the drug conjugate against HBV mRNA in M-Tg HBV transgenic mice at day 70 after the drug conjugate is administered at the dosage of 1 mg/kg or 3 mg/kg.

## DETAILED DESCRIPTION OF THE INVENTION

The specific embodiments of the present disclosure are described in detail as below. It should be understood that the specific embodiments described herein are only for the purpose of illustration and explanation of the present disclosure and are not intended to limit the present disclosure in any respect.

In the context of the present disclosure, unless otherwise specified, C, G, U, A, or T represents the base composition of a nucleotide; d represents that the nucleotide adjacent to the right side of the letter d is a deoxyribonucleotide; m represents that the nucleotide adjacent to the left side of the letter m is a methoxy modified nucleotide; f represents that the nucleotide adjacent to the left side of the letter f is a fluoro modified nucleotide; s represents that the two nucleotides adjacent to both sides of the letter s are linked by a phosphorothioate linkage; P1 represents that the nucleotide adjacent to the right side of P1 is a 5'-phosphate nucleotide or a 5'-phosphate analogue modified nucleotide, especially a vinyl phosphate modified nucleotide (expressed as VP in the Examples below), a 5'-phosphate nucleotide (expressed as P in the Examples below) or a 5'-phosphorothioate modified nucleotide (expressed as Ps in the Examples below).

In the context of the present disclosure, a "fluoro modified nucleotide" refers to a nucleotide formed by substituting the 2'-hydroxy of the ribose group of the nucleotide with a fluorine atom. A "non-fluoro modified nucleotide" refers to a nucleotide formed by substituting the 2'-hydroxy of the ribose group of the nucleotide with a non-fluoro group, or a nucleotide analogue. A "nucleotide analogue" refers to a group that can replace a nucleotide in a nucleic acid, while structurally differs from an adenine ribonucleotide, a guanine ribonucleotide, a cytosine ribonucleotide, a uracil ribonucleotide, or thymine deoxyribonucleotide, such as an isonucleotide, a bridged nucleotide (bridged nucleic acid, BNA) or an acyclic nucleotide. The "methoxy modified nucleotide" refers to a nucleotide formed by substituting the 2'-hydroxy of the ribose group with a methoxy group.

In the context of the present disclosure, expressions "complementary" and "reverse complementary" can be interchangeably used, and have a well-known meaning in the art; namely, the bases in one strand are complementarily paired with those in the other strand of a double-stranded nucleic acid molecule. In DNAs, a purine base adenine (A) is always paired with a pyrimidine base thymine (T) (or a uracil (U) in RNAs); and a purine base guanine (G) is always paired with a pyrimidine base cytosine (C). Each base pair comprises a purine and a pyrimidine. While adenines in one strand are always paired with thymines (or uracils) in another strand, and guanines are always paired with cytosines, the two strands are considered as being complementary to each other; and the sequence of a strand can be deduced from the sequence of its complementary strand. Correspondingly, a "mispairing" means that the bases at corresponding positions are not present in a manner of complementary pairing in a double-stranded nucleic acid.

In the context of the present disclosure, unless otherwise specified, "basically reverse complementary" means that there are no more than 3 base mispairings between two nucleotide sequences. "Essentially reverse complementary" or "substantially reverse complementary" means that there is no more than 1 base mispairing between two nucleotide sequences. "Completely reverse complementary" means that there is no base mispairing between two nucleotide sequences.

In the context of the present disclosure, when a nucleotide sequence has a "nucleotide difference" from another nucleotide sequence, the bases of the nucleotides at the same position therebetween are changed. For example, if a nucleotide base in the second sequence is A and the nucleotide base at the same position in the first sequence is U, C, G, or T, the two nucleotide sequences are considered as having a nucleotide difference at this position. In some embodiments, if a nucleotide at a position is replaced with an abasic nucleotide or a nucleotide analogue, it is also considered that there is a nucleotide difference at the position.

In the context of the present disclosure, particularly in the description of the preparation method or use of the compound as shown by Formula (101), the compound as shown by Formula (111) or the drug conjugate as shown by Formula (301) of the present disclosure, nucleoside monomers are sometimes used. Unless otherwise specified, the "nucleoside monomer" refers to, according to the type and sequence of the nucleotides in the functional oligonucleotide or drug conjugate to be prepared, unmodified or modified nucleoside phosphoramidite monomer (unmodified or modified RNA phosphoramidites; sometimes RNA phosphoramidites are also referred to as nucleoside phosphoramidites) used in a phosphoramidite solid phase synthesis. The phosphoramidite solid phase synthesis is a method for RNA synthesis well known to those skilled in the art. Nucleoside monomers used in the present disclosure are all commercially available.

In the context of the present disclosure, unless otherwise specified, "conjugation" means that two or more chemical moieties each having specific function are linked to each other via a covalent linkage. Correspondingly, a "conjugate" refers to a compound formed by covalent linkage of individual chemical moieties. Furthermore, a "drug conjugate" represents a compound formed by covalently linking one or more chemical moieties each with specific function to an active drug. Hereinafter (in particular the Examples), the drug conjugate of the present disclosure is sometimes abbreviated as "conjugate". According to the context of the present disclosure, the drug conjugate should be understood as the generic term of drug conjugates or specific drug conjugates as shown by specific structural Formulae.

As used herein, a dash ("-") that is not present between two letters or symbols is used to indicate the position that is an attachment point for a substituent. For example, the dash on the far left side in the structure Formula "-$C_1$-$C_{10}$alkyl-$NH_2$" means being linked through the $C_1$-$C_{10}$alkyl.

As used herein, "optional" or "optionally" means that the subsequently described event or circumstance can or can not occur, and that the description includes instances in which the event or circumstance occurs and instances in which it does not. For example, "optionally substituted alkyl" encompasses both "alkyl" and "substituted alkyl" as defined below. Those skilled in the art would understand, with respect to any group containing one or more substituents, that such groups are not intended to introduce any substitution or substitution patterns that are sterically impractical, synthetically infeasible and/or inherently unstable.

As used herein, "alkyl" refers to straight chain and branched chain alkyl having the specified number of carbon atoms, usually from 1 - 20 carbon atoms, for example 1 - 10 carbon atoms, such as, 1 - 8 carbon atoms or 1 - 6 carbon atoms. For example, $C_1$-$C_6$ alkyl encompasses both straight and branched chain alkyl of from 1 - 6 carbon atoms. When an alkyl residue having a specific number of carbon atoms is named, all branched and straight chain forms having that number of carbon atoms are intended to be encompassed; thus, for example, "butyl" is meant to encompass n-butyl, sec-butyl, isobutyl, and t-butyl; "propyl" includes n-propyl and isopropyl. Alkylene is a subset of alkyl, referring to the same residues as alkyl, but having two attachment points.

As used herein, "alkenyl" refers to an unsaturated branched or straight-chain alkyl group having at least one carbon-carbon double bond obtained by repectively removing one hydrogen molecule from two adjacent carbon atoms of the parent alkyl. The group can be in either cis or trans configuration of the double bond(s). Typical alkenyl groups include, but are not limited to, ethenyl; propenyl, such as, prop-1-en-1-yl, prop-1-en-2-yl, prop-2-en-1-yl (allyl), prop-2-en-2-yl; butenyl, such as, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl; and the like. In certain embodiments, an alkenyl group has from 2 - 20 carbon atoms, and in other embodiments, from 2 - 10, 2 - 8, or 2 - 6 carbon atoms. Alkenylene is a subset of alkenyl, referring to the same residues as alkenyl, but having two attachment points.

As used herein, "alkynyl" refers to an unsaturated branched or straight-chain alkyl group having at least one carbon-carbon triple bond obtained by respectively removing two hydrogen molecules from two adjacent carbon atoms of the parent alkyl. Typical alkynyl groups include, but are not limited to, ethynyl; propynyl, such as, prop-1-yn-1-yl, prop-2-yn-1-yl; butynyl, such as, but-1-yn-1-yl, but-1-yn-3-yl, but-3-yn-1-yl; and the like. In certain embodiments, an alkynyl group has from 2 - 20 carbon atoms, and in other embodiments, from 2 - 10, 2 - 8, or 2 - 6 carbon atoms. Alkynylene is a subset of alkynyl, referring to the same residues as alkynyl, but having two attachment points.

As used herein, "alkoxy" refers to an alkyl group of the specified number of carbon atoms linked through an oxygen bridge, for example, methoxy, ethoxy, propoxy, isopropoxy, n-butoxy, sec-butoxy, tert-butoxy, pentyloxy, 2-pentyloxy, isopentyloxy, neopentyloxy, hexyloxy, 2-hexyloxy, 3-hexyloxy, 3-methylpentyloxy, and the like. Alkoxy group usually has from 1 - 10, 1 - 8, 1 - 6, or 1 - 4 carbon atoms linked through oxygen bridge.

As used herein, "aryl" refers to a radical derived from an aromatic monocyclic or multicyclic hydrocarbyl ring system by removing hydrogen atom(s) from a ring carbon atom. The aromatic monocyclic or multicyclic hydrocarbyl ring system contains only hydrogen and carbon, including from 6 - 18 carbon atoms, wherein at least one ring in the ring system is fully unsaturated, i.e., it contains a cyclic, delocalized $(4n+2)\pi$-electron system in accordance with the Hückel theory. Aryl groups include, but are not limited to, groups such as phenyl, fluorenyl, and naphthyl. Arylene is a subset of aryl, referring to the same residues as aryl, but having two attachment points.

As used herein, "cycloalkyl" refers to a non-aromatic carbon ring, usually having from 3 - 7 ring carbon atoms. The ring can be saturated or have one or more carbon-carbon double bonds. Examples of cycloalkyl groups include cyclopropyl, cyclobutyl, cyclopentyl, cyclopentenyl, cyclohexyl, and cyclohexenyl, as well as bridged and caged ring groups, such as norbornane.

As used herein, "halo substituent" or "halo" refers to fluoro, chloro, bromo, and iodo, and the term "halogen" includes fluorine, chlorine, bromine, and iodine.

As used herein, "haloalkyl" refers to alkyl as defined above in which the specified number of carbon atoms are substituted with one or more (up to the maximum allowable number) halogen atoms. Examples of haloalkyl include, but are not limited to, trifluoromethyl, difluoromethyl, 2-fluoroethyl, and penta-fluoroethyl.

"Heterocyclyl" refers to a stable 3 - 18 membered non-aromatic ring radical that comprises 2 - 12 carbon atoms and 1 - 6 heteroatoms selected from nitrogen, oxygen or sulfur. Unless stated otherwise in the specification, the heterocyclyl is a monocyclic, bicyclic, tricyclic, or tetracyclic ring system, which can include fused or bridged ring system(s). The heteroatom(s) in the heterocyclyl can be optionally oxidized. One or more nitrogen atoms, if present, are optionally quaternized. The heterocyclyl is partially or fully saturated. The heterocyclyl can be linked to the rest of the molecule through any ring atom. Examples of such heterocyclyl include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl.

"Heteroaryl" refers to a radical derived from a 3 - 18 membered aromatic ring free radical that comprises 2 - 17 carbon atoms and 1 - 6 heteroatoms selected from nitrogen, oxygen or sulfur. As used herein, the heteroaryl can be a monocyclic, bicyclic, tricyclic or tetracyclic ring system, wherein at least one ring in the ring system is fully unsaturated, i.e., it contains a cyclic, delocalized $(4n+2)\pi$-electron system in accordance with the Hückel theory. Heteroaryl includes fused or bridged ring system(s). The heteroatom(s) in the heteroaryl is optionally oxidized. One or more nitrogen atoms, if present, are optionally quaternized. The heteroaryl is linked to the rest of the molecule through any ring atom. Examples of heteroaryl include, but are not limited to, azepinyl, acridinyl, benzimidazolyl, benzindolyl, 1,3-benzodioxazolyl, benzofuranyl, benzoxazolyl, benzo[d]thiazolyl, benzothiadiazolyl, benzo[*b*][1,4]dioxepinyl, benzo[b][1,4]oxazinyl, 1,4-benzodioxanyl, benzonaphthofuranyl, benzooxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10 hexahydrocycloocta[d]pyrimidinyl, 5,6,7,8,9,10-hexahydrocyclohepta[d]pyridazinyl, 5,6,7,8,9,10- hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinonyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1*H*-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl,

5,6,7,8-tetrahydrobenzo[4,5]thieno[2,3-d]pyrimidinyl, 6,7,8,9-tetrahydro-5H-cyclohepta [4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl and thiophenyl/thienyl.

Various protecting groups can be used in the present disclosure. In geneal, protecting groups render chemical functional groups inert to specific reaction conditions, and can be added to and removed from such functional groups in a molecule without substantially damaging the remainder of the molecule. In some embodiments, the protecting groups used in the present disclosure include, but are not limited to, hydroxyl protecting groups and/or amino protecting groups. Representative hydroxyl protecting groups are disclosed in Beaucage et al., Tetrahedron 1992, 48, 2223-2311, and also in Greene and Wuts, Protective Groups in Organic Synthesis, Chapter 2, 2d ed, John Wiley & Sons, New York, 1991, which are incorporated herein by reference in their entirety. In some embodiments, the hydroxyl protecting group is stable under basic conditions but can be removed under acidic conditions. In some embodiments, non-exclusive examples of the hydroxyl protecting groups that can be used herein include dimethoxytrityl (DMT), monomethoxytrityl, 9-phenylxanthen-9-yl (Pixyl) and 9-(*p*-methoxyphenyl)xanthen-9-yl (Mox). In some embodiments, non-exclusive examples of the hydroxyl protection groups that can be used herein comprises Tr (trityl), MMTr (4-methoxytrityl), DMTr (4,4'-dimethoxytrityl), and TMTr (4,4',4"-trimethoxytrityl). In some embodiments, non-exclusive examples of the amino protecting groups that can be used herein include benzyloxycarbonyl (Cbz), tert-butoxycarbonyl (Boc), fluorenylmethyloxycarbonyl (Fmoc), allyloxycarbonyl (Alloc), trimethylsilylethoxycarbonyl (Teoc), optionally substituted haloacyl (such as, acetyl or trifluoroacetyl) and benzyl (Bn).

The term "subject", as used herein, refers to any animal, e.g., a mammal or marsupial. The subject of the present disclosure includes, but is not limited to, human, non-human primate (e.g., rhesus or other types of macaques), mouse, pig, horse, donkey, cattle, sheep, rat, and any kind of poultry.

As used herein, "treating", "alleviating" or "ameliorating" can be used interchangeably herein. These terms refer to an approach for obtaining beneficial or desirable results, including but not limited to therapeutic benefits. "Therapeutic benefit" refers to eradication or amelioration of the underlying disorder being treated. Also, a therapeutic benefit is achieved by eradicating or ameliorating one or more physiological symptoms associated with the underlying disorder, thereby observing amelioration in the subject, although the subject can still be afflicted with the underlying disorder.

As used herein, "prevention" and "preventing" can be used interchangeably. These terms refer to an approach for obtaining beneficial or desirable results, including but not limited to, prophylactic benefit. In order to obtain "prophylactic benefit", the conjugates or compositions can be administered to a subject at risk of developing a particular disease, or to a subject reporting one or more physiological symptoms of a disease, even a diagnosis of this disease has not been made yet.

## First compound

In one aspect, the present disclosure provides a compound having a structure as shown by Formula (101):

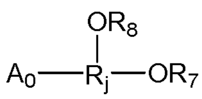

Formula (101)

wherein,

- $R_j$ is a linking group;
- $R_7$ is a functional group capable of forming a phosphoester linkage, phosphorothioate linkage, phosphoroborate linkage, or carboxylate linkage with a hydroxyl group via reaction, or a functional group capable of forming an amide linkage with an amino group via reaction;
- $R_8$ is a hydroxyl protecting group;
- $A_0$ has a structure as shown by Formula (312):

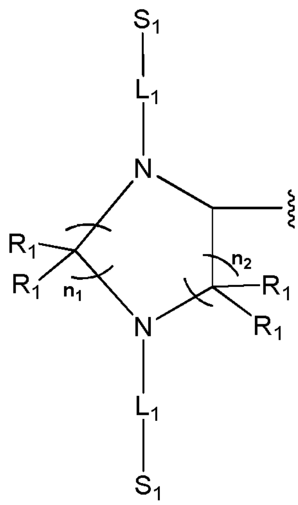

Formula (312)

wherein

$n_1$ is an integer of 1-4;
$n_2$ is an integer of 0-3;
each $L_1$ is a linear alkylene of 1 - 70 carbon atoms in length, wherein one or more carbon atoms are optionally replaced with one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, -$OC_1$-$C_{10}$ alkyl, -$OC_1$-$C_{10}$ alkylphenyl, -$C_1$-$C_{10}$ alkyl-OH, -$OC_1$-$C_{10}$ haloalkyl, -$SC_1$-$C_{10}$ alkyl, -$SC_1$-$C_{10}$ alkylphenyl, -$C_1$-$C_{10}$ alkyl-SH, -$SC_1$-$C_{10}$ haloalkyl, halo, -OH, -SH, -$NH_2$, -$C_1$-$C_{10}$ alkyl-$NH_2$, -N($C_1$-$C_{10}$ alkyl)($C_1$-$C_{10}$ alkyl), -NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, -$CO_2$H, -C(O)O($C_1$-$C_{10}$ alkyl), -CON($C_1$-$C_{10}$ alkyl)($C_1$-$C_{10}$ alkyl), -CONH($C_1$-$C_{10}$ alkyl), -$CONH_2$, -NHC(O)($C_1$-$C_{10}$ alkyl), -NHC(O)(phenyl), -N($C_1$-$C_{10}$ alkyl)C(O)($C_1$-$C_{10}$ alkyl), -N(Ci-Cio alkyl)C(O)(phenyl), -C(O)$C_1$-$C_{10}$ alkyl, -C(O)$C_1$-$C_{10}$ alkylphenyl, -C(O)$C_1$-$C_{10}$ haloalkyl, -OC(O)$C_1$-$C_{10}$ alkyl, - $SO_2$($C_1$-$C_{10}$ alkyl), -$SO_2$(phenyl), -$SO_2$($C_1$-$C_{10}$ haloalkyl), -$SO_2NH_2$, -$SO_2$NH($C_1$-$C_{10}$ alkyl), -$SO_2$NH(phenyl), -$NHSO_2$($C_1$-$C_{10}$ alkyl), -$NHSO_2$(phenyl), and -$NHSO_2$($C_1$-$C_{10}$ haloalkyl).

In some embodiments, $L_1$ can be selected from the group consisting of the groups of Formulae (A1)-(A26) or any combination thereof:

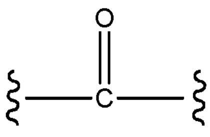,
(A1)

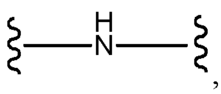,
(A2)

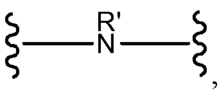,
(A3)

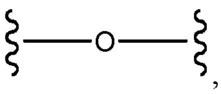,
(A4)

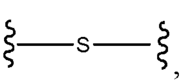,
(A5)

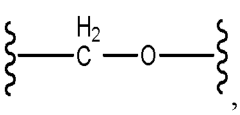,
(A6)

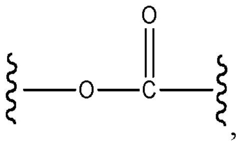,
(A7)

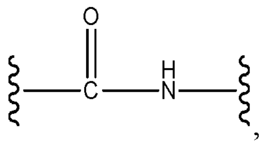,
(A8)

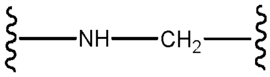,
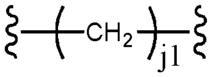,
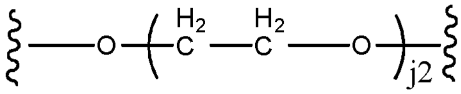,

(A9) (A10) (A11)

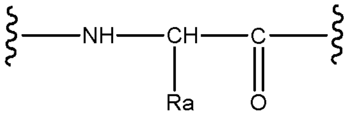,
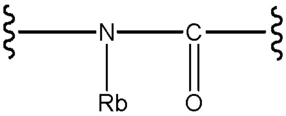,
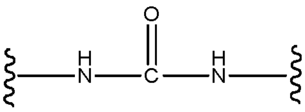,

(A12) (A13) (A14)

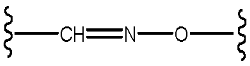,
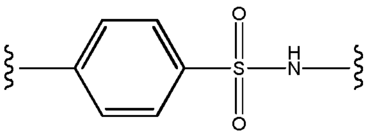,
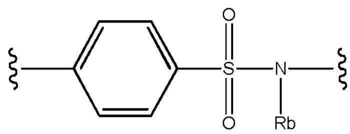,

(A15) (A16) (A17)

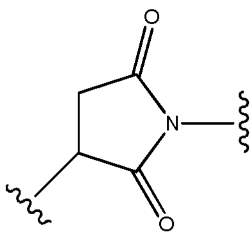,
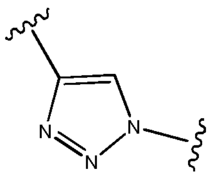,
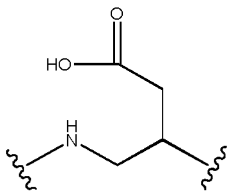,
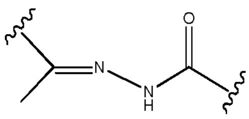,

(A18) (A19) (A20) (A21)

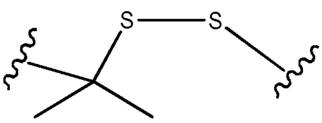,
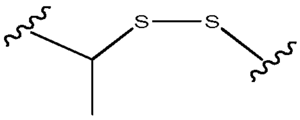,
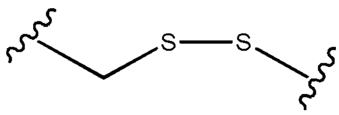,

(A22) (A23) (A24)

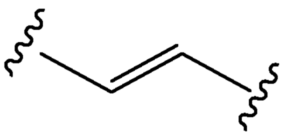,
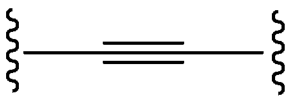;

(A25) (A26)

wherein

j 1 is an integer of 1-20;
j2 is an integer of 1-20;
R' is a $C_1$-$C_{10}$ alkyl;
Ra is a group selected from one of the groups of Formulae (A27)-(A45):

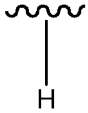,
(A27)

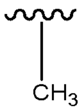,
(A28)

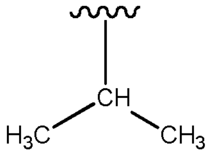,
(A29)

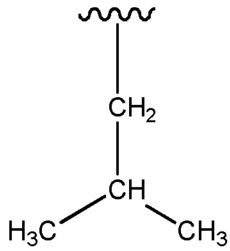,
(A30)

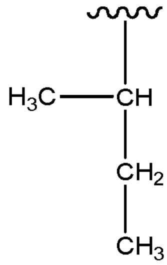,
(A31)

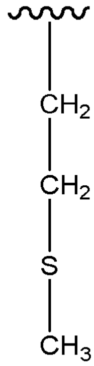,
(A32)

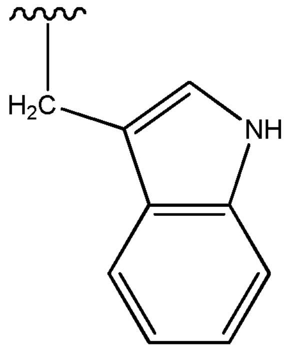,
(A33)

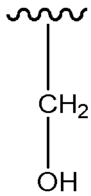,
(A34)

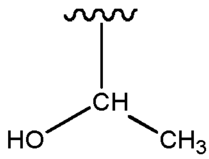,
(A35)

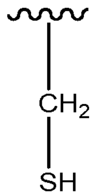,
(A36)

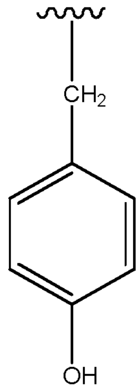,
(A37)

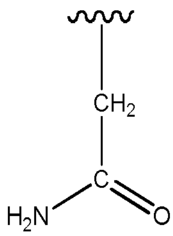,
(A38)

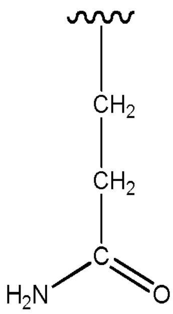,
(A39)

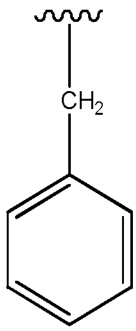,
(A40)

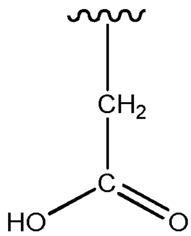,
(A41)

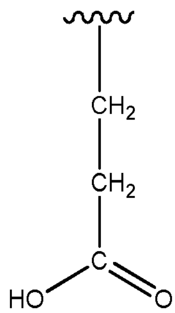,
(A42)

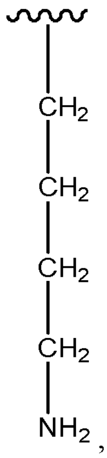 , 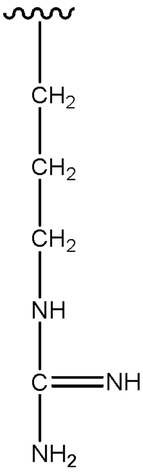 or 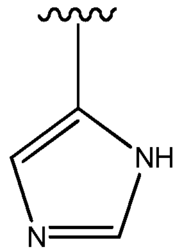 ;

(A43) (A44) (A45)

Rb is a $C_1$-$C_{10}$ alkyl; and

 represents the site where a group is covalently linked.

Those skilled in the art would understand that, though $L_1$ is defined as a linear alkylene for convenience, but it can not be a linear group or be named differently, such as, an amine or alkenylene produced by the above replacement and/or substitution. For the purpose of the present disclosure, the length of $L_1$ is the number of the atoms in the chain linking the two attachment points. For this purpose, a ring obtained by replacing a carbon atom in the linear alkylene, such as, a heterocyclylene or heteroarylene, is counted as one atom.

In some embodiments, in Formula (312), each $S_1$ is independently a $M_1$, wherein all active hydroxyl and/or amino groups (if any) are protected with protecting groups; each $M_1$ is independently selected from a ligand capbale of binding to a cell surface receptor. In some embodiments, in Formula (312), each $R_1$ independently of one another is selected from the group consisting of H, substituted or unsubstituted $C_1$-$C_4$ hydrocarbyl or halogen; $n_1$ can be an integer of 1-4; and $n_2$ can be an integer of 0-3; in some embodiments, $n_1$ is an integer of 1-2; and $n_2$ is an integer of 1-2. The drug conjugate can thus have a more stable structure. In view of various factors such as synthesis convenience, structure/process costs and delivery efficiency, in some embodiments, $n_1$ is 2; and $n_2$ is 1. In this case, $A_0$ has a structure as shown by Formula (120):

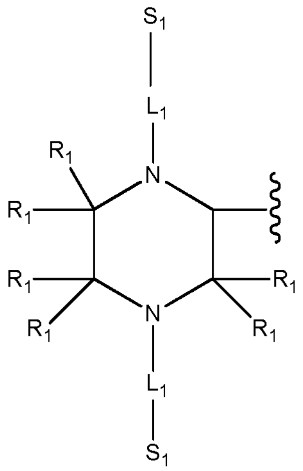

Formula (120).

Those skilled in the art could understand that when each $R_1$ independently of one another is selected from one of H, substituted or unsubstituted $C_1$-$C_4$ hydrocarbyl or halogen, they would not change the properties of the compound as shown by Formula (101) and could all achieve the purpose of the present disclosure. However, for simplifying the

compound of the present disclosure and for ease of synthesis, in some embodiments, each $R_1$ is H.

$R_j$ is a linking group containing three covalent linking sites, and exerts the function of linking Formula $A_0$ to provide an appropriate spatial position in the compound as shown by Formula (101). In some embodiments, $R_j$ is any group capable of achieving linkage with $A_0$, $OR_7$ and $OR_8$. For ease of synthesis, in some embodiments, $R_j$ can have an amide or ester bond structure. In some embodiments, $R_j$ is selected from one of the groups of Formulae (A62)-(A67):

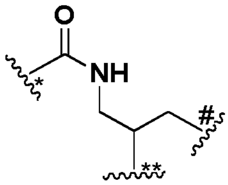,
Formula (A62)

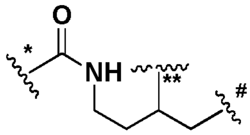,
Formula (A63)

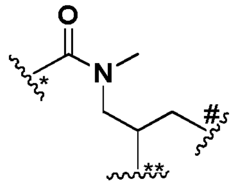,
Formula (A64)

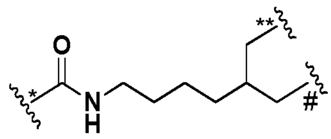,
Formula (A65)

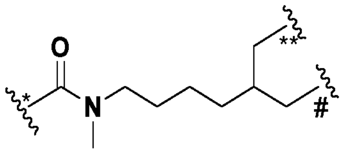, and
Formula (A66)

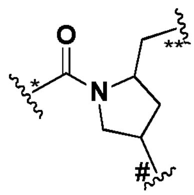
Formula (A67).

In these Formulae, $R_j$ can be arbitrarily linked to $A_0$, $OR_7$, and $OR_8$. In some embodiments, each "*" in Formulae A62-A67 represents a site linking to Ao; and each "**" or "#" in Formulae A62-A67 independently of one another represents a site linking to $OR_7$ or $OR_8$. In some embodiments, $R_j$ has chirality. In some embodiments, $R_j$ is racemic. In some embodiments, $R_j$ is chirally pure.

In some embodiments, the active drug to be conjugated to the compound as shown by Formula (101) is a functional oligonucleotide. In some embodiments, $R_7$ is a functional group capable of forming a phosphoester linkage, phosphorothioate linkage, phosphoroborate linkage, or carboxylate linkage with a hydroxyl group via reaction, or a functional group capable of forming an amide linkage with an amino group via reaction. Therefore, the compound as shown by Formula (101) can be linked to a solid phase support having a hydroxyl group through the above-mentioned phosphoester linkage, phosphorothioate linkage, carboxylate linkage, or amide linkage, thereby providing suitable reaction condition for subsequent linkage with nucleoside monomers. Alternatively, the compound as shown by Formula (101) can be linked to the hydroxyl group in the nucleotide sequence linked to the solid phase support through the above-mentioned phosphoester linkage, phosphorothioate linkage, carboxylate linkage, or amide linkage, thereby conjugating the compound as shown by Formula (101) to the active drug, in particular a functional oligonucleotide.

In some embodiments, $R_7$ is a group containing a phosphoramidite functional group and having a structure as shown by Formula (A46):

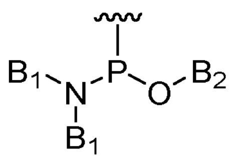

Formual (A46)

wherein, $B_1$ is selected from substituted or unsubstituted $C_1$-$C_5$ hydrocarbyl;

$B_2$ is selected from $C_1$-$C_5$ alkyl, ethylcyano, propcyano, and butyrcyano.

In some embodiments, the group containing a phosphoramidite functional group has a structure as shown by Formula (C3):

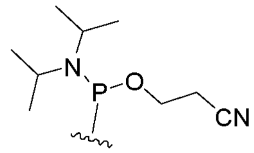

Formula (C3).

In some embodiments, $R_7$ is a group containing a carboxyl or carboxylate functional group and having a structure as shown by Formula (C1) or (C2):

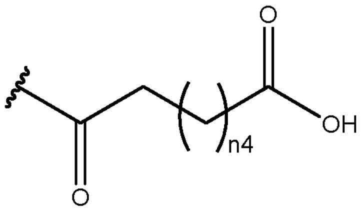

,

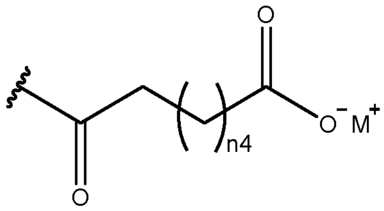

Formula (C1) Formula (C2);

wherein, n4 is an integer of 1 - 4;
$M^+$ is a cation; and
 represents a site where a group is linked.

The hydroxyl protecting group $R_8$ is selected to replace the hydrogen on the hydroxyl group to form a non-reactive group. The protecting group $R_8$ can be removed in the subsequent reaction process, thereby re-releasing the active hydroxyl group to participate in the subsequent reaction. The types of the hydroxyl protecting groups are well-known to those skilled in the art and can be, for example, Tr (trityl), MMTr (4-methoxytrityl), DMTr (4,4'-dimethoxytrityl), or TMTr (4,4',4"-trimethoxytrityl). In some embodiments, $R_8$ can be DMTr, i.e., 4,4'-dimethoxytrityl.

According to the above description, those skilled in the art that could easily understand that as compared with the well-known phosphoramidite solid phase synthesis method in the art, the compound as shown by Formula (101) can be linked to any possible position of the nucleotide sequence via $R_7$ and $R_8$ above; for example, the compound as shown by Formula (101) is linked to a terminal region of the nucleotide sequence, or to a terminal of the nucleotide sequence. Correspondingly, unless otherwise specified, in the following description regarding the reaction of the compound as shown by Formula (101), when referring to the reactions such as "deprotection", "coupling", "capping", "oxidation", "sulfurization", it will be understood that the reaction conditions and agents involved in the well-known phosphoramidite solid phase synthesis method of nucleic acid in the art would also apply to these reactions. Exemplary reaction conditions and agents will be described hereinafter in detail.

$L_1$ exerts the function of linking the $M_1$ ligand capable of binding to a cell surface receptor, or the $S_1$ group obtained by protecting the $M_1$ ligand, to the N atom in the heterocyclic structure of the Formula (312), thereby providing targeting function for the drug conjugate of the present disclosure. In some embodiments, $L_1$ selected from one of the groups of Formulae A1-A26 or any connection combinations thereof could all achieve the desired purpose above. In order to make the spatial position among the $M_1$ ligands in the drug conjugate more suitable for the binding between the $M_1$ ligands and the cell surface receptors, and save cost, in some embodiments, $L_1$ is selected from one of A1, A2, A4, A5, A6, A8, A10, A11, A13 or any connection combinations thereof. In some embodiments, $L_1$ is selected from the connection combinations of at least two of Formulae (A1), (A2), (A4), (A8), (A10), and (A11). In some embodiments, $L_1$ is selected from the connection combinations of at least two of Formulae (A1), (A2), (A8) and (A10).

In order to realize the above function of Li, in some embodiments, $L_1$ can have a length of 3 - 25, 3 - 20, 4 - 15, or 5 - 12 atoms. Unless otherwise specified, in the context of the present disclosure, the length of $L_1$ refers to the number of the chain-forming atoms in the longest atomic chain formed from the atom linked to the N atom in the heterocyclic structure of Formula (302) to the atom linked to $S_1$ (or $M_1$ in the conjugate described below).

In Formulae (A1)-(A26), j 1, j2, R', Ra, and Rb are respectively selected to achieve the linkage between the $M_1$ ligand and the N atom in $A_0$ of the drug conjugate, and make the spatial position among the $M_1$ ligands more suitable for the binding between the $M_1$ ligands and the cell surface receptors. Thus, in some embodiments, j 1 is an integer of 2-10, and in some embodiments, j 1 is an integer of 3-5. In some embodiments, j2 is an integer of 2-10, and in some

embodiments, j2 is an integer of 3-5. In some embodiments, R' is $C_1$-$C_4$ alkyl, and in some embodiments, R' is one of methyl, ethyl, and isopropyl. In some embodiments, Ra is one of Formulae A27, A28, A29 and A30, and in some embodiments, Ra is A27 or A28. Rb is a $C_1$-$C_5$ alkyl, and in some embodiments, Rb is one of methyl, ethyl, isopropyl, or butyl.

In some embodiments, the pharmaceutically acceptable targeting group can be selected from one or more of the ligands fromed by the following targeting molecules or derivatives thereof: lipophilic molecules, such as, cholesterol, bile acids, vitamins (such as vitamin E), lipid molecules with different chain lengths; polymers, such as polyethylene glycol; polypeptides, such as cell-penetrating peptide; aptamers; antibodies; quantum dots; saccharides, such as, lactose, polylactose, mannose, galactose, N-acetylgalactosamine (GalNAc); endosomolytic component; folate; or receptor ligands expressed in hepatic parenchymal cells, such as, asialoglycoprotein, asialo-sugar residue, lipoproteins (such as, high density lipoprotein, low density lipoprotein and the like), glucagon, neurotransmitters (such as adrenaline), growth factors, transferrin and the like.

In some embodiments, each ligand is independently selected from a ligand capable of binding to a cell surface receptor. In some embodiments, at least one ligand is a ligand capable of binding to a surface receptor of a hepatocyte. In some embodiments, at least one ligand is a ligand capable of binding to a surface receptor of a mammalian hepatocyte. In some embodiments, at least one ligand is a ligand capable of binding to a surface receptor of a human hepatocyte. In some embodiments, at least one ligand is a ligand capable of binding to an asialoglycoprotein receptor (ASGPR) on the surface of hepatocytes. At least one ligand is a ligand capable of binding to a surface receptor of a lung cell. In some embodiments, at least one ligand is a ligand capable of binding to a surface receptor of a tumor cell. The types of these ligands are well-known to those skilled in the art, and they typically serve the function of binding to specific receptors on the cell surface, thereby mediating delivery of the double-stranded oligonucleotide linked to the ligand into the cell.

In some embodiments, the pharmaceutically acceptable targeting group can be any ligand that binds to the asialoglycoprotein receptors (ASGPR) on the surface of mammalian hepatocytes. In some embodiments, each ligand is independently an asialoglycoprotein, such as, asialoorosomucoid (ASOR) or asialofetuin (ASF). In some embodiments, the ligand is a saccharide or derivatives thereof. In some embodiments, the pharmaceutically acceptable targeting group can be any ligand that binds to surface receptors of tumor cells. In some embodiments, the ligand is folate or folate derivatives.

In some embodiments, at least one ligand is a saccharide. In some embodiments, each ligand is a saccharide. In some embodiments, at least one ligand is a monosaccharide, polysaccharide, modified monosaccharide, modified polysaccharide, or saccharide derivative. In some embodiments, at least one ligand can be a monosaccharide, disaccharide or trisaccharide. In some embodiments, at least one ligand is a modified saccharide. In some embodiments, each ligand is a modified saccharide. In some embodiments, each ligand is independently selected from a polysaccharide, modified polysaccharide, monosaccharide, modified monosaccharide, polysaccharide derivative, or monosaccharide derivative. In some embodiments, each ligand or at least one ligand can be independently selected from the group consisting of glucose and its derivatives, mannose and its derivatives, galactose and its derivatives, xylose and its derivatives, ribose and its derivatives, fucose and its derivatives, lactose and its derivatives, maltose and its derivatives, arabinose and its derivatives, fructose and its derivatives, and sialic acid. In some embodiments, each $M_1$ can be independently selected from the group consisting of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucopyranose, methyl 2,3,4-tris-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, and L-4-thioribose. In some embodiments, each $M_1$ is N-acetylgalactosamine (GalNAc). Other options of the ligand can be found, for example, in the disclosure of CN105378082A, which is incorporated herein by reference in its entirety.

In some embodiments, each $S_1$ is independently a group formed by protecting all active hydroxyl groups in $M_1$ with hydroxyl protecting groups which will be removed in the subsequent steps to obtain $M_1$ ligands. In some embodiments, the hydroxyl protecting groups are acyl groups having a structure of YCO-.

In some embodiments, each $S_1$ independently of one another is selected from one of the groups of Formulae (A51)-(A59):

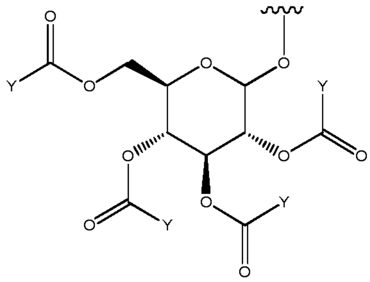,
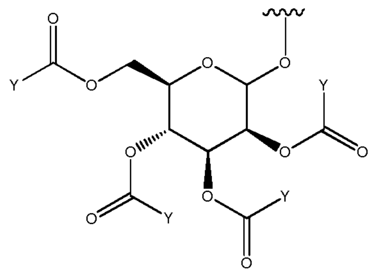,
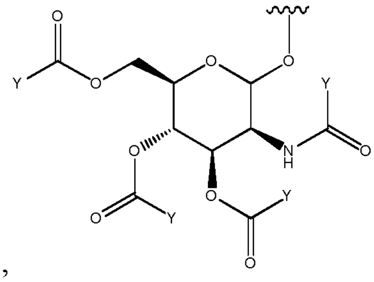,

(A51) (A52) (A53)

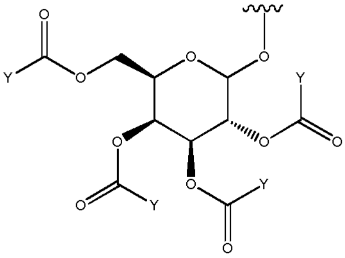,
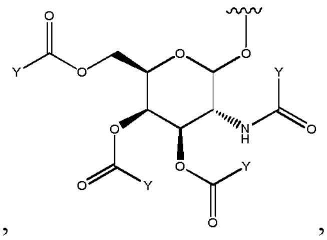,
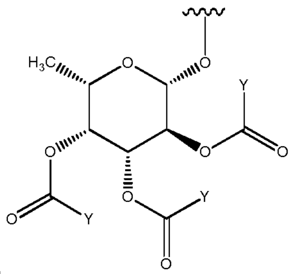,

(A54) (A55) (A56)

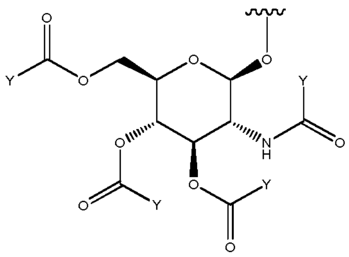,
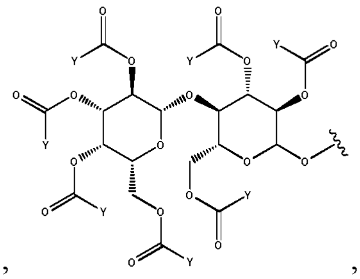, and
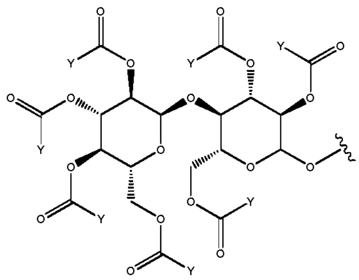

(A57) (A58) (A59).

In some embodiments, $S_1$ is Formula A54 or A55.

Each Y is independently selected from one of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl. For simplifying the compound as shown by Formula (101) of the present disclosure, in some embodiments, Y is methyl.

In some embodiments, each $M_1$ is independently selected from one of the ligands formed by the molecules or derivatives of: lipophilic molecules, saccharides, vitamins, polypeptides, endosomolytic components, steroid compounds, terpene compounds, integrin receptor inhibitors and cationic lipid molecules. In some embodiments, each $M_1$ is independently selected from the ligands formed by one compound of: cholesterol, cholic acid, adamantaneacetic acid, 1-pyrenebutyric acid, dihydrotestosterone, 1,3-Bis-O(hexadecyl)glycerol, hexaglycerol, menthol, mentha-camphor, 1,3-propanediol, palmitic acid, myristic acid, O3-(oleoyl)lithocholic acid, benzoxazine, folate, folate derivatives, vitamin A, vitamin B7 (biotin), pyridoxal, uvaol, triterpene, friedelin, and epifriedelinol-derived lithocholic acid. In some embodiments, each $M_1$ is independently selected from one of geranyloxyhexyl, heptadecyl, dimethoxytrityl, hecogenin, diosgenin, and sarsasapogenin. In some embodiments, each $M_1$ is independently a ligand fromed by folate or derivatives thereof. The folate derivatives can be, for example, folate analogues or folate mimetics. In some embodiments, each $M_1$ is independently a ligand formed by one of the following compounds: folate, folate analogues or folate mimetics. In some embodiments, folate analogues are groups having a similar backbone structure to folate and having similar functional groups at the same receptor binding site. In some embodiments, the folate mimetics are groups having the same main functional groups as folate, which have similar spatial configuration to the corresponding functional groups of folate.

In some embodiments, each $M_1$ independently of one another is selected from one of the groups of Formulae (H1)-(H5):

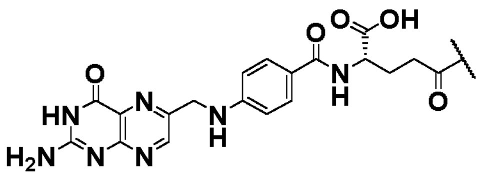
,
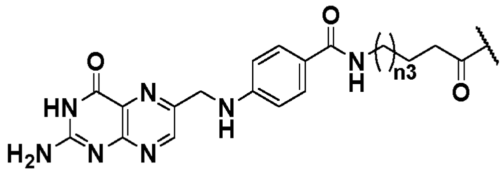
,

Formula (H1) Formula (H2)

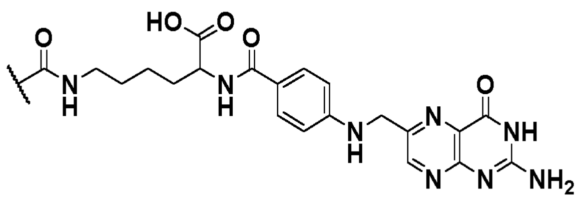
,
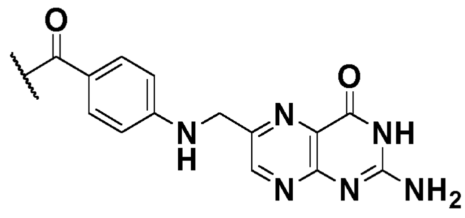
, and

Formula (H3) Formula (H4)

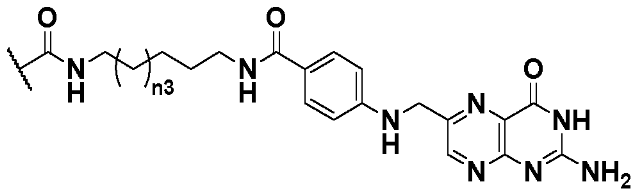
,

Formula (H5)

wherein n3 is an integer of 1-5. In some embodiments, $M_1$ is a group as shown by Formula (HI).

In some embodiments, each $S_1$ is independently selected from one of the groups of Formulae (A71)-(A75):

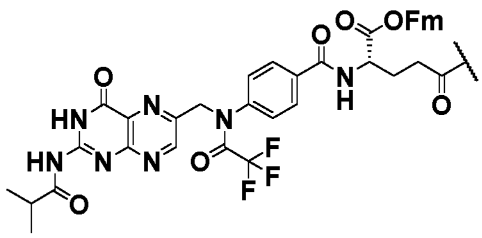
,
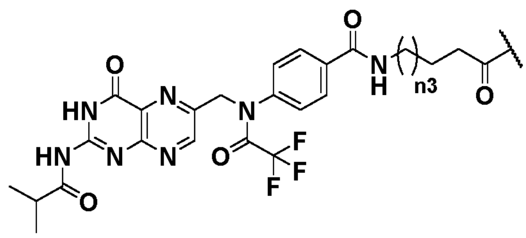
,

Formula (A71) Formula (A72)

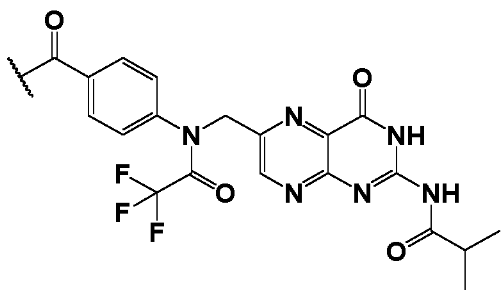
,
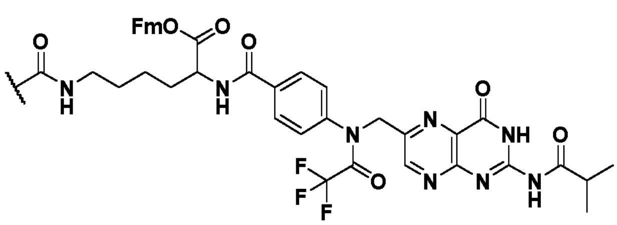
, and

Formula (A73) Formula (A74)

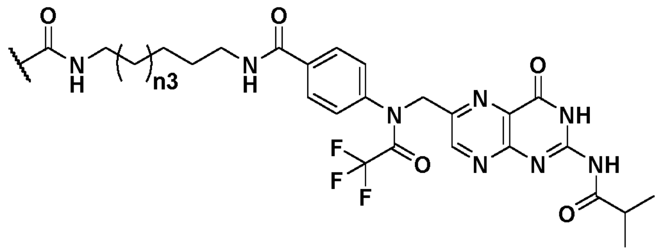

Formula (A75)

wherein n3 is an integer of 1-5, and Fm refers to 9-fluorenemethyl. In some embodiments, $S_1$ is a group of Formula (A71).

According to some embodiments of the present disclosure, the compound as shown by Formula (101) has a structure as shown by Formula (403), (404), (405), (406), (407), or (408):

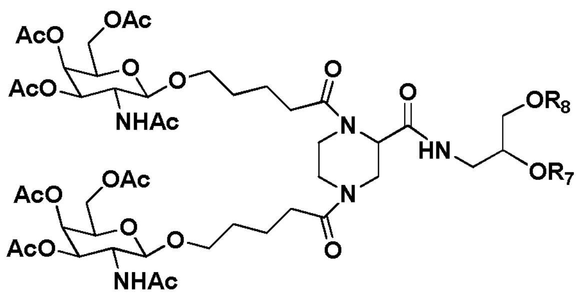

,

Formula (403)

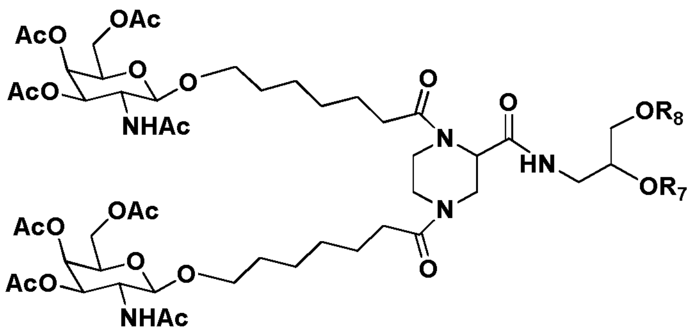

,

Formula (404)

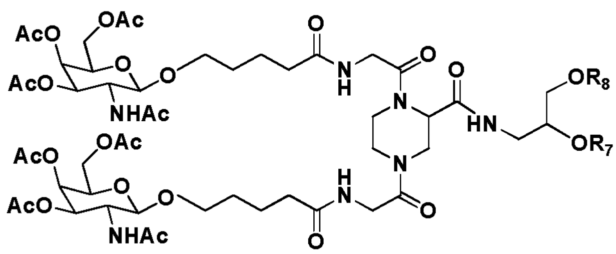

,

Formula (405)

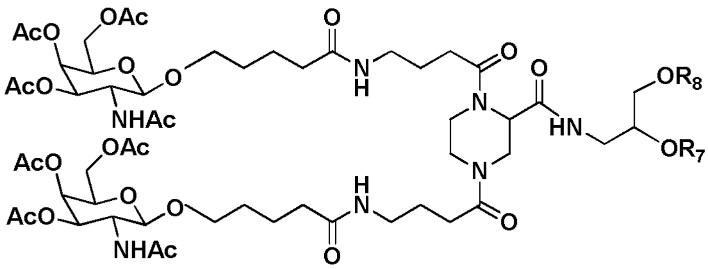

,

Formula (406)

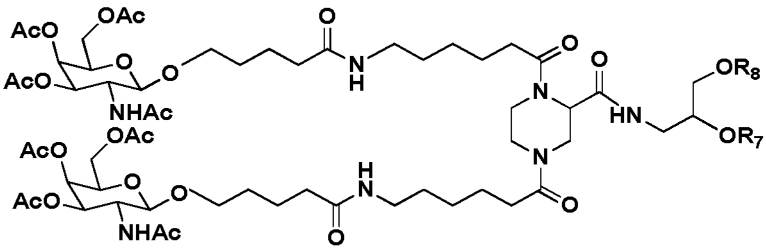

, or

Formula (407)

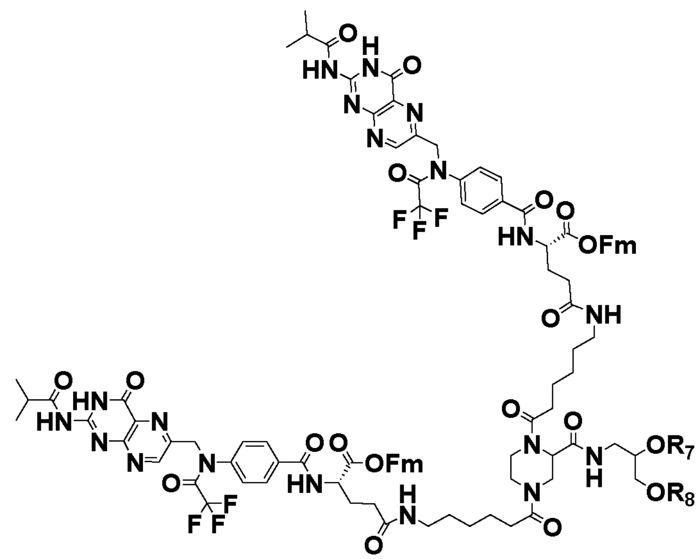

Formula (408).

**Preparation of the compound as shown by Formula (101)**

The compound as shown by Formula (101) can be prepared by any appropriate synthetic routes.

In some embodiments, the compound as shown by Formula (101) can be prepared by the following method, comprising:

contacting a compound as shown by Formula (102) with a phosphorodiamidite as shown by Formula (103) in an organic solvent under substitution reaction condition in the presence of an activator and a catalyst, and isolating the compound as shown by Formula (101),

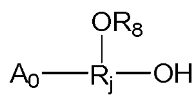

Formula (102)

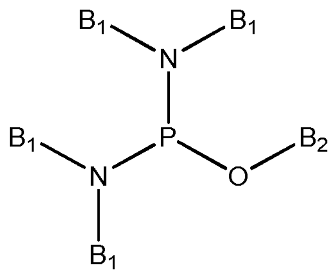

Formula (103)

wherein the definitions and options of $A_0$, $R_j$ and $R_8$ are respectively as described above; each $B_1$ is independently a $C_1$-$C_5$ alkyl; and $B_2$ is selected from one of $C_1$-$C_5$ alkyl, ethylcyano, propcyano and butyrcyano. In this case, $R_7$ in the resultant compound of Formula (101) is a group comprising a phosphoramidite functional group as shown by Formula (C3).

The compound as shown by Formula (103) can be commercially available or synthesized by those skilled in the art via well-known methods. In some embodiments, the compound as shown by Formula (103) is commercially available bis(diisopropylamino)(2-cyanoethoxy)phosphine.

The substitution reaction condition comprises a reaction temperature of 0-100°C and a reaction time of 1-20 hours. In some embodiments, the substitution reaction condition comprises a reaction temperature of 10-40°C, and a reaction time of 2-8 hours.

The organic solvent can be one or more of an epoxy solvent, an ether solvent, an haloalkane solvent, dimethyl sulfoxide, N,N-dimethylformamide, and N,N-diisopropylethylamine. The epoxy solvent can be, for example, dioxane and/or tetrahydrofuran; the ether solvent can be, for example, diethyl ether and/or methyl tertbutyl ether; and the haloalkane solvent can be, for example, one or more of dichloromethane, trichloromethane, and 1,2-dichloroethane. In some embodiments, the organic solvent is dichloromethane. With respect to the compound as shown by Formula (102), the amount of the organic solvent can be 3-50 L/mol, such as 5-20 L/mol.

The activator can be pyridinium trifluoroacetate. The ratio of the activator to the compound as shown by Formula (102) can be 0.1:1 - 5:1, such as 0.5:1 - 3:1.

The catalyst can be imidazole or N-methylimidazole, such as N-methylimidazole. The ratio of the catalyst to the compound as shown by Formula (102) can be 0.1:1 - 5:1, such as 0.5:1 - 3:1.

The molar ratio of the compound as shown by Formula (103) to the compound as shown by Formula (102) can be 0.5:1 - 5:1, such as 0.5:1 - 3:1.

The compound as shown by Formula (101) can be isolated from the reaction mixture by any suitable isolation methods. In some embodiments, the compound as shown by Formula (101) can be isolated by removal of solvent via evaporation followed by chromatography, for example, using the following chromatographic conditions for isolation:

normal phase purification of silica gel: 200-300 mesh silica gel filler, gradient elution of petroleum ether: ethyl acetate = 2:1 - 1:2;
reverse phase purification: C18 and C8 reverse phase filler, gradient elution of methanol:acetonitrile = 0.1:1 - 1:0.1.

In some embodiments, the solvent can be directly removed to obtain a crude product of the compound as shown by Formula (101), which can be directly used in subsequent reactions.

In some embodiments, the compound as shown by Formula (102) can be prepared by the following method, comprising:
contacting a compound as shown by Formula (104) with a compound as shown by Formula (105) in an organic solvent under amidation reaction condition in the presence of an activator and an organic base of tertiary amine, and isolating the compound as shown by Formula (102),

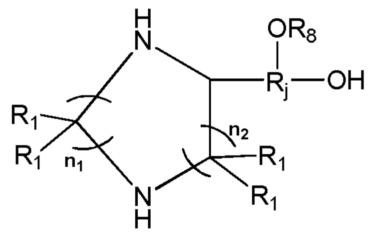

Formula (104)

$S_1$—$L_1$—COOH

Formula (105)

wherein, the definitions and options of $n_1$, $n_2$, $R_1$, $R_8$, and $R_j$ are respectively as described above.

The compound as shown by Formula (105) can be commercially available or prepared by those skilled in the art via various methods. For example, some compounds of Formula (105) can be prepared according to the method disclosed in Example 1 of the US patent US8106022B2, which is incorporated herein by reference in its entirety.

The amidation reaction condition comprises a reaction temperature of 0-100 °C and a reaction time of 8-48 hours. In some embodiments, the amidation reaction condition comprises a reaction temperature of 10-40 °C and a reaction time of 8-20 hours.

The organic solvent can be one or more of an epoxy solvent, an ether solvent, an haloalkane solvent, dimethyl sulfoxide, N,N-dimethylformamide, and N,N-diisopropylethylamine. The epoxy solvent can be, for example, dioxane and/or tetrahydrofuran; the ether solvent can be, for example, diethyl ether and/or methyl tertbutyl ether; and the haloalkane solvent can be, for example, one or more of dichloromethane, trichloromethane, and 1,2-dichloroethane. In some embodiments, the organic solvent is dichloromethane. With respect to the compound as shown by Formula (104), the amount of the organic solvent can be 3-50 L/mol, such as 5-20 L/mol.

The activator can be one of 3-(diethoxyphosphoryloxy)-1,2,3-benzotrizin-4(3H)-one (DEPBT), O-benzotriazol-tetramethyluronium hexafluorophosphate, 2-(7-oxybenzotriazol)-N,N,N',N'-tetramethyluronium hexafluorophosphate, and dicyclohexylcarbodiimide, such as 3-(diethoxyphosphoryloxy)-1,2,3-benzotrizin-4(3H)-one (DEPBT). The ratio of the activator to the compound as shown by Formula (104) is 0.1:1 - 10:1, such as 1:1 - 5:1.

The organic base of tertiary amine can be one of triethylamine, tripropylamine, tributylamine, and diisopropylethylamine, preferably diisopropylethylamine. The ratio of the organic base of tertiary amine to the compound as shown by Formula (104) is 0.5:1 - 20:1, such as 1:1 - 10:1.

The molar ratio of the compound as shown by Formula (105) to the compound as shown by Formula (104) can be 0.5:1 - 100:1, such as 2:1 - 10:1.

Similarly, the compound as shown by Formula (102) can be isolated from the reaction mixture by any suitable isolation methods. In some embodiments, the compound as shown by Formula (102) can be isolated by removal of solvent via evaporation followed by chromatography, for example, using the following chromatographic conditions for isolation:

normal phase purification of silica gel: 200-300 mesh silica gel filler, gradient elution of petroleum ether: ethyl acetate: dichloromethane: methanol=1:1:1:0.05 - 1:1:1:0.2;

reverse phase purification: C18 and C8 reverse phase filler, gradient elution of methanol:acetonitrile = 0.1:1 - 1:0.1.

In some embodiments, the solvent can be directly removed to obtain a crude product of the compound as shown by Formula (102), which can be directly used in subsequent reactions.

In some embodiments, the compound as shown by Formula (104) can be prepared by the following method, comprising:

contacting and reacting a compound as shown by Formula (106) with an alkaline agent in an organic solvent under substitution reaction condition, and isolating the compound as shown by Formula (104),

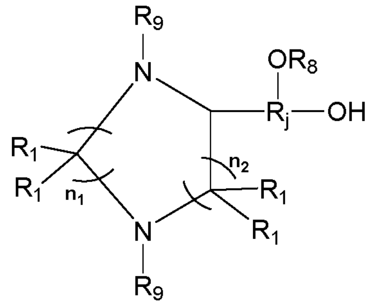

Formula (106)

wherein the definitions and options of $n_1$, $n_2$, $R_1$, $R_8$, and $R_j$ are respectively as described above,

$R_9$ is an amino protection group and can be selected from Formula (A69) or (A70):

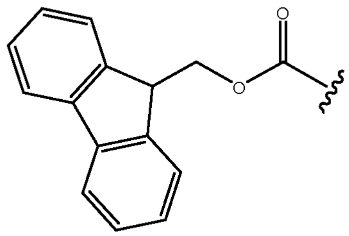

,

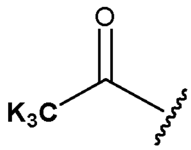

Formula (A69) Formula (A70)

wherein K in Formula (A70) represents halogen; each K is selected from one of F, Cl, Br, and I; in some embodiments, K is F or Cl.

The substitution reaction condition comprises a reaction temperature of 0-100°C, and a reaction time of 5 minutes to 5 hours. In some embodiments, the substitution reaction condition comprises a reaction temperature of 10 - 40°C, and a reaction time of 0.3 - 3 hours.

The organic solvent can be one or more of an epoxy solvent, an ether solvent, an haloalkane solvent, dimethyl sulfoxide, N,N-dimethylformamide, and N,N-diisopropylethylamine. The epoxy solvent can be, for example, dioxane and/or tetrahydrofuran; the ether solvent can be, for example, diethyl ether and/or methyl tertbutyl ether; and the haloalkane solvent can be, for example, one or more of dichloromethane, trichloromethane, and 1,2-dichloroethane. In some embodiments, the organic solvent is N,N-dimethylformamide. With respect to the compound as shown by Formula (106), the amount of the organic solvent can be 1-50 L/mol, such as 1-20 L/mol.

The alkaline agent can be one or more of piperidine, ammonia and methylamine. In some embodiments, ammonia is provided in the form of 25-28 wt% aqueous solution; methylamine is provided in the form of 30-40 wt% aqueous solution. In some embodiments, the alkaline agent is piperidine. The molar ratio of the alkaline agent to the compound as shown by Formula (106) can be 1:1 - 100:1, such as 10:1 - 50:1.

Similarly, the compound as shown by Formula (104) can be isolated from the reaction mixture by any suitable isolation methods. In some embodiments, the compound as shown by Formula (104) can be isolated by removal of solvent via evaporation followed by chromatography, for example, using the following chromatographic conditions for isolation:

normal phase purification of silica gel: 200-300 mesh silica gel filler, gradient elution of ethyl acetate: methanol=1:1 - 1:10;

reverse phase purification: C18 and C8 reverse phase filler, gradient elution of methanol:acetonitrile = 0.1:1-1:0.1.

In some embodiments, the solvent can be directly removed to obtain a crude product of the compound as shown by Formula (104), which can be directly used in subsequent reactions.

In some embodiments, the compound as shown by Formula (106) can be prepared by the following method, comprising:

contacting a compound as shown by Formula (107) with a hydroxyl protection agent in an organic solvent under substitution reaction condition, and isolating the compound as shown by Formula (106),

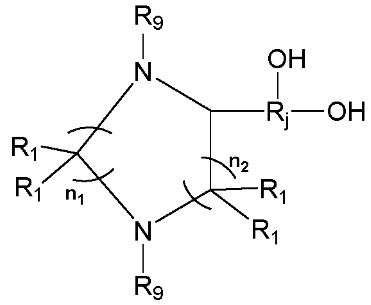

Formula (107)

wherein the definitions and options of $n_1$, $n_2$, $R_1$, $R_9$, and $R_j$ are respectively as described above.

The substitution reaction condition comprises a reaction temperature of 0-100°C, and a reaction time of 8-48 hours. In some embodiments, the substitution reaction condition comprises a reaction temperature of 10-40°C, and a reaction time of 8-24 hours. The organic solvent can be pyridine. With respect to the compound as shown by Formula (107), the amount of the organic solvent can be 1-50 L/mol, such as 1-20 L/mol.

The hydroxyl protection agent can be any agent that can protect the hydroxyl group, and some hydroxyl protection agents are well-known to those skilled in the art. In some embodiments, the two hydroxyl groups linked to $R_j$ have the same chemical environment. In this case, the degree of reaction is controlled by controlling the molar ratio of the hydroxyl protection agent to the compound as shown by Formula (107), so that the main reaction product is a product in which only one hydroxyl group is protected; in some embodiments, only one of the two hydroxyl groups linked to $R_j$ is a hydroxyl group of primary alcohol. In this case, by selecting the hydroxyl protection agent, the main reaction product is a product in which only one hydroxyl group is protected. In some embodiments, the hydroxyl protection agent is one of trityl chloride, 4-methoxytrityl chloride, 4,4'-dimethoxytrityl chloride, and 4,4',4"-trimethoxytrityl chloride. In some embodiments, the hydroxyl protection agent is 4,4'-dimethoxytrityl chloride (DMTrCl). The molar ratio of the hydroxyl protection agent to the compound as shown by Formula (107) can be 1:1 - 50:1, and in some embodiments, 1.2:1 - 2:1. Similarly, the compound of Formula (106) can be isolated from the reaction mixture by any suitable isolation method. In some embodiments, the compound as shown by Formula (106) can be isolated by removal of solvent via evaporation followed by chromatography, for example, using the following chromatographic conditions for isolation:

normal phase purification of silica gel: 200-300 mesh silica gel filler, gradient elution of a mixed solution of petroleum ether: ethyl acetate = 1:1;

reverse phase purification: C18 and C8 reverse phase filler, gradient elution of methanol:acetonitrile = 0.1:1 - 1:0.1.

In some embodiments, the solvent can be directly removed to obtain a crude product of the compound as shown by Formula (106), which can be directly used in subsequent reactions.

In some embodiments, the compound as shown by Formula (107) can be prepared by the following method, comprising:

contacting a compound as shown by Formula (108) with an aminodiol selected from one of the compounds of Formulae (B62)-(B67) and an amidation activator in an organic solvent under amidation reaction condition, and isolating the compound as shown by Formula (107),

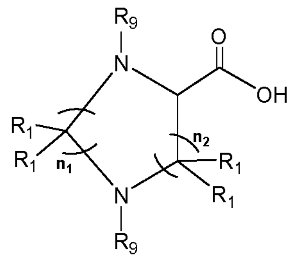

Formula (108)

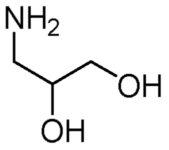, 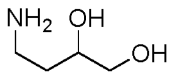, 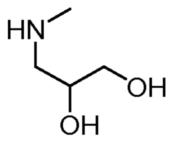, 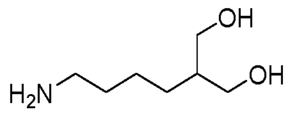,

Formula (B62) Formula (B63) Formula (B64) Formula (B65)

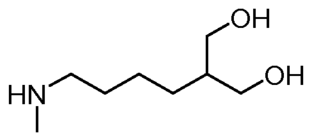, and 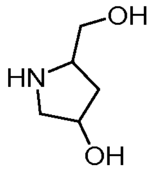

Formula (B66) Formula (B67),

wherein the definitions and options of $n_1$, $n_2$, $R_1$, and $R_9$ are respectively as described above. In this case, $R_j$ in the compound as shown by Formula (107) is selected from one of the groups of Formulae (A62)-(A67).

The aminodiol can be commercially available or synthesized by those skilled in the art via well-known methods. In one specific embodiment, the aminodiol is 3-amino-1,2-propanediol as shown by Formula (B62).

The amidation reaction condition comprises a reaction temperature of 0-100 °C and a reaction time of 8-48 hours. In some embodiments, the amidation reaction condition comprises a reaction temperature of 40-80 °C and a reaction time of 10-30 hours.

The organic solvent can be an amide solvent, alcohol solvent or ether solvent. In some embodiments, the amide solvent is, for example, dimethylformamide; and the alcohol solvent is methanol and/or ethanol. With respect to the compound as shown by Formula (108), the amount of the organic solvent can be 1-50 L/mol, such as 1-20 L/mol.

The molar ratio of one of the compounds as shown by Formula (B62)-(B67) to the compound as shown by Formula (108) can be 0.1:1 - 20:1, such as 0.5:1 - 5:1.

In some embodiments, the amidation activator can be 1-(3-dimethylaminopropyl)-3-ethylcarbodiimide hydrochloride, 2-ethoxy-1-ethoxycarbonyl -1,2-dihydroquinoline or 4-(4,6-dimethoxytriazin-2-yl)-4-methylmorpholine hydrochloride, such as, 1-(3-dimethylaminopropyl)-3-ethylcarbodiimide hydrochloride (EDCI) or 2-ethoxy-1-ethoxycarbonyl-1,2-dihydroquinoline (EEDQ). The molar ratio of the amidation activator to the compound as shown by Formula (108) can be 0.1:1 - 20:1, such as 0.5:1 - 5:1.

Similarly, the compound of Formula (107) can be isolated from the reaction mixture by any suitable isolation method. In some embodiments, the compound as shown by Formula (107) can be isolated by removal of solvent via evaporation followed by chromatography, for example, using the following chromatographic conditions for isolation:

normal phase purification of silica gel: 200-300 mesh silica gel filler, gradient elution of petroleum ether: ethyl acetate: dichloromethane: methanol=1:1:1:0.05 - 1:1:1:0.15;

reverse phase purification: C18 and C8 reverse phase filler, gradient elution of methanol:acetonitrile = 0.1:1 - 1:0.1.

In some embodiments, the solvent can be directly removed to obtain a crude product of the compound as shown by Formula (107), which can be directly used in subsequent reactions.

In some embodiments, the compound as shown by Formula (108) can be prepared by the following method, comprising:

contacting a compound as shown by Formula (109) with an amino protection agent in an organic solvent under substitution reaction condition, and isolating the compound as shown by Formula (108),

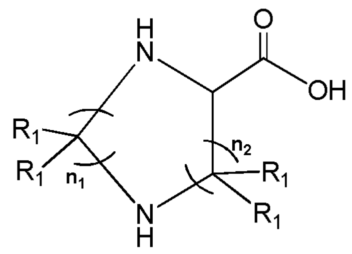

Formula (109)

wherein the definitions and options of $n_1$, $n_2$ and $R_1$ are respectively as described above.

The substitution reaction condition comprises a reaction temperature of 0-100°C, and a reaction time of 4-48 hours. In some embodiments, the substitution reaction condition comprises a reaction temperature of 10-40°C, and a reaction time of 8-30 hours.

The amino protection agent can be 9-fluorenylmethyl chloroformate, trifluoroacetyl chloride or trichloroacetyl chloride. In some embodiments, the amino protection agent is 9-fluorenylmethyl chloroformate (Fmoc-Cl). The molar ratio of the amino protection agent to the compound as shown by Formula (109) can be 0.1:1 - 20:1, such as 1:1 - 10:1.

The organic solvent can be one or more of an epoxy solvent, an ether solvent, an haloalkane solvent, dimethyl sulfoxide, N,N-dimethylformamide, and N,N-diisopropylethylamine. The epoxy solvent can be, for example, dioxane and/or tetrahydrofuran; the ether solvent can be, for example, diethyl ether and/or methyl tertbutyl ether; and the haloalkane solvent can be, for example, one or more of dichloromethane, trichloromethane, and 1,2-dichloroethane. In some embodiments, the organic solvent is dioxane. In some embodiments, the organic solvent is a mixture of water and dioxane. In some embodiments, the volume ratio of water and dioxane can be 5:1 - 1:5, and in some embodiments, 3:1 - 1:3. With respect to the compound as shown by Formula (109), the total amount of the organic solvent can be 0.1-50 L/mol, such as 0.5-10 L/mol.

Similarly, the compound as shown by Formula (108) can be isolated from the reaction mixture by any suitable isolation methods. In some embodiments, the compound as shown by Formula (108) can be isolated by removal of solvent via evaporation followed by chromatography, for example, using the following chromatographic conditions for isolation:

normal phase purification of silica gel: 200-300 mesh silica gel filler, gradient elution of a mixed solution of petroleum ether: ethyl acetate: dichloromethane=1:1:1;
reverse phase purification: C18 and C8 reverse phase filler, gradient elution of methanol:acetonitrile = 0.1:1 - 1:0.1.

In some embodiments, the solvent can be directly removed to obtain a crude product of the compound as shown by Formula (108), which can be directly used in subsequent reactions.

In some embodiments, the compound as shown by Formula (109) can be prepared by various methods, or commercially available. In some embodiments, all $R_1$ are hydrogen; $n_1$ is 2, and $n_2$ is 1. In this case, the compound as shown by Formula (109) is piperazine-2-carboxylic acid dihydrochloride, which is readily commercially available.

## Second compound

In some embodiments, the present disclosure provides a compound having a structure as shown by Formula (111) and the preparation method thereof:

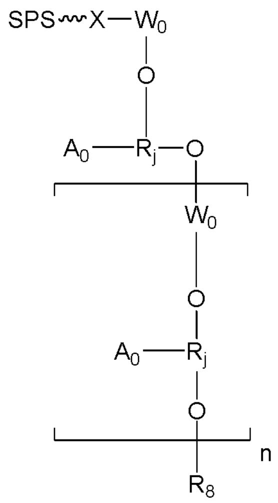

Formula (111)

wherein the definitions and options of each $A_0$, each $R_j$ and $R_8$ are respectively as described above;
Wo is a linking group;
X is selected from O or NH;
SPS represents a solid phase support; and
n is an integer of 0-7.

Wo is used to provide covalent linkages between the compound as shown by Formula (111) and the solid phase support or among multiple $R_j$ groups. Wo can be any linking structure. In some embodiments, Wo has a structure as shown by Formula (C1') or (A81):

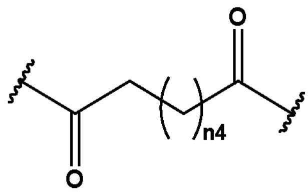

,

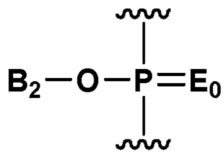

Formula (C1') Formula (A81)

wherein n4 is an integer of 1-4;
each Eo is independently O, S or BH;
each $B_2$ is independently selected from $C_1$-$C_5$ alkyl, ethylcyano, propcyano and butyrcyano;

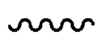 represents the site where the group is linked.

The solid phase support SPS in the compound as shown by Formula (111) can be a well-known solid support in the art for solid phase synthesis of nucleic acids, such as a solid phase support moiety obtained by replacing DMTr in the commercially available universal solid phase support (NittoPhase®HL UnyLinker™ 300 Oligonucleotide Synthesis Support, Kinovate Life Sciences, as shown by Formula B80) with the Wo group:

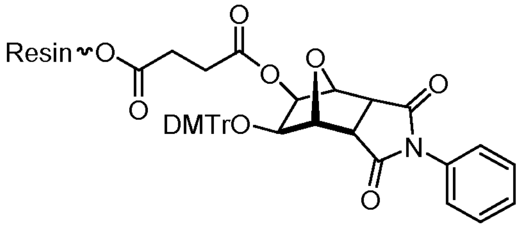

(B80).

In some embodiments, Wo is a linking group obtained by subjecting the phosphoramidite linkage formed by reacting $R_7$ in the compound of Formula (101) with the hydroxyl group on the solid phase support or with other hydroxyl group produced by deprotection of the compound of Formula (101) to oxidation, sulfurization or hydroboration reaction. Thus, the options of $B_2$ are the same as that of the corresponding group in Formula (101), while Eo can be O, S or BH. In the subsequent reaction, $B_2$ group can be hydrolyzed and removed to form a hydroxyl group, then the resultant hydroxyl group and Eo could form a phosphoryloxy group and $E_1$ in Formulae (A60) and (A61) via configurational interconversion, and the corresponding $E_1$ is OH, SH or $BH_2$, respectively. Comprehensive consideration of cost and the need for simplifying the reaction, in some embodiments, $B_2$ is cyanoethyl and Eo is O.

In some embodiments, Wo is a linking group obtained by reacting $R_7$ in the compound of Formula (101) with the hydroxyl or amino group on the solid phase support, or with other hydroxyl or amino group produced by deprotection of the compound of Formula (101) to form an ester or amide bond.

According to the present disclosure, n can be an integer of 0-7 to ensure that the number of $S_1$ groups in the compound as shown by Formula (111) is at least 2. In some embodiments, the $M_1$ ligand is independently selected from one of the ligands that have affinity to the asialoglycoprotein receptors on the surface of mammalian hepatocytes, and n≥1, such that the number of the $M_1$ ligands in the drug conjugate is at least 4, thereby rendering the $M_1$ ligands to more easily bind to the asialoglycoprotein receptors on the surface of hepatocytes, which can futher facilitate the endocytosis of the drug conjugate into cells. Experiments have shown that when the number of the $M_1$ ligand is greater than 4, the ease of the binding between the $M_1$ ligand and the asialoglycoprotein receptor on the surface of hepatocytes is not significantly increased. Therefore, in view of various aspects such as synthesis convenience, structure/process costs and delivery efficiency, in some embodiments, n is an integer of 1-4. In some embodiments, n is an integer of 1-2.

In some embodiments, the $M_1$ ligand is a ligand formed by folate or derivatives thereof, and n=0.

As described below, when the active drug is a functional oligonucleotide, the compound as shown by Formula (111) can be conjugated to a nucleotide sequence by using the compound as shown by Formula (111) as the starting compound in place of the solid phase support used in the conventional phosphoramidite nucleic acid solid phase synthesis method and sequentially linking nucleoside monomers according to the phosphoramidite solid phase synthesis method. After the linking step is completed, the compound as shown by Formula (101) conjugated to the nucleotide sequence can be cleaved from the solid phase support, and then subjected to the steps including isolation and purification, and an optional annealing step depending on the structure of the functional oligonucleotide of interest, thereby finally obtaining the drug conjugate of the present disclosure.

According to some specific embodiments of the present disclosure, the compound as shown by Formula (111) has the structure as shown by Formula (503), (504), (505), (506), (507), (508), (509), or (510):

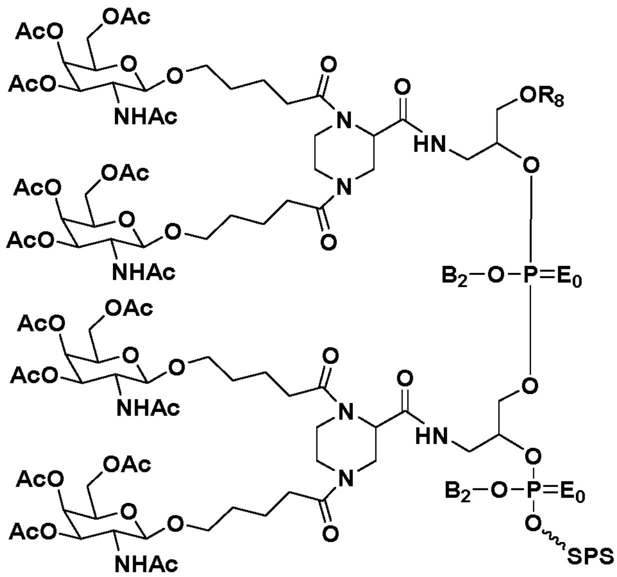

Formula (503),

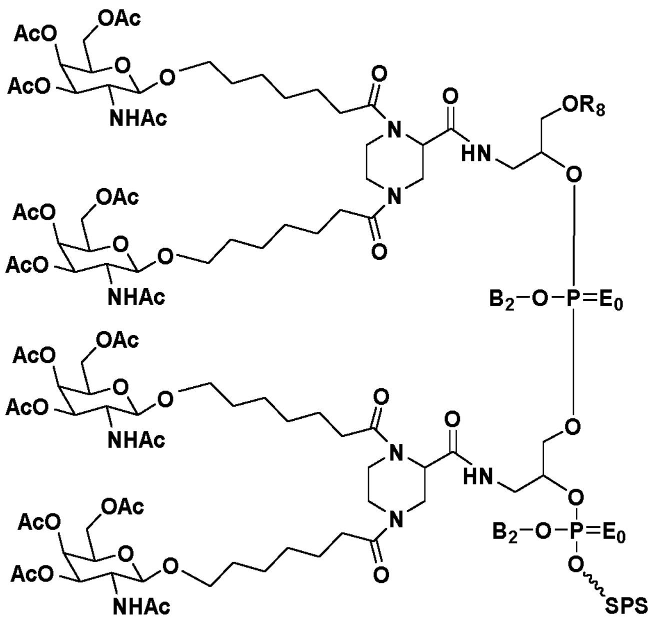

Formula (504),

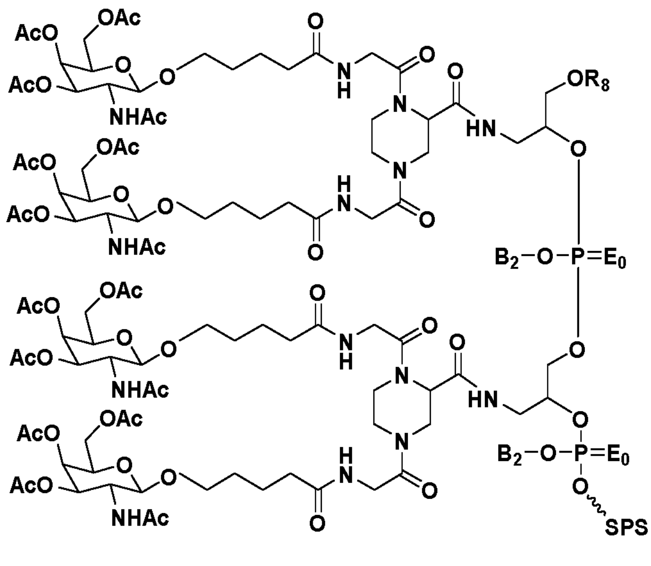

Formula (505),

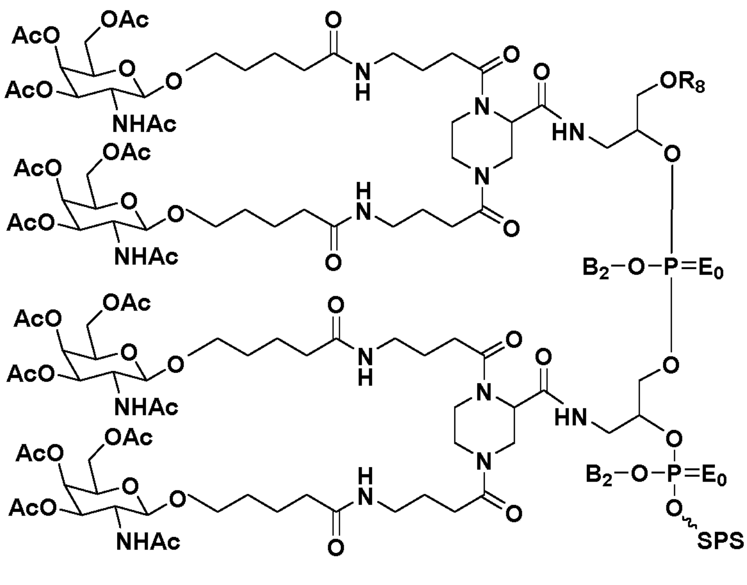

Formula (506),

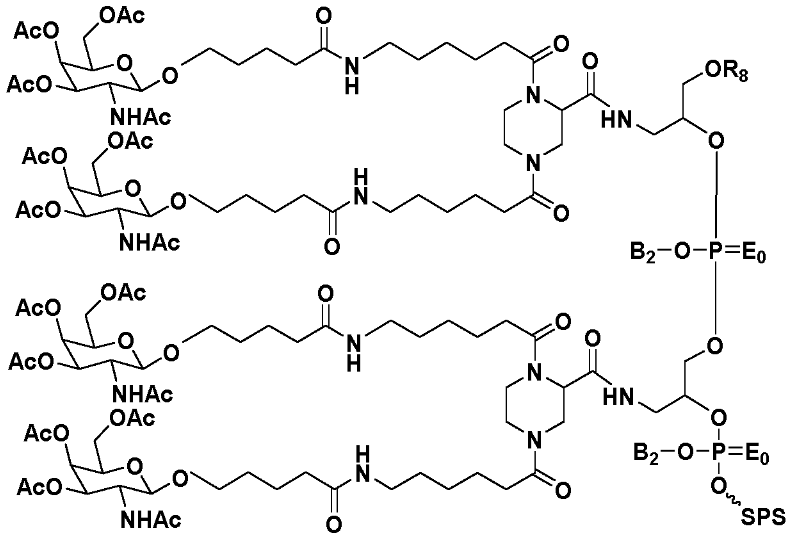

Formula (507),

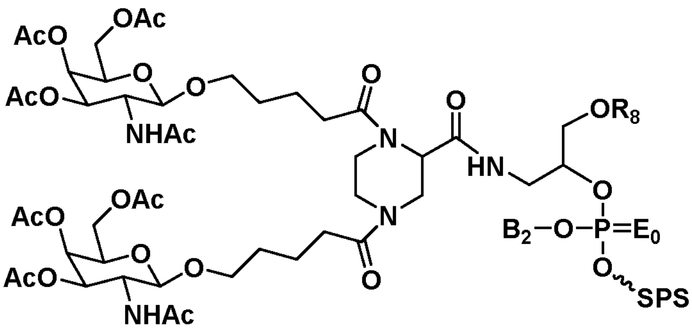

Formula (508),

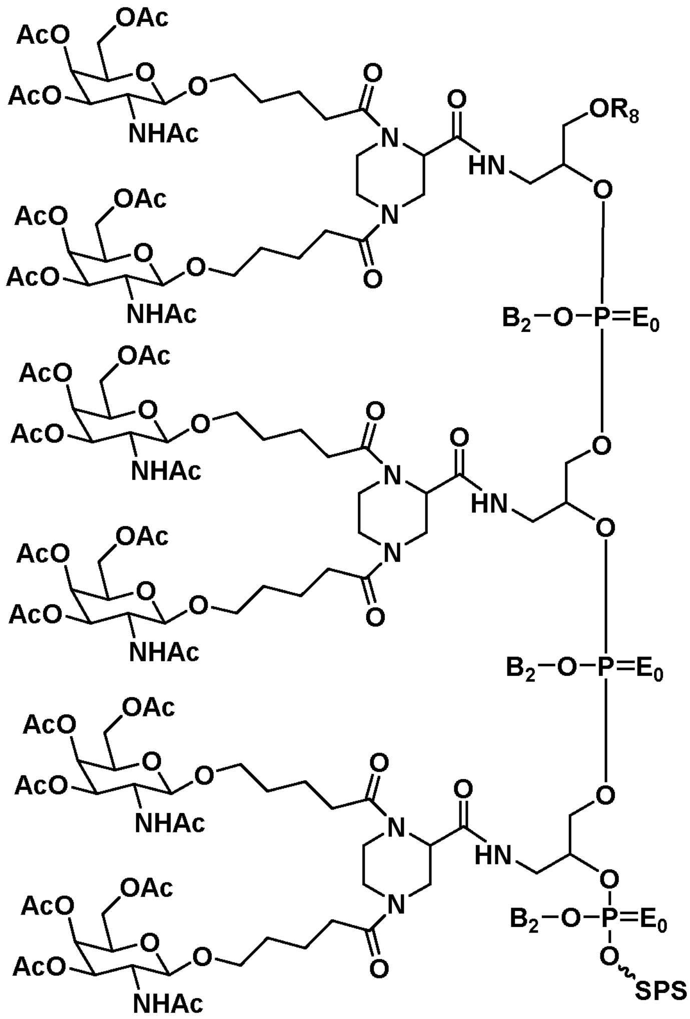

Formula (509),

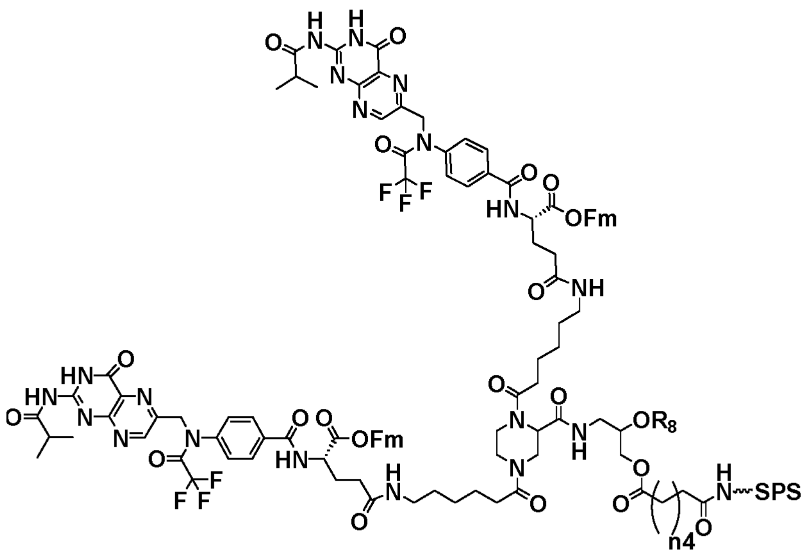

Formula (510),

wherein n4 is an integer of 1-4. In some embodiments, $R_8$ in the above compounds of Formulae (503)-(510) is a hydroxyl

protecting group which is one of trityl, 4-methoxytrityl, 4,4'-dimethoxytrityl and 4,4',4"-trimethoxytrityl, each $B_2$ is ethylcyano, and each Eo is O.

In some embodiments, X is O, Wo and X form a phosphate ester linkage, a phosphorothioate linkage, or a phosphoroborate linkage, and the compound as shown by Formula (111) can be prepared by a method comprising: (1a) removing the protecting group from a solid phase support with a protected hydroxyl group; contacting a compound as shown by Formula (101) with the solid phase support under coupling reaction condition in the presence of a coupling agent; and performing capping reaction, and then oxidation, sulfurization, or hydroboration reaction.

The method of preparing the compound as shown by Formula (111) can further comprise: (IIa) further contacting with the compound as shown by Formula (101) according to the method of (Ia) for n times (the definition of n is the same as that of Formula (111)); deprotecting the product obtained in the previous step each time; and performing capping reaction, and then oxidation, sulfurization, or hydroboration reaction.

The deprotection, coupling, capping, oxidation, sulfurization, or hydroboration reactions can be performed with the same conditions and agents as those of conventional phosphoramidite solid phase synthesis methods; and some typical reaction conditions and agents will be described below in detail.

In some embodiments, X is O or N, Wo and X together form a carboxylate linkage or an amide linkage. The compound as shown by Formula (111) can be prepared by a method comprising: (Ib) removing the protecting group from a solid phase support with a protected hydroxyl or amino group; contacting a compound as shown by Formula (101) with the solid phase support in an organic solvent under condensation reaction condition in the presence of a condensation agent; isolating and obtaining the compound as shown by Formula (111) comprising a carboxylate or amide bond.

In some embodiments, each $S_1$ in the groups as shown by Formula $A_0$ is selected from one of the groups as shown by Formula A71-A75 independently. Each $S_1$ is linked to the $L_1$ group via a carboxylate bond or an amide bond. The compound as shown by Formula (111) can be prepared by a method comprising: (Ic) contacting a compound as shown by Formula (121) with the compound as shown by Formula (401) in an organic solvent, under condensation reaction condition in the presence of an amine hydrochloride, a condensation agent and a heterocyclic organic base; isolating and obtaining the compound as shown by Formula (111).

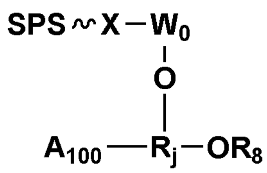

Formula (121),

$S_1$-$X_{401}$

Formula (401),

wherein $A_{100}$ has a structure as shown by Formula (402):

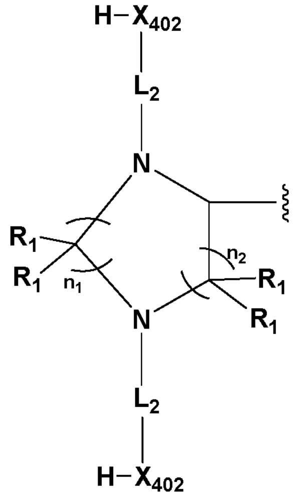

Formula (402)

wherein $X_{401}$ is hydroxyl, amino, halogen, or $O^-M^+$, wherein $M^+$ is a cation; $X_{402}$ is O or NH, $L_2$ and $X_{402}$ group together form an $L_1$ linkage group; i.e., $L_2$ is the part of $L_1$ with $X_{402}$ group removed.

The definitions and options of SPS, X, Wo, $R_j$, $n_1$, $n_2$, $R_8$, and each $R_1$ are respectively the same as above.

The ratio (molar ratio) of the compound as shown by Formula (401) to the compound as shown by Formula (121) can be 1:1 - 5:1, for example, 2:1 - 3:1. The organic solvent is one or more of haloalkane solvents or organonitrile compounds. The haloalkane solvent can be, for example, dichloromethane, trichloromethane, or 1,2-dichloroethane. The organonitrile compound can be, for example, acetonitrile. In some embodiments, the organic solvent is dichloromethane. The amount of the organic solvent can be 3-100 L/mol, such as 5-80 L/mol, with respect to the compound as shown by Formula (121).

The amine hydrochloride can be, for example, 1-ethyl-(3-dimethylamino propyl)carbodiimide hydrochloride (EDCI). The ratio (molar ratio) of the amine hydrochloride to the compound as shown by Formula (121) can be 1:1 - 5:1, such as 2:1 - 4:1.

The condensation agent can be, for example, 1-hydroxy benzotriazole (HOBt), 4-dimethylamino pyridine, dicyclohexyl carbodiimide; in some embodiments, the condensation agent is 1-Hydroxybenzotriazole (HOBt). The ratio (molar ratio) of the condensation agent to the compound as shown by Formula (121) can be 3:1 - 10:1, such as 4:1 - 7:1.

The heterocyclic organic base can be, for example, N-methyl morpholine. The ratio (molar ratio) of the heterocyclic organic base to the compound as shown by Formula (121) can be 1:1 - 5:1, for example, 2:1 - 4:1.

The condensation reaction condition comprises a reaction temperature of 0-100°C and a reaction time of 10-30 hours. In some embodiments, the condensation reaction condition comprises a reaction temperature of 10-40°C and a reaction time of 15-20 hours.

The compound as shown by Formula (111) can be isolated from the reaction mixture by any suitable isolation methods. In some embodiments, the solvent can be removed by suction filtration to obtain a crude product of the compound as shown by Formula (111), which can be directly used in subsequent reactions.

In some embodiments, the compound as shown by Formula (401) can be commercially available by those skilled in the art or readily prepared via known methods. For example, in some embodiments, $S_1$ is a group shown by Formula (A71), $X_{401}$ is hydroxyl. In this case, the compound as shown by Formula (401) can be prepared by the preparation method of compound 152 in Example 2 of the description of WO2009082607.

In some embodiments, the compound as shown by Formula (121) can be commercially available or prepared by those skilled in the art via well-known methods. In some embodiments, the compound as shown by Formula (121) can be prepared by the following method comprising: subjecting the compound as shown by Formula (122) to deprotection reaction in an organic solvent, under deprotection reaction condition in the presence of an heterocyclic organic base, and isolating the compound as shown by Formular (121):

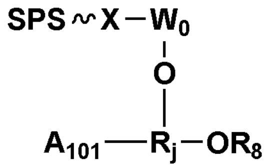

Formula (122)

wherein $A_{101}$ has a structure as shown by Formula (403):

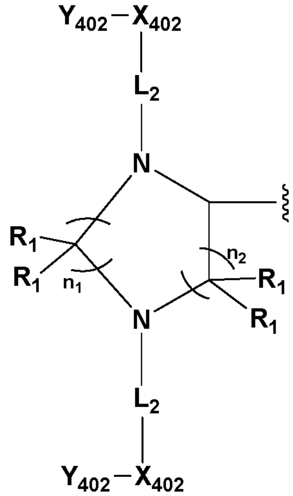

Formula (403)

wherein $Y_{402}$ is a protecting group, and in some embodiments, $X_{402}$ is an amino group, and $Y_{402}$ is an amino protecting group. In some embodiments, $Y_{402}$ is one of benzyloxycarbonyl (Cbz), tert-butoxycarbonyl (Boc), fluorenylmethoxycarbonyl (Fmoc), allyloxycarbonyl (Alloc), trimethylsilylethoxycarbonyl (Teoc), and benzyl (Bn). In some embodiments, $Y_{402}$ is Fmoc protection group.

The definitions and options of $X_{402}$, $L_2$, SPS, X, Wo, $R_j$, $R_8$, $n_1$, $n_2$, and each $R_1$ are respectively the same as above.

The organic solvent can be a haloalkane solvent. For example, the haloalkane solvent can be dichloromethane, trichloromethane, and 1,2-dichloroethane. In some embodiments, the organic solvent is dichloromethane. The amount of the organic solvent is 10-80 L/mol, such as 20-40 L/mol, with respect to the compound as shown by Formula (122).

The heterocyclic organic base can be, for example, pyridine or piperidine. In some embodiments, the heterocyclic organic base can be piperidine. The amount of the heterocyclic organic base is 2-20 L/mol, such as 5-10 L/mol, with respect to the compound as shown by Formula (122).

The deprotection reaction condition comprises a reaction temperature of 0-100°C and a reaction time of 10-20 hours. In some embodiments, the deprotection reaction condition comprises a reaction temperature of 10-40°C and a reaction time of 3-10 hours.

The compound as shown by Formula (121) can be isolated from the reaction mixture by any suitable isolation methods. In some embodiments, the solvent can be removed by suction filtration to obtain a crude product of the compound as shown by Formula (121), which can be directly used in subsequent reactions.

In some embodiments, the compound as shown by Formula (122) can be prepared by the following method comprising: contacting a compound as shown by Formula (123) with the solid phase support with a hydroxyl or amino group, in an organic solvent under condensation reaction condition and in the presence of a condensation agent and a tertiary amine, and isolating the compound as shown by Formula (122):

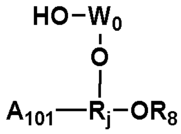

Formula (123)

wherein the definitions and options of $A_{101}$, $R_j$, $R_8$, and $W_0$ are respectively as described above.

The solid phase support can be one of the supports used in the solid phase synthesis of siRNA, which are well-known to those skilled in the art. For example, the solid phase support can be selected from the solid phase supports comprising an active hydroxy or amino functional group. In some embodiments, the solid phase support is an amino resin or hydroxy resin. In some embodiments, the amino or hydroxy resin has the following parameters: particle size of 100-400 mesh and surface amino or hydroxy loading of 0.2- 0.5 mmol/g. The ratio of the compound as shown by Formula (123) to the solid phase support is 10 - 800 μmol compound per gram of the solid phase support (μmol/g). In some embodiments, the ratio of the compound as shown by Formula (321) to the solid phase support is 100 μmol/g to 600 μmol/g.

The organic solvent can be any suitable solvent known to those skilled in the art. In some embodiments, the organic solvent is one or more of haloalkane solvents or organonitrile compounds. For example, the haloalkane solvent can be dichloromethane, trichloromethane, and 1,2-dichloroethane. The organonitrile compound can be acetonitrile. In some embodiments, the organic solvent is acetonitrile. The amount of the organic solvent is 3-50 L/mol, such as 5-30 L/mol, with respect to the compound as shown by Formula (123).

In some embodiments, the condensation agent can be, for example, benzotriazol-1-yl-oxytripyrrolidino phosphonium hexafluorophosphate (PyBop), 3-diethoxyphosphoryl-1,2,3-benzotrizin-4(3H)-one (DEPBT) and/or O-benzotriazol-1-yl-tetramethyluronium hexafluorophosphate (HBTU). In some embodiments, the condensation agent is O-benzotriazol-1-yl-tetramethyluronium hexafluorophosphate. The ratio (molar ratio) of the condensation agent to the compound as shown by Formula (123) can be 1:1 - 20:1, such as 1:1 - 5:1.

The tertiary amine can be, for example, triethylamine and/or N,N-diisopropylethylamine (DIEA), and in some embodiments, N,N-diisopropylethylamine. The ratio (molar ratio) of the tertiary amine to the compound of Formula (123) can be 1:1 - 20:1, such as 1:1 - 5:1.

The condensation reaction condition comprises a reaction temperature of 0-100°C and a reaction time of 10-30 hours. In some embodiments, the condensation reaction condition comprises a reaction temperature of 10-40°C and a reaction time of 15-30 hours.

The compound of Formula (122) can be isolated from the reaction mixture by any suitable isolation methods. In some embodiments, the solvent can be removed by suction filtration to obtain the crude product of the compound of Formula (122), which can be directly used in subsequent reactions.

In some embodiments, the method for preparing the compound of Formula (122) further comprises: contacting the resultant condensation product with a capping agent and an acylation catalyst in an organic solvent under capping reaction condition, and isolating the compound as shown by Formula (122). The capping reaction is used to remove any active functional group that does not completely react, so as to avoid producing unnecessary by-products in subsequent reactions. The capping reaction condition comprises a reaction temperature of 0-50°C (in some embodiments, 15-35°C), and a reaction time of 1-10 hours (in some embodiments, 3-6 hours). The capping agent can be a capping agent used in the solid phase synthesis of siRNA, which is well-known to those skilled in the art.

In some embodiments, the capping agent is composed of a capping agent A (cap A) and a capping agent B (cap B). The cap A is N-methylimidazole, and in some embodiments, provided as a mixed solution of N-methylimidazole in pyridine/acetonitrile, wherein the volume ratio of pyridine to acetonitrile is 1:10 - 1:1, and in some embodiments, 1:3 - 1:1. In some embodiments, the ratio of the total volume of pyridine and acetonitrile to the volume of N-methylimidazole is 1:1 - 10:1, and in some embodiments, 3:1 - 7:1. The capping agent B is acetic anhydride. The cap B is acetic anhydride, and in some embodiments, provided as a solution of acetic anhydride in acetonitrile, wherein the volume ratio of acetic anhydride to acetonitrile is 1:1 - 1:10, and in further embodiments, 1:2 - 1:6.

In some embodiments, the ratio of the volume of the mixed solution of N-methylimidazole in pyridine/acetonitrile to the mass of the compound of Formula (122) is 5 mL/g-50 mL/g, and in some embodiments, 15 mL/g-30 mL/g. The ratio of the volume of the solution of acetic anhydride in acetonitrile to the mass of the compound of Formula (122) is 0.5 mL/g-10 mL/g, and in some embodiments, 1 mL/g-5 mL/g.

In some embodiments, the capping agent comprises equimolar acetic anhydride and N-methylimidazole. In some embodiments, the organic solvent can be one or more of acetonitrile, an epoxy solvent, an ether solvent, an haloalkane solvent, dimethyl sulfoxide, N,N-dimethylformamide, and N,N-diisopropylethylamine. In some embodiments, the organic solvent is acetonitrile. The amount of the organic solvent can be 10-50 L/mol, and in some embodiments,

5-30 L/mol, with respect to the compound as shown by Formula (122).

In some embodiments, the acylation catalyst can be selected from any catalyst that can be used for esterification condensation or amidation condensation, such as alkaline heterocyclic compounds. In some embodiments, the acylation catalyst is 4-dimethylaminopyridine. The mass ratio of the catalyst to the compound as shown by Formula (122) can be 0.001:1 - 1:1, and in some embodiments, 0.01:1 - 0.1:1.

In some embodiments, the compound as shown by Formula (122) can be isolated from the reaction mixture by any suitable isolation methods. In some embodiments, the compound as shown by Formula (122) can be obtained by thoroughly washing with an organic solvent and filtering to remove unreacted reactants, excess capping agents, and other impurities. The organic solvent is selected from one or more of acetonitrile, dichloromethane, and methanol, and in some embodiments, is acetonitrile. In some embodiments, Wo comprises diacyl structures. The compound as shown by Formula (123) can be prepared by the following method comprising: contacting a compound as shown by Formula (125) with a cyclic anhydride in an organic solvent under esterification reaction condition in the presence of a base and an esterification catalyst, and isolating the compound as shown by Formula (123):

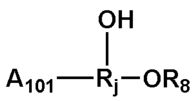

Formula (125),

wherein the definitions and options of $A_{101}$, $R_j$ and $R_8$ are respectively as described above.

In some embodiments, the organic solvent comprises one or more of an epoxy solvent, an ether solvent, a haloalkane solvent, dimethyl sulfoxide, N,N-dimethylformamide, and N,N-diisopropylethylamine. In some embodiments, the epoxy solvent can be dioxane and/or tetrahydrofuran. The ether solvent can be diethyl ether and/or methyl tertbutyl ether. The haloalkane solvent can be one or more of dichloromethane, trichloromethane, and 1,2-dichloroethane. In some embodiments, the organic solvent is dichloromethane. The amount of the organic solvent can be 3-50 L/mol, such as 5-20 L/mol, with respect to the compound as shown by Formula (125).

In some embodiments, the cyclic anhydride can be one of succinic anhydride, glutaric anhydride, adipic anhydride or pimelic anhydride, and in some embodiments, succinic anhydride. The ratio (molar ratio) of the acid anhydride compound the compound of Formula (125) can be 1:1 - 10:1, such as 2:1 - 5:1.

The esterification catalyst can be any catalyst capable of catalyzing esterification. For example, the esterification catalyst can be, such as, 1-hydroxybenzotriazole (HOBt), 4-dimethylaminopyridine, or dicyclohexyl carbodiimide. In some embodiments, the esterification catalyst is 4-dimethylaminopyridine. The ratio (molar ratio) of the esterification catalyst to the compound as shown by Formula (125) can be 1:1 - 10:1, such as 2:1 - 5:1.

In some embodiments, the base can be any inorganic base, organic base, or combination thereof. Considering solubility and product stability, the base can be, for example, a tertiary amine. In some embodiments, the tertiary amine is triethylamine or N,N-diisopropylethylamine. The molar ratio of the tertiary amine to the compound as shown by Formula (125) is 1:1 - 20:1, such as 3:1 - 10:1.

The esterification reaction condition comprises a reaction temperature of 0-100°C and a reaction time of 8-48 hours. In some embodiments, the condensation reaction condition comprises a reaction temperature of 10-40°C and a reaction time of 20-30 hours. After the above reaction is completed, the resultant compound as shown by Formula (123) can also be subjected to optional ion exchange reaction as desired. The ion exchange serves the function of converting the compound as shown by Formula (123) into a desired form of carboxylic acid or carboxylic salt, and the methods of ion exchange are well-known to those skilled in the art. The compound as shown by Formula (101), in which the cation is $M^+$, can be obtained by using a suitable ion exchange solution and ion exchange condition, which is not described here in detail. In some embodiments, the ion exchange reaction is performed using a triethylamine phosphate solution and the concentration of the triethylamine phosphate solution can be 0.2-0.8 M. In some embodiments, the concentration of the triethylamine phosphate solution can be 0.4-0.6 M. With respect to the compound as shown by Formula (123), the amount of the triethylamine phosphate solution can be 3-6 L/mol, and in further embodiments, 4-5 L/mol.

The compound as shown by Formula (123) can be isolated from the reaction mixture by any suitable isolation methods. In some embodiments, the compound as shown by Formula (123) can be isolated by removal of solvent via evaporation followed by chromatography, for example, using the following chromatographic conditions for isolation:

normal phase purification of silica gel: 200-300 mesh silica gel filler, wherein the column was first equilibrated with 0.1%(v/v) triethylamine in petroleum ether, and then eluted with a gradient elution of dichloromethane: methanol = 20:1-10:1;

reverse phase purification: C18 and C8 reverse-phase filler, gradient elution of methanol: acetonitrile = 0.1: 1-1:0.1.

In some embodiments, the solvent is directly removed to obtain a crude product of the compound of Formula (123), which can be directly used in subsequent reactions.

In some embodiments, the compound as shown by Formula (125) can be prepared by the following method comprising: contacting a compound as shown by Formula (126) with a hydroxyl protection group in an organic solvent, under hydroxyl protection reaction condition in the presence of a condensation agent, and isolating the compound as shown by Formula (125):

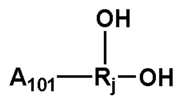

Formula (126)

wherein the definitions and options of Aioi and $R_j$ are respectively as described above.

The condensation agent can be, for example, 1-hydroxybenzotriazole (HOBt), 4-dimethylaminopyridine, and/or dicyclohexyl carbodiimide. In some embodiments, the condensation agent is 4-dimethylaminopyridine. The ratio of a condensation agent to the compound as shown by Formula (126) can be 0.01:1 - 1:1, such as 0.1:1 - 0.5:1.

The organic solvent can be an organic bases solvent. In some embodiments, the organic base solvent can be pyridine. The amount of the organic solvent can be 2-20 L/mol, such as 3-10 L/mol, with respect to the compound as shown by Formula (126).

Similarly, the hydroxyl protection agent can be various hydroxyl protection agents known to those skilled in the art. In some embodiments, the hydroxyl protection agent can be one of trityl chloride, 4-methoxytrityl chloride, 4,4'-dimethoxytrityl chloride, and 4,4',4"-trimethoxytrityl chloride. In some embodiments, the hydroxyl protection agent, for example, can be 4,4'-Dimethoxytrityl chloride (DMTrCl). The ratio (molar ratio) of the hydroxyl protection agent to the compound of Formula (126) can be 1:1 - 1.5:1, such as 1.1:1 - 1.3:1.

The hydroxyl protection reaction condition comprises a reaction temperature of 0-100°C and a reaction time of 5-30 hours. In some embodiments, the hydroxyl protection reaction condition comprises a reaction temperature of 0-40°C and a reaction time of 8-20 hours.

Similarly, the compound as shown by Formula (125) can be isolated from the reaction mixture by any suitable isolation methods. In some embodiments, the compound as shown by Formula (125) can be isolated by removal of solvent via evaporation followed by chromatography, for example, using the following chromatographic conditions for isolation:

normal phase purification of silica gel: 200-300 mesh silica gel filler, wherein the column was first equilibrated with 0.1%(v/v) triethylamine in petroleum ether, and then eluted with a gradient elution of petroleum ether: ethyl acetate: dichloromethane: methanol = 1:1:1:0.1-1: 1: 1:0.3;

reverse phase purification: C18 and C8 reverse-phase fillers, gradient elution of methanol: acetonitrile = 0.1:1 - 1:0.1.

In some embodiments, the solvent can be directly removed to obtain a crude product of the compound as shown by Formula (125), which can be directly used in subsequent reactions.

In some embodiments, the compound as shown by Formula (126) can be prepared by the following method comprising: contacting a compound as shown by Formula (104) with a compound as shown by Formula (127) in an organic solvent, under condensation reaction condition in the presence of an activator and a tertiary amine, followed by isolation:

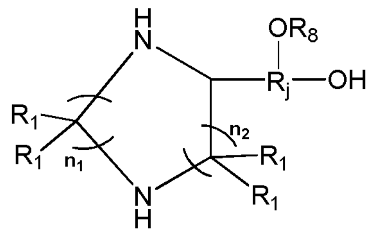

Formula (104)

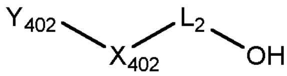

Formula (127)

wherein, $R_j$, $R_8$, $R_1$, $n_1$, $n_2$, $Y_{402}$, $X_{402}$, and $L_2$ are respectively as described above.

The organic solvent can be one or more of an epoxy solvent, an ether solvent, a haloalkane solvent, dimethyl sulfoxide, N,N-dimethylformamide, and N,N-diisopropylethylamine. The epoxy solvent can be dioxane and/or tetrahydrofuran. The ether solvent can be diethyl ether and/or methyl tert-butyl ether. The haloalkane solvent can be one or more of dichloromethane, trichloromethane, and 1,2-dichloroethane. In some embodiments, the organic solvent can be dichloromethane. The amount of the organic solvent can be 3-50 L/mol, such as 5-20 L/mol, with respect to the compound as shown by Formula (104).

In some embodiments, the $L_2$ linking group is linked to the hydroxyl group through the acyl group. In this case, the condensation reaction is a amidation reaction, and the amidation reaction condition include a reaction temperature of 0-100°C and a reaction time of 8-48 hours. In some embodiments, the amidation reaction condition comprises a reaction temperature of 10-40°C and a reaction time of 8-20 hours.

In some embodiments, the activator can be one of 3-(diethoxyphosphoryloxy)-1,2,3-benzotrizin-4(3H)-one (DEPBT), O-benzotriazol-tetramethyluronium hexafluorophosphate, 2-(7-oxybenzotriazol)-N,N,N',N'- tetramethyluronium hexafluorophosphate, and dicyclohexylcarbodiimide, such as 3-(diethoxyphosphoryloxy)-1,2,3-benzotrizin-4(3H)-one (DEPBT). The molar ratio of the activator to the compound as shown by Formula (104) can be 2:1 - 5:1, and in some embodiments is 2.1:1 - 3.5:1.

The tertiary amine can be N-methyl morpholine, triethylamine or N,N-diisopropylethylamine, and in some embodiments, N,N-diisopropylethylamine (DIEA). The molar ratio of the tertiary amine to the compound as shown by Formula (104) can be 2:1 - 10:1, and in some embodiments, 4:1 - 8:1.

The compound as shown by Formula (127) can be readily prepared by those skilled in the art via known methods, or the compound as shown by Formula (127) of a specific structure can be commercially available. For example, when $Y_{402}$ is a Fmoc protection group, $X_{402}$ is NH, and $L_2$ is a hexanoylene group, the compound as shown by Formula (127) can be commercially available 6-(((9H-fluorene-9-yl)methoxy)carbonyl)amino)hexanoic acid (e.g., from Beijing Ouhe Technology Co., Ltd.). The ratio of the compound as shown by Formula (127) to the compound as shown by Formula (104) can be 2:1 - 5:1, and in some embodiments, 2:1 - 3:1. The method for obtaining the compound as shown by Formula (104) is the same as that described above.

Similarly, the compound as shown by Formula (126) can be isolated from the reaction mixture by any suitable isolation methods. In some embodiments, the compound as shown by Formula (126) can be isolated by removal of solvent via evaporation followed by chromatography, for example, using the following chromatographic conditions for isolation:

(1) normal phase purification of silica gel: 200-300 mesh silica gel filler, gradient elution of ethyl acetate : petroleum ether : dichloromethane : methanol = 1:1:1:0.2 - 1:1:1:0.4; and
(2) reverse-phase purification: C18 and C8 reverse-phase fillers, gradient elution of methanol: acetonitrile = 0.1:1 - 1:0.1.

In some embodiments, the solvent can be directly removed to obtain a crude product of the compound as shown by Formula (126), which can be directly used in subsequent reactions.

## Drug Conjugate

In one embodiment, the present disclosure provides a drug conjugate which has the structure as shown by Formula (301):

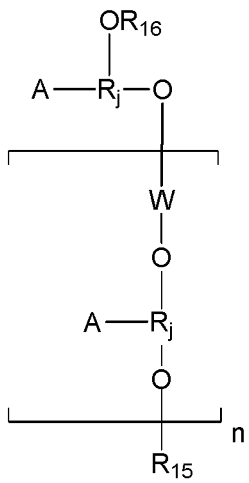

Formula (301),

wherein group A in Formula (301) has a structure as shown by Formula (302):

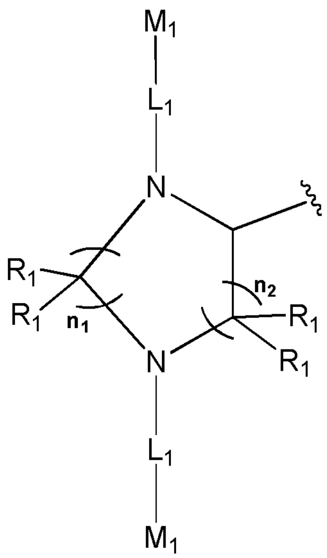

Formula (302)

wherein the definitions and options of $R_j$, $R_1$, $L_1$, $M_1$, n, $n_1$, and $n_2$ are respectively as described above; W is a linking group; $R_{16}$ and $R_{15}$ are respectively H or an active drug group, and at least one of $R_{16}$ and $R_{15}$ is an active drug group.

The "active drug group" is a group formed by an active drug molecule that can be delivered by the compounds disclosed herein. In some embodiments, the active drug is a pharmaceutical agent expected to be delivered to hepatocytes or a pharmaceutical agent expected to be delivered to tumors.

These active drugs or pharmaceutical agents can be small molecule drugs, monoclonal antibody drugs, or nucleic acid drugs. In some embodiments, the active drug is a functional oligonucleotide, especially those disclosed herein, such as siRNA. Although the active drugs of the present disclosure use many functional oligonucleotides, such as siRNA, those of skill in the art could expect that other active drugs, such as small molecule drugs or monoclonal antibody drugs, can also be used as active drug ingredients in the drug conjugates provided by the present disclosure.

The active drug can be a drug for the treatment and/or prevention of various diseases, for example, a drug for the treatment and/or prevention of symptoms or diseases caused by viral infections, such as a drug for the treatment and/or prevention of viral hepatitis (such as hepatitis B or C), a drug for the treatment and/or prevention of Ebola hemorrhagic fever, a drug for the treatment and/or prevention of coronavirus diseases (in particular severe acute respiratory syndrome (SARS) or 2019 coronavirus disease (COVID-19)); a drug for the treatment and/or prevention of metabolic diseases, such as a drug for the treatment and/or prevention of diseases associated with dyslipidemia, a drug

for the treatment and/or prevention of nonalcoholic steatohepatitis, a drug for the treatment and/or prevention of diseases associated with abnormal hormone metabolism, a drug for the treatment and/or prevention of diseases associated with abnormal glycometabolism, a drug for the treatment and/or prevention of diseases associated with abnormal uric acid metabolism, etc.; a drug for the treatment and/or prevention of diseases associated with abnormal uric acid metabolism, etc.; a drug for the treatment and/or prevention of blood diseases, such as a drug for the treatment and/or prevention of diseases associated with abnormal blood coagulation or a drug for the treatment and/or prevention of diseases associated with abnormal blood composition; a drug for the treatment and/or prevention of cancer or tumors, such as a drug for the treatment and/or prevention of epithelial cell carcinoma, a drug for the treatment and/or prevention of solid tumors (sarcoma), a drug for the treatment and/or prevention of leukemia, a drug for the treatment and/or prevention of lymphoma, a drug for the treatment and/or prevention of myeloma, etc.; a drug for the treatment and/or prevention of diseases associated with the central nervous system, such as a drug for the treatment and/or prevention of diseases associated with spinal cord, a drug for the treatment and/or prevention of diseases associated with brain, etc.

In some embodiments, at least one of $R_{16}$ and $R_{15}$ has a structure as shown by Formula (A60):

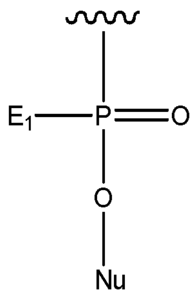

Formula (A60)

wherein $E_1$ is OH, SH or $BH_2$, Nu is a functional oligonucleotide.

In Formula (301), W can be any linking group, as long as it exert the function of linking. In some embodiments, W can be $W_0$, for example, the groups as shown by Formula (C1'). In some embodiments, W can be the product obtained by hydrolysis of Wo, such as the groups as shown by Formula (A61):

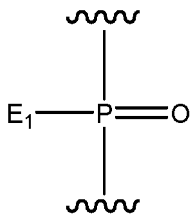

Formula (A61)

wherein $E_1$ is OH, SH or $BH_2$, and considering easy availability of starting materials, OH or SH.

According to some specific embodiments of the present disclosure, the compound as shown by Formula (101) has a structure shown by Formula (303), (304), (305), (306), (307), (308), (310) or (311):

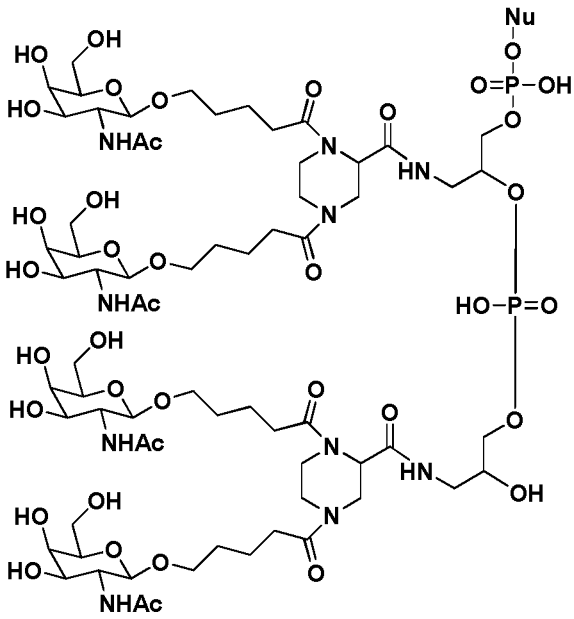

Formula (303),

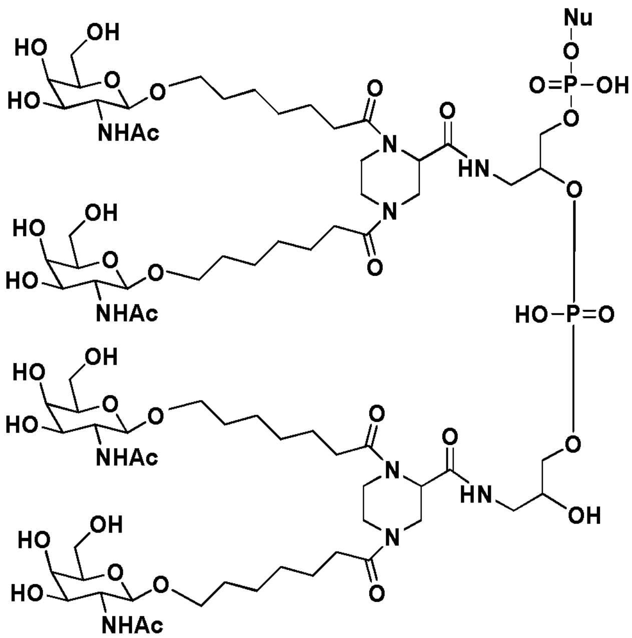

Formula (304),

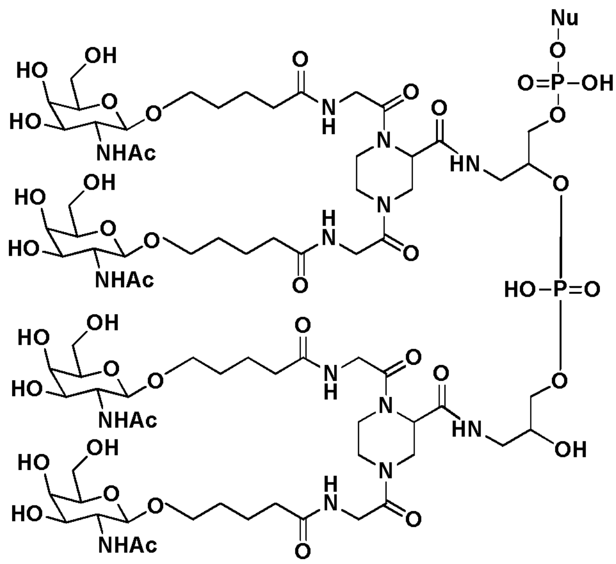

Formula (305),

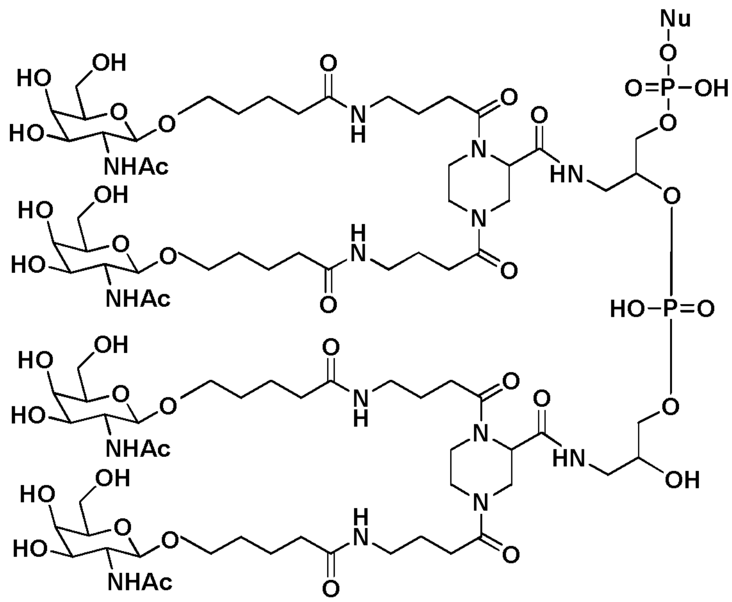

Formula (306),

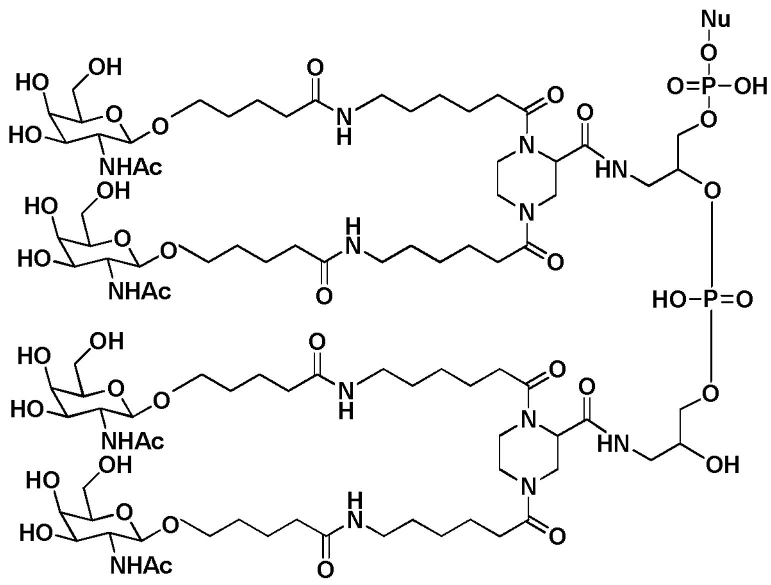

Formula (307),

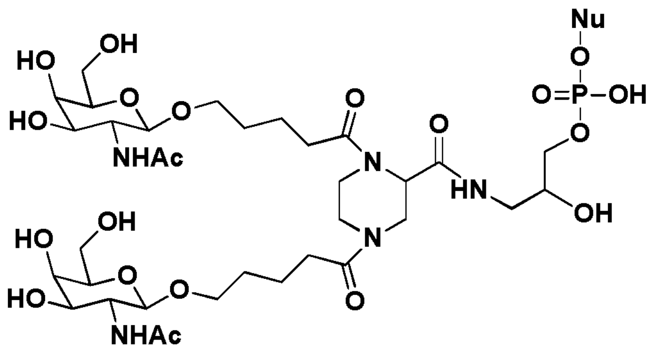

Formula (308),

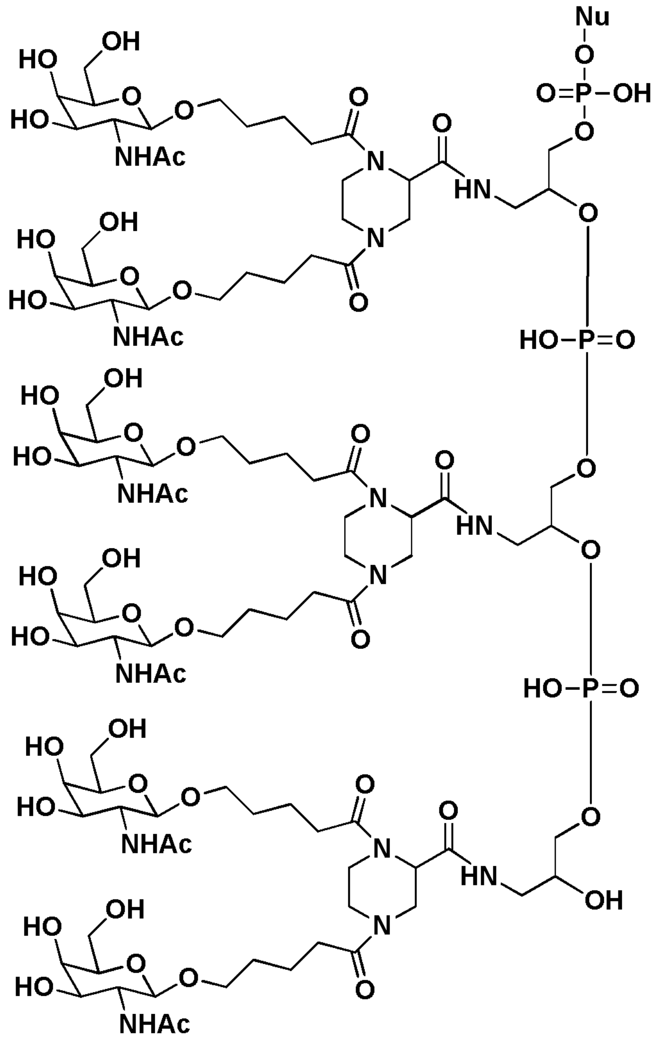

Formula (309),

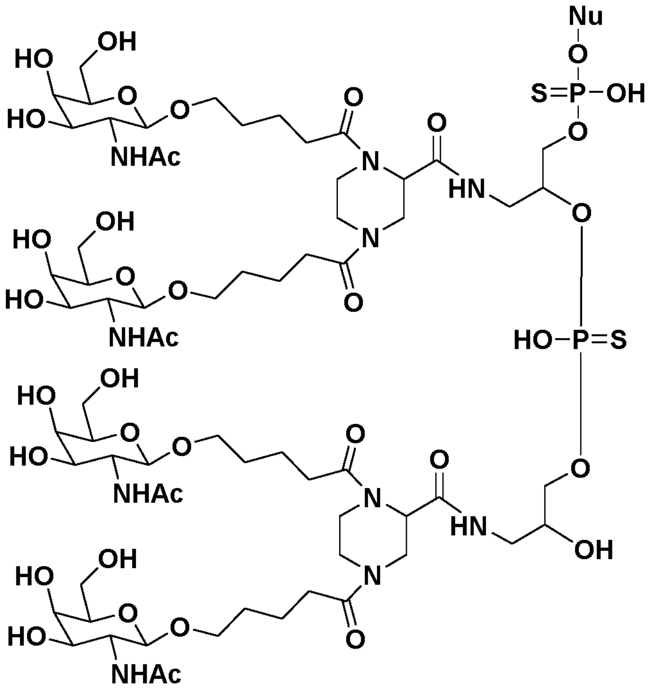

Formula (310),

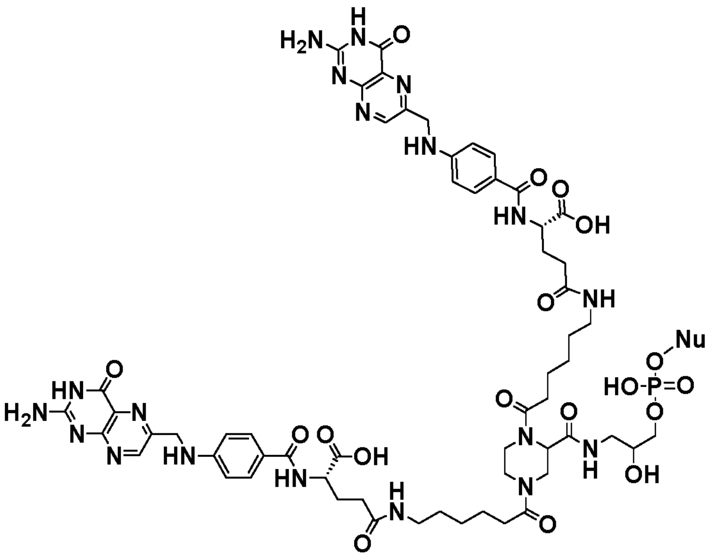

Formula (311).

In some embodiments, the active drug in the drug conjugates of the present disclosure is a unctional oligonucleotide. A functional oligonucleotide is an oligonucleotide capable of stably and specifically hybridizing with a target sequence and up-regulating or down-regulating the expression of the target gene or causing alternative splicing of mRNA by using RNA activation (RNAa), RNA interference (RNAi), antisense nucleic acid technology, exon skipping, etc. In some aspects, a functional oligonucleotide can also be a nucleic acid structure that can stably and specifically bind to a target protein. Furthermore, those of skill in the art could readily appreciate that a polynucleotide (e.g., mRNA itself or fragments thereof) could also be used in the drug conjugate provided by the present disclosure for realizing liver-targeted delivery, thereby regulating the expression of the protein transcribed from the mRNA. Thus, in the context, the concept of "functional oligonucleotide" can also over mRNA or fragments thereof.

In some embodiments, the functional oligonucleotide can interact with the target sequence and affect the normal function of the target sequence molecule, such as causing mRNA breaks, or translation repression, or exon skipping, or triggering alternative splicing of mRNA. For achieving the above interactions, the functional oligonucleotides can be

generally complementary to the bases of the target sequence. In some embodiments, the functional oligonucleotide can be complementary to 89% or more bases of the target sequence, or be complementary to 90% or more bases of the target sequence, or be completely complementary to the target sequence. In some embodiments, the functional oligonucleotide can contain 1, 2, or 3 bases that are not complementary to the target sequence. In some embodiments, the functional oligonucleotide comprises deoxyribonucleotides or ribonucleotides and nucleotides with modifications. In some embodiments, the functional oligonucleotide can be single-stranded DNA, RNA or DNA-RNA chimera, or double-stranded DNA, RNA, or DNA-RNA hybrid.

Thus, suitable functional oligonucleotides can be one of the following: small interfering RNA (siRNA), microRNA, anti-microRNA (antimiR), microRNA antagonist (antagomir), microRNA mimics, decoy oligonucleotide (decoy), immunologic stimulant (immune-stimulatory), G-quadruplex, alternative spliceosome (splice altering), single-stranded RNA (ssRNA), antisense nucleic acid (antisense), Nucleic Acid Aptamer, small activating RNA (saRNA), stem-loop RNA, or DNA. In further embodiments, a suitable functional oligonucleotide can be an oligonucleotide disclosed in WO2009082607A2, WO2009073809A2, or WO2015006740A2, which is incorporated herein by reference in its entirety.

In some embodiments, the active drug is a functional oligonucleotide. The drug conjugate of the present disclosure can regulate abnormal expression of a gene in cells by enhancing the targeted delivery of the functional oligonucleotides and thus the interaction of the functional oligonucleotides with a target sequence in the cell. The genes that are abnormally expressed in cells can be, for example, ApoB, ApoC, ANGPTL3, PCSK9, SCD1, TIMP-1, Col1A1, FVII, STAT3, p53, HBV, and HCV. In some embodiments, the gene abnormally expressed in hepatocytes is HBV gene, ANGPTL3 gene, or APOC3 gene. In the context of the present disclosure, HBV gene refers to the gene having the sequence as shown in Genbank assession number NC_003977.1; ANGPTL3 gene refers to the gene having the mRNA sequence as shown in Genbank assession number NM_014495.3; APOC3 gene refers to the gene having the mRNA sequence as shown in Genbank assession number NM_000040.1.

In some embodiments, a "target sequence" is a target mRNA. In the context of the present disclosure, "target mRNA" refers to the mRNA corresponding to a gene that is abnormally expressed in a cell, which can either be the mRNA corresponding to the overexpressed gene, or be the mRNA corresponding to the underexpressed gene, or the mRNA corresponding to the exogenous gene (e.g., a viral gene). Because most diseases arise from overexpression of mRNA, target mRNA especially refers to the mRNA corresponding to the overexpressed gene in the present disclosure. In some embodiments, the target mRNA can also be an mRNA whose expression level needs to be regulated in order to realize the other desired treatemnt and/or prevention effects although it is expressed at a normal leve. In some embodiments of the present disclosure, corresponding to the above abnormally expressed genes, the target mRNAs can be the mRNA corresponding to ApoB, ApoC, ANGPTL3, PCSK9, SCD1, TIMP-1, Col1A1, FVII, STAT3, p53, HBV, HCV, FXI, FXII, KNG, PNP, XO, PKK, PLG, C9, SARS, SARS-Cov-2, and ACE-2 genes. In some embodiments, the target mRNA can be an mRNA derived from transcription of corresponding HBV gene, an mRNA corresponding to ANGPTL3 gene, or an mRNA corresponding to APOC3 gene.

The P atom in Formula A60 can be linked to any possible position in the oligonucleotide sequence via a phosphate ester bond, for example, to any nucleotide of the oligonucleotide. In some embodiments, the functional oligonucleotide in the drug conjugates of the present disclosure is a single-stranded oligonucleotide (e.g., a single-stranded RNA or aptamer), in which case the P atom in Formula A60 can be linked to a terminal region of the single-stranded oligonucleotide. The terminal region of the single-stranded oligonucleotide refers to the first four nucleotides counted from one terminal of the single-stranded oligonucleotide. In some embodiments, the P atom in Formula A60 is linked to a terminal of the single-stranded oligonucleotide.

In some embodiments, the functional oligonucleotide in the drug conjugates of the present disclosure is a double-stranded oligonucleotide (e.g., siRNA, microRNA, or DNA), wherein the double-stranded oligonucleotide comprises a sense strand and an antisense strand, and the P atom in Formula A59 is linked to a terminal region of the sense strand or antisense strand of the double-stranded oligonucleotide. The terminal region refers to the first four nucleotides counted from one terminal of the sense strand or antisense strand. In some embodiments, the P atom in Formula A60 is linked to the terminal of the sense strand or antisense strand. In some further embodiments, the P atom in Formula A60 is linked to 3' terminal of the sense strand. In the case where the P atom in Formula A60 is linked to the above position in the sense strand of the double-stranded oligonucleotide, after entering into cells, the drug conjugate provided by the present disclosure can release a separate antisense strand of the double-stranded oligonucleotide during unwinding, thereby blocking the translation of the target mRNA into protein and inhibiting the expression of the gene.

The P atom in Formula A60 can be linked to any possible position of a nucleotide in the oligonucleotide sequence, for example, to position 5', 2' or 3', or to the base of the nucleotide. In some embodiments, the P atom in Formula A60 can be linked to position 2', 3', or 5' of a nucleotide in the oligonucleotide sequences by forming a phosphate ester bond. In some specific embodiments, the P atom in Formula A60 is linked to an oxygen atom formed by dehydrogenation of 3'-hydroxy of the nucleotide at 3' terminal of the sense strand in the double-stranded oligonucleotide sequence (in this case, the P atom and the corresponding phosphate group can be considered as the P atom and the phosphate group in the double-stranded oligonucleotide), or the P atom in Formula A60 is linked to a nucleotide by substituting a hydrogen

atom in 2'-hydroxy of a nucleotide of the sense strand in the double-stranded oligonucleotide sequence, or the P atom in Formula A60 is linked to a nucleotide by substituting a hydrogen atom in 5'-hydroxy of the nucleotide at 5' terminal of the sense strand in the double-stranded oligonucleotide sequence.

The following embodiments and examples detailedly describe the case where the active drug in the drug conjugate of the present disclosure is a small interfering RNA (siRNA). In this case, the drug conjugate of the present disclosure is a drug conjugate. In the context herein, the drug conjugates in these embodiments are also referred to as the drug conjugates of the present disclosure. However, this is only for the purpose of convenient description, and the present disclosure is only to illustrate the present disclosure in the form of specific embodiments or examples, and does not mean that the active drug in the drug conjugate of the present disclosure can only be an oligonucleotide or siRNA. According to the target position and actual effect required, those skilled in the art could expect replacing siRNA with other active drugs, for example, small molecule drugs, monoclonal antibody drugs, or other functional oligonucleotides.

It is well-known to those skilled in the art that the siRNA of the present disclosure comprises nucleotides as basic structural units, and the nucleotide comprises a phosphate group, a ribose group, and a base, which is not described here in detail. Typically, an active (i.e., functional) siRNA has a lenth of about 12-40 nucleotides, in some embodiments, about 15-30 nucleotides, each nucleotide in the siRNA can be independently a modified or unmodified nucleotide, and for increasing stability, at least some of the nucleotides in the siRNA are modified nucleotides.

The inventors of the present disclosure have found that the siRNAs in the following embodiments have higher activity and/or stability and thus can be used as siRNAs in some specific embodiments of the present disclosure.

According to some embodiments of the present disclosure, each nucleotide in the siRNA in the drug conjugate of the present disclosure (herein also referred to as the siRNA of the present disclosure) is independently a modified or unmodified nucleotide. The siRNA comprises a sense strand and an antisense strand, wherein the sense strand comprises a nucleotide sequence 1, and the antisense strand comprises a nucleotide sequence 2; the nucleotide sequence 1 and the nucleotide sequence 2 both have a length of 19 nucleotides and at least partly reverse complementary to form a complementary double-stranded region; at least a portion of the nucleotide sequence 2 is complementary to a first segment of the nucleotide sequence, and said first segment of the nucleotide sequence is a segment of the nucleotide sequence in the target mRNA.

In some embodiments, the siRNA of the present disclosure is a siRNA capable of inhibiting at least 50% of the gene expression of hepatitis B virus, at least 50% of the gene expression of angiopoietin-like protein 3, or at least 50% of the gene expression of apolipoprotein C3 at a concentration of 3 mg/kg.

In some embodiments, the nucleotide sequence 1 and the first segment of the nucleotide sequence have an equal length and no more than 3 nucleotide differences; the nucleotide sequence 2 and the nucleotide sequence B have an equal length and no more than 3 nucleotide differences; the nucleotide sequence B is a nucleotide sequence which is completely revese complementary to the first segment of the nucleotide sequence. These specific nucleotide differences will not significantly reduce the ability of the drug conjugates to inhibit the target gene, and these drug conjugates comprising the specific nucleotide differences are also within the scope of the present disclosure.

In some embodiments, the nucleotide sequence 1 is basically reverse complementary, substantially reverse complementary, or completely reverse complementary to the nucleotide sequence 2.

In some embodiments of the present disclosure, the nucleotide sequence 1 and the first segment of the nucleotide sequence have no more than 1 nucleotide difference, and/or the nucleotide sequence 2 and the nucleotide sequence B have no more than 1 nucleotide difference. In some embodiments, the difference between the nucleotide sequence 2 and the nucleotide sequence B comprises in the direction from 5' terminal to 3' terminal, the difference at the first nucleotide position Z' of the nucleotide sequence 2. In some embodiments, in the direction from 5' terminal to 3' terminal, the last nucleotide Z of the nucleotide sequence 1 is a nucleotide complementary to Z'.

In some embodiments, the sense strand further comprises a nucleotide sequence 3; the antisense strand further comprises a nucleotide sequence 4; the nucleotide sequence 3 and the nucleotide sequence 4 independently of one another have a length of 1 - 4 nucleotides, and the positions of the nucleotide sequence 3 and the nucleotide sequence 4 correspond to each other. In some embodiments, the nucleotide sequence 4 is at least partially complementary to the nucleotides at the corresponding positions in the target mRNA. In some embodiments, the nucleotide sequence 4 is completely complementary to the nucleotides at the corresponding positions in the target mRNA.

In some embodiments, the nucleotide sequence 3 is linked to 5' terminal of the nucleotide sequence 1, and the nucleotide sequence 4 is linked to 3' terminal of the nucleotide sequence 2. In some embodiments, the nucleotide sequence 3 and the nucleotide sequence 4 have an equal length and are reverse complementary to each other. Therefore, the sense strand and the antisense strand can have a length of 19-23 nucleotides. In some embodiments, the siRNA of the present disclosure further comprises a nucleotide sequence 5, which has a length of 1-3 nucleotides and is linked to 3' terminal of the antisense strand, thereby forming a 3' overhang of the antisense strand. In some embodiments, the nucleotide sequence 5 has a length of 1 or 2 nucleotides. As such, the length ratio of the sense strand to the antisense strand in the siRNA of the present disclosure can be 19/20, 19/21, 20/21, 20/22, 21/22, 21/23, 22/23, 22/24, 23/24, or 23/25.

In some embodiments, the nucleotide sequence 5 has a length of 2 nucleotides; and in the direction from 5'

terminal to 3' terminal, the nucleotide sequence 5 comprises 2 consecutive thymidine deoxynucleotides, 2 consecutive uracil nucleotides, or is complementary to a third segment of the nucleotide sequence, wherein the third segment of the nucleotide sequence is a nucleotide sequence in the target mRNA which is adjacent to the first segment of the nucleotide sequence, or adjacent to the second segment of the nucleotide sequence, and has an equal length to the nucleotide sequence 5. In one embodiment, the length ratio of the sense strand and the antisense strand of the siRNA of the present disclousure is 19/21 or 21/23. In this case, the siRNA of the present disclosure exhibits better silencing activity against cellular mRNA.

In some embodiments, in the siRNA of the present disclosure, each nucleotide is independently a modified or unmodified nucleotide. In some embodiments, the siRNA of the present disclosure does not comprise modified nucleotides; in some embodiments, the siRNA of the present disclosure comprises modified nucleotides, and the siRNA comprising these modified nucleotides has higher stability and silencing activity against the target mRNA.

In some embodiments, the siRNA of the conjugate comprises at least one modified nucleotide. In the context of the present disclosure, the term "modified nucleotide" used herein refers to a nucleotide formed by replacing the 2'-hydroxy of the ribose group with other groups, or a nucleotide analog, or a nucleotide in which the base is a modified base. The drug conjugates containing these modified nucleotides have higher stability and silencing activity against the target mRNA; for example, the modified nucleotides as disclosed in J.K. Watts, G.F. Deleavey, and M.J. Damha, Chemically modified siRNA: tools and applications. Drug Discov Today, 2008, 13(19-20): 842-55 can be selected. In some embodiments of the present disclosure, at least one nucleotide in the sense or antisense strand is a modified nucleotide, and/or at least one phosphate group is a phosphate group with modified group(s). In other words, at least a portion of the phosphate and/or ribose groups in the phosphate-ribose backbone of at least one single strand of the sense strand and the antisense strand are phosphate and/or ribose groups with modified groups. In some embodiments of the present disclosure, all nucleotides in the sense strand and/or the antisense strand are modified nucleotides.

In some embodiments, each nucleotide in the sense strand and the antisense strand is independently a fluoro modified nucleotide or a non-fluoro modified nucleotide.

In some embodiments, the nucleotide sequence 1 comprises no more than 5 fluoro modified nucleotides; in some embodiments, the nucleotide sequence 2 comprises no more than 7 fluoro modified nucleotides.

In some embodiments, the fluoro modified nucleotides are in the nucleotide sequence 1 and the nucleotide sequence 2, wherein the nucleotide sequence 1 comprises no more than 5 fluoro modified nucleotides, and the nucleotides at positions 7, 8 and 9 in the nucleotide sequence 1 in the direction from 5' terminal to 3' terminal are fluoro modified nucleotides; the nucleotide sequence 2 comprises no more than 7 fluoro modified nucleotides, and the nucleotides at positions 2, 6, 14 and 16 in the direction from 5' terminal to 3' terminal in the nucleotide sequence 2 are fluoro modified nucleotides.

In some specific embodiments, in the direction from 5' terminal to 3' terminal, the nucleotides at positions 7, 8 and 9 or 5, 7, 8 and 9 of the nucleotide sequence 1 in the sense strand are fluoro modified nucleotides, and the nucleotides at the other positions in the sense strand are non-fluoro modified nucleotides; and the nucleotides at positions 2, 6, 14 and 16 or 2, 6, 8, 9, 14 and 16 of the nucleotide sequence 2 in the antisense strand are fluoro modified nucleotides, and the nucleotides at the other positions in the antisense strand are non-fluoro modified nucleotides.

The fluoro modified nucleotide refers to a nucleotide formed by substituting the 2'-hydroxy of the ribose group of the nucleotide with a fluoro group, which has a structure as shown by Formula (207). The non-fluoro modified nucleotide refers to a nucleotide formed by substituting the 2'-hydroxy of the ribose group of the nucleotide with a non-fluoro group, or a nucleotide analogue. In some embodiments, each non-fluoro modified nucleotide is independently selected from the group consisting of a nucleotide formed by substituting the 2'-hydroxy of the ribose group of the nucleotide with a non-fluoro group, or a nucleotide analogue. A nucleotide formed by substituting the 2'-hydroxy of the ribose group with a non-fluoro group is well-known to those skilled in the art, and can be selected from one of 2'-alkoxy modified nucleotide, 2'-substituted alkoxy modified nucleotide, 2'-alkyl modified nucleotide, 2'-substituted alkyl modified nucleotide, 2'-amino modified nucleotide, 2'-substituted amino modified nucleotide and 2'-deoxy nucleotide.

In some embodiments, the 2'-alkoxy modified nucleotide is a methoxy modified nucleotide (2'-OMe), as shown by Formula (208). The 2'-substituted alkoxy modified nucleotide is, for example, a 2'-O-methoxyethyl modified nucleotide (2'-MOE) as shown by Formula (209). The 2'-amino modified nucleotide (2'-$NH_2$) is as shown by Formula (210). The 2'-deoxy nucleotide (DNA) is as shown by Formula (211).

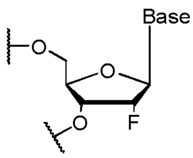

Formula (207)

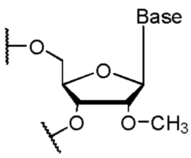

Formula (208)

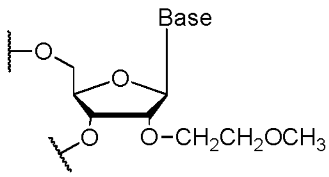

Formula (209)

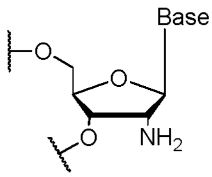

Formula (210)

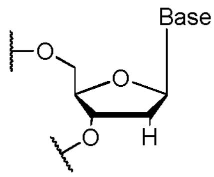

Formula (211)

A "nucleotide analogue" refers to a group that can replace a nucleotide in the nucleic acid, while structurally differs from an adenine ribonucleotide, a guanine ribonucleotide, a cytosine ribonucleotide, a uracil ribonucleotide or thymine deoxyribonucleotide, such as an isonucleotide, a bridged nucleic acid (BNA) nucleotide or an acyclic nucleotide.

A BNA is a nucleotide that is constrained or is not accessible. BNA can contain a 5-, 6-membered or even a 7-membered ring bridged structure with a "fixed" C3'-endo sugar puckering. The bridge is typically incorporated at the 2'- and 4'-position of the ribose to afford a 2', 4'-BNA nucleotide, such as LNA, ENA and cET BNA, which are as shown by Formula (212), (213) and (214), respectively.

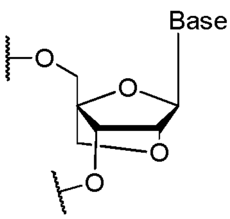

Formula (212)

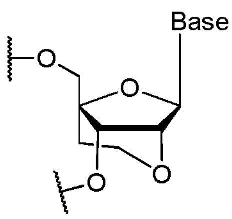

Formula (213)

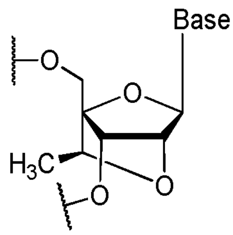

Formula (214)

An acyclic nucleotide is a nucleotide in which the ribose ring is opened, such as an unlocked nucleic acid (UNA) nucleotide and a glycerol nucleic acid (GNA) nucleotide, which are as shown by Formula (215) and (216), respectively.

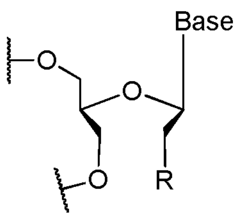

,

Formula (215)

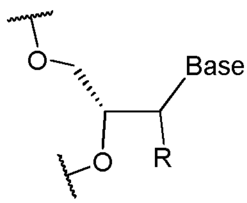

.

Formula (216)

In the above Formulae (215) and (216), R is selected from H, OH, or alkoxy (O-alkyl).

An isonucleotide is a compound in which the position of the base on the ribose ring in the nucleotide is changed, such as a compound in which the base is transposed from position-1' to position-2' or -3' on the ribose ring, as shown by Formula (217) or (218), respectively.

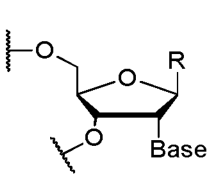

,

Formula (217)

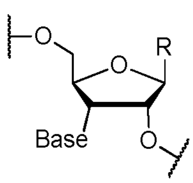

.

Formula (218)

In the above compounds of Formulae (217)-(218), "Base" represents a base, such as, A, U, G, C, or T; R is selected from H, OH, F, or a non-fluoro group described above.

In some embodiments, a nucleotide analogue is selected from the group consisting of an isonucleotide, LNA, ENA, cET, UNA, and GNA. In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group.

In the context of the present disclosure, a "fluoro modified nucleotide", a "2'-fluoro modified nucleotide", a "nucleotide in which 2'-hydroxy of the ribose group is substituted with a fluoro group" and a "nucleotide comprising 2'-fluororibosyl" have the same meaning, referring to the nucleotide formed by substituting the 2'-hydroxy of the ribose group with a fluoro group, having the structure as shown by Formula (207). A "methoxy modified nucleotide", a "2'-methoxy modified nucleotide", a "nucleotide in which 2'-hydroxy of a ribose group is substituted with a methoxy" and a "nucleotide comprising 2'-methoxyribosyl" have the same meaning, referring to a compound in which 2'-hydroxy of the ribose group in the nucleotide is substituted with a methoxy, and has a structure as shown by Formula (208).

In some embodiments, the siRNA in the drug conjugates of the present disclosure is a siRNA with the following modifications: in the direction from 5' terminal to 3' terminal, the nucleotides at positions 7, 8 and 9 or 5, 7, 8 and 9 of the nucleotide sequence 1 in the sense strand are fluoro modified nucleotides, and the nucleotides at the other positions in the sense strand are methoxy modified nucleotides; and the nucleotides at positions 2, 6, 14 and 16 or 2, 6, 8, 9, 14 and 16 of the nucleotide sequence 2 in the antisense strand are fluoro modified nucleotides, and the nucleotides at the other positions in the antisense strand are methoxy modified nucleotides.

In some specific embodiments, the siRNA of the present disclosure is a siRNA with the following modifications: in the direction from 5' terminal to 3' terminal, the nucleotides at positions 7, 8 and 9 of the nucleotide sequence 1 in the sense strand of the siRNA are fluoro modified nucleotides, and the nucleotides at the other positions in the sense strand are methoxy modified nucleotides; and the nucleotides at positions 2, 6, 14 and 16 of the nucleotide sequence 2 in the antisense strand are fluoro modified nucleotides, and the nucleotides at the other positions in the antisense strand are methoxy modified nucleotides; alternatively, in the direction from 5' terminal to 3' terminal, the nucleotides at positions 5, 7, 8 and 9 of the nucleotide sequence 1 in the sense strand are fluoro modified nucleotides, and the nucleotides at the other positions in the sense strand are methoxy modified nucleotides; and, in the direction from 5' terminal to 3' terminal, the nucleotides at positions 2, 6, 8, 9, 14 and 16 of the nucleotide sequence 2 in the antisense strand are fluoro modified nucleotides, and the nucleotides at the other positions in the antisense strand are methoxy modified nucleotides; alternatively, in the direction from 5' terminal to 3' terminal, the nucleotides at positions 5, 7, 8 and 9 of the nucleotide sequence 1 in the sense strand are fluoro modified nucleotides, and the nucleotides at other positions in the sense strand are methoxy modified nucleotides; and, in the direction from 5' terminal to 3' terminal, the nucleotides at positions 2, 6, 14 and 16 of the nucleotide sequence 2 in the antisense strand are fluoro modified nucleotides, and the nucleotides at the other positions in the antisense strand are methoxy modified nucleotides.

The siRNAs with the above modifications not only have lower costs, but also make it difficult for the ribonucleases in blood to cleave the nucleic acid, thereby increasing the stability of the nucleic acid and rendering the nucleic acid to have stronger resistance against nuclease hydrolysis.

In some embodiments, the nucleotide has a modification on the phosphate group.In the context of the present disclosure, the modification on a phosphate group may be a phosphorothioate modification as shown by Formula (201), that is, substituting a non-bridging oxygen atom in a phosphodiester bond used as a linkage between adjacent nucleotides with a sulfur atom so that the phosphodiester bond is changed to a phosphorothioate diester bond. This modification can stabilize the structure of the siRNA, while maintaining high specificity and high affinity of base pairing.

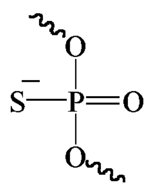

Formula (201).

In some embodiments, in the siRNA, at least one linkage selected from the group consisting of the following inter-nucleotide linkages is a phosphorothioate linkage:

the linkage between the first and second nucleotides at 5' terminal of the sense strand;
the linkage between the second and third nucleotides at 5' terminal of the sense strand;
the linkage between the first and second nucleotides at 3' terminal of the sense strand;
the linkage between the second and third nucleotides at 3' terminal of the sense strand;

the linkage between the first and second nucleotides at 5' terminal of the antisense strand;
the linkage between the second and third nucleotides at 5' terminal of the antisense strand;
the linkage between the first and second nucleotides at 3' terminal of the antisense strand; and
the linkage between the second and third nucleotides at 3' terminal of the antisense strand.

In some embodiments, the nucleotide at 5'-terminal of the antisense strand sequence of the siRNA molecule is a 5'-phosphate nucleotide or a 5'-phosphate analogue modified nucleotide.

As well known to those skilled in the art, the 5'-phosphate nucleotide has a structure as shown by Formula (202):

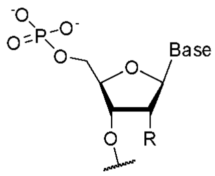

Formula (202).

Meanwhile, common types of the 5'-phosphate analogue modified nucleotide are well-known to those skilled in the art, such as, the following four nucleotides as shown by Formula (203)-(206) as disclosed in Anastasia Khvorova and Jonathan K. Watts, The chemical evolution of oligonucleotide therapies of clinical utility. Nature Biotechnology, 2017, 35(3): 238-48:

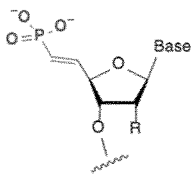 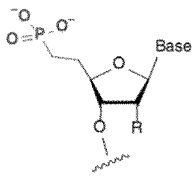 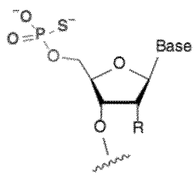 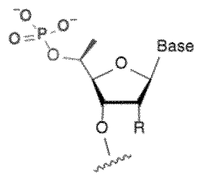

Formula (203) Formula (204) Formula (205) Formula (206),

wherein R represents a group selected from the group consisting of H, OH, F and methoxy; and "Base" represents a base selected from A, U, C, G, or T.

In some specific embodiments, the 5'-phosphate nucleotide or 5'-phosphate analogue modified nucleotide is a nucleotide with E-vinylphosphonat (E-VP) as shown by Formula (203); a nucleotide with 5'-phosphate as shown by Formula (202) or a nucleotide with 5'-phosphorothioate modification as shown by Formula (205).

The inventors of the present disclosure have surprisingly found that the drug conjugate of the present disclosure exhibits significantly improved stability in serum without significantly reduced silencing activity against target mRNA and further shows excellent inhibitory effect on gene expressions. Hence, the drug conjugate of the present disclosure has higher *in vivo* delivery efficiency. According to some embodiments of the present disclosure, a drug conjugate of the present disclosure is a drug conjugate comprising the siRNAs which are, for example, the siRNAs as shown in Tables 1A-1H.

Table 1: siRNA sequences in some embodiments

Table 1A

| Number | | Sequence direction 5' - 3' | SEQ ID NO |
|---|---|---|---|
| siHBa1 | S | CCUUGAGGCAUACUUCAAA | 1 |
| | AS | UUUGAAGUAUGCCUCAAGGUU | 2 |
| siHBa2 | S | GACCUUGAGGCAUACUUCAAA | 3 |
| | AS | UUUGAAGUAUGCCUCAAGGUCGG | 4 |
| siHBa1M1 | S | CmCmUmUmGmAmGfGfCfAmUmAmCmUmUmCmAmAmAm | 5 |
| | AS | UmUfUmGmAmAfGmUmAmUmGmCmCmUfCmAfAmGmGmUmUm | 6 |
| siHBa1M2 | S | CmCmUmUmGfAmGfGfCfAmUmAmCmUmUmCmAmAmAm | 7 |
| | AS | UmUfUmGmAmAfGmUfAfUmGmCmCmUfCmAfAmGmGmUmUm | 8 |
| siHBa2M1 | S | GmAmCmCmUmUmGmAmGfGfCfAmUmAmCmUmUmCmAmAmAm | 9 |
| | AS | UmUfUmGmAmAfGmUmAmUmGmCmCmUfCmAfAmGmGmUmCmGmGm | 10 |
| siHBa2M2 | S | GmAmCmCmUmUmGfAmGfGfCfAmUmAmCmUmUmCmAmAmAm | 11 |
| | AS | UmUfUmGmAmAfGmUfAfUmGmCmCmUfCmAfAmGmGmUmCmGmGm | 12 |
| siHBa1M1S | S | CmsCmsUmUmGmAmGfGfCfAmUmAmCmUmUmCmAmAmAm | 13 |
| | AS | UmsUfsUmGmAmAfGmUmAmUmGmCmCmUfCmAfAmGmGmsUmsUm | 14 |
| siHBa1M2S | S | CmsCmsUmUmGfAmGfGfCfAmUmAmCmUmUmCmAmAmAm | 15 |
| | AS | UmsUfsUmGmAmAfGmUfAfUmGmCmCmUfCmAfAmGmGmsUmsUm | 16 |
| siHBa2M1S | S | GmsAmsCmCmUmUmGmAmGfGfCfAmUmAmCmUmUmCmAmAmAm | 17 |
| | AS | UmsUfsUmGmAmAfGmUmAmUmGmCmCmUfCmAfAmGmGmUmCmsGmsGm | 18 |
| siHBa2M2S | S | GmsAmsCmCmUmUmGfAmGfGfCfAmUmAmCmUmUmCmAmAmAm | 19 |
| | AS | UmsUfsUmGmAmAfGmUfAfUmGmCmCmUfCmAfAmGmGmUmCmsGmsGm | 20 |
| siHBa1M1P1 | S | CmCmUmUmGmAmGfGfCfAmUmAmCmUmUmCmAmAmAm | 21 |
| | AS | P1-UmUfUmGmAmAfGmUmAmUmGmCmCmUfCmAfAmGmGmUmUm | 22 |
| siHBa1M2P1 | S | CmCmUmUmGfAmGfGfCfAmUmAmCmUmUmCmAmAmAm | 23 |
| | AS | P1-UmUfUmGmAmAfGmUfAfUmGmCmCmUfCmAfAmGmGmUmUm | 24 |
| siHBa2M1P1 | S | GmAmCmCmUmUmGmAmGfGfCfAmUmAmCmUmUmCmAmAmAm | 25 |
| | AS | P1-UmUfUmGmAmAfGmUmAmUmGmCmCmUfCmAfAmGmGmUmCmGmGm | 26 |
| siHBa2M2P1 | S | GmAmCmCmUmUmGfAmGfGfCfAmUmAmCmUmUmCmAmAmAm | 27 |
| | AS | P1-UmUfUmGmAmAfGmUfAfUmGmCmCmUfCmAfAmGmGmUmCmGmGm | 28 |
| siHBa1M1SP1 | S | CmsCmsUmUmGmAmGfGfCfAmUmAmCmUmUmCmAmAmAm | 29 |
| | AS | P1-UmsUfsUmGmAmAfGmUmAmUmGmCmCmUfCmAfAmGmGmsUmsUm | 30 |
| siHBa1M2SP1 | S | CmsCmsUmUmGfAmGfGfCfAmUmAmCmUmUmCmAmAmAm | 31 |
| | AS | P1-UmsUfsUmGmAmAfGmUfAfUmGmCmCmUfCmAfAmGmGmsUmsUm | 32 |
| siHBa2M1SP1 | S | GmsAmsCmCmUmUmGmAmGfGfCfAmUmAmCmUmUmCmAmAmAm | 33 |
| | AS | P1-UmsUfsUmGmAmAfGmUmAmUmGmCmCmUfCmAfAmGmGmUmCmsGmsGm | 34 |
| siHBa2M2SP1 | S | GmsAmsCmCmUmUmGfAmGfGfCfAmUmAmCmUmUmCmAmAmAm | 35 |
| | AS | P1-UmsUfsUmGmAmAfGmUfAfUmGmCmCmUfCmAfAmGmGmUmCmsGmsGm | 36 |

Table 1B

| Number | Sequence direction 5' - 3' | | SEQ ID NO |
|---|---|---|---|
| siHBb1 | S | UGCUAUGCCUCAUCUUCUA | 37 |
| | AS | UAGAAGAUGAGGCAUAGCAGC | 38 |
| siHBb2 | S | UGCUAUGCCUCAUCUUCUA | 39 |
| | AS | UAGAAGAUGAGGCAUAGCAUU | 40 |
| siHBb1M1 | S | UmGmCmUmAmUmGfCfCfUmCmAmUmCmUmUmCmUmAm | 41 |
| | AS | UmAfGmAmAmGfAmUmGmAmGmGmCmAfUmAfGmCmAmGmCm | 42 |
| siHBb2M1 | S | UmGmCmUmAmUmGfCfCfUmCmAmUmCmUmUmCmUmAm | 43 |
| | AS | UmAfGmAmAmGfAmUmGmAmGmGmCmAfUmAfGmCmAmUmUm | 44 |
| siHBb1M2 | S | UmGmCmUmAfUmGfCfCfUmCmAmUmCmUmUmCmUmAm | 45 |
| | AS | UmAfGmAmAmGfAmUfGfAmGmGmCmAfUmAfGmCmAmGmCm | 46 |
| siHBb2M2 | S | UmGmCmUmAfUmGfCfCfUmCmAmUmCmUmUmCmUmAm | 47 |
| | AS | UmAfGmAmAmGfAmUfGfAmGmGmCmAfUmAfGmCmAmUmUm | 48 |
| siHBb1M1S | S | UmsGmsCmUmAmUmGfCfCfUmCmAmUmCmUmUmCmUmAm | 49 |
| | AS | UmsAfsGmAmAmGfAmUmGmAmGmGmCmAfUmAfGmCmAmsGmsCm | 50 |
| siHBb2M1S | S | UmsGmsCmUmAmUmGfCfCfUmCmAmUmCmUmUmCmUmAm | 51 |
| | AS | UmsAfsGmAmAmGfAmUmGmAmGmGmCmAfUmAfGmCmAmsUmsUm | 52 |
| siHBb1M2S | S | UmsGmsCmUmAfUmGfCfCfUmCmAmUmCmUmUmCmUmAm | 53 |
| | AS | UmsAfsGmAmAmGfAmUfGfAmGmGmCmAfUmAfGmCmAmsGmsCm | 54 |
| siHBb2M2S | S | UmsGmsCmUmAfUmGfCfCfUmCmAmUmCmUmUmCmUmAm | 55 |
| | AS | UmsAfsGmAmAmGfAmUfGfAmGmGmCmAfUmAfGmCmAmsUmsUm | 56 |
| siHBb1M1P1 | S | UmGmCmUmAmUmGfCfCfUmCmAmUmCmUmUmCmUmAm | 57 |
| | AS | P1-UmAfGmAmAmGfAmUmGmAmGmGmCmAfUmAfGmCmAmGmCm | 58 |
| siHBb2M1P1 | S | UmGmCmUmAmUmGfCfCfUmCmAmUmCmUmUmCmUmAm | 59 |
| | AS | P1-UmAfGmAmAmGfAmUmGmAmGmGmCmAfUmAfGmCmAmUmUm | 60 |
| siHBb1M2P1 | S | UmGmCmUmAfUmGfCfCfUmCmAmUmCmUmUmCmUmAm | 61 |
| | AS | P1-UmAfGmAmAmGfAmUfGfAmGmGmCmAfUmAfGmCmAmGmCm | 62 |
| siHBb2M2P1 | S | UmGmCmUmAfUmGfCfCfUmCmAmUmCmUmUmCmUmAm | 63 |
| | AS | P1-UmAfGmAmAmGfAmUfGfAmGmGmCmAfUmAfGmCmAmUmUm | 64 |
| siHBb1M1SP1 | S | UmsGmsCmUmAmUmGfCfCfUmCmAmUmCmUmUmCmUmAm | 65 |
| | AS | P1-UmsAfsGmAmAmGfAmUmGmAmGmGmCmAfUmAfGmCmAmsGmsCm | 66 |
| siHBb2M1SP1 | S | UmsGmsCmUmAmUmGfCfCfUmCmAmUmCmUmUmCmUmAm | 67 |
| | AS | P1-UmsAfsGmAmAmGfAmUmGmAmGmGmCmAfUmAfGmCmAmsUmsUm | 68 |
| siHBb1M2SP1 | S | UmsGmsCmUmAfUmGfCfCfUmCmAmUmCmUmUmCmUmAm | 69 |
| | AS | P1-UmsAfsGmAmAmGfAmUfGfAmGmGmCmAfUmAfGmCmAmsGmsCm | 70 |
| siHBb2M2SP1 | S | UmsGmsCmUmAfUmGfCfCfUmCmAmUmCmUmUmCmUmAm | 71 |
| | AS | P1-UmsAfsGmAmAmGfAmUfGfAmGmGmCmAfUmAfGmCmAmsUmsUm | 72 |

Table 1C

| Number | | Sequence direction 5' - 3' | SEQ ID NO |
|---|---|---|---|
| siHBc1 | S | UCUGUGCCUUCUCAUCUGA | 73 |
| | AS | UCAGAUGAGAAGGCACAGACG | 74 |
| siHBc1M1 | S | UmCmUmGmUmGmCfCfUfUmCmUmCmAmUmCmUmGmAm | 75 |
| | AS | UmCfAmGmAmUfGmAmGmAmAmGmGmCfAmCfAmGmAmCmGm | 76 |
| siHBc1M2 | S | UmCmUmGmUfGmCfCfUfUmCmUmCmAmUmCmUmGmAm | 77 |
| | AS | UmCfAmGmAmUfGmAfGfAmAmGmGmCfAmCfAmGmAmCmGm | 78 |
| siHBc1M1S | S | UmsCmsUmGmUmGmCfCfUfUmCmUmCmAmUmCmUmGmAm | 79 |
| | AS | UmsCfsAmGmAmUfGmAmGmAmAmGmGmCfAmCfAmGmAmsCmsGm | 80 |
| siHBc1M2S | S | UmsCmsUmGmUfGmCfCfUfUmCmUmCmAmUmCmUmGmAm | 81 |
| | AS | UmsCfsAmGmAmUfGmAfGfAmAmGmGmCfAmCfAmGmAmsCmsGm | 82 |
| siHBc1M1P1 | S | UmCmUmGmUmGmCfCfUfUmCmUmCmAmUmCmUmGmAm | 83 |
| | AS | P1-UmCfAmGmAmUfGmAmGmAmAmGmGmCfAmCfAmGmAmCmGm | 84 |
| siHBc1M2P1 | S | UmCmUmGmUfGmCfCfUfUmCmUmCmAmUmCmUmGmAm | 85 |
| | AS | P1-UmCfAmGmAmUfGmAfGfAmAmGmGmCfAmCfAmGmAmCmGm | 86 |
| siHBc1M1SP1 | S | UmsCmsUmGmUmGmCfCfUfUmCmUmCmAmUmCmUmGmAm | 87 |
| | AS | P1-UmsCfsAmGmAmUfGmAmGmAmAmGmGmCfAmCfAmGmAmsCmsGm | 88 |
| siHBc1M2SP1 | S | UmsCmsUmGmUfGmCfCfUfUmCmUmCmAmUmCmUmGmAm | 89 |
| | AS | P1-UmsCfsAmGmAmUfGmAfGfAmAmGmGmCfAmCfAmGmAmsCmsGm | 90 |

Table 1D

| Number | | Sequence direction 5' - 3' | SEQ ID NO |
|---|---|---|---|
| siHBd1 | S | CGUGUGCACUUCGCUUCAA | 91 |
| | AS | UUGAAGCGAAGUGCACACGGU | 92 |
| siHBd1M1 | S | CmGmUmGmUmGmCfAfCfUmUmCmGmCmUmUmCmAmAm | 93 |
| | AS | UmUfGmAmAmGfCmGmAmAmGmUmGmCfAmCfAmCmGmGmUm | 94 |
| siHBd1M2 | S | CmGmUmGmUfGmCfAfCfUmUmCmGmCmUmUmCmAmAm | 95 |
| | AS | UmUfGmAmAmGfCmGfAfAmGmUmGmCfAmCfAmCmGmGmUm | 96 |
| siHBd1M1S | S | CmsGmsUmGmUmGmCfAfCfUmUmCmGmCmUmUmCmAmAm | 97 |
| | AS | UmsUfsGmAmAmGfCmGmAmAmGmUmGmCfAmCfAmCmGmsGmsUm | 98 |
| siHBd1M2S | S | CmsGmsUmGmUfGmCfAfCfUmUmCmGmCmUmUmCmAmAm | 99 |
| | AS | UmsUfsGmAmAmGfCmGfAfAmGmUmGmCfAmCfAmCmGmsGmsUm | 100 |
| siHBd1M1P1 | S | CmGmUmGmUmGmCfAfCfUmUmCmGmCmUmUmCmAmAm | 101 |
| | AS | P1-UmUfGmAmAmGfCmGmAmAmGmUmGmCfAmCfAmCmGmGmUm | 102 |
| siHBd1M2P1 | S | CmGmUmGmUfGmCfAfCfUmUmCmGmCmUmUmCmAmAm | 103 |
| | AS | P1-UmUfGmAmAmGfCmGfAfAmGmUmGmCfAmCfAmCmGmGmUm | 104 |
| siHBd1M1SP1 | S | CmsGmsUmGmUmGmCfAfCfUmUmCmGmCmUmUmCmAmAm | 105 |
| | AS | P1-UmsUfsGmAmAmGfCmGmAmAmGmUmGmCfAmCfAmCmGmsGmsUm | 106 |
| siHBd1M2SP1 | S | CmsGmsUmGmUfGmCfAfCfUmUmCmGmCmUmUmCmAmAm | 107 |
| | AS | P1-UmsUfsGmAmAmGfCmGfAfAmGmUmGmCfAmCfAmCmGmsGmsUm | 108 |

Table1E

| Number | | Sequence direction 5' - 3' | SEQ ID NO |
|---|---|---|---|
| siHBe1 | S | GAAAGUAUGUCAACGAAUU | 109 |
| | AS | AAUUCGUUGACAUACUUUCUU | 110 |
| siHBe2 | S | GAAAGUAUGUCAACGAAUU | 111 |
| | AS | AAUUCGUUGACAUACUUUCCA | 112 |
| siHBe3 | S | GAAAGUAUGUCAACGAAUA | 113 |
| | AS | UAUUCGUUGACAUACUUUCUU | 114 |
| siHBe4 | S | GAAAGUAUGUCAACGAAUA | 115 |
| | AS | UAUUCGUUGACAUACUUUCCA | 116 |
| siHBe5 | S | UGGAAAGUAUGUCAACGAAUA | 117 |
| | AS | UAUUCGUUGACAUACUUUCCAUU | 118 |
| siHBe6 | S | UGGAAAGUAUGUCAACGAAUA | 119 |
| | AS | UAUUCGUUGACAUACUUUCCAAU | 120 |
| siHBe7 | S | UGGAAAGUAUGUCAACGAAUU | 121 |
| | AS | AAUUCGUUGACAUACUUUCCAUU | 122 |
| siHBe1M1 | S | GmAmAmAmGmUmAfUfGfUmCmAmAmCmGmAmAmUmUm | 123 |
| | AS | AmAfUmUmCmGfUmUmGmAmCmAmUmAfCmUfUmUmCmUmUm | 124 |
| siHBe2M1 | S | GmAmAmAmGmUmAfUfGfUmCmAmAmCmGmAmAmUmUm | 125 |
| | AS | AmAfUmUmCmGfUmUmGmAmCmAmUmAfCmUfUmUmCmCmAm | 126 |
| siHBe3M1 | S | GmAmAmAmGmUmAfUfGfUmCmAmAmCmGmAmAmUmAm | 127 |
| | AS | UmAfUmUmCmGfUmUmGmAmCmAmUmAfCmUfUmUmCmUmUm | 128 |
| siHBe4M1 | S | GmAmAmAmGmUmAfUfGfUmCmAmAmCmGmAmAmUmAm | 129 |
| | AS | UmAfUmUmCmGfUmUmGmAmCmAmUmAfCmUfUmUmCmCmAm | 130 |
| siHBe1M2 | S | GmAmAmAmGfUmAfUfGfUmCmAmAmCmGmAmAmUmUm | 131 |
| | AS | AmAfUmUmCmGfUmUfGfAmCmAmUmAfCmUfUmUmCmUmUm | 132 |
| siHBe2M2 | S | GmAmAmAmGfUmAfUfGfUmCmAmAmCmGmAmAmUmUm | 133 |
| | AS | AmAfUmUmCmGfUmUfGfAmCmAmUmAfCmUfUmUmCmCmAm | 134 |
| siHBe3M2 | S | GmAmAmAmGfUmAfUfGfUmCmAmAmCmGmAmAmUmAm | 135 |
| | AS | UmAfUmUmCmGfUmUfGfAmCmAmUmAfCmUfUmUmCmUmUm | 136 |
| siHBe4M2 | S | GmAmAmAmGfUmAfUfGfUmCmAmAmCmGmAmAmUmAm | 137 |
| | AS | UmAfUmUmCmGfUmUfGfAmCmAmUmAfCmUfUmUmCmCmAm | 138 |
| siHBe1M3 | S | GmAmAmAmGfUmAfUfGfUmCmAmAmCmGmAmAmUmUm | 139 |
| | AS | AmAfUmUmCmGfUmUmGmAmCmAmUmAfCmUfUmUmCmUmUm | 140 |
| siHBe2M3 | S | GmAmAmAmGfUmAfUfGfUmCmAmAmCmGmAmAmUmUm | 141 |
| | AS | AmAfUmUmCmGfUmUmGmAmCmAmUmAfCmUfUmUmCmCmAm | 142 |
| siHBe3M3 | S | GmAmAmAmGfUmAfUfGfUmCmAmAmCmGmAmAmUmAm | 143 |
| | AS | UmAfUmUmCmGfUmUmGmAmCmAmUmAfCmUfUmUmCmUmUm | 144 |
| siHBe4M3 | S | GmAmAmAmGfUmAfUfGfUmCmAmAmCmGmAmAmUmAm | 145 |
| | AS | UmAfUmUmCmGfUmUmGmAmCmAmUmAfCmUfUmUmCmCmAm | 146 |
| siHBe5M1 | S | UmGmGmAmAmAmGmUmAfUfGfUmCmAmAmCmGmAmAmUmAm | 147 |
| | AS | UmAfUmUmCmGfUmUmGmAmCmAmUmAfCmUfUmUmCmCmAmUmUm | 148 |
| siHBe6M2 | S | UmGmGmAmAmAmGfUmAfUfGfUmCmAmAmCmGmAmAmUmAm | 149 |
| | AS | UmAfUmUmCmGfUmUfGfAmCmAmUmAfCmUfUmUmCmCmAmAmUm | 150 |
| SiHBe7M3 | S | UmGmGmAmAmAmGfUmAfUfGfUmCmAmAmCmGmAmAmUmUm | 151 |
| | AS | AmAfUmUmCmGfUmUmGmAmCmAmUmAfCmUfUmUmCmCmAmUmUm | 152 |
| siHBe1M1S | S | GmsAmsAmAmGmUmAfUfGfUmCmAmAmCmGmAmAmUmUm | 153 |

| | | | |
|---|---|---|---|
| | AS | AmsAfsUmUmCmGfUmUmGmAmCmAmUmAfCmUfUmUmCmsUmsUm | 154 |
| siHBe2M1S | S | GmsAmsAmAmGmUmAfUfGfUmCmAmAmCmGmAmAmUmUm | 155 |
| | AS | AmsAfsUmUmCmGfUmUmGmAmCmAmUmAfCmUfUmUmCmsCmsAm | 156 |
| siHBe3M1S | S | GmsAmsAmAmGmUmAfUfGfUmCmAmAmCmGmAmAmUmAm | 157 |
| | AS | UmsAfsUmUmCmGfUmUmGmAmCmAmUmAfCmUfUmUmCmsUmsUm | 158 |
| siHBe4M1S | S | GmsAmsAmAmGmUmAfUfGfUmCmAmAmCmGmAmAmUmAm | 159 |
| | AS | UmsAfsUmUmCmGfUmUmGmAmCmAmUmAfCmUfUmUmCmsCmsAm | 160 |
| siHBe1M2S | S | GmsAmsAmAmGfUmAfUfGfUmCmAmAmCmGmAmAmUmUm | 161 |
| | AS | AmsAfsUmUmCmGfUmUfGfAmCmAmUmAfCmUfUmUmCmsUmsUm | 162 |
| siHBe2M2S | S | GmsAmsAmAmGfUmAfUfGfUmCmAmAmCmGmAmAmUmUm | 163 |
| | AS | AmsAfsUmUmCmGfUmUfGfAmCmAmUmAfCmUfUmUmCmsCmsAm | 164 |
| siHBe3M2S | S | GmsAmsAmAmGfUmAfUfGfUmCmAmAmCmGmAmAmUmAm | 165 |
| | AS | UmsAfsUmUmCmGfUmUfGfAmCmAmUmAfCmUfUmUmCmsUmsUm | 166 |
| siHBe4M2S | S | GmsAmsAmAmGfUmAfUfGfUmCmAmAmCmGmAmAmUmAm | 167 |
| | AS | UmsAfsUmUmCmGfUmUfGfAmCmAmUmAfCmUfUmUmCmsCmsAm | 168 |
| siHBe1M3S | S | GmsAmsAmAmGfUmAfUfGfUmCmAmAmCmGmAmAmUmUm | 169 |
| | AS | AmsAfsUmUmCmGfUmUmGmAmCmAmUmAfCmUfUmUmCmsUmsUm | 170 |
| siHBe2M3S | S | GmsAmsAmAmGfUmAfUfGfUmCmAmAmCmGmAmAmUmUm | 171 |
| | AS | AmsAfsUmUmCmGfUmUmGmAmCmAmUmAfCmUfUmUmCmsCmsAm | 172 |
| siHBe3M3S | S | GmsAmsAmAmGfUmAfUfGfUmCmAmAmCmGmAmAmUmAm | 173 |
| | AS | UmsAfsUmUmCmGfUmUmGmAmCmAmUmAfCmUfUmUmCmsUmsUm | 174 |
| siHBe4M3S | S | GmsAmsAmAmGfUmAfUfGfUmCmAmAmCmGmAmAmUmAm | 175 |
| | AS | UmsAfsUmUmCmGfUmUmGmAmCmAmUmAfCmUfUmUmCmsCmsAm | 176 |
| siHBe5M1S | S | UmsGmsGmAmAmAmGmUmAfUfGfUmCmAmAmCmGmAmAmUmAm | 177 |
| | AS | UmsAfsUmUmCmGfUmUmGmAmCmAmUmAfCmUfUmUmCmCmAmUmsUm | 178 |
| siHBe6M2S | S | UmsGmsGmAmAmAmGfUmAfUfGfUmCmAmAmCmGmAmAmUmAm | 179 |
| | AS | UmsAfsUmUmCmGfUmUfGfAmCmAmUmAfCmUfUmUmCmCmAmsAmsUm | 180 |
| SiHBe7M3S | S | UmsGmsGmAmAmAmGfUmAfUfGfUmCmAmAmCmGmAmAmUmUm | 181 |
| | AS | AmsAfsUmUmCmGfUmUmGmAmCmAmUmAfCmUfUmUmCmCmAmsUmsUm | 182 |
| siHBe1M1P1 | S | GmAmAmAmGmUmAfUfGfUmCmAmAmCmGmAmAmUmUm | 183 |
| | AS | P1-AmAfUmUmCmGfUmUmGmAmCmAmUmAfCmUfUmUmCmUmUm | 184 |
| siHBe2M1P1 | S | GmAmAmAmGmUmAfUfGfUmCmAmAmCmGmAmAmUmUm | 185 |
| | AS | P1-AmAfUmUmCmGfUmUmGmAmCmAmUmAfCmUfUmUmCmCmAm | 186 |
| siHBe3M1P1 | S | GmAmAmAmGmUmAfUfGfUmCmAmAmCmGmAmAmUmAm | 187 |
| | AS | P1-UmAfUmUmCmGfUmUmGmAmCmAmUmAfCmUfUmUmCmUmUm | 188 |
| siHBe4M1P1 | S | GmAmAmAmGmUmAfUfGfUmCmAmAmCmGmAmAmUmAm | 189 |
| | AS | P1-UmAfUmUmCmGfUmUmGmAmCmAmUmAfCmUfUmUmCmCmAm | 190 |
| siHBe1M2P1 | S | GmAmAmAmGfUmAfUfGfUmCmAmAmCmGmAmAmUmUm | 191 |

| | | | |
|---|---|---|---|
| | AS | P1-AmAfUmUmCmGfUmUfGfAmCmAmUmAfCmUfUmUmCmUmUm | 192 |
| siHBe2M2P1 | S | GmAmAmAmGfUmAfUfGfUmCmAmAmCmGmAmAmUmUm | 193 |
| | AS | P1-AmAfUmUmCmGfUmUfGfAmCmAmUmAfCmUfUmUmCmCmAm | 194 |
| siHBe3M2P1 | S | GmAmAmAmGfUmAfUfGfUmCmAmAmCmGmAmAmUmAm | 195 |
| | AS | P1-UmAfUmUmCmGfUmUfGfAmCmAmUmAfCmUfUmUmCmUmUm | 196 |
| siHBe4M2P1 | S | GmAmAmAmGfUmAfUfGfUmCmAmAmCmGmAmAmUmAm | 197 |
| | AS | P1-UmAfUmUmCmGfUmUfGfAmCmAmUmAfCmUfUmUmCmCmAm | 198 |
| siHBe1M3P1 | S | GmAmAmAmGfUmAfUfGfUmCmAmAmCmGmAmAmUmUm | 199 |
| | AS | P1-AmAfUmUmCmGfUmUmGmAmCmAmUmAfCmUfUmUmCmUmUm | 200 |
| siHBe2M3P1 | S | GmAmAmAmGfUmAfUfGfUmCmAmAmCmGmAmAmUmUm | 201 |
| | AS | P1-AmAfUmUmCmGfUmUmGmAmCmAmUmAfCmUfUmUmCmCmAm | 202 |
| siHBe3M3P1 | S | GmAmAmAmGfUmAfUfGfUmCmAmAmCmGmAmAmUmAm | 203 |
| | AS | P1-UmAfUmUmCmGfUmUmGmAmCmAmUmAfCmUfUmUmCmUmUm | 204 |
| siHBe4M3P1 | S | GmAmAmAmGfUmAfUfGfUmCmAmAmCmGmAmAmUmAm | 205 |
| | AS | P1-UmAfUmUmCmGfUmUmGmAmCmAmUmAfCmUfUmUmCmCmAm | 206 |
| siHBe5M1P1 | S | UmGmGmAmAmAmGmUmAfUfGfUmCmAmAmCmGmAmAmUmAm | 207 |
| | AS | P1-UmAfUmUmCmGfUmUmGmAmCmAmUmAfCmUfUmUmCmCmAmUmUm | 208 |
| siHBe6M2P1 | S | UmGmGmAmAmAmGfUmAfUfGfUmCmAmAmCmGmAmAmUmAm | 209 |
| | AS | P1-UmAfUmUmCmGfUmUfGfAmCmAmUmAfCmUfUmUmCmCmAmAmUm | 210 |
| SiHBe7M3P1 | S | UmGmGmAmAmAmGfUmAfUfGfUmCmAmAmCmGmAmAmUmUm | 211 |
| | AS | P1-AmAfUmUmCmGfUmUmGmAmCmAmUmAfCmUfUmUmCmCmAmUmUm | 212 |
| siHBe1M1SP1 | S | GmsAmsAmAmGmUmAfUfGfUmCmAmAmCmGmAmAmUmUm | 213 |
| | AS | P1-AmsAfsUmUmCmGfUmUmGmAmCmAmUmAfCmUfUmUmCmsUmsUm | 214 |
| siHBe2M1SP1 | S | GmsAmsAmAmGmUmAfUfGfUmCmAmAmCmGmAmAmUmUm | 215 |
| | AS | P1-AmsAfsUmUmCmGfUmUmGmAmCmAmUmAfCmUfUmUmCmsCmsAm | 216 |
| siHBe3M1SP1 | S | GmsAmsAmAmGmUmAfUfGfUmCmAmAmCmGmAmAmUmAm | 217 |
| | AS | P1-UmsAfsUmUmCmGfUmUmGmAmCmAmUmAfCmUfUmUmCmsUmsUm | 218 |
| siHBe4M1SP1 | S | GmsAmsAmAmGmUmAfUfGfUmCmAmAmCmGmAmAmUmAm | 219 |
| | AS | P1-UmsAfsUmUmCmGfUmUmGmAmCmAmUmAfCmUfUmUmCmsCmsAm | 220 |
| siHBe1M2SP1 | S | GmsAmsAmAmGfUmAfUfGfUmCmAmAmCmGmAmAmUmUm | 221 |
| | AS | P1-AmsAfsUmUmCmGfUmUfGfAmCmAmUmAfCmUfUmUmCmsUmsUm | 222 |
| siHBe2M2SP1 | S | GmsAmsAmAmGfUmAfUfGfUmCmAmAmCmGmAmAmUmUm | 223 |
| | AS | P1-AmsAfsUmUmCmGfUmUfGfAmCmAmUmAfCmUfUmUmCmsCmsAm | 224 |
| siHBe3M2SP1 | S | GmsAmsAmAmGfUmAfUfGfUmCmAmAmCmGmAmAmUmAm | 225 |
| | AS | P1-UmsAfsUmUmCmGfUmUfGfAmCmAmUmAfCmUfUmUmCmsUmsUm | 226 |
| siHBe4M2SP1 | S | GmsAmsAmAmGfUmAfUfGfUmCmAmAmCmGmAmAmUmAm | 227 |
| | AS | P1-UmsAfsUmUmCmGfUmUfGfAmCmAmUmAfCmUfUmUmCmsCmsAm | 228 |
| siHBe1M3SP1 | S | GmsAmsAmAmGfUmAfUfGfUmCmAmAmCmGmAmAmUmUm | 229 |
| | AS | P1-AmsAfsUmUmCmGfUmUmGmAmCmAmUmAfCmUfUmUmCmsU | 230 |

| | | | |
|---|---|---|---|
| | | msUm | |
| siHBe2M3SP1 | S | GmsAmsAmAmGfUmAfUfGfUmCmAmAmCmGmAmAmUmUm | 231 |
| | AS | P1-AmsAfsUmUmCmGfUmUmGmAmCmAmUmAfCmUfUmUmCmsCmsAm | 232 |
| siHBe3M3SP1 | S | GmsAmsAmAmGfUmAfUfGfUmCmAmAmCmGmAmAmUmAm | 233 |
| | AS | P1-UmsAfsUmUmCmGfUmUmGmAmCmAmUmAfCmUfUmUmCmsUmsUm | 234 |
| siHBe4M3SP1 | S | GmsAmsAmAmGfUmAfUfGfUmCmAmAmCmGmAmAmUmAm | 235 |
| | AS | P1-UmsAfsUmUmCmGfUmUmGmAmCmAmUmAfCmUfUmUmCmsCmsAm | 236 |
| siHBe5M1SP1 | S | UmsGmsGmAmAmAmGmUmAfUfGfUmCmAmAmCmGmAmAmUmAm | 237 |
| | AS | P1-UmsAfsUmUmCmGfUmUmGmAmCmAmUmAfCmUfUmUmCmCmAmsUmsUm | 238 |
| siHBe6M2SP1 | S | UmsGmsGmAmAmAmGfUmAfUfGfUmCmAmAmCmGmAmAmUmAm | 239 |
| | AS | P1-UmsAfsUmUmCmGfUmUfGfAmCmAmUmAfCmUfUmUmCmCmAmsAmsUm | 240 |
| siHBe7M3SP1 | S | UmsGmsGmAmAmAmGfUmAfUfGfUmCmAmAmCmGmAmAmUmUm | 241 |
| | AS | P1-AmsAfsUmUmCmGfUmUmGmAmCmAmUmAfCmUfUmUmCmCmAmsUmsUm | 242 |

Table1F

| Number | | Sequence direction 5' - 3' | SEQ ID NO |
|---|---|---|---|
| siAN1 | S | CCAAGAGCACCAAGAACUA | 243 |
| | AS | UAGUUCUUGGUGCUCUUGGCU | 244 |
| siAN2 | S | AGCCAAGAGCACCAAGAACUA | 245 |
| | AS | UAGUUCUUGGUGCUCUUGGCUUG | 246 |
| siAN1M1 | S | CmCmAmAmGfAmGfCfAfCmCmAmAmGmAmAmCmUmAm | 247 |
| | AS | UmAfGmUmUmCfUmUfGfGmUmGmCmUfCmUfUmGmGmCmUm | 248 |
| siAN2M1 | S | AmGmCmCmAmAmGfAmGfCfAfCmCmAmAmGmAmAmCmUmAm | 249 |
| | AS | UmAfGmUmUmCfUmUfGfGmUmGmCmUfCmUfUmGmGmCmUmUmGm | 250 |
| siAN1M2 | S | CmCmAmAmGfAmGfCfAfCmCmAmAmGmAmAmCmUmAm | 251 |
| | AS | UmAfGmUmUmCfUmUmGmGmUmGmCmUfCmUfUmGmGmCmUm | 252 |
| siAN2M2 | S | AmGmCmCmAmAmGfAmGfCfAfCmCmAmAmGmAmAmCmUmAm | 253 |
| | AS | UmAfGmUmUmCfUmUmGmGmUmGmCmUfCmUfUmGmGmCmUmUmGm | 254 |
| siAN1M3 | S | CmCmAmAmGmAmGfCfAfCmCmAmAmGmAmAmCmUmAm | 255 |
| | AS | UmAfGmUmUmCfUmUmGmGmUmGmCmUfCmUfUmGmGmCmUm | 256 |
| siAN2M3 | S | AmGmCmCmAmAmGmAmGfCfAfCmCmAmAmGmAmAmCmUmAm | 257 |
| | AS | UmAfGmUmUmCfUmUmGmGmUmGmCmUfCmUfUmGmGmCmUmUmGm | 258 |
| siAN1M1S | S | CmsCmsAmAmGfAmGfCfAfCmCmAmAmGmAmAmCmUmAm | 259 |
| | AS | UmsAfsGmUmUmCfUmUfGfGmUmGmCmUfCmUfUmGmGmsCmsUm | 260 |
| siAN2M1S | S | AmsGmsCmCmAmAmGfAmGfCfAfCmCmAmAmGmAmAmCmUmAm | 261 |
| | AS | UmsAfsGmUmUmCfUmUfGfGmUmGmCmUfCmUfUmGmGmCmUmsUmsGm | 262 |
| siAN1M2S | S | CmsCmsAmAmGfAmGfCfAfCmCmAmAmGmAmAmCmUmAm | 263 |
| | AS | UmsAfsGmUmUmCfUmUmGmGmUmGmCmUfCmUfUmGmGmsCmsUm | 264 |
| siAN2M2S | S | AmsGmsCmCmAmAmGfAmGfCfAfCmCmAmAmGmAmAmCmUmAm | 265 |
| | AS | UmsAfsGmUmUmCfUmUmGmGmUmGmCmUfCmUfUmGmGmCmUm | 266 |

| | | | |
|---|---|---|---|
| siAN1M3S | S | CmsCmsAmAmGmAmGfCfAfCmCmAmAmGmAmAmCmUmAm | 267 |
| | AS | UmsAfsGmUmUmCfUmUmGmGmUmGmCmUfCmUfUmGmGmsCmsUm | 268 |
| siAN2M3S | S | AmsGmsCmCmAmAmGmAmGfCfAfCmCmAmAmGmAmAmCmUmAm | 269 |
| | AS | UmsAfsGmUmUmCfUmUmGmGmUmGmCmUfCmUfUmGmGmCmUmsUmsGm | 270 |
| siAN1M1P1 | S | CmCmAmAmGfAmGfCfAfCmCmAmAmGmAmAmCmUmAm | 271 |
| | AS | P1-UmAfGmUmUmCfUmUfGfGmUmGmCmUfCmUfUmGmGmCmUm | 272 |
| siAN2M1P1 | S | AmGmCmCmAmAmGfAmGfCfAfCmCmAmAmGmAmAmCmUmAm | 273 |
| | AS | P1-UmAfGmUmUmCfUmUfGfGmUmGmCmUfCmUfUmGmGmCmUmUmGm | 274 |
| siAN1M2P1 | S | CmCmAmAmGfAmGfCfAfCmCmAmAmGmAmAmCmUmAm | 275 |
| | AS | P1-UmAfGmUmUmCfUmUmGmGmUmGmCmUfCmUfUmGmGmCmUm | 276 |
| siAN2M2P1 | S | AmGmCmCmAmAmGfAmGfCfAfCmCmAmAmGmAmAmCmUmAm | 277 |
| | AS | P1-UmAfGmUmUmCfUmUmGmGmUmGmCmUfCmUfUmGmGmCmUmUmGm | 278 |
| siAN1M3P1 | S | CmCmAmAmGmAmGfCfAfCmCmAmAmGmAmAmCmUmAm | 279 |
| | AS | P1-UmAfGmUmUmCfUmUmGmGmUmGmCmUfCmUfUmGmGmCmUm | 280 |
| siAN2M3P1 | S | AmGmCmCmAmAmGmAmGfCfAfCmCmAmAmGmAmAmCmUmAm | 281 |
| | AS | P1-UmAfGmUmUmCfUmUmGmGmUmGmCmUfCmUfUmGmGmCmUmUmGm | 282 |
| siAN1M1SP1 | S | CmsCmsAmAmGfAmGfCfAfCmCmAmAmGmAmAmCmUmAm | 283 |
| | AS | P1-UmsAfsGmUmUmCfUmUfGfGmUmGmCmUfCmUfUmGmGmsCmsUm | 284 |
| siAN2M1SP1 | S | AmsGmsCmCmAmAmGfAmGfCfAfCmCmAmAmGmAmAmCmUmAm | 285 |
| | AS | P1-UmsAfsGmUmUmCfUmUfGfGmUmGmCmUfCmUfUmGmGmCmUmsUmsGm | 286 |
| siAN1M2SP1 | S | CmsCmsAmAmGfAmGfCfAfCmCmAmAmGmAmAmCmUmAm | 287 |
| | AS | P1-UmsAfsGmUmUmCfUmUmGmGmUmGmCmUfCmUfUmGmGmsCmsUm | 288 |
| siAN2M2SP1 | S | AmsGmsCmCmAmAmGfAmGfCfAfCmCmAmAmGmAmAmCmUmAm | 289 |
| | AS | P1-UmsAfsGmUmUmCfUmUmGmGmUmGmCmUfCmUfUmGmGmCmUmsUmsGm | 290 |
| siAN1M3SP1 | S | CmsCmsAmAmGmAmGfCfAfCmCmAmAmGmAmAmCmUmAm | 291 |
| | AS | P1-UmsAfsGmUmUmCfUmUmGmGmUmGmCmUfCmUfUmGmGmsCmsUm | 292 |
| siAN2M3SP1 | S | AmsGmsCmCmAmAmGmAmGfCfAfCmCmAmAmGmAmAmCmUmAm | 293 |
| | AS | P1-UmsAfsGmUmUmCfUmUmGmGmUmGmCmUfCmUfUmGmGmCmUmsUmsGm | 294 |

Table1G

| Number | | Sequence direction 5' - 3' | SEQ ID NO |
|---|---|---|---|
| siAP1 | S | CAAUAAAGCUGGACAAGAA | 295 |
| | AS | UUCUUGUCCAGCUUUAUUGG | 296 |
| siAP2 | S | CCCAAUAAAGCUGGACAAGAA | 297 |
| | AS | UUCUUGUCCAGCUUUAUUGGGAG | 298 |
| siAP1M1 | S | CmAmAmUmAfAmAfGfCfUmGmGmAmCmAmAmGmAmAm | 299 |
| | AS | UmUfCmUmUmGfUmCfCfAmGmCmUmUfUmAfUmUmGmGmGm | 300 |
| siAP2M1 | S | CmCmCmAmAmUmAfAmAfGfCfUmGmGmAmCmAmAmGmAmAm | 301 |
| | AS | UmUfCmUmUmGfUmCfCfAmGmCmUmUfUmAfUmUmGmGmGmAmGm | 302 |
| siAP1M2 | S | CmAmAmUmAmAmAfGfCfUmGmGmAmCmAmAmGmAmAm | 303 |
| | AS | UmUfCmUmUmGfUmCmCmAmGmCmUmUfUmAfUmUmGmGmGm | 304 |
| siAP2M2 | S | CmCmCmAmAmUmAmAmAfGfCfUmGmGmAmCmAmAmGmAmAm | 305 |
| | AS | UmUfCmUmUmGfUmCmCmAmGmCmUmUfUmAfUmUmGmGmGmAmGm | 306 |
| siAP1M1S | S | CmsAmsAmUmAfAmAfGfCfUmGmGmAmCmAmAmGmAmAm | 307 |
| | AS | UmsUfsCmUmUmGfUmCfCfAmGmCmUmUfUmAfUmUmGmsGmsGm | 308 |
| siAP2M1S | S | CmsCmsCmAmAmUmAfAmAfGfCfUmGmGmAmCmAmAmGmAmAm | 309 |
| | AS | UmsUfsCmUmUmGfUmCfCfAmGmCmUmUfUmAfUmUmGmGmGmsAmsGm | 310 |
| siAP1M2S | S | CmsAmsAmUmAmAmAfGfCfUmGmGmAmCmAmAmGmAmAm | 311 |
| | AS | UmsUfsCmUmUmGfUmCmCmAmGmCmUmUfUmAfUmUmGmsGmsGm | 312 |
| siAP2M2S | S | CmsCmsCmAmAmUmAmAmAfGfCfUmGmGmAmCmAmAmGmAmAm | 313 |
| | AS | UmsUfsCmUmUmGfUmCmCmAmGmCmUmUfUmAfUmUmGmGmGmsAmsGm | 314 |
| siAP1M1P1 | S | CmAmAmUmAfAmAfGfCfUmGmGmAmCmAmAmGmAmAm | 315 |
| | AS | P1-UmUfCmUmUmGfUmCfCfAmGmCmUmUfUmAfUmUmGmGmGm | 316 |
| siAP2M1P1 | S | CmCmCmAmAmUmAfAmAfGfCfUmGmGmAmCmAmAmGmAmAm | 317 |
| | AS | P1-UmUfCmUmUmGfUmCfCfAmGmCmUmUfUmAfUmUmGmGmGmAmGm | 318 |
| siAP1M2P1 | S | CmAmAmUmAmAmAfGfCfUmGmGmAmCmAmAmGmAmAm | 319 |
| | AS | P1-UmUfCmUmUmGfUmCmCmAmGmCmUmUfUmAfUmUmGmGmGm | 320 |
| siAP2M2P1 | S | CmCmCmAmAmUmAmAmAfGfCfUmGmGmAmCmAmAmGmAmAm | 321 |
| | AS | P1-UmUfCmUmUmGfUmCmCmAmGmCmUmUfUmAfUmUmGmGmGmAmGm | 322 |
| siAP1M1SP1 | S | CmsAmsAmUmAfAmAfGfCfUmGmGmAmCmAmAmGmAmAm | 323 |
| | AS | P1-UmsUfsCmUmUmGfUmCfCfAmGmCmUmUfUmAfUmUmGmsGmsGm | 324 |
| siAP2M1SP1 | S | CmsCmsCmAmAmUmAfAmAfGfCfUmGmGmAmCmAmAmGmAmAm | 325 |
| | AS | P1-UmsUfsCmUmUmGfUmCfCfAmGmCmUmUfUmAfUmUmGmGmGmsAmsGm | 326 |
| siAP1M2SP1 | S | CmsAmsAmUmAmAmAfGfCfUmGmGmAmCmAmAmGmAmAm | 327 |
| | AS | P1-UmsUfsCmUmUmGfUmCmCmAmGmCmUmUfUmAfUmUmGmsGmsGm | 328 |
| siAP2M2SP1 | S | CmsCmsCmAmAmUmAmAmAfGfCfUmGmGmAmCmAmAmGmAmAm | 329 |
| | AS | P1-UmsUfsCmUmUmGfUmCmCmAmGmCmUmUfUmAfUmUmGmGmGmsAmsGm | 330 |

Table1H

| Number | | Sequence direction 5' - 3' | SEQ ID NO |
|---|---|---|---|
| FC-siSTAT1 | S | CmsUmsAmGmAmAmAfAfCfUmGmGmAmUmAmAmCmGmUm | 719 |
| | AS | AmsCfsGmUmUmAfUmCmCmAmGmUmUmUfUmCfUmAmGmsCmsCm | 720 |

Among above, S represents a snese strand; AS represents a antisnese strand; C, G, U, and A represent the

base composition of a nucleotide; m represents that the nucleotide adjacent to the left side of the letter m is a 2'-methoxy modified nucleotide; f represents that the nucleotide adjacent to the left side of the letter f is a 2'-fluoro modified nucleotide; s represents that the two nucleotides adjacent to both sides of the letter s are linked by a phosphorothioate linkage; P1 represents that the nucleotide adjacent to the right side of P1 is a 5'-phosphate nucleotide or a 5'-phosphate analogue modified nucleotide. In some embodiments, P1 represents a vinyl phosphate modified nucleotide (expressed as VP in the Examples below), a 5'-phosphate nucleotide (expressed as P in the Examples below) or a phosphorothioate modified nucleotide (expressed as Ps in the Examples below).

Those skilled in the art clearly know that a modified nucleotide can be introduced into the siRNA of the present disclosure by a nucleoside monomer with a corresponding modification. The methods for preparing a nucleoside monomer with a corresponding modification and the methods for introducing a modified nucleotide into a siRNA are also well-known to those skilled in the art. All nucleoside monomers with modifications can be commercially available or prepared by known methods.

## Preparation of drug conjugate

The drug conjugate of the present disclosure can be prepared by any appropriate synthesis routes. The following examples use oligonucleotides as active drugs to illustrate the preparation method of the drug conjugates provided in the present disclosure. Those skilled in the art could expect that other active drugs can be prepared by referring to the following methods, except omitting the process of preparing the nucleotide sequences; alternatively, the following method could be correspondingly changed according to structural characteristics of the specific active drug.

In some embodiments, a method for preparing the drug conjugate comprising: successively linking nucleoside monomers in the direction from 3' terminal to 5' terminal according to the nucleotide type and sequence of the functional oligonucleotides respectively, under the condition of phosphoramidite solid phase synthesis, wherein the step of linking each nucleoside monomer includes a four-step reaction of deprotection, coupling, capping, and oxidation or sulfurization. The method further comprises: replacing the solid phase support with the compound as shown by Formula (111) and linking the first nucleotide to the compound as shown by Formula (111). Alternatively, the method further comprises: after forming the nucleotide sequence linked to the solid phase support, contacting a compound as shown by Formula (101) with the nucleotide sequence linked to solid phase support under coupling reaction condition in the presence of a coupling agent, and performing capping reaction, and then oxidation, sulfurization or borohydride reactions. Optionally, the method futher comprises: further contacting with the compound as shown by Formula (101) for n times (the definition of n is the same as that of Formula (301)); deprotecting the product obtained in the previous step each time; contacting with the compound of Formula (101); performing capping reaction, and then oxidation, sulfurization, or hydroboration reaction.

In some embodiments, the method further comprises the steps of removing the protection group and cleaving the solid phase support, isolation and purification.

In some embodiments, the oligonucleotide is a double-stranded oligonucleotide, the method for preparing the drug conjugate comprises the following steps: contacting a compound as shown by Formula (111) with the first nucleoside monomer at 3' terminal of the sense strand or the antisense strand under coupling reaction condition in the presence of a coupling agent, thereby linking the compound as shown by Formula (111) to the first nucleotide in the sequence; sequentially linking nucleoside monomers in 3' to 5' direction to synthesize the sense or antisense strand of the oligonucleotides according to the desired nucleotide type and sequence of the sense or antisense strand, under the condition for phosphoramidite solid phase synthesis; wherein the compound as shown by Formula (111) is deprotected before being linked to the first nucleoside monomer; and the linking of each nucleoside monomer comprises a four-step reaction of deprotection, coupling, capping, and oxidation or sulfurization; thus obtaining a sense or antisense strand of the nucleic acid; sequentially linking nucleoside monomers in 3' to 5' direction to synthesize the antisense or sense strand of the nucleic acid according to the nucleotide type and sequence of the sense or antisense strand, under the condition for phosphoramidite solid phase synthesis; wherein the linking of each nucleoside monomer includes a four-step reaction of deprotection, coupling, capping, and oxidation or sulfurization; removing the protection group and cleaving the solid phase support; isolating and purifying the sense strand and the antisense strand of the nucleic acid; and annealing.

In some embodiments, the oligonucleotide is a double-stranded oligonucleotide, the method for preparing the drug conjugate comprises the following steps: successively linking nucleoside monomers in 3' to 5' direction to synthesize the sense strand or the antisense strand according to the nucleotide type and sequence of the sense or antisense strand in the double-stranded oligonucleotide; wherein the linking of each nucleoside monomer comprises a four-step reaction of deprotection, coupling, capping, and oxidation or sulfurization, thus obtaining a sense strand linked to the solid phase support and an antisense strand linked to the solid phase support; removeing the hydroxyl protection group of the terminal nucleoside of the sense strand linked to the solid phase support and the antisense strand linked to the solid phase support; contacting the compound as shown by Formula (101) with the sense strand linked to the solid phase support or the antisense strand linked to the solid phase support, under coupling reaction condition in the presence of a coupling

agent, thereby linking the compound as shown by Formula (101) to the sense strand or the antisense strand; removing the protection groups and cleaving the solid phase support; respectively isolating and purifying the sense or antisense strand of the oligonucleotides; and annealing.

In one specific embodiment, the P atom in Formula A59 is linked to 3' terminal of the sense strand of the siRNA, and the method for preparing the drug conjugate of the present disclosure comprises:

(1) removing the hydroxyl protection group $R_8$ in the compound as shown by Formula (111); contacting the compound with a nucleoside monomer to obtain a nucleotide monomer linked to a solid phase support via the compound of the present disclosure, under coupling reaction condition in the presence of a coupling agent;
(2) starting from the nucleoside monomer linked to a solid phase support via the compound of the present disclosure, synthesizing a sense strand of the siRNA in 3' to 5' direction by a phosphoramidite solid phase synthesis method;
(3) synthesizing an antisense strand of the siRNA by a phosphoramidite solid phase synthesis method; and
(4) isolating the sense strand and the antisense strand of the siRNA and annealing the same to obtain the drug conjugate of the present disclosure;

wherein in step (1), the method for removing the protection group $R_8$ in the compound as shown by Formula (111) comprises: contacting a compound as shown by Formula (111) with a deprotection agent under deprotection condition. The deprotection condition comprises a temperature of 0-50°C (in some embodiments, 15-35°C), and a reaction time of 30-300 seconds (in some embodiments, 50-150 seconds). The deprotection agent can be selected from one or more of trifluoroacetic acid, trichloroacetic acid, dichloroacetic acid, and monochloroacetic acid, and in some embodiments, dichloroacetic acid. The molar ratio of the deprotection agent to the compound as shown by Formula (111) is 10:1 - 1000:1, and in some embodiments, 50:1 - 500:1.

The coupling reaction condition and the coupling agent can be any conditions and agents suitable for the above coupling reaction. For the purpose of simplifying the process, in some embodiments, the same condition and agent as those of the coupling reaction in the solid phase synthesis method employed can be used.

Typically, the coupling reaction condition comprises a reaction temperature of 0-50°C, and in some embodiments, 15-35°C. The molar ratio of the compound of Formula (321) to the nucleoside monomer can be 1:1 to 1:50, and in one embodiment, 1:2 to 1:5; the molar ratio of the compound as shown by Formula (321) to the coupling agent can be 1:1 - 1:50, and in some embodiments, 1:3 - 1:10. The reaction time is 200-3,000 seconds, and in some embodiments, 500-1,500 seconds. The coupling agent can be selected from one or more of 1H-tetrazole, 5-ethylthio-1H-tetrazole, and 5-benzylthio-1H-tetrazole, and in some embodiments, 5-ethylthio-1H-tetrazole. The coupling reaction can be performed in an organic solvent. The organic solvent can be selected from one or more of anhydrous acetonitrile, anhydrous DMF, and anhydrous dichloromethane, and in some embodiments, anhydrous acetonitrile. The amount of the organic solvent can be 3-50 L/mol, and in some embodiments, 5-20 L/mol, with respect to the compound as shown by Formula (321).

In step (2), a sense strand S of the drug conjugate is synthesized in a 3' to 5' direction by the phosphoramidite solid phase synthesis method, starting from the nucleoside monomer linked to solid phase support via the compounds of the present disclosure prepared in the above steps. In this case, the compound as shown by Formula (101) is linked to 3' terminal of the resultant sense strand.

Other conditions for the solid phase synthesis in steps (2) and (3), including the deprotection condition for the nucleoside monomer, the type and amount of the deprotection agent, the coupling reaction condition, the type and amount of the coupling agent, the capping reaction condition, the type and amount of the capping agent, the oxidation reaction condition, the type and amount of the oxidation agent, the sulfurization reaction condition, and the type and amount of the sulfurization agent, adopt various agents, amounts, and conditions conventionally used in the art.

Those skilled in the art could readily appreciate that like the nucleoside monomers used in the phosphoramidite solid phase synthesis method, the compound as shown by Formula (101) also has a phosphoramidite group and a hydroxyl protection group, and thus the compound of Formula (101) can be considered as a nucleoside monomer; and can be linked to a solid phase via deprotection, coupling, capping, oxidation or sulfurization reaction by using the well-known phosphoramidite solid phase synthesis method in the art, and then subsequently linked to another compound of Formula (101) or another nucleoside monomer until the nucleotide sequence of the target product is obtained. Correspondingly, in the following description of the reaction involving the compound as shown by Formula (101), when referring to the reactions such as "deprotection", "coupling", "capping", "oxidation", "sulfurization", it should be understood that the reaction conditions and agents involved in the well-known phosphoramidite solid phase synthesis method in the art would also apply to these reactions. Exemplary reaction conditions and agents will be described hereinafter.

In the above-mentioned methods, the solid phase support is a well-known support in the art for solid phase synthesis of a nucleic acid, such as commercially available universal solid phase support (NittoPhase®HL UnyLinker™ 300 Oligonucleotide Synthesis Support, Kinovate Life Sciences, shown by Formula B80):

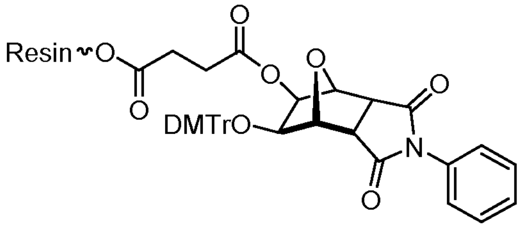

(B80).

In some embodiments, the solid phase synthesis in the above methods can be performed by using the following conditions:
The deprotection condition comprises a temperature of 0-50°C, such as 15-35°C, and a reaction time of 30-300 seconds, such as 50-150 seconds. The deprotection agent can be selected from one or more of trifluoroacetic acid, trichloroacetic acid, dichloroacetic acid, monochloroacetic acid, and in some embodiments, dichloroacetic acid. The molar ratio of the deprotection agent to the protection group 4,4'-dimethoxytrityl on the solid phase support is 2:1 - 100:1, such as 3:1 - 50:1. By such deprotection, reactive free hydroxy groups are obtained on the surface of the solid phase support, on the compound as shown by Formula (101) linked to the solid phase support or on the terminal nucleotide of a nucleic acid sequence linked to the solid phase support via the compound as shown by Formula (101), for facilitating the subsequent coupling reaction.

The coupling reaction condition comprises a temperature of 0-50°C, such as 15-35°C. The molar ratio of the nucleic acid sequence linked to the solid phase support (included in the calculation at the beginning of the solid phase synthesis is the reactive free hydroxyl group formed during the above deprotection step) to the nucleoside monomer or the compound as shown by Formula (101) can be 1:1-1:50, for example, 1:5-1:15. The molar ratio of the nucleic acid sequence linked to the solid phase support to the coupling agent can be 1:1 - 1:100, such as, 1:50 - 1:80. The reaction time can be 200-3000 seconds, for example, 500-1500 seconds. The coupling agent is selected from one or more of 1H-tetrazole, 5-ethylthio-1H-tetrazole, and 5-benzylthio-1H-tetrazole, such as 5-ethylthio-1H-tetrazole. The coupling reaction can be performed in an organic solvent. The organic solvent is selected from one or more of anhydrous acetonitrile, anhydrous DMF, and anhydrous dichloromethane, such as anhydrous acetonitrile. With respect to the compound as shown by Formula (101), the amount of the organic solvent is 3-50 L/mol, such as 5-20 L/mol. By such a coupling reaction, the free hydroxy groups formed in the deprotection reaction react with the phosphoramidite groups on the nucleoside monomers or the compounds shown by Formula (101) to form a phosphate ester linkage.

The capping reaction is used to deactivate any active functional group that does not completely react in the above coupling reaction by excessive capping agent, so as to avoid producing unnecessary by-products in subsequent reactions. The capping reaction condition comprises a temperature of 0-50°C, such as 15-35°C, and a reaction time of 5-500 seconds, such as 10-100 seconds. The capping reaction is carried out in the presence of a capping agent. The capping agent can be the capping agent used in the solid phase synthesis of siRNA, which is well-known to those skilled in the art. In some embodiments, the capping agentcan, for example, be composed of a capping agent A (capA) and a capping agent B (capB). The capA is N-methylimidazole, and in some embodiments, N-methylimidazole is provided as a mixed solution of N-methylimidazole in pyridine/acetonitrile, wherein the volume ratio of pyridine to acetonitrile is 1:10 - 1:1, such as 1:3 - 1:1. In some embodiments, the ratio of the total volume of pyridine and acetonitrile to the volume of N-methylimidazole is 1:1 - 10:1, such as 3:1 - 7:1. The capping agent B is acetic anhydride, and in some embodiments, provided as a solution of acetic anhydride in acetonitrile, wherein the volume ratio of acetic anhydride to acetonitrile is 1:1 - 1:10, such as 1:2 - 1:6. In steps (i) and (ii), the ratio of the volume of the mixed solution of N-methylimidazole in pyridine/acetonitrile to the sum mass of the compound of Formula (101) and the solid phase support is 5 mL/g-50 mL/g, such as 15 mL/g-30 mL/g. The ratio of the volume of the solution of acetic anhydride in acetonitrile to the sum mass of the compound of Formula (101) and the solid phase support is 0.5 mL/g-10 mL/g, such as 1 mL/g-5 mL/g. In some embodiments, the capping agent uses equimolar acetic anhydride and N-methylimidazole. In steps (iii) and (iv), the molar ratio of the total amount of the capping agent to the nucleic acid sequence linked to the solid phase support is 1:100 - 100:1, such as 1:10 - 10:1. In the case where the capping agent uses equimolar acetic anhydride and N-methylimidazole, the molar ratio of acetic anhydride, N-methylimidazole, and the nucleic acid sequence linked to the solid phase support can be 1:1:10 - 10:10:1, such as 1:1:2 - 2:2:1.

When the adjacent nucleosides at the target position in the sequence are linked by a phosphate ester bond, after linking the latter nucleoside monomer via the coupling reaction, the oxidation reaction is conducted under oxidation reaction condition in the presence of an oxidation agent. The oxidation reaction condition comprises a temperature of 0-50°C, such as 15-35°C, and a reaction time of 1-100 seconds, such as 5-50 seconds. The oxidation agent can be, for example, iodine (in some embodiments, provided as iodine water). The molar ratio of the oxidation agent to the nucleic

acid sequence linked to the solid phase support in the coupling step can be 1:1 - 100:1, such as 5:1 - 50:1. In some specific embodiments, the oxidation reaction is performed in a mixed solvent of tetrahydrofuran: water: pyridine = 3:1:1-1:1:3.

When the adjacent nucleosides at the target position in the sequence are linked by a phosphorothioate bonds, after linking the latter nucleoside monomer via the coupling reaction, the sulfurization reaction is conducted under sulfurization reaction condition in the presence of a sulfurization agent. The sulfurization reaction condition comprises a temperature of 0-50°C, such as 15-35°C, and a reaction time of 50-2000 seconds, such as 100-1000 seconds. The sulfurization agent can be, for example, xanthane hydride. The molar ratio of the sulfurization agent to the nucleic acid sequence linked to the solid phase support in the coupling step can be 10:1 - 1000:1, such as 10:1 - 500:1. In some specific embodiments, the sulfurization reaction is performed in a mixed solvent of acetonitrile: pyridine is 1:3-3:1. The previously obtained phosphite linkage is oxidized to a stable phosphate ester or phosphorothioate linkage by the oxidation/sulfurization reaction, thereby completing this phosphoramidite solid phase synthesis cycle.

According to the present disclosure, the method further comprises isolating the sense strand and the antisense strand of the siRNA after linking all nucleoside monomers and before the annealing. Methods for isolation are well known to those skilled in the art and generally comprise cleaving the synthesized nucleotide sequence from the solid phase support, removing the protection groups on the bases, phosphate groups, and ligands, purifying and desalting.

The conventional cleavage and deprotection methods in the synthesis of siRNAs can be used to cleave the synthesized nucleotide sequence from the solid phase support, and remove the protecting groups on the bases, phosphate groups and ligands. For example, the resultant nucleotide sequence linked to the solid phase support is contacted with concentrated aqueous ammonia; during deprotection, the protection groups in groups A51-A59 are removed and $A_0$ is converted to A, while the nucleotide sequence linked to the compounds of the present disclosure is cleaved from the solid phase support. The amount of the concentrated aqueous ammonia can be 0.2 mL/μmol-0.8 mL/μmol with respect to the target siRNA sequence.

When there is at least one 2'-O-TBDMS protection on the synthesized nucleotide sequence, the method further comprises contacting the nucleotide sequence removed from the solid phase support with triethylamine trihydrofluoride to remove the 2'-O-TBDMS protection. Here, the resultant target siRNA sequence comprises the corresponding nucleoside having a free 2'-hydroxy. The amount of pure triethylamine trihydrofluoride can be 0.4 mL/μmol - 1.0 mL/μmol with respect to the target siRNA sequence.

Methods for purification and desalination are well-known to those skilled in the art. For example, nucleic acid purification can be performed using a preparative ion chromatography purification column with a gradient elution of NaBr or NaCl; after collecting and combining the product, the desalination can be performed using a reverse phase chromatography purification column.

During synthesis, the purity and molecular weight of the nucleic acid sequence may be determined at any time, in order to better control the synthesis quality. Such determination methods are well-known to those skilled in the art. For example, the purity of the nucleic acid may be determined by ion exchange chromatography, and the molecular weight may be determined by liquid chromatography-mass spectrometry (LC-MS).

Methods for annealing are also well-known to those skilled in the art. For example, the synthesized sense strand (S strand) and the antisense strand (AS strand) can be mixed in water for injection at an equimolar ratio, heated to 70-95°C, and then cooled at room temperature to form a double-stranded structure via hydrogen bond. Thus, the drug conjugate of the present disclosure can be obtained.

After obtaining the drug conjugate of the present disclosure, in some embodiment, the synthesized drug conjugate can also be characterized by the means (such as molecular weight detection) using the methods (such as LC-MS), to confirm that the synthesized drug conjugate is the designed drug conjugate of interest, and the sequence of the synthesized oligonucleotide is the sequence of the desired oligonucleotide sequence to be synthesized; for example, is one of the sequences listed in Tables 1 above.

In some embodiments, the siRNA in the drug conjugate of the present disclosure is the following first siRNA.

Each nucleotide in the first siRNA is independently a modified or unmodified nucleotide; the siRNA comprises a sense strand and an antisense strand, wherein the sense strand comprises a nucleotide sequence 1, and the antisense strand comprises a nucleotide sequence 2; the nucleotide sequence 1 and the nucleotide sequence 2 are at least partly reverse complementary to form a double-stranded region; the nucleotide sequence 1 and the nucleotide sequence as shown in SEQ ID NO: 717 have an equal length and no more than 3 nucleotide differences; and the nucleotide sequence 2 and the nucleotide sequence as shown in SEQ ID NO: 718 have an equal length and no more than 3 nucleotide differences:

5'- CCUUGAGGCAUACUUCAAZ -3' (SEQ ID NO: 717);
5'- Z'UUGAAGUAUGCCUCAAGG -3' (SEQ ID NO: 718);

wherein Z is A, Z' is U; the nucleotide sequence 1 comprises a nucleotide $Z_A$ at the position corresponding to Z; the

nucleotide sequence 2 comprises a nucleotide $Z'_B$ at the position corresponding to Z'; the $Z'_B$ is the first nucleotide from 5' terminal of the antisense strand.

In some embodiments, the nucleotide sequence 1 and the nucleotide sequence as shown in SEQ ID NO: 717 have no more than 1 nucleotide difference, and/or the nucleotide sequence 2 and the nucleotide sequence as shown in SEQ ID NO: 718 have no more than 1 nucleotide difference.

In some embodiments, the nucleotide difference between the nucleotide sequence 2 and the nucleotide sequence as shown in SEQ ID NO: 718 includes a difference at the position of $Z'_B$, and $Z'_B$ is selected from A, C, or G. In some embodiments, the nucleotide difference is a difference at the position of $Z'_B$, and $Z'_B$ is selected from A, C, or G, and ZA is a nucleotide complementary to Z'B. These nucleotide differences will not significantly reduce the ability of the drug conjugates to inhibit the target gene, and these drug conjugates comprising the specific nucleotide differences are also within the protection scope of the present disclosure.

In some embodiments, the nucleotide sequence 1 is basically reverse complementary, substantially reverse complementary, or completely reverse complementary to the nucleotide sequence 2.

In some embodiments, the sense strand further comprises a nucleotide sequence 3; the antisense strand further comprises a nucleotide sequence 4; the nucleotide sequence 3 and the nucleotide sequence 4 independently of one another have a length of 1 - 4 nucleotides, and the positions of the nucleotide sequence 3 and the nucleotide sequence 4 correspond to each other. In some embodiments, the nucleotide sequence 4 is at least partially complementary to the nucleotides at the corresponding positions in the target mRNA. In some embodiments, the nucleotide sequence 4 is completely complementary to the nucleotides at the corresponding positions in the target mRNA.

In some embodiments, the nucleotide sequence 3 is linked to 5' terminal of the nucleotide sequence 1, and the nucleotide sequence 4 is linked to 3' terminal of the nucleotide sequence 2. In some embodiments, the nucleotide sequence 3 and the nucleotide sequence 4 have an equal length and are reverse complementary to each other. Therefore, the sense strand and the antisense strand can have a length of 19-23 nucleotides.

In some embodiments, the nucleotide sequence 3 and the nucleotide sequence 4 both have a length of 1 nucleotide. The base of the nucleotide sequence 3 is A; in this case, the double-stranded region can have a length of 20 nucleotides, i.e., the length ratio of the sense strand to the antisense strand in the siRNA of the present disclosure can be 20/20.

The nucleotide sequence 3 and the nucleotide sequence 4 both have a length of 2 nucleotides. In the direction from 5' terminal to 3' terminal, the bases of the nucleotide sequence 3 are G and A in succession; in this case, the double-stranded region can have a length of 21 nucleotides, i.e., the length ratio of the sense strand to the antisense strand in the siRNA of the present disclosure can be 21/21.

The nucleotide sequence 3 and the nucleotide sequence 4 both have a length of 3 nucleotides. In the direction from 5' terminal to 3' terminal, the bases of the nucleotide sequence 3 are C, G and A in succession; in this case, the double-stranded region can have a length of 22 nucleotides, i.e., the length ratio of the sense strand to the antisense strand in the siRNA of the present disclosure can be 22/22.

The nucleotide sequence 3 and the nucleotide sequence 4 both have a length of 4 nucleotides. In the direction from 5' terminal to 3' terminal, the bases of the nucleotide sequence 3 are C, C, G and A in succession; in this case, the double-stranded region may have a length of 23 nucleotides, i.e., the length ratio of the sense strand to the antisense strand in the siRNA of the present disclosure can be 23/23.

In some specific embodiments, the nucleotide sequence 3 has a length of 2 nucleotides. In the direction from 5' terminal to 3' terminal, the bases of the nucleotide sequence 3 are G and A in succession.

In some specific embodiments, in the above groups, the nucleotide sequence 3 and the nucleotide sequence 4 have an equal length and are completely reverse complementary to each other. Thus, once the bases of the nucleotide sequence 3 are provided, the bases of the nucleotide sequence 4 are also determined.

In some embodiments, the siRNA of the present disclosure further comprises a nucleotide sequence 5, which has a length of 1-3 nucleotides and is linked to 3' terminal of the antisense strand, thereby forming a 3' overhang of the antisense strand. In some embodiments, the nucleotide sequence 5 has a length of 1 or 2 nucleotides. As such, the length ratio of the sense strand to the antisense strand in the siRNA of the present disclosure can be 19/20, 19/21, 20/21, 20/22, 21/22, 21/23, 22/23, 22/24, 23/24, or 23/25.

In some embodiments, the nucleotide sequence 5 has a length of 2 nucleotides; and in the direction from 5' terminal to 3' terminal, the nucleotide sequence 5 comprises 2 consecutive thymidine deoxyribonucleotides, 2 consecutive uridine ribonucleotides or 2 nucleotides complementary to the target mRNA. Thus, in some embodiments, the length ratio of the sense strand to the antisense strand in the siRNA of the present disclosure is 19/21 or 21/23. Here, the siRNA of the present disclosure exhibits better silencing activity against HBV mRNA and/or activity of effectively reducing the expression of the surface antigen HBsAg.

In some embodiments, the nucleotide sequence 1 comprises the nucleotide sequence as shown by SEQ ID NO: 1, and the nucleotide seqeucne 2 comrpises the nucleotide sequence as shown by SEQ ID NO: 2:

5'- CCUUGAGGCAUACUUCAAZ -3' (SEQ ID NO: 1);
5'- Z'UUGAAGUAUGCCUCAAGGUU -3' (SEQ ID NO: 2).

In some embodiments, the siRNA of the present disclosure is siHBa1 or SiHBa2:

siHBa1
Sense strand: 5'- CCUUGAGGCAUACUUCAAZ -3' (SEQ ID NO: 1),
Antisense strand: 5'- Z'UUGAAGUAUGCCUCAAGGUU -3' (SEQ ID NO: 2),
siHBa2
Sense strand: 5'- GACCUUGAGGCAUACUUCAAZ -3' (SEQ ID NO: 2),
Antisense strand: 5'- Z'UUGAAGUAUGCCUCAAGGUCGG -3' (SEQ ID NO: 4),
wherein Z is A, Z' is U.

As mentioned above, each nucleotide in the first siRNA is independently a modified or unmodified nucleotide. In some embodiments, the nucleotides in the first siRNA are unmodified nucleotides; in some embodiments, some or all nucleotides in the first siRNA are modified nucleotides, wherein the modifications in the nucleotides would not cause significant impairment or loss of the function of the drug conjugate of the present disclosure for inhibiting the expression of HBV gene. In some embodiments, the nucleotides in the first siRNA are modified as mentioned above. In some embodiments, the first siRNA can be any siRNA as listed in Table 1A.

Use **of the drug conjugate of the present disclosure**

The drug conjugate of the present disclosure has excellent targeting specificity, and therefore can efficiently deliver the conjugated functional oligonucleotide to a target organ or tissue, thereby effectively regulating intracellular gene expression. Thus, the drug conjugate of the present disclosure has a wide application prospect.

According to one embodiment of the present disclosure, the present disclosure provides use of the drug conjugate of the present disclosure in the manufacture of a medicament for the treatment and/or prevention of a pathological condition or disease caused by the expression of a gene in a cell. The gene can be an endogenous gene expressed in a cell or a pathogen gene amplified in a cell. In some embodiments, the gene is selected from ApoB, ApoC, ANGPTL3, PCSK9, SCD1, TIMP-1, Col1A1, FVII, STAT3, p53, HBV, HCV, and the like. In some embodiments, the gene is selected from hepatitis B virus gene, angiopoietin-like protein 3 gene, or apolipoprotein C3 gene. Accordingly, the disease is selected from chronic liver diseases, hepatitis, hepatic fibrosis diseases, hepatic proliferative diseases and dyslipidemia. In some embodiments, the dyslipidemia is hypercholesterolemia, hypertriglyceridemia or atherosclerosis. In some embodiments, the gene is selected from Signal Transducer and Activator of Transcription 3 (STAT3) gene.

According to one embodiment of the present disclosure, the present disclosure provides a method for treating a pathological condition or disease caused by the expression of a gene in a cell, comprising administering the drug conjugate of the present disclosure to a patient. In some embodiments, the gene is selected from ApoB, ApoC, ANGPTL3, PCSK9, SCD1, TIMP-1, Col1A1, FVII, STAT3, p53, HBV, HCV, and the like. In some embodiments, the gene is selected from hepatitis B virus gene, angiopoietin-like protein 3 gene, apolipoprotein C3 gene, or Signal Transducer and Activator of Transcription 3 (STAT3) gene. Accordingly, the disease is selected from chronic liver diseases, hepatitis, hepatic fibrosis diseases, hepatic proliferative diseases, dyslipidemia and tumors. In some embodiments, the dyslipidemia is hypercholesterolemia, hypertriglyceridemia or atherosclerosis.

According to another embodiment of the present disclosure, the present disclosure provides a method for regulating gene expression in a cell, wherein the regulating comprising inhibiting or enhancing the expression of the gene, the method comprising contacting the drug conjugate of the present disclosure with the cell.

By administering the drug conjugate of the present disclosure to a patient in need thereof, the purpose of the prevention and/or treatment of a pathological condition or disease caused by the expression of a gene in a cell can be achieved by a mechanism that regulates gene expression.

Thus, the drug conjugate of the present disclosure can be used for the prevention and/or treatment of the pathological condition or disease, or for the preparation of a medicament for the prevention and/or treatment of the pathological condition or disease.

As used herein, the term "administration/administer" refers to placing the drug conjugate into a patient's body by a method or a route where at least partly locates the drug conjugate at a desired site to achieve a desired effect. The administration routes suitable for the method of the present disclosure include topical administration and systemic administration. In general, topical administration results in the delivery of more drug conjugates to a particular site as compared with the whole body of the patient; whereas systemic administration results in the delivery of the drug conjugate to substantially the whole body of the patient.

In some embodiments, the administration to a patient can be achieved by any suitable routes known in the art,

including but not limited to, oral or parenteral route, including intravenous administration, intramuscular administration, subcutaneous administration, transdermal administration, intratracheal administration (aerosol), pulmonary administration, nasal administration, rectal administration, and topical administration (including buccal administration and sublingual administration). The administration frequency can be once or more times daily, weekly, monthly, or yearly.

The dose of the drug conjugate of the present disclosure can be a conventional dose in the art, which can be determined according to various parameters, especially age, weight and gender of a patient. Toxicity and efficacy can be determined in cell cultures or experimental animals by standard pharmaceutical procedures, for example, by determining LD50 (the lethal dose that causes 50% population death) and ED50 (the dose that can cause 50% of the maximum response intensity in a quantitative response, and that causes 50% of the experimental subjects to have a positive response in a qualitative response). The dose range for human use can be derived based on the data obtained from cell culture analysis and animal studies.

When the drug conjugate of the present disclosure is administered, for example, to male or female C57BL/6J or C3H/HeNCrlVr mice of 6 - 12 weeks old and 18 - 25 g body weight, for the drug conjugate formed by the functional oligonucleotide and the pharmaceutically acceptable compound as shown by Formula (101), the amount of the oligonucleotide can be 0.001 to 100 mg/kg body weight, in some embodiments 0.01 - 50 mg/kg body weight, in some embodiments 0.05 - 20 mg/kg body weight, and in one specific embodiment 0.1 - 10 mg/kg body weight, as calculated based on the amount of the oligonucleotide in the drug conjugate. When administering the drug conjugate of the present disclosure, the above amounts can be referred to.

In addition, the purpose of regulating the expression of a gene in a cell can also be achieved through the mechanism of gene expression regulation by introducing the drug conjugate of the present disclosure into the cell with abnormal gene expression,. In some embodiments, the cells are hepatitis cells, and in some embodiments HepG2.2.15 cells. In some embodiments, the cells can be selected from hepatoma cell lines such as Hep3B, HepG2 and Huh7, or isolated primary hepatocytes, and in some embodiments Huh7 hepatoma cells.

In the case where the expression of the gene in a cell is inhibited by the method provided by the present disclosure, the amount of the functional oligonucleotide in the drug conjugate provided can be readily determined by those skilled in the art based on the desired effects. For example, in some embodiments, the drug conjugate is a drug conjugate, and the amount of the siRNA in the drug conjugate provided is such an amount that is sufficient to reduce the expression of the target gene and results in an extracellular concentration of 1 pM to 1 μM, or 0.01 nM to 100 nM, or 0.05 nM to 50 nM or to about 5 nM on the surface of the cell. The amount required to achieve this topical concentration will vary with various factors, including the delivery method, the delivery site, the number of cell layers between the delivery site and the cells or tissues, the delivery route (topical or systemic), etc. The concentration at the delivery site can be significantly higher than that on the surface of the cells or tissues.

## Kit

The present disclosure provides a kit comprising the drug conjugate as described above.

In some embodiments of the kit of the present disclosure, the drug conjugate can be kept in a container, while the kit may or may not comprise at least another container for providing or not providing pharmaceutically acceptable excipients. In addition to the drug conjugate and optional pharmaceutically acceptable excipients, the kit may further comprise additional ingredients, such as stabilizers or preservatives. The additional ingredients may be comprised in the kit, but present in a container other than that for providing the drug conjugate and optional pharmaceutically acceptable excipients. In these embodiments, the kit can comprise an instruction for mixing the drug conjugate with pharmaceutically acceptable excipients (if any) or other ingredients.

In the kit of the present disclosure, the drug conjugate and optional pharmaceutically acceptable excipients may be provided in any form, e.g., in a liquid form, a dry form, or a lyophilized form. In some embodiments, the siRNA conjugate and optional pharmaceutically acceptable excipients are substantially pure and/or sterile. Sterile water may optionally be provided in the kits of the present disclosure.

In some embodiments, the drug conjugate provided in the present disclosure can have higher stability, lower toxicity, and/or higher activity *in vivo.* In some embodiments, the siRNA, the siRNA composition or the drug conjugate provided in the present disclosure exhibits an inhibition rate of the target gene expression of at least 20%, 30%, 40%, 50%, 60%, 70%, 80%, 90%, or 95% *in vivo.* In some embodiments, the siRNA, the siRNA composition or the drug conjugate provided in the present disclosure exhibits an inhibition rate of the hepatic target gene expression of at least 20%, 30%, 40%, 50%, 60%, 70%, 80%, 90%, or 95% *in vivo.* In some embodiments, the siRNA, the siRNA composition or the drug conjugate provided in the present disclosure exhibits an inhibition rate of the hepatic target gene expression of at least 20%, 30%, 40%, 50%, 60%, 70%, 80%, 90%, or 95% in an animal model *in vivo.* In some embodiments, the siRNA, the siRNA composition or the drug conjugate provided in the present disclosure exhibits an inhibition rate of the target surface antigen expression of at least 20%, 30%, 40%, 50%, 60%, 70%, 80%, 90%, or 95% *in vivo.* In some embodiments, the siRNA, the composition or the drug conjugate provided in the present disclosure does not exhibit

significant off-target effect. The off-target effect can be, for example, inhibition of normal expression of a gene that is no the target gene. The off-target effect is considered as non-significant if the binding/inhibition of the expression of the off-target gene is 50%, 40%, 30%, 20%, or 10% lower than that of the on-target gene effect.

In some embodiments, the drug conjugate provided in the present disclosure has a lower animal level toxicity.

In some embodiments, the drug conjugate provided in the present disclosure can remain undegraded in human plasma over a period of up to 72 hours, exhibiting excellent stability in human plasma.

In some embodiments, the drug conjugate provided in the present disclosure can remain undegraded in cynomolgus monkey plasma over a period of up to 72 hours, exhibiting excellent stability in monkey plasma.

In some embodiments, the drug conjugate provided in the present disclosure exhibits satisfactory stability in both human-derived and murine-derived lysosomal lysates, remaining undegraded for at least 24 hours.

In some embodiments, the drug conjugate provided in the present disclosure can be specifically and significantly enriched in liver and remain stable, with a high targeting activity.

In some embodiments, the drug conjugate provided in the present disclosure exhibits high *in vivo* inhibitory activity against the target mRNA in mice in many experiments at different test time points.

In some embodiments, the drug conjugate provided in the present disclosure exhibits persistent and efficient inhibitory efficiency against the target mRNA in different animal models, and exhibits a regular dose dependence.

In some embodiments, the drug conjugate provided in the present disclosure has a higher activity *in vitro* and also a low off-target effect.

Hereinafter, the present disclosure will be further illustrated by way of Preparation Examples and Examples, but is not limited thereto in any respect.

## Example

The present disclosure will be described in detail with reference to the examples. Unless otherwise specified, the reagents and culture media used in the following examples are all commercially available, and the procedures used such as nucleic acid electrophoresis, real-time PCR, and the like are all performed according to the protocols well known to those skilled in the art. For example, they can be performed according to the method described in Molecular Cloning (Cold Spring Harbor LBboratory Press (1989)).

HepG2.2.15 cells were purchased from ATCC, and cultured in DMEM complete medium (Gibco) containing 10% fetal bovine serum (FBS, Gibco), 2 mM L-glutamine (Gibco) and 380 μg/mL G418 at 37°C in an incubator containing 5% $CO_2$/95% air.

Huh7 cells were purchased from ATCC, and cultured in DMEM complete medium (Gibco) containing 10% fetal bovine serum (FBS, Gibco), 2 mM L-glutamine (Gibco) and 380 μg/mL G418 at 37°C in an incubator containing 5% $CO_2$/95% air.

HBV transgenic mice C57BL/6J-Tg(Alb1HBV)44Bri/J: purchased from Department of Laboratory Animal Science, Peking University Health Science Center. Mice with S/COV > 10 were selected before the experiment. Hereinafter, the transgenic mice were sometimes abbreviated as 44Bri model mice;

HBV transgenic mice: named as M-Tg HBV, purchased from the Animal Department of Shanghai Public Health Center. The preparation method of the transgenic mice was as described in Ren J et al., J. Medical Virology. 2006, 78: 551-560. Hereinafter, the transgenic mice were sometimes abbreviated as M-Tg model mice;

AAV-HBV transgenic mice: AAV-HBV model mice were prepared according to the method in the literature (Dong Xiaoyan et al., Chin J Biotech 2010, May 25; 26 (5): 679-686). The rAAV8-1.3HBV, type D (ayw) virus (purchased from Beijing FivePlus Molecular Medicine Institute Co. Ltd., $1\times10^{12}$ viral genome (v.g.)/mL, Lot No.2016123011) was diluted to $5\times10^{11}$ v.g./mL with sterilized PBS, and 200 μL of diluted rAAV8-1.3HBV was injected into each mouse (that is, each mouse was injected with $1\times10^{10}$ v.g.). On day 28 after virus injection, orbital blood (about 100 μL) was collected from all mice for collecting serum for the detection of HBsAg and HBV DNA. Hereinafter, the transgenic mice were sometimes abbreviated as AAV-HBV model mice;

Low-concentration AAV-HBV transgenic mice: the modeling method used is substantially identical with that mentioned above, except that the virus was diluted to $1\times10^{11}$ (v.g.)/mL with sterilized PBS before the experiment, and 100 μL of virus was injected into each mouse ,that is, each mouse was injected with $1\times10^{10}$ v.g,Hereinafter, the transgenic mice were sometimes abbreviated as AAV-HBV low-concentration model mice.

HBV transgenic mice C57BL/6-HBV: strain name: B6-Tg HBV/Vst (1.28 copy, genotype A), purchased from Beijing Vitalstar Biotechnology Co., Ltd. Mice with COI > $10^4$ were selected before the experiment. Hereinafter, the transgenic mice were sometimes abbreviated as the 1.28 copy model mice.

When the drug conjugates synthesized in Preparation Example 7-Preparation Example 8 below are used to transfect cells, Lipofectamine™2000 (Invitrogen) is used as a transfection reagent. The specific procedures could refer to the instruction provided by the manufacturer.

Unless otherwise specified, ratios of the reagents provided below are all calculated by volume ratio (v/v).

**Preparation Example 1 Synthesis of the compound as shown by N-6**

In this preparation example, a compound as shown by Formula (N-6) was synthesized according to the following method:

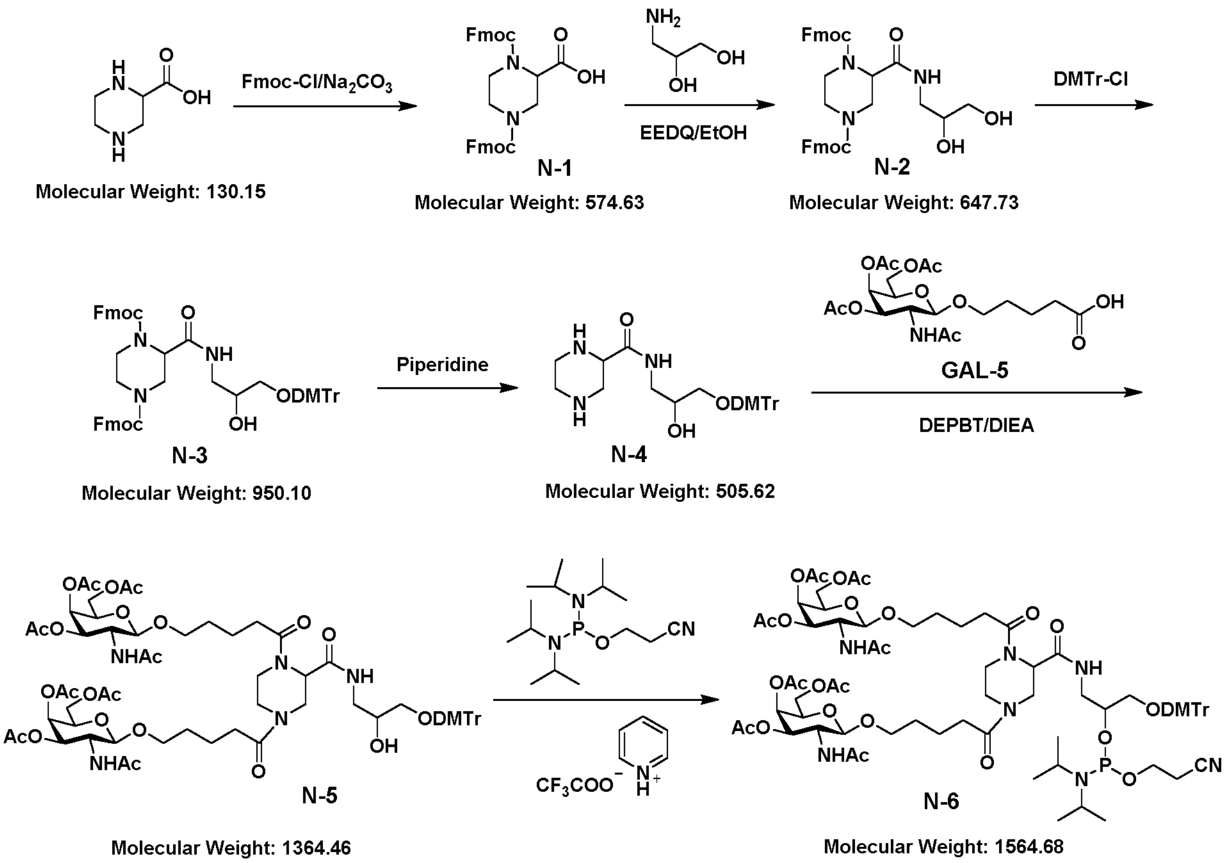


(1-1) Synthesis of GAL-5 (a terminal segment of the conjugation molecule)

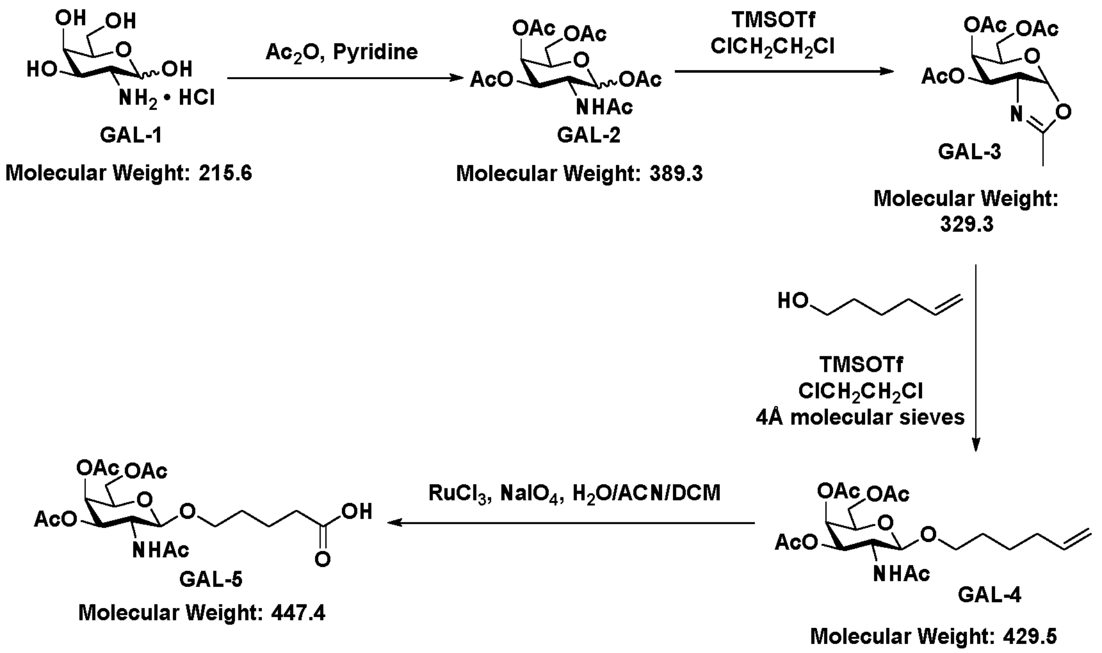


GAL-1
Molecular Weight: 215.6
GAL-2
Molecular Weight: 389.3
GAL-3
Molecular Weight: 329.3
GAL-4
Molecular Weight: 429.5
GAL-5
Molecular Weight: 447.4

(1-1a) Synthesis of GAL-2

20.0 g of GAL-1 (N-acetyl-D-galactosamine hydrochloride, CAS No.: 1772-03-8, purchased from Ning Bo hongxiang bio-chem Co., Ltd., 92.8 mmol) was dissolved in 200 mL of anhydrous pyridine, to which 108 ml of acetic anhydride (purchased from Enox Inc., 1113 mmol) was added in an ice water bath to react under stirring at room temperature for 24 hours. The resultant reaction solution was poured into 2 L of ice water and subjected to suction filtration under reduced pressure. The residue was washed with 500 mL of ice water, and then added with a mixed solvent of acetonitrile/toluene (v/v ratio of acetonitrile: toluene = 1:1) until completely dissolved. The solvent was evaporated to dryness to give 30.6 g of product GAL-2 as a white solid. $^1$H NMR (400 MHz, Chloroform-d) $\delta$ 5.72 (d, J = 8.8 Hz, 1H), 5.40 (m, 2H), 5.11 (dd, J = 11.3, 3.3 Hz, 1H), 4.47 (q, J = 10.7, 10.0 Hz, 1H), 4.24 - 4.10 (m, 2H), 4.04 (t, J = 6.5 Hz, 1H), 2.20 (s, 3H), 2.16 (s, 3H), 2.07 (s, 3H), 2.05 (s, 3H), 1.97 (s, 3H).

(1-1b) Synthesis of GAL-3

GAL-2 (11.5 g, 29.5 mmol) obtained in step (1-1a) was dissolved in 70 mL of anhydrous 1,2-dichloroethane, to which 6.4 mL of trimethylsilyl trifluoromethanesulfonate (TMSOTf, CAS No.: 27607-77-8, purchased from Macklin Inc., 35.5 mmol) was added in an ice water bath under nitrogen atmosphere to react at room temperature overnight.

The resultant reaction solution was added with 100 mL of saturated aqueous sodium bicarbonate solution, and stirred for 10 minutes. The organic phase was isolated. The aqueous phase remained was extracted twice, each with 100 mL of dichloroethane. The organic phases were combined and washed with 100 mL of saturated aqueous sodium bicarbonate solution and 100 mL of saturated brine, respectively. The organic phase was isolated and dried with anhydrous sodium sulfate. The solvent was evaporated to dryness under reduced pressure to give 10.2 g of product GAL-3 as a light yellow viscous syrup. $^1$H NMR (400 MHz, Chloroform-d) $\delta$ 5.99 (d, J = 6.8 Hz, 1H), 5.46 (t, J = 3.0 Hz, 1H), 4.90 (dd, J = 7.5, 3.3 Hz, 1H), 4.30 - 4.15 (m, 2H), 4.10 (m, 1H), 4.02 - 3.94 (m, 1H), 2.12 (s, 3H), 2.07 (d, J = 0.9 Hz, 6H), 2.05 (d, J = 1.2 Hz, 3H).

(1-1c) Synthesis of GAL-4

GAL-3 (9.5 g, 28.8 mmol) obtained in step (1-1b) was dissolved in 50 mL of anhydrous 1,2-dichloroethane, added with 10 g of dry 4Å molecular sieve powder followed by 3.2 g of 5-hexen-1-ol (CAS No.: 821-41-0, purchased from Adamas-beta Inc., 31.7 mmol), and stirred at room temperature for 30 minutes. To which 2.9 mL of TMSOTf (14.4 mmol) was added in an ice bath under nitrogen atmosphere to react under stirring at room temperature overnight. The 4Å molecular sieve powder was removed by filtration. The filtrate was added with 100 mL of saturated aqueous sodium bicarbonate solution and stirred for 10 minutes. The organic phase was isolated. The aqueous phase remained was extracted once with 100 mL of dichloroethane. The organic phases were combined and washed with 100 mL of saturated aqueous sodium bicarbonate solution and 100 mL of saturated brine, respectively. The organic phase was isolated and

dried with anhydrous sodium sulfate. The solvent was evaporated to dryness under reduced pressure to give 13.3 g of product GAL-4 as a yellow syrup, which was directly used in the next oxidation reaction without purification.

(1-1d) Synthesis of GAL-5

GAL-4 obtained according to the method described in step (1-1c) (17.5 g, 40.7 mmol, obtained by combining two batches of products) was dissolved in a mixed solvent of 80 mL of dichloromethane and 80 mL of acetonitrile, added with 130 mL of deionized water and 34.8 g of sodium periodate (CAS No.: 7790-28-5, purchased from Aladdin Inc., 163 mmol) respectively, and stirred in an ice water bath for 10 minutes. Ruthenium trichloride (CAS No.: 14898-67-0, purchased from Energy Chemical, 278 mg, 1.34 mmol) was added to react at room temperature overnight, and the system temperature was controlled not to exceed 30°C. The resultant reaction solution was diluted by adding 300 mL of water under stirring, and adjusted to a pH of about 7.5 by adding saturated sodium bicarbonate. The organic phase was isolated and discarded. The aqueous phase remained was extracted three times, each with 200 mL of dichloromethane, and the organic phase was discarded. The pH of aqueous phase was adjusted to about 3 with citric acid solids and extracted three times, each with 200 mL of dichloromethane, and the organic phases were combined and dried with anhydrous sodium sulfate. The solvent was evaporated to dryness under reduced pressure to give 6.5 g of product GAL-5 as a white foamy solid. $^1H$ NMR (400 MHz, DMSO-d6) δ 12.01 (s, 1H), 7.82 (d, J = 9.2 Hz, 1H), 5.21 (d, J = 3.4 Hz, 1H), 4.95 (dd, J = 11.2, 3.4 Hz, 1H), 4.48 (d, J = 8.5 Hz, 1H), 4.02 (s, 3H), 3.87 (dt, J = 11.3, 8.9 Hz, 1H), 3.75 - 3.66 (m, 1H), 3.46 - 3.36 (m, 1H), 2.19 (t, J = 7.1 Hz, 2H), 2.10 (s, 3H), 2.00 (s, 3H), 1.89 (s, 3H), 1.77 (s, 3H), 1.55 - 1.43 (m, 4H).

Hereinafter, a compound as shown by Formula (N-6) was synthesized by the following process route using the Compound GAL-5 obtained accordig to the above method:

(1-2) Synthesis of N-1

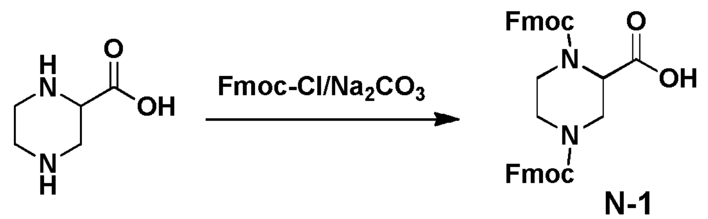


N-1

Piperazine-2-carboxylic acid (33.2 g, 163.3 mmol, purchased from Alfa Aesar (China) Chemical Co., Ltd., CAS No.: 2762-32-5) was dissolved in 200 mL of dioxane and 50 mL of 10% aqueous sodium carbonate solution, to which 9-fluorenylmethyl chloroformate (100.0 g, 391.9 mmol) dissolved in 50 mL of dioxane was added in an ice water bath to react under stirring at room temperature for 24 hours. The resultant reaction solution was poured into water, and subjected to suction filtration under reduced pressure. The residue was acidified with acidic aqueous solution, and then extracted once with dichloromethane. The organic phase was washed once with saturated brine, and then dried. The solvent was evaporated to dryness. The residue was purified by column chromatography (200-300 mesh normal phase silica gel, with an elution of a mixed solution of petroleum ether: ethyl acetate: dichloromethane =1:1:1). The target product was collected, concentrated, and dissolved in dichloromethane. The resultant solution was washed with acidic aqueous solution until the pH of the aqueous phase was 5, washed once with saturated brine, and then dried. The solvent was evaporated to dryness (the pH of the aqueous phase was 6) to give 83.0 g of product N-1. $^1H$ NMR (400 MHz, DMSO-d6) δ 7.85 - 7.72 (m, 6H), 7.60 - 7.39 (m, 10H), 5.09 (t, J = 7.0 Hz, 1H), 4.70 (d, J = 5.7 Hz, 4H), 4.23 (td, J = 12.1, 11.5, 6.3 Hz, 3H), 4.16 - 4.04 (m, 1H), 4.04 - 3.95 (m, 1H), 3.82 (dd, J = 12.4, 7.0 Hz, 1H), 3.58 (dt, J = 12.0, 6.8 Hz, 1H), 3.48 (dt, J = 12.1, 6.8 Hz, 1H). MS m/z: $C_{35}H_{29}N_2O_6$, [M-H]-, calculated: 573.20, measured: 573.32.

(1-3) Synthesis of N-2

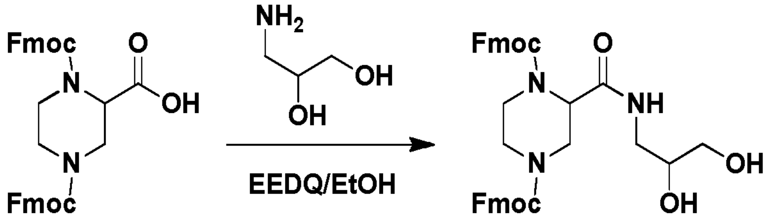


N-1 N-2

N-1 (13.9 g, 24.28 mmol) obtained in step (1-2), 3-amino-1,2-propanediol (2.5 g, 27.4 mmol) and 2-ethoxy-1-ethoxycarbonyl-1,2-dihydroquinoline (7.3 g, 29.6 mmol) were added to 120 mL of ethanol to react under stirring at room temperature for 5 minutes. Then the resultant reaction solution was put into an oil bath to react under stirring at 60°C for 18 hours. The solvent was evaporated to dryness. The residue was purified by column chromatography (200-300 mesh normal phase silica gel, with a gradient elution of petroleum ether: ethyl acetate: dichloromethane: methanol =1:1:1:0.05 - 1:1:1:0.15). The target product was collected and concentrated to give 9.4 g of product N-2. $^1$H NMR (400 MHz, DMSO-d6) δ 8.15 (s, 2H), 7.87 - 7.76 (m, 7H), 7.75 (dd, J = 7.5, 1.6 Hz, 3H), 7.66 (ddd, J = 24.6, 7.4, 1.7 Hz, 4H), 7.59 (d, J = 1.8 Hz, 0H), 7.55 (tdd, J = 7.5, 5.4, 1.6 Hz, 10H), 7.46 (dddd, J = 9.2, 7.6, 4.8, 2.0 Hz, 8H), 5.35 (t, J = 5.5 Hz, 2H), 5.16 (t, J = 7.0 Hz, 2H), 5.07 (d, J = 5.0 Hz, 2H), 4.70 (dd, J = 3.7, 1.9 Hz, 8H), 4.46 (t, J = 1.9 Hz, 2H), 4.26 - 4.14 (m, 4H), 3.95 (dt, J = 12.3, 7.0 Hz, 2H), 3.84 - 3.61 (m, 6H), 3.53 (dd, J = 12.4, 7.0 Hz, 2H), 3.47 - 3.20 (m, 10H). MS m/z: $C_{38}H_{38}N_3O_7$, [M+H]+, calculated: 648.27, measured: 648.35.

(1-4) Synthesis of N-3

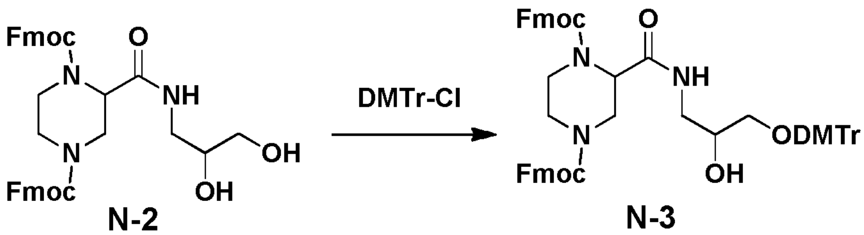


N-2 N-3

N-2 (8.27 g, 12.8 mmol) obtained in step (1-3) was dissolved in 60 mL of anhydrous pyridine, added with 4,4'-dimethoxytrityl chloride (5.2 g, 15.4 mmol) to react under stirring at room temperature for 18 hours. The reaction was quenched by addition of 50 mL of methanol. The solvent was evaporated to dryness. The residue was purified by column chromatography (200-300 mesh normal phase silica gel, with an elution of a mixed solution of petroleum ether: ethyl acetate =1:1). The target product was collected and concentrated to give 12.7 g of product N-3. $^1$H NMR (400 MHz, DMSO-d6) δ 7.95 (dd, J = 7.3, 1.6 Hz, 1H), 7.87 - 7.71 (m, 5H), 7.67 - 7.41 (m, 12H), 7.36 - 7.25 (m, 7H), 6.89 - 6.81 (m, 4H), 5.16 (t, J = 7.0 Hz, 1H), 4.70 (dd, J = 5.1, 1.1 Hz, 4H), 4.41 (pd, J = 7.0, 5.0 Hz, 1H), 4.27 (t, J = 6.2 Hz, 1H), 4.13 - 3.93 (m, 3H), 3.89 - 3.72 (m, 2H), 3.79 (s, 7H), 3.67 - 3.36 (m, 6H), 3.21 (dd, J = 12.4, 7.0 Hz, 1H). MS m/z: $C_{59}H_{56}N_3O_9$, [M+H]+, calculated: 950.40, measured: 950.32.

(1-5) Synthesis of N-4

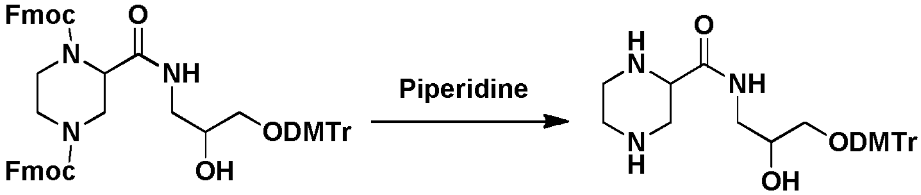


N-3 N-4

N-3 (12.7 g, 13.4 mmol) obtained in step (1-4) was dissolved in 70 mL dimethylformamide, and added with piperidine (34.2 g, 401.5 mmol). The resultant recation solution was diluted by adding 500 mL of water under stirring, and extracted with ethyl acetate for three times. The organic phases were combined, washed once with saturated brine, and dried. The solvent was evaporated to dryness. The residue was purified by column chromatography (200-300 mesh normal phase silica gel, with a gradient elution of ethyl acetate: methanol = 1:1 - 1:10). The target product was collected and concentrated to give 5.77 g of the target product N-4. $^1$H NMR (400 MHz, DMSO-d6) δ 8.15 (s, 1H), 7.50 - 7.42 (m, 2H), 7.36 - 7.25 (m, 7H), 6.89 - 6.81 (m, 4H), 4.68 (d, J = 5.0 Hz, 1H), 4.41 (pd, J = 7.0, 5.0 Hz, 1H), 3.79 (s, 6H), 3.62 (ddd, J = 10.1, 7.0, 2.8 Hz, 2H), 3.50 (dd, J = 12.3, 7.0 Hz, 1H), 3.28 (dd, J = 12.4, 7.0 Hz, 1H), 3.06 (dd, J = 12.4, 7.0 Hz, 1H), 2.92 - 2.81 (m, 2H), 2.85 - 2.74 (m, 2H), 2.78 - 2.68 (m, 2H), 2.09 (s, 1H), 1.83 (s, 1H). MS m/z: $C_{29}H_{36}N_3O_5$, [M+H]+, calculated: 506.27, measured: 506.35.

(1-6) Synthesis of N-5

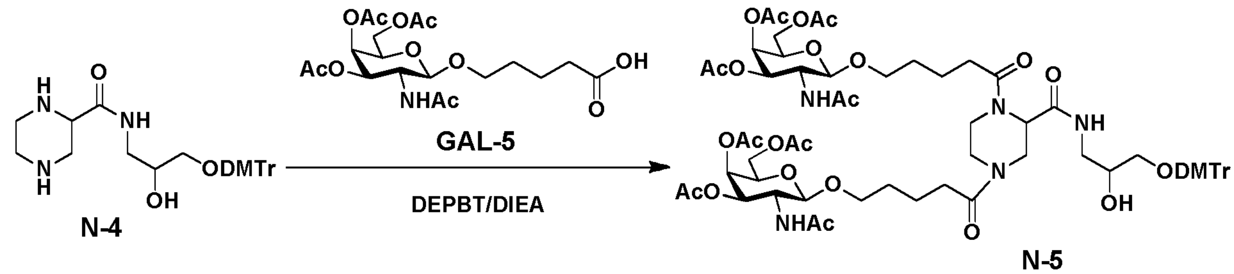


N-4 N-5

10 mL of dichloromethane was added with GAL-5 (1.074 g, 2.4 mmol), 3-(diethoxyphosphoryloxy)-1,2,3-benzotrizin-4(3H)-one (0.897 g, 3.0 mmol) and diisopropylethylamine (0.775 g, 6.0 mmol) to react at room temperature for 1 hour. Then N-4 (0.505 g, 1.0 mmol) was added to react under stirring at 25°C for 18 hours. The solvent was evaporated to dryness. The residue was purified by column chromatography (200-300 mesh normal phase silica gel, with a gradient elution of petroleum ether: ethyl acetate: dichloromethane: methanol = 1:1:1:0.05 - 1:1:1:0.2). The target product was collected and concentrated to give 1.2 g of the target product N-5. $^1$H NMR (400 MHz, DMSO-d6) δ 8.15 (s, 1H), 7.85 (d, J = 5.0 Hz, 1H), 7.46 (s, 3H), 7.50 - 7.41 (m, 1H), 7.36 - 7.25 (m, 7H), 6.89 - 6.81 (m, 4H), 6.05 - 5.94 (m, 4H), 5.16 (t, J = 7.0 Hz, 1H), 4.98 (dt, J = 9.4, 6.8 Hz, 2H), 4.65 - 4.34 (m, 7H), 4.02 - 3.88 (m, 3H), 3.79 (s, 6H), 3.75 - 3.31 (m, 7H), 3.22 - 2.92 (m, 5H), 2.13 - 1.87 (m, 28H), 1.63 - 1.51 (m, 2H), 1.55 - 1.39 (m, 2H). MS m/z: $C_{67}H_{90}N_5O_{25}$, [M+H]+, calculated: 1364.59, measured: 1364.52.

(1-7) Synthesis of N-6

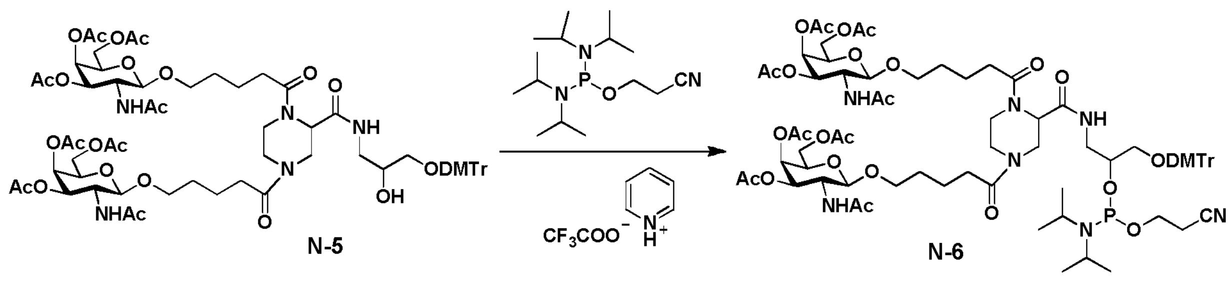

N-5 N-6

10 mL of anhydrous dichloromethane was added with N-5 synthesized according to step (1-6) (1.365 g, 1.0 mmol, obtained by combining two batches of products), pyridine trifluoroacetate (0.232 g, 1.2 mmol), N-methylimidazole (0.099 g, 1.2 mmol), bis(diisopropylamino)(2-cyanoethoxy)phosphine (0.452 g, 1.5 mmol, purchased from ACROS Reagent, CAS No.: 102691-36-1) under argon atmosphere to react under stirring at room temperature for 5 hours. The solvent was evaporated to dryness. The residue was purified by column chromatography (200-300 mesh normal phase silica gel, with a gradient elution of petroleum ether: ethyl acetate = 2:1 - 1:2). The target product was collected and concentrated to give 1.346 g of the target product N-6. $^1$H NMR (400 MHz, DMSO-d6) δ 7.46 (s, 1H), 7.36 - 7.25 (m, 3H), 6.88 - 6.82 (m, 2H), 6.05 - 5.94 (m, 1H), 5.20 - 4.98 (m, 2H), 4.49 - 3.91 (m, 5H), 3.79 (s, 3H), 3.94 - 3.67 (m, 2H),

3.67 - 3.27 (m, 3H), 3.26 - 2.88 (m, 2H), 2.91 - 2.75 (m, 1H), 2.29 - 2.16 (m, 1H), 2.15 - 1.95 (m, 11H), 1.76- 1.49 (m, 1H), 1.36 - 1.11 (m, 1H), 1.11- 0.99 (m, 4H), 0.61 (d, J = 6.9 Hz, 1H). 31P NMR (161 MHz, DMSO-d6) δ 149.23-148.06. MS m/z: $C_{76}H_{107}N_7O_{26}P$, [M+H]+, calculated: 1564.70, measured: 1564.62. The resultant compound of Formula (N-6) has a structure conforming to Formula (403).

**Preparation Example 2 Synthesis of the compound as shown by Formula (X-2)**

In this preparation example, a compound as shown by Formula (X-2) was synthesized according to the following method:

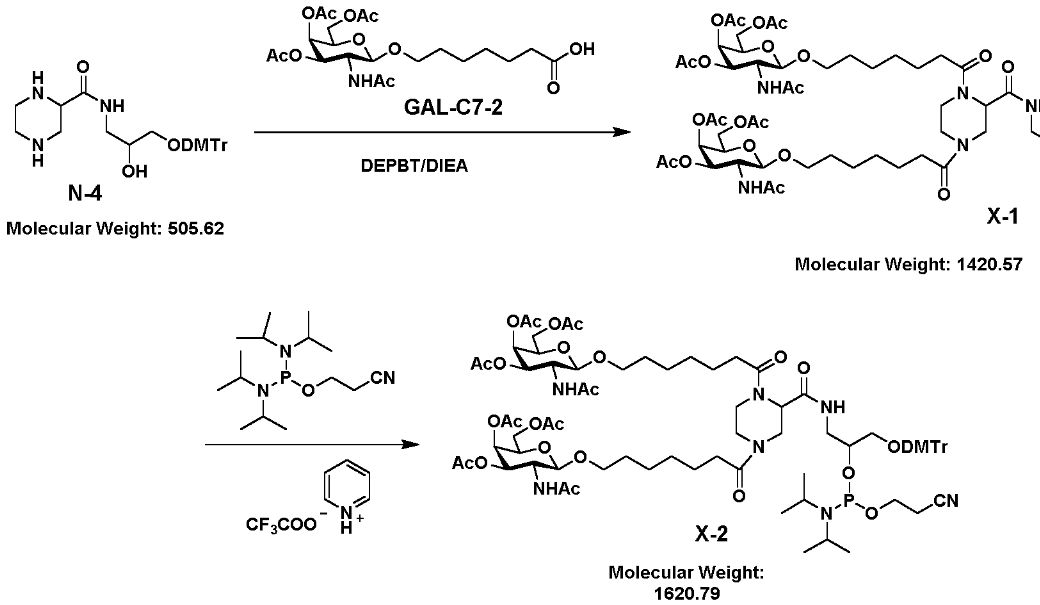


(2-1) Synthesis of GAL-C7-2

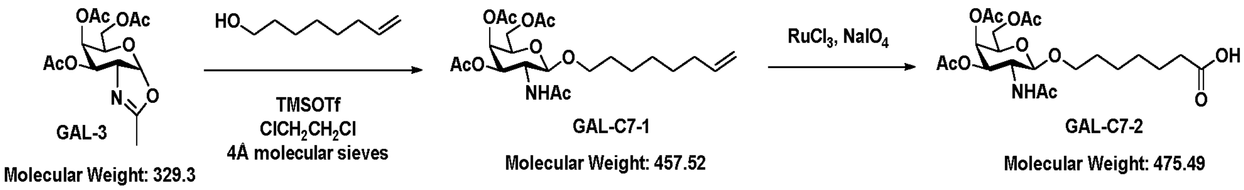


(2-1a) Synthesis of GAL-C7-1

GAL-3 (9.5 g, 28.8 mmol) was dissolved in 50 mL of anhydrous 1,2-dichloroethane, added with 10 g of activated 4Å molecular sieve powder, and then added with 7-octene-1-ol (4.1 g, 31.7 mmol) to react under stirring at room temperature for 30 minutes. Trimethylsilyl trifluoromethanesulfonate (TMSOTf, 2.9 mL, 14.4 mmol) was added in an ice bath under nitrogen atmosphere to react under stirring at room temperature for 16 hours. The 4Å molecular sieve powder was removed by filtration with diatomite. The filtrate was added with 100 mL of saturated aqueous sodium bicarbonate solution to react under stirring for 10 minutes. The organic phase was isolated. The aqueous phase was extracted once with 100 mL of dichloroethane. The organic phases were combined and washed once with saturated aqueous sodium bicarbonate solution and saturated brine, respectively. The organic phase was isolated and dried with anhydrous sodium sulfate. The solvent was removed by evaporation under reduced pressure and pumped to dryness with an oil pump to give 13.1g of GAL-C7-1, which was directly used in the next oxidation reaction without purification.

(2-1b) Synthesis of GAL-C7-2

GAL-C7-1 synthesized according to step (2-1a) (18.6 g, 40.7 mmol, obtained by combining two batches of

products) was dissolved in a mixed solvent of 80 mL of dichloromethane and 80 mL of acetonitrile, added with 130 mL of water and sodium periodate solid (34.8 g, 163 mmol) respectively in an ice water bath to react under stirring for 10 minutes. Then catalyst ruthenium trichloride (278 mg, 1.34 mmol) was added to react under stirring at room temperature for 16 hours. The resultant reaction solution was added with 300mL of water, and then adjusted pH of 7.5 by adding saturated sodium bicarbonate. The organic phase was isolated and discarded. The aqueous phase was extracted three times with dichloromethane, and the organic phase was discarded. The pH of aqueous phase was adjusted to 3 with citric acid solid and extracted three times (each with 200 mL of dichloromethane), and the organic phases were combined and dried with anhydrous sodium sulfate. The solvent was removed by evaporation under reduced pressure. The residue was purified by column chromatography (200-300 mesh normal phase silica gel, with a gradient elution of dichloromethane: methanol = 10:1 - 3:1) to give 14.2 g of product GAL-C7-2. $^1$H NMR (400 MHz, DMSO-d6) δ 7.46 (s, 1H), 6.05 - 5.94 (m, 2H), 5.18 (t, J = 7.0 Hz, 1H), 4.52 (q, J = 7.0 Hz, 1H), 4.30 (dd, J = 12.4, 7.0 Hz, 1H), 3.98 (t, J = 7.0 Hz, 1H), 3.85 (dd, J = 12.4, 6.9 Hz, 1H), 3.35 - 3.23 (m, 1H), 2.88 (td, J = 12.3, 3.1 Hz, 1H), 2.69 - 2.58 (m, 1H), 2.30 - 2.14 (m, 2H), 2.13 - 1.95 (m, 13H), 1.80 (dddt, J = 13.7, 12.5, 9.5, 1.2 Hz, 1H), 1.52 (dt, J = 12.2, 9.2 Hz, 1H), 1.41 - 1.24 (m, 1H), 1.28 - 1.14 (m, 1H). MS m/z: $C_{21}H_{32}NO_{11}$, [M-H]-, calculated: 474.20, measured: 474.31.

(2-2) Synthesis of X-1

10 mL of dichloromethane was added with GAL-C7-2 (1.141 g, 2.4 mmol), 3-(diethoxyphosphoryloxy)-1,2,3-benzotrizin-4(3H)-one (0.897 g, 3.0 mmol) and diisopropylethylamine (0.775 g, 6.0 mmol) to react at room temperature for 1 hour. Then N-4 (0.505 g, 1.0 mmol) was added to react under stirring at 25°C for 18 hours. The solvent was evaporated to dryness. The residue was purified by column chromatography (200-300 mesh normal phase silica gel, with a gradient elution of petroleum ether: ethyl acetate: dichloromethane: methanol = 1:1:1:0.05 - 1:1:1:0.2). The target product was collected and concentrated to give 1.196 g of the target product X-1. $^1$H NMR (400 MHz, DMSO-d6) δ 8.15 (s, 1H), 7.46 (s, 3H), 7.35 - 7.29 (m, 4H), 7.33 - 7.20 (m, 4H), 6.89 - 6.82 (m, 4H), 6.05 - 5.94 (m, 4H), 5.12 (dt, J = 31.6, 7.0 Hz, 2H), 4.58 - 4.27 (m, 7H), 4.18 - 3.99 (m, 2H), 4.01 - 3.88 (m, 2H), 3.79 (s, 6H), 3.81 - 3.64 (m, 2H), 3.59 - 3.30 (m, 4H), 3.19 - 3.00 (m, 2H), 2.96 - 2.81 (m, 2H), 2.09 - 1.92 (m, 24H), 1.62 - 1.16 (m, 8H), 1.18 - 0.99 (m, 2H), 1.03 - 0.87 (m, 2H). MS m/z: $C_{71}H_{98}N_5O_{25}$, [M+H]+, calculated: 1420.66, measured: 1420.74.

(2-3) Synthesis of X-2

10 mL of anhydrous dichloromethane was added with X-1 synthesized according to step (2-2) (1.421 g, 1.0 mmol, obtained by combining two batches of products), pyridine trifluoroacetate (0.232 g, 1.2 mmol), N-methylimidazole (0.099 g, 1.2 mmol), bis(diisopropylamino)(2-cyanoethoxy)phosphine (0.452 g, 1.5 mmol) under argon atmosphere to react under stirring at room temperature for 5 hours. The solvent was evaporated to dryness. The residue was purified by column chromatography (200-300 mesh normal phase silica gel, with a gradient elution of petroleum ether: ethyl acetate = 2:1-1:2). The target product was collected and concentrated to give 1.446 g of the target product X-2. $^1$H NMR (400 MHz, DMSO-d6) δ 7.46 (s, 1H), 7.36 - 7.25 (m, 3H), 6.89 - 6.82 (m, 2H), 6.05 - 5.94 (m, 1H), 5.12 (dt, J = 30.4, 7.0 Hz, 1H), 4.77 (dq, J = 27.5, 7.0 Hz, 1H), 4.52 - 4.35 (m, 1H), 4.35 - 4.15 (m, 1H), 3.96 - 3.78 (m, 1H), 3.79 (s, 3H), 3.74 - 3.56 (m, 2H), 3.55 - 3.03 (m, 3H), 2.90 - 2.75 (m, 1H), 2.74 - 2.34 (m, 2H), 2.23 - 1.95 (m, 11H), 1.85 - 1.17 (m, 4H), 1.11 - 0.90 (m, 6H), 0.66 - 0.45 (m, 1H). 31P NMR (161 MHz, DMSO-d6) δ 148.83-147.65. MS m/z: $C_{80}H_{115}N_7O_{26}P$, [M+H]+, calculated: 1620.76, measured: 1620.82. The resultant compound of Formula (X-2) has a structure conforming to Formula (404).

**Preparation Example 3 Preparation of the compound as shown by Formula (W-2)**

In this preparation example, a compound as shown by Formula (W-2) was synthesized according to the following method:

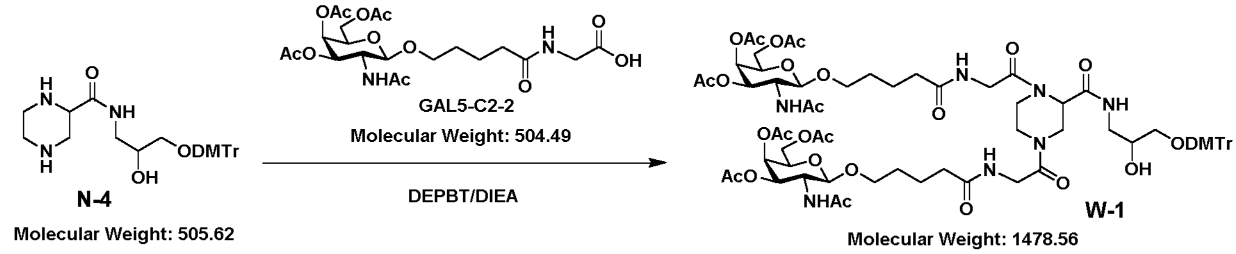

N-4
Molecular Weight: 505.62
GAL5-C2-2
Molecular Weight: 504.49
W-1
Molecular Weight: 1478.56

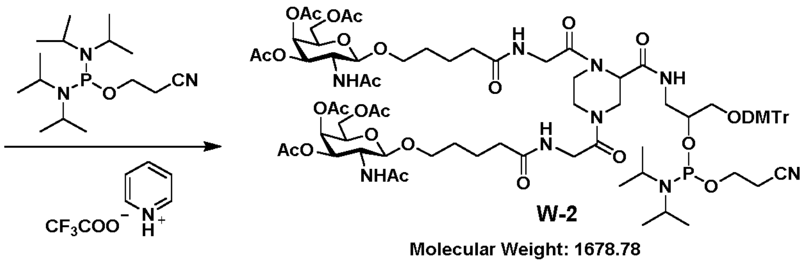
W-2
Molecular Weight: 1678.78

## (3-1) Synthesis of GAL5-C2-2

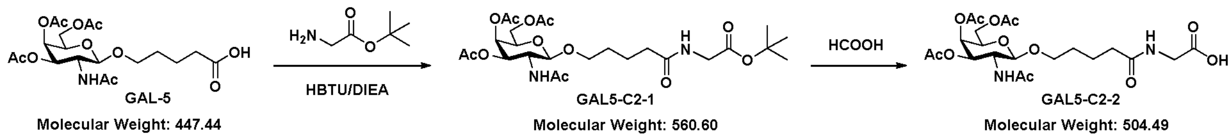

GAL-5
Molecular Weight: 447.44
GAL5-C2-1
Molecular Weight: 560.60
GAL5-C2-2
Molecular Weight: 504.49

(3-1a) Synthesis of GAL-C2-1

40 mL of N,N-dimethylformamide was added with GAL-5 (4.5 g, 10.0 mmol), glycine tert butyl ester hydrochloride (2.0 g, 12.0 mmol), O-benzotriazol-tetramethyluronium hexafluorophosphate (5.7 g, 15.0 mmol) and diisopropylethylamine (3.9 g, 30.0 mmol) to react under stirring at room temperature for 4 hours. The resultant reaction solution was added with 100 mL of saturated aqueous sodium bicarbonate solution, and extracted three times (each with 100 mL of ethyl acetate). The organic phases were combined and washed once with 100 mL of saturated brine. The organic phase was isolated and dried with anhydrous sodium sulfate. The solvent was removed by evaporation under reduced pressure, and pumped to dryness with an oil pump to give 10.1 g of a crude product of GAL-C2-1, which was directly used in the next reaction.

(3-1b) Synthesis of GAL-C2-2

The crude product GAL-C2-1 (10.1 g, 10 mmol) was dissolved in 60 mL of formic acid to react under stirring at room temperature for 16 hours. The solvent was evaporated to dryness. The residue was purified by column chromatography (200-300 mesh normal phase silica gel, with a gradient elution of dichloromethane: methanol = 10:1-3:1). The target product was collected and concentrated to give 5.0 g of the target product CAL-C2-2. $^1$H NMR (400 MHz, DMSO-d6) δ 8.49 (s, 1H), 7.46 (s, 1H), 6.05 - 5.94 (m, 2H), 5.01 - 4.90 (m, 1H), 4.52 - 4.33 (m, 3H), 3.79 - 3.61 (m, 3H), 3.14 (td, J = 12.2, 3.2 Hz, 1H), 2.68 (td, J = 12.2, 3.3 Hz, 1H), 2.27 (td, J = 12.6, 2.8 Hz, 1H), 2.15 (td, J = 12.6, 2.9 Hz, 1H), 2.07 - 1.95 (m, 12H), 1.82 (qt, J = 12.9, 2.8 Hz, 1H), 1.54 (qt, J = 12.6, 2.6 Hz, 1H), 1.15 - 0.99 (m, 1H), 0.97 - 0.81 (m, 1H). MS m/z: $C_{21}H_{31}N_2O_{12}$, [M-H]-, calculated: 503.19, measured: 503.26.

(3-2) Synthesis of W-1

10 mL of dichloromethane was added with GAL5-C2-2 (1.211 g, 2.4 mmol), 3-(diethoxyphosphoryloxy)-1,2,3-benzotrizin-4(3H)-one (0.897 g, 3.0 mmol) and diisopropylethylamine (0.775 g, 6.0 mmol) to react at room temperature for 1 hour. Then N-4 (0.505 g, 1.0 mmol) was added to react under stirring at 25°C for 18 hours. The solvent was evaporated to dryness. The residue was purified by column chromatography (200-300 mesh normal phase silica gel,

with a gradient elution of petroleum ether: ethyl acetate: dichloromethane: methanol = 1:1:1:0.1-1:1:1:0.4). The target product was collected and concentrated to give 1.291 g of the target product W-1. $^1$H NMR (400 MHz, DMSO-d6) δ 8.49 (s, 1H), 7.46 (s, 2H), 7.36 - 7.25 (m, 4H), 6.88 - 6.82 (m, 2H), 6.05 - 5.94 (m, 1H), 5.60 - 5.38 (m, 2H), 5.14 (dt, J = 13.7, 6.9 Hz, 1H), 4.83 - 4.67 (m, 1H), 4.53 - 4.09 (m, 4H), 3.79 (s, 3H), 3.75 - 3.24 (m, 6H), 3.22 - 3.09 (m, 1H), 3.09 - 2.76 (m, 2H), 2.28 - 2.07 (m, 2H), 2.09 - 1.95 (m, 13H), 1.80 - 1.44 (m, 2H), 1.43 - 1.31 (m, 1H). MS m/z: $C_{71}H_{96}N_7O_{27}$, [M+H]+, calculated: 1478.64, measured: 1478.56.

(3-3) Synthesis of W-2

10 mL of anhydrous dichloromethane was added with W-1 synthesized according to step (3-2) (1.479 g, 1.0 mmol, obtained by combining two batches of products), pyridine trifluoroacetate (0.232 g, 1.2 mmol), N-methylimidazole (0.099 g, 1.2 mmol), bis(diisopropylamino)(2-cyanoethoxy)phosphine (0.452 g, 1.5 mmol) under argon atmosphere to react under stirring at room temperature for 5 hours. The solvent was evaporated to dryness. The residue was purified by column chromatography (200-300 mesh normal phase silica gel, with a gradient elution of petroleum ether: ethyl acetate = 3:1-1:3). The target product was collected and concentrated to give 1.534 g of the target product W-2. $^1$H NMR (400 MHz, DMSO-d6) δ 8.49 (s, 1H), 7.46 (s, 1H), 7.36 - 7.25 (m, 3H), 6.89 - 6.82 (m, 2H), 6.05 - 5.94 (m, 1H), 5.20 - 5.05 (m, 1H), 4.81 - 4.57 (m, 2H), 4.47 - 4.24 (m, 2H), 4.05 - 3.66 (m, 4H), 3.79 (s, 3H), 3.67 - 3.45 (m, 2H), 3.40 - 3.26 (m, 1H), 3.16 - 2.93 (m, 2H), 2.92 - 2.66 (m, 3H), 2.39 - 2.22 (m, 1H), 2.10 - 1.93 (m, 11H), 1.77 - 1.53 (m, 1H), 1.56 - 1.32 (m, 1H), 1.09 - 1.01 (m, 4H), 0.97 (d, J = 6.7 Hz, 1H). 31P NMR (161 MHz, DMSO-d6) δ 149.15-147.72. MS m/z: $C_{80}H_{113}N_9O_{28}P$, [M+H]+, calculated: 1678.74, measured: 1678.66. The resultant compound of Formula (W-2) has a structure conforming to Formula (405).

**Preparation Example 4 Preparation of the compound as shown by Formula (V-2)**

In this preparation example, a compound as shown by Formula (V-2) was synthesized according to the following method:

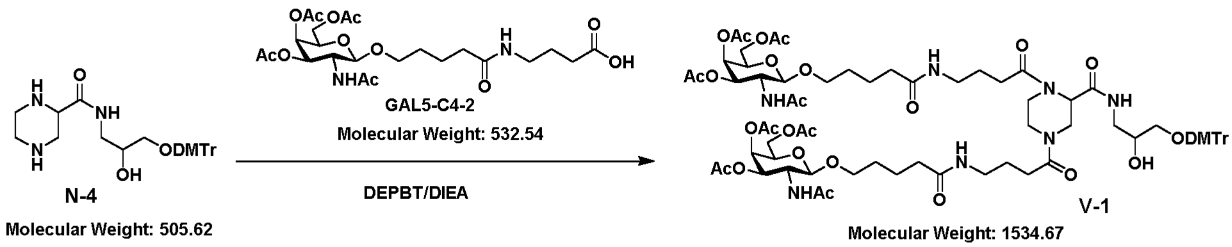


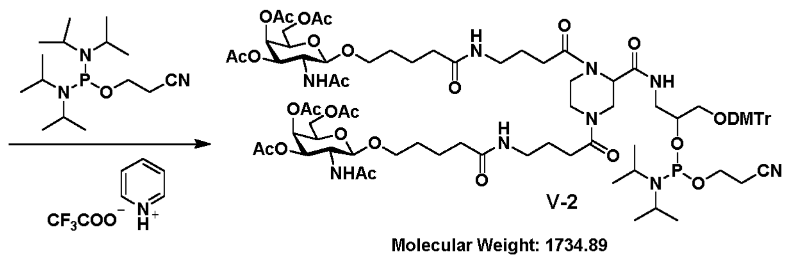


(4-1) Synthesis of GAL5-C4-2

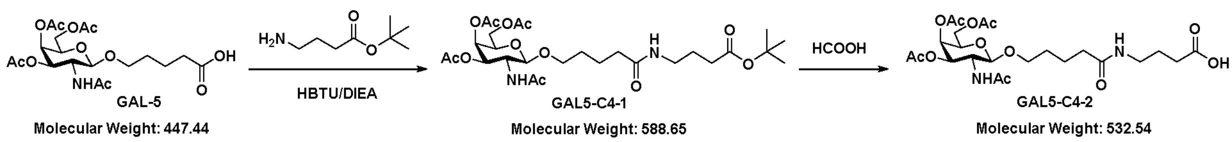

(4-1a) Synthesis of GAL-C4-1

40 mL of N,N-dimethylformamide was added with GAL-5 (4.5 g, 10.0 mmol), 4-amino tert-butyl ester hydrochloride (1.9 g, 12.0 mmol), O-benzotriazol-tetramethyluronium hexafluorophosphate (5.7 g, 15.0 mmol) and diisopropylethylamine (3.9 g, 30.0 mmol). The resultant solution was dissolved uniformly and react completely under stirring at room temperature for 4 hours. The resultant reaction solution was slowly added with 100 mL of saturated aqueous sodium bicarbonate solution, and extracted three times, each with 100 mL of ethyl acetate. The organic phases were combined and washed once with 100 mL of saturated brine. The organic phase was isolated and dried with anhydrous sodium sulfate. The solvent was removed by evaporation under reduced pressure, and pumped to dryness with an oil pump to give 10.3 g of a crude product of GAL-C4-1, which was directly used in the next reaction.

(4-1b) Synthesis of GAL-C4-2

The crude product GAL-C4-1 (10.3 g, 10 mmol) was dissolved in 60 mL of formic acid to react under stirring at room temperature for 16 hours. The solvent was evaporated to dryness. The residue was purified by column chromatography (200-300 mesh normal phase silica gel, with a gradient elution of dichloromethane: methanol = 10:1-2:1). The target product was collected and concentrated to give 5.1 g of the target product. $^1$H NMR (400 MHz, DMSO-d6) δ 7.89 (s, 1H), 7.46 (s, 1H), 6.05 - 5.94 (m, 2H), 5.25 (t, J = 7.0 Hz, 1H), 4.53 - 4.35 (m, 2H), 4.14 (t, J = 7.0 Hz, 1H), 3.81 (dd, J = 12.1, 6.8 Hz, 1H), 3.46 (td, J = 12.1, 3.3 Hz, 1H), 3.30 (td, J = 12.4, 3.0 Hz, 1H), 3.01 (td, J = 12.1, 2.8 Hz, 1H), 2.75 (td, J = 12.4, 3.0 Hz, 1H), 2.45 - 2.08 (m, 6H), 2.07 - 1.95 (m, 12H), 1.81 - 1.49 (m, 3H), 1.35 - 1.20 (m, 1H). MS m/z: $C_{23}H_{35}N_2O_{12}$, [M-H]-, calculated: 531.22, measured: 531.15.

(4-2) Synthesis of V-1

10 mL of dichloromethane was added with GAL5-C4-2 (1.278 g, 2.4 mmol), 3-(diethoxyphosphoryloxy)-1,2,3-benzotrizin-4(3H)-one (0.897 g, 3.0 mmol) and diisopropylethylamine (0.775 g, 6.0 mmol) to react at room temperature for 1 hour. Then N-4 (0.505 g, 1.0 mmol) was added to react under stirring at 25°C for 18 hours. The solvent was evaporated to dryness. The residue was purified by column chromatography (200-300 mesh normal phase silica gel, with a gradient elution of petroleum ether: ethyl acetate: dichloromethane: methanol = 1:1:1:0.1 - 1:1:1:0.5). The target product was collected and concentrated to give 1.315 g of the target product V-1. $^1$H NMR (400 MHz, DMSO-d6) δ 9.83 (s, 1H), 8.81 (s, 1H), 8.15 (s, 1H), 7.46 (s, 3H), 7.50 - 7.41 (m, 1H), 7.36 - 7.25 (m, 7H), 6.89 - 6.81 (m, 4H), 6.08 - 5.94 (m, 3H), 5.60 (d, J = 6.9 Hz, 1H), 5.18 (dt, J = 19.2, 7.0 Hz, 2H), 4.64 - 4.47 (m, 2H), 4.46 - 4.27 (m, 2H), 4.29 - 4.07 (m, 4H), 3.98 - 3.73 (m, 3H), 3.79 (s, 6H), 3.67 - 3.50 (m, 2H), 3.54 - 3.45 (m, 2H), 3.37 (ddd, J = 25.2, 12.5, 7.0 Hz, 2H), 3.32 - 3.15 (m, 2H), 3.20 - 3.11 (m, 2H), 3.03 - 2.83 (m, 3H), 2.80 - 2.48 (m, 5H), 2.42 - 2.25 (m, 3H), 2.15 - 1.89 (m, 28H), 1.86 - 1.68 (m, 2H), 1.70 - 1.48 (m, 2H). MS m/z: $C_{75}H_{104}N_7O_{27}$, [M+H]+, calculated: 1534.70, measured: 1534.63.

(4-3) Synthesis of V-2

10 mL of anhydrous dichloromethane was added with V-1 synthesized according to step (4-2) (1.535 g, 1.0 mmol, obtained by combining multiple batches of products), pyridine trifluoroacetate (0.232 g, 1.2 mmol), N-methylimidazole (0.099 g, 1.2 mmol), bis(diisopropylamino)(2-cyanoethoxy)phosphine (0.452 g, 1.5 mmol) under argon atmosphere to react under stirring at room temperature for 5 hours. The solvent was evaporated to dryness. The residue was purified by column chromatography (200-300 mesh normal phase silica gel, with a gradient elution of petroleum ether: ethyl acetate = 2:1-1:3). The target product was collected and concentrated to give 1.490 g of the target product. $^1$H NMR (400 MHz, DMSO-d6) δ 8.15 (s, 0H), 8.07 (s, 0H), 7.86 (s, 0H), 7.46 (s, 0H), 7.50 - 7.41 (m, 0H), 7.36 - 7.25 (m, 0H), 6.89 - 6.81 (m, 0H), 5.55 (dd, J = 22.4, 7.0 Hz, 0H), 4.49 - 4.35 (m, 0H), 4.31 - 4.19 (m, 0H), 4.23 - 4.09 (m, 0H), 4.13 - 3.99 (m, 0H), 4.01 - 3.88 (m, 0H), 3.79 (s, 0H), 3.82 - 3.21 (m, 1H), 3.10 (ddd, J = 12.5, 6.1, 3.2 Hz, 0H), 2.98 - 2.75 (m, 0H), 2.74 - 2.52 (m, 0H), 2.54 - 2.38 (m, 0H), 2.41 - 2.25 (m, 0H), 2.29 - 2.21 (m, 0H), 2.20 - 1.93 (m, 1H), 1.83 - 1.66 (m, 0H), 1.70 - 1.50 (m, 0H), 1.39 - 1.21 (m, 0H), 1.18 - 1.02 (m, 0H), 0.95 - 0.79 (m, 0H), 0.70 (d, J = 6.8 Hz, 0H). $^{31}$P NMR (161 MHz, DMSO-d6) δ 148.79-147.75. MS m/z: $C_{84}H_{121}N_9O_{28}P$, [M+H]+, calculated: 1734.81, measured: 1734.89. The resultant compound of Formula (V-2) has a structure conforming to Formula (406).

**Preparation Example 5 Synthesis of the compound as shown by Formula (O-2)**

In this preparation example, a compound as shown by Formula **(O-2)**was synthesized according to the following method:

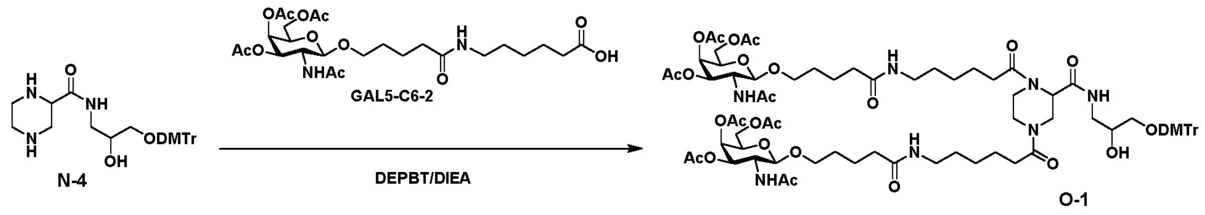


N-4 GAL5-C6-2 O-1

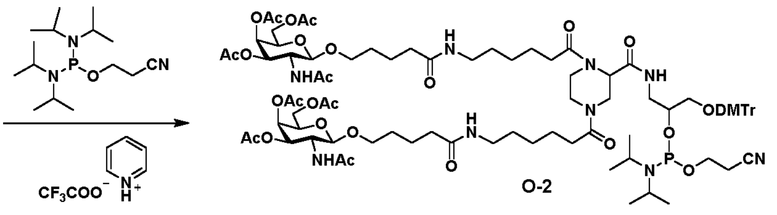


O-2

(5-1) Synthesis of GAL5-C6-2

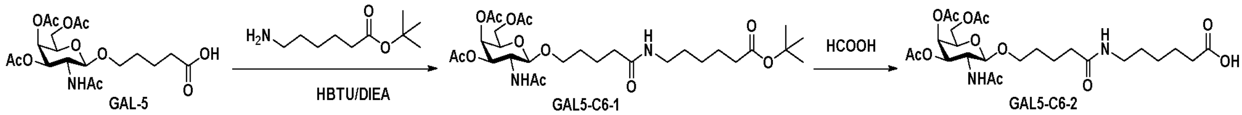


GAL-5 GAL5-C6-1 GAL5-C6-2

(5-1a) Synthesis of GAL-C6-1

40 mL of N,N-dimethylformamide was added with GAL-5 (4.5 g, 10.0 mmol), tert-butyl 6-aminocaproate hydrochloride (2.2 g, 12.0 mmol), O-benzotriazol-tetramethyluronium hexafluorophosphate (5.7 g, 15.0 mmol) and diisopropylethylamine (3.9 g, 30.0 mmol) to react under stirring at room temperature for 4 hours. The resultant reaction solution was added with 100 mL of saturated aqueous sodium bicarbonate solution, and extracted three times, each with 100 mL of ethyl acetate. The organic phases were combined and washed once with 100 mL of saturated brine. The organic phase was isolated and dried with anhydrous sodium sulfate. The solvent was removed by evaporation under reduced pressure, and pumped to dryness with an oil pump to give 10.5 g of a crude product of GAL-C6-1, which was directly used in the next reaction.

(5-1b) Synthesis of GAL-C6-2

The crude product GAL-C6-1 (10.5 g, 10 mmol) was dissolved in 60 mL of formic acid to react under stirring at room temperature for 16 hours. The solvent was evaporated to dryness. The residue was purified by column chromatography (200-300 mesh normal phase silica gel, with a gradient elution of dichloromethane: methanol = 10:1 - 3:1). The target product was collected and concentrated to give 5.2 g of the target product. $^1$H NMR (400 MHz, DMSO-d6) δ 7.87 (s, 0H), 7.46 (s, 0H), 6.05 - 5.94 (m, 0H), 5.17 (t, J = 7.0 Hz, 0H), 4.54 (dd, J = 12.4, 6.9 Hz, 0H), 4.33 (q, J = 7.0 Hz, 0H), 3.88 (t, J = 7.0 Hz, 0H), 3.75 - 3.58 (m, 0H), 3.38 (td, J = 12.3, 2.1 Hz, 0H), 3.17 - 3.07 (m, 0H), 2.57 - 2.46 (m, 0H), 2.50 - 2.35 (m, 0H), 2.28 (ddd, J = 12.3, 4.5, 2.1 Hz, 0H), 2.27 - 2.08 (m, 0H), 2.09 - 1.90 (m, 1H), 1.76 - 1.63 (m, 0H), 1.62 - 1.37 (m, 0H), 1.05 - 0.92 (m, 0H). MS m/z: $C_{25}H_{39}N_2O_{12}$, [M-H]-, calculated: 559.25, measured: 559.32.

(5-2) Synthesis of O-1

10 mL of dichloromethane was added with GAL5-C6-2 (1.345 g, 2.4 mmol), 3-(diethoxyphosphoryloxy)-1,2,3-benzotrizin-4(3H)-one (0.897 g, 3.0 mmol) and diisopropylethylamine (0.775 g, 6.0 mmol) to react at room temperature for 1 hour. Then N-4 (0.505 g, 1.0 mmol) was added to react under stirring at 25°C for 18 hours. The solvent was evaporated to dryness. The residue was purified by column chromatography (200-300 mesh normal phase silica gel, with a gradient elution of petroleum ether: ethyl acetate: dichloromethane: methanol = 1:1:1:0.1 - 1:1:1:0.3). The target product was collected and concentrated to give 1.298 g of the target product. $^1$H NMR (400 MHz, DMSO-d6) δ 7.46 (s, 3H), 7.50 - 7.41 (m, 1H), 7.36 - 7.25 (m, 7H), 6.89 - 6.81 (m, 4H), 6.05 - 5.94 (m, 2H), 5.39 - 5.28 (m, 2H), 5.16 (td, J = 7.0, 2.9 Hz, 2H), 4.57 (ddd, J = 32.7, 12.3, 6.9 Hz, 2H), 4.46 - 4.25 (m, 2H), 4.29 - 4.16 (m, 2H), 4.10 - 3.83 (m, 3H), 3.82 - 3.66 (m, 8H), 3.71 - 3.57 (m, 2H), 3.55 - 3.34 (m, 3H), 3.33 - 2.80 (m, 13H), 2.28 - 2.07 (m, 4H), 2.03 (d, J = 12.0 Hz, 19H), 1.97 (s, 6H), 1.98 - 1.72 (m, 3H), 1.73 - 1.57 (m, 1H), 1.44 - 0.84 (m, 8H). MS m/z: $C_{79}H_{112}N_7O_{27}$,[M+H]+,

calculated: 1590.76, measured: 1590.83.

(5-3) Synthesis of O-2

10 mL of anhydrous dichloromethane was added with O-1 synthesized according to step (5-2) (1.591 g, 1.0 mmol, obtained by combining two batches of products), pyridine trifluoroacetate (0.232 g, 1.2 mmol), N-methylimidazole (0.099 g, 1.2 mmol), bis(diisopropylamino)(2-cyanoethoxy)phosphine (0.452 g, 1.5 mmol) under argon atmosphere to react under stirring at room temperature for 5 hours. The solvent was evaporated to dryness. The residue was purified by column chromatography (200-300 mesh normal phase silica gel, with a gradient elution of petroleum ether: ethyl acetate = 2:1-1:2). The target product was collected and concentrated to give 1.501 g of the target product. $^{1}$H NMR (400 MHz, DMSO-d6) δ 7.70 (s, 1H), 7.46 (s, 1H), 7.36 - 7.25 (m, 3H), 6.89 - 6.81 (m, 2H), 6.05 - 5.94 (m, 2H), 4.53 - 4.35 (m, 3H), 4.18 (ddd, J = 12.0, 9.2, 7.0 Hz, 1H), 3.97 - 3.78 (m, 2H), 3.79 (s, 3H), 3.67 - 3.45 (m, 1H), 3.45 - 3.22 (m, 2H), 3.15 (dt, J = 12.3, 7.1 Hz, 1H), 3.00 (t, J = 7.0 Hz, 2H), 2.83 (hept, J = 6.8 Hz, 1H), 2.27 (td, J = 7.1, 3.1 Hz, 1H), 2.17 - 2.09 (m, 2H), 2.14 - 1.95 (m, 12H), 1.63 - 1.45 (m, 5H), 1.34 (dtd, J = 9.0, 7.0, 2.4 Hz, 4H), 1.05 (dd, J = 20.0, 6.8 Hz, 5H). $^{31}$P NMR (161 MHz, DMSO-d6) δ 148.66-147.93. MS m/z: $C_{88}H_{129}N_9O_{28}P$, [M+H]+, calculated: 1790.87, measured: 1790.81. The synthesized compound of Formula **(O-2)**has a structure conforming to Formula (407).

**Preparation Example 6 Preparation of the compound as shown by Formula (101) linked to a solid phase support**

In this preparation example, starting from the compounds as shown by the Formula (N-6), (W-2), (V-2), (X-2) or (O-2), a compound linked to a solid phase support was prepared according to the following method.

(6-1) Synthesis of Compound N-$6_2$ linked to a solid phase support

In this step, Compound N-$6_2$ was prepared by sequentially linking two compounds as shown by Formula (N-6) to a solid phase support.

Starting from a universal solid phase support (NittoPhase®HL UnyLinker™ 300 Oligonucleotide Synthesis Support, Kinovate Life Sciences, as shown by Formula B80, and with a loading of 300 μmol/g), the protecting group on the solid phase support was removed. The compound as shown by Formula (N-6) obtained in the Preparation Example 1 was contacted with the solid phase support under coupling reaction condition and in the presence of a coupling reagent, followed by subjected to capping reaction and then oxidation reaction. Subsequently, the resultant product was subjected to deprotection, followed by contacted with the compound as shown by Formula (N-6) again, and then subjected to capping reaction and oxidation reaction, to obtain a compound in which two compounds as shown by Formula (N-6) were sequentially linked to the solid phase support, that is, Compound N-$6_2$ linked to a solid phase support. The compound has a structure as shown by Formula (503).

(6-2) Synthesis of a compound linked to a solid phase support

A compound linked to a solid phase support of the present disclosure was prepared by the same method as that in (6-1), except that two compounds as shown by Formula (W-2), (V-2), (X-2) or (O-2) were sequentially linked to the solid phase support instead of the compound as shown by Formula (N-6) in (6-1), respectively. The resultant Compound X-$2_2$ linked to the solid phase support, Compound W-$2_2$ linked to the solid phase support, Compound V-$2_2$ linked to the solid phase support, and Compound O-$2_2$ linked to the solid phase support respectively have the structures as shown by Formula (504), (505), (506), or (507).

(6-3) Synthesis of a compound linked to a solid phase support

A compound linked to a solid phase support was prepared by the same method as that in (6-1), except that starting from a univeral solid phase support, the compound as shown by Formula (N-6) was contacted with the solid phas support only once, followed by subjected to capping reaction and oxidation reaction, to obtain Compound N-$6_1$ in which only one compound as shown by Formula (N-6) was linked to the solid phase support; alternatively, the hydroxyl protecting group on Compound N-$6_2$ linked to the solid phase support was removed, and then contacted with the compound as shown by Formula (N-6) again, and then subjected to capping and oxidation reactions to obtain Compound N-$6_3$ in which three compound as shown by Formula (N-6) were sequentially linked to the solid phase support. The above Compound N-$6_1$ linked to the solid phase support and Compound N-$6_3$ linked to the solid phase support respetively have the structures as shown by Formula (508) or (509).

**Preparation Example 7 Synthesis of Conjugate N6-siHBa1 (Conjugate 13)**

In this preparation example, Conjugate **N6-siHBa1** (Conjugate 13) was prepared according to the following method, starting from the Compound N-$6_2$ linked to the solid phase support prepared as described above:
In this step, the siRNA of the drug conjugate have the sequences numbered siHBa1:

Sense strand: 5'-CCUUGAGGCAUACUUCAAA-3' (SEQ ID NO: 331),
Antisense strand: 5'-UUUGAAGUAUGCCUCAAGGUU-3' (SEQ ID NO: 332);

(7-1) Synthesis of the sense strand

Nucleoside monomers were linked one by one in 3' to 5' direction according to the above sequence order by a phosphoramidite solid phase synthesis method of nucleic acid, starting the cycles from the Compound N-$6_2$ linked to a solid phase support prepared in the above step. The linking of each nucleoside monomer included a four-step reaction of deprotection, coupling, capping, and oxidation. The synthesis conditions are as follows.
The nucleoside monomers are provided in a 0.1 M acetonitrile solution. The condition for deprotection reaction in each step is identical, i.e., a temperature of 25 °C, a reaction time of 70 seconds, a solution of dichloroacetic acid in dichloromethane (3% v/v) as a deprotection reagent, and a molar ratio of dichloroacetic acid to the protection group 4,4'-dimethoxytrityl on the solid phase support of 5:1.
The condition for coupling reaction in each step is identical, including a temperature of 25°C, a molar ratio of the nucleic acid sequence linked to the solid phase support to nucleoside monomers of 1:10, a molar ratio of the nucleic acid sequence linked to the solid phase support to a coupling reagent of 1:65, a reaction time of 600 seconds, and a solution of 0.5 M 5-ethylthio-1H-tetrazole in acetonitrile as a coupling reagent.
The condition for capping reaction in each step is identical, including a temperature of 25°C, a reaction time of 15 seconds, a mixed solution of Cap A and Cap B in a molar ratio of 1:1 as a capping agent, in which CapA is a 20% by volume of mixed solution of N-methylimidazole in pyridine/acetonitrile, with the volume ratio of pyridine to acetonitrile = 3:5, and CapB is a 20% by volume solution of acetic anhydride in acetonitrile; and a molar ratio of the capping agent to the nucleic acid sequence linked to the solid phase support of 1:1:1 (acetic anhydride: N-methylimidazole: the nucleic acid sequence linked to the solid phase support).
The condition for oxidation reaction in each step is identical, including a temperature of 25 °C, a reaction time of 15 seconds, and 0.05 M iodine water as an oxidation reagent; and a molar ratio of iodine to the nucleic acid sequence linked to the solid phase support in the coupling step of 30:1. The reaction is carried out in a mixed solvent of tetrahydrofuran: water: pyridine (3:1:1).
The conditions for cleavage and deprotection are as follows: adding the synthesized nucleotide sequence linked to the support into 25 wt% aqueous ammonia to react at 55 °C for 16 hours, wherein the amount of the aqueous ammonia is 0.5 mL/μmol. The liquid was removed, and the residue is concentrated to dryness in vacuum. After treatment with aqueous ammonia, with respect to the amount of single-stranded nucleic acid, the product was dissolved with 0.4 mL/μmol N-methylpyrrolidone, then added with 0.3 mL/μmol triethylamine and 0.6 mL/μmol triethylamine trihydrofluoride to remove the 2'-O-TBDMS protection from the ribose. Purification and desalination: purification of the nucleic acid is achieved by using a preparative ion chromatography purification column (Source 15Q) with a gradient elution of NaCl. Specifically, eluent A is 20 mM sodium phosphate (pH 8.1), solvent is water/acetonitrile in 9:1 (v/v); eluent B is 1.5 M sodium chloride, 20 mM sodium phosphate (pH 8.1), solvent is water/acetonitrile in 9:1 (v/v); elution gradient: the ratio of eluent A: eluent B = 100:0-50:50. The eluate is collected, combined and desalted by using a reverse phase chromatography purification column. The specific condition comprises: using a Sephadex column for desalination (filler: Sephadex G25) and eluting with deionized water.
Detection: the purity is determined by Ion exchange chromatography (IEX-HPLC) with a purity of 92.4%. The molecular weight was analyzed by liquid chromatography-mass spectrometry (LC-MS). Calculated: 7748.37, measured: 7747.50.
Thus, in this step, Compound N-$6_2$ was linked to 3' terminal of the resultant sense strand to obtain an sense strand S of the siRNA in which Compound N-$6_2$ was conjugated to 3' terminal of the siRNA.

(7-2) Synthesis of the antisense strand

In this step, the antisense strand AS of Conjugate N6-siHBa1 was synthesized using a universal solid phase support (UnyLinker™ loaded NittoPhase®HL Solid Supports, Kinovate Life Sciences, Inc.). The conditions of deprotection, coupling, capping, oxidation, deprotection and cleavage, and isolation in the solid phase synthesis method were the same as those used for the synthesis of the sense strand, to obtain the antisense strand AS.
Detection: The purity was determined by ion exchange chromatography (IEX-HPLC) with a purity of 93.2%;

and the molecular weight was analyzed by liquid chromatography-mass spectrometry (LC-MS). Calculated: 6675.04, measured: 6674.18.

(7-3) Synthesis of Conjugate N6-siHBa1

The sense strand synthesized in step (7-1) and the antisense strand synthesized in step (7-2) were mixed in an equimolar ratio, dissolved in water for injection and heated to 95°C, and then cooled at room temperature, such that they could form a double-stranded structure by hydrogen bonding.

After the above synthesis, molecular weight was determined using liquid chromatography-mass spectrometry (LC-MS, purchased from Waters Crop., model: LCT Premier). As a result, the calculated values of S and As are respectively 7748.37 and 6675.04; and the measured values of S and AS are respectively 7747.82 and 6674.43. The fact that the measured values were in conformity with the calculated values indicates that the synthesized drug conjugate has the designed target double-stranded nucleic acid sequence conjugated with two consecutive compounds as shown by Formula (N-6). Conjugate N6-siHBa1 (Conjugate 13) has a structure as shown by Formula (303).

**Preparation Example 8 Preparation of Conjugates 14-184 and Comparative Conjugate 1**

The drug conjugates were prepared by the same method as that in Preparation Example 7, except that: 1) the conjugated siRNAs had the sequences as shown in Tables 2A to 2G corresponding to Conjugates 14-184 and Comparative Conjugate 1; 2) for Conjugates 38-42, 60-64, 77-81, 94-98, 116-120, 154-158, and 179-184, the Compound N-$6_2$ linked to the solid phase support was respectively replaced with the Compound X-$2_2$ linked to the solid phase support, the Compound W-$2_2$ linked to the solid phase support, the Compound V-$2_2$ linked to the solid phase support, or the Compound O-$2_2$ linked to the solid phase support obtained in the above preparation example 6 (for example, Conjugates 38, 60, 77, 94, 116, and 154 were obtained when the Compound N-$6_2$ linked to the solid phase support was replaced by the Compound X-$2_2$ linked to the solid phase support; Conjugates 39, 61, 78, 95, 117, and 155 were obtained when the Compound N-$6_2$ linked to the solid phase support was replaced with the Compound W-$2_2$ linked to the solid phase support, and so on); 3) when the two nucleotides in the target sequence are linked via a phosphorothioate linkage, the oxidation reaction step in the linking of the latter one of the two nucleotides is replaced with the following sulfurization reaction step; the condition for sulfurization reaction in each step is identical, including a temperature of 25 °C, a reaction time of 300 seconds, and xanthane hydride as a sulfurization reagent; the molar ratio of the sulfurization reagent to the nucleic acid sequence linked to the solid phase support in the coupling step is 120:1; the reaction is carried out in a mixed solvent of acetonitrile: pyridine =1:1; and 4) when the 2'-positions of all nucleotides in the target sequence are modified hydroxyl groups, the cleavage and deprotection conditions do not include a step of removing 2'-O-TBDMS protection from the ribose.

Thus, drug Conjugates 14-183 and Comparative Conjugate 1 of the present disclosure were prepared and respectively numbered according to Tables 2A-2G. The molecular weight was determined by liquid chromatography-mass spectrometry (LC-MS) to confirm that the above conjugates had the structures as shown by Formula (303), (304), (305), (306), (307) or (308), respectively.

**Preparation Example 1B (synthesis of Compound FC-10)**

In this preparation example, Compound FC-10 was synthesized according to the follow method:

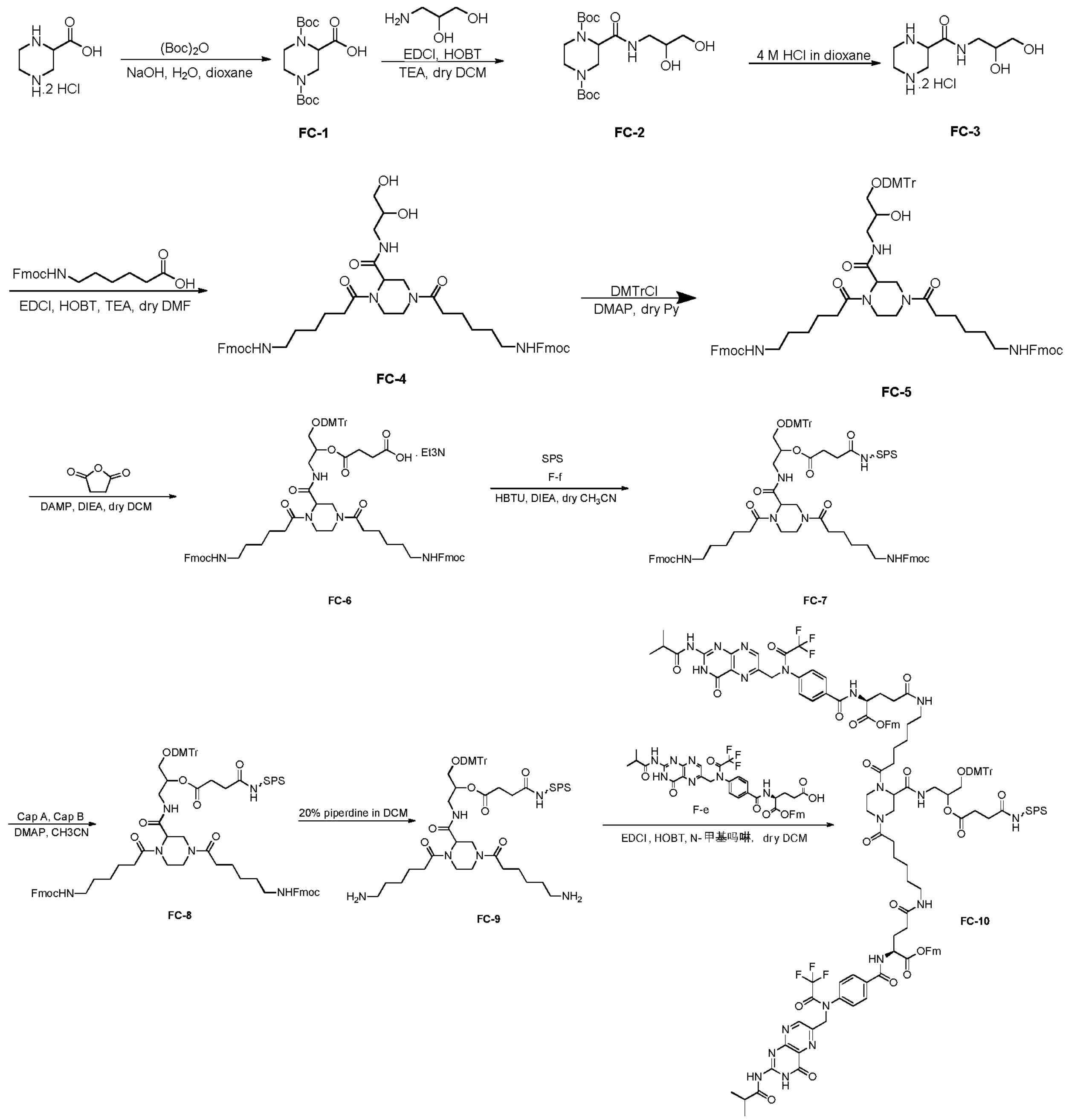


FC-1 FC-2 FC-3 FC-4 FC-5 FC-6 FC-7 FC-8 FC-9 FC-10

(1B-1) Synthesis of F-e (a terminal segment of the conjugation molecule)

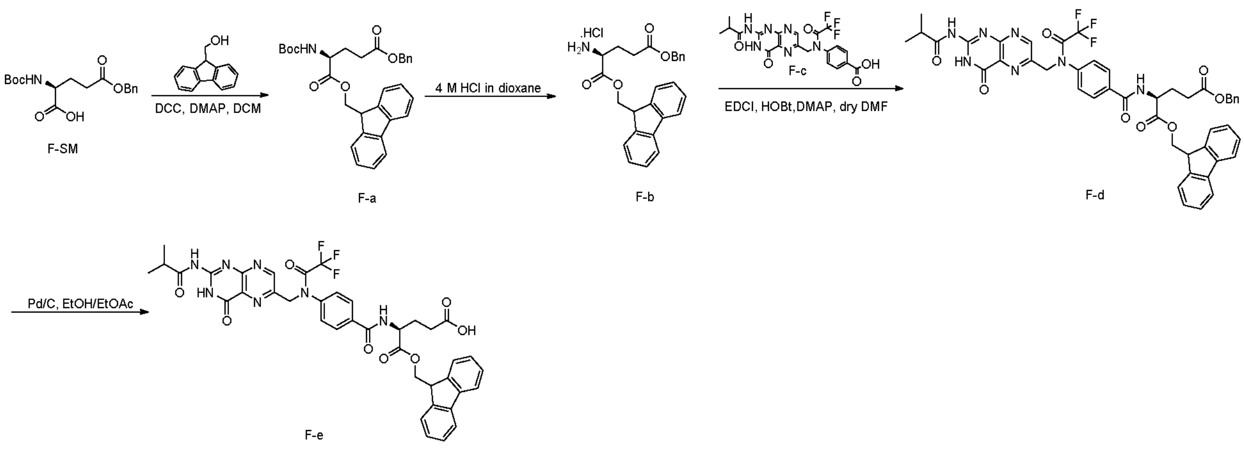


(1B-1-1a) Synthesis of F-a

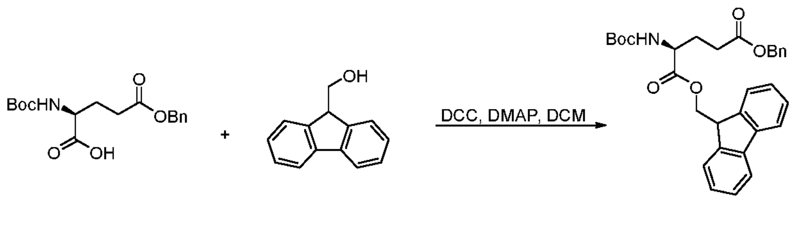


F-SM F-a

The compound as shown by Formula F-SM (purchased from Beijing Ouhe Technology Co., Ltd., CAS No.: 5793-73-8, 5.0 g), (9H-fluorene-9-yl)methanol (purchased from Beijing Ouhe Technology Co., Ltd., CAS No.: 24324-17-2, 3.2 g) and 4-dimethylaminopyridine (DMAP, CAS No.: 1122-58-3, purchased from Beijing Ouhe Technology Co., Ltd., 0.4 g) were dissloved in 80 mL of anhydrous dichloromethane, and added with dicyclohexylcarbodiimide (DCC, 3.7 g, purchased from Beijing Ouhe Technology Co., Ltd., CAS No.: 538-75-0) at 0°C under nitrogen atmosphere. After addition, the mixture was heated to room temperature and stirred for 4 hours. The solid formed was removed by filtration and the filtrate was concentrated under reduced pressure. The residue was purified by column chromatography (200-300 mesh normal phase silica gel, with a gradient elution of ethyl acetate: petroleum ether =1:10 - 1:6). The target product was collected and concentrated to give 4.6 g of product F-a as a colorless oil. MS m/z:[M+H]+ caculated: 516.2, measured: [M+H-BOC]: 515.9.

(1B-1-1b) Synthesis of F-b

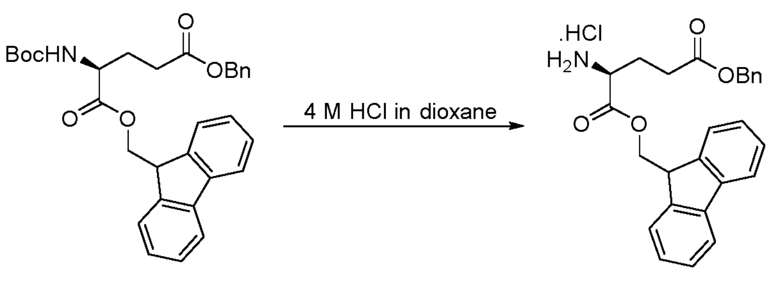


F-a F-b

Compound F-a (4.6 g) obtained in step (1B-1-1a) was dissolved in 40 mL solution of 4 mol/L of hydrogen chloride in dioxane. The mixture was stirred at room temperature for 2 hours. The mixture was concentrated under

reduce pressure to give 4.3 g of product F-b as a white gelatin. Caculated molecular weight: MS m/z: [M+H]+: 451.2, measured molecular weight: [M-HC1+H]+: 415.9.

(1B-1-1c) Synthesis of F-d

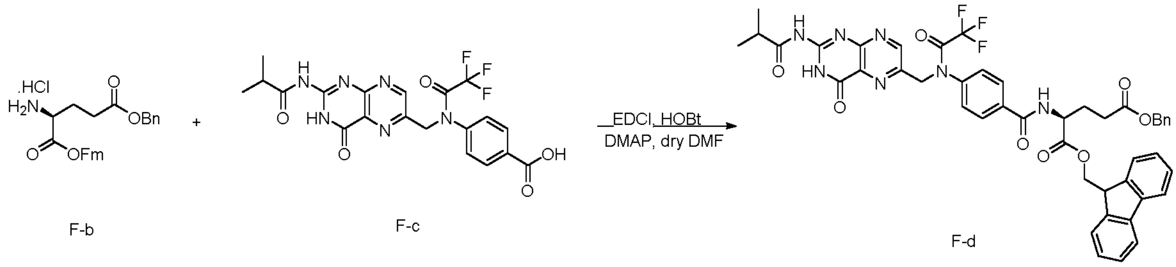


F-b F-c F-d

wherein, Fm in Formula F-b represents 9-fluorenylmethyl.

F-b (3.8 g) obtained in step (1B-1-1b), Compound F-c (4.0 g, CAS No. 252847-30-6, prepared with reference to the preparation method of Compounds 151 to 152 on page 113 as described in WO2009082607), 1-(3-dimethylaminopropyl)-3-ethylcarbodiimide hydrochloride (EDCI, CAS No. 25952-53-8, purchased from Beijing Ouhe Technology Co., Ltd., 1.9 g) and 1-hydroxybenzotriazole (HOBt, CAS No. 2592-95-2, purchased from Beijing Ouhe Technology Co., Ltd., 1.4 g) were dissloved in 40 mL of anhydrous dimethylformamide (DMF). N,N-diisopropylethylamine (DIEA, 4.3 g) were added to the mixed solution at 0°C under nitrogen atmosphere. After addition, the mixture was heated to room temperature and stirred for 2 hours, then quenched with 100 mL of saturated brine and extracted twice, each with 100 mL of ethyl acetate. The organic layers were combined and washed twice, each with 100 mL of saturated brine, then dried with anhydrous sodium sulfate, filtered to obtain an organic layer, which was concentrated under reduced pressure. The residue was purified by silica gel chromatography (with a gradient elution of ethyl acetate: petroleum ether =1:1 - 1:0) to give 1.0 g of product F-d as a light white solid. $^1$H NMR (300MHz, DMSO, P P m) 12.26 (s, 1H), 11.95 (s, 1H), 9.04-8.76 (m, 2H), 7.99-7.87 (m, 3H), 7.82 (dd, J=7.5, 4.1Hz, 2H), 7.72 (d, J=8.2Hz, 2H), 7.66-7.57 (m, 2H), 7.58-7.29 (m, 8H), 7.14 (d d, J=8.4, 6.1H z, 2H), 5.25 (s, 2H), 5.07 (d, J=5.1hz, 2H), 4.41 (d t, J=16.1, 11.6hz, 3H), 4.23 (t, J=6.7hz, 1H), 2.89 (s, 2H), 2.83-2.74 (m, 1H), 2.73 (s, 2H), 1.13 (d, J=6.8hz, 6H), MS m/z: [M+H]+ calculated: 876.3, measured: 875.7.

(1B-1-1d) Synthesis of F-e

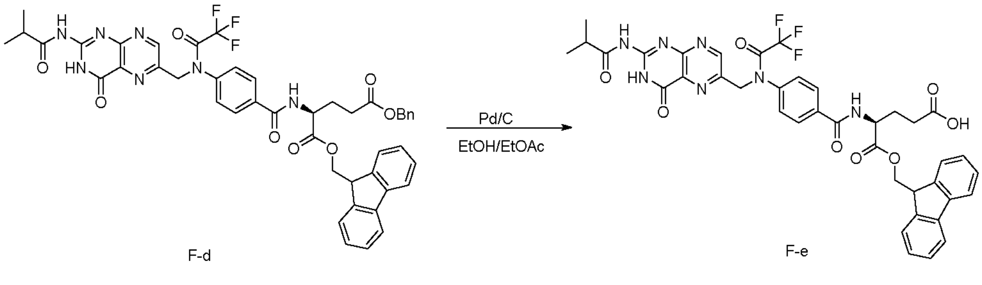


F-d F-e

F-d (1.0g) obtained in step (1B-1-1c) was dissolved in 30 mL of ethanol and 10 mL of ethyl acetate, and then the mixed solution was added with Pd/C (200 mg, 10 %w/w). The mixture was stirred at room temperature under hydrogen atmosphere for 4 hours and then stirred at room temperature in open air for 8 hours. The mixture was filtered and the filtrate was concentrated under reduced pressure. The residue was purified by a reversed-phase C18 column (with a gradient elution of water: acetonitrile = 0:1 - 4:6) and the solvent was removed by freeze-evaporation to give 400 mg of product F-e as a yellow solid. $^1$H NMR (300 MHz, DMSO) δ12.15 (d, J=124.0 Hz, 5H), 9.02-8.95 (m, 1H), 8.90 (d, J=6.1 Hz, 1H), 8.01-7.55 (m, 10H), 7.45-7.51 (m, 3H), 7.25-7.12 (m, 2H), 5.29-5.20 (s, 2H), 4.60-4.45 (m, 1H), 4.42-4.31 (m, 2H), 4.29-4.20 (m,1H), 2.84-2.73 (m,1H), 2.40-2.25 (m, 3H), 2.15-2.00 (m, 2H), 2.00-1.85 (m, 1H), 1.13 (d, J=6.8Hz, 6H), MS m/z: [M+H]+ calculated: 786.2, measured: 785.7.

Hereinafter, Compound FC-10 was synthesized by the following process route by using the Compound F-e

obtained according to the above method:

(1B-2) Synthesis of FC-1

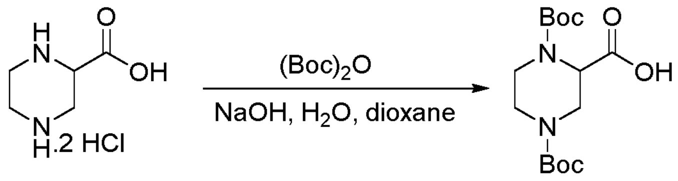


**FC-1**

Sodium hydroxide (15.76 g) was dissolved in 300 mL of water at a temperature of 0°C - 5°C, and the resultant solution was added with piperazine-2-carboxylic acid dihydrochloride (CAS No. 3022-15-9, purchased from Beijing Ouhe Technology Co., Ltd., 20.00 g) to obtain the reaction mixture. Di-tert-butyl methyl dicarbonate ($(Boc)_2O$, CAS No. 24424-99-5, purchased from Beijing Ouhe Technology Co., Ltd., 47.30 g) was dissolved in 200 mL of a dioxane solution and added to the reaction mixture. The mixture was stirred at room temperature for 15 hours. The organic solvent was removed under reduced pressure and the remaining aqueous phase was cooled to 0°C-5°C, acidified to pH=3 with 3N hydrochloric acid and extracted twice, each with 300 mL of ethyl acetate. The organic layers were combined and washed once with 300 mL of water, dried with anhydrous sodium sulfate, filtered and concentrated under reduced pressure to give 29.0 g of product FC-1 as a white solid. 1H NMR (300 MHz, DMSO) δ 12.88 (s, 1H), 4.54-4.16 (m, 2H), 3.79 (d, J=10.3 Hz, 1H), 3.62 (d, J=12.7 Hz, 1H), 3.16-2.87 (m, 2H), 2.85-2.65 (m, 1H), 1.38-1.28 (m, 18H), MS m/z: [M+H]+ calculated: 330.2, measured: $[2M+H^++Na^+]$=682.9.

(1B-3) Synthesis of FC-2

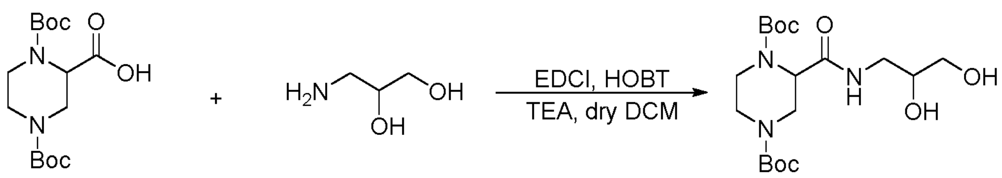


**FC-1** **FC-2**

FC-1 (27.70 g) obtained in step (1B-2), 1-hydroxybenzotriazole (HOBt, 13.60 g) and triethylamine (TEA, 34.00 g, Beijing Ouhe Technology Co., Ltd., CAS No. 121-44-8) were dissloved in 150 mL of anhydrous dimethylformamide and continuously stirred. The stirred mixed solution was added with 1-(3-dimethylaminopropyl)-3-ethylcarbodiimide hydrochloride (EDCI, 19.30 g) and then 3-aminopropane-1,2-diol (8.40 g, Beijing Ouhe Technology Co., Ltd., Cas No. 616-30-8) in portions at a temperature of 0 °C. After addition, the mixture was stirred at room temperature for 5 hours. The reaction was cooled by addition of 150 mL of saturated brine, and the recation solution was extracted twice, each with 150 mL of ethyl acetate. The organic layers were combined and washed twice, each with 150 mL saturated brine, then dried with anhydrous sodium sulfate, filtered, and concentrated under reduced pressure. The concentrate was purified by silica gel chromatography (with a gradient elution of ethyl acetate: petroleum ether: dichloromethane: methanol =1:1:1:0 - 1:1:1:0.2) to give 21.0 g of product FC-2 as a white solid.

$^1$HNMR (300 MHz, CDCl3) δ6.67-6.51 (m, 1H), 4.56 (s, 1H), 4.44 (d, J=13.3 Hz, 1H), 3.95-3.66 (m, J=43.2, 7.2 Hz, 3H), 3.65-2.81 (m, 9H), 1.43 (d, J=5.5 Hz, 18H), MS m/z: [M+H]+ calculated: 404.2, measured: 404.0.

(1B-4) Synthesis of FC-3

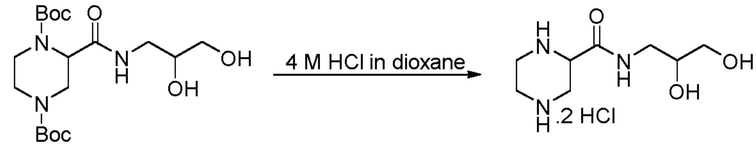


FC-2 FC-3

FC-2 (17.00 g) obtained in step (1B-3) was dissolved in 100 mL solution of 4 mol/L hydrogen chloride in dioxane under stirring at room temperature for 1 hour. The mixture was concentrated under reduced pressure to give 12.0 g of product FC-3 as a white solid. MS m/z: [M+H]+ calculated: 204.1, measured: 204.1.

(1B-5) Synthesis of FC-4

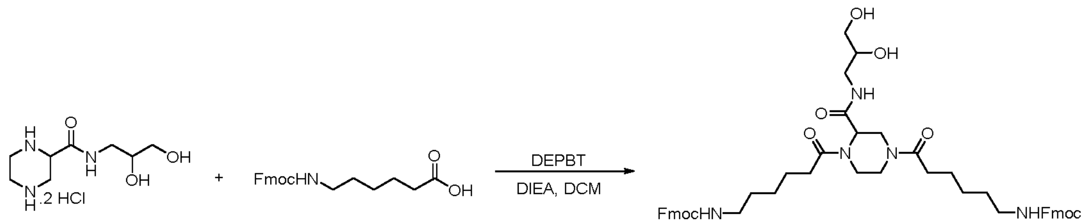


FC-3 FC-4

250 mL of anhydrous dichloromethane was added with 3-(diethoxyphosphoryloxy)-1,2,3-benzotrizin-4(3H)-one (DEPBT, 25.40 g, purchased from Beijing Ouhe Technology Co., Ltd., CAS No. 165534-43-0) at room temperature under continuously stirring. Then, the mixed solution was added with 6-((((9H-fluoren-9-yl)methoxy)carbonyl)amino)hexanoic acid (CAS No. 88574-06-5, purchased from Beijing Ouhe Technology Co., Ltd., 24.00 g) and N,N-diisopropylethylamine (DIEA, 22.00 g). The mixture was stirred at room temperature for 30 minutes. At a temperature of 0°C, FC-3 (7.80 g) obtained in step (1B-4) was added. After addition, the mixture was stirred at room temperature for 18 hours, quenched with 150 mL saturated brine and extracted twice, each with 150 mL of dichloromethane. The organic layers were combined and washed twice, each with 150 mL of saturated brine, dried with anhydrous sodium sulfate, filtered, and concentrated under reduced pressure. The residue was purified by column chromatography (200-300 mesh normal phase silica gel, with a gradient elution of ethyl acetate: petroleum ether: dichloromethane: methanol =1:1:1:0.2 - 1:1:1:0.4) to give 14.7 g of product FC-4 as a light yellow solid. MS m/z: [M+H]+ calculated: 874.4, measured: 873.8.

(1B-6) Synthesis of FC-5

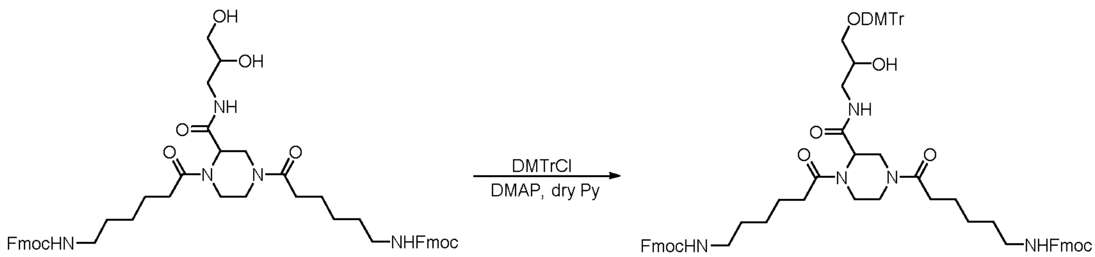


FC-4 FC-5

40 mL of anhydrous pyridine was added with 4,4'-di-methoxytrityl chloride (DMTrCl, 3.70 g) under continuously stirring at 0°C. Then, the mixed solution was added with product FC-4 (8.00 g) obtained in 1B-5 and 4-dimethylaminopyridine (0.22 g). The mixture was stirred overnight at room temperature under nitrogen atmosphere. The mixture was

concentrated under reduced pressure and purified by column chromatography (200-300 mesh normal phase silica gel, wherein the columen was first equilibrated with 0.1%(v/v) triethylamine in petroleum ether, and then eluted with a gradient elution of ethyl acetate: petroleum ether: dichloromethane: methanol =1:1:1:0.1 - 1:1:1:0.3) to give 7.5 g of product FC-5 as a white solid. MS m/z:[m+Na]+ calculated: 1198.6, measured: 1198.6.

(1B-7) Synthesis of FC-6

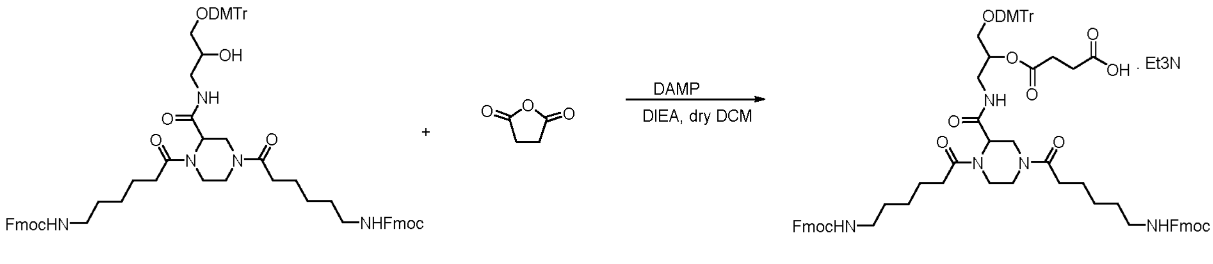


FC-5 FC-6

60 mL of anhydrous dichloromethane was added with succinic anhydride (CAS No. 108-30-5, purchased from Beijing Ouhe Technology Co., Ltd., 0.43 g), FC-5(3.60 g) at room temperature. The mixed solution was added with FC-5 (3.6 g) obtained in step (1B-6), N,N-diisopropylethylamine (DIEA 1.97 g) and 4-dimethylaminopyridine (DMAP 37.30 mg), and then the resultant mixture was stirred overnight at room temperature under nitrogen atmosphere. The mixture was purified by silica gel chromatography (the columen was first equilibrated with 0.1%(v/v) triethylamine in petroleum ether, and then eluted with a gradient elution of dichloromethane: methanol =20:1 - 10:1) to give 4.0 g of product FC-6 as a white solid. MS m/z: [m+Na]+ calculated: 1399.7, measured: [m-Et3N+Na]: 1298.6.

(1B-8) Synthesis of FC-7

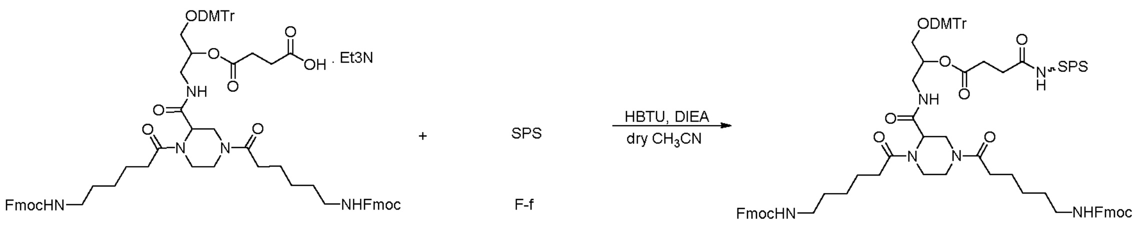


SPS F-f

FC-6 FC-7

40 mL of anhydrous acetonitrile was added with benzotriazol-N,N,N',N'-tetramethylurea hexafluorophosphate (HBTU, CAS No. 94790-37-1, purchased from Beijing Ouhe Technology Co., Ltd., 0.83 g) at room temperature under continuously stirring, and then added with FC-6 (2.0 g) obtained in step (1B-7) and N,N-diisopropylethylamine (DIEA, 0.40 g). The resultant mixture was then stirred at room temperature under nitrogen atmosphere for 30 minutes. Then, the mixture was added with amino resin (4.1 g, 400 μmol/g, purchased from Tianjin Nankai Hecheng Technology Co., Ltd., product model: HC4025), and rotated to react for 21 hours in a shaking reactor. The mixture was filtered, washed once with 50 mL of dichloromethane and once with 50 mL of acetonitrile. The residue was dried under reduced pressure to give 5.4 g of product FC-7 as a yellow solid, with a loading of 269 μmol/g.

(1B-9) Synthesis of FC-8

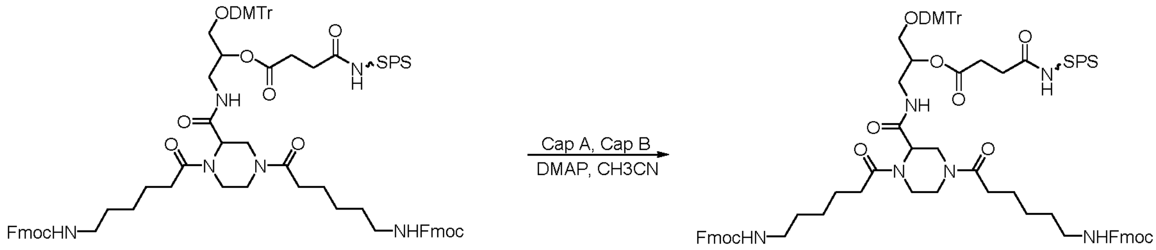


FC-7 FC-8

The capping reagent A (CapA, 121.5 mL, 22.5 mL/g) and the capping reagent B (CapB, 13.5 mL, 2.5 mL/g) were mixed well, wherein CapA is a 20% by volume of mixed solution of N-methylimidazole in pyridine/acetonitrile with a volume ratio of pyridine to acetonitrile of 3:5; and CapB is a 20% by volume solution of acetic anhydride in acetonitrile. The resultant mixture was then added to a mixture of 4-dimethylaminopyridine (DMAP, 67.5 mg, 0.0125 g/g) and acetonitrile (13.5 mL, 2.5 mL/g). The above mixture was mixed thoroughly and added with FC-7 (5.4 g, 1.0 g/g) obtained in step (1B-8). The resultant mixture was rotated to react at room temperature for 5 hours in a shaking reactor. The reaction mixture was isolated by filtration, and the residue was washed once with 50 mL of acetonitrile and dried under reduced pressure to give 5.6 g of product FC-8 as a light yellow solid.

(1B-10) Synthesis of FC-9

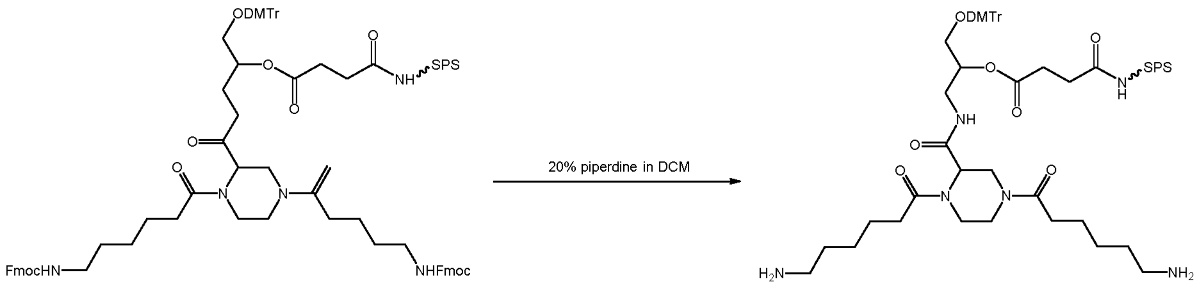


FC-9

FC-8

5.6 g of FC-8 obtained in step (1B-9) was added to 44 mL solution of piperidine in dichloromethane (20% v/v) and the resultant mixture was rotated to react for 5 hours in a shaking reactor. The resultant mixture was filtered, washed once with 150 mL of acetonitrile, and the residue was dried under reduced pressure to give 5.0 g of product FC-9 as a yellow solid.

(1B-11) Synthesis of FC-10

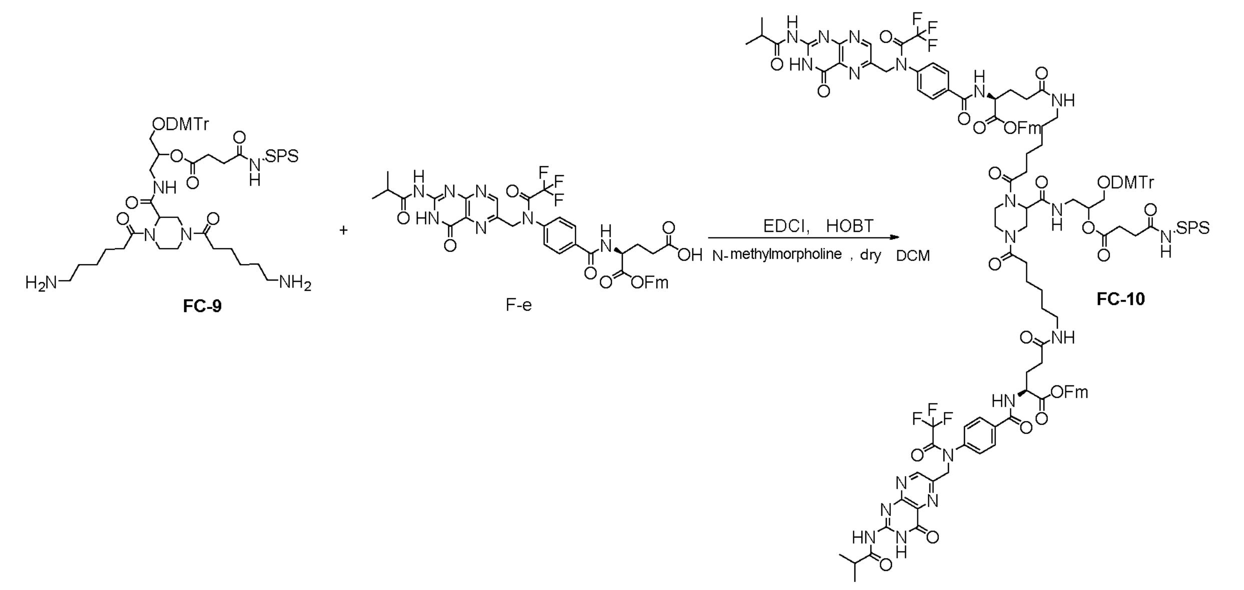


FC-9 + F-e → FC-10

FC-9 (0.86 g) obtained in step (1B-10), Compound F-e (400 mg) obtained in step (1B-1-1d), 1-hydroxybenzotriazole (HOBt, 78 mg), N-methylmorpholine (151 mg), and 1-ethyl-(3-dimethylaminopropyl)carbodiimide hydrochloride (EDCI, 112 mg) were dissolved in 12 mL of anhydrous 1,2-dichloroethane. The resultant mixture was rotated at room temperature for 18 hours in a shaking reactor. The resultant mixture was filtered, and washed with 30 mL of acetonitrile and 30 mL of dichloromethane. The residue was dried under reduced pressure to give 800 mg of product FC-10 as a yellow solid, with a loading of 269 μmol/g.

**Preparation Example 2B Synthesis of Conjugate 185**

(2B-1) Synthesis of the sense strand of Conjugate FC-siSTAT1

In this step, the siRNA of the drug conjugate is the sequences numbered siSTAT1:

siSTAT1
Sense strand:
5'-CmsUmsAmGmAmAmAfAfCfUmGmGmAmUmAmAmCmGmUm-3' (SEQ ID NO: 723),
Antisense strand:
5'-AmsCfsGmUmUmAfUmCmCmAmGmUmUmUfUmCfUmAmGmsCmsCm-3' (SEQ ID NO: 724);
wherein 3' terminal of the sense strand was conjugated with Compound FC-10, and 5' terminal of the antisense strand was linked to a Cy5 fluorescent group.

Nucleoside monomers were linked one by one in 3' to 5' direction according to the above sequence order by a phosphoramidite solid phase synthesis method, starting from Compound FC-10 prepared in the above step instead of the solid phase support in the solid phase synthesis method. The linking of each nucleoside monomer included a four-step reaction of deprotection, coupling, capping, and oxidation. Therein, when two nucleotides are linked via a phosphoester linkage, a four-step reaction of deprotection, coupling, capping, and oxidation was included during the linking of the latter nucleoside monomer; and when two nucleotides are linked via a phosphorothioate linkage, a four-step reaction of deprotection, coupling, capping, and sulfurization was included during the linking of the latter nucleoside monomer. The synthesis conditions are as follows.

The nucleoside monomers are provided in a 0.1 M acetonitrile solution. The condition for deprotection reaction in each step is identical, i.e., a temperature of 25°C, a reaction time of 70 seconds, a solution of dichloroacetic acid in dichloromethane (3% v/v) as a deprotection reagent, and a molar ratio of dichloroacetic acid to the protection group 4,4'-dimethoxytrityl on the solid phase support of 5:1.

The condition for coupling reaction in each step is identical, including a temperature of 25°C, a molar ratio of the nucleic acid sequence linked to the solid phase support to nucleoside monomers of 1:10, a molar ratio of the nucleic acid sequence linked to the solid phase support to a coupling reagent of 1:65, a reaction time of 600 seconds, and a solution of 0.5 M 5-ethylthio-1H-tetrazole (ETT) in acetonitrile as a coupling reagent.

The condition for capping reaction in each step is identical, including a temperature of 25°C, a reaction time of

15 seconds, a mixed solution of Cap A and Cap B in a molar ratio of 1:1 as a capping agent, in which CapA is a 20% by volume of mixed solution of N-methylimidazole in pyridine/acetonitrile, with the volume ratio of pyridine to acetonitrile being 3:5; CapB is a 20% by volume solution of acetic anhydride in acetonitrile; and a molar ratio of the capping agent to the nucleic acid sequence linked to the solid phase support of 1:1:1 (acetic anhydride: N-methylimidazole: the nucleic acid sequence linked to the solid phase support).

The condition for oxidation reaction in each step is identical, including a temperature of 25 °C, a reaction time of 15 seconds, and 0.05 M iodine water as an oxidation reagent; and a molar ratio of iodine to the nucleic acid sequence linked to the solid phase support in the coupling step of 30:1. The reaction is carried out in a mixed solvent of tetrahydrofuran: water: pyridine (3:1:1).

The condition for sulfurization reaction in each step is identical, including a temperature of 25 °C, a reaction time of 300 seconds, and xanthane hydride as a sulfurization reagent; and a molar ratio of the sulfurization reagent to the nucleic acid sequence linked to the solid phase support in the coupling step of 120:1. The reaction is carried out in a mixed solvent of acetonitrile: pyridine = 1:1.

After the linking of the last nucleoside monomer, the nucleic acid sequence linked on the solid phase support is sequentially subjected to cleavage, deprotection, purification, desalination, and then lyophilization to obtain the sense strand. Therein, the conditions for cleavage and deprotection are as follows: adding the synthesized nucleotide sequence linked to the support into 25 wt% aqueous ammonia to react at 55 °C for 16 hours, wherein the amount of the aqueous ammonia is 0.5 mL/μmol. The remaining support was removed by filtration. The supernatant was concentrated to dryness in vacuum, then added with an excess solution of 20% piperidine in dichloromethane, incubated at room temperature for 4h to remove the Fm group, and concentrated to dryness in vacuum.

The conditions of purification and desalination are as follows: purification of the nucleic acid is achieved by using a preparative ion chromatography purification column (Source 15Q) with a gradient elution of NaCl. Specifically, eluent A is 20 mM sodium phosphate (pH 8.1), solvent is water/acetonitrile in 9:1 (v/v); eluent B is 1.5 M sodium chloride, 20 mM sodium phosphate (pH 8.1), solvent is water/acetonitrile in 9:1 (v/v); elution gradient: the ratio of eluent A: eluent B = 100:0-50:50. The eluate is collected, combined and desalted by using a reverse phase chromatography purification column. The specific condition comprises: using a Sephadex column for desalination (filler: Sephadex G25) and eluting with deionized water.

Detection: The purity was determined by Ion exchange chromatography (IEX-HPLC) with a purity of 92.4%. The molecular weight was analyzed by liquid chromatography-mass spectrometry (LC-MS), calculated: 7253.96, measured: 7253.12.

Thus, in this step, Compound FC-10 was linked to 3' terminal of the resultant sense strand to obtain an sense strand S of the siRNA in which Compound FC-10 was conjugated to 3' terminal of the siRNA.

(2B-2) Synthesis of the antisense strand of Conjugate FC-siSTAT1

The antisense strand of Conjugate FC-siSTAT1 was prepared by the same method as that for preparing the antisense strand of Conjugate 29, except that: (1) after the linking of the last nucleoside monomer in the antisense strand, an additional Cy5 fluorescent group was linked to the antisense strand; (2) the conditions for cleavage and deprotection of the antisense strand are different.

Specifically, in (1), during the process of preparing the antisense strand by the solid phase phosphoramidite method as described in step (7-2) of Preparation Example 1, after the linking of the last nucleoside monomer in antisense strand, the Cy5 phosphoramidite monomer as shown by Formula (901) (purchased from Shanghai Zhaowei Technology Development Co., Ltd., Cat No. OP-057) was linked to 5' terminal of the antisense strand through a four-step reaction of deprotection, coupling, capping, and oxidation. Therein, the reaction conditions for deprotection, coupling, capping and oxidation used are the same as those for the synthesis of the antisense strand in step (7-2), except that: 1) the reaction time of deprotection is extended to 300 seconds; 2) the reaction time of Cy5 coupling is extended to 900 seconds.

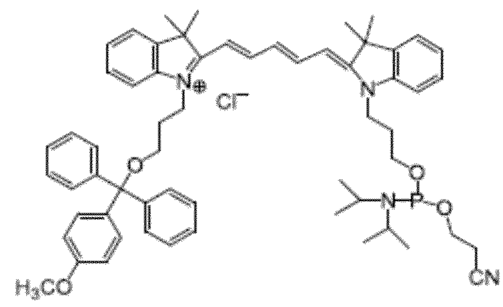

Formula (901)

In (2), the conditions for cleavage and deprotection are as follows: adding the synthesized nucleotide sequence linked to the support into an AMA solution (a mixed solution of 40 wt% aqueous methylamine solution and 25 wt% aqueous ammonia in a volume ratio of 1: 1) to react in a water bath of 25°C for 2h, wherein the amount of the AMA solution is 0.5 mL/μmol. The remaining support was removed by filtration, and the supernatant was concentrated in vacuum to dryness. The conditions for purification and desalination of the antisense strand are the same as those for synthesis of the antisense strand in step (7-2). Subsequently, the antisense strand was lyophilized to give the antisense strand AS of Conjugate FC-siSTAT1.

Thus, the drug conjugate Conjugate FC-siSTAT1, was obtained, which has a Cy5 fluorescent group covalently linked to 5' terminal of the antisense strand of the siRNA thereof, and has the sense strand sequences and the antisense strand sequences as shown in Table 2H corresponding to the drug conjugate, Conjugate FC-siSTAT1.

Detection: The purity was determined by ion exchange chromatography (IEX-HPLC) with a purity of 93.2%; The molecular weight was analyzed by liquid chromatography-mass spectrometry (LC-MS). Calculated: 6675.04, measured: 6674.50.

(2B-3) Synthesis of Conjugate FC-siSTAT1

The sense strand obtained in step (2B-1) and the antisense strand obtained in step (2B-2) were dissolved in water for injection, respectively, to obtain a solution of 40 mg/mL. They were mixed in an equimolar ratio, heated at 50°C for 15 minutes, and cooled to room temperature to form a double-stranded structure by hydrogen bond.

After the above synthesis, the conjugate was diluted to a concentration of 0.2 mg/mL by using ultra-pure water (homemade by Milli-Q ultra-pure water instrument, with resistivity of 18.2 MΩ* cm (25°C)). The molecular weight was determined by a LC-MS instrument (LC-MS, liquid chromatography-mass spectrometry, purchased from Waters Crop., model: LCT Premier). As a result, the calculated value of molecular weight for S was 7253.96, and that for AS was 6675.04; the measured value of molecular weight for S was 7253.24, and that for AS was 6674.61. The fact that the measured value was in conformity with the calculated value indicated that the synthesized conjugate has the designed target double-stranded nucleic acid sequence with Compound FC-10. Conjugate FC-siSTAT1 (Conjugate 185) has a structure as shown by Formula (311).

Table 2 drug conjugates

Table 2A

| Conjugate serial number | Conjugate No. | Sequence direction 5' - 3' | | SEQ ID NO |
|---|---|---|---|---|
| Conjugate 14 | N6-siHBa1 | S | CCUUGAGGCAUACUUCAAA | 331 |
| | | AS | UUUGAAGUAUGCCUCAAGGUU | 332 |
| Conjugate 15 | N6-siHBa2 | S | GACCUUGAGGCAUACUUCAAA | 333 |
| | | AS | UUUGAAGUAUGCCUCAAGGUCGG | 334 |

(continued)

| Conjugate serial number | Conjugate No. | Sequence direction 5' - 3' | | SEQ ID NO |
|---|---|---|---|---|
| Conjugate 16 | N6-siHBa1M1 | S | CmCmUmUmGmAmGfGfCfAmUmAmCmUmUm CmAmAmAm | 335 |
| | | AS | UmUfUmGmAmAfGmUmAmUmGmCmCmUfCm AfAmGmGmUmUm | 336 |
| Conjugate 17 | N6-siHBa2M2 | S | CmCmUmUmGfAmGfGfCfAmUmAmCmUmUmC mAmAmAm | 337 |
| | | AS | UmUfUmGmAmAfGmUfAfUmGmCmCmUfCmAf AmGmGmUmUm | 338 |
| Conjugate 18 | N6-siHBa2M1 | S | GmAmCmCmUmUmGmAmGfGfCfAmUmAmCm UmUmCmAmAmAm | 339 |
| | | AS | UmUfUmGmAmAfGmUmAmUmGmCmCmUfCm AfAmGmGmUmCmGmGm | 340 |
| Conjugate 19 | N6-siHBa2M2 | S | GmAmCmCmUmUmGfAmGfGfCfAmUmAmCmU mUmCmAmAmAm | 341 |
| | | AS | UmUfUmGmAmAfGmUfAfUmGmCmCmUfCmAf AmGmGmUmCmGmGm | 342 |
| Conjugate 20 | N6-siHBa1M1S | S | CmsCmsUmUmGmAmGfGfCfAmUmAmCmUmU mCmAmAmAm | 343 |
| | | AS | UmsUfsUmGmAmAfGmUmAmUmGmCmCmUfC mAfAmGmGmsUmsUm | 344 |
| Conjugate 21 | N6-siHBa1M2S | S | CmsCmsUmUmGfAmGfGfCfAmUmAmCmUmUm CmAmAmAm | 345 |
| | | AS | UmsUfsUmGmAmAfGmUfAfUmGmCmCmUfCm AfAmGmGmsUmsUm | 346 |
| Conjugate 22 | N6-siHBa2M1S | S | GmsAmsCmCmUmUmGmAmGfGfCfAmUmAmC mUmUmCmAmAmAm | 347 |
| | | AS | UmsUfsUmGmAmAfGmUmAmUmGmCmCmUfC mAfAmGmGmUmCmsGmsGm | 348 |
| Conjugate 23 | N6-siHBa2M2S | S | GmsAmsCmCmUmUmGfAmGfGfCfAmUmAmCm UmUmCmAmAmAm | 349 |
| | | AS | UmsUfsUmGmAmAfGmUfAfUmGmCmCmUfCm AfAmGmGmUmCmsGmsGm | 350 |

(continued)

| Conjugate serial number | Conjugate No. | Sequence direction 5' - 3' | | SEQ ID NO |
|---|---|---|---|---|
| Conjugate 24 | N6-siHBa1M1 VP | S | CmCmUmUmGmAmGfGfCfAmUmAmCmUmUm CmAmAmAm | 351 |
| | | AS | VP-UmUfUmGmAmAfGmUmAmUmGmCmCmUfCm AfAmGmGmUmUm | 352 |
| Conjugate 25 | N6-siHBa1M2VP | S | CmCmUmUmGfAmGfGfCfAmUmAmCmUmUmC mAmAmAm | 353 |
| | | AS | VP-UmUfUmGmAmAfGmUfAfUmGmCmCmUfCmAf AmGmGmUmUm | 354 |
| Conjugate 26 | N6-siHBa2M1VP | S | GmAmCmCmUmUmGmAmGfGfCfAmUmAmCm UmUmCmAmAmAm | 355 |
| | | AS | VP-UmUfUmGmAmAfGmUmAmUmGmCmCmUfCm AfAmGmGmUmCmGmGm | 356 |
| Conjugate 27 | N6-siHBa2M2VP | S | GmAmCmCmUmUmGfAmGfGfCfAmUmAmCmU mUmCmAmAmAm | 357 |
| | | AS | VP-UmUfUmGmAmAfGmUfAfUmGmCmCmUfCmAf AmGmGmUmCmGmGm | 358 |
| Conjugate 28 | N6-siHBa1M1SVP | S | CmsCmsUmUmGmAmGfGfCfAmUmAmCmUmU mCmAmAmAm | 359 |
| | | AS | VP-UmsUfsUmGmAmAfGmUmAmUmGmCmCmUfC mAfAmGmGmsUmsUm | 360 |
| Conjugate 29 | N6-siHBa1M1SP | S | CmsCmsUmUmGmAmGfGfCfAmUmAmCmUmU mCmAmAmAm | 361 |
| | | AS | P-UmsUfsUmGmAmAfGmUmAmUmGmCmCmUfC mAfAmGmGmsUmsUm | 362 |
| Conjugate 30 | N6-siHBa1M1SPs | S | CmsCmsUmUmGmAmGfGfCfAmUmAmCmUmU mCmAmAmAm | 363 |
| | | AS | Ps-UmsUfsUmGmAmAfGmUmAmUmGmCmCmUfC mAfAmGmGmsUmsUm | 364 |

(continued)

| Conjugate serial number | Conjugate No. | Sequence direction 5' - 3' | | SEQ ID NO |
|---|---|---|---|---|
| Conjugate 31 | N6-siHBa3M1S | S | CmsCmsUmUmGmAmGfGfCfAmUmAmCmUmUmCmAmAmUm | 365 |
| | | AS | AmsUfsUmGmAmAfGmUmAmUmGmCmCmUfCmAfAmGmGmsUmsUm | 366 |
| Conjugate 32 | N6-siHBa1M2SVP | S | CmsCmsUmUmGfAmGfGfCfAmUmAmCmUmUmCmAmAmAm | 367 |
| | | AS | VP-UmsUfsUmGmAmAfGmUfAfUmGmCmCmUfCmAfAmGmGmsUmsUm | 368 |
| Conjugate 33 | N6-siHBa2M1SVP | S | GmsAmsCmCmUmUmGmAmGfGfCfAmUmAmCmUmUmCmAmAmAm | 369 |
| | | AS | VP-UmsUfsUmGmAmAfGmUmAmUmGmCmCmUfCmAfAmGmGmUmCmsGmsGm | 370 |
| Conjugate 34 | N6-siHBa2M2SVP | S | GmsAmsCmCmUmUmGfAmGfGfCfAmUmAmCmUmUmCmAmAmAm | 371 |
| | | AS | VP-UmsUfsUmGmAmAfGmUfAfUmGmCmCmUfCmAfAmGmGmUmCmsGmsGm | 372 |
| Conjugate 35 | N6-siHBa1M5SVP | S | CmsCmsUmUmGfAmGfGfCfAmUmAmCmUmUmCmAmAmAm | 373 |
| | | AS | VP-UmsUfsUmGmAmAfGmUmAmUmGmCmCmUfCmAfAmGmGmsUmsUm | 374 |
| Conjugate 36 | N6-siHBa1M3SVP | S | CmsCmsUmUmGmAmGfGmCfAmUfAmCmUmUmCmAmAmAm | 375 |
| | | AS | VP-UmsUfsUmGmAmAfGmUmAmUmGmCmCmUfCmAfAmGmGmsUmsUm | 376 |
| Conjugate 37 | N6-siHBa1M4SVP | S | CmsCmsUmUmGmAmGfGfCfAmUmAmCmUmUmCmAmAmAm | 377 |
| | | AS | VP-UmsUfsUmGmAmAfGmUfAmUmGmCmCmUfCmAfAmGmGmsUmsUm | 378 |

(continued)

| Conjugate serial number | Conjugate No. | Sequence direction 5' - 3' | | SEQ ID NO |
|---|---|---|---|---|
| Conjugate 38 | X2-siHBa1M2SVP | S | CmsCmsUmUmGfAmGfGfCfAmUmAmCmUmUmCmAmAmAm | 379 |
| | | AS | VP-UmsUfsUmGmAmAfGmUfAfUmGmCmCmUfCmAfAmGmGmsUmsUm | 380 |
| Conjugate 39 | W2-siHBa1M2SVP | S | CmsCmsUmUmGfAmGfGfCfAmUmAmCmUmUmCmAmAmAm | 381 |
| | | AS | VP-UmsUfsUmGmAmAfGmUfAfUmGmCmCmUfCmAfAmGmGmsUmsUm | 382 |
| Conjugate 40 | V2-siHBa1M2SVP | S | CmsCmsUmUmGfAmGfGfCfAmUmAmCmUmUmCmAmAmAm | 383 |
| | | AS | VP-UmsUfsUmGmAmAfGmUfAfUmGmCmCmUfCmAfAmGmGmsUmsUm | 384 |
| Conjugate 41 | 02-siHBa1M1SVP | S | CmsCmsUmUmGmAmGfGfCfAmUmAmCmUmUmCmAmAmAm | 385 |
| | | AS | VP-UmsUfsUmGmAmAfGmUmAmUmGmCmCmUfCmAfAmGmGmsUmsUm | 386 |
| Conjugate 42 | P2-siHBa1M1SVP | S | CmsCmsUmUmGmAmGfGfCfAmUmAmCmUmUmCmAmAmAm | 387 |
| | | AS | VP-UmsUfsUmGmAmAfGmUmAmUmGmCmCmUfCmAfAmGmGmsUmsUm | 388 |
| Comparative Conjugate 1 | N6-NC | S | UUCUCCGAACGUGUCACGU | 389 |
| | | AS | ACGUGACACGUUCGGAGAAUU | 390 |

Table 2B

| Conjugate serial number | Conjugate No. | Sequence direction 5'-3' | | SEQ ID NO |
|---|---|---|---|---|
| Conjugate 43 | N6-siHBb1M1S | S | UmsGmsCmUmAmUmGfCfCfUmCmAmUmCmUmUmCmUmAm | 391 |
| | | AS | UmsAfsGmAmAmGfAmUmGmAmGmGmCmAfUmAfGmCmAmsGmsCm | 392 |

(continued)

| Conjugate serial number | Conjugate No. | Sequence direction 5'-3' | | SEQ ID NO |
|---|---|---|---|---|
| Conjugate 44 | N6-siHBb2M1S | S | UmsGmsCmUmAmUmGfCfCfUmCmAmUmCmUmUmCmUmAm | 393 |
| | | AS | UmsAfsGmAmAmGfAmUmGmAmGmGmCmAfUmAfGmCmAmsUmsUm | 394 |
| Conjugate 45 | N6-siHBb1M2 | S | UmGmCmUmAfUmGfCfCfUmCmAmUmCmUmUmCmUmAm | 395 |
| | | AS | UmAfGmAmAmGfAmUfGfAmGmGmCmAfUmAfGmCmAmGmCm | 396 |
| Conjugate 46 | N6-siHBb2M2 | S | UmGmCmUmAfUmGfCfCfUmCmAmUmCmUmUmCmUmAm | 397 |
| | | AS | UmAfGmAmAmGfAmUfGfAmGmGmCmAfUmAfGmCmAmUmUm | 398 |
| Conjugate 47 | N6-siHBb1M1SV P | S | UmsGmsCmUmAmUmGfCfCfUmCmAmUmCmUmUmCmUmAm | 399 |
| | | AS | VP-UmsAfsGmAmAmGfAmUmGmAmGmGmCmAfUmAfGmCmAmsGmsCm | 400 |
| Conjugate 48 | N6-siHBb2M1SP | S | UmsGmsCmUmAmUmGfCfCfUmCmAmUmCmUmUmCmUmAm | 401 |
| | | AS | P-UmsAfsGmAmAmGfAmUmGmAmGmGmCmAfUmAfGmCmAmsGmsCm | 402 |
| Conjugate 49 | N6-siHBb2M1SP s | S | UmsGmsCmUmAmUmGfCfCfUmCmAmUmCmUmUmCmUmAm | 403 |
| | | AS | Ps-UmsAfsGmAmAmGfAmUmGmAmGmGmCmAfUmAfGmCmAmsGmsCm | 404 |
| Conjugate 50 | N6-siHBb3M1S | S | UmsGmsCmUmAmUmGfCfCfUmCmAmUmCmUmUmCmUmUm | 405 |
| | | AS | AmsAfsGmAmAmGfAmUmGmAmGmGmCmAfUmAfGmCmAmsGmsCm | 406 |
| Conjugate 51 | N6-siHBb2M1SV P | S | UmsGmsCmUmAmUmGfCfCfUmCmAmUmCmUmUmCmUmAm | 407 |
| | | AS | VP-UmsAfsGmAmAmGfAmUmGmAmGmGmCmAfUmAfGmCmAmsUmsUm | 408 |

(continued)

| Conjugate serial number | Conjugate No. | Sequence direction 5'-3' | | SEQ ID NO |
|---|---|---|---|---|
| Conjugate 52 | N6-siHBb1M2SV P | S | UmsGmsCmUmAfUmGfCfCfUmCmAmUmCmUmUmCmUmAm | 409 |
| | | AS | VP-UmsAfsGmAmAmGfAmUfGfAmGmGmCmAfUmAfGmCmAmsGmsCm | 410 |
| Conjugate 53 | N6-siHBb2M2SV P | S | UmsGmsCmUmAfUmGfCfCfUmCmAmUmCmUmUmCmUmAm | 411 |
| | | AS | VP-UmsAfsGmAmAmGfAmUfGfAmGmGmCmAfUmAfGmCmAmsUmsUm | 412 |
| Conjugate 54 | N6-siHBb1M5SV P | S | UmsGmsCmUmAfUmGfCfCfUmCmAmUmCmUmUmCmUmAm | 413 |
| | | AS | VP-UmsAfsGmAmAmGfAmUmGmAmGmGmCmAfUmAfGmCmAmsGmsCm | 414 |
| Conjugate 55 | N6-siHBb1M3SV | S | UmsGmsCmUmAfUmGfCmCfUmCmAmUmCmUmUmCmUmAm | 415 |
| | P | AS | VP-UmsAfsGmAmAmGfAmUmGmAmGmGmCmAfUmAfGmCmAmsGmsCm | 416 |
| Conjugate 56 | N6-siHBb1M4SV P | S | UmsGmsCmUmAmUmGfCfCfUmCmAmUmCmUmUmCmUmAm | 417 |
| | | AS | VP-UmsAfsGmAmAmGfAmUfGmAmGmGmCmAfUmAfGmCmAmsGmsCm | 418 |
| Conjugate 57 | N6-siHBb4M1SV P | S | GmsCmsUmGmCmUmAmUmGfCfCfUmCmAmUmCmUmUmCmUmAm | 419 |
| | | AS | VP-UmsAfsGmAmAmGfAmUmGmAmGmGmCmAfUmAfGmCmAmGmCmsGmsCm | 420 |
| Conjugate 58 | N6-siHBb1 | S | UGCUAUGCCUCAUCUUCUA | 421 |
| | | AS | UAGAAGAUGAGGCAUAGCAGC | 422 |
| Conjugate 59 | N6-siHBb2 | S | UGCUAUGCCUCAUCUUCUA | 423 |
| | | AS | UAGAAGAUGAGGCAUAGCAUU | 424 |

(continued)

| Conjugate serial number | Conjugate No. | Sequence direction 5'-3' | | SEQ ID NO |
|---|---|---|---|---|
| Conjugate 60 | X2-siHBb2M1SV P | S | UmsGmsCmUmAmUmGfCfCfUmCmAmUmCmUmUmCmUmAm | 425 |
| | | AS | VP-UmsAfsGmAmAmGfAmUmGmAmGmGmCmAfUmAfGmCmAmsUmsUm | 426 |
| Conjugate 61 | W2-siHBb2M1SV P | S | UmsGmsCmUmAmUmGfCfCfUmCmAmUmCmUmUmCmUmAm | 427 |
| | | AS | VP-UmsAfsGmAmAmGfAmUmGmAmGmGmCmAfUmAfGmCmAmsUmsUm | 428 |
| Conjugate 62 | V2-siHBb2M1SV P | S | UmsGmsCmUmAmUmGfCfCfUmCmAmUmCmUmUmCmUmAm | 429 |
| | | AS | VP-UmsAfsGmAmAmGfAmUmGmAmGmGmCmAfUmAfGmCmAmsUmsUm | 430 |
| Conjugate 63 | 02-siHBb2M1SV P | S | UmsGmsCmUmAmUmGfCfCfUmCmAmUmCmUmUmCmUmAm | 431 |
| | | AS | VP-UmsAfsGmAmAmGfAmUmGmAmGmGmCmAfUmAfGmCmAmsUmsUm | 432 |
| Conjugate 64 | P2-siHBb2M1SV P | S | UmsGmsCmUmAmUmGfCfCfUmCmAmUmCmUmUmCmUmAm | 433 |
| | | AS | VP-UmsAfsGmAmAmGfAmUmGmAmGmGmCmAfUmAfGmCmAmsUmsUm | 434 |

Table 2C

| Conjugate serial number | Conjugate No. | Sequence direction 5' - 3' | | SEQ ID NO |
|---|---|---|---|---|
| Conjugate 65 | N6-siHBc1 | S | UCUGUGCCUUCUCAUCUGA | 435 |
| | | AS | UCAGAUGAGAAGGCACAGACG | 436 |
| Conjugate 66 | N6-siHBc1M1 | S | UmCmUmGmUmGmCfCfUfUmCmUmCmAmUmCmUmGmAm | 437 |
| | | AS | UmCfAmGmAmUfGmAmGmAmAmGmGmCfAmCfAmGmAmCmGm | 438 |

(continued)

| Conjugate serial number | Conjugate No. | Sequence direction 5' - 3' | | SEQ ID NO |
|---|---|---|---|---|
| Conjugate 67 | N6-siHBc1M2 | S | UmCmUmGmUfGmCfCfUfUmCmUmCmAmUmCmUmGmAm | 439 |
| | | AS | UmCfAmGmAmUfGmAfGfAmAmGmGmCfAmCfAmGmAmCmGm | 440 |
| Conjugate 68 | N6-siHBc1M1SVP | S | UmsCmsUmGmUmGmCfCfUfUmCmUmCmAmUmCmUmGmAm | 441 |
| | | AS | VP-UmsCfsAmGmAmUfGmAmGmAmAmGmGmCfAmCfAmGmAmsCmsGm | 442 |
| Conjugate 69 | N6-siHBc1M2SVP | S | UmsCmsUmGmUfGmCfCfUfUmCmUmCmAmUmCmUmGmAm | 443 |
| | | AS | VP-UmsCfsAmGmAmUfGmAfGfAmAmGmGmCfAmCfAmGmAmsCmsGm | 444 |
| Conjugate 70 | N6-siHBc1M3SVP | S | UmsCmsUmGmUfGmCfCmUfUmCmUmCmAmUmCmUmGmAm | 445 |
| | | AS | VP-UmsCfsAmGmAmUfGmAmGmAmAmGmGmCfAmCfAmGmAmsCmsGm | 446 |
| Conjugate 71 | N6-siHBc1M4SVP | S | UmsCmsUmGmUmGmCfCfUfUmCmUmCmAmUmCmUmGmAm | 447 |
| | | AS | VP-UmsCfsAmGmAmUfGmAfGmAmAmGmGmCfAmCfAmGmAmsCmsGm | 448 |
| Conjugate 72 | N6-siHBc1M5SVP | S | UmsCmsUmGmUfGmCfCfUfUmCmUmCmAmUmCmUmGmAm | 449 |
| | | AS | VP-UmsCfsAmGmAmUfGmAmGmAmAmGmGmCfAmCfAmGmAmsCmsGm | 450 |
| Conjugate 73 | N6-siHBc2M1SVP | S | CmsGmsUmCmUmGmUmGmCfCfUfUmCmUmCmAmUmCmUmGmAm | 451 |
| | | AS | VP-UmsCfsAmGmAmUfGmAfGfAmAmGmGmCfAmCfAmGmAmsCmsGmGmGm | 452 |

(continued)

| Conjugate serial number | Conjugate No. | Sequence direction 5' - 3' | | SEQ ID NO |
|---|---|---|---|---|
| Conjugate 74 | N6-siHBc1M1SP | S | UmsCmsUmGmUmGmCfCfUfUmCmUmCmAmUmCmUmGmAm | 453 |
| | | AS | P-UmsCfsAmGmAmUfGmAmGmAmAmGmGmCfAmCfAmGmAmsCmsGm | 454 |
| Conjugate 75 | N6-siHBc1M1SPs | S | UmsCmsUmGmUmGmCfCfUfUmCmUmCmAmUmCmUmGmAm | 455 |
| | | AS | Ps-UmsCfsAmGmAmUfGmAmGmAmAmGmGmCfAmCfAmGmAmsCmsGm | 456 |
| Conjugate 76 | N6-siHBc4M1S | S | UmsCmsUmGmUmGmCfCfUfUmCmUmCmAmUmCmUmGmUm | 457 |
| | | AS | AmsCfsAmGmAmUfGmAmGmAmAmGmGmCfAmCfAmGmAmsCmsGm | 458 |
| Conjugate 77 | X2-siHBc1M1SVP | S | UmsCmsUmGmUmGmCfCfUfUmCmUmCmAmUmCmUmGmAm | 459 |
| | | AS | VP-UmsCfsAmGmAmUfGmAmGmAmAmGmGmCfAmCfAmGmAmsCmsGm | 460 |
| Conjugate 78 | W2-siHBc1M1SVP | S | UmsCmsUmGmUmGmCfCfUfUmCmUmCmAmUmCmUmGmAm | 461 |
| | | AS | VP-UmsCfsAmGmAmUfGmAmGmAmAmGmGmCfAmCfAmGmAmsCmsGm | 462 |
| Conjugate 79 | V2-siHBc1M1SVP | S | UmsCmsUmGmUmGmCfCfUfUmCmUmCmAmUmCmUmGmAm | 463 |
| | | AS | VP-UmsCfsAmGmAmUfGmAmGmAmAmGmGmCfAmCfAmGmAmsCmsGm | 464 |
| Conjugate 80 | 02-siHBc1M1SVP | S | UmsCmsUmGmUmGmCfCfUfUmCmUmCmAmUmCmUmGmAm | 465 |
| | | AS | VP-UmsCfsAmGmAmUfGmAmGmAmAmGmGmCfAmCfAmGmAmsCmsGm | 466 |

(continued)

| Conjugate serial number | Conjugate No. | Sequence direction 5' - 3' | | SEQ ID NO |
|---|---|---|---|---|
| Conjugate 81 | P2-siHBc1M1SVP | S | UmsCmsUmGmUmGmCfCfUfUmCmUmCmAmUmCmUmGmAm | 467 |
| | | AS | VP-UmsCfsAmGmAmUfGmAmGmAmAmGmGmCfAmCfAmGmAmsCmsGm | 468 |

Table 2D

| Conjugate serial number | Conjugate No. | Sequence direction 5' - 3' | | SEQ ID NO |
|---|---|---|---|---|
| Conjugate 82 | N6-siHBd1 | S | CGUGUGCACUUCGCUUCAA | 469 |
| | | AS | UUGAAGCGAAGUGCACACGGU | 470 |
| Conjugate 83 | N6-siHBd1M1 | S | CmGmUmGmUmGmCfAfCfUmUmCmGmCmUmUmCmAmAm | 471 |
| | | AS | UmUfGmAmAmGfCmGmAmAmGmUmGmCfAmCfAmCmGmGmUm | 472 |
| Conjugate 84 | N6-siHBd1M2 | S | CmGmUmGmUfGmCfAfCfUmUmCmGmCmUmUmCmAmAm | 473 |
| | | AS | UmUfGmAmAmGfCmGfAfAmGmUmGmCfAmCfAmCmGmGmUm | 474 |
| Conjugate 85 | N6-siHBd1M1SVP | S | CmsGmsUmGmUmGmCfAfCfUmUmCmGmCmUmUmCmAmAm | 475 |
| | | AS | VP-UmsUfsGmAmAmGfCmGmAmAmGmUmGmCfAmCfAmCmGmsGmsUm | 476 |
| Conjugate 86 | N6-siHBd1M2SVP | S | CmsGmsUmGmUfGmCfAfCfUmUmCmGmCmUmUmCmAmAm | 477 |
| | | AS | VP-UmsUfsGmAmAmGfCmGfAfAmGmUmGmCfAmCfAmCmGmsGmsUm | 478 |
| Conjugate 87 | N6-siHBd1M3SVP | S | CmsGmsUmGmUfGmCfAmCfUmUmCmGmCmUmUmCmAmAm | 479 |
| | | AS | VP-UmsUfsGmAmAmGfCmGmAmAmGmUmGmCfAmCfAmCmGmsGmsUm | 480 |

(continued)

| Conjugate serial number | Conjugate No. | Sequence direction 5' - 3' | | SEQ ID NO |
|---|---|---|---|---|
| Conjugate 88 | N6-siHBd1M4SVP | S | CmsGmsUmGmUmGmCfAfCfUmUmCmGmCmUmUmCmAmAm | 481 |
| | | AS | VP-UmsUfsGmAmAmGfCmGfAmAmGmUmGmCfAmCfAmCmGmsGmsUm | 482 |
| Conjugate 89 | N6-siHBd1M5SVP | S | CmsGmsUmGmUfGmCfAfCfUmUmCmGmCmUmUmCmAmAm | 483 |
| | | AS | VP-UmsUfsGmAmAmGfCmGmAmAmGmUmGmCfAmCfAmCmGmsGmsUm | 484 |
| Conjugate 90 | N6-siHBd2M1SVP | S | AmsCmsCmGmUmGmUmGmCfAfCfUmUmCmGmCmUmUmCmAmAm | 485 |
| | | AS | VP-UmsUfsGmAmAmGfCmGmAmAmGmUmGmCfAmCfAmCmGmGmUmsCmsCm | 486 |
| Conjugate 91 | N6-siHBd1M1SP | S | CmsGmsUmGmUmGmCfAfCfUmUmCmGmCmUmUmCmAmAm | 487 |
| | | AS | P-UmsUfsGmAmAmGfCmGmAmAmGmUmGmCfAmCfAmCmGmsGmsUm | 488 |
| Conjugate 92 | N6-siHBd1M1SPs | S | CmsGmsUmGmUmGmCfAfCfUmUmCmGmCmUmUmCmAmAm | 489 |
| | | AS | Ps-UmsUfsGmAmAmGfCmGmAmAmGmUmGmCfAmCfAmCmGmsGmsUm | 490 |
| Conjugate 93 | N6-siHBd4M1S | S | CmsGmsUmGmUmGmCfAfCfUmUmCmGmCmUmUmCmAmUm | 491 |
| | | AS | AmsUfsGmAmAmGfCmGmAmAmGmUmGmCfAmCfAmCmGmsGmsUm | 492 |
| Conjugate 94 | X2-siHBd1M1SVP | S | CmsGmsUmGmUmGmCfAfCfUmUmCmGmCmUmUmCmAmAm | 493 |
| | | AS | VP-UmsUfsGmAmAmGfCmGmAmAmGmUmGmCfAmCfAmCmGmsGmsUm | 494 |

(continued)

| Conjugate serial number | Conjugate No. | Sequence direction 5' - 3' | | SEQ ID NO |
|---|---|---|---|---|
| Conjugate 95 | W2-siHBd1M1SVP | S | CmsGmsUmGmUmGmCfAfCfUmUmCmGmCmUmUmCmAmAm | 495 |
| | | AS | VP-UmsUfsGmAmAmGfCmGmAmAmGmUmGmCfAmCfAmCmGmsGmsUm | 496 |
| Conjugate 96 | V2-siHBd1M1SVP | S | CmsGmsUmGmUmGmCfAfCfUmUmCmGmCmUmUmCmAmAm | 497 |
| | | AS | VP-UmsUfsGmAmAmGfCmGmAmAmGmUmGmCfAmCfAmCmGmsGmsUm | 498 |
| Conjugate 97 | 02-siHBd1M1SVP | S | CmsGmsUmGmUmGmCfAfCfUmUmCmGmCmUmUmCmAmAm | 499 |
| | | AS | VP-UmsUfsGmAmAmGfCmGmAmAmGmUmGmCfAmCfAmCmGmsGmsUm | 500 |
| Conjugate 98 | P2-siHBd1M1SVP | S | CmsGmsUmGmUmGmCfAfCfUmUmCmGmCmUmUmCmAmAm | 501 |
| | | AS | VP-UmsUfsGmAmAmGfCmGmAmAmGmUmGmCfAmCfAmCmGmsGmsUm | 502 |

Table 2E

| Conjugate serial number | Conjugate No. | Sequence direction 5' - 3' | | SEQ ID NO |
|---|---|---|---|---|
| Conjugate 99 | N6-siHBe3M1S | S | GmsAmsAmAmGmUmAfUfGfUmCmAmAmCmGmAmAmUmAm | 503 |
| | | AS | UmsAfsUmUmCmGfUmUmGmAmCmAmUmAfCmUfUmUmCmsUmsUm | 504 |
| Conjugate 100 | N6-siHBe4M1S | S | GmsAmsAmAmGmUmAfUfGfUmCmAmAmCmGmAmAmUmAm | 505 |
| | | AS | UmsAfsUmUmCmGfUmUmGmAmCmAmUmAfCmUfUmUmCmsCmsAm | 506 |

(continued)

| Conjugate serial number | Conjugate No. | Sequence direction 5' - 3' | | SEQ ID NO |
|---|---|---|---|---|
| Conjugate 101 | N6-siHBe3M1 | S | GmAmAmAmGmUmAfUfGfUmCmAmAmCm GmAmAmUmAm | 507 |
| | | AS | UmAfUmUmCmGfUmUmGmAmCmAmUmAf CmUfUmUmCmUmUm | 508 |
| Conjugate 102 | N6-siHBe4M1 | S | GmAmAmAmGmUmAfUfGfUmCmAmAmCm GmAmAmUmAm | 509 |
| | | AS | UmAfUmUmCmGfUmUmGmAmCmAmUmAf CmUfUmUmCmCmAm | 510 |
| Conjugate 103 | N6-siHBe3M1SVP | S | GmsAmsAmAmGmUmAfUfGfUmCmAmAmC mGmAmAmUmAm | 511 |
| | | AS | VP-UmsAfsUmUmCmGfUmUmGmAmCmAmUmA fCmUfUmUmCmsUmsUm | 512 |
| Conjugate 104 | N6-siHBe3M1SPs | S | GmsAmsAmAmGmUmAfUfGfUmCmAmAmC mGmAmAmUmAm | 513 |
| | | AS | Ps-UmsAfsUmUmCmGfUmUmGmAmCmAmUmA fCmUfUmUmCmsUmsUm | 514 |
| Conjugate 105 | N6-siHBe3M1SP | S | GmsAmsAmAmGmUmAfUfGfUmCmAmAmC mGmAmAmUmAm | 515 |
| | | AS | P-UmsAfsUmUmCmGfUmUmGmAmCmAmUmA fCmUfUmUmCmsUmsUm | 516 |
| Conjugate 106 | N6-siHBe3M1VP | S | GmAmAmAmGmUmAfUfGfUmCmAmAmCm GmAmAmUmAm | 517 |
| | | AS | VP-UmAfUmUmCmGfUmUmGmAmCmAmUmAf CmUfUmUmCmUmUm | 518 |
| Conjugate 107 | N6-siHBe3M2 | S | GmAmAmAmGfUmAfUfGfUmCmAmAmCmG mAmAmUmAm | 519 |
| | | AS | UmAfUmUmCmGfUmUfGfAmCmAmUmAfCm UfUmUmCmUmUm | 520 |

(continued)

| Conjugate serial number | Conjugate No. | Sequence direction 5' - 3' | | SEQ ID NO |
|---|---|---|---|---|
| Conjugate 108 | N6-siHBe3M2SVP | S | GmsAmsAmAmGfUmAfUfGfUmCmAmAmCmGmAmAmUmAm | 521 |
| | | AS | VP-UmsAfsUmUmCmGfUmUfGfAmCmAmUmAfCmUfUmUmCmsUmsUm | 522 |
| Conjugate 109 | N6-siHBe3M3SVP | S | GmsAmsAmAmGfUmAfUfGfUmCmAmAmCmGmAmAmUmAm | 523 |
| | | AS | VP-UmsAfsUmUmCmGfUmUmGmAmCmAmUmAfCmUfUmUmCmsUmsUm | 524 |
| Conjugate 110 | N6-siHBe3M4SVP | S | GmsAmsAmAmGfUmAfUmGfUmCmAmAmCmGmAmAmUmAm | 525 |
| | | AS | VP-UmsAfsUmUmCmGfUmUmGmAmCmAmUmAfCmUfUmUmCmsUmsUm | 526 |
| Conjugate 111 | N6-siHBe3M5SVP | S | GmsAmsAmAmGmUmAfUfGfUmCmAmAmCmGmAmAmUmAm | 527 |
| | | AS | VP-UmsAfsUmUmCmGfUmUfGmAmCmAmUmAfCmUfUmUmCmsUmsUm | 528 |
| Conjugate 112 | N6-siHBe1M1S | S | GmsAmsAmAmGmUmAfUfGfUmCmAmAmCmGmAmAmUmUm | 529 |
| | | AS | AmsAfsUmUmCmGfUmUmGmAmCmAmUmAfCmUfUmUmCmsUmsUm | 530 |
| Conjugate 113 | N6-siHBe5M1SVP | S | UmsGmsGmAmAmAmGmUmAfUfGfUmCmAmAmCmGmAmAmUmAm | 531 |
| | | AS | VP-UmsAfsUmUmCmGfUmUmGmAmCmAmUmAfCmUfUmUmCmCmAmsUmsUm | 532 |
| Conjugate 114 | N6-siHBe0M1 | S | GmAmAmAmGmUmAfUfGfUmCmAmAmCmGmAmAmUmUm | 533 |
| | | AS | AmAfUmUmCmGfUmUmGmAmCmAmUmAfCmUfUmUmCm | 534 |
| Conjugate 115 | N6-siHBe2 | S | GAAAGUAUGUCAACGAAUU | 535 |
| | | AS | AAUUCGUUGACAUACUUUCCA | 536 |

(continued)

| Conjugate serial number | Conjugate No. | Sequence direction 5' - 3' | | SEQ ID NO |
|---|---|---|---|---|
| Conjugate 116 | X2-siHBe3M1SVP | S | GmsAmsAmAmGmUmAfUfGfUmCmAmAmCmGmAmAmUmAm | 537 |
| | | AS | VP-UmsAfsUmUmCmGfUmUmGmAmCmAmUmAfCmUfUmUmCmsUmsUm | 538 |
| Conjugate 117 | W2-siHBe3M1SVP | S | GmsAmsAmAmGmUmAfUfGfUmCmAmAmCmGmAmAmUmAm | 539 |
| | | AS | VP-UmsAfsUmUmCmGfUmUmGmAmCmAmUmAfCmUfUmUmCmsUmsUm | 540 |
| Conjugate 118 | V2-siHBe3M1SVP | S | GmsAmsAmAmGmUmAfUfGfUmCmAmAmCmGmAmAmUmAm | 541 |
| | | AS | VP-UmsAfsUmUmCmGfUmUmGmAmCmAmUmAfCmUfUmUmCmsUmsUm | 542 |
| Conjugate 119 | 02-siHBe3M1SVP | S | GmsAmsAmAmGmUmAfUfGfUmCmAmAmCmGmAmAmUmAm | 543 |
| | | AS | VP-UmsAfsUmUmCmGfUmUmGmAmCmAmUmAfCmUfUmUmCmsUmsUm | 544 |
| Conjugate 120 | P2-siHBe3M1SVP | S | GmsAmsAmAmGmUmAfUfGfUmCmAmAmCmGmAmAmUmAm | 545 |
| | | AS | VP-UmsAfsUmUmCmGfUmUmGmAmCmAmUmAfCmUfUmUmCmsUmsUm | 546 |

Table 2F

| Conjugate serial number | Conjugate No. | Sequence direction 5' - 3' | | SEQ ID NO |
|---|---|---|---|---|
| Conjugate 121 | N6-siAN1 | S | CCAAGAGCACCAAGAACUA | 547 |
| | | AS | UAGUUCUUGGUGCUCUUGGCU | 548 |
| Conjugate 122 | N6-siAN2 | S | AGCCAAGAGCACCAAGAACUA | 549 |
| | | AS | UAGUUCUUGGUGCUCUUGGCUUG | 550 |

(continued)

| Conjugate serial number | Conjugate No. | Sequence direction 5' - 3' | | SEQ ID NO |
|---|---|---|---|---|
| Conjugate 123 | N6-siAN1-M1 | S | CmCmAmAmGfAmGfCfAfCmCmAmAmGmAmAmCmUmAm | 551 |
| | | AS | UmAfGmUmUmCfUmUfGfGmUmGmCmUfCmUfUmGmGmCmUm | 552 |
| Conjugate 124 | N6-siAN2-M1 | S | AmGmCmCmAmAmGfAmGfCfAfCmCmAmAmGmAmAmCmUmAm | 553 |
| | | AS | UmAfGmUmUmCfUmUfGfGmUmGmCmUfCmUfUmGmGmCmUmUmGm | 554 |
| Conjugate 125 | N6-siAN1-M2 | S | CmCmAmAmGfAmGfCfAfCmCmAmAmGmAmAmCmUmAm | 555 |
| | | AS | UmAfGmUmUmCfUmUmGmGmUmGmCmUfCmUfUmGmGmCmUm | 556 |
| Conjugate 126 | N6-siAN2-M2 | S | AmGmCmCmAmAmGfAmGfCfAfCmCmAmAmGmAmAmCmUmAm | 557 |
| | | AS | UmAfGmUmUmCfUmUmGmGmUmGmCmUfCmUfUmGmGmCmUmUmGm | 558 |
| Conjugate 127 | N6-siAN1-M3 | S | CmCmAmAmGmAmGfCfAfCmCmAmAmGmAmAmCmUmAm | 559 |
| | | AS | UmAfGmUmUmCfUmUmGmGmUmGmCmUfCmUfUmGmGmCmUm | 560 |
| Conjugate 128 | N6-siAN2-M3 | S | AmGmCmCmAmAmGmAmGfCfAfCmCmAmAmGmAmAmCmUmAm | 561 |
| | | AS | UmAfGmUmUmCfUmUmGmGmUmGmCmUfCmUfUmGmGmCmUmUmGm | 562 |
| Conjugate 129 | N6-siAN1-M1VP | S | CmCmAmAmGfAmGfCfAfCmCmAmAmGmAmAmCmUmAm | 563 |
| | | AS | VP-UmAfGmUmUmCfUmUfGfGmUmGmCmUfCmUfUmGmGmCmUm | 564 |
| Conjugate 130 | N6-siAN2-M1VP | S | AmGmCmCmAmAmGfAmGfCfAfCmCmAmAmGmAmAmCmUmAm | 565 |
| | | AS | VP-UmAfGmUmUmCfUmUfGfGmUmGmCmUfCmUfUmGmGmCmUmUmGm | 566 |

(continued)

| Conjugate serial number | Conjugate No. | Sequence direction 5' - 3' | | SEQ ID NO |
|---|---|---|---|---|
| Conjugate 131 | N6-siAN1-M2VP | S | CmCmAmAmGfAmGfCfAfCmCmAmAmGmAmAmCmUmAm | 567 |
| | | AS | VP-UmAfGmUmUmCfUmUmGmGmUmGmCmUfCmUfUmGmGmCmUm | 568 |
| Conjugate 132 | N6-siAN2-M2VP | S | AmGmCmCmAmAmGfAmGfCfAfCmCmAmAmGmAmAmCmUmAm | 569 |
| | | AS | VP-UmAfGmUmUmCfUmUmGmGmUmGmCmUfCmUfUmGmGmCmUmUmGm | 570 |
| Conjugate 133 | N6-siAN1-M3VP | S | CmCmAmAmGmAmGfCfAfCmCmAmAmGmAmAmCmUmAm | 571 |
| | | AS | VP-UmAfGmUmUmCfUmUmGmGmUmGmCmUfCmUfUmGmGmCmUm | 572 |
| Conjugate 134 | N6-siAN2-M3VP | S | AmGmCmCmAmAmGmAmGfCfAfCmCmAmAmGmAmAmCmUmAm | 573 |
| | | AS | VP-UmAfGmUmUmCfUmUmGmGmUmGmCmUfCmUfUmGmGmCmUmUmGm | 574 |
| Conjugate 135 | N6-siAN1-M1S | S | CmsCmsAmAmGfAmGfCfAfCmCmAmAmGmAmAmCmUmAm | 575 |
| | | AS | UmsAfsGmUmUmCfUmUfGfGmUmGmCmUfCmUfUmGmGmsCmsUm | 576 |
| Conjugate 136 | N6-siAN2-M1S | S | AmsGmsCmCmAmAmGfAmGfCfAfCmCmAmAmGmAmAmCmUmAm | 577 |
| | | AS | UmsAfsGmUmUmCfUmUfGfGmUmGmCmUfCmUfUmGmGmCmUmsUmsGm | 578 |
| Conjugate 137 | N6-siAN1-M2S | S | CmsCmsAmAmGfAmGfCfAfCmCmAmAmGmAmAmCmUmAm | 579 |
| | | AS | UmsAfsGmUmUmCfUmUmGmGmUmGmCmUfCmUfUmGmGmsCmsUm | 580 |
| Conjugate 138 | N6-siAN2-M2S | S | AmsGmsCmCmAmAmGfAmGfCfAfCmCmAmAmGmAmAmCmUmAm | 581 |
| | | AS | UmsAfsGmUmUmCfUmUmGmGmUmGmCmUfCmUfUmGmGmCmUmsUmsGm | 582 |

(continued)

| Conjugate serial number | Conjugate No. | Sequence direction 5' - 3' | | SEQ ID NO |
|---|---|---|---|---|
| Conjugate 139 | N6-siAN1-M3S | S | CmsCmsAmAmGmAmGfCfAfCmCmAmAmGmAmAmCmUmAm | 583 |
| | | AS | UmsAfsGmUmUmCfUmUmGmGmUmGmCmUfCmUfUmGmGmsCmsUm | 584 |
| Conjugate 140 | N6-siAN2-M3S | S | AmsGmsCmCmAmAmGmAmGfCfAfCmCmAmAmGmAmAmCmUmAm | 585 |
| | | AS | UmsAfsGmUmUmCfUmUmGmGmUmGmCmUfCmUfUmGmGmCmUmsUmsGm | 586 |
| Conjugate 141 | N6-siAN1-M1SVP | S | CmsCmsAmAmGfAmGfCfAfCmCmAmAmGmAmAmCmUmAm | 587 |
| | | AS | VP-UmsAfsGmUmUmCfUmUfGfGmUmGmCmUfCmUfUmGmGmsCmsUm | 588 |
| Conjugate 142 | N6-siAN2-M1SVP | S | AmsGmsCmCmAmAmGfAmGfCfAfCmCmAmAmGmAmAmCmUmAm | 589 |
| | | AS | VP-UmsAfsGmUmUmCfUmUfGfGmUmGmCmUfCmUfUmGmGmCmUmsUmsGm | 590 |
| Conjugate 143 | N6-siAN1-M2SVP | S | CmsCmsAmAmGfAmGfCfAfCmCmAmAmGmAmAmCmUmAm | 591 |
| | | AS | VP-UmsAfsGmUmUmCfUmUmGmGmUmGmCmUfCmUfUmGmGmsCmsUm | 592 |
| Conjugate 144 | N6-siAN2-M2SVP | S | AmsGmsCmCmAmAmGfAmGfCfAfCmCmAmAmGmAmAmCmUmAm | 593 |
| | | AS | VP-UmsAfsGmUmUmCfUmUmGmGmUmGmCmUfCmUfUmGmGmCmUmsUmsGm | 594 |
| Conjugate 145 | N6-siAN1-M3SVP | S | CmsCmsAmAmGmAmGfCfAfCmCmAmAmGmAmAmCmUmAm | 595 |
| | | AS | VP-UmsAfsGmUmUmCfUmUmGmGmUmGmCmUfCmUfUmGmGmsCmsUm | 596 |

(continued)

| Conjugate serial number | Conjugate No. | Sequence direction 5' - 3' | | SEQ ID NO |
|---|---|---|---|---|
| Conjugate 146 | N6-siAN1-M3SP | S | CmsCmsAmAmGmAmGfCfAfCmCmAmAmGmAmAmCmUmAm | 597 |
| | | AS | P-UmsAfsGmUmUmCfUmUmGmGmUmGmCmUfCmUfUmGmGmsCmsUm | 598 |
| Conjugate 147 | N6-siAN1-M3SPs | S | CmsCmsAmAmGmAmGfCfAfCmCmAmAmGmAmAmCmUmAm | 599 |
| | | AS | Ps-UmsAfsGmUmUmCfUmUmGmGmUmGmCmUfCmUfUmGmGmsCmsUm | 600 |
| Conjugate 148 | N6-siAN4-M3S | S | CmsCmsAmAmGmAmGfCfAfCmCmAmAmGmAmAmCmUmUm | 601 |
| | | AS | AmsAfsGmUmUmCfUmUmGmGmUmGmCmUfCmUfUmGmGmsCmsUm | 602 |
| Conjugate 149 | N6-siAN2-M3SVP | S | AmsGmsCmCmAmAmGmAmGfCfAfCmCmAmAmGmAmAmCmUmAm | 603 |
| | | AS | VP-UmsAfsGmUmUmCfUmUmGmGmUmGmCmUfCmUfUmGmGmCmUmsUmsGm | 604 |
| Conjugate 150 | N6-siAN1-M4S | S | CmsCmsAmAmGmAmGfCfAfCmCmAmAmGmAmAmCmUmAm | 605 |
| | | AS | UmsAfsGmUmUmCfUmUfGmGmUmGmCmUfCmUfUmGmGmsCmsUm | 606 |
| Conjugate 151 | N6-siAN1-M4SVP | S | CmsCmsAmAmGmAmGfCfAfCmCmAmAmGmAmAmCmUmAm | 607 |
| | | AS | VP-UmsAfsGmUmUmCfUmUfGmGmUmGmCmUfCmUfUmGmGmsCmsUm | 608 |
| Conjugate 152 | N6-siAN1-M5S | S | CmsCmsAmAmGfAmGfCmAfCmCmAmAmGmAmAmCmUmAm | 609 |
| | | AS | UmsAfsGmUmUmCfUmUmGmGmUmGmCmUfCmUfUmGmGmsCmsUm | 610 |
| Conjugate 153 | N6-siAN1-M5SVP | S | CmsCmsAmAmGfAmGfCmAfCmCmAmAmGmAmAmCmUmAm | 611 |
| | | AS | VP-UmsAfsGmUmUmCfUmUmGmGmUmGmCmUfCmUfUmGmGmsCmsUm | 612 |

(continued)

| Conjugate serial number | Conjugate No. | Sequence direction 5' - 3' | | SEQ ID NO |
|---|---|---|---|---|
| Conjugate 154 | X2-siAN1-M3SVP | S | CmsCmsAmAmGmAmGfCfAfCmCmAmAmGmAmAmCmUmAm | 613 |
| | | AS | VP-UmsAfsGmUmUmCfUmUmGmGmUmGmCmUfCmUfUmGmGmsCmsUm | 614 |
| Conjugate 155 | W2-siAN1-M3SVP | S | CmsCmsAmAmGmAmGfCfAfCmCmAmAmGmAmAmCmUmAm | 615 |
| | | AS | VP-UmsAfsGmUmUmCfUmUmGmGmUmGmCmUfCmUfUmGmGmsCmsUm | 616 |
| Conjugate 156 | V2-siAN1-M3SVP | S | CmsCmsAmAmGmAmGfCfAfCmCmAmAmGmAmAmCmUmAm | 617 |
| | | AS | VP-UmsAfsGmUmUmCfUmUmGmGmUmGmCmUfCmUfUmGmGmsCmsUm | 618 |
| Conjugate 157 | O2-siAN1-M3SVP | S | CmsCmsAmAmGmAmGfCfAfCmCmAmAmGmAmAmCmUmAm | 619 |
| | | AS | VP-UmsAfsGmUmUmCfUmUmGmGmUmGmCmUfCmUfUmGmGmsCmsUm | 620 |
| Conjugate 158 | P2-siAN1-M3SVP | S | CmsCmsAmAmGmAmGfCfAfCmCmAmAmGmAmAmCmUmAm | 621 |
| | | AS | VP-UmsAfsGmUmUmCfUmUmGmGmUmGmCmUfCmUfUmGmGmsCmsUm | 622 |

Table 2G

| Conjugate serial number | Conjugate No. | Sequence direction 5' - 3' | | SEQ ID NO |
|---|---|---|---|---|
| Conjugate 159 | N6-siAP1 | S | CAAUAAAGCUGGACAAGAA | 623 |
| | | AS | UUCUUGUCCAGCUUUAUUGGG | 624 |
| Conjugate 160 | N6-siAP2 | S | CCCAAUAAAGCUGGACAAGAA | 625 |
| | | AS | UUCUUGUCCAGCUUUAUUGGGAG | 626 |
| Conjugate 161 | N6-siAP1-M1 | S | CmAmAmUmAfAmAfGfCfUmGmGmAmCmAmAmGmAmAm | 627 |
| | | AS | UmUfCmUmUmGfUmCfCfAmGmCmUmUfUmAfUmUmGmGmGm | 628 |

(continued)

| Conjugate serial number | Conjugate No. | Sequence direction 5' - 3' | | SEQ ID NO |
|---|---|---|---|---|
| Conjugate 162 | N6-siAP2-M1 | S | CmCmCmAmAmUmAfAmAfGfCfUmGmGmAmCmAmAmGmAmAm | 629 |
| | | AS | UmUfCmUmUmGfUmCfCfAmGmCmUmUfUmAfUmUmGmGmGmAmGm | 630 |
| Conjugate 163 | N6-siAP1-M2 | S | CmAmAmUmAmAmAfGfCfUmGmGmAmCmAmAmGmAmAm | 631 |
| | | AS | UmUfCmUmUmGfUmCmCmAmGmCmUmUfUmAfUmUmGmGmGm | 632 |
| Conjugate 164 | N6-siAP2-M2 | S | CmCmCmAmAmUmAmAmAfGfCfUmGmGmAmCmAmAmGmAmAm | 633 |
| | | AS | UmUfCmUmUmGfUmCmCmAmGmCmUmUfUmAfUmUmGmGmGmAmGm | 634 |
| Conjugate 165 | N6-siAP1-M1VP | S | CmAmAmUmAfAmAfGfCfUmGmGmAmCmAmAmGmAmAm | 635 |
| | | AS | VP-UmUfCmUmUmGfUmCfCfAmGmCmUmUfUmAfUmUmGmGmGm | 636 |
| Conjugate 166 | N6-siAP2-M1VP | S | CmCmCmAmAmUmAfAmAfGfCfUmGmGmAmCmAmAmGmAmAm | 637 |
| | | AS | VP-UmUfCmUmUmGfUmCfCfAmGmCmUmUfUmAfUmUmGmGmGmAmGm | 638 |
| Conjugate 167 | N6-siAP1-M2VP | S | CmAmAmUmAmAmAfGfCfUmGmGmAmCmAmAmGmAmAm | 639 |
| | | AS | VP-UmUfCmUmUmGfUmCmCmAmGmCmUmUfUmAfUmUmGmGmGm | 640 |
| Conjugate 168 | N6-siAP2-M2VP | S | CmCmCmAmAmUmAmAmAfGfCfUmGmGmAmCmAmAmGmAmAm | 641 |
| | | AS | VP-UmUfCmUmUmGfUmCmCmAmGmCmUmUfUmAfUmUmGmGmGmAmGm | 642 |
| Conjugate 169 | N6-siAP1-M1S | S | CmsAmsAmUmAfAmAfGfCfUmGmGmAmCmAmAmGmAmAm | 643 |
| | | AS | UmsUfsCmUmUmGfUmCfCfAmGmCmUmUfUmAfUmUmGmsGmsGm | 644 |

(continued)

| Conjugate serial number | Conjugate No. | Sequence direction 5' - 3' | | SEQ ID NO |
|---|---|---|---|---|
| Conjugate 170 | N6-siAP2-M1S | S | CmsCmsCmAmAmUmAfAmAfGfCfUmGmGmAmCmAmAmGmAmAm | 645 |
| | | AS | UmsUfsCmUmUmGfUmCfCfAmGmCmUmUfUmAfUmUmGmGmGmsAmsGm | 646 |
| Conjugate 171 | N6-siAP1-M2S | S | CmsAmsAmUmAmAmAfGfCfUmGmGmAmCmAmAmGmAmAm | 647 |
| | | AS | UmsUfsCmUmUmGfUmCmCmAmGmCmUmUfUmAfUmUmGmsGmsGm | 648 |
| Conjugate 172 | N6-siAP2-M2S | S | CmsCmsCmAmAmUmAmAmAfGfCfUmGmGmAmCmAmAmGmAmAm | 649 |
| | | AS | UmsUfsCmUmUmGfUmCmCmAmGmCmUmUfUmAfUmUmGmGmGmsAmsGm | 650 |
| Conjugate 173 | N6-siAP1-M1SVP | S | CmsAmsAmUmAfAmAfGfCfUmGmGmAmCmAmAmGmAmAm | 651 |
| | | AS | VP-UmsUfsCmUmUmGfUmCfCfAmGmCmUmUfUmAfUmUmGmsGmsGm | 652 |
| Conjugate 174 | N6-siAP2-M1SVP | S | CmsCmsCmAmAmUmAfAmAfGfCfUmGmGmAmCmAmAmGmAmAm | 653 |
| | | AS | VP-UmsUfsCmUmUmGfUmCfCfAmGmCmUmUfUmAfUmUmGmGmGmsAmsGm | 654 |
| Conjugate 175 | N6-siAP1-M2SVP | S | CmsAmsAmUmAmAmAfGfCfUmGmGmAmCmAmAmGmAmAm | 655 |
| | | AS | VP-UmsUfsCmUmUmGfUmCmCmAmGmCmUmUfUmAfUmUmGmsGmsGm | 656 |
| Conjugate 176 | N6-siAP1-M2SP | S | CmsAmsAmUmAmAmAfGfCfUmGmGmAmCmAmAmGmAmAm | 657 |
| | | AS | P-UmsUfsCmUmUmGfUmCmCmAmGmCmUmUfUmAfUmUmGmsGmsGm | 658 |
| Conjugate 177 | N6-siAP1-M2SPs | S | CmsAmsAmUmAmAmAfGfCfUmGmGmAmCmAmAmGmAmAm | 659 |
| | | AS | Ps-UmsUfsCmUmUmGfUmCmCmAmGmCmUmUfUmAfUmUmGmsGmsGm | 660 |

(continued)

| Conjugate serial number | Conjugate No. | Sequence direction 5' - 3' | | SEQ ID NO |
|---|---|---|---|---|
| Conjugate 178 | N6-siAP4-M2S | S | CmsAmsAmUmAmAmAfGfCfUmGmGmAmCmAmAmGmAmUm | 661 |
| | | AS | AmsUfsCmUmUmGfUmCmCmAmGmCmUmUfUmAfUmUmGmsGmsGm | 662 |
| Conjugate 179 | X2-siAP1-M2SVP | S | CmsCmsCmAmAmUmAmAmAfGfCfUmGmGmAmCmAmAmGmAmAm | 663 |
| | | AS | VP-UmsUfsCmUmUmGfUmCmCmAmGmCmUmUfUmAfUmUmGmGmGmsAmsGm | 664 |
| Conjugate 180 | W2-siAP1-M2SVP | S | CmsAmsAmUmAmAmAfGfCfUmGmGmAmCmAmAmGmAmAm | 665 |
| | | AS | VP-UmsUfsCmUmUmGfUmCmCmAmGmCmUmUfUmAfUmUmGmsGmsGm | 666 |
| Conjugate 181 | V2-siAP1-M2SVP | S | CmsAmsAmUmAmAmAfGfCfUmGmGmAmCmAmAmGmAmAm | 667 |
| | | AS | VP-UmsUfsCmUmUmGfUmCmCmAmGmCmUmUfUmAfUmUmGmsGmsGm | 668 |
| Conjugate 182 | O2-siAP1-M2SVP | S | CmsAmsAmUmAmAmAfGfCfUmGmGmAmCmAmAmGmAmAm | 669 |
| | | AS | VP-UmsUfsCmUmUmGfUmCmCmAmGmCmUmUfUmAfUmUmGmsGmsGm | 670 |
| Conjugate 183 | P2-siAP1-M2SVP | S | CmsAmsAmUmAmAmAfGfCfUmGmGmAmCmAmAmGmAmAm | 671 |
| | | AS | VP-UmsUfsCmUmUmGfUmCmCmAmGmCmUmUfUmAfUmUmGmsGmsGm | 672 |
| Conjugate 184 | N6-siTTR-M2SVP | S | AfsAmsCfAmGfUmGfUmUfCfUfUmGfCmUfCmUfAmUfAmAf | 673 |
| | | AS | VP-UmsUfsAmUfAmGfAmGfCmAfAmGmAmAfCmAfCmUfGmUfUmsUmsUm | 674 |

Table 2H

| Conjugate serial number | Conjugate No. | Sequence direction 5' - 3' | | SEQ ID NO |
|---|---|---|---|---|
| Conjugate 185 | FC-siSTAT1 | S | CmsUmsAmGmAmAmAfAfCfUmGmGmAmU mAmAmCmGmUm | 675 |
| | | AS | AmsCfsGmUmUmAfUmCmCmAmGmUmUmU fUmCfUmAmGmsCmsCm | 676 |

Among above, S represents a sense strand; AS represents a antisense strand; C, G, U, and A represent the base composition of a nucleotide; m represents that the nucleotide adjacent to the left side of the letter m is a 2'-methoxy modified nucleotide; f represents that the nucleotide adjacent to the left side of the letter f is a 2'-fluoro modified nucleotide; s represents that the two nucleotides adjacent to both sides of the letter s are linked by a phosphorothioate linkage; VP represents that the nucleotide adjacent to the right side of VP is a vinyl phosphate modified nucleotide; P represents that the nucleotide adjacent to the right side of the letter P is a phosphate nucleotide; and Ps represents that the nucleotide adjacent to the right side of Ps is a phosphorothioate modified nucleotide.

In the above Preparation Examples 7 and 8, when 5' ternimal of the AS strand is the vinyl phosphate modified and 2'-methoxy modified uracil nucleotide as shown by VP-Um, the corresponding vinyl phosphate modified and 2'-methoxy modified uridine monomer (Compound VP-U-6) was synthesized according to the following method:

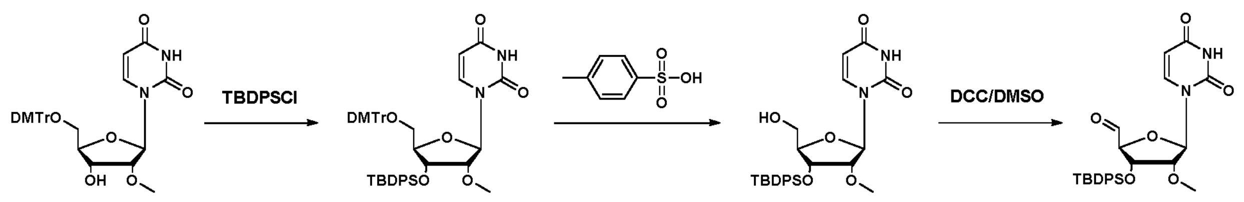


2'-OMe-U VP-U-1 VP-U-2 VP-U-3

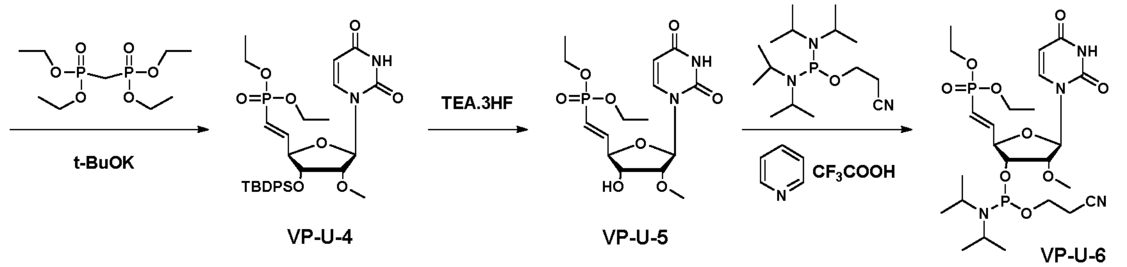


VP-U-4 VP-U-5 VP-U-6

(13-1) Synthesis of VP-U-2

A VP-U-2 molecule was synthesized according to the following method:

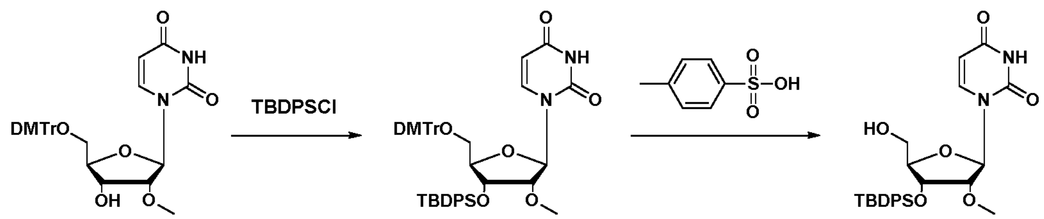


2'-OMe-U VP-U-1 VP-U-2

A 2'-methoxy modified uracil nucleotide (2'-OMe-U, 51.30 g, 91.6 mmol), tert-butyl diphenylchlorosilane (TBDPSCl, 50.35 g, 183.2 mmol), and imidazole (12.47 g, 183.2 mmol) were mixed and dissolved in 450 mL of N,N-dimethylformamide (DMF) to react under stirring at room temperature for 20 h. DMF was removed by evaporation, and the residue was dissolved in 600 mL of dichloromethane and then washed with 300 mL of saturated sodium bicarbonate. The aqueous phase was extracted three times, each with 300 mL of dichloromethane (DCM). The organic phases were combined and washed with 5% oxalic acid until the pH of the aqueous phase was <5. The solvent was evaporated to dryness to give a crude product VP-U-1, which was directly used in the subsequent synthesis of VP-U-2.

The crude product VP-U-1 was dissolved in 100 mL of dichloromethane and then stirred for 10 minutes in an ice bath, added with 450 mL of 2% p-toluenesulfonic acid solution (the solvent is a mixed solvent of methanol and dichloromethane in a volume ratio of 3:7) pre-cooled in a refrigerator at 4°C to react for 10 minutes. The reaction was quenched by addition of 200 mL of saturated sodium bicarbonate. The organic phase was washed to pH=8 by addition of saturated aqueous sodium bicarbonate solution. The aqueous phases were combined and extracted twice, each with 200 mL of dichloromethane. The organic phases were combined, then washed once with 200 mL of saturated brine, and the solvent was evaporated to dryness. The residue was purified by a normal phase silica gel column (200-300 mesh). The column was packed with petroleum ether and eluted with a gradient elution of petroleum ether: ethyl acetate: dichloromethane: methanol = 1:1:1:0.05 - 1:1:1:0.25. The eluate was collected and the solvent was evaporated to dryness under reduced pressure. The residue was foam-dried in a vacuum oil pump to give a total of 40.00 g of pure product VP-U-2. 1H NMR (400 MHz, DMSO-d6) δ 7.96 (d, J = 7.8 Hz, 1H), 7.64 (dtd, J = 5.1, 4.0, 2.2 Hz, 4H), 7.41-7.30 (m, 6H), 6.79 (d, J = 4.7 Hz, 1H), 5.73 (d, J = 7.6 Hz, 1H), 4.94 (t, J = 7.0 Hz, 1H), 4.12 (td, J = 4.6, 3.9 Hz, 1H), 4.05 (dd, J = 4.8, 4.0 Hz, 1H), 3.96 (t, J = 4.7 Hz, 1H), 3.68 (ddd, J = 11.8, 7.0, 4.6 Hz, 1H), 3.57 - 3.46 (m, 1H), 3.39 (s, 3H), 1.05 (s, 8H). MS m/z: $C_{26}H_{33}N_2O_6Si$, [M+H]+, calculated: 497.21, measured: 497.45.

(13-2) Synthesis of VP-U-4;

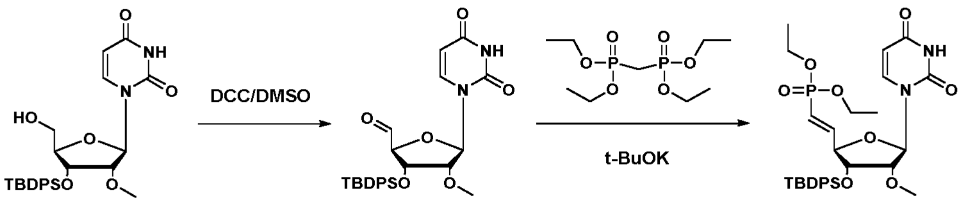


VP-U-2 VP-U-3 VP-U-4

VP-U-2 (19.84 g, 40.0 mmol), dicyclohexylcarbodiimide (DCC, 16.48 g, 80.0 mmol), pyridine (4.20 g, 53.2 mmol), and trifluoroacetic acid (6.61 g, 53.2 mmol) were mixed and dissolved in 200 mL of dimethyl sulfoxide (DMSO) to react under stirring at room temperature for 20 hours. Separately, tetraethyl methylenediphosphate (21.44 g, 74.4 mmol) was dissolved in 120 mL of THF, cooled in an ice bath, added with t-BuOK (11.36 g, 101.2 mmol) at a temperature of the ice bath to react for 10 min, warmed to room temperature to react for 0.5 hour and added into the above reaction solution over about 1 hour. The reaction was carried out at a temperature of the ice bath for 1 hour and then warmed to room temperature to react for 18 hours. The reaction was quenched by addition of water. The aqueous phase was extracted three times, each with 200 mL of dichloromethane. The organic phases were combined and washed once with 200 mL of saturated brine. The solvent was evaporated to dryness, and the residue was purified by using a normal phase silica gel column (200-300 mesh). The column was packed with petroleum ether and eluted with a gradient elution of petroleum ether: ethyl acetate = 1:1-1:4. The eluate was collected. The solvent was evaporated to dryness under reduced pressure, and the residue was foam-dried in a vacuum oil pump to give a total of 14.00 g of pure product VP-U-4. 1H NMR (400 MHz, DMSO-d6) δ 7.96 (d, J = 7.8 Hz, 1H), 7.64 (dtd, J = 5.1, 4.0, 2.2 Hz, 4H), 7.41 - 7.30 (m, 6H), 6.82 - 6.71 (m, 2H), 5.90 (ddd, J = 25.9, 15.0, 1.0 Hz, 1H), 5.73 (d, J = 7.6 Hz, 1H), 4.36 - 4.21 (m, 3H), 4.18 (t, J = 4.9 Hz, 1H), 4.05 (ddq, J = 9.7, 8.5, 6.9 Hz, 2H), 3.87 (t, J = 4.8 Hz, 1H), 3.39 (s, 3H), 1.32 (td, J = 6.9, 0.7 Hz, 6H), 1.05 (s, 8H). MS m/z: $C_{31}H_{42}N_2O_8PSi$, [M+H]+, calculated: 629.24, measured: 629.51.

(13-3) Synthesis of VP-U-5:

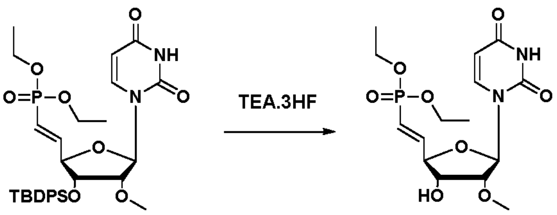


VP-U-4 VP-U-5

VP-U-4 (14.00 g, 22.29 mmol) was dissolved in 100 mL of tetrahydrofuran, added with triethylamine trihydrofluoride (17.96 g, 111.45 mmol), and stirred at room temperature for 20 hours to react completely. The solvent was directly evaporated to dryness, the residue was dissolved in dichoromethane, and then the solvent was evaporated to dryness again. The above dissolution and evaporation steps were performed twice, each with 50 mL of dichloromethane, to give a crude product. The crude product was purified by using a normal phase silica gel column (200-300 mesh). The column was packed with petroleum ether and eluted with a gradient elution of petroleum ether: ethyl acetate: dichloromethane: methanol = 1:1:1:0.05-1:1:1:0.25. The eluate was collected, the solvent was evaporated to dryness under reduced pressure, and the residue was foam-dried in a vacuum oil pump to give a total of 6.70 g of pure product VP-U-5. 1H NMR (400 MHz, DMSO-d6) δ 7.96 (d, J = 7.8 Hz, 1H), 6.77 (dd, J = 15.0, 6.2 Hz, 1H), 5.99 - 5.82 (m, 2H), 5.73 (d, J = 7.6 Hz, 1H), 5.27 (d, J = 5.1 Hz, 1H), 5.10 (dd, J = 5.3, 4.7 Hz, 1H), 4.29 (ddq, J = 9.8, 8.6, 7.0 Hz, 2H), 4.17 (ddd, J = 6.2, 5.2, 1.0 Hz, 1H), 4.12 - 3.98 (m, 3H), 3.39 (s, 2H), 1.32 (td, J = 6.9, 0.6 Hz, 6H). MS m/z: $C_{15}H_{24}N_2O_8P$, [M+H]+, calculated: 391.13, measured: 391.38.

(13-4) Synthesis of VP-U-6:

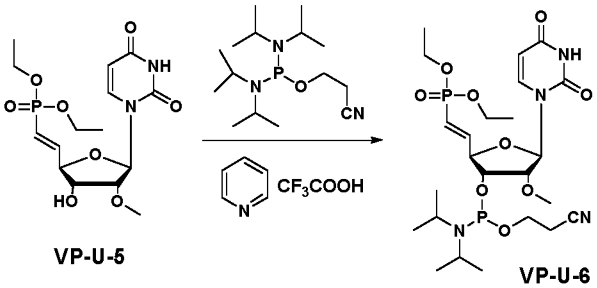


VP-U-5 VP-U-6

VP-U-5 (391 mg, 1.0 mmol), pyridine trifluoroacetate (0.232 g, 1.2 mmol), N-methylimidazole (0.099 g, 1.2 mmol), and bis(diisopropylamino)(2-cyanoethoxy)phosphine (0.452 g, 1.5 mmol) were added into 10 mL of anhydrous dichloromethane under argon atmosphere to react under stirring at room temperature for 5 hours. The solvent was evaporated to dryness, and then the residue was purified by column chromatography (200-300 mesh normal phase silica gel, with a gradient elution of dichloromethane: acetonitrile (containing 0.5 wt% triethylamine) = 3:1-1:3). The eluate was collected and concentrated to remove the solvent to give a total of 508 mg of the target product VP-U-6. 31P NMR (161 MHz, DMSO-d6) δ 150.34, 150.29, 17.07, 15.50. MS m/z: $C_{24}H_{41}N_4O_9P_2$, [M+H]+, calculated: 591.23, measured: 591.55. The above data indicated that VP-U-6 was the target product VP-Um, which was involved in the synthesis of RNA strands as a nucleoside monomer.

The 5'-phosphate modification was linked to 5' terminal of the antisense strand using the follow method:

The raw material was phosphorylated structural monomer with the structure as shown by Formula CPR-I, which was provided by Suzhou GenePharma Inc. as Cat#13-2601-XX:

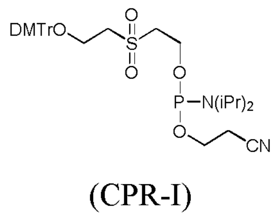

(CPR-I)

After all the nucleoside monomers of the antisense strand were linked, according to the phosphoramidite nucleic acid solid phase synthesis method, CPR-I monomer was linked to 5' terminal of the antisense strand by a four-step reaction of deprotection, coupling, capping and oxidation. Subsequently, the antisense strand was subjected to cleavage and deprotection according to the following conditions to give the antisense strand:
The synthesized nucleotide sequence linked to the support was added to 25 wt% aqueous ammonia to react at 55°C for 16h, wherein the amount of the aqueous ammonia is 0.5 mL/μmol. The liquid was removed, and the residue was concentrated to dryness in vacuum.

After treatment with aqueous ammonia, with respect to the amount of single-stranded nucleic acid, the product was dissolved in 0.4 mL/μmol N-methylpyrrolidone, then added with 0.3 mL/μmol triethylamine and 0.6 mL/μmol triethylamine trihydrofluoride to remove the 2'-O-TBDMS protection from the ribose. Purification and desalination: purification of the nucleic acid is achieved by using a preparative ion chromatography purification column (Source 15Q) with a gradient elution of NaCl. Specifically, eluent A is 20 mM sodium phosphate (pH 8.1), solvent is water/acetonitrile in 9:1 (v/v); eluent B is 1.5 M sodium chloride, 20 mM sodium phosphate (pH 8.1), solvent is water/acetonitrile in 9:1 (v/v); elution gradient: the ratio of eluent A: eluent B = 100:0-50:50. The eluate is collected, combined and desalted by using a reverse phase chromatography purification column. The specific condition comprises: using a Sephadex column for desalination (filler: Sephadex G25) and eluting with deionized water.

In the case where the target product has a 5'-phosphorothioate modification, the same steps as those described above were used, except that during the linking, a sulfurization reaction was performed under the sulfurization reaction condition in place of the oxidation reaction condition described above.

For the synthesized sense strand and the antisense strand above, the purity was determined using Ion Exchange Chromatography (IEX-HPLC) and the molecular weight was analyzed by Liquid Chromatography-Mass Spectrometry (LC-MS) to confirm that the synthesized nucleic acid sequences were the siRNAs corresponding to the conjugates and comparative conjugate in Table 2.

**Experimental Example 1 This experiment demonstrates the animal level toxicity of the drug conjugates of the present disclosure.**

For C57BL/6J mice (purchased from Beijing Vital River Experimental Animal Technology Co., Ltd.), each mouse was subcutaneously adminstrated with Conjugates 28-30, 38-42, 47-49, 68-70, 90-92, 145-147 and 175-177 at a single dose of 300 mg/kg (calculated based on siRNA), respectively, and were continuously observed for 14 days: no animal death and no clinical symptoms associated with adverse drug reactions were observed. After the observation, gross anatomy of each mouse was performed and no abnormality was found. Thus, the above results specified that the drug conjugates of the present disclosure have lower animal level toxicity.

In the following Experimental Examples 2 - 8, the properties and effects of the drug conjugates in Tables 2A to 2G were experimentally verified respectively according to the target position and sequence correlation of the siRNA.

**Experimental Example 2 Effect experiments of the drug conjugates in Table 2A Experimental Example 2-1 *In vitro* stability of the conjugates of the present disclosure in human plasma**

Conjugate 32 (provided in the form of 20 μM in 0.9% sodium chloride aqueous solution (calculated based on siRNA), 12 μL per group) was mixed well with 108 μL of 90% human plasma (purchased from Jiangsu Hematology Institute and diluted with 1×PBS (pH 7.4)) to obtain a mixed solution, and was incubated at a constant temperature of 37°C. During the incubation, 10 μL of the mixed solution was taken at each time point of 0, 2, 4, 6, 8, 24, 48 and 72 hours respectively, and immediately frozen in liquid nitrogen and cryopreserved in a -80°C refrigerator. Therein, 0 hour refers to the time when 10 μL of the mixed solution was taken immediately after the conjugate solution was mixed well with 90% human plasma. After sampling at each time point, each mixed solution was diluted 5-fold with 1×PBS (pH7.4), and 10 μL of each diluted mixed solution was taken for electrophoresis. Meanwhile, an equimolar amount of drug Conjugate 32 solution (2 μL, siRNA concentration is 2 μM) was mixed well with 8 μL of 1×PBS (pH 7.4) to prepare 10 μL of sample untreated with human plasma (designated as Con).

20 wt% of non-denatured polyacrylamide gel was prepared. The samples for electrophoresis in each group were mixed with 4 μL of loading buffer (20 mM EDTA, 36 wt% glycerol, and 0.06 wt% aqueous solution of bromophenol blue) respectively and then loaded into each gel well for electrophoresis under 80 mA constant current for 60 minutes. After electrophoresis, the gel was taken out, stained with 1 × Sybr Gold dye (Invitrogen, Cat.11494) for 15 minutes for imaging. The results are shown in Figure 1. The Comparative Sequence 1 is as follow:

Sense strand: 5'-CCUUGAGGCAUACUUCAAA-3' (SEQ ID NO: 1)
Antisense strand: 5'-UUUGAAGUAUGCCUCAAGGUU-3' (SEQ ID NO: 2)

Figure 1 shows that the semiquantitative test results of the stability of the tested drug conjugates in human plasma *in vitro.* The result shows that the conjugates of the present disclosure remain undegraded in human plasma over a period of up to 72 hours, showing excellent stability in human plasma.

**Experimental Example 2-2 *In vitro* stability of the conjugates of the present disclosure in cynomolgus monkey plasma**

Conjugate 32 and Comparative Sequence 1 (provided in the form of 20 μM in 0.9% sodium chloride aqueous solution respectively (calculated based on siRNA), 12 μL for each group) were mixed well with 108 μL of 90% cynomolgus monkey plasma (purchased from Guangzhou Hongquan Biotechnology Co., Ltd., HQ70082, diluted with 1×PBS) respectively to obtain a mixed solution, and was incubated at a constant temperature of 37°C. During the incubation, 10 μL of the sample was taken at each time point of 0, 2, 4, 6, 8, 24, 48 and 72 hours respectively, and immediately frozen in liquid nitrogen and cryopreserved in a -80°C refrigerator. Therein, 0 hour is the time when 10 μL of the mixed solution was taken immediately after the conjugate solution was mixed well with 90% cynomolgus monkey plasma. After sampling at each time point, each mixed solution was diluted 5-fold with 1×PBS (pH7.4), and 10 μL of each diluted mixed solution was taken for electrophoresis. Meanwhile, an equimolar amount of Conjugate 32 (2 μL, the concentration is 2 μM calculated based on siRNA) was mixed well with 8 μL of 1×PBS (pH 7.4) to obtain 10 μL of sample untreated with cynomolgus monkey plasma (designated as Con).

20 wt% of non-denatured polyacrylamide gel was prepared. The samples for electrophoresis in each group of the above diluted samples were mixed with 4 μL of loading buffer (20 mM EDTA, 36 wt% glycerol, and 0.06 wt% aqueous solution of bromophenol blue) and loaded into each gel well for electrophoresis under 80 mA constant current for 60 minutes. After electrophoresis, the gel was stained with 1×Sybr Gold dye (Invitrogen, Cat. 11494) for 15 minutes and imaged. The results are shown in Figure 2.

Figure 2 shows that the semiquantitative test results of the stability of the tested drug conjugates in cynomolgus monkey plasma *in vitro.* As can be seen, Conjugate 32 of the present disclosure remain undegraded in cynomolgus monkey plasma over a period of up to 72 hours, showing excellent stability in cynomolgus monkey plasma.

**Experimental Example 2-3 This experiment demonstrates the *in vivo* pharmacokinetic of Conjugate 32 in rats.**

In this Experimental Example, the rats in each experimental group (10 rats in each group, half male and half female) were administered with Conjugate 32 by subcutaneous injection respectively at a single dose of 1 mg/kg and 0.5 mg/kg of rat body weight. The plasma drug concentration and liver tissue drug concentration in rats at each time point were then measured.

First, SD rats were randomly divided into groups according to the body weight and gender of the rat using the PRISTIMA version 7.2.0 data system, and then the conjugates were administered at the designed doses, respectively. All animals were dosed based on body weight and subcutaneously administered at a single doses of 1 and 0.5 mg/kg (provided in the form of 0.1 mg/mL and 0.05 mg/mL in 0.9% aqueous sodium chloride solution, with a volume of 10 mL/kg). The rat's whole blood was collected from the jugular vein before administration and 5 minutes (±30 seconds), 30 minutes (± 1 minute), 1 hour (±2 minutes), 2 hours (±2 minutes), 6 hours (±5 minutes), 24 hours (±10 minutes), 48 hours (±20 minutes), 72 hours (±20 minutes), 120 hours (±30 minutes), and 168 hours (±30 minutes) after administration. The whole blood samples were then centrifuged at a centrifugal force of 1800×g for 10 minutes at 2-8°C to isolate plasma. About 70 μL of plasma sample was placed in one tube, and the rest of the same sample was placed in the other tube, both of which were cryopreserved at -70 to -86°C to be tested. Rats' liver tissues were collected at approximately 24, 48, 72, 120, and 168 hours after administration. The collection method included: anesthetizing the rats with pentobarbital sodium (intraperitoneal injection of 60 mg/kg) according to their body weight, euthanizing the rats by collecting abdominal aortic blood, and performing gross anatomy. The liver of each rat was sampled and stored in 1mL freezing tube below -68°C until determination and analysis.

The concentration of Conjugate 32 in rat plasma and liver tissue was quantitatively determined by HPLC-FLD (High Performance Liquid Fluorescence Chromatography) according to the following steps:

(1) The tissue was grinded to a tissue mass of no more than 80 mg, and added with Tissue and Cell Lysis Solution (supplier: Epicentre, as MTC096H) to prepare 66.7 mg/mL of tissue homogenate;
(2) The tissue homogenate was subjected to ultrasonication treatment (150 W, 30 s) to break cells;
(3) For each group of tissue samples, 75 μL of tissue samples were taken respectively and added to different culture wells of a 96-well PCR plate, and 5 μL of protease K (supplier: Invitrogen, as 25530-015) and 10 μL of mixed aqueous solution of 10 wt% acetonitrile and 0.01wt% Tween 20 were added to each culture well;
For each group of plasma samples, 20 μL of plasma was taken respectively and added to different culture wells of a 96-well PCR plate, and 45 μL of tissue and cell lysate, 5 μL of protease K, and 20 μL of mixed solution of 10wt%

acetonitrile and 0.01wt% Tween 20 were added to each culture well;

(4) The plate was blocked, placed into a PCR Thermal Cycler (supplier: Applied Biosystems, model: GeneAmp® PCR system 9700) and incubated at 65°C for 45 minutes;

(5) After incubation, each culture well was added with 10 μL of 3M KCl aqueous solution (supplier: Sigma-aldrich, Cat No. 60135-250ML), shaken well, and centrifuged at 3200 rcf for 15 minutes at 4°C, to store the supernatant for later use;

(6) For each group of tissue samples, 80 μL of the above supernatant was taken and added to 120 μL of hybridization mixture (formulation: 0.5 mL of 6 μM PNA probe (supplier:

Hangzhou TAHEPNA Biotechnology Co., Ltd.), 1 mL of 200 mM Trizma/pH=8, 5 mL of 8 M aqueous urea solution, 3.5 mL of $H_2O$, and 2 mL of acetonitrile);

For each group of plasma samples, 40 μL of the supernatant was taken and added to 160 μL of the hybridization mixture (formulation: 0.5 mL of 6 μM PNA probe, 1 mL of 200 mM Trizma/pH=8, 5 mL of 8 M aqueous urea solution, 7.5 mL of $H_2O$, and 2 mL of acetonitrile);

(7) The plate was blocked, placed in a PCR Thermal Cycler and incubated at 95°C for 15 minutes, and then immediately placed on ice for 5 minute to obtain incubated sample;

(8) Each incubated sample was transferred to a different well in a new conical-bottomed 96-well plate, shaken well, and centrifuged at 3200 rcf for 1 minute;

(9) For sample injection detection, quantitative analysis was performed using HPLC-FLD (liquid phase system supplier: Thermo Fisher, chromatographic model: ultimate 3000).

The analysis results are shown in Figures 3 and 4. Figures 3 and 4 respectively show the time-dependent metabolic curve of PK/TK plasma concentration of Conjugate 32 in rat plasma and the time-dependent metabolic curve of PK/TK tissue concentration of Conjugate 32 in rat liver.

In particular, Figure 3 is the time-dependent metabolic curve of PK/TK plasma concentration of Conjugate 32 in rat plasma at administration dosage of 1 mg/kg or 0.5 mg/kg.

Figure 4 is the time-dependent metabolic curve of the PK/TK tissue concentration of Conjugate 32 in rat liver at administration dosage of 1 mg/kg or 0.5 mg/kg.

As can be seen from the results of Figures 3 and 4, the concentration of Conjugate 32 in rat plasma rapidly decreased to below detection limit within a few hours at both low dose (0.5 mg/kg) and relatively higher dose (1 mg/kg); in contrast, the concentrations of Conjugate 32 in liver tissue maintained a higher and stable level for at least 150 hours. This shows that by conjugating the Compound N-$6_2$, the resultant drug conjugate can be specifically and significantly enriched in the liver and remained stable, with a high degree of targeting activity.

**Experimental Example 2-4 This experiment demonstrates the inhibitory efficiency of the drug conjugates of the present disclosure on the expression level of HBV mRNA *in vivo.***

In this experimental example, the inhibitory efficiency of Conjugate 32 on the expression level of HBV mRNA in HBV-transgenic mice C57BL/6J-Tg (A lb1HBV) 44Bri/J was investigated.

The HBV transgenic mice C57BL/6J-Tg (Alb1HBV) 44Bri/J used in this experimental example were purchased from the Department of Laboratory Animal Science, Peking University Health Science Center.

First, C57BL/6J-Tg (Alb1HBV) 44Bri/J mice (also abbreviated as 44Bri mice hereinafter) were randomly divided into groups (all female, 4 mice per group) according to serum HBsAg content, and were administered with different doses of Conjugate 32 as well as PBS (as control group). All animals were dosed based on body weight and was administered at a single dose by subcutaneous injection. Each conjugate was administered in the form of 0.2 mg/mL or 0.02 mg/mL (both calculated based on siRNA) in 0.9% aqueous sodium chloride solution, with an administration volume of 5 mL/kg mouse body weight; that is, the administration doses of each conjugate (calculated based on siRNA) were 1 mg/kg and 0.1 mg/kg. On day 14 after administrion, the animals were sacrificed, and the liver tissue of each mouse was collected separately and stored in RNA later (Sigma Aldrich Crop.). The liver tissue was homogenized with a tissue homogenizer and then extracted with Trizol according to standard operation procedures for total RNA extraction to obtain the total RNA.

For each mouse, 1 μg of total RNA was taken, and the extracted total RNA was reverse-transcribed into cDNA using ImProm-II™ reverse transcription kit (Promega Crop.) according to the instructions thereof to obtain a solution containing cDNA, and then the expression level of HBV mRNA in liver tissue was determined by the fluorescent quantitative PCR kit (Beijing Cowin Biosciences Co., Ltd.). In this fluorescent quantitative PCR method, β-actin gene was used as an internal control gene, and HBV and β-actin were detected by using primers for HBV and β-actin, respectively. The sequences of the primers for detection are shown in Table 3A.

Table 3A Sequences of the primers for detection

| genes | Upstream primers | Downstream primers |
|---|---|---|
| HBV | 5'CCGTCTGTGCCTTCTCATCT-3' (SEQ ID NO: 677) | 5'TAATCTCCTCCCCCAACTCC-3' (SEQ ID NO: 678) |
| β-actin | 5'-AGCTTCTTTGCAGCTCCTTCGTTG-3' (SEQ ID NO: 679) | 5'-TTCTGACCCATTCCCACCATCACA-3' (SEQ ID NO: 680) |

The relative quantitative calculation of the expression level of and inhibition rate against the target gene in each test group was carried out using the comparative Ct (ΔΔCt) method, and the calculation method was as follows:

$$\Delta Ct \text{ (test group)} = Ct \text{ (target gene of test group)} - Ct \text{ (internal control gene of test group)}$$

$$\Delta Ct \text{ (control group)} = Ct \text{ (target gene of control group)} - Ct \text{ (internal control gene of control group)}$$

$$\Delta\Delta Ct \text{ (test group)} = \Delta Ct \text{ (test group)} - \Delta Ct \text{ (average of control group)}$$

$$\Delta\Delta Ct \text{ (control group)} = \Delta Ct \text{ (control group)} - \Delta Ct \text{ (average of control group)}$$

wherein ΔCt (average of control group) was the arithmetic mean of the ΔCt (control group) of the four mice of the control group. Thus, each mouse in the test and control groups corresponded to a ΔΔCt value.

The expression level of HBV mRNA in the test group was normalized based on the control group, and the expression level of HBV mRNA in the blank control group was defined as 100%,

$$\text{Relative expression level of HBV mRNA in the test group} = 2^{-\Delta\Delta Ct} \text{ (test group)} \times 100\%$$

$$\text{Inhibition rate against HBV mRNA in the test group} = (1 - \text{relative expression level of HBV mRNA in the test group}) \times 100\%$$

Figure 5 is a scatter plot of the expression level of HBV mRNA in liver tissue of mice in the control group and after being administered with 1 mg/kg and 0.1 mg/kg of drug Conjugate 32, respectively. As can be seen from the results of Figure 5, Conjugate 32 showed excellent inhibition rate against the mRNA of HBV gene in liver tissue of 44Bri mice in an *in vivo* experiment, wherein the inhibition rate showed a significant dose dependence, and on day 14 after administration, was up to 89.86% at a dose of 1 mg/kg.

**Experimental Example 2-5 This experiment demonstrates the time correlation test of the inhibitory efficiency of the siRNA of the drug conjugates of the present disclosure on the expression levels of serum HBsAg and HBV DNA in HBV transgenic mice.**

M-Tg HBV mice (purchased from the Animal Department of Shanghai Public Health Center) were used. The preparation method of transgenic mice is as decribed in Ren J et al., J. Medical Virology. 2006, 78: 551-560. The AAV virus used is rAAV8-1.3HBV, type D (ayw) virus, purchased from Beijing FivePlus Molecular Medicine Institute Co. Ltd., $1\times10^{12}$ viral genome (v.g.)/mL, Lot No.2016123011. The AAV virus was diluted to $5\times10^{11}$ v.g./mL with sterilized PBS prior to the experiment. Each mouse was injected with 200 μL (that is, each mouse was injected with $1\times10^{11}$ v.g). On day 28 post virus injection, orbital blood (about 100 μL) was collected from all mice for collecting serum for dectection of HBsAg. After the animals were successfully modeled, they were randomly divided into groups according to serum

HBsAg content (five mice per group), and were administered with Conjugate 32 and PBS as blank control. All animals were dosed based on body weight and was administered at a single dose by subcutaneous injection. Each conjugate was administered in the form of 0.2 mg/mL or 0.6 mg/mL (both calculated based on siRNA) in 0.9% aqueous sodium chloride solution, with an administration volume of 5 mL/kg mouse body weight; that is, the administration doses of each conjugate (calculated based on siRNA) were 1 mg/kg and 3 mg/kg. The blank control group was administered with 5 mL/kg of 1×PBS. The blood was collected from mouse orbital venous plexus before administration and on day 7, 14, 21, 28, 35, 42, 49, 63, and 70 days after administration, and the serum HBsAg level was determined at each time point.

About 0.1mL orbital blood was taken each time, and no less than 20 μL serum was collected after centrifugation. The expression level of HBsAg in serum was determined by using HBsAg CLIA kit (Autobio, CL0310) according to the instructions provided by the manufacturer.

The inhibition rate against HBsAg is calculated according to the following equation:

$$\text{The inhibition rate against HBsAg} = (1\text{-HBsAg content after administration/HBsAg content before administration}) \times 100\%,$$

wherein HBsAg content is expressed as the equivalents (UI) of HBsAg per millilitre (mL) of serum.

Figure 6 is a time-dependent curve of serum HbsAg level in transgenic mice administered with different doses of Conjugate 32 and in transgenic mice of the blank control group at different time points after administration. As can be seen from Figure 6, the PBS negative control group showed no inhibitory effect at different time points after administration; in contrast, Conjugate 32 at different doses (3 mg/kg and 1 mg/kg) showed excellent inhibitory effect on HBsAg. In particular, at the dose of 3 mg/kg, Conjugate 32 showed a high inhibition rate of up to 97.80% against serum HBsAg over a period of up to 70 days, which indicates that it can stably and efficiently inhibit the expression of HBV gene in the HBV transgenic mice over a long time period.

**Experimental Example 2-6 This experiment demonstrates the *in vivo* inhibitory effect of the conjugates of the present disclosure on the expresion of HBV mRNA in mice.**

In this experimental example, the inhibitory efficiency of different concentrations of Conjugate 32 on the expression level of HBV mRNA in HBV transgenic mice C57BL/6J-Tg (Alb1HBV) 44Bri/J was investigated.

First, C57BL/6J-Tg (Alb1HBV) 44Bri/J mice were randomly divided into groups (all female, five mice per group) according to serum HBsAg content, and numbered respectively, and a NS (normal saline) group was added as a control group. All animals were dosed based on body weight. Conjugate 32 was subcutaneously administered at doses of 1 mg/kg and 0.1 mg/kg, respectively. The conjugate was administered in the form of 0.2 mg/mL or 0.02 mg/mL conjugate (both calculated based on siRNA) in 0.9% aqueous sodium chloride solution, with an administration volume of 5 mL/kg. On day 7 after administrion, the animals were sacrificed, and the liver tissues of each mouse was collected separately and stored in RNA later (Sigma Aldrich). The liver tissue was homogenized with a tissue homogenizer and then extracted with Trizol according to standard operation procedures for total RNA extraction to obtain the total RNA.

The expression level of HBV mRNA in the liver tissue of each mouse was determined by real-time fluorescent quantitative PCR. Specifically, the total RNA extracted from the liver tissue of each mouse was reverse-transcribed into cDNA using ImProm-II™ reverse transcription kit (Promega) according to the instructions thereof, and then the expression level of HBV mRNA in liver tissue was determined by the fluorescent quantitative PCR kit (Beijing Cowin Biosciences Co., Ltd.) and the inhibitory efficiency was calculated. In this fluorescent quantitative PCR method, GAPDH gene was used as an internal control gene, and the HBV and GAPDH were detected by using the primers for HBV and GAPDH, respectively.

The sequences of the primers for detection are shown in Table 4A.

The relative quantitative calculation of the expression level of the target HBV gene in each test group and the control group was carried out using the comparative Ct (ΔΔCt) method, and the calculation method was as follows:

ΔCt (test group) = Ct (target gene of test group) - Ct (internal control gene of test group)
ΔCt (control group) = Ct (target gene of control group) - Ct (internal control gene of control group)
ΔΔCt (test group) = ΔCt (test group) - ΔCt (average of control group)
ΔΔCt (control group) = ΔCt (control group) - ΔCt (average of control group)
wherein ΔCt (average of control group) was the arithmetic mean of the ΔCt (control group) of the five mice in the control group. Thus, each mouse in the test and control groups corresponded to a ΔΔCt value.

The expression level of HBV mRNA in the test group was normalized based on the control group, and the

expression level of HBV mRNA in the control group was defined as 100%,

$$\text{Relative expression level of HBV mRNA in the test group} = 2^{-\Delta\Delta Ct}\ (\text{test group}) \times 100\%$$

For the siRNA in the same test group, the mean of the relative expression level of HBV mRNA in the test group at each concentration was the arithmetic mean of the relative expression levels of the five mice at that concentration.

Therein, the control group refers to the mice in the control group administered with PBS in this experiment, and each test group refers to the mice in the administration group adminstered with different drug conjugates. The results are shown in Figure 7A:

Table 4A Sequences of the primers for detection

| genes | Upstream primers | Downstream primers |
|---|---|---|
| HBV | 5'-CGTTTCTCCTGGCTCAGTTTA-3' (SEQ ID NO: 729) | 5'-CAGCGGTAAAAAGGGACTCAA-3' (SEQ ID NO: 730) |
| GAPDH | 5'-AGAAGGCTGGGGCTCATTTG-3' (SEQ ID NO: 731) | 5'-AGGGGCCATCCACAGTCTTC-3' (SEQ ID NO: 1732) |

Figure 7A is a scatter plot of relative expression levels of HBV mRNA in liver tissue of mice on day 7 after being administered with the blank control and different doses of Conjugate 32. As can be seen from the results of Figure 7A, Conjugate 32 showed excellent inhibition rate against the mRNA of HBV gene in liver tissue of 44Bri mice in an *in vivo* experiment, which was up to 91.96% at a dose of 1 mg/kg on day 7 after administration. Also, the relatively higher concentration of Conjugate 32 showed significantly higher inhibition rate against the mRNA of HBV gene in liver tissue of 44Bri hepatitis B mice in an *in vivo* experiments as compared with that of the low concentration of conjugate.

Conjugates 38, 39, and 40 (X2-siHBa1M2SVP, W2-siHBa1M2SVP and V2-siHBa1M2SVP) at doses of 1 mg/kg and 0.1 mg/kg were tested using the same procedure, except that the conjugates used were Conjugates 38, 39, and 40, respectively. The results are shown in Figure 7B.

Figure 7B is a scatter plot of relative expression levels of HBV mRNA in liver tissue of mice on day 7 after administered with the blank control and different doses of Conjugates 38, 39 and 40. As can be seen from the results of Figure 7B, different conjugates of the present disclosure showed excellent inhibition rate against the mRNA of HBV gene in liver tissue of 44Bri mice in an *in vivo* experiment, which was up to 90.37-95.03% at a dose of 1 mg/kg on day 7 after administration.

**Experimental Example 2-7 This experiment demonstrates the time correlation test of the inhibitory efficiency of Conjugate 32 on HBsAg expression and HBV DNA in the serum of HBV transgenic mice.**

The AAV-HBV low concentration model mice were used. After the animal models were successfully established, they were randomly divided into groups according to serum HBsAg content (five mice per group). Each group was respectively administered with two different doses of Conjugate 32 and with PBS as blank control. All animals were dosed based on body weight and were administered by subcutaneous injection at single doses 3 mg/kg or 1 mg/kg of Conjugate 32, respectively using 0.6 mg/mL or 0.2 mg/mL conjugate in 0.9% aqueous sodium chloride solution with an administration volume of 5 mL/kg. The blank group was administered with only 5 mL/kg 1×PBS. Blood was collected from mouse orbital venous plexus before administration and on day 14, 28, 42, 56, 70, 84, 98, 112, 126, 140, 154, 168, 182, and 196 days after administration, and the serum HBsAg level was determined at each time point.

About 100 μL orbital blood was taken each time, and no less than 20 μL serum was collected after centrifugation. The HBsAg content in serum was determined by using HBsAg CLIA kit (Autobio, CL0310) according to the instructions provided by the manufacturer. DNA in serum was extracted according to the instructions of QIAamp 96 DNA Blood Kit, and was subject to quantitative PCR to determine the expression level of HBV DNA.

$$\text{Normalized expression level of HBsAg} = (\text{HBsAg content after administration}/\text{HBsAg content before administration}) \times 100\%.$$

The inhibition rate against HBsAg = (1-HBsAg content after administration/HBsAg content before administration) $\times$ 100%, wherein HBsAg content is expressed as the equivalents (UI) of HBsAg per millilitre (mL) of serum.

Normalized expression level of HBV DNA = (HBV DNA content after administration/HBV DNA content before administration) $\times$100%.

The inhibition rate against HBV DNA = (1-HBV DNA content after administration/HBV DNA content before administration) $\times$ 100%, wherein HBV DNA content is expressed as the copies of HBV DNA per millilitre (mL) of serum.

Figures 8A and 8B are respectively curves showing the *in vivo* relative levels of HBsAg and HBV DNA in HBV transgenic mice administered with different doses of Conjugate 32 or PBS. As can be seen from the results of Figures 8A and 8B, the NS negative control group showed no inhibitory effect at different time points after administration; in contrast, Conjugate 32 at a concentration of 3 mg/kg exhibited high inhibition rate against HBsAg and excellent inhibitory effect on HBV DNA at different time points over a period of up to 100 days after the administration. The inhibition rate of Conjugate 32 at a dose of 3 mg/kg against serum HBsAg was up to 90.9%, and the inhibition rate against HBV DNA was up to 85.7%, and its inhibitory effects at different time points were all higher than the inhibitory effects of Conjugate 32 at a lower concentration of 1 mg/kg, which indicates that Conjugate 32 can stably and efficiently inhibit the expression of HBV gene over a longer time period.

In a further experiment, the inhibitory effect on HBV mRNA on day 70 was tested according to the detection method of Example 6 above, and the results are shown in Figure 9.

As can be seen from Figure 9, different concentrations of the Conjugate 32 can still inhibit the expression level of HBV mRNA to a certain extent on day 70 after administration, thereby showing a special application prospect in terms of long-term effect.

**Experimental Example 3 Effect experiments of the drug conjugates in Table 2B**

**Experimental Example 3-1 This experiment illustrates the inhibitory efficiency of the drug conjugates in Table 2B against the expression level of HBV mRNA in HepG2.2.15 cells.**

The HepG2.2.15 cells were seeded into a 24-well plate at 7 $\times$ $10^4$ cells/well with H-DMEM complete medium. After 16 hours, when the cell growth density reached 70-80%, the H-DMEM complete medium in the culture well was aspirated, and 500 $\mu$L Opti-MEM medium (GIBCO company) was added to each well, and the cells were cultured for another 1.5 hours.

Each of Conjugates 43-64 and Comparative Conjugate 1 was respectively formulated into working solutions of drug conjugate at 3 different concentrations of 50 $\mu$M, 5 $\mu$M and 0.5 $\mu$M (all calculated based on siRNA) with DEPC water. For each drug conjugate, 3A1 to 3A3 solutions were formulated, respectively. Each of 3A1 to 3A3 solutions contained 0.6 $\mu$L of the above working solution of drug conjugate at one of the above 3 concentrations and 50 $\mu$L of Opti-MEM medium.

Formlaution of 3B solution: each 3B solution contained 1 $\mu$L of Lipofectamine™ 2000 and 50 $\mu$L of Opti-MEM medium.

One portion of 3B solution was respectively mixed with the resultang 3A1, 3A2 or 3A3 solution of each drug conjugate, and respectively incubated at room temperature for 20 minutes to obtain a transfection complex 3X1, 3X2 or 3X3 of each siRNA.

One portion of 3B solution was mixed with 50 $\mu$L of Opti-MEM medium and incubated for 20 minutes at room temperature to obtain a transfection complex 3X4.

The transfection complex 3X1, 3X2 or 3X3 of each drug conjugate was respectively added to the culture wells in an amount of 100 $\mu$L/well and mixed well to obtain a transfection complex with the final concentration of each siRNA of about 50 nM, 5 nM or 0.5 nM, respectively. Each transfection complex was respectively used to transfect three culture wells to obtain drug conjugate-containing transfection mixtures (designated as test group).

The transfection complex 3X4 was respectively added to another three culture wells in an amount of 100 $\mu$L/well to obtain siRNA-free transfection mixtures (designated as blank control group).

After the drug conjugate-containing transfection mixtures and the drug conjugate-free transfection mixture were cultured in the culture wells for 4 hours, each well was supplemented with 1 mL of H-DMEM complete medium containing 20% FBS. The 24-well plate was placed in a $CO_2$ incubator and cultured for another 24 hours.

Subsequently, the total RNA of the cells in each well was extracted by RNeasy Mini Kit (QIAGEN company, Cat No. 74106) according to the detailed preoceudre as described in the instruction.

For the cells of each well, 1 $\mu$g of the total RNA was taken, and was formulated into a 20 $\mu$L reverse transcription

reaction system for reverse transcription of the total RNA of the cell by using the reagent provided in the reverse transcription kit GoldenstarTM RT6 cDNA Synthesis Kit (purchased from Beijing Tsingke Biotechnology Co., Ltd., Cat. No. TSK301M) according to the precedures for reverse transcription in the instruction of the kit, in which GoldenstarTM Oligo (dT)17 was selected as the primer. Conditions for reverse transcription were as follows: the reverse transcription reaction system was incubated at 50°C for 50 minutes, then incubated at 85°C for 5 minutes, and finally incubated at 4°C for 30 seconds; after the reaction was completed, 80 μL of DEPC water was added to the reverse transcription reaction system to obtain a cDNA-containing solution.

For each reverse transcription reaction system, 5 μL of the above cDNA-containing solution was taken as the template, and was formulated into a 20 μL qPCR reaction system by using the reagent provided in the NovoStart® SYBR qPCR SuperMix Plus kit (purchased from Novoprotein Scientific Co., Ltd., Cat No. E096-01B), wherein the sequences of PCR primers for amplifying the target gene HBV and the internal control gene GAPDH are shown in Table 3B, and the final concentration of each primer was 0.25 μM. Each qPCR reaction system was placed in an ABI StepOnePlus Real-Time PCR Thermal Cycler, and the amplification was performed by a three-step method using the following amplification procedure: pre-denaturation at 95°C for 10 minutes, then denaturation at 95°C for 30 s, annealing at 60°C for 30 s, and extension at 72°C for 30 s (wherein the above process of denaturation, annealing and extension was repeated for 40 times), to obtain a product W1 containing the amplified target gene HBV and the amplified internal control gene GAPDH. The product W1 was then sequentially incubated at 95°C for 15 s, 60°C for 1 min, and 95°C for 15 s. The melting curves of the target gene HBV and the internal control gene GAPDH in the product W1 were respectively collected using a real-time fluorescent quantitative PCR Thermal Cycler, and the Ct values of the target gene HBV and the internal control gene GAPDH were obtained.

Table 3B The sequences of the primers for detection

| Gene | Upstream primers | Downstream primers |
|---|---|---|
| HBV | 5'- CGTTTCTCCTGGCTCAGTTTA - 3' (SEQ ID NO: 681) | 5'- CAGCGGTAAAAAGGGACTCAA -3' (SEQ ID NO: 682) |
| GAPDH | 5'- AGAAGGCTGGGGCTCATTTG- 3' (SEQ ID NO: 683) | 5'- AGGGGCCATCCACAGTCTTC -3' (SEQ ID NO: 684) |

The relative quantitative calculation of the expression level of the target HBV gene in each test group and the control group was carried out using the comparative Ct (ΔΔCt) method, and the calculation method was as follows:

ΔCt (test group) = Ct (target gene of test group) - Ct (internal control gene of test group)
ΔCt (control group) = Ct (target gene of control group) - Ct (internal control gene of control group)
ΔΔCt (test group) = ΔCt (test group) - ΔCt (average of control group)
ΔΔCt (control group) = ΔCt (control group) - ΔCt (average of control group)
wherein ΔCt (average of control group) was the arithmetic mean of the ΔCt (control group) of the three culture wells of the control group. Thus, each culture well in the test and control groups corresponded to a ΔΔCt value.

The expression level of HBV mRNA in the test group was normalized based on the control group, and the expression level of HBV mRNA in the control group was defined as 100%,

$$\text{Relative expression level of HBV mRNA in the test group} = 2^{-\Delta\Delta Ct\text{ (the test group)}} \times 100\%$$

For the siRNA in the same test group, the mean of the relative expression level of HBV mRNA in the test group at each concentration was the arithmetic mean of the relative expression levels of the three culture wells at that concentration.

Therein, each test group was HepG2.2.15 cells respectively treated with the drug conjugates listed in Table 2B, and the drug conjugates included the drug conjugates Conjugates 43-64 and the control drug conjugate Comparative Conjugate 1.

Table 4B below shows the determination results of the inhibitory activities of the test drug conjugates listed in Table 2B and the control drug conjugate against the expression of HBV mRNA in HepG2.2.15 cells.

Table 4B *In vitro* inhibition rates of the drug conjugates at different concentrations against HBV mRNA

| Conjugate Serial No. | Conjugate No. | Inhibition rate against mRNA (%) | | |
|---|---|---|---|---|
| | | 50 nM | 10 nM | 1 nM |
| Conjugate 43 | N6-siHBb1M1S | 54.1 | 40.2 | 24.9 |
| Conjugate 44 | N6-siHBb2M1S | 53.9 | 37.5 | 29.2 |
| Conjugate 45 | N6-siHBb1M2 | 54.3 | 35.5 | 26.5 |
| Conjugate 46 | N6-siHBb2M2 | 52.2 | 38.5 | 27.1 |
| Conjugate 47 | N6-siHBb 1M1 SVP | 55.2 | 48.2 | 28.7 |
| Conjugate 48 | N6-siHBb2M1SP | 55.0 | 48.0 | 28.6 |
| Conjugate 49 | N6-siHBb2M1SPs | 54.9 | 47.8 | 28.4 |
| Conjugate 50 | N6-siHBb3M1S | 54.0 | 47.5 | 28.0 |
| Conjugate 51 | N6-siHBb2M1SVP | 54.1 | 48.3 | 30.8 |
| Conjugate 52 | N6-siHBb 1M2SVP | 52.0 | 38.2 | 29.2 |
| Conjugate 53 | N6-siHBb2M2SVP | 53.2 | 37.3 | 27.2 |
| Conjugate 54 | N6-siHBb1M5SVP | 52.2 | 36.4 | 25.6 |
| Conjugate 55 | N6-siHBb1M3SVP | 43.0 | 28.4 | 21.1 |
| Conjugate 56 | N6-siHBb 1M4SVP | 39.4 | 25.2 | 17.1 |
| Conjugate 57 | N6-siHBb4M1SVP | 53.5 | 36.4 | 24.2 |
| Conjugate 58 | N6-siHBb 1 | 57.4 | 45.1 | 32.3 |
| Conjugate 59 | N6-siHBb2 | 56.5 | 49.9 | 29.3 |
| Conjugate 60 | X2-siHBb2M1SVP | 54.5 | 49.2 | 30.6 |
| Conjugate 61 | W2-siHBb2M1SVP | 54.4 | 48.6 | 30.4 |
| Conjugate 62 | V2-siHBb2M1SVP | 54.2 | 48.5 | 30.2 |
| Conjugate 63 | O2-siHBb2M1SVP | 54.2 | 48.8 | 30.4 |
| Conjugate 64 | P2-siHBb2M1SVP | 54.1 | 48.5 | 30.2 |
| Comparative Conjugate 1 | N6-NC | 2.1 | 1.5 | -2.8 |

As can be seen from the results of Table 4B, each drug conjugate in Table 2B exhibited very high inhibitory activity against HBV mRNA in HepG2.2.15 cells *in vitro* and could show an inhibition rate of up to 57.4% against HBV mRNA at the siRNA concentration of 50 nM.

**Experimental Example 3-2 This experiment illustrates the stability of the drug conjugates in Table 2B in human plasma *in vitro.***

The drug conjugates Conjugates 43-57 and 60-64 (provided as 20 μM in 0.9 wt% NaCl aqueous solution (calculated based on siRNA), 12 μL per group) were respectively mixed well with 108 μL of 90% human plasma (purchased from Jiangsu Hematology Institute and diluted with 1×PBS (pH 7.4)) to obtain mixed solutions, and were incubated at a constant temperature of 37°C. 10 μL mixed solution was taken at 0 h, 8 h, 24 h and 48 h, respectively, and immediately frozen in liquid nitrogen and cryopreserved in a -80°C freezer. Therein, 0 hour refers to the time when 10 μL of the mixed solution was taken immediately after the conjugate solution was mixed well with 90% human plasma. After sampling at each time point, each mixed solution was diluted 5-fold with 1×PBS (pH 7.4) and then taken in a volume of 10 μL for electrophoresis. Meanwhile, an equimolar amount of each of the above conjugate solutions (2 μL, the concentration is 2 μM calculated based on siRNA) was mixed well with 8 μL of 1× PBS (pH 7.4) respectively to prepare 10 μL of sample untreated with human plasma (designated as "untreated") for electrophoresis.

20 wt% of non-denatured polyacrylamide gel was prepared. The above samples for electrophoresis in each group were respectively mixed with 4 μL of loading buffer (20 mM EDTA, 36 wt% glycerol, and 0.06 wt% bromophenol

blue) and then loaded into each gel hole for electrophoresis under 80 mA constant current for about 60 minutes. After electrophoresis, the gel was taken out, stained with 1×Sybr Gold dye (Invitrogen, Cat. 11494) for 15 minutes for imaging. The stability results were calculated and the results are shown in Table 5B.

Table 5B shows the semiquantitative test result of the stability of the drug conjugates listed in Table 2B in human plasma *in vitro.* The result is expressed as the ratio (RL) of the longest fragment remaining after the incubation of the drug conjugate with human plasma to the longest fragment of untreated siRNA.

Table 5B The plasma stability of different drug conjugates over time

| Conjugate Serial No. | Conjugate No. | RL (%) | | | | |
|---|---|---|---|---|---|---|
| | | Untreated | 0h | 8h | 24h | 48h |
| Conjugate 43 | N6-siHBb1M1S | 100 | 100 | 99.6 | 96.0 | 94.7 |
| Conjugate 44 | N6-siHBb2M1S | 100 | 100 | 99.7 | 96.2 | 94.7 |
| Conjugate 45 | N6-siHBb1M2 | 100 | 100 | 99.6 | 96.1 | 94.8 |
| Conjugate 46 | N6-siHBb2M2 | 100 | 100 | 99.4 | 96.7 | 95.3 |
| Conjugate 47 | N6-siHBb1M1SVP | 100 | 100 | 99.3 | 96.5 | 95.5 |
| Conjugate 48 | N6-siHBb2M1SP | 100 | 100 | 99.2 | 96.2 | 95.1 |
| Conjugate 49 | N6-siHBb2M1SPs | 100 | 100 | 99.5 | 96.1 | 95.2 |
| Conjugate 50 | N6-siHBb3M1S | 100 | 100 | 99.2 | 96.0 | 94.9 |
| Conjugate 51 | N6-siHBb2M1SVP | 100 | 100 | 99.7 | 96.7 | 95.8 |
| Conjugate 52 | N6-siHBb1M2SVP | 100 | 100 | 99.7 | 97.1 | 95.1 |
| Conjugate 53 | N6-siHBb2M2SVP | 100 | 100 | 99.4 | 96.4 | 95.5 |
| Conjugate 54 | N6-siHBb1M5SVP | 100 | 100 | 99.7 | 95.5 | 94.6 |
| Conjugate 55 | N6-siHBb1M3SVP | 100 | 100 | 99.7 | 96.4 | 96.3 |
| Conjugate 56 | N6-siHBb1M4SVP | 100 | 100 | 99.5 | 97.3 | 94.4 |
| Conjugate 57 | N6-siHBb4M1SVP | 100 | 100 | 99.6 | 97.1 | 94.3 |
| Conjugate 60 | X2-siHBb2M1SVP | 100 | 100 | 99.8 | 96.3 | 94.4 |
| Conjugate 61 | W2-siHBb2M1SVP | 100 | 100 | 99.5 | 96.6 | 94.5 |
| Conjugate 62 | V2-siHBb2M1SVP | 100 | 100 | 99.4 | 97.1 | 94.8 |
| Conjugate 63 | O2-siHBb2M1SVP | 100 | 100 | 99.6 | 96.1 | 95.2 |
| Conjugate 64 | P2-siHBb2M1SVP | 100 | 100 | 99.7 | 95.7 | 95.0 |

As can be seen from Table 5B, each drug conjugate exhibited excellent stability in human plasma.

**Experimental example 3-3 This experiment illustrates the inhibitory efficiency of the drug conjugates in Table 2B against the expression of HBV mRNA *in vivo*.**

In this experimental example, the inhibitory efficiency of the drug conjugates Conjugates 43-57 and 60-64 against the expression level of HBV mRNA in the HBV transgenic mice C57BL/6J-Tg(Alb1HBV)44Bri/J was investigated.

The HBV transgenic mice C57BL/6J-Tg (Alb1HBV) 44Bri/J used in this experimental example were purchased from the Department of Laboratory Animal Science, Peking University Health Science Center.

First, C57BL/6J-Tg(Alb1HBV)44Bri/J mice were randomly divided into groups (all female, five mice per group) according to serum HBsAg content, and numbered according to the drug conjugates in Table 2B, and a PBS control group was added. All animals were dosed based on body weight and was administered at a single dose by subcutaneous injection. Each conjugate was administered in the form of 0.2 mg/mL (calculated based on siRNA) in 0.9% aqueous sodium chloride solution, with an administration volume of 5 mL/kg mouse body weight; that is, the administration doses of each conjugate (calculated based on siRNA) were 1 mg/kg. Each mouse in the control group was only administered with 5 mL/kg 1×PBS. On day 14 after administrion, the animals were sacrificed, and the liver tissue of each mouse was

collected separately and stored in RNA later (Sigma Aldrich Crop.). The liver tissue was homogenized with a tissue homogenizer and then extracted with Trizol according to standard operation procedures for total RNA extraction to obtain the total RNA.

For the liver tissue of each mouse, 1 μg of total RNA was taken, and the extracted total RNA was reverse-transcribed into cDNA using ImProm-II™ reverse transcription kit (Promega Crop.) according to the instructions thereof to obtain a solution containing cDNA, and then the expression level of HBV mRNA in liver tissue was determined by the fluorescent quantitative PCR kit (Beijing Cowin Biosciences Co., Ltd.). In this fluorescent quantitative PCR method, β-actin gene was used as an internal control gene, and HBV and β-actin were detected by using primers for HBV and β-actin, respectively. The sequences of the primers for detection are shown in Table 6B.

Table 6B The sequences of the primers for detection

| Gene | Upstream primers | Downstream primers |
|---|---|---|
| HBV | 5'- CGTTTCTCCTGGCTCAGTTTA -3' (SEQ ID NO: 685) | 5'- CAGCGGTAAAAAGGGACTCAA -3' (SEQ ID NO: 686) |
| β-actin | 5'- AGCTTCTTTGCAGCTCCTTCGTTG - 3' (SEQ ID NO: 687) | 5'- TTCTGACCCATTCCCACCATCACA- 3' (SEQ ID NO: 688) |

The relative quantitative calculation of the expression level of and inhibition rate against the target gene in each test group was carried out using the comparative Ct (ΔΔCt) method, and the calculation method was as follows:

$$\Delta Ct \text{ (test group)} = Ct \text{ (target gene of test group)} - Ct \text{ (internal control gene of test group)}$$

$$\Delta Ct \text{ (control group)} = Ct \text{ (target gene of control group)} - Ct \text{ (internal control gene of control group)}$$

$$\Delta\Delta Ct \text{ (test group)} = \Delta Ct \text{ (test group)} - \Delta Ct \text{ (average of control group)}$$

$$\Delta\Delta Ct \text{ (control group)} = \Delta Ct \text{ (control group)} - \Delta Ct \text{ (average of control group)}$$

wherein ΔCt (average of control group) was the arithmetic mean of the ΔCt (control group) the four mice of the control group. Thus, each mouse in the test and control groups corresponded to a ΔΔCt value.

The expression level of HBV mRNA in the test group was normalized based on the control group, and the expression level of HBV mRNA in the blank control group was defined as 100%,

$$\text{Relative expression level of HBV mRNA in the test group} = 2^{-\Delta\Delta Ct} \text{ (test group)} \times 100\%$$

$$\text{Inhibition rate against HBV mRNA in the test group} = (1 - \text{relative expression level of HBV mRNA in the test group}) \times 100\%$$

Table 7B shows the inhibition rates against HBV mRNA in the liver tissue of mice in the control group and after being administration of 1 mg/kg of Drug Conjugates 43-64, respectively.

Table 7B Inhibition of the expression of HBV mRNA by the drug conjugates in mouse liver

| Conjugate Serial No. | Conjugate No. | Administration dose (mg/kg) | Inhibition rate against HBV mRNA in liver (%) |
|---|---|---|---|
| Conjugate 43 | N6-siHBb1M1S | 1 | 79.4 |
| Conjugate 44 | N6-siHBb2M1S | 1 | 84.2 |
| Conjugate 45 | N6-siHBb1M2 | 1 | 80.0 |
| Conjugate 46 | N6-siHBb2M2 | 1 | 84.5 |
| Conjugate 47 | N6-siHBb1M1SVP | 1 | 82.2 |
| Conjugate 48 | N6-siHBb2M1SP | 1 | 88.5 |
| Conjugate 49 | N6-siHBb2M1SPs | 1 | 82.0 |
| Conjugate 50 | N6-siHBb3M1S | 1 | 79.0 |
| Conjugate 51 | N6-siHBb2M1SVP | 1 | 88.2 |
| Conjugate 52 | N6-siHBb1M2SVP | 1 | 78.7 |
| Conjugate 53 | N6-siHBb2M2SVP | 1 | 85.4 |
| Conjugate 54 | N6-siHBb1M5SVP | 1 | 76.1 |
| Conjugate 55 | N6-siHBb1M3SVP | 1 | 68.3 |
| Conjugate 56 | N6-siHBb1M4SVP | 1 | 68.6 |

(continued)

| Conjugate Serial No. | Conjugate No. | Administration dose (mg/kg) | Inhibition rate against HBV mRNA in liver (%) |
|---|---|---|---|
| Conjugate 57 | N6-siHBb4M1SVP | 1 | 86.0 |
| Conjugate 60 | X2-siHBb2M1SVP | 1 | 86.1 |
| Conjugate 61 | W2-siHBb2M1SVP | 1 | 86.4 |
| Conjugate 62 | V2-siHBb2M1SVP | 1 | 86.5 |
| Conjugate 63 | O2-siHBb2M1SVP | 1 | 86.8 |
| Conjugate 64 | P2-siHBb2M1SVP | 1 | 86.2 |
| PBS | - | NA | NA |

As can be seen from the above results, all conjugates in the Examples of the present disclosure showed high *in vivo* inhibitory activity against HBV mRNA in mice, and exhibited an inhibition rate of up to 88.5% against HBV mRNA at a dose of 1 mg/kg.

**Experimental example 3-4 This experiment illustrates the time correlation test of the inhibitory efficiency of the siRNAs in the drug conjugates in Table 2B against the expression level of HBsAg and HBV DNA in the serum of HBV transgenic mice.**

AAV-HBV models were prepared according to the method in the literature (DONG Xiaoyan et al., Chin J Biotech 2010, May 25; 26(5): 679-686) by using rAAV8-1.3HBV, type D (ayw) virus (purchased from Beijing FivePlus Molecular Medicine Institute Co. Ltd., $1\times10^{12}$ viral genome (v.g.)/mL, Lot No. 2016123011). The AAV virus was diluted to $5\times10^{11}$ v.g./mL with sterilized PBS prior to the experiment. Each mouse was injected with 200 $\mu$L (that is, each mouse was injected with $1\times10^{11}$ v.g). On day 28 post virus injection, orbital blood (about 100 $\mu$L) was collected from all mice for collecting serum for dectection of HBsAg and HBV DNA. After the animals were successfully modeled, they were randomly divided into groups according to serum HBsAg content (five mice per group), and were administered with the drug conjugates Conjugates 43-49, 52-53, 57 and 60-64, and PBS as blank control, respectively. All animals were dosed based on body weight and was administered at a single dose by subcutaneous injection. Each conjugate was administered in the form of 0.6 mg/mL (calculated based on siRNA) in 0.9 % NaCl aqueous solution, with an administration volume of 5 mL/kg mouse body weight; that is, the administration doses of each conjugate (calculated based on siRNA) was 3 mg/kg. Each mouse in the blank control group was only administered with 5 mL/kg mouse body weight of 1×PBS. The blood was collected from mouse orbital venous plexus before administration and on day 7, 14, 21, 28, 56 and 84 after administration, and the serum HBsAg level was determined at each time point.

About 100 $\mu$L orbital blood was taken each time, and no less than 20 $\mu$L serum was collected after centrifugation. The expression level of HBsAg in serum was determined by using HBsAg CLIA kit (Autobio, CL0310) according to the instructions provided by the manufacturer. DNA in serum was extracted according to the instructions of QIAamp 96 DNA Blood Kit, and was subject to quantitative PCR to determine the expression level of HBV DNA.

The inhibition rate against HBsAg is calculated according to the following equation:

$$\text{The inhibition rate against HBsAg} = (1\text{-HBsAg content after administration/HBsAg content before administration}) \times 100\%,$$

wherein the content of HBsAg was expressed as the equivalents (UI) of HBsAg per milliliter (mL) of serum.

The inhibition rate against HBV DNA is calculated according to the following equation:

$$\text{The inhibition rate against HBV DNA} = (1 - \text{the content of HBV DNA after administration/the content of HBV DNA before administration}) \times 100\%,$$

wherein the content of HBV DNA was expressed as the copies of HBV DNA per milliliter (mL) of serum.

Tables 8B and 9B respectively show the *in vivo* inhibition rates against HBsAg and HBV DNA in HBV transgenic mice administered with different doses of each drug conjugate or PBS.

Table 8B Inhibition of the expression of HBsAg by the drug conjugates in mouse serum

| Conjugate Serial No. | Conjugate No. | Inhibition rate against HBsAg in serum (%) | | | | | |
|---|---|---|---|---|---|---|---|
| | | D7 | D14 | D21 | D28 | D56 | D84 |
| Conjugate 43 | N6-siHBb1M1S | 93.1 | 93.2 | 92.6 | 88.3 | 81.4 | 73.4 |
| Conjugate 44 | N6-siHBb2M1S | 93.8 | 94.6 | 93.6 | 90.1 | 82.6 | 79.4 |
| Conjugate 45 | N6-siHBb1M2 | 93.5 | 94.3 | 93.1 | 85.7 | 78.8 | 71.7 |
| Conjugate 46 | N6-siHBb2M2 | 94.4 | 94.9 | 94.2 | 87.6 | 79.2 | 76.1 |
| Conjugate 47 | N6-siHBb1M1SVP | 95.3 | 95.6 | 95.1 | 94.8 | 91.9 | 85.6 |
| Conjugate 48 | N6-siHBb2M1SP | 95.3 | 96.8 | 98.1 | 96.3 | 94.7 | 89.2 |
| Conjugate 49 | N6-siHBb2M1SPs | 95.4 | 95.6 | 95.2 | 94.9 | 89.6 | 82.5 |
| Conjugate 52 | N6-siHBb1M2SVP | 94.5 | 95.8 | 94.9 | 93.6 | 89.2 | 83.5 |
| Conjugate 53 | N6-siHBb2M2SVP | 95.7 | 96.5 | 96.8 | 95.3 | 91.4 | 84.8 |
| Conjugate 57 | N6-siHBb4M1SVP | 94.7 | 95.0 | 94.2 | 93.1 | 90.3 | 84.2 |
| Conjugate 60 | X2-siHBb2M1SVP | 93.2 | 94.0 | 93.4 | 92.9 | 87.5 | 80.1 |
| Conjugate 61 | W2-siHBb2M1SVP | 93.7 | 94.1 | 93.3 | 91.9 | 86.4 | 79.8 |
| Conjugate 62 | V2-siHBb2M1SVP | 93.6 | 94.3 | 93.5 | 91.7 | 86.8 | 80.6 |
| Conjugate 63 | O2-siHBb2M1SVP | 93.8 | 94.8 | 93.1 | 92.1 | 87.1 | 79.8 |
| Conjugate 64 | P2-siHBb2M1SVP | 93.6 | 94.5 | 93.0 | 91.8 | 86.2 | 79.9 |
| - | PBS | 1.8 | -9.3 | -11.5 | -8.9 | -42.1 | -103.5 |

As can be seen from the results of Table 8B, the PBS negative control group showed no inhibitory effect at different time points after administration; in contrast, each drug conjugate showed excellent inhibitory effect on HBsAg at different time points after administration. In particular, the drug conjugates Conjugates 47-53 and 57 consistently showed a high inhibition rate of up to 98.1% against HBsAg in serum over a period of up to 84 days, indicating that they can stably and efficiently inhibit the expression of HBV gene over a longer time period.

Table 9B Inhibition of the expression of HBV DNA by the drug conjugates in mouse serum

| Conjugate Serial No. | Conjugate No. | Inhibition rate against HBV DNA in serum (%) | | | | | |
|---|---|---|---|---|---|---|---|
| | | D7 | D14 | D21 | D28 | D56 | D84 |
| Conjugate 43 | N6-siHBb1M1S | 74.3 | 91.2 | 90.2 | 82.4 | 75.2 | 67.2 |
| Conjugate 44 | N6-siHBb2M1S | 74.2 | 90.8 | 90.4 | 83.5 | 73.2 | 67.3 |
| Conjugate 45 | N6-siHBb1M2 | 75.5 | 91.3 | 90.5 | 79.4 | 72.5 | 63.8 |
| Conjugate 46 | N6-siHBb2M2 | 74.6 | 91.4 | 90.6 | 81.6 | 73.8 | 65.4 |
| Conjugate 47 | N6-siHBb1M1SVP | 76.2 | 92.6 | 90.8 | 85.6 | 78.4 | 70.1 |
| Conjugate 48 | N6-siHBb2M1SP | 76.6 | 92.1 | 90.1 | 86.4 | 82.1 | 75.3 |
| Conjugate 49 | N6-siHBb2M1SPs | 76.0 | 92.3 | 90.3 | 82.5 | 78.6 | 69.1 |
| Conjugate 52 | N6-siHBb1M2SVP | 75.7 | 91.9 | 89.9 | 82.1 | 79.5 | 69.4 |
| Conjugate 53 | N6-siHBb2M2SVP | 76.2 | 93.2 | 90.1 | 82.6 | 79.8 | 70.2 |
| Conjugate 57 | N6-siHBb4M1SVP | 76.4 | 93.5 | 90.3 | 81.8 | 81.2 | 73.1 |
| Conjugate 60 | X2-siHBb2M1SVP | 76.5 | 92.4 | 90.1 | 82.5 | 79.4 | 69.5 |

(continued)

| Conjugate Serial No. | Conjugate No. | Inhibition rate against HBV DNA in serum (%) | | | | | |
|---|---|---|---|---|---|---|---|
| | | D7 | D14 | D21 | D28 | D56 | D84 |
| Conjugate 61 | W2-siHBb2M1SVP | 76.3 | 92.4 | 90.6 | 85.1 | 82.1 | 73.4 |
| Conjugate 62 | V2-siHBb2M1SVP | 76.2 | 91.8 | 90.1 | 85.3 | 81.6 | 73.4 |
| Conjugate 63 | O2-siHBb2M1SVP | 76.1 | 92.1 | 90.2 | 84.2 | 81.7 | 73.9 |
| Conjugate 64 | P2-siHBb2M1SVP | 76.8 | 91.0 | 89.7 | 83.4 | 81.9 | 72.1 |
| - | PBS | 1.7 | -8.5 | -11.4 | -18.4 | -42.1 | -80.5 |

As can be seen from Table 9B, similar to the inhibitory effect on HBsAg, the drug conjugate of each example also showed efficient inhibition of the expression of HBV DNA with an inhibition rate of up to 93.5%, and maintained a substantially stable inhibition rate over a period of up to 84 days.

**Experimental Example 4 Effect experiments of the drug conjugates in Table 2C**

**Experimental Example 4-1 Determination of the inhibitory efficiency of the drug conjugates of the present disclosure against the expression level of HBV mRNA in HepG2.2.15 cells**

The HepG2.2.15 cells were seeded into a 24-well plate at $7 \times 10^4$ cells/well with H-DMEM complete medium. After 16 hours, when the cell growth density reached 70-80%, the H-DMEM complete medium in the culture well was aspirated, and 500 μL Opti-MEM medium (GIBCO company) was added to each well, and the cells were cultured for another 1.5 hours.

Each drug conjugate of the drug conjugates below was respectively formulated into working solutions of drug conjugate at 3 different concentrations of 50 μM, 5 μM and 0.5 μM with DEPC water, wherein the drug conjugates used are respectively those listed in Table 4C.

For each siRNA, 4A1, 4A2 and 4A3 solutions were formulated, respectively. Each of 4A1-4A3 solutions contained 0.6 μL of the above siRNA working solution at one of the above 3 concentrations and 50 μL of Opti-MEM medium.

FormLaution of 4B solution: each 4B solution contained 1 μL of Lipofectamine™ 2000 and 50 μL of Opti-MEM medium.

One portion of 4B solution was respectively mixed with one portion of the resultant 4A1, 4A2 or 4A3 solution of each siRNA, and incubated at room temperature for 20 minutes respectively to obtain transfection complexes 4X1, 4X2 and 4X3 of each siRNA.

One portion of 4B solution was mixed with 50 μL of Opti-MEM medium and incubated for 20 minutes at room temperature to obtain a transfection complex 4X4.

The transfection complex 4X1, 4X2 or 4X3 of each drug conjugate was respectively added to the culture wells in an amount of 100 μL/well and mixed well to obtain a transfection mixture with the final concentration of each drug conjugate of about 50 nM, 5 nM or 0.5 nM, respectively. Each transfection complex was respectively used to transfect three culture wells to obtain drug conjugate-containing transfection mixtures (designated as test group).

The transfection complex 4X4 was respectively added to another three culture wells in an amount of 100 μL/well to obtain drug conjugate-free transfection mixtures (designated as control group).

After the drug conjugate-containing transfection mixtures and the drug conjugate-free transfection mixture were cultured in the culture wells for 4 hours, each well was supplemented with 1 mL of H-DMEM complete medium containing 20% FBS. The 24-well plate was placed in a $CO_2$ incubator and cultured for another 24 hours.

Subsequently, the total RNA of the cells in each well was extracted by RNeasy Mini Kit (QIAGEN company, Cat No. 74106) according to the detailed preoceudre as described in the instruction.

For the cells of each well, 1 μg of the total RNA was taken, and was formulated into a 20 μL reverse transcription reaction system for reverse transcription of the total RNA of the cell by using the reagent provided in the reverse transcription kit GoldenstarTM RT6 cDNA Synthesis Kit (purchased from Beijing Tsingke Biotechnology Co., Ltd., Cat. No. TSK301M) according to the precedures for reverse transcription in the instruction of the kit, in which GoldenstarTM Oligo (dT)17 was selected as the primer. Conditions for reverse transcription were as follows: the reverse transcription reaction system was incubated at 50°C for 50 minutes, then incubated at 85°C for 5 minutes, and finally incubated at 4°C for 30 seconds; after the reaction was completed, 80 μL of DEPC water was added to the reverse transcription reaction system to obtain a cDNA-containing solution.

For each reverse transcription reaction system, 5 μL of the above cDNA-containing solution was taken as the

template, and was formulated into a 20 μL qPCR reaction system by using the reagent provided in the NovoStart® SYBR qPCR SuperMix Plus kit (purchased from Novoprotein Scientific Co., Ltd., Cat No. E096-01B), wherein the sequences of PCR primers for amplifying the target gene HBV and the internal control gene GAPDH are shown in Table 3B, and the final concentration of each primer was 0.25 μM. Each qPCR reaction system was placed in an ABI StepOnePlus Real-Time PCR Thermal Cycler, and the amplification was performed by a three-step method using the following amplification procedure: pre-denaturation at 95°C for 10 minutes, then denaturation at 95°C for 30 s, annealing at 60°C for 30 s, and extension at 72°C for 30 s (wherein the above process of denaturation, annealing and extension was repeated for 40 times), to obtain a product W1 containing the amplified target gene HBV and the amplified internal control gene GAPDH. The product W1 was then sequentially incubated at 95°C for 15 s, 60°C for 1 min, and 95°C for 15 s. The melting curves of the target gene HBV and the internal control gene GAPDH in the product W1 were respectively collected using a real-time fluorescent quantitative PCR Thermal Cycler, and the Ct values of the target gene HBV and the internal control gene GAPDH were obtained.

The relative quantitative calculation of the expression level of the target HBV gene in each test group and the control group was carried out using the comparative Ct (ΔΔCt) method, and the calculation method was as follows:

$$\Delta Ct\text{ (test group)} = Ct\text{ (target gene of test group)} - Ct\text{ (internal control gene of test group)}$$

$$\Delta Ct\text{ (control group)} = Ct\text{ (target gene of control group)} - Ct\text{ (internal control gene of control group)}$$

$$\Delta\Delta Ct\text{ (test group)} = \Delta Ct\text{ (test group)} - \Delta Ct\text{ (average of control group)}$$

$$\Delta\Delta Ct\text{ (control group)} = \Delta Ct\text{ (control group)} - \Delta Ct\text{ (average of control group)}$$

wherein ΔCt (average of control group) was the arithmetic mean of the ΔCt (control group) of the three culture wells of the control group. Thus, each culture well in the test and control groups corresponded to a ΔΔCt value.

The expression level of HBV mRNA in the test group was normalized based on the control group, and the expression level of HBV mRNA in the control group was defined as 100%,

$$\text{Relative expression level of HBV mRNA in the test group} = 2^{-\Delta\Delta Ct\text{ (the test group)}} \times 100\%.$$

For the siRNA in the same test group, the mean of the relative expression level of HBV mRNA in the test group at each concentration was the arithmetic mean of the relative expression levels of the three culture wells at that concentration.

Therein, each test group was HepG2.2.15 cells respectively treated with the drug conjugates listed in Table 2C, and the drug conjugates included Conjugates 65-81 and Comparative Conjugate 1.

Table 4C below shows the determination results of the inhibitory activities of the drug conjugates listed in Table 2C and Comparative Conjugate 1 against the expression of HBV mRNA in HepG2.2.15 cells.

Table 4C *In vitro* inhibition rates of the drug conjugates at different concentrations against HBV mRNA

| Conjugate Serial No. | Conjugate No. | Inhibition rate against mRNA (%) | | |
|---|---|---|---|---|
| | | 50 nM | 10 nM | 1 nM |
| Conjugate 65 | N6-siHBc1 | 53.5 | 39.2 | 22.3 |
| Conjugate 66 | N6-siHBc1M1 | 53.3 | 38.1 | 27.5 |
| Conjugate 67 | N6-siHBc1M2 | 53.8 | 36.2 | 24.6 |
| Conjugate 68 | N6-siHBc1M1SVP | 53.1 | 37.6 | 25.8 |
| Conjugate 69 | N6-siHBc1M2SVP | 54.2 | 47.1 | 27.1 |
| Conjugate 70 | N6-siHBc1M3SVP | 54.6 | 26.5 | 18.3 |

(continued)

| Conjugate Serial No. | Conjugate No. | Inhibition rate against mRNA (%) | | |
|---|---|---|---|---|
| | | 50 nM | 10 nM | 1 nM |
| Conjugate 71 | N6-siHBc1M4SVP | 55.2 | 25.8 | 18.0 |
| Conjugate 72 | N6-siHBc1M5SVP | 54.3 | 42.1 | 26.2 |
| Conjugate 73 | N6-siHBc2M1SVP | 53.6 | 43.2 | 27.1 |
| Conjugate 74 | N6-siHBc1M1SP | 52.8 | 44.1 | 28.0 |
| Conjugate 75 | N6-siHBc1M1SPs | 53.3 | 44.6 | 26.6 |
| Conjugate 76 | N6-siHBc4M1S | 52.9 | 44.2 | 26.9 |
| Conjugate 77 | X2-siHBc1M1SVP | 55.2 | 42.6 | 27.3 |
| Conjugate 78 | W2-siHBc1M1SVP | 54.9 | 41.2 | 28.3 |
| Conjugate 79 | V2-siHBc1M1SVP | 54.9 | 41.6 | 26.4 |
| Conjugate 80 | O2-siHBc1M1SVP | 54.1 | 45.2 | 25.9 |
| Conjugate 81 | P2-siHBc1M1SVP | 53.8 | 46.3 | 28.1 |
| Comparative Conjugate 1 | N6-NC | 2.0 | 1.7 | -2.6 |

As can be seen from the results of Table 4C, the drug conjugates of the present disclosure exhibited very high inhibitory activity against HBV mRNA in HepG2.2.15 cells *in vitro,* and could show an inhibition rate of up to 55.2% against HBV mRNA at the siRNA concentration of 50 nM.

**Experimental Example 4-2 The stability of the drug conjugates of the present disclosure in human plasma *in vitro***

Each drug conjugate of the drug conjugates (provided as 20 μM in 0.9 wt% NaCl aqueous solution (calculated based on siRNA), 12 μL per group) was respectively mixed well with 108 μL of 90% human plasma (purchased from Jiangsu Hematology Institute and diluted with 1×PBS (pH 7.4)) to obtain mixed solutions, wherein the drug conjugates used are respectively those listed in Table 5C. The mixed solution was incubated at a constant temperature of 37°C. 10 μL of the sample was taken at 0 h, 8 h, 24 h and 48 h, respectively, and immediately frozen in liquid nitrogen and cryopreserved in a -80°C freezer. Therein, 0 hour refers to the time when 10 μL of the mixed solution was taken immediately after the conjugate solution was mixed well with 90% human plasma. After sampling at each time point, each mixed solution was diluted 5-fold with 1×PBS (pH 7.4) and then taken in a volume of 10 μL for electrophoresis. An equimolar amount of each of the above conjugate solutions (2 μL, the concentration is 2 μM calculated based on siRNA) was mixed well with 8 μL of 1× PBS (pH 7.4) respectively to prepare 10 μL of sample untreated with human plasma (designated as "untreated") for electrophoresis.

20 wt% of non-denatured polyacrylamide gel was prepared. The above samples for electrophoresis in each group were respectively mixed with 4 μL of loading buffer (20 mM EDTA, 36 wt% glycerol, and 0.06 wt% bromophenol blue) and then loaded into each gel hole for electrophoresis under 80 mA constant current for about 60 minutes. After electrophoresis, the gel was taken out, stained with 1×Sybr Gold dye (Invitrogen, Cat.11494) for 15 minutes for imaging. The stability results were calculated and the results are shown in Table 5C.

Table 5C shows the semiquantitative test result of the stability of the drug conjugates listed in Table 2C in human plasma *in vitro.* The result is expressed as the ratio (RL) of the longest fragment remaining after the incubation of the drug conjugate with human plasma to the longest fragment of untreated siRNA.

Table 5C The plasma stability of different drug conjugates over time

| Conjugate Serial No. | Conjugate No. | RL (%) | | | | |
|---|---|---|---|---|---|---|
| | | Untreated | 0h | 8h | 24h | 48h |
| Conjugate 66 | N6-siHBc1M1 | 100 | 100 | 99.5 | 96.2 | 94.2 |
| Conjugate 67 | N6-siHBc1M2 | 100 | 100 | 99.5 | 96.4 | 94.1 |
| Conjugate 68 | N6-siHBc1M1SVP | 100 | 100 | 99.2 | 96.0 | 94.0 |

(continued)

| Conjugate Serial No. | Conjugate No. | RL (%) | | | | |
|---|---|---|---|---|---|---|
| | | Untreated | 0h | 8h | 24h | 48h |
| Conjugate 69 | N6-siHBc1M2SVP | 100 | 100 | 99.1 | 96.2 | 95.0 |
| Conjugate 70 | N6-siHBc1M3SVP | 100 | 100 | 99.5 | 96.7 | 95.2 |
| Conjugate 71 | N6-siHBc1M4SVP | 100 | 100 | 99.4 | 96.0 | 94.8 |
| Conjugate 72 | N6-siHBc1M5SVP | 100 | 100 | 99.2 | 95.8 | 95.1 |
| Conjugate 73 | N6-siHBc2M1SVP | 100 | 100 | 99.0 | 96.3 | 94.5 |
| Conjugate 74 | N6-siHBc1M1SP | 100 | 100 | 99.6 | 96.2 | 95.2 |
| Conjugate 75 | N6-siHBc1M1SPs | 100 | 100 | 99.5 | 96.1 | 94.6 |
| Conjugate 76 | N6-siHBc4M1S | 100 | 100 | 99.1 | 95.4 | 95.1 |
| Conjugate 77 | X2-siHBc1M1SVP | 100 | 100 | 99.5 | 95.3 | 94.2 |
| Conjugate 78 | W2-siHBc1M1SVP | 100 | 100 | 99.4 | 96.0 | 94.1 |
| Conjugate 79 | V2-siHBc1M1SVP | 100 | 100 | 99.1 | 96.1 | 94.2 |
| Conjugate 80 | O2-siHBc1M1SVP | 100 | 100 | 99.2 | 95.1 | 95.0 |
| Conjugate 81 | P2-siHBc1M1SVP | 100 | 100 | 99.5 | 95.2 | 94.5 |

As can be seen from the results of Table 5C, all the drug conjugates of the present disclosure exhibited excellent stability in plasma, and still showed a length ratio of the siRNA fragments of higher than 94% at 48 h.

**Experimental example 4-3 Determination of the inhibitory efficiency of the drug conjugates of the present disclosure against the expression of HBV mRNA *in vivo***

The HBV transgenic mice C57BL/6J-Tg (Alb1HBV) 44Bri/J used in this experimental example were purchased from the Department of Laboratory Animal Science, Peking University Health Science Center. C57BL/6J-Tg(Alb1HBV)44Bri/J mice were randomly divided into groups (all female, five mice per group) according to serum HBsAg content, and each group of mice was numbered according to the drug conjugates in Table 2C. Then, each group of mice was respectively administered with each of the test conjugates in Table 7C. All animals were dosed based on body weight and was administered at a single dose by subcutaneous injection. Each conjugate was administered in the form of 0.2 mg/mL in 0.9% aqueous sodium chloride solution, with an administration volume of 5 mL/kg mouse body weight; that is, the administration doses of each conjugate were 1 mg/kg.

Each mouse in another group was administered with 1 $\times$PBS with an administration volume of 5 mL/kg mouse body weight, as a control group.

On day 14 after administrion, the animals were sacrificed, and the liver tissue of each mouse was collected separately and stored in RNA later (Sigma Aldrich Crop.). The liver tissue was homogenized with a tissue homogenizer and then extracted with Trizol (Thermo Fisher company) according to the operation procedures described in the instruction to obtain the total RNA.

For the liver tissue of each mouse, 1 $\mu$g of total RNA was taken, and the extracted total RNA was reverse-transcribed into cDNA using ImProm-II™ reverse transcription kit (Promega Crop.) according to the instructions thereof to obtain a solution containing cDNA, and then the expression level of HBV mRNA in liver tissue was determined by the fluorescent quantitative PCR kit (Beijing Cowin Biosciences Co., Ltd.). In this fluorescent quantitative PCR method, β-actin gene was used as an internal control gene, and HBV and β-actin were detected by using primers for HBV and β-actin, respectively. The sequences of the primers for detection are shown in Table 6B.

The relative quantitative calculation of the expression level of the target HBV gene in each test group and the control group was carried out using the comparative Ct (ΔΔCt) method, and the calculation method was as follows:

$$\Delta Ct\ (\text{test group}) = Ct\ (\text{target gene of test group}) - Ct\ (\text{internal control gene of test group})$$

$$\Delta Ct\ (\text{control group}) = Ct\ (\text{target gene of control group}) - Ct\ (\text{internal control gene of control group})$$

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

wherein ΔCt (average of control group) was the arithmetic mean of the ΔCt (control group) of the five mice of the control group. Thus, each mouse in the test and control groups corresponded to a ΔΔCt value.

The expression level of HBV mRNA in the test group was normalized based on the control group, and the expression level of HBV mRNA in the control group was defined as 100%,

Relative expression level of HBV mRNA in the test group = $2^{-\Delta\Delta Ct\ (\text{the test group})} \times 100\%$.

For the siRNA in the same test group, the mean of the relative expression level of HBV mRNA in the test group at each concentration was the arithmetic mean of the relative expression levels in the five mice at that concentration.

Therein, the control group refers to the mice in the control group administered with PBS in this experiment, and each test group refers to the mice in the administration group adminstered with different drug conjugates. The results are shown in Table 7C below.

Table 7C Inhibition rates of different drug conjugates against the expression of HBV mRNA in mouse liver

| Conjugate Serial No. | Conjugate No. | Administration dose (mg/kg) | Inhibition rate against HBV mRNA in liver (%) |
|---|---|---|---|
| Conjugate 66 | N6-siHBc1M1 | 1 | 78.2 |
| Conjugate 67 | N6-siHBc1M2 | 1 | 82.3 |
| Conjugate 68 | N6-siHBc1M1SVP | 1 | 79.6 |
| Conjugate 69 | N6-siHBc1M2SVP | 1 | 82.1 |
| Conjugate 70 | N6-siHBc1M3SVP | 1 | 58.2 |
| Conjugate 71 | N6-siHBc1M4SVP | 1 | 50.4 |
| Conjugate 72 | N6-siHBc1M5SVP | 1 | 81.3 |
| Conjugate 73 | N6-siHBc2M1SVP | 1 | 77.9 |
| Conjugate 74 | N6-siHBc1M1SP | 1 | 82.1 |
| Conjugate 75 | N6-siHBc1M1SPs | 1 | 83.5 |
| Conjugate 76 | N6-siHBc4M1S | 1 | 76.2 |
| Conjugate 77 | X2-siHBc1M1SVP | 1 | 83.2 |
| Conjugate 78 | W2-siHBc1M1SVP | 1 | 84.2 |
| Conjugate 79 | V2-siHBc1M1SVP | 1 | 83.6 |
| Conjugate 80 | 02-siHBc1M1SVP | 1 | 82.9 |
| Conjugate 81 | P2-siHBc1M1SVP | 1 | 84.6 |
| PBS | - | - | NA |

As can be seen from the results in Table 7C, the drug conjugates of the present disclosure showed very high *in vivo* inhibitory activity against liver HBV mRNA in the C57BL/6J-Tg(Alb1HBV)44Bri/J mouse at an administration dose of 1 mg/kg of siRNA, and exhibited an inhibition rate of up to 84.6% against HBV mRNA at a dose of 1 mg/kg.

**Experimental example 4-4 The time correlation test of the inhibitory efficiency of the siRNAs in the drug conjugates provided in this experiment against the expression level of HBsAg and HBV DNA in the serum of HBV transgenic mice**

AAV-HBV models were prepared according to the method in the literature (DONG Xiaoyan et al., Chin J Biotech 2010, May 25; 26(5): 679-686), rAAV8-1.3HBV, type D (ayw) virus (purchased from Beijing FivePlus Molecular Medicine Institute Co. Ltd., $1\times10^{12}$ viral genome (v.g.)/mL, Lot No. 2016123011). The AAV virus was diluted to $5\times10^{11}$ v.g./mL with sterilized PBS prior to the experiment. Each mouse was injected with 200 μL (that is, each mouse was injected with $1\times10^{11}$ v.g). On day 28 post virus injection, orbital blood (about 100 μL) was collected from all mice for collecting serum for dectection of HBsAg and HBV DNA.

After the animals were successfully modeled, they were randomly divided into groups according to serum HBsAg content (five mice per group) and respectively numbered. Each group of mice was administered with each of the test conjugates in Table 8C, respectively. All animals were dosed based on body weight and was administered at a single dose by subcutaneous injection. Each conjugate was administered in the form of 0.6 mg/mL in 0.9 % NaCl aqueous solution, with an administration volume of 5 mL/kg mouse body weight; that is, the administration doses of each conjugate was 3 mg/kg. Each mouse in another group was administered with 1×PBS with an administration volume of 5 mL/kg mouse body weight, as a control group. The blood was collected from mouse orbital venous plexus before administration and on day 7, 14 and 21 after administration, and the serum HBsAg level was determined at each time point.

About 100 μL orbital blood was taken each time, and no less than 20 μL serum was collected after centrifugation. The expression level of HBsAg in serum was determined by using HBsAg CLIA kit (Autobio, CL0310) according to the instructions provided in the kit. DNA in serum was extracted according to the instructions of QIAamp 96 DNA Blood Kit, and was subject to quantitative PCR to determine the expression level of HBV DNA.

The inhibition rate against HBsAg is calculated according to the following equation:

$$\text{The inhibition rate against HBsAg} = (1\text{-HBsAg content after administration/HBsAg content before administration}) \times 100\%,$$

wherein the content of HBsAg was expressed as the equivalents (UI) of HBsAg per milliliter (mL) of serum.

The inhibition rate against HBV DNA is calculated according to the following equation:

$$\text{The inhibition rate against HBV DNA} = (1 - \text{the content of HBV DNA after administration/the content of HBV DNA before administration}) \times 100\%,$$

wherein the content of HBV DNA was expressed as the copies of HBV DNA per milliliter (mL) of serum.

Tables 8C and 9C respectively show the *in vivo* inhibition rates against HBsAg and HBV DNA in HBV transgenic mice administered with different doses of each drug conjugate or PBS.

Table 8C Inhibition rates of different drug conjugates against HBsAg in mouse serum

| Conjugate Serial No. | Conjugate No. | Inhibition rate against HBsAg in serum (%) | | |
|---|---|---|---|---|
| | | D7 | D14 | D21 |
| Conjugate 68 | N6-siHBc1M1SVP | 81.2 | 82.3 | 74.8 |
| Conjugate 69 | N6-siHBc1M2SVP | 81.5 | 82.5 | 73.8 |
| Conjugate 73 | N6-siHBc2M1SVP | 80.1 | 82.1 | 74.6 |
| Conjugate 74 | N6-siHBc1M1SP | 79.8 | 81.8 | 74.1 |
| Conjugate 75 | N6-siHBc1M1SPs | 78.2 | 81.6 | 73.5 |
| Conjugate 77 | X2-siHBc1M1SVP | 80.3 | 81.3 | 72.0 |
| Conjugate 78 | W2-siHBc1M1SVP | 81.6 | 81.2 | 71.5 |
| Conjugate 79 | V2-siHBc1M1SVP | 81.0 | 80.1 | 71.6 |
| Conjugate 80 | 02-siHBc1M1SVP | 81.3 | 79.2 | 71.4 |

(continued)

| Conjugate Serial No. | Conjugate No. | Inhibition rate against HBsAg in serum (%) | | |
|---|---|---|---|---|
| | | D7 | D14 | D21 |
| Conjugate 81 | P2-siHBc1M1SVP | 81.2 | 81.3 | 71.5 |
| - | PBS | 2.2 | -19.3 | -31.8 |

As can be seen from the results of Table 8C, the PBS negative control group showed no inhibitory effect at different time points after administration; in contrast, the drug conjugate of the present disclosure showed excellent inhibitory effect on HBsAg at different time points after administration, and in particular consistently showed a high inhibition rate of up to 81.8% against HBsAg in serum over a period of 21 days, indicating that it can stably and efficiently inhibit the expression of HBV mRNA over a longer time period.

Table 9C Inhibition of HBV DNA by different drug conjugates in mouse serum

| Conjugate Serial No. | Conjugate No. | Inhibition rate against HBV DNA in serum (%) | | |
|---|---|---|---|---|
| | | D7 | D14 | D21 |
| Conjugate 68 | N6-siHBc1M1SVP | 75.2 | 82.3 | 71.3 |
| Conjugate 69 | N6-siHBc1M2SVP | 74.3 | 82.4 | 70.2 |
| Conjugate 73 | N6-siHBc2M1SVP | 76.1 | 81.9 | 73.2 |
| Conjugate 74 | N6-siHBc1M1SP | 75.3 | 82.6 | 71.2 |
| Conjugate 75 | N6-siHBc1M1SPs | 74.2 | 83.2 | 70.8 |
| Conjugate 77 | X2-siHBc1M1SVP | 75.3 | 82.7 | 73.2 |
| Conjugate 78 | W2-siHBc1M1SVP | 76.4 | 82.1 | 72.4 |
| Conjugate 79 | V2-siHBc1M1SVP | 75.1 | 80.9 | 72.1 |
| Conjugate 80 | O2-siHBc1M1SVP | 74.6 | 83.9 | 71.9 |
| Conjugate 81 | P2-siHBc1M1SVP | 75.0 | 82.1 | 71.6 |
| - | PBS | 1.2 | 3.5 | -3.8 |

As can be seen from the results in Table 9C, the drug conjugates of the present disclosure also showed excellent inhibition effect on HBV DNA, and maintained a higher inhibition rate of up to 83.9% over a period of 21 days.

**Experimental Example 5 Effect experiments of the drug conjugates of the present disclosure**

**Experimental Example 5-1 Determination of the inhibitory efficiency of the drug conjugates of the present disclosure against the expression level of HBV mRNA in HepG2.2.15 cells**

The HepG2.2.15 cells were seeded into a 24-well plate at 7 $\times$ $10^4$ cells/well with H-DMEM complete medium. After 16 hours, when the cell growth density reached 70-80%, the H-DMEM complete medium in the culture well was aspirated, and 500 μL Opti-MEM medium (GIBCO company) was added to each well, and the cells were cultured for another 1.5 hours.

Each drug conjugate of the drug conjugates below was respectively formulated into working solutions of drug conjugate at 3 different concentrations of 50 μM, 5 μM and 0.5 μM with DEPC water, wherein the drug conjugates used are respectively the test conjugates in Table 4D.

For each siRNA, 5A1, 5A2 and 5A3 solutions were formulated, respectively. Each of 5A1-5A3 solutions contained 0.6 μL of the above siRNA working solution at one of the above 3 concentrations and 50 μL of Opti-MEM medium.

FormLaution of 5B solution: each 5B solution contained 1 μL of Lipofectamine™ 2000 and 50 μL of Opti-MEM medium.

One portion of 5B solution was respectively mixed with one portion of the resultant 5A1, 5A2 or 5A3 solution of each siRNA, and incubated at room temperature for 20 minutes respectively to obtain transfection complexes 5X1, 5X2 and 5X3 of each siRNA.

One portion of 5B solution was mixed with 50 μL of Opti-MEM medium and incubated for 20 minutes at room temperature to obtain a transfection complex 5X4.

The transfection complexes 5X1-5X3 of each drug conjugate was respectively added to the culture wells in an amount of 100 μL/well and mixed well to obtain a transfection complexes with the final concentration of each drug conjugate of about 50 nM, 5 nM or 0.5 nM, respectively. Each transfection complex was respectively used to transfect three culture wells to obtain drug conjugate-containing transfection mixtures (designated as test group).

The transfection complex 5X4 was respectively added to another three culture wells in an amount of 100 μL/well to obtain a siRNA-free transfection mixture (designated as control group).

After the drug conjugate-containing transfection mixtures and the drug conjugate-free transfection mixture were cultured in the culture wells for 4 hours, each well was supplemented with 1 mL of H-DMEM complete medium containing 20% FBS. The 24-well plate was placed in a $CO_2$ incubator and cultured for another 24 hours.

Subsequently, the total RNA of the cells in each well was extracted by RNeasy Mini Kit (QIAGEN company, Cat No. 74106) according to the detailed preoceudre as described in the instruction.

For the cells of each well, 1 μg of the total RNA was taken, and was formulated into a 20 μL reverse transcription reaction system for reverse transcription of the total RNA of the cell by using the reagent provided in the reverse transcription kit GoldenstarTM RT6 cDNA Synthesis Kit (purchased from Beijing Tsingke Biotechnology Co., Ltd., Cat. No. TSK301M) according to the precedures for reverse transcription in the instruction of the kit, in which GoldenstarTM Oligo (dT)17 was selected as the primer. Conditions for reverse transcription were as follows: the reverse transcription reaction system was incubated at 50°C for 50 minutes, then incubated at 85°C for 5 minutes, and finally incubated at 4°C for 30 seconds; after the reaction was completed, 80 μL of DEPC water was added to the reverse transcription reaction system to obtain a cDNA-containing solution.

For each reverse transcription reaction system, 5 μL of the above cDNA-containing solution was taken as the template, and was formulated into a 20 μL qPCR reaction system by using the reagent provided in the NovoStart® SYBR qPCR SuperMix Plus kit (purchased from Novoprotein Scientific Co., Ltd., Cat No. E096-01B), wherein the sequences of PCR primers for amplifying the target gene HBV and the internal control gene GAPDH are shown in Table 3B, and the final concentration of each primer was 0.25 μM. Each qPCR reaction system was placed in an ABI StepOnePlus Real-Time PCR Thermal Cycler, and the amplification was performed by a three-step method using the following amplification procedure: pre-denaturation at 95°C for 10 minutes, then denaturation at 95°C for 30 s, annealing at 60°C for 30 s, and extension at 72°C for 30 s (wherein the above process of denaturation, annealing and extension was repeated for 40 times), to obtain a product W2 containing the amplified target gene HBV and the amplified internal control gene GAPDH. The product W2 was then sequentially incubated at 95°C for 15 s, 60°C for 1 min, and 95°C for 15 s. The melting curves of the target gene HBV and the internal control gene GAPDH in the product W2 were respectively collected using a real-time fluorescent quantitative PCR Thermal Cycler, and the Ct values of the target gene HBV and the internal control gene GAPDH were obtained.

The relative quantitative calculation of the expression level of the target HBV gene in each test group and the control group was carried out using the comparative Ct (ΔΔCt) method, and the calculation method was as follows:

$$\Delta Ct\ (\text{test group}) = Ct\ (\text{target gene of test group}) - Ct\ (\text{internal control gene of test group})$$

$$\Delta Ct\ (\text{control group}) = Ct\ (\text{target gene of control group}) - Ct\ (\text{internal control gene of control group})$$

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

wherein ΔCt (average of control group) was the arithmetic mean of the ΔCt (control group) of the three culture wells of the control group. Thus, each culture well in the test and control groups corresponded to a ΔΔCt value.

The expression level of HBV mRNA in the test group was normalized based on the control group, and the expression level of HBV mRNA in the control group was defined as 100%,

Relative expression level of HBV mRNA in the test group = $2^{-\Delta\Delta Ct\ (\text{the test group})} \times 100\%$.

For the siRNA in the same test group, the mean of the relative expression level of HBV mRNA in the test group at each concentration was the arithmetic mean of the relative expression levels of the three culture wells at that concentration.

Therein, each test group was HepG2.2.15 cells respectively treated with the drug conjugates listed in Table 2D, and the drug conjugates included Conjugates 82-98 and Comparative Conjugate 1.

Table 4D below shows the determination results of the inhibitory activities of the drug conjugates listed in Table 2D and the comparative conjugate against the expression of HBV mRNA in HepG2.2.15 cells.

Table 4D *In vitro* inhibition rates of the drug conjugates at different concentrations against HBV mRNA

| Conjugate Serial No. | Conjugate No. | Inhibition rate against HBV mRNA (%) | | |
|---|---|---|---|---|
| | | 50 nM | 10 nM | 1 nM |
| Conjugate 82 | N6-siHBd1 | 53.2 | 39.6 | 22.6 |
| Conjugate 83 | N6-siHBd1M1 | 52.8 | 38.8 | 26.8 |
| Conjugate 84 | N6-siHBd1M2 | 52.7 | 36.5 | 23.9 |
| Conjugate 85 | N6-siHBdlM1SVP | 52.7 | 36.9 | 24.9 |
| Conjugate 86 | N6-siHBd1M2SVP | 53.8 | 45.2 | 26.5 |
| Conjugate 87 | N6-siHBd1M3SVP | 53.9 | 24.5 | 17.8 |
| Conjugate 88 | N6-siHBd1M4SVP | 53.9 | 23.6 | 17.5 |
| Conjugate 89 | N6-siHBd1M5SVP | 52.7 | 41.6 | 25.9 |
| Conjugate 90 | N6-siHBd2M1SVP | 51.9 | 43.1 | 26.5 |
| Conjugate 91 | N6-siHBd1M1SP | 52.6 | 43.5 | 27.6 |
| Conjugate 92 | N6-siHBd1M1SPs | 53.1 | 43.8 | 26.9 |
| Conjugate 93 | N6-siHBd4M1S | 52.1 | 53.2 | 25.8 |
| Conjugate 94 | X2-siHBd1M1SVP | 53.8 | 41.8 | 26.7 |
| Conjugate 95 | W2-siHBd1M1SVP | 53.2 | 41.6 | 27.4 |
| Conjugate 96 | V2-siHBd1M1SVP | 52.9 | 41.2 | 25.9 |
| Conjugate 97 | O2-siHBd1M1SVP | 52.4 | 44.2 | 25.7 |
| Conjugate 98 | P2-siHBd1M1SVP | 52.9 | 45.3 | 27.5 |
| Comparative Conjugate 1 | N6-NC | 1.3 | 1.3 | -2.3 |

As can be seen from the results of Table 4D, the drug conjugates of the present disclosure exhibited very high inhibitory activity against HBV mRNA in HepG2.2.15 cells *in vitro,* and could show an inhibition rate of 53.9% against HBV mRNA at the siRNA concentration of 50 nM.

**Experimental Example 5-2 The stability of the drug conjugates of the present disclosure in human plasma *in vitro***

Each drug conjugate of the drug conjugates (provided as 20 μM in 0.9 wt% NaCl aqueous solution (calculated based on siRNA), 12 μL per group) was respectively mixed well with 108 μL of 90% human plasma (purchased from Jiangsu Hematology Institute and diluted with 1×PBS (pH 7.4)) to obtain mixed solutions, wherein the drug conjugates used are respectively those listed in Table 5D. The mixed solution was incubated at a constant temperature of 37°C. 10 μL of the sample was taken at 0 h, 8 h, 24 h and 48 h, respectively, and immediately frozen in liquid nitrogen and cryopreserved in a -80°C freezer. Therein, 0 hour refers to the time when 10 μL of the mixed solution was taken immediately after the conjugate solution was mixed well with 90% human plasma. After sampling at each time point, each mixed solution was diluted 5-fold with 1×PBS (pH 7.4) and then taken in a volume of 10 μL for electrophoresis. An equimolar amount of each of the above conjugate solutions (2 μL, the concentration is 2 μM calculated based on siRNA) was mixed well with 8 μL of 1× PBS (pH 7.4) respectively to prepare 10 μL of sample untreated with human plasma (designated as "untreated") for electrophoresis.

20 wt% of non-denatured polyacrylamide gel was prepared. The above samples for electrophoresis in each group were respectively mixed with 4 μL of loading buffer (20 mM EDTA, 36 wt% glycerol, and 0.06 wt% bromophenol blue) and then loaded into each gel hole for electrophoresis under 80 mA constant current for about 60 minutes. After

electrophoresis, the gel was taken out, stained with 1×Sybr Gold dye (Invitrogen, Cat.11494) for 15 minutes for imaging. The stability results were calculated and the results are shown in Table 5D.

Table 5D shows the semiquantitative test result of the stability of the drug conjugates listed in Table 2D in human plasma *in vitro.* The result is expressed as the ratio (RL) of the longest fragment remaining after the incubation of the drug conjugate with human plasma to the longest fragment of untreated siRNA.

Table 5D The plasma stability of different drug conjugates over time

| Conjugate Serial No. | Conjugate No. | RL (%) | | | | |
|---|---|---|---|---|---|---|
| | | Untreated | 0h | 8h | 24h | 48h |
| Conjugate 83 | N6-siHBd1M1 | 100 | 100 | 99.2 | 96.3 | 94.2 |
| Conjugate 84 | N6-siHBd1M2 | 100 | 100 | 99.8 | 96.0 | 94.1 |
| Conjugate 85 | N6-siHBdIM1SVP | 100 | 100 | 99.5 | 96.0 | 94.0 |
| Conjugate 86 | N6-siHBd1M2SVP | 100 | 100 | 99.1 | 96.2 | 95.0 |
| Conjugate 87 | N6-siHBd1M3SVP | 100 | 100 | 99.3 | 96.1 | 95.2 |
| Conjugate 88 | N6-siHBd1M4SVP | 100 | 100 | 99.6 | 96.0 | 95.0 |
| Conjugate 89 | N6-siHBd1M5SVP | 100 | 100 | 99.2 | 96.0 | 95.1 |
| Conjugate 90 | N6-siHBd2M1SVP | 100 | 100 | 99.1 | 96.3 | 94.5 |
| Conjugate 91 | N6-siHBd1M1SP | 100 | 100 | 99.5 | 96.5 | 95.2 |
| Conjugate 92 | N6-siHBd1M1SPs | 100 | 100 | 99.4 | 97.3 | 94.5 |
| Conjugate 93 | N6-siHBd4M1S | 100 | 100 | 99.2 | 96.2 | 94.1 |
| Conjugate 94 | X2-siHBd1M1SVP | 100 | 100 | 99.1 | 95.1 | 94.0 |
| Conjugate 95 | W2-siHBd1M1SVP | 100 | 100 | 99.5 | 96.0 | 94.3 |
| Conjugate 96 | V2-siHBd1M1SVP | 100 | 100 | 99.6 | 97.0 | 94.2 |
| Conjugate 97 | O2-siHBd1M1SVP | 100 | 100 | 99.4 | 96.8 | 94.0 |
| Conjugate 98 | P2-siHBd1M1SVP | 100 | 100 | 99.2 | 96.1 | 94.1 |

As can be seen from the results of Table 5D, all the drug conjugates of the present disclosure exhibited excellent stability in plasma, and still showed a length ratio of the siRNA fragments of higher than 94% at 48 h.

**Experimental example 5-3 Determination of the inhibitory efficiency of the drug conjugates of the present disclosure against the expression of HBV mRNA in the HBV transgenic mice C57BL/6J-Tg(Alb1HBV)44Bri/J**

The HBV transgenic mice C57BL/6J-Tg (Alb1HBV) 44Bri/J used in this experimental example were purchased from the Department of Laboratory Animal Science, Peking University Health Science Center.

First, C57BL/6J-Tg(Alb1HBV)44Bri/J mice were randomly divided into groups (all female, five mice per group) according to serum HBsAg content, and each group of mice was numbered according to the drug conjugates in Table 2D. Then, each group of mice was respectively administered with each of the test conjugates in Table 7D. All animals were dosed based on body weight and was administered at a single dose by subcutaneous injection. Each drug conjugate was administered in the form of 0.2 mg/mL in 0.9% aqueous sodium chloride solution, with an administration volume of 5 mL/kg mouse body weight; that is, the administration doses of each conjugate were 1 mg/kg.

Each mouse in another group was administered with 1×PBS with an administration volume of 5 mL/kg mouse body weight, as a control group.

On day 14 after administrion, the animals were sacrificed, and the liver tissue of each mouse was collected separately and stored in RNA later (Sigma Aldrich Crop.). The liver tissue was homogenized with a tissue homogenizer and then extracted with Trizol (Thermo Fisher company) according to the operation procedures described in the instruction to obtain the total RNA.

For the liver tissue of each mouse, 1 μg of total RNA was taken, and the extracted total RNA was reverse-transcribed into cDNA using ImProm-II™ reverse transcription kit (Promega Crop.) according to the instructions thereof to obtain a solution containing cDNA, and then the expression level of HBV mRNA in liver tissue was determined by the

fluorescent quantitative PCR kit (Beijing Cowin Biosciences Co., Ltd.). In this fluorescent quantitative PCR method, β-actin gene was used as an internal control gene, and HBV and β-actin were detected by using primers for HBV and β-actin, respectively. The sequences of the primers for detection are shown in Table 6B.

The relative quantitative calculation of the expression level of the target HBV gene in each test group and the control group was carried out using the comparative Ct (ΔΔCt) method, and the calculation method was as follows:

$$\Delta Ct\ (\text{test group}) = Ct\ (\text{target gene of test group}) - Ct\ (\text{internal control gene of test group})$$

$$\Delta Ct\ (\text{control group}) = Ct\ (\text{target gene of control group}) - Ct\ (\text{internal control gene of control group})$$

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

wherein ΔCt (average of control group) was the arithmetic mean of the ΔCt (control group) of the five mice in the control group. Thus, each mouse in the test and control groups corresponded to a ΔΔCt value.

The expression level of HBV mRNA in the test group was normalized based on the control group, and the expression level of HBV mRNA in the control group was defined as 100%,

$$\text{Relative expression level of HBV mRNA in the test group} = 2^{-\Delta\Delta Ct\ (\text{the test group})} \times 100\%.$$

For the siRNA in the same test group, the mean of the relative expression level of HBV mRNA in the test group at each concentration was the arithmetic mean of the relative expression levels in the five mice at that concentration. Therein, the control group refers to the mice in the control group administered with PBS in this experiment, and each test group refers to the mice in the administration group adminstered with different drug conjugates. The results are shown in Table 7D below.

Table 7D Inhibition rates of different drug conjugates against the expression of HBV mRNA in mouse liver

| Conjugate Serial No. | Conjugate No. | Administration dose (mg/kg) | Inhibition rate against HBV mRNA in liver (%) |
|---|---|---|---|
| Conjugate 83 | N6-siHBd1M1 | 1 | 76.3 |
| Conjugate 84 | N6-siHBd1M2 | 1 | 82.1 |
| Conjugate 85 | N6-siHBdlM1SVP | 1 | 79.8 |
| Conjugate 86 | N6-siHBd1M2SVP | 1 | 83.2 |
| Conjugate 87 | N6-siHBd1M3SVP | 1 | 66.3 |
| Conjugate 88 | N6-siHBd1M4SVP | 1 | 65.8 |
| Conjugate 89 | N6-siHBd1M5SVP | 1 | 81.0 |
| Conjugate 90 | N6-siHBd2M1SVP | 1 | 77.9 |
| Conjugate 91 | N6-siHBd1M1SP | 1 | 79.6 |
| Conjugate 92 | N6-siHBd1M1SPs | 1 | 79.2 |
| Conjugate 93 | N6-siHBd4M1S | 1 | 83.1 |
| Conjugate 94 | X2-siHBd1M1SVP | 1 | 81.6 |
| Conjugate 95 | W2-siHBd1M1SVP | 1 | 80.8 |
| Conjugate 96 | V2-siHBd1M1SVP | 1 | 84.1 |

(continued)

| Conjugate Serial No. | Conjugate No. | Administration dose (mg/kg) | Inhibition rate against HBV mRNA in liver (%) |
|---|---|---|---|
| Conjugate 97 | O2-siHBd1M1SVP | 1 | 83.4 |
| Conjugate 98 | P2-siHBd1M1SVP | 1 | 82.9 |
| PBS | - | - | NA |

As can be seen from the results in Table 7D, the drug conjugates of the present disclosure showed very high *in vivo* inhibitory activity against liver HBV mRNA in the C57BL/6J-Tg(Alb1HBV)44Bri/J mouse at an administration dose of 1 mg/kg of siRNA, and exhibited an inhibition rate of up to 83.4% against HBV mRNA at a dose of 1 mg/kg.

**Experimental example 5-4 The time correlation test of the inhibitory efficiency of the siRNAs in the drug conjugates of the present disclosure against the expression level of HBsAg and HBV DNA in the serum of HBV transgenic mice**

AAV-HBV models were prepared according to the method in the literature (DONG Xiaoyan et al., Chin J Biotech 2010, May 25; 26(5): 679-686), rAAV8-1.3HBV, type D (ayw) virus (purchased from Beijing FivePlus Molecular Medicine Institute Co. Ltd., $1\times10^{12}$ viral genome (v.g.)/mL, Lot No. 2016123011). The AAV virus was diluted to $5\times10^{11}$ v.g./mL with sterilized PBS prior to the experiment. Each mouse was injected with 200 μL (that is, each mouse was injected with $1\times10^{11}$ v.g). On day 28 post virus injection, orbital blood (about 100 μL) was collected from all mice for collecting serum for dectection of HBsAg and HBV DNA.

After the animals were successfully modeled, they were randomly divided into groups according to serum HBsAg content (five mice per group) and respectively numbered. Each group of mice was administered with each of the test conjugates in Table 8D, respectively. All animals were dosed based on body weight and was substaneously administered at a single dose. The administration dose was 3 mg/mL and the volume was 5 mL/kg. Each mouse in another group was administered with 1×PBS with an administration volume of 5 mL/kg mouse body weight, as a control group. The blood was collected from mouse orbital venous plexus before administration and on day 7, 14, 21, 28 and 56 after administration, and the serum HBsAg level was determined at each time point.

About 100 μL orbital blood was taken each time, and no less than 20 μL serum was collected after centrifugation. The expression level of HBsAg in serum was determined by using HBsAg CLIA kit (Autobio, CL0310) according to the instructions provided in the kit. DNA in serum was extracted according to the instructions of QIAamp 96 DNA Blood Kit, and was subject to quantitative PCR to determine the expression level of HBV DNA.

The inhibition rate against HBsAg is calculated according to the following equation:

$$\text{The inhibition rate against HBsAg} = (1\text{-HBsAg content after administration/HBsAg content before administration}) \times 100\%,$$

wherein the content of HBsAg was expressed as the equivalents (UI) of HBsAg per milliliter (mL) of serum.

The inhibition rate against HBV DNA is calculated according to the following equation:

$$\text{The inhibition rate against HBV DNA} = (1 - \text{the content of HBV DNA after administration/the content of HBV DNA before administration}) \times 100\%,$$

wherein the content of HBV DNA was expressed as the copies of HBV DNA per milliliter (mL) of serum.

Tables 8D and 9D respectively show the *in vivo* inhibition rates against HBsAg and HBV DNA in HBV transgenic mice administered with different doses of each drug conjugate or PBS.

Table 8D Inhibition rates of different drug conjugates against HBsAg in mouse serum

| Conjugate Serial No. | Conjugate No. | Inhibition rate against HBsAg in serum (%) | | | | |
|---|---|---|---|---|---|---|
| | | D7 | D14 | D21 | D28 | D56 |
| Conjugate 85 | N6-siHBdlM1SVP | 91.8 | 91.2 | 91.2 | 86.1 | 79.2 |
| Conjugate 86 | N6-siHBd1M2SVP | 91.2 | 92.8 | 91.3 | 88.2 | 79.1 |
| Conjugate 90 | N6-siHBd2M1SVP | 91.6 | 92.9 | 91.6 | 86.1 | 77.6 |
| Conjugate 91 | N6-siHBd1M1SP | 92.4 | 92.9 | 92.8 | 86.2 | 77.2 |
| Conjugate 92 | N6-siHBd1M1SPs | 93.8 | 93.4 | 93.4 | 93.1 | 85.3 |
| Conjugate 94 | X2-siHBd1M1SVP | 93.7 | 93.1 | 94.2 | 92.4 | 83.4 |
| Conjugate 95 | W2-siHBd1M1SVP | 95.1 | 95.2 | 95.6 | 94.8 | 84.2 |
| Conjugate 96 | V2-siHBd1M1SVP | 94.2 | 94.2 | 94.0 | 92.6 | 83.8 |
| Conjugate 97 | O2-siHBd1M1SVP | 92.1 | 92.9 | 93.8 | 92.2 | 84.1 |
| Conjugate 98 | P2-siHBd1M1SVP | 91.8 | 92.8 | 93.5 | 91.1 | 82.1 |
| - | PBS | 1.8 | -9.3 | -12.4 | -8.8 | -44.2 |

As can be seen from the results of Table 8D, the PBS negative control group showed no inhibitory effect at different time points after administration; in contrast, the drug conjugate of the present disclosure showed excellent inhibitory effect on HBsAg at different time points after administration, and in particular consistently showed a high inhibition rate of up to 95.6% against HBsAg in serum over a period of 21 days, indicating that it can stably and efficiently inhibit the expression of HBV mRNA over a longer time period.

Table 9D Inhibition rate of different drug conjugates against HBV DNA in mouse serum

| Conjugate Serial No. | Conjugate No. | Inhibition rate against HBV DNA in serum (%) | | | | |
|---|---|---|---|---|---|---|
| | | D7 | D14 | D21 | D28 | D56 |
| Conjugate 85 | N6-siHBdlM1SVP | 76.5 | 91.3 | 90.2 | 87.3 | 85.6 |
| Conjugate 86 | N6-siHBd1M2SVP | 75.2 | 90.5 | 89.5 | 84.8 | 82.1 |
| Conjugate 90 | N6-siHBd2M1SVP | 75.8 | 91.6 | 90.2 | 85.3 | 80.3 |
| Conjugate 91 | N6-siHBd1M1SP | 76.3 | 92.2 | 91.1 | 86.2 | 83.5 |
| Conjugate 92 | N6-siHBd1M1SPs | 76.5 | 91.6 | 90.3 | 85.4 | 83.2 |
| Conjugate 94 | X2-siHBd1M1SVP | 75.8 | 92.6 | 91.2 | 83.2 | 81.6 |
| Conjugate 95 | W2-siHBd1M1SVP | 76.4 | 93.1 | 91.3 | 82.9 | 80.2 |
| Conjugate 96 | V2-siHBd1M1SVP | 76.6 | 92.4 | 91.5 | 84.3 | 82.1 |
| Conjugate 97 | O2-siHBd1M1SVP | 75.2 | 92.6 | 91.6 | 84.2 | 82.1 |
| Conjugate 98 | P2-siHBd1M1SVP | 75.1 | 91.8 | 90.0 | 83.8 | 83.1 |
| - | PBS | 0.4 | 3.5 | -1.8 | -1.8 | -8 |

As can be seen from the results in Table 9D, the drug conjugates of the present disclosure also showed excellent inhibition effect on HBV DNA, and maintained a higher inhibition rate of up to 93.1% over a period of up to 56 days.

**Experimental Example 6 Effect experiments of the drug conjugates of the present disclosure**

**Experimental Example 6-1 Determination of the inhibitory efficiency of the drug conjugates of the present disclosure against the expression level of HBV mRNA in HepG2.2.15 cells**

The HepG2.2.15 cells were seeded into a 24-well plate at 7 $\times$ $10^4$ cells/well with H-DMEM complete medium.

After 16 hours, when the cell growth density reached 70-80%, the H-DMEM complete medium in the culture well was aspirated, and 500 μL Opti-MEM medium (GIBCO company) was added to each well, and the cells were cultured for another 1.5 hours.

Each drug conjugate of the drug conjugates below was respectively formulated into working solutions of drug conjugate at 3 different concentrations of 50 μM, 5 μM and 0.5 μM with DEPC water, wherein the drug conjugates used are respectively the test conjugates in Table 4E.

For each siRNA, 6A1, 6A2 and 6A3 solutions were formulated, respectively. Each of 6A1-6A3 solutions contained 0.6 μL of the above siRNA working solution at one of the above 3 concentrations and 50 μL of Opti-MEM medium.

Formulation of 6B solution: each 6B solution contained 1 μL of Lipofectamine™ 2000 and 50 μL of Opti-MEM medium.

One portion of 6B solution was respectively mixed with one portion of the resultant 6A1, 6A2 or 6A3 solution of each siRNA, and incubated at room temperature for 20 minutes respectively to obtain transfection complexes 6×1, 6X2 and 6X3 of each siRNA.

One portion of 6B solution was mixed with 50 μL of Opti-MEM medium and incubated for 20 minutes at room temperature to obtain a transfection complex 6X4.

The transfection complex 6X1, 6X2 or 6X3 of each drug conjugate was respectively added to the culture wells in an amount of 100 μL/well and mixed well to obtain a transfection complexes with the final concentration of each drug conjugate of about 50 nM, 5 nM or 0.5 nM, respectively. Each transfection complex was respectively used to transfect three culture wells to obtain drug conjugate-containing transfection mixtures (designated as test group).

The transfection complex 6X4 was respectively added to another three culture wells in an amount of 100 μL/well to obtain a siRNA-free transfection mixture (designated as control group).

After the drug conjugate-containing transfection mixtures and the drug conjugate-free transfection mixture were cultured in the culture wells for 4 hours, each well was supplemented with 1 mL of H-DMEM complete medium containing 20% FBS. The 24-well plate was placed in a $CO_2$ incubator and cultured for another 24 hours.

Subsequently, the total RNA of the cells in each well was extracted by RNeasy Mini Kit (QIAGEN company, Cat No. 74106) according to the detailed preoceudre as described in the instruction.

For the cells of each well, 1 μg of the total RNA was taken, and was formulated into a 20 μL reverse transcription reaction system for reverse transcription of the total RNA of the cell by using the reagent provided in the reverse transcription kit GoldenstarTM RT6 cDNA Synthesis Kit (purchased from Beijing Tsingke Biotechnology Co., Ltd., Cat. No. TSK301M) according to the precedures for reverse transcription in the instruction of the kit, in which GoldenstarTM Oligo (dT)17 was selected as the primer. Conditions for reverse transcription were as follows: the reverse transcription reaction system was incubated at 50°C for 50 minutes, then incubated at 85°C for 5 minutes, and finally incubated at 4°C for 30 seconds; after the reaction was completed, 80 μL of DEPC water was added to the reverse transcription reaction system to obtain a cDNA-containing solution.

For each reverse transcription reaction system, 5 μL of the above cDNA-containing solution was taken as the template, and was formulated into a 20 μL qPCR reaction system by using the reagent provided in the NovoStart® SYBR qPCR SuperMix Plus kit (purchased from Novoprotein Scientific Co., Ltd., Cat No. E096-01B), wherein the sequences of PCR primers for amplifying the target gene HBV and the internal control gene GAPDH are shown in Table 3E, and the final concentration of each primer was 0.25 μM. Each qPCR reaction system was placed in an ABI StepOnePlus Real-Time PCR Thermal Cycler, and the amplification was performed by a three-step method using the following amplification procedure: pre-denaturation at 95°C for 10 minutes, then denaturation at 95°C for 30 s, annealing at 60°C for 30 s, and extension at 72°C for 30 s (wherein the above process of denaturation, annealing and extension was repeated for 40 times), to obtain a product W containing the amplified target gene HBV and the amplified internal control gene GAPDH. The product W was then sequentially incubated at 95°C for 15 s, 60°C for 1 min, and 95°C for 15 s. The melting curves of the target gene HBV and the internal control gene GAPDH in the product W were respectively collected using a real-time fluorescent quantitative PCR Thermal Cycler, and the Ct values of the target gene HBV and the internal control gene GAPDH were obtained.

Table 3E The sequences of the primers for detection

| Gene | Upstream Primers | Downstream Primers |
|---|---|---|
| HBV | 5'-GTCTTTTGGGTTTTGCTGCC -3' (SEQ ID NO: 689) | 5'- GCAACGGGGTAAAGGTTCAG -3' (SEQ ID NO: 690) |
| GAPDH | 5'- GGTCGGAGTCAACGGATTT - 3' (SEQ ID NO: 691) | 5'- CCAGCATCGCCCCACTTGA -3' (SEQ ID NO: 692) |

The relative quantitative calculation of the expression level of the target HBV gene in each test group and the

control group was carried out using the comparative Ct (ΔΔCt) method, and the calculation method was as follows:

$$\Delta Ct\ (\text{test group}) = Ct\ (\text{target gene of test group}) - Ct\ (\text{internal control gene of test group})$$

$$\Delta Ct\ (\text{control group}) = Ct\ (\text{target gene of control group}) - Ct\ (\text{internal control gene of control group})$$

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

wherein ΔCt (average of control group) was the arithmetic mean of the ΔCt (control group) of the three culture wells of the control group. Thus, each culture well in the test and control groups corresponded to a ΔΔCt value.

The expression level of HBV mRNA in the test group was normalized based on the control group, and the expression level of HBV mRNA in the control group was defined as 100%,

Relative expression level of HBV mRNA in the test group = $2^{-\Delta\Delta Ct\ (\text{the test group})} \times 100\%$.

For the siRNA in the same test group, the mean of the relative expression level of HBV mRNA in the test group at each concentration was the arithmetic mean of the relative expression levels of the three culture wells at that concentration.

Table 4E below shows the determination results of the inhibitory activities of the drug conjugates listed in Table 2E and the drug conjugate of Comparative Conjugate 1 against the expression of HBV mRNA in HepG2.2.15 cells.

Table 4E Inhibition rates of the drug conjugates at different concentrations against HBV mRNA

| Conjugate Serial No. | Conjugate No. | Inhibition rate against HBV mRNA (%) | | |
|---|---|---|---|---|
| | | 50 nM | 10 nM | 1 nM |
| Conjugate 99 | N6-siHBe3M1S | 78.1 | 44.4 | 28.9 |
| Conjugate 100 | N6-siHBe4M1S | 78.9 | 49.5 | 29.2 |
| Conjugate 101 | N6-siHBe3M1 | 78.8 | 47.5 | 26.5 |
| Conjugate 102 | N6-siHBe4M1 | 79.9 | 45.5 | 27.1 |
| Conjugate 103 | N6-siHBe3M1SVP | 79.9 | 50.4 | 28.7 |
| Conjugate 104 | N6-siHBe3M1SPs | 79.0 | 50.0 | 28.6 |
| Conjugate 105 | N6-siHBe3M1SP | 78.9 | 49.8 | 28.3 |
| Conjugate 106 | N6-siHBe3M1VP | 78.2 | 49.5 | 28.0 |
| Conjugate 107 | N6-siHBe3M2 | 78.1 | 48.4 | 30.8 |
| Conjugate 108 | N6-siHBe3M2SVP | 79.0 | 49.4 | 29.2 |
| Conjugate 109 | N6-siHBe3M3SVP | 78.9 | 49.4 | 27.2 |
| Conjugate 110 | N6-siHBe3M4SVP | 52.9 | 35.4 | 15.6 |
| Conjugate 111 | N6-siHBe3M5SVP | 55.2 | 30.4 | 11.1 |
| Conjugate 112 | N6-siHBe1M1S | 79.8 | 47.4 | 27.1 |
| Conjugate 113 | N6-siHBe5M1SVP | 70.7 | 45.4 | 23.2 |
| Conjugate 114 | N6-siHBe0M1 | 65.8 | 42.1 | 23.3 |
| Conjugate 115 | N6-siHBe2 | 68.7 | 45.9 | 25.3 |
| Conjugate 116 | X2-siHBe3M1SVP | 78.7 | 51.3 | 30.6 |

(continued)

| Conjugate Serial No. | Conjugate No. | Inhibition rate against HBV mRNA (%) | | |
|---|---|---|---|---|
| | | 50 nM | 10 nM | 1 nM |
| Conjugate 117 | W2-siHBe3M1SVP | 75.8 | 50.6 | 30.3 |
| Conjugate 118 | V2-siHBe3M1SVP | 78.9 | 50.5 | 30.2 |
| Conjugate 119 | O2-siHBe3M1SVP | 73.9 | 50.8 | 30.3 |
| Conjugate 120 | P2-siHBe3M1SVP | 78.1 | 50.5 | 30.2 |
| Comparative Conjugate 1 | N6-NC | 3.1 | 1.8 | -4.8 |

As can be seen from the results of Table 4E, the drug conjugates of the present disclosure exhibited very high inhibitory activity against HBV mRNA in HepG2.2.15 cells *in vitro,* and could show an inhibition rate of up to 79.9% against HBV mRNA at the siRNA concentration of 50 nM.

**Experimental Example 6-2 The stability of the drug conjugates of the present disclosure in human plasma *in vitro***

Each drug conjugate of the drug conjugates and the drug conjugate of Comparative Conjugate 1 (provided as 20 μM in 0.9 wt% NaCl aqueous solution (calculated based on siRNA), 12 μL per group) was respectively mixed well with 108 μL of 90% human plasma (purchased from Jiangsu Hematology Institute and diluted with 1×PBS (pH 7.4)) to obtain mixed solutions, wherein the drug conjugates used are respectively those listed in Table 5E. The mixed solution was incubated at a constant temperature of 37°C. 10 μL of the sample was taken at 0 h, 8 h, 24 h and 48 h, respectively, and immediately frozen in liquid nitrogen and cryopreserved in a - 80°C freezer. Therein, 0 hour refers to the time when 10 μL of the mixed solution was taken immediately after the conjugate solution was mixed well with 90% human plasma. After sampling at each time point, each mixed solution was diluted 5-fold with 1×PBS (pH 7.4) and then taken in a volume of 10 μL for electrophoresis.

20 wt% of non-denatured polyacrylamide gel was prepared. The above samples for electrophoresis in each group were respectively mixed with 4 μL of loading buffer (20 mM EDTA, 36 wt% glycerol, and 0.06 wt% bromophenol blue) and then loaded into each gel hole for electrophoresis under 80 mA constant current for about 60 minutes. After electrophoresis, the gel was taken out, stained with 1×Sybr Gold dye (Invitrogen, Cat.11494) for 15 minutes for imaging. The stability results were calculated and the results are shown in Table 5E.

Table 5E shows the semiquantitative test result of the stability of the test drug conjugates listed in Table 2E in human plasma *in vitro.* The result is expressed as the ratio (RL) of the longest fragment remaining after the incubation of the test drug conjugate with human plasma to the longest fragment of untreated siRNA.

Table 5E The plasma stability of different drug conjugates over time

| Conjugate Serial No. | Conjugate No. | RL (%) | | | | |
|---|---|---|---|---|---|---|
| | | Untreated | 0h | 8h | 24h | 48h |
| Conjugate 99 | N6-siHBe3M1S | 100 | 100 | 99.6 | 96.0 | 94.7 |
| Conjugate 100 | N6-siHBe4M1S | 100 | 100 | 99.7 | 96.4 | 94.7 |
| Conjugate 101 | N6-siHBe3M1 | 100 | 100 | 99.6 | 96.1 | 94.8 |
| Conjugate 102 | N6-siHBe4M1 | 100 | 100 | 99.4 | 96.7 | 95.4 |
| Conjugate 103 | N6-siHBe3M1SVP | 100 | 100 | 99.4 | 96.5 | 95.5 |
| Conjugate 104 | N6-siHBe3M1SPs | 100 | 100 | 99.4 | 96.4 | 95.1 |
| Conjugate 105 | N6-siHBe3M1SP | 100 | 100 | 99.5 | 96.1 | 95.4 |
| Conjugate 106 | N6-siHBe3M1VP | 100 | 100 | 99.4 | 96.0 | 94.9 |
| Conjugate 107 | N6-siHBe3M2 | 100 | 100 | 99.6 | 96.7 | 95.8 |
| Conjugate 108 | N6-siHBe3M2SVP | 100 | 100 | 99.1 | 97.1 | 95.1 |
| Conjugate 109 | N6-siHBe3M3SVP | 100 | 100 | 99.4 | 96.4 | 95.5 |

(continued)

| Conjugate Serial No. | Conjugate No. | RL (%) | | | | |
|---|---|---|---|---|---|---|
| | | Untreated | 0h | 8h | 24h | 48h |
| Conjugate 110 | N6-siHBe3M4SVP | 100 | 100 | 99.6 | 95.5 | 94.6 |
| Conjugate 111 | N6-siHBe3M5SVP | 100 | 100 | 99.7 | 96.4 | 96.4 |
| Conjugate 112 | N6-siHBe1M1S | 100 | 100 | 99.5 | 97.4 | 94.4 |
| Conjugate 113 | N6-siHBe5M1SVP | 100 | 100 | 99.6 | 97.1 | 94.4 |
| Conjugate 114 | N6-siHBe0M1 | 100 | 100 | 99.8 | 94.4 | 93.4 |
| Conjugate 115 | N6-siHBe2 | 100 | 100 | 95.5 | 93.6 | 92.5 |
| Conjugate 116 | X2-siHBe3M1SVP | 100 | 100 | 99.4 | 97.1 | 94.8 |
| Conjugate 117 | W2-siHBe3M1SVP | 100 | 100 | 99.6 | 96.1 | 95.4 |
| Conjugate 118 | V2-siHBe3M1SVP | 100 | 100 | 99.3 | 95.7 | 95.0 |
| Conjugate 119 | O2-siHBe3M1SVP | 100 | 100 | 99.6 | 96.4 | 96.4 |
| Conjugate 120 | P2-siHBe3M1SVP | 100 | 100 | 99.2 | 96.0 | 94.7 |
| Comparative Conjugate 1 | N6-NC | 100 | 0 | 0 | 0 | 0 |

As can be seen from the results of Table 5E, all the drug conjugates of the present disclosure exhibited excellent stability in plasma, and still showed a length ratio of the siRNA fragments of higher than 92.5% at 48 h.

**Experimental example 6-3 Determination of the inhibitory efficiency of the drug conjugates of the present disclosure against the expression of HBV mRNA in the HBV transgenic mice C57BL/6J-Tg(Alb1HBV)44Bri/J**

The HBV transgenic mice C57BL/6J-Tg (Alb1HBV) 44Bri/J used in this experimental example were purchased from the Department of Laboratory Animal Science, Peking University Health Science Center. C57BL/6J-Tg(Alb1HBV)44Bri/J mice were randomly divided into groups (all female, five mice per group) according to serum HBsAg content, and each group of mice was numbered according to the drug conjugates in Table 2C. Then, each group of mice was respectively administered with each of the test conjugates listed in Table 7E. All animals were dosed based on body weight and was administered at a single dose by subcutaneous injection. Each conjugate was administered in the form of 0.2 mg/mL in 0.9% aqueous sodium chloride solution, with an administration volume of 5 mL/kg mouse body weight; that is, the administration doses of each conjugate were 1 mg/kg.

Each mouse in another group was administered with 1 ×PBS with an administration volume of 5 mL/kg mouse body weight, as a control group.

On day 14 after administrion, the animals were sacrificed, and the liver tissue of each mouse was collected separately and stored in RNA later (Sigma Aldrich Crop.). The liver tissue was homogenized with a tissue homogenizer and then extracted with Trizol (Thermo Fisher company) according to the operation procedures described in the instruction to obtain the total RNA.

For the liver tissue of each mouse, 1 μg of total RNA was taken, and the extracted total RNA was reverse-transcribed into cDNA using ImProm-II™ reverse transcription kit (Promega Crop.) according to the instructions thereof to obtain a solution containing cDNA, and then the expression level of HBV mRNA in liver tissue was determined by the fluorescent quantitative PCR kit (Beijing Cowin Biosciences Co., Ltd.). In this fluorescent quantitative PCR method, β-actin gene was used as an internal control gene, and HBV and β-actin were detected by using primers for HBV and β-actin, respectively. The sequences of the primers for detection are shown in Table 6E.

Table 6E The sequences of the primers for detection

| Gene | Upstream primers | Downstream primers |
|---|---|---|
| HBV | 5'-GTCTTTTGGGTTTTGCTGCC -3' (SEQ ID NO: 693) | 5'- GCAACGGGGTAAAGGTTCAG-3' (SEQ ID NO: 694) |
| β-actin | 5'-AGCTTCTTTGCAGCTCCTTCGTTG -3' (SEQ ID NO: 695) | 5'-TTCTGACCCATTCCCACCATCACA -3' (SEQ ID NO: 696) |

The relative quantitative calculation of the expression level of the target HBV gene in each test group and the control group was carried out using the comparative Ct (ΔΔCt) method, and the calculation method was as follows:

$$\Delta Ct\ (\text{test group}) = Ct\ (\text{target gene of test group}) - Ct\ (\text{internal control gene of test group})$$

$$\Delta Ct\ (\text{control group}) = Ct\ (\text{target gene of control group}) - Ct\ (\text{internal control gene of control group})$$

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

wherein ΔCt (average of control group) was the arithmetic mean of the ΔCt (control group) of the five mice in the control group. Thus, each mouse in the test and control groups corresponded to a ΔΔCt value.

The expression level of HBV mRNA in the test group was normalized based on the control group, and the expression level of HBV mRNA in the control group was defined as 100%,

Relative expression level of HBV mRNA in the test group = $2^{-\Delta\Delta Ct\ (\text{the test group})} \times 100\%$.

For the siRNA in the same test group, the mean of the relative expression level of HBV mRNA in the test group at each concentration was the arithmetic mean of the relative expression levels in the five mice at that concentration.

Therein, the control group refers to the mice in the control group administered with PBS in this experiment, and each test group refers to the mice in the administration group adminstered with different drug conjugates. The results are shown in Table 7E below.

Table 7E Inhibition rates of different drug conjugates against HBV mRNA in mouse liver

| Conjugate Serial No. | Conjugate No. | Administration dose (mg/kg) | Inhibition rate against HBV mRNA in liver (%) |
|---|---|---|---|
| Conjugate 99 | N6-siHBe3M1S | 1 | 86.4 |
| Conjugate 100 | N6-siHBe4M1S | 1 | 84.2 |
| Conjugate 101 | N6-siHBe3M1 | 1 | 84.0 |
| Conjugate 102 | N6-siHBe4M1 | 1 | 82.5 |
| Conjugate 103 | N6-siHBe3M1SVP | 1 | 88.5 |
| Conjugate 104 | N6-siHBe3M1SPs | 1 | 88.2 |
| Conjugate 105 | N6-siHBe3M1SP | 1 | 88.0 |
| Conjugate 106 | N6-siHBe3M1VP | 1 | 84.0 |
| Conjugate 107 | N6-siHBe3M2 | 1 | 84.2 |
| Conjugate 108 | N6-siHBe3M2SVP | 1 | 88.7 |
| Conjugate 109 | N6-siHBe3M3SVP | 1 | 88.6 |
| Conjugate 112 | N6-siHBe1M1S | 1 | 85.3 |
| Conjugate 113 | N6-siHBe5M1SVP | 1 | 80.5 |
| Conjugate 116 | X2-siHBe3M1SVP | 1 | 88.1 |

(continued)

| Conjugate Serial No. | Conjugate No. | Administration dose (mg/kg) | Inhibition rate against HBV mRNA in liver (%) |
|---|---|---|---|
| Conjugate 117 | W2-siHBe3M1SVP | 1 | 88.4 |
| Conjugate 118 | V2-siHBe3M1SVP | 1 | 88.5 |
| Conjugate 119 | O2-siHBe3M1SVP | 1 | 88.6 |
| Conjugate 120 | P2-siHBe3M1SVP | 1 | 88.3 |
| PBS | - | NA | 2.0 |

As can be seen from the above results, the drug conjugates of the present disclosure showed very high *in vivo* inhibitory activity against liver HBV mRNA in the C57BL/6J-Tg(Alb1HBV)44Bri/J mice at an administration dose of 1 mg/kg of siRNA. In particular, Conjugates 103-106, 108-109 and 116-120 showed very high inhibitory activity against HBV mRNA in the liver tissue of hepatitis B mice in the *in vivo* experiments of C57BL/6J-Tg(Alb1HBV)44Bri/J mice, and exhibited an inhibition rate of up to 88.6% against HBV mRNA at a dose of 1 mg/kg.

**Experimental example 6-4 The time correlation test of the inhibitory efficiency of the siRNAs in the drug conjugates of the present disclosure against the expression level of HBsAg and HBV DNA in the serum of HBV transgenic mice**

AAV-HBV models were prepared according to the method in the literature (DONG Xiaoyan et al., Chin J Biotech 2010, May 25; 26(5): 679-686),rAAV8-1.3HBV, type D (ayw) virus (purchased from Beijing FivePlus Molecular Medicine Institute Co. Ltd., $1\times10^{12}$ viral genome (v.g.)/mL, Lot No. 2016123011). The AAV virus was diluted to $5\times10^{11}$ v.g./mL with sterilized PBS prior to the experiment. Each mouse was injected with 200 μL (that is, each mouse was injected with $1\times10^{11}$ v.g). On day 28 post virus injection, orbital blood (about 100 μL) was collected from all mice for collecting serum for dectection of HBsAg and HBV DNA.

After the animals were successfully modeled, they were randomly divided into groups according to serum HBsAg content (five mice per group). Each group of mice was administered with each of the test drug conjugates in Table 8E, respectively. All animals were dosed based on body weight and was substaneously administered at a single dose. The administration dose was 3 mg/mL and the volume was 5 mL/kg. Each mouse in another group was administered with 1×PBS with an administration volume of 5 ml/kg mouse body weight, as a control group. The blood was collected from mouse orbital venous plexus before administration and on day 7, 14, 21, 28, 56, 84, 98 and 112 after administration, and the levels of HBsAg and DNA in serum were determined at each time point.

About 100 μl orbital blood was taken each time, and no less than 20 μl serum was collected after centrifugation. The expression level of HBsAg in serum was determined by using HBsAg CLIA kit (Autobio, CL0310) according to the instructions provided in the kit. DNA in serum was extracted according to the instructions of QIAamp 96 DNA Blood Kit, and was subject to quantitative PCR to determine the expression level of HBV DNA.

The inhibition rate against HBsAg is calculated according to the following equation:

$$\text{The inhibition rate against HBsAg} = (1\text{-HBsAg content after administration/HBsAg content before administration}) \times 100\%,$$

wherein the content of HBsAg was expressed as the equivalents (UI) of HBsAg per milliliter (mL) of serum.

The inhibition rate against HBV DNA is calculated according to the following equation:

$$\text{The inhibition rate against HBV DNA} = (1 - \text{the content of HBV DNA after administration/the content of HBV DNA before administration}) \times 100\%,$$

wherein the content of HBV DNA was expressed as the copies of HBV DNA per milliliter (mL) of serum.

Tables 8E and 9E respectively show the *in vivo* inhibition rates against HBsAg and HBV DNA in HBV transgenic mice administered with different doses of each drug conjugate or PBS.

Table 8E Inhibition of the exression of HBsAg by different drug conjugates in mouse serum at different time points

| Conjugate Serial No. | Conjugate No. | Inhibition rate against HBsAg in serum (%) | | | | | | | | | |
|---|---|---|---|---|---|---|---|---|---|---|---|
| | | D7 | D14 | D21 | D28 | D40 | D56 | D70 | D84 | D98 | D112 |
| Conjugate 99 | N6-siHBe3M1S | 99.6 | 99.5 | 99.6 | 99.4 | 98.8 | 96.5 | 95.6 | 93.1 | 84.2 | 80.9 |
| Conjugate 112 | N6-siHBe1M1S | 99.5 | 99.4 | 99.7 | 99.2 | 98.8 | 96.4 | 94.5 | 92.0 | 85.6 | 80.8 |
| Conjugate 101 | N6-siHBe3M1 | 99.4 | 99.1 | 99.1 | 99.0 | 98.6 | 96.9 | 94.7 | 92.8 | 84.9 | 78.2 |
| Conjugate 103 | N6-siHBe3M1SVP | 99.5 | 99.8 | 99.7 | 99.5 | 99.0 | 97.4 | 95.5 | 93.2 | 87.0 | 82.5 |
| Conjugate 104 | N6-siHBe3M1SPs | 99.6 | 99.6 | 99.6 | 99.4 | 99.0 | 96.0 | 94.0 | 91.6 | 80.0 | 75.6 |
| Conjugate 105 | N6-siHBe3M1 SP | 99.3 | 99.7 | 99.6 | 99.4 | 98.8 | 97.5 | 95.0 | 92.6 | 84.8 | 78.6 |
| Conjugate 108 | N6-siHBe3M2SVP | 99.4 | 99.4 | 99.3 | 99.3 | 99.3 | 97.5 | 95.4 | 92.8 | 85.0 | 82.2 |
| Conjugate 109 | N6-siHBe3M3 SVP | 99.0 | 99.6 | 99.2 | 99.2 | 99.4 | 97.6 | 94.9 | 93.8 | 81.6 | 79.0 |
| Conjugate 116 | X2-siHBe3M1SVP | 99.4 | 99.6 | 99.3 | 99.5 | 99.0 | 97.2 | 95.8 | 93.1 | 86.5 | 82.3 |
| Conjugate 117 | W2-siHBe3M1SVP | 99.5 | 99.7 | 99.7 | 99.4 | 99.1 | 97.8 | 95.1 | 93.2 | 86.7 | 82.4 |
| Conjugate 118 | V2-siHBe3M1SVP | 99.4 | 99.7 | 99.6 | 99.3 | 99.2 | 97.7 | 95.3 | 92.9 | 87.2 | 84.4 |
| Conjugate 119 | O2-siHBe3M1SVP | 99.0 | 99.8 | 99.5 | 99.5 | 99.2 | 97.7 | 95.3 | 92.8 | 87.2 | 82.0 |
| Conjugate 120 | P2-siHBe3M1SVP | 99.7 | 99.9 | 99.8 | 99.6 | 99.3 | 97.6 | 95.2 | 93.0 | 86.9 | 81.9 |
| - | PBS | 1.9 | 2.4 | -10.5 | -6.5 | -2.8 | 5.6 | 3.6 | 9.0 | -1.0 | -5.9 |

As can be seen from the results of Table 8E, the PBS negative control group showed no inhibitory effect at different time points after administration; in contrast, the drug conjugate of the present disclosure showed excellent inhibitory effect on HBsAg at different time points after administration, and in particular consistently showed a high inhibition effect of up to 99.9% against HBsAg in serum over a period of up to 140 days, indicating that it can stably and efficiently inhibit the expression of HBV mRNA over a longer time period.

Table 9E Inhibition rates of different drug conjugates against HBV DNA in mouse serum at different time points

| siRNA Conjugate | No. | Inhibition rate against HBsAg in serum (%) | | | | | | | | | |
|---|---|---|---|---|---|---|---|---|---|---|---|
| | | D7 | D14 | D21 | D28 | D40 | D56 | D70 | D84 | D98 | D112 |
| Conjugate 99 | N6-siHBe3M1S | 97.6 | 99.0 | 98.0 | 96.6 | 96.3 | 93.2 | 90.0 | 88.7 | 80.1 | 78.3 |
| Conjugate 112 | N6-siHBe1M1S | 96.9 | 99.0 | 98.9 | 96.8 | 96.2 | 92.2 | 91.0 | 89.6 | 80.5 | 76.5 |
| Conjugate 101 | N6-siHBe3M1 | 97.0 | 99.1 | 97.7 | 95.8 | 96.0 | 90.1 | 90.5 | 88.6 | 82.0 | 77.6 |
| Conjugate 103 | N6-siHBe3M1SVP | 97.6 | 99.2 | 98.3 | 97.0 | 96.6 | 93.8 | 92.6 | 90.1 | 84.2 | 79.8 |
| Conjugate 104 | N6-siHBe3M1SPs | 97.6 | 99.4 | 98.6 | 96.4 | 96.1 | 93.7 | 92.2 | 90.0 | 78.9 | 73.2 |

(continued)

| siRNA Conjugate | No. | Inhibition rate against HBsAg in serum (%) | | | | | | | | | |
|---|---|---|---|---|---|---|---|---|---|---|---|
| | | D7 | D14 | D21 | D28 | D40 | D56 | D70 | D84 | D98 | D112 |
| Conjugate 105 | N6-siHBe3M1 SP | 97.5 | 99.3 | 98.1 | 96.4 | 96.4 | 93.9 | 92.1 | 89.8 | 80.6 | 76.9 |
| Conjugate 108 | N6-siHBe3M2SVP | 96.8 | 99.0 | 98.0 | 96.2 | 96.0 | 92.7 | 91.9 | 88.7 | 78.9 | 75.4 |
| Conjugate 109 | N6-siHBe3M3 SVP | 96.9 | 99.1 | 97.9 | 96.6 | 95.9 | 92.5 | 91.0 | 88.9 | 78.3 | 76.2 |
| Conjugate 116 | X2-siHBe3M1SVP | 97.8 | 99.1 | 98.5 | 96.1 | 96.2 | 93.6 | 92.5 | 90.1 | 80.2 | 80.9 |
| Conjugate 117 | W2-siHBe3M1SVP | 97.5 | 99.2 | 98.6 | 96.4 | 96.1 | 93.7 | 92.7 | 90.0 | 82.9 | 78.5 |
| Conjugate 118 | V2-siHBe3M1SVP | 97.6 | 99.2 | 98.9 | 96.7 | 96.3 | 93.2 | 92.2 | 90.8 | 80.9 | 77.7 |
| Conjugate 119 | O2-siHBe3M1SVP | 87.2 | 99.3 | 97.6 | 96.7 | 96.5 | 93.6 | 92.0 | 89.9 | 81.6 | 76.6 |
| Conjugate 120 | P2-siHBe3M1SVP | 97.1 | 99.6 | 97.4 | 97.2 | 96.7 | 93.1 | 92.7 | 89.8 | 83.3 | 79.9 |
| - | PBS | 2.6 | 2.9 | -0.5 | -5.6 | -12.8 | -8.7 | -3.8 | 6.0 | -1.0 | -4.9 |

As can be seen from the results in Table 9E, the drug conjugates of the present disclosure also showed excellent inhibition effect on HBV DNA, and maintained a higher inhibition rate of up to 99.6% over a period of up to 112 days.

**Experimental Example 7 Effect experiments of the drug conjugates in Table 2F**

**Experimental Example 7-1 This experiment illustrates the determination of the inhibitory efficiency of the drug conjugates in Table 2F against the expression level of ANGPTL3 mRNA in Huh7 cells.**

The Huh7 cells were seeded into a 24-well plate at 7.5 $\times$ $10^4$ cells/well with H-DMEM complete medium. After 16 hours, when the cell growth density reached 70-80%, the H-DMEM complete medium in the culture well was aspirated, and 500 μL Opti-MEM medium (GIBCO company) was added to each well, and the cells were cultured for another 1.5 hours.

The drug conjugates below were respectively formulated into working solutions of drug conjugate at 3 different concentrations of 5 μM, 0.5 μM and 0.05 μM with DEPC water (calculated based on siRNA), wherein the drug conjugates were Conjugates 121-158 in Table 2F and Comparative Conjugate 1 in Table 2A, and the Comparative Conjugate 1 was used as negative control.

For each drug conjugate, 7A1, 7A2 and 7A3 solutions were formulated, respectively. Each of the 7A1-7A3 solutions contained 0.6 μL of the above drug conjugate working solution at one of the above 3 concentrations and 50 μL of Opti-MEM medium.

FormLaution of 7B solution: each 7B solution contained 1 μL of Lipofectamine™ 2000 and 50 μL of Opti-MEM medium.

One portion of 7B solution was respectively mixed with the resultant 7A1, 7A2 or 7A3 solution of each drug conjugate, and incubated at room temperature for 20 minutes respectively to obtain transfection complexes 7X1-7X3 of each siRNA conjugate.

One portion of 7B solution was mixed with 50 μL of Opti-MEM medium and incubated for 20 minutes at room temperature to obtain a transfection complex 7X4.

The transfection complexes 7X1-7X3 of each drug conjugate were respectively added to the culture wells in an amount of 100 μL/well and mixed well to obtain transfection complexes with the final concentration of each drug conjugate of about 5 nM, 0.5 nM and 0.05 nM, respectively. The transfection complexes 7X1-7X3 of each drug conjugate were respectively used to transfect three culture wells to obtain drug conjugate-containing transfection mixtures (designated as test group).

The transfection complex 7X4 was respectively added to another three culture wells in an amount of 100 μL/well

to obtain a siRNA-free transfection mixture (designated as blank control group).

After the drug conjugate-containing transfection mixtures and the drug conjugate-free transfection mixture were cultured in the culture wells for 4 hours, each well was supplemented with 1 mL of H-DMEM complete medium containing 20% FBS. The 24-well plate was placed in a $CO_2$ incubator and cultured for another 24 hours.

Subsequently, the total RNA in the cells in each well was extracted by using RNAVzol (purchased from Vigorous Biotechnology Beijing Co., Ltd., Cat. No. N002) according to the method as described in the instruction.

For the cells of each well, 1 μg of the total RNA was taken, and was formulated into a 20 μL reverse transcription reaction system for reverse transcription of the total RNA of the cells in each well by using the reagent provided in the reverse transcription kit GoldenstarTM RT6 cDNA Synthesis Kit (purchased from Beijing Tsingke Biotechnology Co., Ltd., Cat. No. TSK301M) according to the precedures for reverse transcription in the instruction of the kit, in which GoldenstarTM Oligo (dT)17 was selected as the primer. Conditions for reverse transcription were as follows: for each reverse transcription reaction system, the reverse transcription reaction system was incubated at 50°C for 50 minutes, then incubated at 85°C for 5 minutes, and finally incubated at 4°C for 30 seconds; after the reaction was completed, 80 μL of DEPC water was added to the reverse transcription reaction system to obtain a cDNA-containing solution.

For each reverse transcription reaction system, 5 μL of the above cDNA-containing solution was taken as the template, and was formulated into a 20 μL qPCR reaction system by using the reagent provided in the NovoStart® SYBR qPCR SuperMix Plus kit (purchased from Novoprotein Scientific Co., Ltd., Cat No. E096-01B), wherein the sequences of PCR primers for amplifying the target gene ANGPTL3 and the internal control gene β-actin are shown in Table 3F, and the final concentration of each primer was 0.25 μM. Each qPCR reaction system was placed in an ABI StepOnePlus Real-Time PCR Thermal Cycler, and the amplification was performed by a three-step method using the following amplification procedure: pre-denaturation at 95°C for 10 minutes, then denaturation at 95°C for 30 s, annealing at 60°C for 30 s, and extension at 72°C for 30 s (wherein the above process of denaturation, annealing and extension was repeated for 40 times), to obtain a product W containing the amplified target gene ANGPTL3 and the amplified internal control gene β-actin. The product W was then incubated at 95°C for 15 s, 60°C for 1 min, and 95°C for 15 s. The melting curves of the target gene ANGPTL3 and the internal control gene β-actin in the product W were respectively collected using a real-time fluorescent quantitative PCR Thermal Cycler, and the Ct values of the target gene ANGPTL3 and the internal control gene β-actin were obtained.

Table 3F Information of the primers

| Gene | Upstream Primers | Downstream Primers |
|---|---|---|
| Human ANGPTL3 | 5'-ACCAACTATACGCTACAT-3' (SEQ ID NO: 697) | 5'- CCTCCTGAATAACCCTCT -3' (SEQ ID NO: 698) |
| Human β-actin | 5'- CCAACCGCGAGAAGATGA - 3' (SEQ ID NO: 699) | 5'- CCAGAGGCGTACAGGGATAG -3' (SEQ ID NO: 700) |

The relative quantitative calculation of the target gene ANGPTL3 in each test group was carried out using the comparative Ct (ΔΔCt) method, and the calculation method was as follows:

$$\Delta Ct\ (\text{test group}) = Ct\ (\text{target gene of test group}) - Ct\ (\text{internal control gene of test group})$$

$$\Delta Ct\ (\text{control group}) = Ct\ (\text{target gene of control group}) - Ct\ (\text{internal control gene of control group})$$

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

wherein ΔCt (average of control group) was the arithmetic mean of the ΔCt (control group) of the three culture wells of the control group. Thus, each culture well in the test and control groups corresponded to a ΔΔCt value.

The expression levels of ANGPTL3 mRNA in the test groups were normalized based on the control group, and the expression level of ANGPTL3 mRNA in the blank control group was defined as 100%,

$$\text{Relative expression level of ANGPTL3 mRNA in the test group} = 2^{-\Delta\Delta Ct \text{ (the test group)}} \times 100\%.$$

$$\text{Inhibition rate against ANGPTL3 mRNA in the test group} = (1 - \text{the relative expression level of ANGPTL3 mRNA in the test group}) \times 100\%.$$

Table 4F summarized the inhibition rate of each siRNA against ANGPTL3 mRNA. For the siRNA in the same test group, the inhibition rate against the mRNA was the arithmetic mean of the inhibition rates against ANGPTL3 mRNA in the test group determined in the three culture wells.

Table 4F Inhibition rates of the drug conjugates at different concentrations against ANGPTL3 mRNA in Huh7 cells

| Conjugate Serial No. | Conjugate No. | Inhibition rate against mRNA (%) | | |
|---|---|---|---|---|
| | | 0.05 nM | 0.5 nM | 5 nM |
| Conjugate 121 | N6-siAN1 | 42.4 | 64.5 | 73.8 |
| Conjugate 122 | N6-siAN2 | 39.8 | 64.2 | 70.1 |
| Conjugate 123 | N6-siAN1-M1 | 42.1 | 62.6 | 75.4 |
| Conjugate 124 | N6-siAN2-M1 | 42.3 | 64.1 | 71.4 |
| Conjugate 125 | N6-siAN1-M2 | 44.5 | 60.3 | 75.6 |
| Conjugate 126 | N6-siAN2-M2 | 49.2 | 60.5 | 74.2 |
| Conjugate 127 | N6-siAN1-M3 | 51.3 | 57.3 | 78.1 |
| Conjugate 128 | N6-siAN2-M3 | 52.5 | 65.9 | 72.5 |
| Conjugate 129 | N6-siAN1-M1VP | 43.8 | 59.8 | 72.3 |
| Conjugate 130 | N6-siAN2-M1VP | 49.4 | 57.5 | 76.2 |
| Conjugate 131 | N6-siAN1-M2VP | 45.3 | 66.6 | 76.3 |
| Conjugate 132 | N6-siAN2-M2VP | 42.9 | 72.6 | 81.4 |
| Conjugate 133 | N6-siAN1-M3VP | 47.4 | 65.9 | 76.4 |
| Conjugate 134 | N6-siAN2-M3VP | 50.2 | 62.4 | 82.1 |
| Conjugate 135 | N6-siAN1-M1S | 42.9 | 62.1 | 71.5 |
| Conjugate 136 | N6-siAN2-M1S | 43.1 | 61.3 | 71.3 |
| Conjugate 137 | N6-siAN1-M2S | 45.8 | 70.1 | 67.2 |
| Conjugate 138 | N6-siAN2-M2S | 44.9 | 62.4 | 75.8 |
| Conjugate 139 | N6-siAN1-M3S | 48.4 | 71.0 | 78.3 |
| Conjugate 140 | N6-siAN2-M3S | 41.2 | 65.8 | 75.2 |
| Conjugate 141 | N6-siAN1-M1SVP | 52.5 | 68.8 | 80.1 |
| Conjugate 142 | N6-siAN2-M1SVP | 55.3 | 68.1 | 74.2 |
| Conjugate 143 | N6-siAN1-M2SVP | 52.3 | 67.5 | 76.4 |
| Conjugate 144 | N6-siAN2-M2SVP | 49.6 | 72.3 | 80.2 |
| Conjugate 145 | N6-siAN1-M3SVP | 49.3 | 72.4 | 76.4 |
| Conjugate 146 | N6-siAN1-M3SP | 48.8 | 70.3 | 76.5 |
| Conjugate 147 | N6-siAN1-M3SPs | 48.2 | 69.8 | 76.8 |
| Conjugate 148 | N6-siAN4-M3S | 42.5 | 67.4 | 75.4 |
| Conjugate 149 | N6-siAN2-M3SVP | 50.8 | 78.1 | 80.8 |

(continued)

| Conjugate Serial No. | Conjugate No. | Inhibition rate against mRNA (%) | | |
|---|---|---|---|---|
| | | 0.05 nM | 0.5 nM | 5 nM |
| Conjugate 150 | N6-siAN1-M4S | 35.1 | 48.5 | 62.3 |
| Conjugate 151 | N6-siAN1-M4SVP | 32.4 | 47.6 | 62.1 |
| Conjugate 152 | N6-siAN1-M5S | 33.1 | 52.3 | 60.8 |
| Conjugate 153 | N6-siAN1-M5SVP | 32.5 | 51.8 | 63.4 |
| Conjugate 154 | X2-siAN1-M3SVP | 49.6 | 67.4 | 75.6 |
| Conjugate 155 | W2-siAN1-M3SVP | 48.6 | 68.2 | 74.8 |
| Conjugate 156 | V2-siAN1-M3SVP | 50.2 | 70.1 | 74.9 |
| Conjugate 157 | 02-siAN1-M3SVP | 53.6 | 67.2 | 75.2 |
| Conjugate 158 | P2-siAN1-M3SVP | 54.1 | 69.3 | 76.2 |
| Comparative Conjugate 1 | N6-NC | 3.6 | 8.2 | 17.1 |

As can be seen from the results of Table 4F, at each concentration, the drug conjugates of the present disclosure showed excellent inhibitory activity against the expression of ANGPTL3 mRNA at the cellular level. At a concentration of 5 nM, the drug conjugates showed an inhibition rate of up to more than 60%, with some of the conjugates exhibiting an inhibition rate of up to more than 80%.

**Experimental Example 7-2 This experiment illustrates the determination of the stability of the drug conjugates in Table 2F in human plasma.**

Conjugates 123-158 and Comparative Conjugate 1 were respectively formulated intio 20 μM solutions (calculated based on siRNA) with DEPC water. Each of the above Conjugates 123-158 and Comparative Conjugate 1 (provided as 20 μM solution, 12 μL per group) was quickly mixed well with 108 μL of 90% human plasma (available from Jiangsu Institute of Hematology, and diluted with 1×PBS (pH 7.4)) to obtain a mixed solution. The mixed solution was incubated at a constant temperature of 37°C. During the incubation, 10 μL of the sample was taken at 0 h, 8 h, 24 h and 48 h, respectively, and immediately frozen in liquid nitrogen and cryopreserved in a -80°C freezer. Therein, 0 hour refers to the time when 10 μL of the mixed solution was taken immediately after the conjugate solution was mixed well with 90% human plasma. After sampling at each time point, each mixed solution was diluted 5-fold with 1×PBS (pH 7.4) and then taken in a volume of 10 μL for electrophoresis. Meanwhile, an equimolar amount of each of the solutions of Conjugates 123-158 (2 μL, the concentration is 2 μM calculated based on siRNA) was mixed well with 8 μL of 1 × PBS (pH 7.4) respectively to prepare 10 μL of sample untreated with human plasma (designated as "untreated") for electrophoresis.

20 wt% of non-denatured polyacrylamide gel was prepared. The above samples for electrophoresis in each group were respectively mixed well with 4 μL of loading buffer (20 mM EDTA, 36 wt% glycerol, and 0.06 wt% bromophenol blue) and then loaded into each gel hole for electrophoresis under 80 mA constant current for about 60 minutes. After electrophoresis, the gel was taken out, stained with 1×Sybr Gold dye (Invitrogen, Cat. 11494) for 15 minutes for imaging. The stability results were calculated.

Table 5F shows the semiquantitative test result of the stability of the drug conjugates listed in Table 2F and the comparative conjugate in human plasma *in vitro.* The result is expressed as the ratio (RL) of the longest fragment remaining after the incubation of the drug conjugates and the comparative conjugate with human plasma to the longest fragment of untreated siRNA.

Table 5F The plasma stability of different drug conjugates over time

| Conjugate Serial No. | Conjugate No. | RL (%) | | | | |
|---|---|---|---|---|---|---|
| | | Untreated | 0h | 8h | 24h | 48h |
| Conjugate 123 | N6-siAN1-M1 | 100 | 100 | 97.1 | 95.4 | 95.1 |
| Conjugate 124 | N6-siAN2-M1 | 100 | 100 | 97.3 | 95.2 | 96.3 |
| Conjugate 125 | N6-siAN1-M2 | 100 | 100 | 97.4 | 95.3 | 95.2 |

(continued)

| Conjugate Serial No. | Conjugate No. | RL (%) | | | | |
|---|---|---|---|---|---|---|
| | | Untreated | 0h | 8h | 24h | 48h |
| Conjugate 126 | N6-siAN2-M2 | 100 | 100 | 97.6 | 96.4 | 95.1 |
| Conjugate 127 | N6-siAN1-M3 | 100 | 100 | 95.1 | 94.3 | 95.3 |
| Conjugate 128 | N6-siAN2-M3 | 100 | 100 | 96.3 | 98.0 | 95.3 |
| Conjugate 129 | N6-siAN1-M1VP | 100 | 100 | 95.2 | 96.1 | 94.8 |
| Conjugate 130 | N6-siAN2-M1VP | 100 | 100 | 96.3 | 95.2 | 95.9 |
| Conjugate 131 | N6-siAN1-M2VP | 100 | 100 | 95.8 | 95.3 | 95.2 |
| Conjugate 132 | N6-siAN2-M2VP | 100 | 100 | 97.4 | 95.1 | 95.1 |
| Conjugate 133 | N6-siAN1-M3VP | 100 | 100 | 95.5 | 95.1 | 96.2 |
| Conjugate 134 | N6-siAN2-M3VP | 100 | 100 | 95.6 | 95.2 | 95.6 |
| Conjugate 135 | N6-siAN1-M1S | 100 | 100 | 97.1 | 94.3 | 95.1 |
| Conjugate 136 | N6-siAN2-M1S | 100 | 100 | 98.2 | 95.2 | 96.1 |
| Conjugate 137 | N6-siAN1-M2S | 100 | 100 | 98.1 | 98.4 | 95.0 |
| Conjugate 138 | N6-siAN2-M2S | 100 | 100 | 997.3 | 95.1 | 95.1 |
| Conjugate 139 | N6-siAN1-M3S | 100 | 100 | 95.4 | 94.2 | 95.3 |
| Conjugate 140 | N6-siAN2-M3S | 100 | 100 | 94.5 | 96.3 | 95.4 |
| Conjugate 141 | N6-siAN1-M1SVP | 100 | 100 | 98.1 | 96.1 | 97.2 |
| Conjugate 142 | N6-siAN2-M1SVP | 100 | 100 | 98.3 | 96.2 | 98.1 |
| Conjugate 143 | N6-siAN1-M2SVP | 100 | 100 | 98.4 | 96.4 | 97.1 |
| Conjugate 144 | N6-siAN2-M2SVP | 100 | 100 | 97.8 | 95.5 | 98.1 |
| Conjugate 145 | N6-siAN1-M3SVP | 100 | 100 | 97.1 | 96.5 | 97.4 |
| Conjugate 146 | N6-siAN1-M3SP | 100 | 100 | 96.5 | 96.2 | 98.3 |
| Conjugate 147 | N6-siAN1-M3SPs | 100 | 100 | 97.2 | 97.3 | 98.5 |
| Conjugate 148 | N6-siAN4-M3S | 100 | 100 | 97.5 | 95.8 | 99.1 |
| Conjugate 149 | N6-siAN2-M3SVP | 100 | 100 | 98.7 | 97.2 | 99.1 |
| Conjugate 150 | N6-siAN1-M4S | 100 | 100 | 98.4 | 98.3 | 99.2 |
| Conjugate 151 | N6-siAN1-M4SVP | 100 | 100 | 98.3 | 98.2 | 98.5 |
| Conjugate 152 | N6-siAN1-M5S | 100 | 100 | 99.1 | 98.1 | 98.1 |
| Conjugate 153 | N6-siAN1-M5SVP | 100 | 100 | 98.6 | 98.2 | 94.1 |
| Conjugate 154 | X2-siAN1-M3SVP | 100 | 100 | 95.9 | 98.2 | 98.2 |
| Conjugate 155 | W2-siAN1-M3SVP | 100 | 100 | 98.2 | 97.6 | 95.3 |
| Conjugate 156 | V2-siAN1-M3SVP | 100 | 100 | 97.9 | 94.4 | 95.2 |
| Conjugate 157 | 02-siAN1-M3SVP | 100 | 100 | 98.3 | 95.4 | 95.6 |
| Conjugate 158 | P2-siAN1-M3SVP | 100 | 100 | 98.5 | 95.2 | 96.8 |
| Comparative Conjugate 1 | N6-NC | 100 | 0 | 0 | 0 | 0 |

As can be seen from the results of Table 5E, the drug conjugates of the present disclosure exhibited excellent stability in plasma, and still showed a length ratio of the siRNA fragments of higher than 94% at 48 h.

**Experimental example 7-3 This experiment illustrates the inhibitory efficiency of the drug conjugates in Table 2F against the expression level of ANGPTL3 mRNA *in vivo.***

In this Experimental Example, the *in vivo* inhibition of the expression level of ANGPTL3 by Conjugates 141, 144, 145-147 and 154-158 in liver tissue of BALB/c mice was investigated.

BALB/c mice (6-8 week old, purchased from Beijing Vital River Laboratory Animal Technology Co., Ltd.) were randomly divided into groups (six mice per group, half male and half female) according to body weights, and each group of mice was numbered according to the drug conjugates in Table 2F. Then, each group of mice was respectively administered with each of test Conjugates 141, 144, 145-147 and 154-158. All animals were dosed based on body weight and was administered at a single dose by subcutaneous injection. Each conjugate was administered in the form of 0.3 mg/mL in 0.9% NaCl aqueous solution, with an administration volume of 10 mL/kg; that is, the administration doses of each conjugate were 3 mg/kg.

Each mouse in another group was administered with 1×PBS with an administration volume of 10 mL/kg mouse body weight, as a control group.

On day 14 after administrion, the animals were sacrificed, and the liver tissue of each mouse was collected separately and stored in RNA later (Sigma Aldrich Crop.). The liver tissue was homogenized with a tissue homogenizer and then extracted with Trizol (Thermo Fisher company) according to the operation procedures described in the instruction to obtain the total RNA.

For the liver tissue of each mouse, 1 μg of total RNA was taken, and the extracted total RNA was reverse-transcribed into cDNA using ImProm-II™ reverse transcription kit (Promega Crop.) according to the instructions thereof to obtain a solution containing cDNA, and then the expression level of ANGPTL3 mRNA in liver tissue was determined by the fluorescent quantitative PCR kit (Beijing Cowin Biosciences Co., Ltd.). In this fluorescent quantitative PCR method, β-actin gene was used as an internal control gene, and ANGPTL3 and β-actin were detected by using primers for ANGPTL3 and β-actin, respectively. The sequences of the primers for detection are shown in Table 6F.

The relative quantitative calculation of the expression level of the target ANGPTL3 gene in each test group and the control group was carried out using the comparative Ct (ΔΔCt) method, and the calculation method was as follows:

$$\Delta Ct\ (\text{test group}) = Ct\ (\text{target gene of test group}) - Ct\ (\text{internal control gene of test group})$$

$$\Delta Ct\ (\text{control group}) = Ct\ (\text{target gene of control group}) - Ct\ (\text{internal control gene of control group})$$

$$\Delta\Delta Ct\ (\text{test group}) = \Delta Ct\ (\text{test group}) - \Delta Ct\ (\text{average of control group})$$

$$\Delta\Delta Ct\ (\text{control group}) = \Delta Ct\ (\text{control group}) - \Delta Ct\ (\text{average of control group})$$

wherein ΔCt (average of control group) was the arithmetic mean of the ΔCt (control group) of the six mice in the control group. Thus, each mouse in the test and control groups corresponded to a ΔΔCt value.

The expression level of ANGPTL3 mRNA in the test group was normalized based on the control group, and the expression level of ANGPTL3 mRNA in the control group was defined as 100%,

$$\text{Relative expression level of ANGPTL3 mRNA in the test group} = 2^{-\Delta\Delta Ct\ (\text{the test group})} \times 100\%.$$

For the siRNA in the same test group, the mean of the relative expression level of ANGPTL3 mRNA in the test group at each concentration was the arithmetic mean of the relative expression levels in the six mice at that concentration.

Therein, the control group refers to the mice in the control group administered with PBS in this experiment, and each test group refers to the mice in the administration group adminstered with different drug conjugates. The results are shown in Table 7F below.

Table 6F The sequences of the primers

| Gene | Upstream primers | Downstream primers |
| --- | --- | --- |
| Mouse ANGPTL3 | 5'-GAGGAGCAGCTAACCAACTTAAT-3' (SEQ ID NO: 701) | 5'-TCTGCATGTGCTGTTGACTTAAT-3' (SEQ ID NO: 702) |
| Mouse β-actin | 5'-AGCTTCTTTGCAGCTCCTTCGTTG-3' (SEQ ID NO: 703) | 5'-TTCTGACCCATTCCCACCATCACA-3' (SEQ ID NO: 704) |

Table 7F Inhibition rates of the drug conjugates against the expression of ANGPTL3 mRNA in mouse liver

| Conjugate Serial No. | Conjugate No. | Inhibition rate against ANGPTL3 mRNA in liver (%) |
|---|---|---|
| -- | PBS | 0 |
| Conjugate 141 | N6-siAN1-M1SVP | 93.4 |
| Conjugate 144 | N6-siAN2-M2SVP | 93.1 |
| Conjugate 145 | N6-siAN1-M3SVP | 92.5 |
| Conjugate 146 | N6-siAN1-M3SP | 92.9 |
| Conjugate 147 | N6-siAN1-M3SPs | 93.6 |
| Conjugate 154 | X2-siAN1-M3SVP | 92.1 |
| Conjugate 155 | W2-siAN1-M3SVP | 92.7 |
| Conjugate 156 | V2-siAN1-M3SVP | 93.3 |
| Conjugate 157 | 02-siAN1-M3SVP | 91.8 |
| Conjugate 158 | P2-siAN1-M3SVP | 92.5 |

As can be seen from the results in Table 7F, compared with PBS, the drug conjugates of the present disclosure showed higher inhibitory activity against ANGPTL3 mRNA, and could exhibit an inhibition rate of at least 91.8% and up to 93.6% against ANGPTL3 mRNA.

**Experimental example 7-4 This experiment illustrates the inhibition efficiency of the drug conjugates in Table 2F against the expression level of ANGPTL3 mRNA *in vivo* and the effects thereof on blood lipid.**

In this Experimental Example, the *in vivo* inhibition rates of the drug conjugates Conjugate 141 (N6-siAN1M1SVP) and Conjugates 145-147 (N6-siAN1-M3SVP, N6-siAN1-M3SP and N6-siAN1-M3SPs) against the expression level of ANGPTL3 in the liver tissue of ob/ob model mice, and their effects on the contents of total cholesterol (CHO), triglyceride (TG) and low-density lipoprotein cholesterol (LDL-c) in the serum were investigated.

Ob/ob mice (6-8 week old, purchased from Changzhou Cavens Laboratory Animal Co., Ltd) were randomly divided into 9 groups (five mice per group): (1) PBS control group; (2) 3 mg/kg N6-siAN1-M1SVP group; (3) 3 mg/kg N6-siAN1-M3SVP group; (4) 3 mg/kg N6-siAN1-M3SP group; 5) 3 mg/kg N6-siAN1-M3SPs group; (6) 1 mg/kg N6-siAN1-M1SVP group; 7) 1 mg/kg N6-siAN1-M3SVP group; (8) 1 mg/kg N6-siAN1-M3SP group; and 9) 1 mg/kg N6-siAN1-M3SPs group. All animals were dosed based on body weight and was administered at a single dose by subcutaneous injection, with an administration volume of 10 mL/kg.

About 100 μL orbital blood was taken 2 days before the administration (marked as day -2) and on day 7, 14, 21, 28, 35, 42, and 49 after administration for determining blood lipid levels.

On day 49 after administrion, the mice were sacrificed, nad the liver was collected and stored in RNA later (Sigma Aldrich Crop.). The liver tissue was homogenized with a tissue homogenizer and then extracted with Trizol (Thermo Fisher company) according to the standard operation procedures for total RNA extraction to obtain the total RNA.

The expression level of ANGPTL3 mRNA in liver tissue was determined by real-time fluorescent quantitative PCR using the same method as that in Experimental Example 7-3. The results are shown in Table 8F below.

Table 8F Inhibition rates of the drug conjugates against the expression of ANGPTL3 mRNA in mouse liver

| Conjugate Serial No. | Conjugate No. | Administration dose (mg/kg) | Inhibition rate against ANGPTL3 mRNA in liver (%) |
|---|---|---|---|
| -- | PBS | -- | 0 |
| Conjugate 141 | N6-siAN1-M1SVP | 3 | 86.4 |
| Conjugate 145 | N6-siAN1-M3SVP | 3 | 77.6 |
| Conjugate 146 | N6-siAN1-M3SP | 3 | 76.9 |
| Conjugate 147 | N6-siAN1-M3SPs | 3 | 77.2 |

(continued)

| Conjugate Serial No. | Conjugate No. | Administration dose (mg/kg) | Inhibition rate against ANGPTL3 mRNA in liver (%) |
|---|---|---|---|
| Conjugate 141 | N6-siAN1-M1SVP | 1 | 53.2 |
| Conjugate 145 | N6-siAN1-M3SVP | 1 | 56.4 |
| Conjugate 146 | N6-siAN1-M3SP | 1 | 56.2 |
| Conjugate 147 | N6-siAN1-M3SPs | 1 | 56.0 |

The blood collected from orbit was centrifuged to obtain serum. The contents of total cholesterol (CHO), triglyceride (TG) and low-density lipoprotein cholesterol (LDL-c) in serum were further determined by using a PM1P000/3 full-automatic serum biochemical analyzer (SABA, Italy). The results of blood lipid were normalized and the inhibition ratio against blood lipid levels was calculated by the equation:

$$\text{Inhibition ratio} = (1-\text{the blood lipid content in the test group after administration/the blood lipid content in the test group before administration})\times 100\%.$$

The blood lipid refers to total cholesterol, triglyceride or low-density lipoprotein cholesterol.

The determination results are shown in Tables 9F, 10F and 11F below.

Table 9F The effects of the drug conjugates on the expression level of total cholesterol in mouse serum

| Conjugate Serial No. | Conjugate No. | Administration dose (mg/kg) | CHO (normalized, the data of Day -2 being defined as 1) | | | | | | | |
|---|---|---|---|---|---|---|---|---|---|---|
| | | | Day -2 | Day 7 | Day 14 | Day 21 | Day 28 | Day 35 | Day 42 | Day 49 |
| -- | PBS | -- | 1.00 | 1.07 | 1.41 | 1.06 | 0.97 | 0.97 | 1.07 | 1.05 |
| Conjugate 141 | N6-siAN1-M1SVP | 3 | 1.00 | 0.22 | 0.24 | 0.22 | 0.28 | 0.33 | 0.41 | 0.49 |
| Conjugate 145 | N6-siAN1-M3SVP | 3 | 1.00 | 0.21 | 0.23 | 0.24 | 0.30 | 0.36 | 0.42 | 0.50 |
| Conjugate 146 | N6-siAN1-M3SP | 3 | 1.00 | 0.22 | 0.22 | 0.23 | 0.31 | 0.37 | 0.43 | 0.49 |
| Conjugate 147 | N6-siAN1-M3 SPs | 3 | 1.00 | 0.21 | 0.22 | 0.24 | 0.31 | 0.36 | 0.43 | 0.50 |
| Conjugate 141 | N6-siAN1-M1SVP | 1 | 1.00 | 0.27 | 0.33 | 0.32 | 0.38 | 0.42 | 0.52 | 0.58 |
| Conjugate 145 | N6-siAN1-M3SVP | 1 | 1.00 | 0.31 | 0.35 | 0.36 | 0.42 | 0.44 | 0.54 | 0.52 |
| Conjugate 146 | N6-siAN1-M3SP | 1 | 1.00 | 0.30 | 0.36 | 0.35 | 0.41 | 0.43 | 0.53 | 0.53 |
| Conjugate 147 | N6-siAN1-M3 SPs | 1 | 1.00 | 0.31 | 0.35 | 0.35 | 0.42 | 0.43 | 0.52 | 0.51 |

Table 10F The effects of the drug conjugates on the expression level of triglyceride in mouse serum

| Conjugate Serial No. | Conjugate No. | Administration dose(mg/kg) | TG (normalized, the data of Day -2 being defined as 1) | | | | | | | |
|---|---|---|---|---|---|---|---|---|---|---|
| | | | Day -2 | Day 7 | Day 14 | Day 21 | Day 28 | Day 35 | Day 42 | Day 49 |
| -- | PBS | -- | 1.00 | 1.04 | 1.23 | 0.94 | 0.95 | 1.04 | 0.98 | 1.01 |
| Conjugate 141 | N6-siAN1-M1SVP | 3 | 1.00 | 0.17 | 0.21 | 0.19 | 0.26 | 0.25 | 0.28 | 0.38 |
| Conjugate 145 | N6-siAN1-M3SVP | 3 | 1.00 | 0.20 | 0.18 | 0.23 | 0.24 | 0.28 | 0.31 | 0.37 |
| Conjugate 146 | N6-siAN1-M3 SP | 3 | 1.00 | 0.21 | 0.17 | 0.24 | 0.23 | 0.27 | 0.30 | 0.38 |
| Conjugate 147 | N6-siAN1-M3 SPs | 3 | 1.00 | 0.20 | 0.18 | 0.25 | 0.24 | 0.28 | 0.29 | 0.37 |
| Conjugate 141 | N6-siAN1-M1SVP | 1 | 1.00 | 0.25 | 0.26 | 0.28 | 0.33 | 0.31 | 0.45 | 0.47 |
| Conjugate 145 | N6-siAN1-M3SVP | 1 | 1.00 | 0.25 | 0.27 | 0.32 | 0.39 | 0.40 | 0.41 | 0.49 |
| Conjugate 146 | N6-siAN1-M3 SP | 1 | 1.00 | 0.26 | 0.28 | 0.33 | 0.40 | 0.41 | 0.40 | 0.50 |
| Conjugate 147 | N6-siAN1-M3 SPs | 1 | 1.00 | 0.24 | 0.29 | 0.34 | 0.39 | 0.42 | 0.40 | 0.49 |

Table 11F The effects of the drug conjugates on the expression level of low-density lipoprotein cholesterol in mouse serum

| Conjugate Serial No. | Conjugate No. | Administration dose(mg/kg) | LDL-c (normalized, the data of Day -2 being defined as 1) | | | | | | | |
|---|---|---|---|---|---|---|---|---|---|---|
| | | | Day -2 | Day 7 | Day 14 | Day 21 | Day 28 | Day 35 | Day 42 | Day 49 |
| -- | PBS | -- | 1.00 | 1.04 | 1.02 | 0.97 | 1.08 | 1.04 | 1.02 | 1.03 |
| Conjugate 141 | N6-siAN1-M1SVP | 3 | 1.00 | 0.24 | 0.26 | 0.24 | 0.33 | 0.35 | 0.34 | 0.44 |
| Conjugate 145 | N6-siAN1-M3SVP | 3 | 1.00 | 0.25 | 0.27 | 0.28 | 0.31 | 0.35 | 0.36 | 0.44 |
| Conjugate 146 | N6-siAN1-M3SP | 3 | 1.00 | 0.24 | 0.27 | 0.29 | 0.32 | 0.35 | 0.36 | 0.45 |
| Conjugate 147 | N6-siAN1-M3 SPs | 3 | 1.00 | 0.25 | 0.25 | 0.29 | 0.31 | 0.36 | 0.36 | 0.46 |
| Conjugate 141 | N6-siAN1-M1SVP | 1 | 1.00 | 0.34 | 0.36 | 0.34 | 0.44 | 0.48 | 0.48 | 0.54 |
| Conjugate 145 | N6-siAN1-M3SVP | 1 | 1.00 | 0.37 | 0.37 | 0.38 | 0.41 | 0.47 | 0.46 | 0.56 |
| Conjugate 146 | N6-siAN1-M3SP | 1 | 1.00 | 0.38 | 0.38 | 0.36 | 0.42 | 0.48 | 0.47 | 0.55 |
| Conjugate 147 | N6-siAN1-M3 SPs | 1 | 1.00 | 0.37 | 0.37 | 0.37 | 0.43 | 0.48 | 0.46 | 0.55 |

As can be seen from the results of Table 9F, Table 10F, and Table 11F above, the different doses of the drug

conjugate Conjugate 141, Conjugate 145, Conjugate 146 or Conjugate 147 can significantly inhibit the expression of ANGPTL3 in mouse liver tissue, and have a significant dose-dependent response. The drug conjugate Conjugate 141 (N6-siAN1-M1SP) showed an inhibition rate of 53.2% against the expression of ANGPTL3 gene at a low dose of 1 mg/kg, and showed an inhibition rate of up to 86.4% against the expression of ANGPTL3 gene at a high dose of 3 mg/kg. Meanwhile, the contents of CHO, TG and LDL-c in the serum of mice were monitored, and the results showed that in the serum of mice treated with the drug conjugate Conjugate 141, Conjugate 145, Conjugate 146 or Conjugate 147, the contents of CHO, TG and LDL-c were reduced significantly, and a higher effect of reducing blood lipid level can still be observed at least on Day 49.

**Experimental Example 8 Effect experiments of the drug conjugates in Table 2G**

**Experimental Example 8-1 This experiment illustrates the determination of the inhibitory efficiency of the drug conjugates in Table 2G against the expression level of APOC3 mRNA in Huh7 cells.**

The Huh7 cells were seeded into a 24-well plate at $7.5 \times 10^4$ cells/well with H-DMEM complete medium. After 16 hours, when the cell growth density reached 70-80%, the H-DMEM complete medium in the culture well was aspirated, and 500 μL Opti-MEM medium (GIBCO company) was added to each well, and the cells were cultured for another 1.5 hours.

The drug conjugates below were respectively formulated into working solutions of drug conjugate at 3 different concentrations of 5 μM, 0.5 μM and 0.05 μM with DEPC water (calculated based on siRNA), wherein the drug conjugates were Conjugates 159-183 in Table 2G and Comparative Conjugate 1 in Table 2A, and the Comparative Conjugate 1 was used as negative control.

Formulation of 8A1-8A3 solutions: for each drug conjugate, 8A1, 8A2 and 8A3 solutions were formulated, respectively. Each of the 8A1-8A3 solutions contained 0.6 μL of the above drug conjugate working solution at one of the above 3 concentrations and 50 μL of Opti-MEM medium.

FormLaution of 8B solution: each 8B solution contained 1 μL of Lipofectamine™ 2000 and 50 μL of Opti-MEM medium.

One portion of 8B solution was respectively mixed with the resultant 8A1, 8A2 or 8A3 solution of each drug conjugate, and incubated at room temperature for 20 minutes respectively to obtain transfection complexes 8×1-8×3 of each drug conjugate.

One portion of 8B solution was mixed with 50 μL of Opti-MEM medium and incubated for 20 minutes at room temperature to obtain a transfection complex 8X4.

The transfection complexes 8X1-8X3 of each drug conjugate were respectively added to the culture wells in an amount of 100 μL/well and mixed well to obtain transfection complexes with the final concentration of each drug conjugate of about 5 nM, 0.5 nM and 0.05 nM respectively (calculated based on siRNA). The transfection complexes 8X1-8X3 of each drug conjugate were respectively used to transfect three culture wells to obtain siRNA-containing transfection mixtures (designated as test group).

The transfection complex 8X4 was respectively added to another three culture wells in an amount of 100 μL/well to obtain a drug conjugate-free transfection mixture (designated as blank control group).

After the drug conjugate-containing transfection mixtures and the drug conjugate-free transfection mixture were cultured in the culture wells for 4 hours, each well was supplemented with 1 mL of H-DMEM complete medium containing 20% FBS. The 24-well plate was placed in a $CO_2$ incubator and cultured for another 24 hours.

Subsequently, the total RNA in the cells in each well was extracted by using RNAVzol (purchased from Vigorous Biotechnology Beijing Co., Ltd., Cat. No. N002) according to the method as described in the instruction.

For the cells of each well, 1 μg of the total RNA was taken, and was formulated into a 20 μL reverse transcription reaction system for reverse transcription of the total RNA of the cells in each well by using the reagent provided in the reverse transcription kit GoldenstarTM RT6 cDNA Synthesis Kit (purchased from Beijing Tsingke Biotechnology Co., Ltd., Cat. No. TSK301M) according to the precedures for reverse transcription in the instruction of the kit, in which GoldenstarTM Oligo (dT)17 was selected as the primer. Conditions for reverse transcription were as follows: for each reverse transcription reaction system, the reverse transcription reaction system was incubated at 50°C for 50 minutes, then incubated at 85°C for 5 minutes, and finally incubated at 4°C for 30 seconds; after the reaction was completed, 80 μL of DEPC water was added to the reverse transcription reaction system to obtain a cDNA-containing solution.

For each reverse transcription reaction system, 5 μL of the above cDNA-containing solution was taken as the template, and was formulated into a 20 μL qPCR reaction system by using the reagent provided in the NovoStart® SYBR qPCR SuperMix Plus kit (purchased from Novoprotein Scientific Co., Ltd., Cat No. E096-01B), wherein the sequences of PCR primers for amplifying the target gene APOC3 and the internal control gene β-actin are shown in Table 3G, and the final concentration of each primer was 0.25 μM. Each qPCR reaction system was placed in an ABI StepOnePlus Real-Time PCR Thermal Cycler, and the amplification was performed by a three-step method using the following amplification procedure: pre-denaturation at 95°C for 10 minutes, then denaturation at 95°C for 30 s, annealing at 60°C for 30 s, and extension at 72°C for 30 s (wherein the above process of denaturation, annealing and extension was repeated for 40 times), to obtain a product W containing the amplified target gene APOC3 and the amplified internal control gene β-actin. The product W was then incubated at 95°C for 15 s, 60°C for 1 min, and 95°C for 15 s. The melting curves of the target gene APOC3 and the internal control gene β-actin in the product W were respectively collected using a real-time fluorescent quantitative PCR Thermal Cycler, and the Ct values of the target gene APOC3 and the internal control gene β-actin were obtained.

Table 3G Information of the primers

| Gene | Upstream primers | Downstream primers |
|---|---|---|
| Human APOC3 | 5'- GTGACCGATGGCTTCAGTTC -3' (SEQ ID NO: 705) | 5'- ATGGATAGGCAGGTGGACTT -3' (SEQ ID NO: 706) |
| Human β-actin | 5'- CCAACCGCGAGAAGATGA - 3' (SEQ ID NO: 707) | 5'- CCAGAGGCGTACAGGGATAG -3' (SEQ ID NO: 708) |

The relative quantitative calculation of the target gene APOC3 in each test group was carried out using the comparative Ct (ΔΔCt) method, and the calculation method was as follows:

$$\Delta Ct \text{ (test group)} = Ct \text{ (target gene of test group)} - Ct \text{ (internal control gene of test group)}$$

$$\Delta Ct \text{ (control group)} = Ct \text{ (target gene of control group)} - Ct \text{ (internal control gene of control group)}$$

$$\Delta\Delta Ct \text{ (test group)} = \Delta Ct \text{ (test group)} - \Delta Ct \text{ (average of control group)}$$

$$\Delta\Delta Ct \text{ (control group)} = \Delta Ct \text{ (control group)} - \Delta Ct \text{ (average of control group)}$$

wherein ΔCt (average of control group) was the arithmetic mean of the ΔCt (control group) of the three culture wells of the control group. Thus, each culture well in the test and control groups corresponded to a ΔΔCt value.

The expression levels of APOC3 mRNA in the test groups were normalized based on the control group, and the expression level of APOC3 mRNA in the blank control group was defined as 100%,

$$\text{Relative expression level of APOC3 mRNA in the test group} = 2^{-\Delta\Delta Ct \text{ (the test group)}} \times 100\%.$$

$$\text{Inhibition rate against APOC3 mRNA in the test group} = (1 - \text{the relative expression level of APOC3 mRNA in the test group}) \times 100\%.$$

Table 4G summarized the inhibition rate of each drug conjugate against APOC3 mRNA. For the drug conjugates in the same test group, the inhibition rate against the mRNA was the arithmetic mean of the inhibition rates against APOC3 mRNA in the test group determined in the three culture wells.

Table 4G Inhibition rates of the drug conjugates at different concentrations against APOC3 mRNA in Huh7 cells

| Conjugate Serial No. | Conjugate No. | Inhibition rate against mRNA (%) | | |
|---|---|---|---|---|
| | | 5 nM | 0.5 nM | 0.05 nM |
| Conjugate 159 | N6-siAP 1 | 53.3 | 33.3 | 12.5 |
| Conjugate 160 | N6-siAP2 | 54.1 | 35.4 | 13.6 |

(continued)

| Conjugate Serial No. | Conjugate No. | Inhibition rate against mRNA (%) | | |
|---|---|---|---|---|
| | | 5 nM | 0.5 nM | 0.05 nM |
| Conjugate 161 | N6-siAP1-M1 | 53.5 | 35.2 | 13.7 |
| Conjugate 162 | N6-siAP2-M1 | 55.2 | 33.1 | 12.8 |
| Conjugate 163 | N6-siAP1-M2 | 55.1 | 34.5 | 14.4 |
| Conjugate 164 | N6-siAP2-M2 | 57.6 | 34.2 | 15.1 |
| Conjugate 165 | N6-siAP1-M1VP | 53.2 | 34.3 | 15.0 |
| Conjugate 166 | N6-siAP2-M1VP | 57.4 | 32.8 | 14.6 |
| Conjugate 167 | N6-siAP1-M2VP | 56.2 | 33.4 | 16.3 |
| Conjugate 168 | N6-siAP2-M2VP | 58.3 | 32.7 | 13.4 |
| Conjugate 169 | N6-siAP1-M1S | 57.1 | 41.2 | 16.8 |
| Conjugate 170 | N6-siAP2-M1S | 57.3 | 38.7 | 16.1 |
| Conjugate 171 | N6-siAP1-M2S | 60.2 | 43.1 | 17.4 |
| Conjugate 172 | N6-siAP2-M2S | 57.5 | 37.6 | 15.4 |
| Conjugate 173 | N6-siAP1-M1SVP | 57.8 | 41.2 | 15.2 |
| Conjugate 174 | N6-siAP2-M1SVP | 58.2 | 42.6 | 17.3 |
| Conjugate 175 | N6-siAP1-M2SVP | 59.5 | 43.2 | 20.1 |
| Conjugate 176 | N6-siAP1-M2SP | 59.1 | 42.2 | 20.5 |
| Conjugate 177 | N6-siAP1-M2SPs | 57.4 | 41.3 | 20.8 |
| Conjugate 178 | N6-siAP4-M2S | 57.1 | 38.5 | 21.9 |
| Conjugate 179 | X2-siAP1-M2SVP | 57.8 | 39.8 | 18.3 |
| Conjugate 180 | W2-siAP1-M2SVP | 56.4 | 40.2 | 21.1 |
| Conjugate 181 | V2-siAP1-M2SVP | 61.3 | 41.5 | 18.9 |
| Conjugate 182 | 02-siAP1-M2SVP | 59.4 | 40.3 | 18.4 |
| Conjugate 183 | P2-siAP1-M2SVP | 57.7 | 44.1 | 19.6 |
| Comparative Conjugate 1 | N6-NC | 7.8 | 3.1 | 0.2 |

As can be seen from the results of Table 4G, at each concentration, the drug conjugates of the present disclosure showed excellent inhibitory activity against the expression of APOC3 mRNA at the cellular level. At a concentration of 5 nM, the drug conjugates showed an inhibition rate of up to more than 53.2%, with some of the conjugates exhibiting an inhibition rate of up to more than 61%.

**Experimental Example 8-2 This experiment illustrates the stability of the drug conjugates in Table 2G in human plasma *in vitro.***

Conjugates 161-183 and Comparative Conjugate 1 were respectively formulated intio 20 μM solutions (calculated based on siRNA) with DEPC water.

Each of the above Conjugates 161-183 and Comparative Conjugate 1 (provided as 20 μM solution, 12 μL per group) was quickly mixed well with 108 μL of 90% human plasma (available from Jiangsu Institute of Hematology, and diluted with 1×PBS (pH 7.4)) to obtain a mixed solution. The mixed solution was incubated at a constant temperature of 37°C. During the incubation, 10 μL of the sample was taken at 0 h, 8 h, 24 h and 48 h, respectively, and immediately frozen in liquid nitrogen and cryopreserved in a -80°C freezer. Therein, 0 hour refers to the time when 10 μL of the mixed solution was taken immediately after the conjugate solution was mixed well with 90% human plasma. After sampling at each time point, each mixed solution was diluted 5-fold with 1×PBS (pH 7.4) and then taken in a volume of 10 μL for

electrophoresis. Meanwhile, an equimolar amount of each of the solutions of Conjugates 161-183 (2 μM, 2 μL) was mixed well with 8 μL of 1× PBS (pH 7.4) respectively to prepare 10 μL of sample untreated with human plasma (designated as "untreated") for electrophoresis.

20 wt% of non-denatured polyacrylamide gel was prepared. The above samples for electrophoresis in each group were mixed well with 4 μL of loading buffer (20 mM EDTA, 36 wt% glycerol, and 0.06 wt% bromophenol blue) and then loaded into each gel hole for electrophoresis under 80 mA constant current for about 60 minutes. After electrophoresis, the gel was taken out, stained with 1×Sybr Gold dye (Invitrogen, Cat. 11494) for 15 minutes for imaging. The stability results were calculated.

Table 5G shows the semiquantitative test result of the stability of the drug conjugates listed in Table 2G and the comparative conjugate in human plasma *in vitro.* The result is expressed as the ratio (RL) of the longest fragment remaining after the incubation of the drug conjugates and the comparative conjugate with human plasma to the longest fragment of untreated siRNA.

Table 5G The plasma stability of different drug conjugates over time

| Conjugate Serial No. | Conjugate No. | RL (%) | | | | |
|---|---|---|---|---|---|---|
| | | Untreated | 0h | 8h | 24h | 48h |
| Conjugate 161 | N6-siAP1-M1 | 100 | 100 | 99.4 | 98.0 | 95.1 |
| Conjugate 162 | N6-siAP2-M1 | 100 | 100 | 99.4 | 96.2 | 96.2 |
| Conjugate 163 | N6-siAP1-M2 | 100 | 100 | 99.6 | 96.1 | 98.3 |
| Conjugate 164 | N6-siAP2-M2 | 100 | 100 | 99.7 | 96.2 | 98.4 |
| Conjugate 165 | N6-siAP1-M1VP | 100 | 100 | 99.6 | 98.3 | 93.1 |
| Conjugate 166 | N6-siAP2-M1VP | 100 | 100 | 99.5 | 95.1 | 95.2 |
| Conjugate 167 | N6-siAP1-M2VP | 100 | 100 | 99.5 | 96.2 | 96.7 |
| Conjugate 168 | N6-siAP2-M2VP | 100 | 100 | 99.1 | 97.0 | 98.4 |
| Conjugate 169 | N6-siAP1-M1S | 100 | 100 | 99.4 | 93.1 | 98.8 |
| Conjugate 170 | N6-siAP2-M1S | 100 | 100 | 99.3 | 95.4 | 94.4 |
| Conjugate 171 | N6-siAP1-M2S | 100 | 100 | 98.5 | 98.3 | 98.2 |
| Conjugate 172 | N6-siAP2-M2S | 100 | 100 | 99.2 | 96.1 | 99.3 |
| Conjugate 173 | N6-siAP1-M1SVP | 100 | 100 | 99.1 | 98.4 | 99.1 |
| Conjugate 174 | N6-siAP2-M1SVP | 100 | 100 | 99.1 | 96.5 | 96.9 |
| Conjugate 175 | N6-siAP1-M2SVP | 100 | 100 | 99.1 | 98.3 | 98.2 |
| Conjugate 176 | N6-siAP1-M2SP | 100 | 100 | 99.3 | 95.8 | 94.4 |
| Conjugate 177 | N6-siAP1-M2SPs | 100 | 100 | 99.2 | 98.1 | 95.1 |
| Conjugate 178 | N6-siAP4-M2S | 100 | 100 | 99.1 | 96.1 | 98.2 |
| Conjugate 179 | X2-siAP1-M2SVP | 100 | 100 | 99.0 | 97.2 | 99.0 |
| Conjugate 180 | W2-siAP1-M2SVP | 100 | 100 | 99.1 | 98.0 | 95.1 |
| Conjugate 181 | V2-siAP1-M2SVP | 100 | 100 | 99.1 | 95.4 | 94.2 |
| Conjugate 182 | 02-siAP1-M2SVP | 100 | 100 | 99.2 | 96.2 | 95.3 |
| Example 183 | P2-siAP1-M2SVP | 100 | 100 | 99.3 | 98.1 | 96.2 |
| Comparative Conjugate 1 | N6-NC | 100 | 100 | 99.1 | 98.3 | 98.2 |

As can be seen from the results of Table 5G, the drug conjugates of the present disclosure exhibited excellent stability in plasma, and still showed a length ratio of the siRNA fragments of higher than 93% at 48 h.

**Experimental example 8-3 This experiment illustrates the inhibitory efficiency of the drug conjugates in Table 2G against the expression level of APOC3 mRNA *in vivo.***

In this Experimental Example, the *in vivo* inhibition of the expression level of APOC3 by Conjugates 172, 173, 175-177 and 179-183 in liver tissue of Human APOC3 transgenic mice (B6; CBA-Tg(APOC3)3707Bres/J, purchased from Jackson Lab) was investigated.

Human APOC3 transgenic mice (6-8 week old) were randomly divided into groups (six mice per group, half male and half female), and each group of mice was numbered according to the drug conjugates in Table 2G. Then, each group of mice was respectively administered with each of the drug conjugates Conjugates 172, 173, 175-177 and 179-183. All animals were dosed based on body weight and was administered at a single dose by subcutaneous injection. Each conjugate was administered in the form of 0.3 mg/mL in 0.9% NaCl aqueous solution, with an administration volume of 10 mL/kg; that is, the administration doses of each conjugate were 3 mg/kg. Each mouse in another group was administered with 1×PBS with an administration volume of 10 mL/kg mouse body weight, as a control group.

On day 28 after administrion, the micewere sacrificed, and the liver tissue of each mouse was collected separately and stored in RNA later (Sigma Aldrich Crop.). The liver tissue was homogenized with a tissue homogenizer and then extracted with Trizol (Thermo Fisher company) according to the standard operation procedures for extracting total RNA to obtain the total RNA.

For the liver tissue of each mouse, 1 μg of total RNA was taken, and the extracted total RNA was reverse-transcribed into cDNA using ImProm-II™ reverse transcription kit (Promega Crop.) according to the instructions thereof to obtain a solution containing cDNA, and then the expression level of APOC3 mRNA in liver tissue was determined by the fluorescent quantitative PCR kit (Beijing Cowin Biosciences Co., Ltd.). In this fluorescent quantitative PCR method, β-actin gene was used as an internal control gene, and APOC3 and β-actin were detected by using primers for APOC3 and β-actin, respectively. The sequences of the primers for detection are shown in Table 6G.

The relative quantitative calculation of the expression level of the target APOC3 gene in each test group and the control group was carried out using the comparative Ct (ΔΔCt) method, and the calculation method was as follows:

$$\Delta Ct\text{ (test group)} = Ct\text{ (target gene of test group)} - Ct\text{ (internal control gene of test group)}$$

$$\Delta Ct\text{ (control group)} = Ct\text{ (target gene of control group)} - Ct\text{ (internal control gene of control group)}$$

$$\Delta\Delta Ct\text{ (test group)} = \Delta Ct\text{ (test group)} - \Delta Ct\text{ (average of control group)}$$

$$\Delta\Delta Ct\text{ (control group)} = \Delta Ct\text{ (control group)} - \Delta Ct\text{ (average of control group)}$$

wherein ΔCt (average of control group) was the arithmetic mean of the ΔCt (control group) of the six mice in the control group. Thus, each mouse in the test and control groups corresponded to a ΔΔCt value.

The expression level of APOC3 mRNA in the test group was normalized based on the control group, and the expression level of APOC3 mRNA in the control group was defined as 100%,

$$\text{Relative expression level of APOC3 mRNA in the test group} = 2^{-\Delta\Delta Ct\text{ (the test group)}} \times 100\%.$$

For the siRNA in the same test group, the mean of the relative expression level of APOC3 mRNA in the test group at each concentration was the arithmetic mean of the relative expression levels in the six mice at that concentration.

Therein, the control group refers to the mice in the control group administered with PBS in this experiment, and each test group refers to the mice in the administration group adminstered with different drug conjugates. The results are shown in Table 7G below.

Table 6G The sequences of the primers

| Gene | Upstream primers | Downstream primers |
|---|---|---|
| Human APOC3 | 5'- GTGACCGATGGCTTCAGTTC -3' (SEQ ID NO: 709) | 5'- ATGGATAGGCAGGTGGACTT -3' (SEQ ID NO: 710) |
| Mouse β-actin | 5'-AGCTTCTTTGCAGCTCCTTCGTTG-3' (SEQ ID NO: 711) | 5'-TTCTGACCCATTCCCACCATCACA-3' (SEQ ID NO: 712) |

Table 7G Inhibition rates of the drug conjugates against the expression of APOC3 mRNA in mouse liver

| Conjugate Serial No. | Conjugate No. | Dosage (mg/kg) | Inhibition rate against APOC3 mRNA in liver (%) |
|---|---|---|---|
| -- | PBS | -- | 0 |
| Conjugate 172 | N6-siAP2-M2S | 3 | 71.4 |
| Conjugate 173 | N6-siAP1-M1SVP | 3 | 81.2 |
| Conjugate 175 | N6-siAP1-M2SVP | 3 | 82.5 |
| Conjugate 176 | N6-siAP1-M2SP | 3 | 81.5 |
| Conjugate 177 | N6-siAP1-M2SPs | 3 | 81.9 |
| Conjugate 179 | X2-siAP1-M2SVP | 3 | 80.6 |
| Conjugate 180 | W2-siAP1-M2SVP | 3 | 78.2 |
| Conjugate 181 | V2-siAP1-M2SVP | 3 | 79.4 |
| Conjugate 182 | 02-siAP1-M2SVP | 3 | 80.8 |
| Conjugate 183 | P2-siAP1-M2SVP | 3 | 77.5 |

As can be seen from the results in Table 7G, compared with PBS, all the drug conjugates of the present disclosure showed excellent inhibitory activity against APOC3 mRNA, and could exhibit an inhibition rate of at least 71.4% and up to 82.5% against APOC3 mRNA.

**Experimental example 8-4 This experiment illustrates the effects of the drug conjugates Conjugates 175-177 on the blood lipid content *in vivo.***

In this Experimental Example, the *in vivo* effects of the drug conjugates Conjugates 175-177 (N6-siAP1-M2SVP, N6-siAP1-M2SP and N6-siAP1-M2SPs) on the contents of total cholesterol (CHO) and triglyceride (TG) in serum in human APOC3 transgenic mice (B6; CBA-Tg(APOC3)3707Bres/J, purchased from Jackson Lab) in serum were investigated.

Human APOC3 transgenic mice (6-8 week old) were randomly divided into 3 groups (six mice per group, half male and half female): (1) PBS control group; (2) 3 mg/kg Conjugate 175 group; (3) 1 mg/kg Conjugate 175 group; (4) 3 mg/kg Conjugate 176 group; (5) 1 mg/kg Conjugate 176 group; (6) 3 mg/kg Conjugate 177 group; and 7) 1 mg/kg Conjugate 177 group. All animals were dosed based on body weight and was administered at a single dose by subcutaneous injection, with an administration volume of 10 mL/kg.

About 100 μL orbital blood was taken 1 days before the administration (marked as day -1) and on day 7, 14, 21, 28, 35, 42, 49, and 65 after administration. The blood was centrifuged to obtain serum. The contents of total cholesterol (CHO) and triglyceride (TG) in serum were further determined by using a PM1P000/3 full-automatic serum biochemical analyzer (SABA, Italy). The results of blood lipid were normalized and the inhibition ratio against blood lipid levels was calculated by the equation:

the inhibition ratio=(1−the blood lipid content in the test group after administration/the blood lipid content in the test group before administration)×100%.

The blood lipid refers to total cholesterol or triglyceride. The determination results are shown in FIG. 8G below.

Table 8G The effects of the drug conjugates on the expression levels of total cholesterol and triglyceride in mouse serum

| Detection points | Inhibition rate % | | | | | | | | | | | |
|---|---|---|---|---|---|---|---|---|---|---|---|---|
| | Conjugate 175 3 mg/kg | | Conjugate 175 1 mg/kg | | Conjugate 176 3 mg/kg | | Conjugate 176 1 mg/kg | | Conjugate 177 3 mg/kg | | Conjugate 177 1 mg/kg | |
| | CHO | TG | CHO | TG | CHO | TG | CHO | TG | CHO | TG | CHO | TG |
| Day 7 | 54.2 | 84.3 | 57.3 | 85.1 | 53.8 | 83.9 | 56.8 | 85.3 | 53.7 | 83.8 | 57.0 | 84.7 |
| Day 14 | 53.1 | 82.6 | 51.2 | 80.0 | 53.2 | 82.4 | 51.0 | 79.6 | 52.9 | 81.9 | 50.8 | 80.2 |
| Day 21 | 46.5 | 83.6 | 44.5 | 83.2 | 46.1 | 83.2 | 44.3 | 82.8 | 46.4 | 83.2 | 44.6 | 83.1 |
| Day 28 | 55.1 | 88.1 | 53.5 | 82.5 | 54.8 | 87.5 | 53.4 | 82.1 | 54.5 | 87.2 | 53.2 | 82.7 |
| Day 35 | 53.6 | 85.3 | 50.1 | 79.6 | 53.2 | 85.1 | 49.5 | 79.8 | 53.8 | 84.6 | 50.5 | 79.1 |
| Day 42 | 55.2 | 85.1 | 55.2 | 77.4 | 54.8 | 85.0 | 55.1 | 77.6 | 54.9 | 84.8 | 54.2 | 77.1 |
| Day 49 | 52.9 | 79.3 | 40.5 | 71.4 | 52.5 | 78.8 | 40.2 | 72.1 | 52.8 | 79.8 | 41.3 | 71.2 |
| Day 65 | 58.4 | 75.2 | 49.3 | 66.7 | 57.5 | 75.0 | 75.3 | 66.5 | 56.9 | 75.4 | 48.8 | 66.2 |

As can be seen from the results of Table 8G, the drug conjugates Conjugates 175-177 siginificantly reduced the contents of total cholesterol or triglyceride in mouse serum and still showed a higher effect of reducing blood lipid level at least on Day 65.

**Experimental example 8-5 This experiment illustrates the inhibitory efficiency of the drug conjugates against the target mRNA in C57BL/6J mice.**

In this Experimental Example, the *in vivo* inhibition of the expression level of APOC3 by Conjugate 184 against in the liver tissue of C57BL/6J mice was investigated.

C57BL/6J mice (6-8 week old, purchased from Department of Laboratory Animal Science, Peking University Health Science Center) were randomly divided into groups (five mice per group, all female) according to body weights, and each group of mice was numbered according to Conjugate 184. Then, each group of mice was respectively administered with test Conjugate 184. All animals were dosed based on body weight and was administered at a single dose by subcutaneous injection. Each conjugate was respectively administered in the form of 0.2 mg/mL and 0.02 mg/mL in 0.9% NaCl aqueous solution, with an administration volume of 5 mL/kg; that is, the administration doses of each conjugate were 1 mg/kg and 0.1 mg/kg.

Mice in one group were administered with 1×PBS with an administration volume of 5 mL/kg mouse body weight, as a control group.

At 72 hours after administrion, the animals were sacrificed, and the liver tissue of each mouse was collected separately and stored in RNA later (Sigma Aldrich Crop.). The liver tissue was homogenized with a tissue homogenizer and then extracted with Trizol (Thermo Fisher company) according to the operation procedures described in the instruction to obtain the total RNA.

For the liver tissue of each mouse, 1 μg of total RNA was taken, and the extracted total RNA was reverse-transcribed into cDNA using ImProm-II™ reverse transcription kit (Promega Crop.) according to the instructions thereof to obtain a solution containing cDNA, and then the expression level of TTr mRNA in liver tissue was determined by the fluorescent quantitative PCR kit (Beijing Cowin Biosciences Co., Ltd.). In this fluorescent qPCR method, GAPDH gene was used as an internal control gene, TTr and GAPDH were detected by using primers for TTr and GAPDH, respectively. The sequences of the primers for detection are shown in Table 9G.

The relative quantitative calculation of the expression level of the target TTr gene in each test group and the control group was carried out using the comparative Ct (ΔΔCt) method, and the calculation method was as follows:

$$\Delta Ct \text{ (test group)} = Ct \text{ (target gene of test group)} - Ct \text{ (internal control gene of test group)}$$

$$\Delta Ct \text{ (control group)} = Ct \text{ (target gene of control group)} - Ct \text{ (internal control gene of control group)}$$

$$\Delta\Delta Ct \text{ (test group)} = \Delta Ct \text{ (test group)} - \Delta Ct \text{ (average of control group)}$$

$$\Delta\Delta Ct \text{ (control group)} = \Delta Ct \text{ (control group)} - \Delta Ct \text{ (average of control group)}$$

wherein ΔCt (average of control group) was the arithmetic mean of the ΔCt (control group) of the five mices in the control group. Thus, each mouse in the test and control groups corresponded to a ΔΔCt value.

The expression level of TTr mRNA in the test group was normalized based on the control group, and the expression level of TTr mRNA in the control group was defined as 100%,

$$\text{Relative expression level of TTr mRNA in the test group} = 2^{-\Delta\Delta Ct \text{ (the test group)}} \times 100\%.$$

For the siRNA in the same test group, the mean of the relative expression level of TTr mRNA in the test group at each concentration was the arithmetic mean of the relative expression levels in the five mice at that concentration.

Therein, the control group refers to the mice in the control group administered with PBS in this experiment, and each test group refers to the mice in the administration group adminstered with different drug conjugates. The results are shown in Table 10G below.

Table 9G The sequences of the primers

| Gene | Upstream primers | Downstream primers |
|---|---|---|
| mTTR | 5'- CCGTCTGTGCCTTCTCATCT -3' (SEQ ID NO: 715) | 5'- TAATCTCCTCCCCCAACTCC -3' (SEQ ID NO: 716) |
| GAPDH | 5'- AGAAGGCTGGGGCTCATTTG- 3' (SEQ ID NO: 717) | 5'- AGGGGCCATCCACAGTCTTC -3' (SEQ ID NO: 718) |

Table 10G Inhibitory effect on mTTR mRNA in mice

| Conjugate Serial No. | Conjugate No. | Inhibition rate against mRNA (%) | |
|---|---|---|---|
| | | 1 mg/kg | 0.1 mg/kg |
| Conjugate 184 | N6-siTTR-M2SVP | 90 | 38 |

As can be seen from the results, Conjugate 184 showed an excellent *in vivo* inhibitory effect on the target mRNA (TTR mRNA) in mice, and could exhibit an inhibition rate of up to 90%.

Some specific embodiments of the present disclosure are described in detail above, but the present disclosure is not limited to the specific details of the above embodiments. Various simple variations to the technical solutions of the present disclosure can be made within the scope of the technical concept of the present disclosure, and these simple variations are also within the scope of the present disclosure.

It is to be noted that each of the specific technical features described in the above specific embodiments can be combined in any suitable manner provided that no contradiction is caused. In order to avoid unnecessary repetition, various possible combination manners are no longer described in the present disclosure.

In addition, various different embodiments of the present disclosure can also be combined as long as it does not deviate from the idea of the present disclosure, which should also be regarded as the disclosure of the present disclosure.

## Claims

**1.** A compound having a structure as shown by Formula (101):

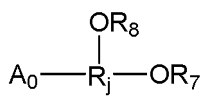

Formula (101)

wherein Ao has a structure as shown by Formula (312):

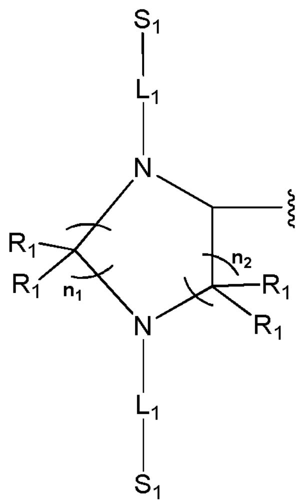

Formula (312)

wherein

$n_1$ is an integer of 1-4;
$n_2$ is an integer of 0-3;
each $R_1$ independently of one another is selected from one of H, substituted or unsubstituted $C_1$-$C_4$ hydrocarbyl or halogen;
each $L_1$ is a linear alkylene of 1 to 70 carbon atoms in length, wherein one or more carbon atoms are optionally replaced with one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein

$L_1$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, $OC_1$-$C_{10}$ alkyl, $OC_1$-$C_{10}$ alkylphenyl, -$C_1$-$C_{10}$ alkylOH, $OC_1$-$C_{10}$ haloalkyl, $SC_1$-$C_{10}$ alkyl, $SC_1$-$C_{10}$ alkylphenyl, -$C_1$-$C_{10}$ alkylSH, $SC_1$-$C_{10}$ haloalkyl, halo, OH, -SH, $NH_2$, $C_1$-$C_{10}$ alkyl-$NH_2$, N($C_1$-$C_{10}$ alkyl)($C_1$-$C_{10}$ alkyl), NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, $CO_2H$, C(O)O($C_1$-$C_{10}$ alkyl), - CON($C_1$-$C_{10}$ alkyl)($C_1$-$C_{10}$ alkyl), CONH($C_1$-$C_{10}$ alkyl), $CONH_2$, NHC(O)($C_1$-$C_{10}$ alkyl), NHC(O)(phenyl), N($C_1$-$C_{10}$ alkyl)C(O)($C_1$-$C_{10}$ alkyl), N($C_1$-$C_{10}$ alkyl)C(O)(phenyl), C(O)$C_1$-$C_{10}$ alkyl, C(O)$C_1$-$C_{10}$ alkylphenyl, C(O)$C_1$-$C_{10}$ haloalkyl, OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, $SO_2NH$($C_1$-$C_{10}$ alkyl), $SO_2NH$(phenyl), -$NHSO_2$($C_1$-$C_{10}$ alkyl), -$NHSO_2$(phenyl), and $NHSO_2$($C_1$-$C_{10}$ haloalkyl);

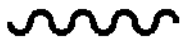 represents the site where a group is covalently linked;
each $S_1$ is independently a Mi, in which all active hydroxyl groups and/or amino groups, if any, are protected with protecting groups;
each $M_1$ is independently selected from a ligand capable of binding to a cell surface receptor;
$R_j$ is a linking group;
$R_7$ is a functional group capable of forming a phosphoester linkage, phosphorothioate linkage, phosphoroborate linkage, or carboxylate linkage with a hydroxyl group via reaction, or a functional group capable of forming an amide linkage with an amino group via reaction; and$R_8$ is a hydroxyl protecting group.

2. The compound according to claim 1, wherein each $L_1$ is independently selected from one of the groups of Formulae A1-A26 or any connection combinations thereof:

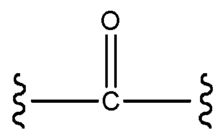,
(A1)

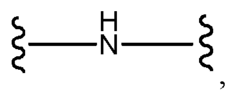,
(A2)

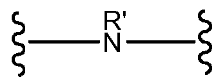,
(A3)

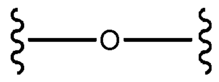,
(A4)

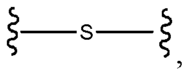,
(A5)

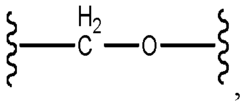,
(A6)

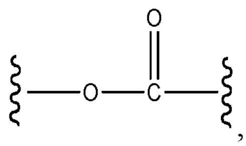,
(A7)

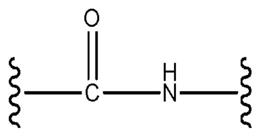,
(A8)

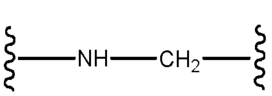,
(A9)

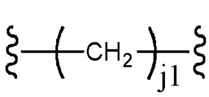,
(A10)

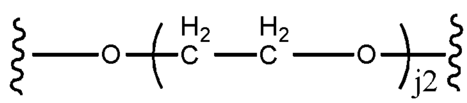,
(A11)

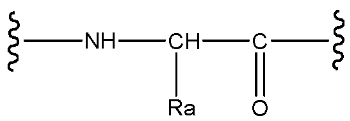,
(A12)

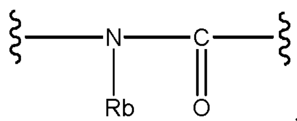,
(A13)

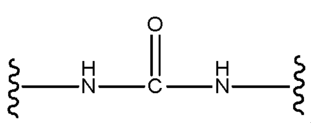,
(A14)

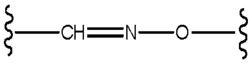, 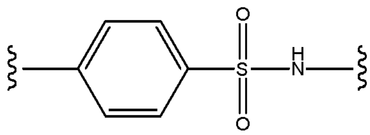, 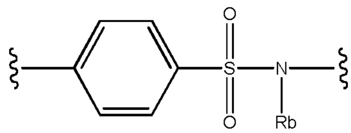,

(A15) (A16) (A17)

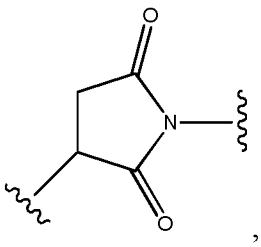, 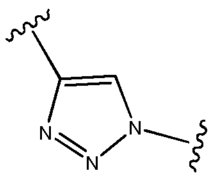, 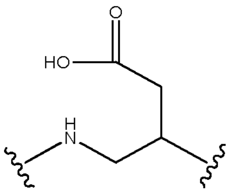, 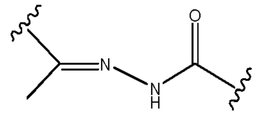,

(A18) (A19) (A20) (A21)

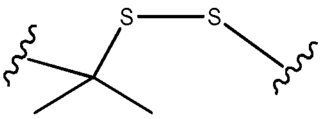, 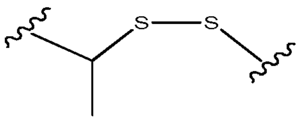, 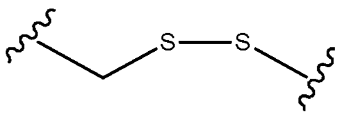,

(A22) (A23) (A24)

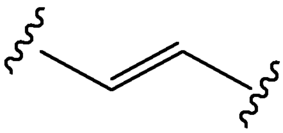, 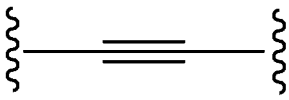;

(A25) (A26)

wherein j 1 is an integer of 1-20;
j2 is an integer of 1-20;
R' is a $C_1$-$C_{10}$ alkyl;
Ra is selected from one of the groups of Formulae A27-A45:

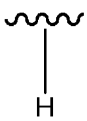, 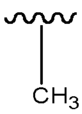, 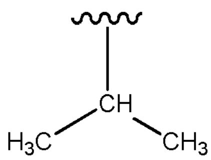, 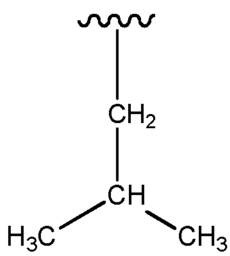, 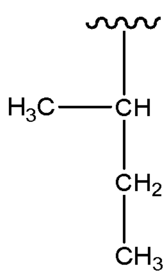, 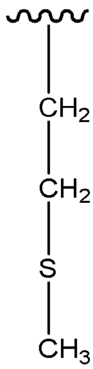,

(A27) (A28) (A29) (A30) (A31) (A32)

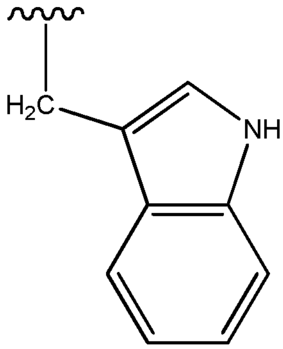 , 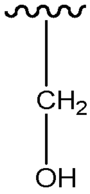 , 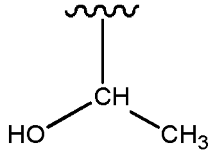 , 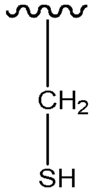 , 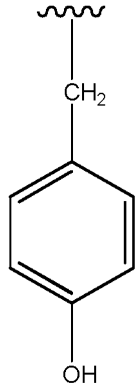 ,

(A33) (A34) (A35) (A36) (A37)

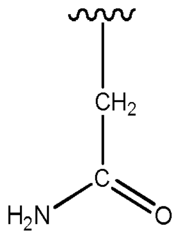 , 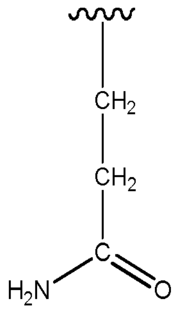 , 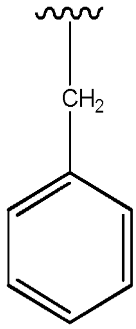 , 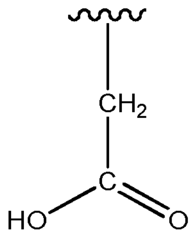 , 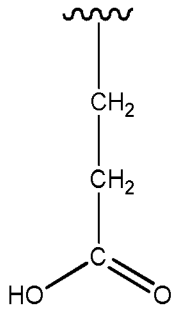 ,

(A38) (A39) (A40) (A41) (A42)

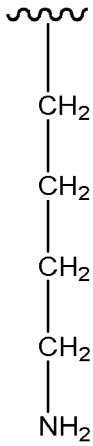 , 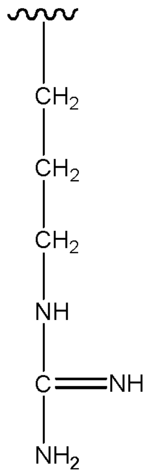 or 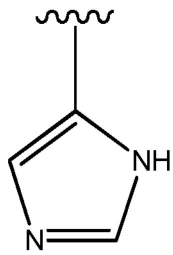 ;

(A43) (A44) (A45)

Rb is a $C_1$-$C_{10}$ alkyl.

**3.** The compound according to claim 2, wherein $L_1$ is selected from one of A1, A2, A4, A5, A6, A8, A10, A11, A13 or any connection combinations thereof.

**4.** The compound according to claim 1, 2 or 3, wherein $L_1$ has a length of 3 to 25 atoms; and the length of $L_1$ refers to the number of the chain-forming atoms in the longest atomic chain formed from the atom linked to the N atom in Ao to the atom linked to $S_1$.

**5.** The compound according to claim 1 or 2, wherein $n_1$ is 1 or 2; and $n_2$ is 1 or 2.

**6.** The compound according to claim 1 or 2, wherein Ao has a structure as shown by Formula (120):

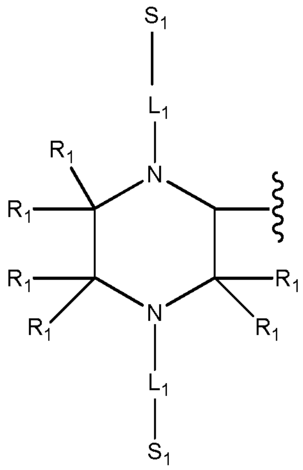

Formula (120).

**7.** The compound according to claim 1 or 2, wherein each $M_1$ is independently selected from one of the ligands formed by lipophilic molecules, saccharides, vitamins, polypeptides, endosomal lysates, steroid compounds, terpene compounds, integrin receptor inhibitors, cationic lipid molecules, and derivatives thereof.

**8.** The compound according to claim 7, wherein each $M_1$ is independently selected from one of D-mannopyranose, L-mannopyranose, D-arabinose, D-xylofuranose, L-xylofuranose, D-glucose, L-glucose, D-galactose, L-galactose, $\alpha$-D-mannofuranose, $\beta$-D-mannofuranose, $\alpha$-D-mannopyranose, $\beta$-D-mannopyranose, $\alpha$-D-glucopyranose, $\beta$-D-glucopyranose, $\alpha$-D-glucofuranose, $\beta$-D-glucofuranose, $\alpha$-D-fructofuranose, $\alpha$-D-fructopyranose, $\alpha$-D-galactopyranose, $\beta$-D-galactopyranose, $\alpha$-D-galactofuranose, $\beta$-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-$\beta$-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-$\alpha$-neuraminic acid, 5-thio-$\beta$-D-glucopyranose, methyl 2,3,4-tris-O-acetyl-1-thio-6-O-trityl-$\alpha$-D-glucopyranoside, 4-thio-$\beta$-D-galactopyranose, ethyl 3,4,6,7-tetra-O-acetyl-2-deoxy-1,5-dithio-$\alpha$-D-glucoheptopyranoside, 2,5-anhydro-D-allononitrile, ribose, D-ribose, D-4-thioribose, L-ribose, and L-4-thioribose.

**9.** The compound according to claim 1 or 2, wherein each $S_1$ is independently selected from one of the groups of Formulae A51-A59:

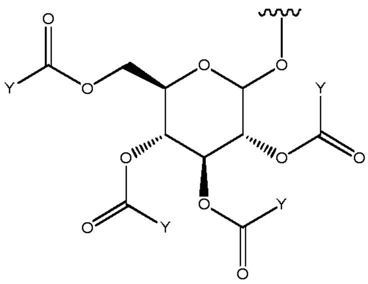 ,

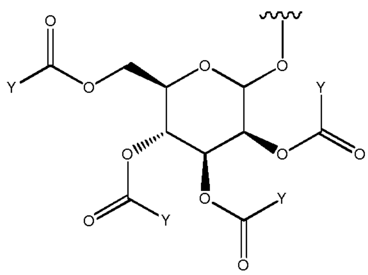 ,

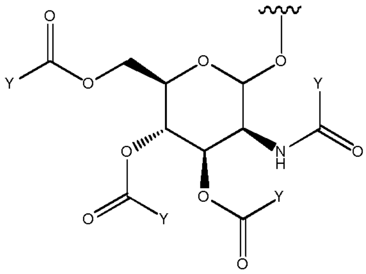

,

(A51) (A52) (A53)

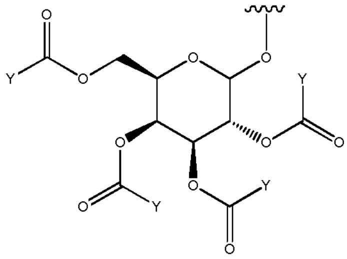

,

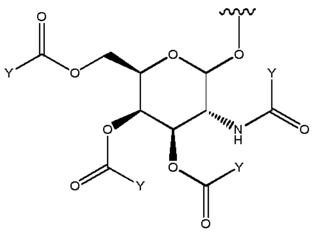

,

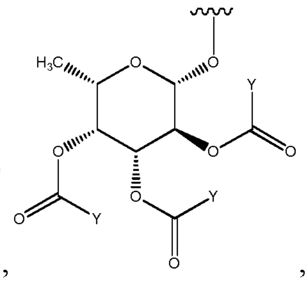

,

(A54) (A55) (A56)

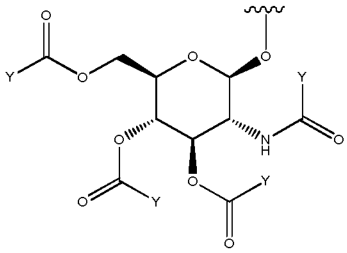

,

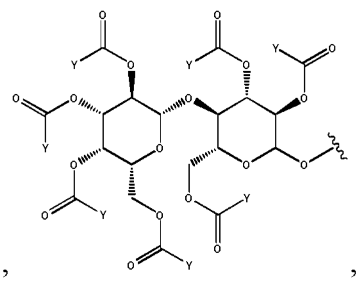

,

and

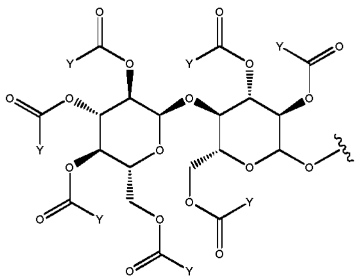

(A57) (A58) (A59)

wherein each Y is independently selected from one of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl.

**10.** The compound according to claim 1 or 2, wherein each $M_1$ is independently selected from one of the following groups: a ligand formed by one compound of cholesterol, cholic acid, adamantaneacetic acid, 1-pyrenebutyric acid, dihydrotestosterone, 1,3-Bis-O(hexadecyl)glycerol, hexaglycerol, menthol, mentha-camphor, 1,3-propanediol, palmitic acid, myristic acid, O3-(oleoyl)lithocholic acid, benzoxazine, folate, folate derivatives, vitamin A, vitamin B7 (biotin), pyridoxal, uvaol, triterpene, friedelin, and epifriedelinol-derived lithocholic acid, or geranyloxyhexyl, heptadecyl, dimethoxytrityl, hecogenin, diosgenin, and sarsasapogenin.

**11.** The compound according to claim 1 or 2, wherein each $S_1$ is independently selected from one of the groups of Formulae A71-A75:

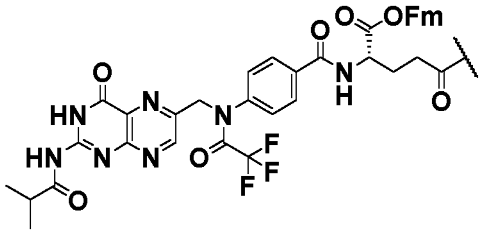

,

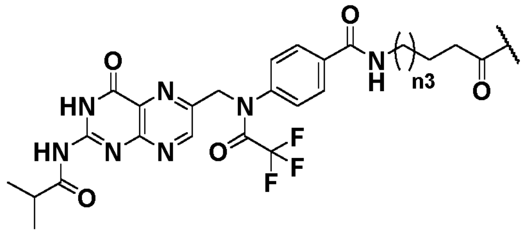

,

Formula (A71) Formula (A72)

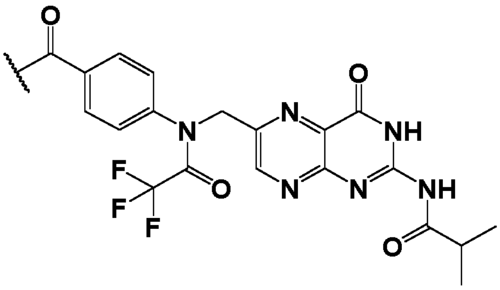

,

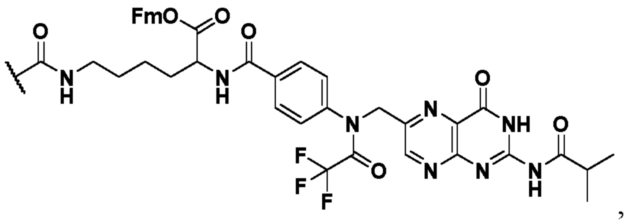

, and

Formula (A73) Formula (A74)

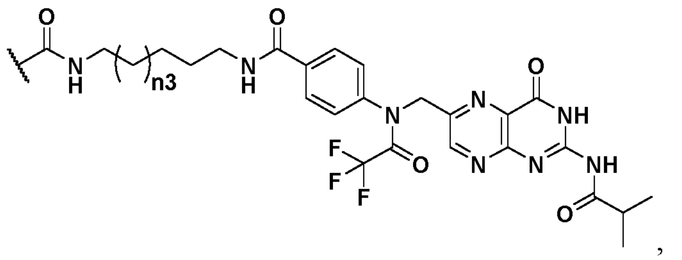

,

Formula (A75)

wherein n3 is an integer of 1-5.

**12.** The compound according to claim 1 or 2, wherein $R_j$ is selected from one of the groups of Formulae A62-A67:

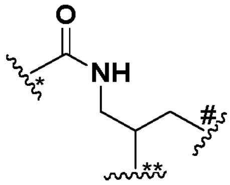, 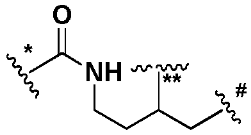, 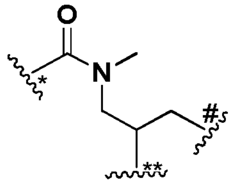, 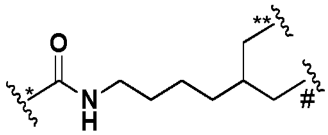,

Formula (A62) Formula (A63) Formula (A64) Formula (A65)

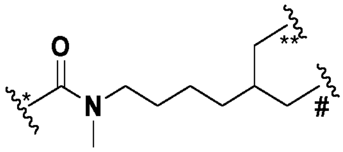, and 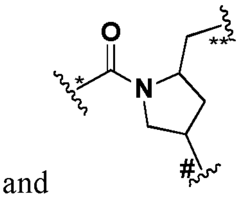

Formula (A66) Formula (A67).

**13.** The compound according to claim 1 or 2, wherein $R_7$ is a group comprising a phosphoramidite, carboxyl or carboxylate functional group.

**14.** The compound according to claim 13, wherein $R_7$ has a structure as shown by Formula (A46), (C1) or (C2):

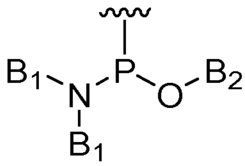, 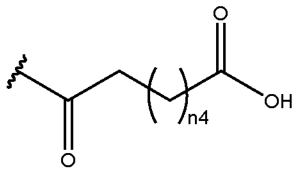, 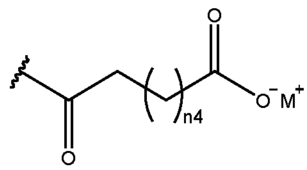,

Formual (A46) Formula (C1) Formula (C2)

wherein each $B_1$ is the same or different, and is selected from substituted or unsubstituted $C_1$-$C_5$ hydrocarbyl; $B_2$ is selected from one of $C_1$-$C_5$ alkyl, ethylcyano, propylcyano and butylcyano; n4 is an integer of 1 to 4; $M^+$ is a cation; and

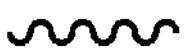 represents the site where a group is covalently linked.

**15.** The compound according to claim 1, 2, 13, or 14, wherein the compound has a structure as shown by any one of Formulae (403) - (408):

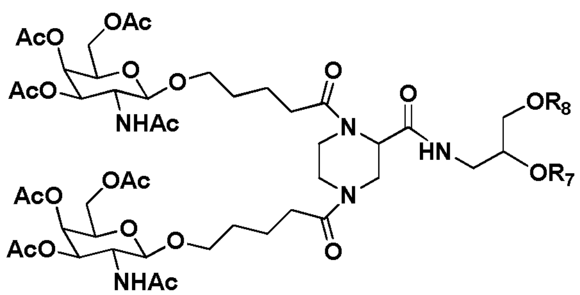,

Formula (403)

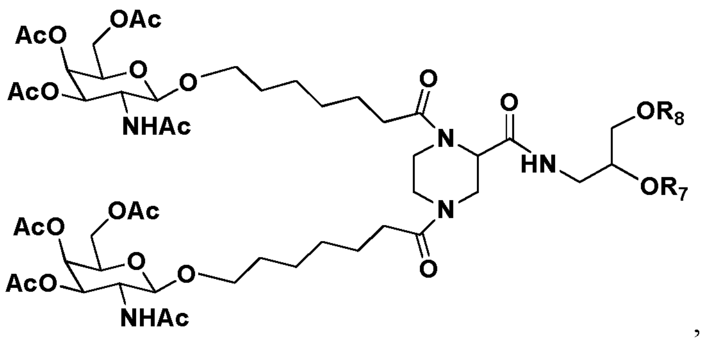

,

Formula (404)

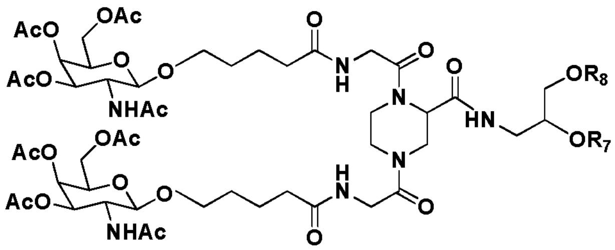

,

Formula (405)

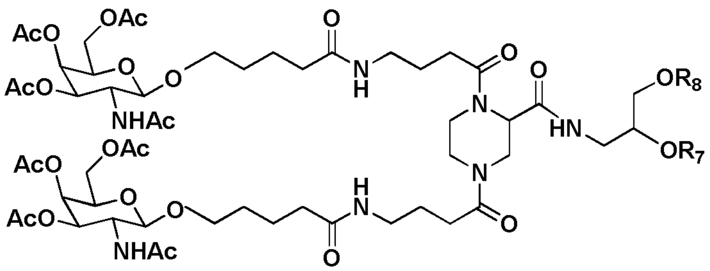

,

Formula (406)

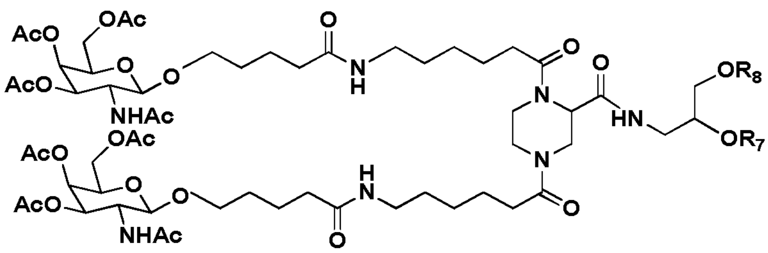

,

Formula (407)

or

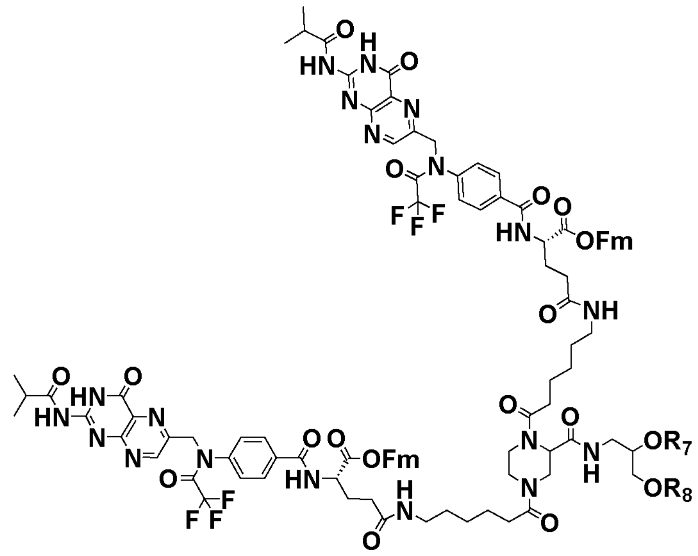

Formula (408).

**16.** A compound having a structure as shown by Formula (111):

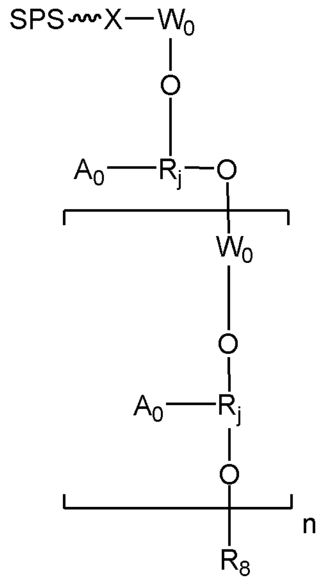

Formula (111)

wherein Ao has a structure as shown by Formula (312):

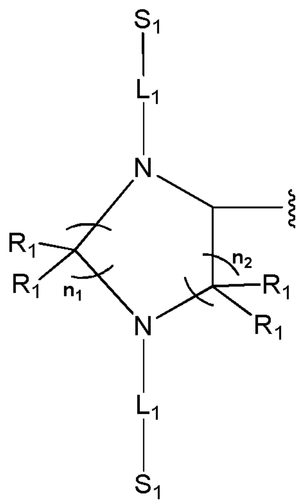

Formula (312)

wherein

$n_1$ is an integer of 1-4;
$n_2$ is an integer of 0-3;
each $R_1$ independently of one another is selected from H, substituted or unsubstituted $C_1$-$C_4$ hydrocarbyl or halogen;
each $L_1$ is a linear alkylene of 1 to 70 carbon atoms in length, wherein one or more carbon atoms are optionally replaced with any one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, $OC_1$-$C_{10}$ alkyl, $OC_1$-$C_{10}$ alkylphenyl, -$C_1$-$C_{10}$ alkylOH, $OC_1$-$C_{10}$ haloalkyl, $SC_1$-$C_{10}$ alkyl, $SC_1$-$C_{10}$ alkylphenyl, -$C_1$-$C_{10}$ alkylSH, $SC_1$-$C_{10}$ haloalkyl, halo, OH, -SH, $NH_2$, $C_1$-$C_{10}$ alkyl$NH_2$, N($C_1$-$C_{10}$ alkyl)($C_1$-$C_{10}$ alkyl), NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, $CO_2H$, C(O)O($C_1$-$C_{10}$ alkyl), - CON($C_1$-$C_{10}$ alkyl)($C_1$-$C_{10}$ alkyl), CONH($C_1$-$C_{10}$ alkyl), $CONH_2$, NHC(O)($C_1$-$C_{10}$ alkyl), NHC(O)(phenyl), N($C_1$-$C_{10}$ alkyl)C(O)($C_1$-$C_{10}$ alkyl), N($C_1$-$C_{10}$ alkyl)C(O)(phenyl), C(O)$C_1$-$C_{10}$ alkyl, C(O)$C_1$-$C_{10}$ alkylphenyl, C(O)$C_1$-$C_{10}$ haloalkyl, OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, $SO_2NH$($C_1$-$C_{10}$ alkyl), $SO_2NH$(phenyl), -$NHSO_2$($C_1$-$C_{10}$ alkyl), -$NHSO_2$(phenyl), and $NHSO_2$($C_1$-$C_{10}$ haloalkyl);
each $S_1$ is independently a Mi, in which all active hydroxyl groups and/or amino groups, if any, are protected with protecting groups;
each $M_1$ is independently selected from a ligand capable of binding to a cell surface receptor;
$W_0$ is a linking group;
X is selected from O or NH;
$R_j$ is a linking group;
SPS represents a solid phase support;
$R_8$ is a hydroxyl protecting group; and
n is an integer of 0-7.

**17.** The compound according to claim 16, wherein each $L_1$ is independently selected from one of the groups of Formulae A1-A26 or any connection combinations thereof:

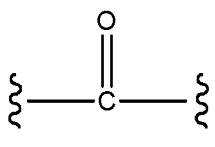
,
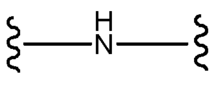
,
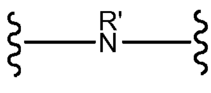
,
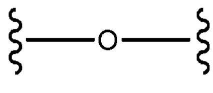
,

(A1) (A2) (A3) (A4)

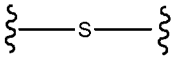, 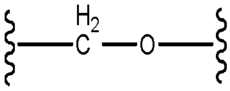, 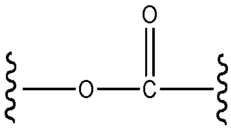, 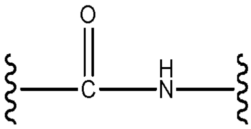,

(A5) (A6) (A7) (A8)

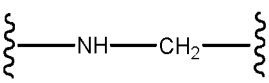, 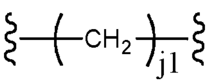, 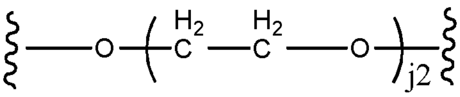,

(A9) (A10) (A11)

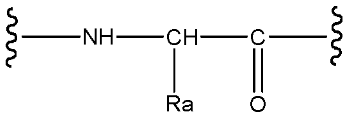, 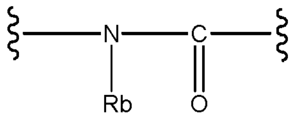, 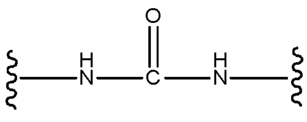,

(A12) (A13) (A14)

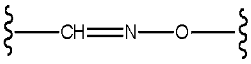, 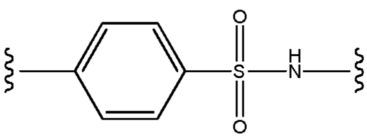, 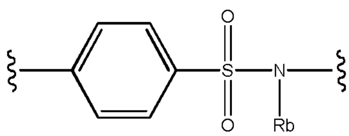,

(A15) (A16) (A17)

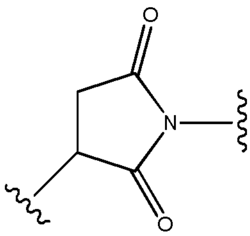, 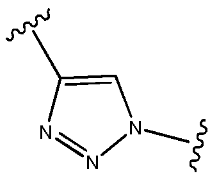, 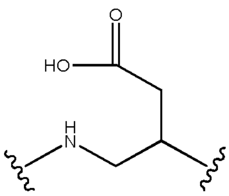, 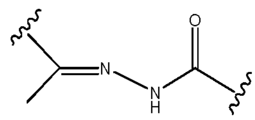,

(A18) (A19) (A20) (A21)

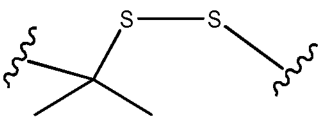, 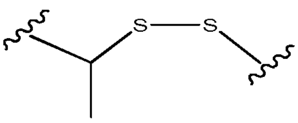, 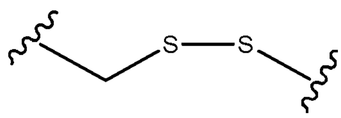,

(A22) (A23) (A24)

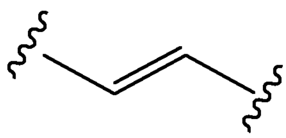, 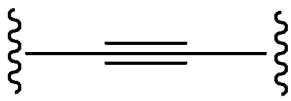;

(A25) (A26)

wherein j 1 is an integer of 1-20;
j2 is an integer of 1-20;

R' is a $C_1$-$C_{10}$ alkyl;
Ra is selected from one of the groups of Formulae A27-A45:

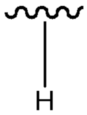
,
(A27)

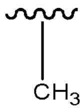
,
(A28)

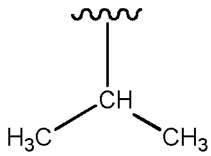
,
(A29)

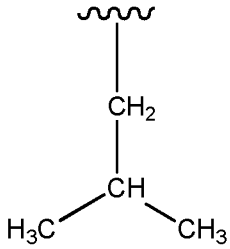
,
(A30)

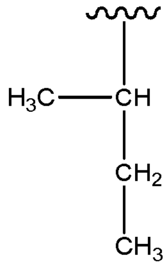
,
(A31)

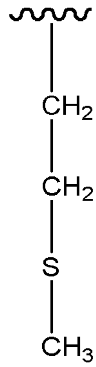
,
(A32)

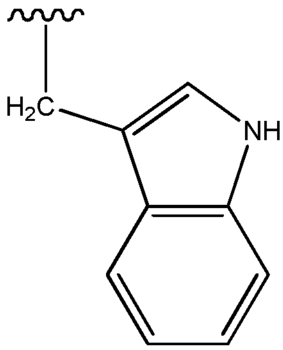
,
(A33)

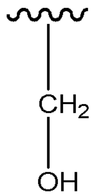
,
(A34)

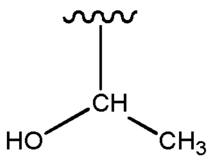
,
(A35)

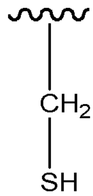
,
(A36)

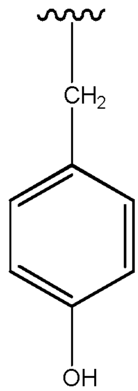
,
(A37)

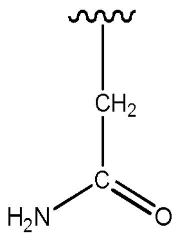
,
(A38)

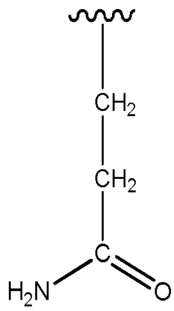
,
(A39)

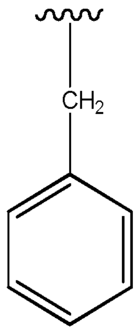
,
(A40)

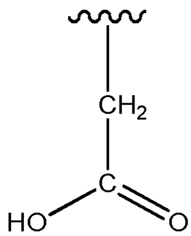
,
(A41)

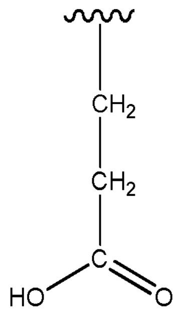
,
(A42)

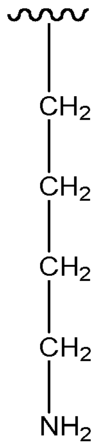, 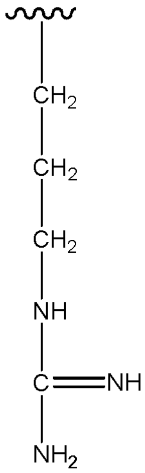 or 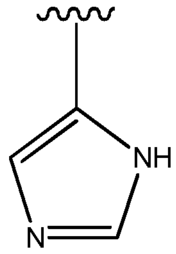 ;

(A43) (A44) (A45)

Rb is a $C_1$-$C_{10}$ alkyl; and

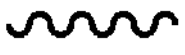 represents the site where a group is covalently linked.

**18.** The compound according to claim 16 or 17, wherein $L_1$ is selected from one of A1, A2, A4, A5, A6, A8, A10, A11, A13 or any connection combinations thereof.

**19.** The compound according to claim 16 or 17, wherein $L_1$ has a length of 3 to 25 atoms; and the length of L1 refers to the number of the chain-forming atoms in the longest atomic chain formed from the atom linked to the N atom in Ao to the atom linked to $S_1$.

**20.** The compound according to claim 16 or 17, wherein $n_1$ is 1 or 2; and $n_2$ is 1 or 2.

**21.** The compound according to claim 20, wherein Ao has a structure as shown by Formula (120):

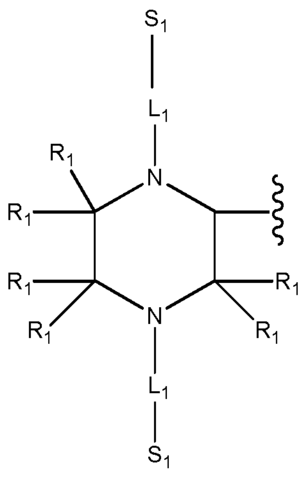

Formula (120).

**22.** The compound according to claim 16 or 17, wherein each $M_1$ is independently selected from one of the ligands formed by lipophilic molecules, saccharides, vitamins, polypeptides, endosomal lysates, steroid compounds, terpene compounds, integrin receptor inhibitors, cationic lipid molecules, or derivatives thereof.

**23.** The compound according to claim 22, wherein each $M_1$ is independently selected from one of D-mannopyranose,

L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucopyranose, methyl 2,3,4-tris-O-acetyl-1-thio-6-O-trityl-α-D-glucopyranoside, 4-thio-β-D-galactopyranose, ethyl 3,4,6,7-tetra-O-acetyl-2-deoxy-1,5-dithio-β-D-glucoheptopyranoside, 2,5-anhydro-D-allononitrile, ribose, D-ribose, D-4-thioribose, L-ribose, and L-4-thioribose.

**24.** The compound according to claim 16 or 17, wherein each $S_1$ is independently selected from one of the groups of Formulae A51-A59:

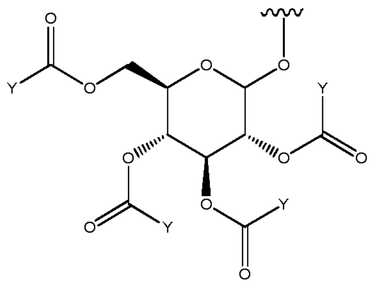
,
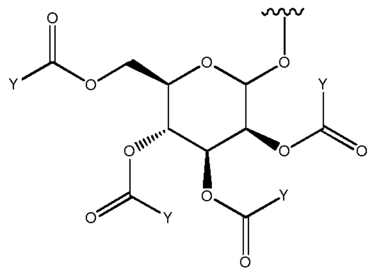
,
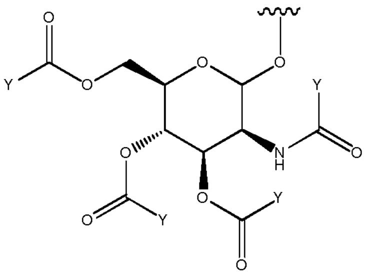
,

(A51) (A52) (A53)

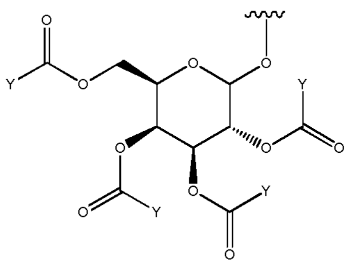
,
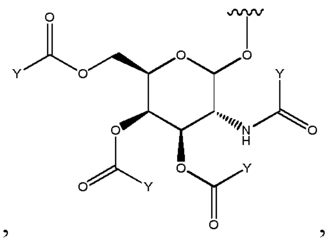
,
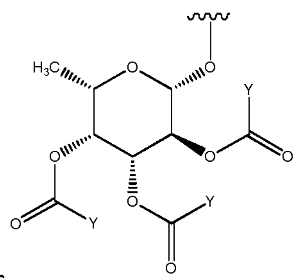
,

(A54) (A55) (A56)

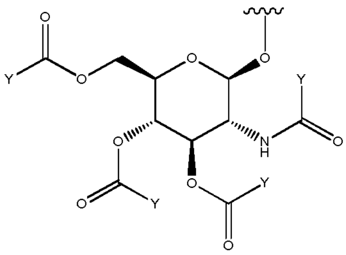
,
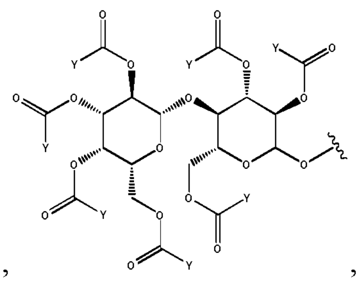
,

and

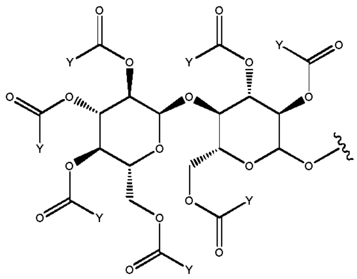

(A57) (A58) (A59)

wherein each Y is independently selected from one of methyl, trifluoromethyl, difluoromethyl, monofluoromethyl, trichloromethyl, dichloromethyl, monochloromethyl, ethyl, n-propyl, isopropyl, phenyl, halophenyl, and alkylphenyl.

**25.** The compound according to claim 16 or 17, wherein each $M_1$ is independently selected from one of the following groups: a ligand formed by one compound of cholesterol, cholic acid, adamantaneacetic acid, 1-pyrenebutyric acid, dihydrotestosterone, 1,3-Bis-O(hexadecyl)glycerol, geranyloxyhexyl, hexaglycerol, menthol, mentha-camphor, 1,3-propanediol, heptadecyl, palmitic acid, myristic acid, O3-(oleoyl)lithocholic acid, dimethoxytrityl, benzoxazine, folate, folate derivatives, vitamin A, vitamin B7 (biotin), biotin, pyridoxal, uvaol, hecogenin, diosgenin, triterpene, sarsasapogenin, friedelin, and epifriedelinol-derived lithocholic acid, or geranyloxyhexyl, heptadecyl, dimethoxytrityl, hecogenin, diosgenin, or sarsasapogenin.

**26.** The compound according to claim 16 or 17, wherein each $S_1$ is independently selected from one of the groups of Formulae A71-A75:

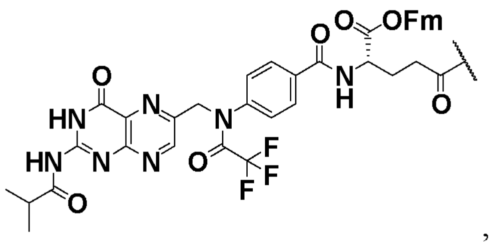

,

Formula (A71)

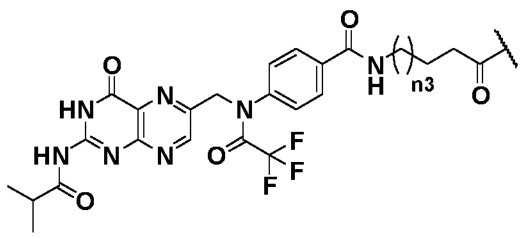

,

Formula (A72)

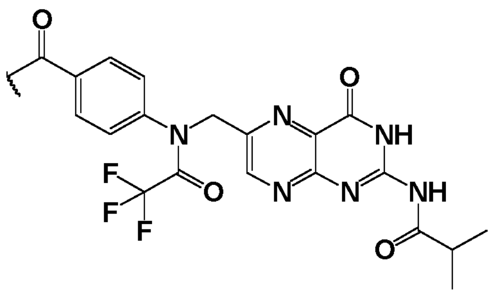

,

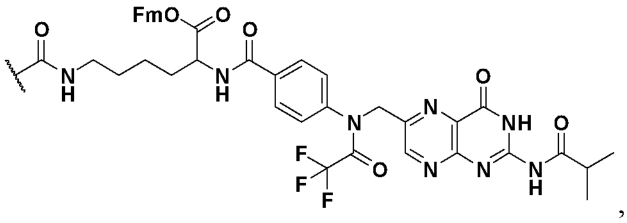

, and

Formula (A73) Formula (A74)

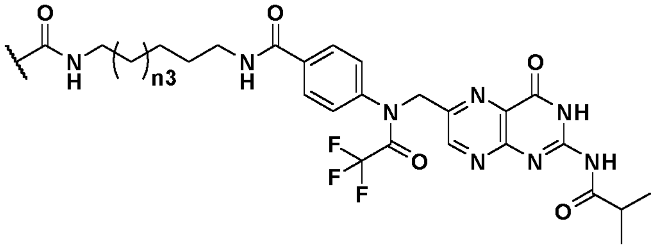

,

Formula (A75)

wherein n3 is an integer of 1-5; and



represents the site where a group is covalently linked.

**27.** The compound according to claim 16, wherein $R_j$ is selected from one of the groups of Formulae A62-A67:

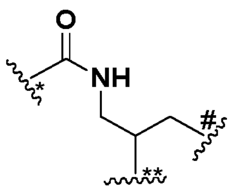

,

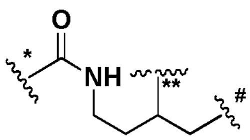

,

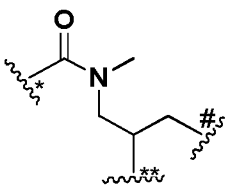

,

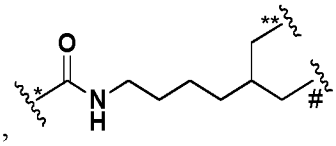

,

Formula (A62) Formula (A63) Formula (A64) Formula (A65)

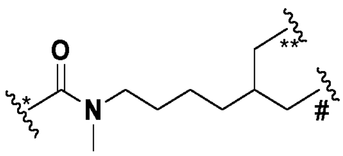

, and

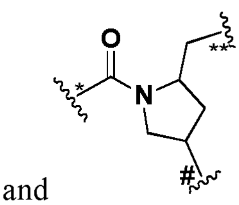

Formula (A66) Formula (A67).

**28.** The compound according to claim 16 or 17, wherein each Wo independently has a structure as shown by Formula (C1') or (A81):

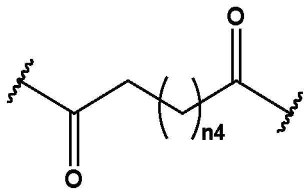 , 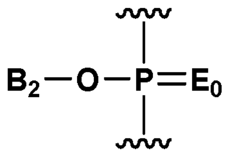

Formula (C1')　　　Formula (A81)

wherein

n4 is an integer of 1-4;
each Eo is independently O, S or BH;
each $B_2$ is independently selected from $C_1$-$C_5$ alkyl, ethylcyano, propylcyano and butylcyano;

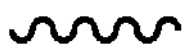 represents the site where a group is linked.

**29.** The compound according to claim 16 or 17, wherein n is an integer of 1-4.

**30.** The compound according to claim 16 or 17, wherein the compound has a structure as shown by any of Formulae (503)-(510):

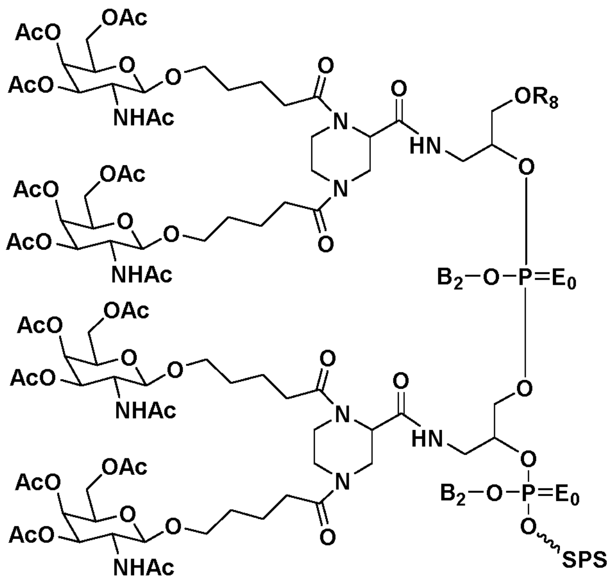

Formula (503)

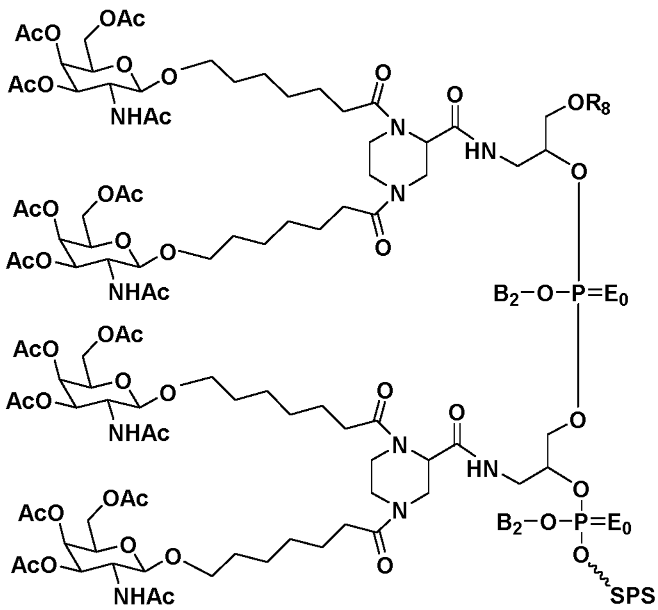

Formula (504)

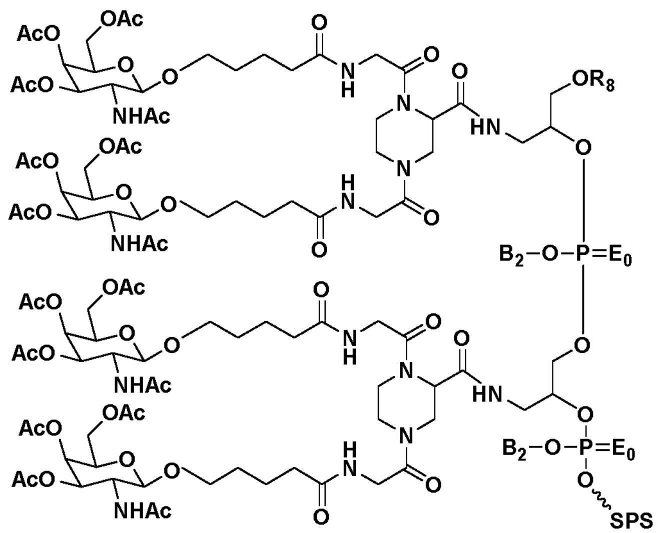

Formula (505)

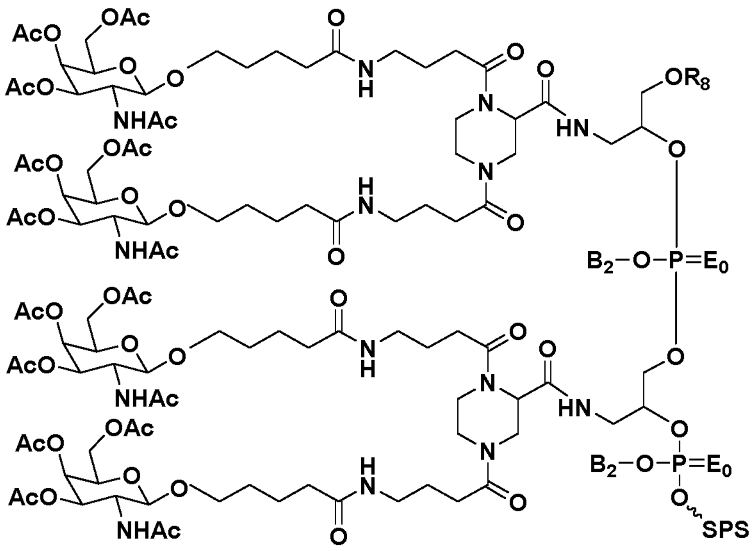

Formula (506)

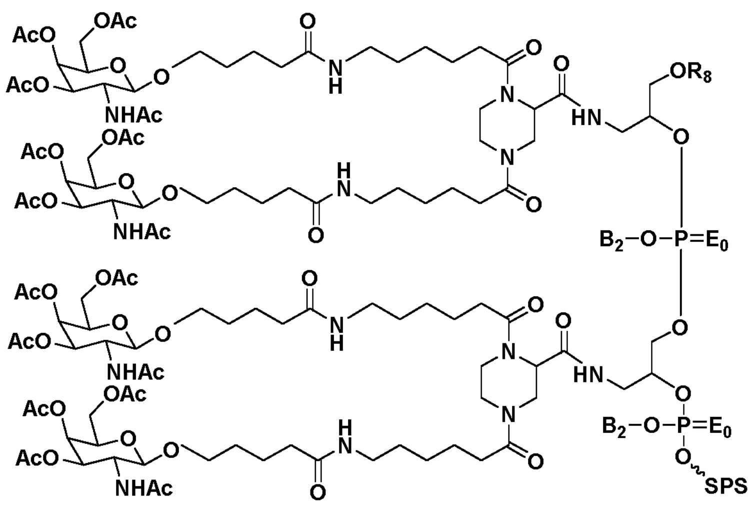

Formula (507)

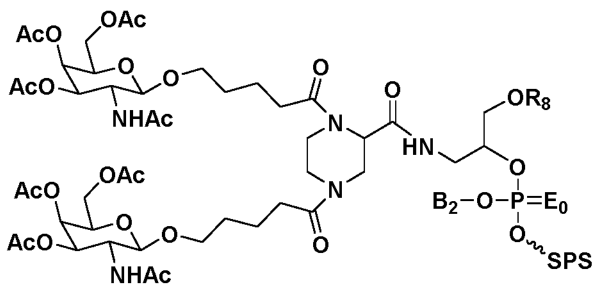

Formula (508)

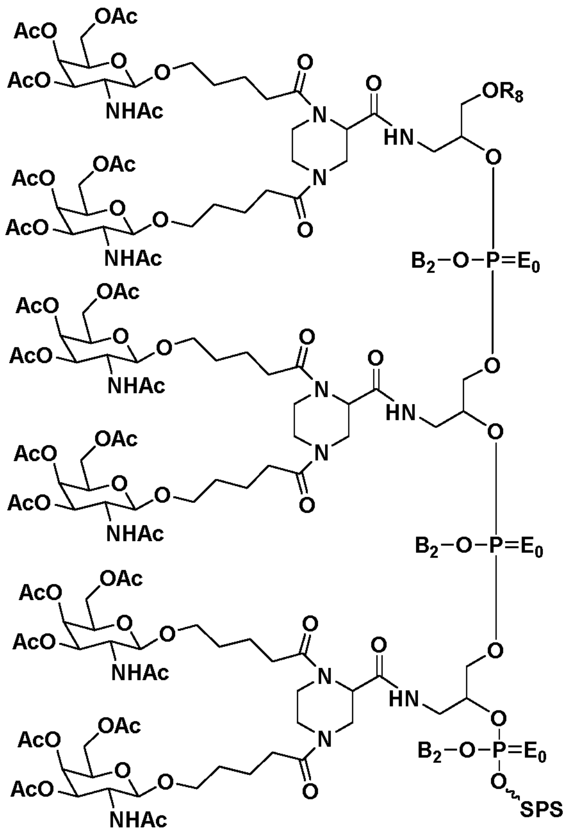

Formula (509),

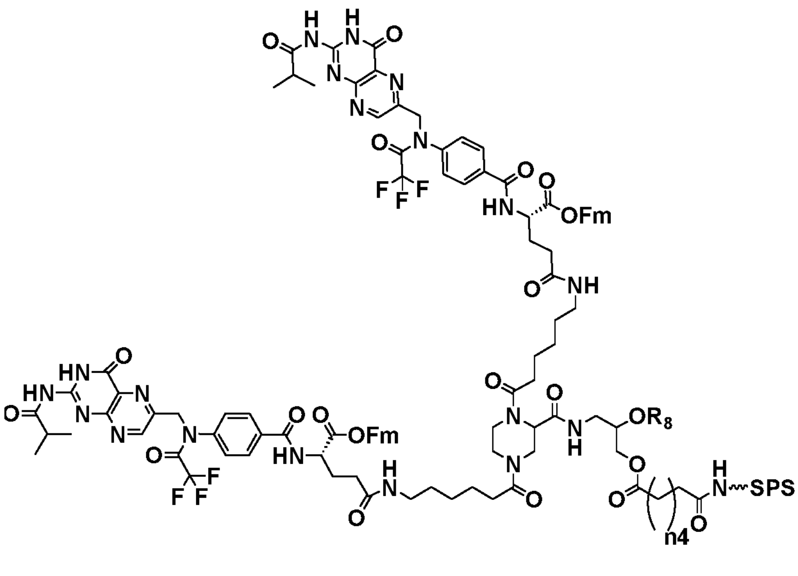

,

Formula (510)

wherein

n4 is an integer of 1-4;
each $B_2$ is independently selected from one of $C_1$-$C_5$ alkyl, ethylcyano, propylcyano and butylcyano;
each Eo is independently O, S or BH; and

SPS represents a solid phase support.

**31.** A drug conjugate having a structure as shown by Formula (301):

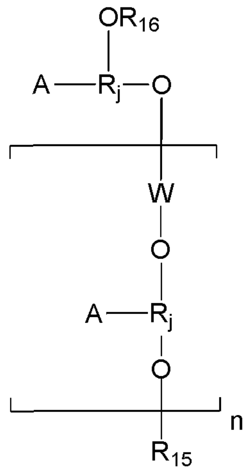

Formula (301)

wherein
A has a structure as shown by Formula (302):

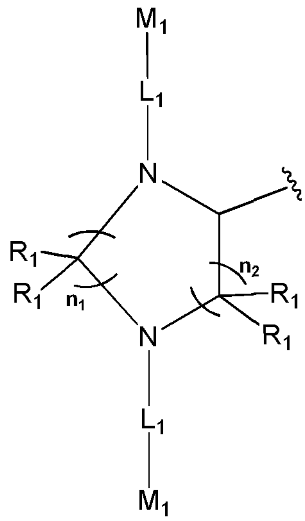
,

Formula (302)

wherein

$n_1$ is an integer of 1-4;
$n_2$ is an integer of 0-3;
n is an integer of 0-7;
each $R_1$ independently of one another is selected from H, substituted or unsubstituted $C_1$-$C_4$ hydrocarbyl or halogen;
each $L_1$ is a linear alkylene of 1 to 70 carbon atoms in length, wherein one or more carbon atoms are optionally replaced with any one or more groups selected from the group consisting of: C(O), NH, O, S, CH=N, $S(O)_2$, $C_2$-$C_{10}$ alkenylene, $C_2$-$C_{10}$ alkynylene, $C_6$-$C_{10}$ arylene, $C_3$-$C_{18}$ heterocyclylene, and $C_5$-$C_{10}$ heteroarylene; and wherein $L_1$ optionally has any one or more substituents selected from the group consisting of: $C_1$-$C_{10}$ alkyl, $C_6$-$C_{10}$ aryl, $C_5$-$C_{10}$ heteroaryl, $C_1$-$C_{10}$ haloalkyl, $OC_1$-$C_{10}$ alkyl, $OC_1$-$C_{10}$ alkylphenyl, -$C_1$-$C_{10}$ alkylOH, $OC_1$-$C_{10}$

haloalkyl, $SC_1$-$C_{10}$ alkyl, $SC_1$-$C_{10}$ alkylphenyl, -$C_1$-$C_{10}$ alkylSH, $SC_1$-$C_{10}$ haloalkyl, halo, OH, -SH, $NH_2$, $C_1$-$C_{10}$ alkyl$NH_2$, N($C_1$-$C_{10}$ alkyl)($C_1$-$C_{10}$ alkyl), NH($C_1$-$C_{10}$ alkyl), -N($C_1$-$C_{10}$ alkyl) ($C_1$-$C_{10}$ alkylphenyl), -NH($C_1$-$C_{10}$ alkylphenyl), cyano, nitro, $CO_2H$, C(O)O($C_1$-$C_{10}$ alkyl), - CON($C_1$-$C_{10}$ alkyl)($C_1$-$C_{10}$ alkyl), CONH($C_1$-$C_{10}$ alkyl), $CONH_2$, NHC(O)($C_1$-$C_{10}$ alkyl), NHC(O)(phenyl), N($C_1$-$C_{10}$ alkyl)C(O)($C_1$-$C_{10}$ alkyl), N($C_1$-$C_{10}$ alkyl)C(O)(phenyl), C(O)$C_1$-$C_{10}$ alkyl, C(O)$C_1$-$C_{10}$ alkylphenyl, C(O)$C_1$-$C_{10}$ haloalkyl, OC(O)$C_1$-$C_{10}$ alkyl, -$SO_2$($C_1$-$C_{10}$ alkyl), -$SO_2$(phenyl), -$SO_2$($C_1$-$C_{10}$ haloalkyl), -$SO_2NH_2$, $SO_2NH$($C_1$-$C_{10}$ alkyl), $SO_2NH$(phenyl), -$NHSO_2$($C_1$-$C_{10}$ alkyl), -$NHSO_2$(phenyl), and $NHSO_2$($C_1$-$C_{10}$ haloalkyl);



represents the site where a group is covalently linked;
each $M_1$ is independently selected from a ligand capable of binding to a cell surface receptor;
$R_j$ is a linking group;
$R_{16}$ and $R_{15}$ each are H or an active drug group, and at least one of $R_{16}$ and $R_{15}$ is an active drug group; and
W is a linking group.

**32.** The drug conjugate according to claim 31, wherein each $L_1$ is independently selected from one of the groups of Formulae A1-A26 or any connection combinations thereof:

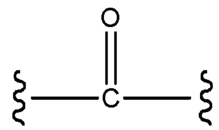,
(A1)

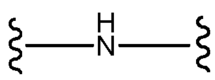,
(A2)

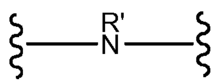,
(A3)

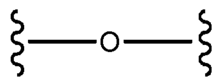,
(A4)

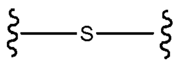,
(A5)

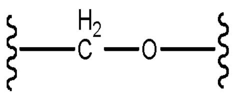,
(A6)

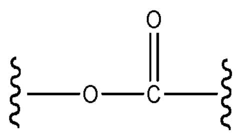,
(A7)

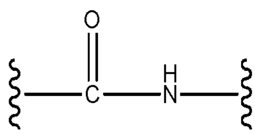,
(A8)

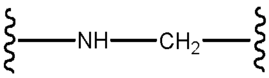,
(A9)

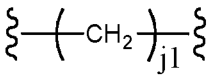,
(A10)

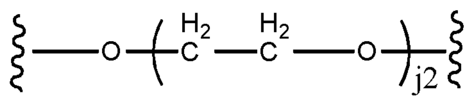,
(A11)

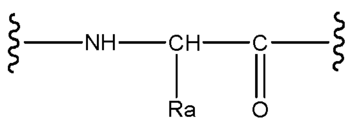,
(A12)

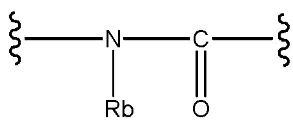,
(A13)

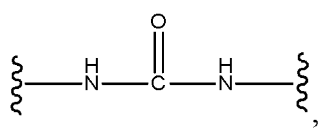,
(A14)

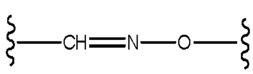,
(A15)

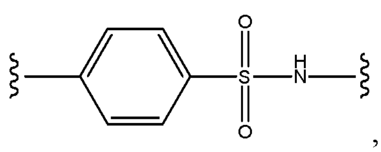,
(A16)

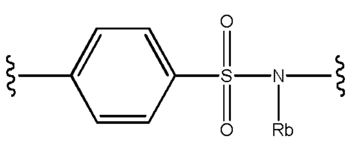,
(A17)

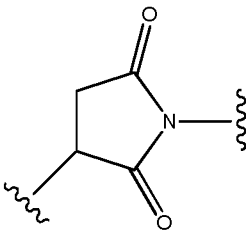, 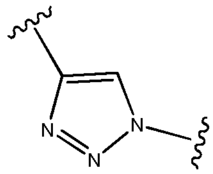, 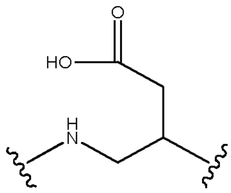, 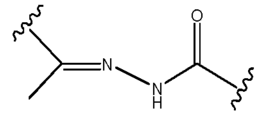,

(A18) (A19) (A20) (A21)

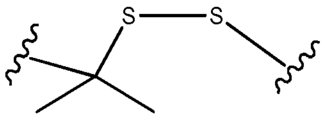, 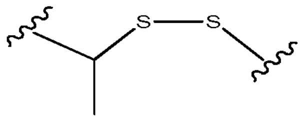, 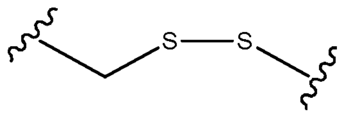,

(A22) (A23) (A24)

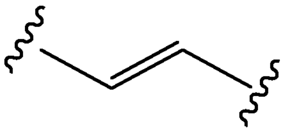, 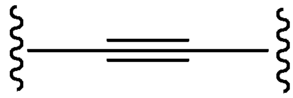;

(A25) (A26)

wherein

Rb is a $C_1$-$C_{10}$ alkyl;
j 1 is an integer of 1-20;
j2 is an integer of 1-20;
R' is a $C_1$-$C_{10}$ alkyl;
Ra is selected from one of the groups of Formulae A27-A45:

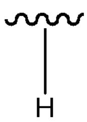, 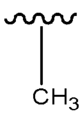, 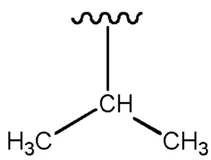, 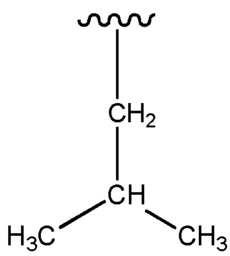, 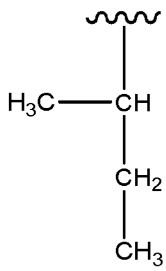, 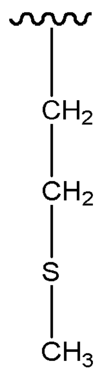,

(A27) (A28) (A29) (A30) (A31) (A32)

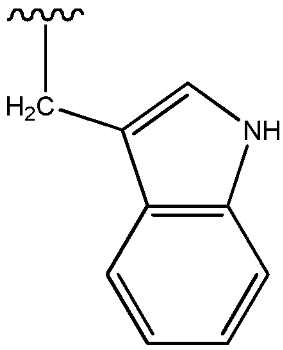, 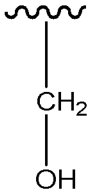, 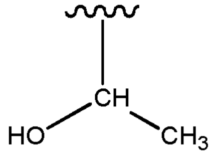, 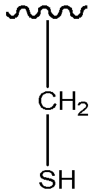, 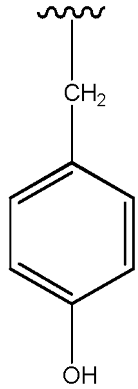,

(A33) (A34) (A35) (A36) (A37)

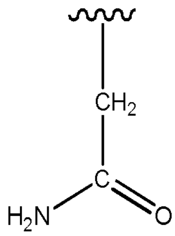, 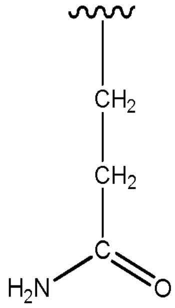, 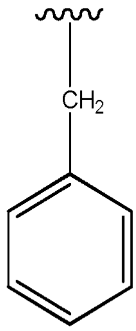, 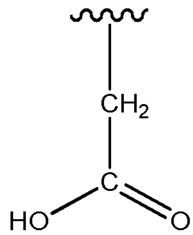, 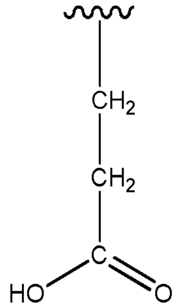,

(A38) (A39) (A40) (A41) (A42)

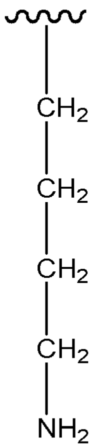, 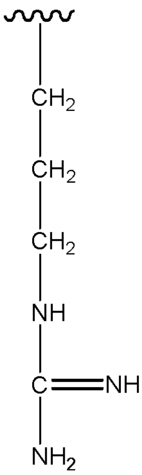 or 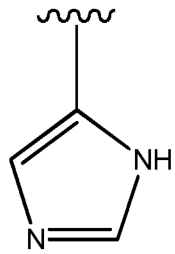.

(A43) (A44) (A45)

**33.** The drug conjugate according to claim 32, wherein $L_1$ is selected from one of A1, A2, A4, A5, A6, A8, A10, A11, A13 or any connection combinations thereof.

**34.** The drug conjugate according to claim 31 or 32, wherein $L_1$ has a length of 3 to 25 atoms; and the length of L1 refers to the number of the chain-forming atoms in the longest atomic chain formed from the atom linked to the N atom in A to the atom linked to $M_1$.

**35.** The drug conjugate according to claim 31 or 32, wherein $n_1$ is 1 or 2; and $n_2$ is 1 or 2.

**36.** The drug conjugate according to claim 35, wherein A has a structure as shown by Formula (320):

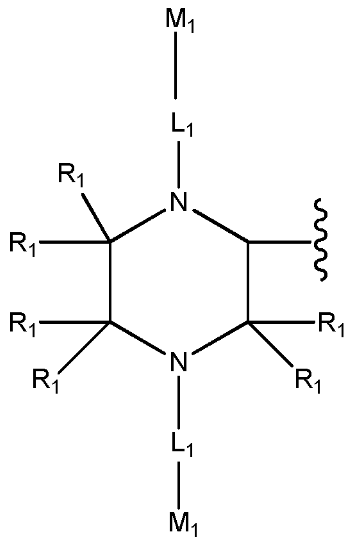

Formula (320).

**37.** The drug conjugate according to claim 31 or 32, wherein the drug conjugate is an oligonucleotide conjugate, wherein the active drug group has a structure as shown by Formula A60

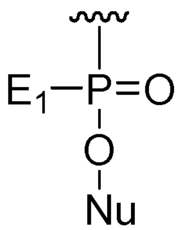

Formula (A60)

wherein

$E_1$ is OH, SH or $BH_2$;

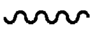 represents the site where a group is linked; and
Nu is a functional oligonucleotide.

**38.** The drug conjugate according to claim 31 or 32, wherein each $M_1$ is independently selected from one of the ligands formed by lipophilic molecules, saccharides, vitamins, polypeptides, endosomal lysates, steroid compounds, terpene compounds, integrin receptor inhibitors, cationic lipid molecules, or derivatives thereof。

**39.** The drug conjugate according to claim 38, wherein each $M_1$ is independently selected from one of D-mannopyranose, L-mannopyranose, D-arabinose, D-xylofuranose, L-xylofuranose, D-glucose, L-glucose, D-galactose, L-galactose, α-D-mannofuranose, β-D-mannofuranose, α-D-mannopyranose, β-D-mannopyranose, α-D-glucopyranose, β-D-glucopyranose, α-D-glucofuranose, β-D-glucofuranose, α-D-fructofuranose, α-D-fructopyranose, α-D-galactopyranose, β-D-galactopyranose, α-D-galactofuranose, β-D-galactofuranose, glucosamine, sialic acid, galactosamine, N-acetylgalactosamine, N-trifluoroacetylgalactosamine, N-propionylgalactosamine, N-n-butyrylgalactosamine, N-isobutyrylgalactosamine, 2-amino-3-O-[(R)-1-carboxyethyl]-2-deoxy-β-D-glucopyranose, 2-deoxy-2-methylamino-L-glucopyranose, 4,6-dideoxy-4-formamido-2,3-di-O-methyl-D-mannopyranose, 2-deoxy-2-sulfoamino-D-glucopyranose, N-glycolyl-α-neuraminic acid, 5-thio-β-D-glucopyranose, methyl 2,3,4-tris-O-acetyl-1-thio-6-O-trityl-α-D-glucopyranoside, 4-thio-β-D-galactopyranose, ethyl 3,4,6,7-tetra-O-acetyl-2-deoxy-1,5-dithio-α-D-glucoheptopyranoside, 2,5-anhydro-D-allononitrile, ribose, D-ribose, D-4-thioribose, L-ribose, and L-4-thioribose.

**40.** The drug conjugate according to claim 31 or 32, wherein each $M_1$ is independently selected from one of the following groups: a ligand formed by cholesterol, cholic acid, adamantaneacetic acid, 1-pyrenebutyric acid, dihydrotestosterone, 1,3-Bis-O(hexadecyl)glycerol, hexaglycerol, menthol, mentha-camphor, 1,3-propanediol, palmitic acid, myristic acid, O3-(oleoyl)lithocholic acid, benzoxazine, folate, folate derivatives, vitamin A, vitamin B7 (biotin), pyridoxal, uvaol, triterpene, friedelin, or epifriedelinol-derived lithocholic acid, or geranyloxyhexyl, heptadecyl, dimethoxytrityl, hecogenin, diosgenin, or sarsasapogenin.

**41.** The drug conjugate according to claim 40, wherein each $M_1$ is independently selected from a ligand formed by one of the following compounds: folate, folate analogues or folate mimetics.

**42.** The drug conjugate according to claim 41, wherein each $M_1$ is independently selected from one of the groups of Formulae H1-H5:

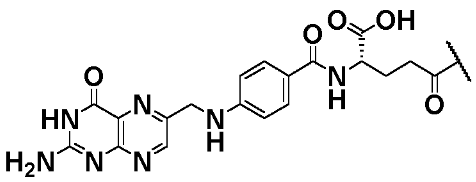,

Formula (H1)

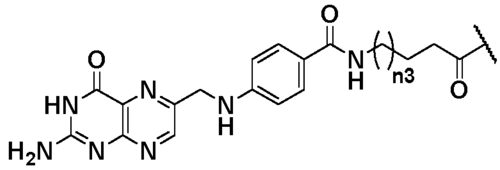,

Formula (H2)

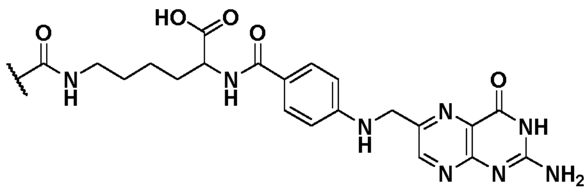,

Formula (H3)

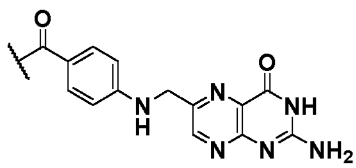, and

Formula (H4)

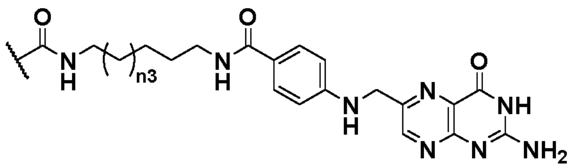,

Formula (H5)

wherein n3 is an integer of 1-5; and

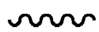

represents the site where a group is covalently linked.

**43.** The drug conjugate according to claim 31 or 32, wherein n is an integer of 0-4.

**44.** The drug conjugate according to claim 31 or 32, wherein $R_j$ is selected from one of the groups of Formulae A62-A67:

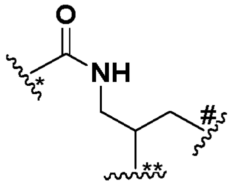 , 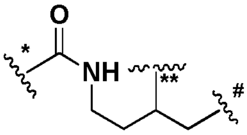 , 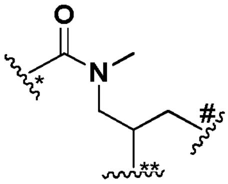 , 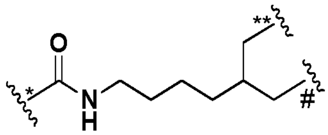 ,

Formula (A62) Formula (A63) Formula (A64) Formula (A65)

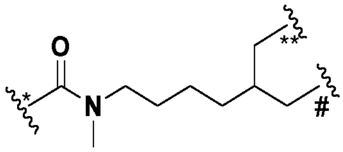 , and 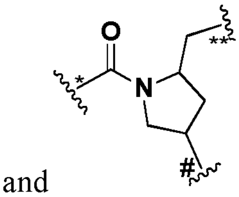

Formula (A66) Formula (A67).

**45.** The drug conjugate according to claim 31 or 32, wherein W is a group as shown by Formula (A61) or (C1'):

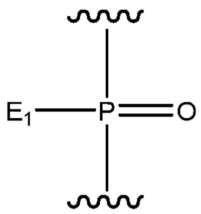 , 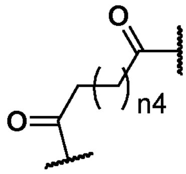

Formula (A61) Formula (C1')

wherein

$E_1$ is OH, SH or $BH_2$; and
n4 is an integer of 1-4.

**46.** The drug conjugate according to claim 31 or 32, wherein the drug conjugate has a structure as shown by Formula (303), (304), (305), (306), (307), (308), (309), (310), or (311):

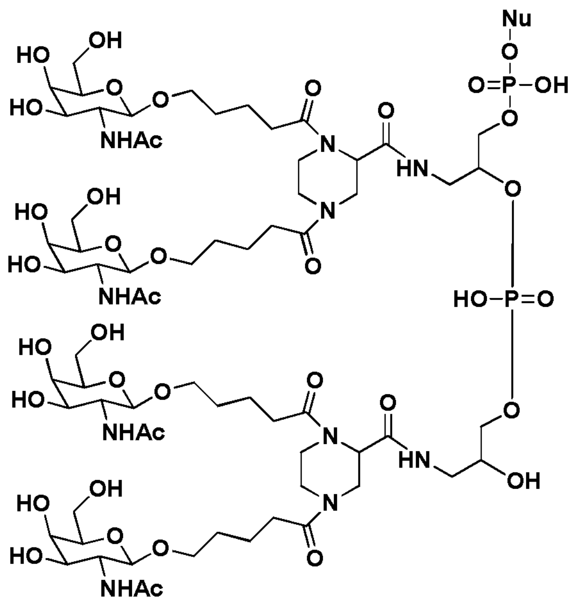

,

Formula (303)

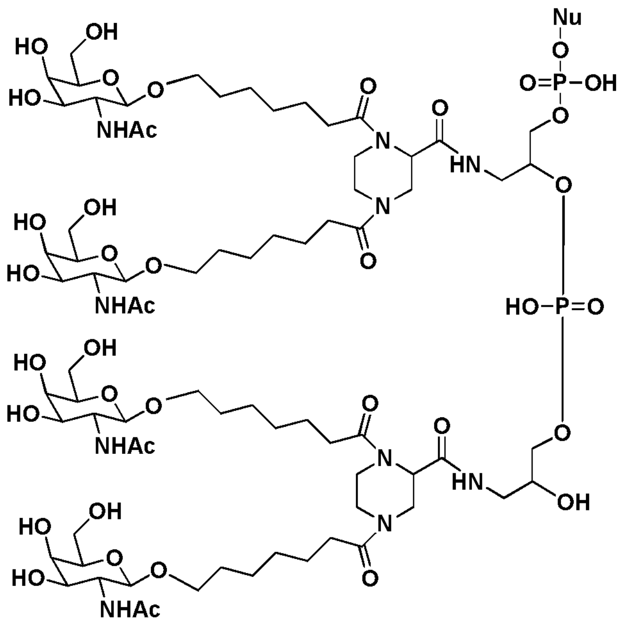

,

Formula (304)

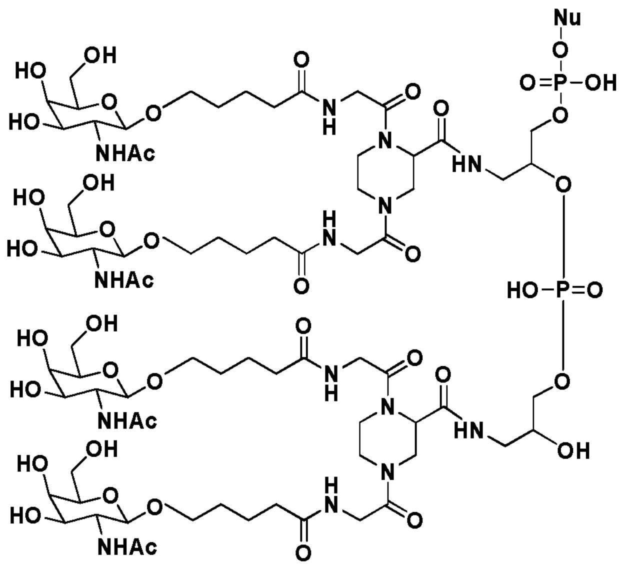

,

Formula (305)

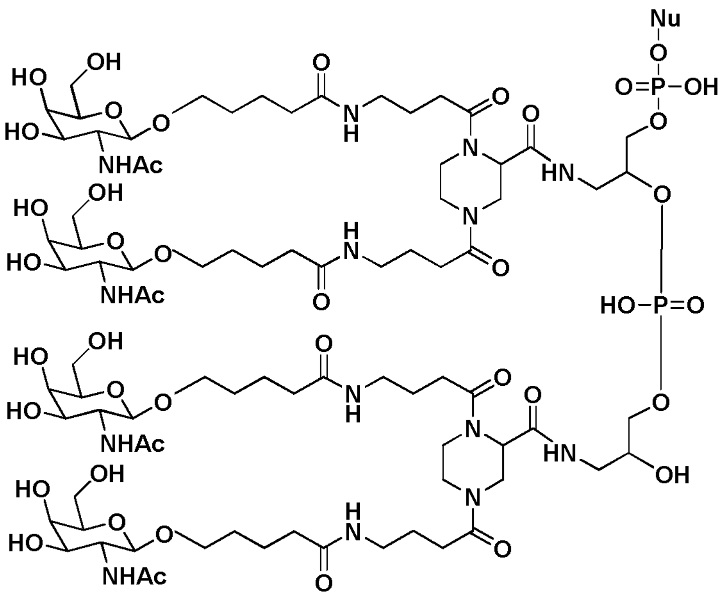

,

Formula (306)

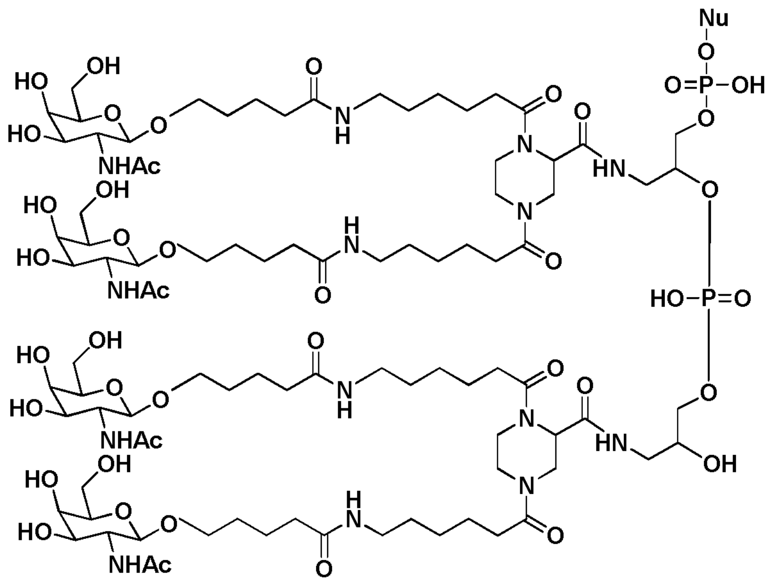

,

Formula (307)

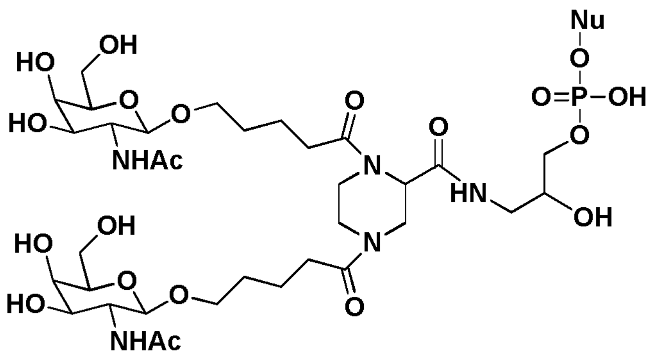

,

Formula (308)

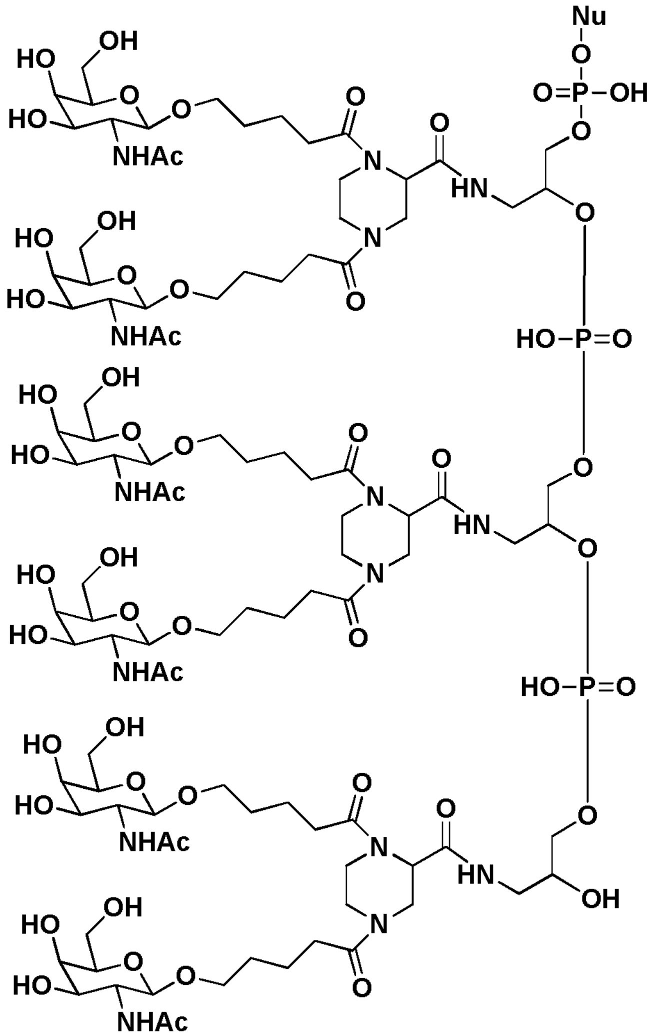

,

Formula (309)

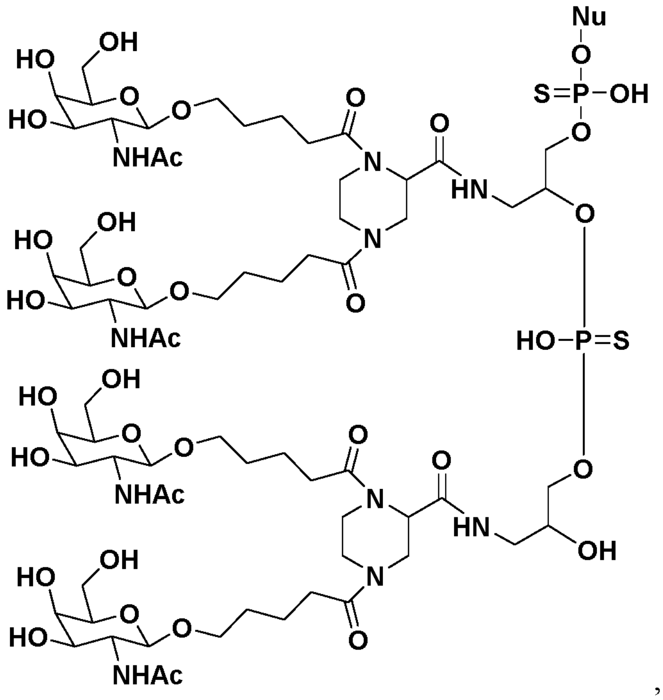

Formula (310)

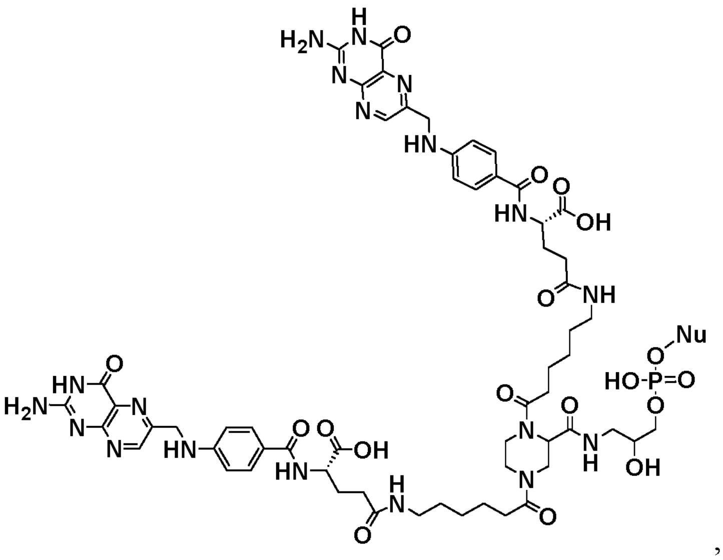

Formula (311)

wherein Nu represents a functional oligonucleotide.

**47.** The drug conjugate according to claim 37 or 46, wherein the functional oligonucleotide is selected from one of the following: small interfering RNA, microRNA, anti-microRNA, microRNA antagonist, microRNA mimetic, decoy oligonucleotide, immunologic stimulant, G-quadrupole, alternative spliceosome, single-stranded RNA, antisense nucleic acid, nucleic acid aptamer, stem-loop RNA, mRNA fragment, activating RNA or DNA.

**48.** The drug conjugate according to claim 47, wherein the functional oligonucleotide is a single-stranded or a double-stranded oligonucleotide.

**49.** The drug conjugate according to claim 48, wherein the functional oligonucleotide is an siRNA.

**50.** The drug conjugate according to claim 49, wherein each nucleotide in the siRNA is independently of one another a modified or unmodified nucleotide; the siRNA comprises a sense strand and an antisense strand; wherein the sense strand comprises a nucleotide sequence 1, and the antisense strand comprises a nucleotide sequence 2; the nucleotide sequence 1 and the nucleotide sequence 2 have a length of 19 nucleotides and are at least partly reverse complementary to form a double-stranded region; the nucleotide sequence 2 is at least partly complementary to a first segment of nucleotide sequence which is a segment of nucleotide sequence in a target mRNA; wherein the target mRNA is an mRNA corresponding to the gene that is abnormally expressed in a cell.

**51.** The drug conjugate according to claim 50, wherein the cell is a hepatic cell, lung cell, or tumor cell.

**52.** The drug conjugate according to claim 50, wherein the target mRNA is selected from one of the mRNAs corresponding to the following genes: ApoB, ApoC, ANGPTL3, PCSK9, SCD1, TIMP-1, Col1A1, FVII, STAT3, p53, HBV, and HCV.

**53.** Use of the drug conjugate according to any one of claims 31-52 in the manufacture of a medicament for treating and/or preventing a pathological condition or disease caused by the expression of a gene in a cell.

**54.** The use according to claim 53, wherein the gene is selected from hepatitis B virus gene, angiopioetin-like protein 3 gene, apolipoprotein C3 gene, or signal transducer and activator of transcription 3 gene.

**55.** The use according to claim 53, wherein the disease is selected from chronic liver diseases, hepatitis, hepatic fibrosis diseases, hepatic proliferative diseases, and diseases caused by dyslipidemia or tumor.

**56.** A method for treating a pathological condition or disease caused by the expression of a gene in a cell, comprising administering the drug conjugate according to any one of claims 31-52 to a patient suffering from the disease.

**57.** The method according to claim 56, wherein the gene is selected from hepatitis B virus gene, angiopioetin-like protein 3 gene, apolipoprotein C3 gene, or signal transducer and activator of transcription 3 gene.

**58.** The method according to claim 56, wherein the disease is selected from chronic liver diseases, hepatitis, hepatic fibrosis diseases, hepatic proliferative diseases, diseases caused by dyslipidemia or tumor.

**59.** A method for regulation of the expression of a gene in a cell, comprising contacting the drug conjugate according to any one of claims 31-52 with the cell, wherein the regulation comprises inhibiting or enhancing the expression of the gene.

**60.** The method according to claim 59, wherein the gene is selected from one of the following genes: ApoB, ApoC, ANGPTL3, PCSK9, SCD1, TIMP-1, Col1A1, FVII, STAT3, p53, HBV, and HCV

**61.** A kit, comprising the drug conjugate according to any one of claims 31-52.

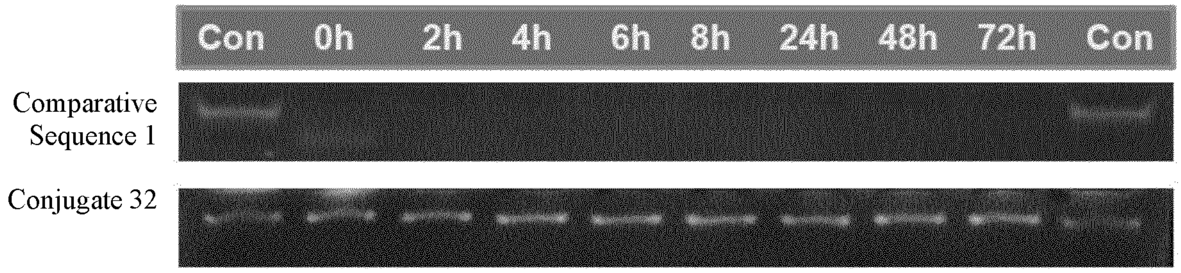


Figure 1

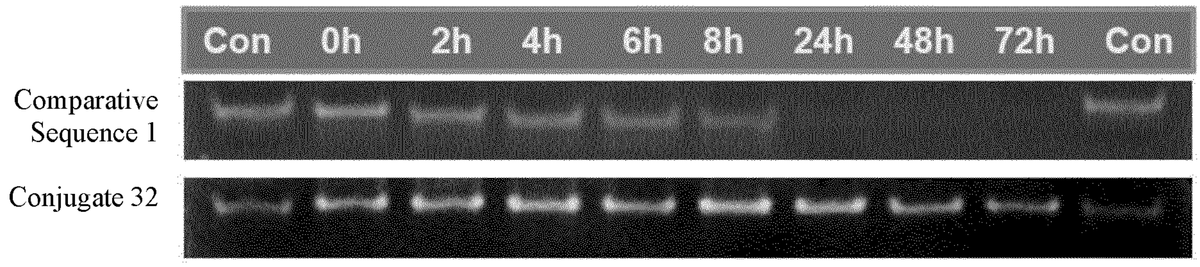


Figure 2

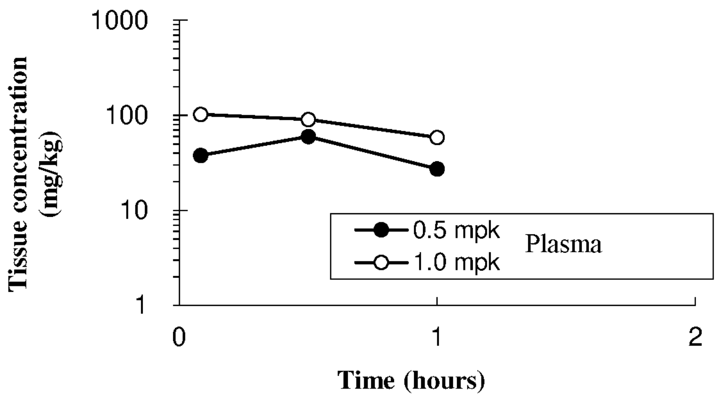


Figure 3

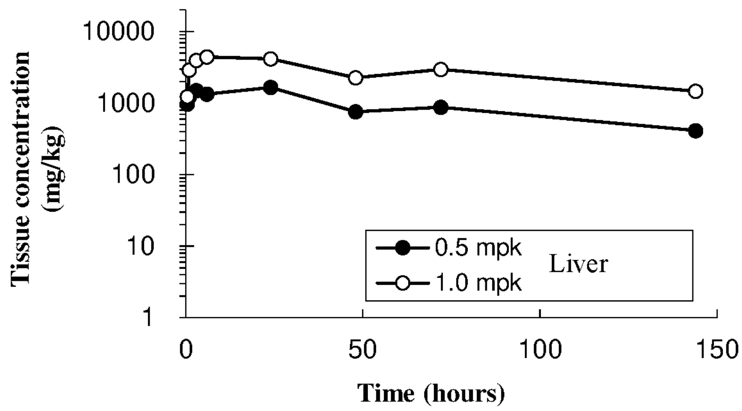


Figure 4

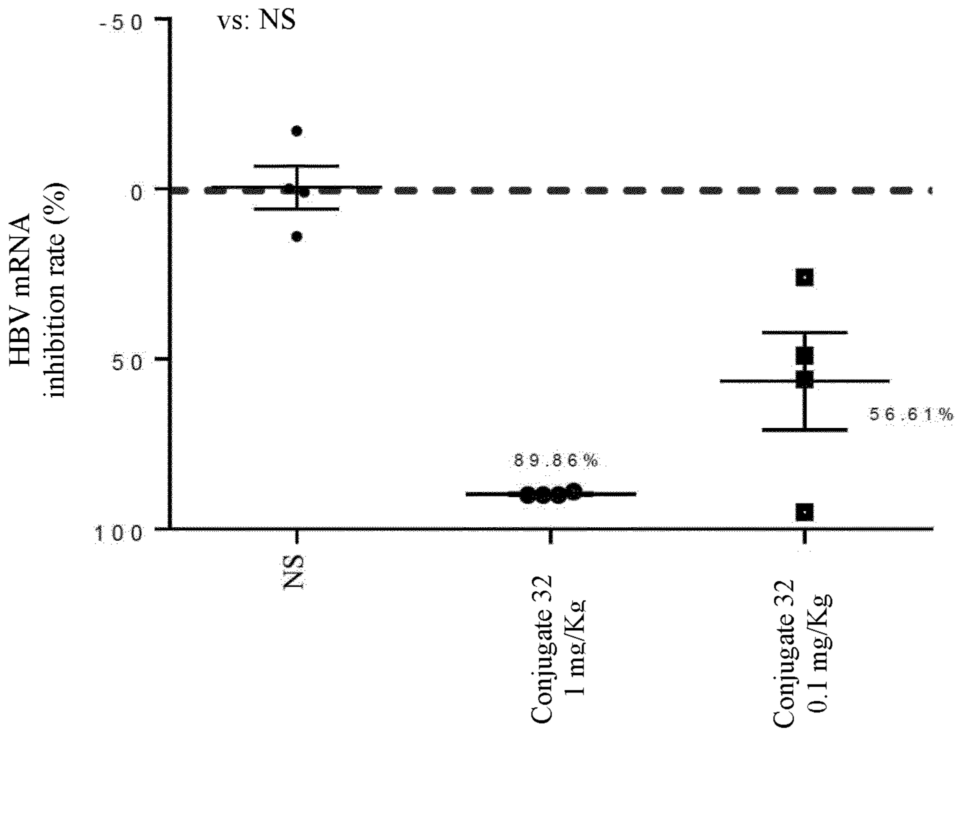


Figure 5

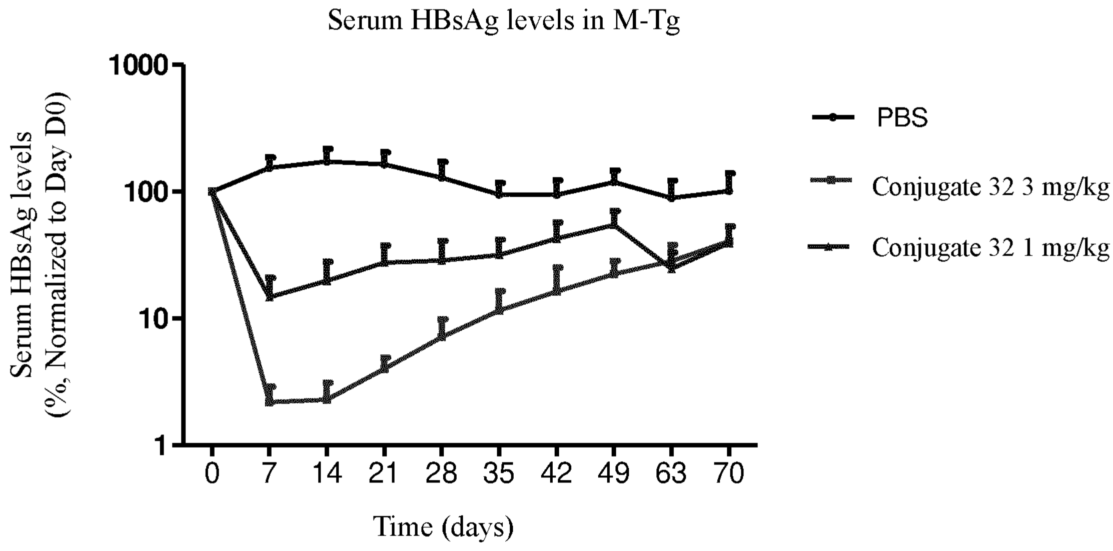


Figure 6

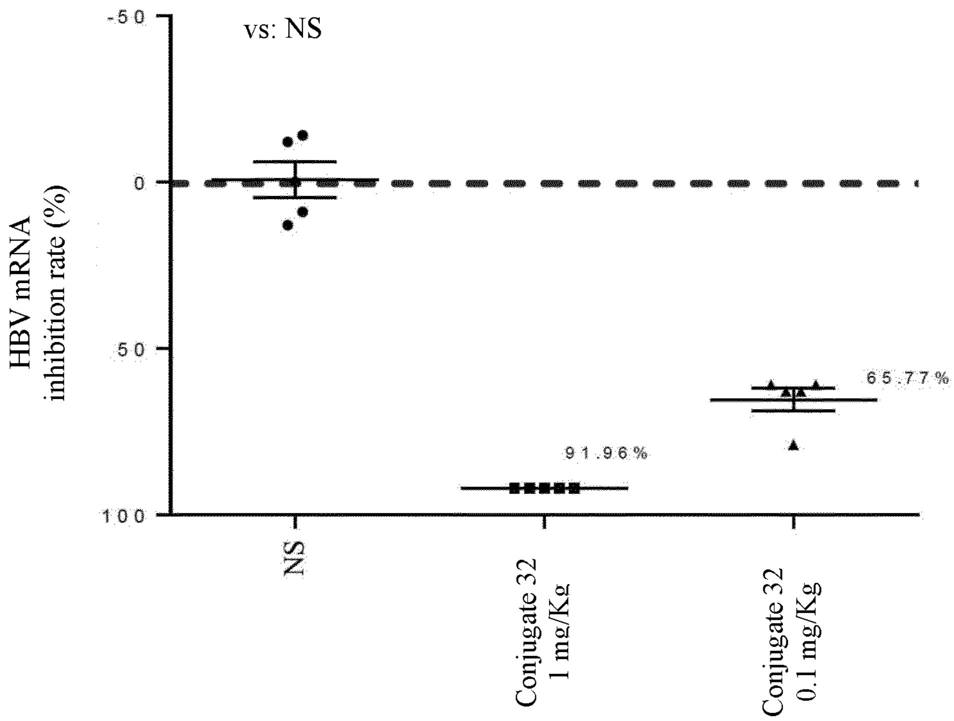


Figure 7A

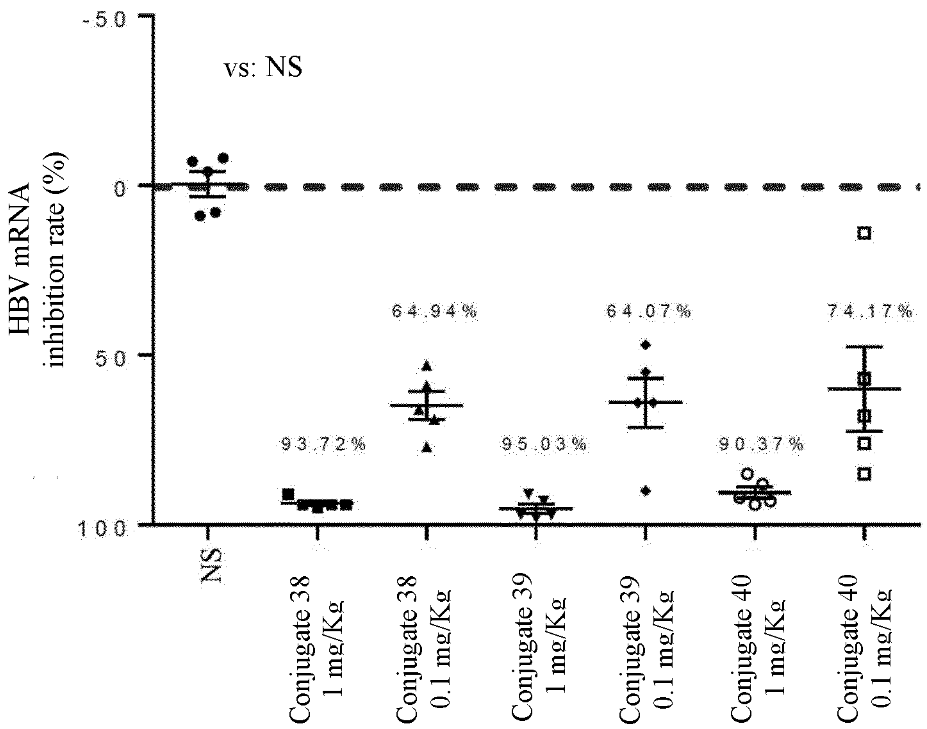


Figure 7B

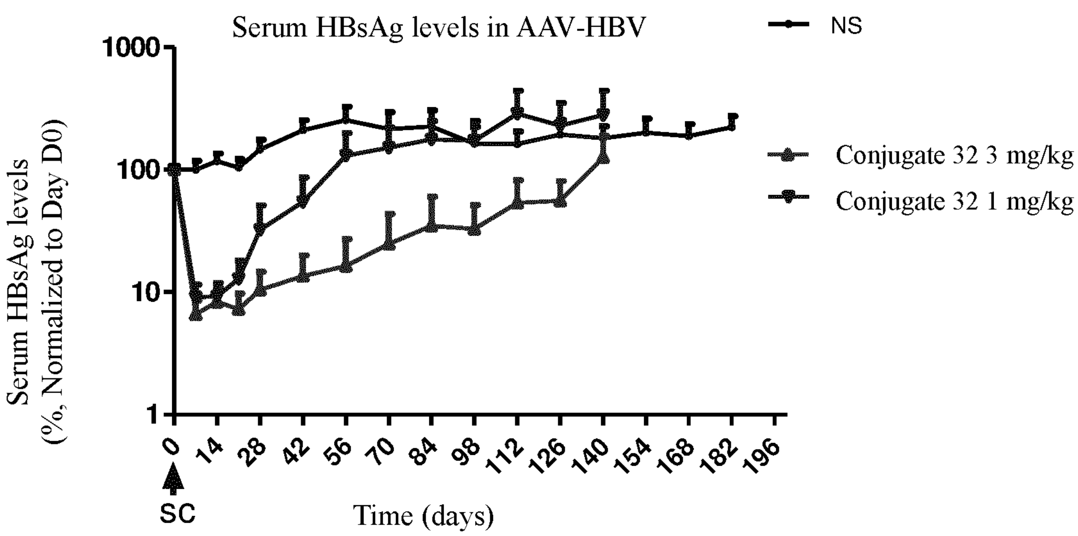


Figure 8A

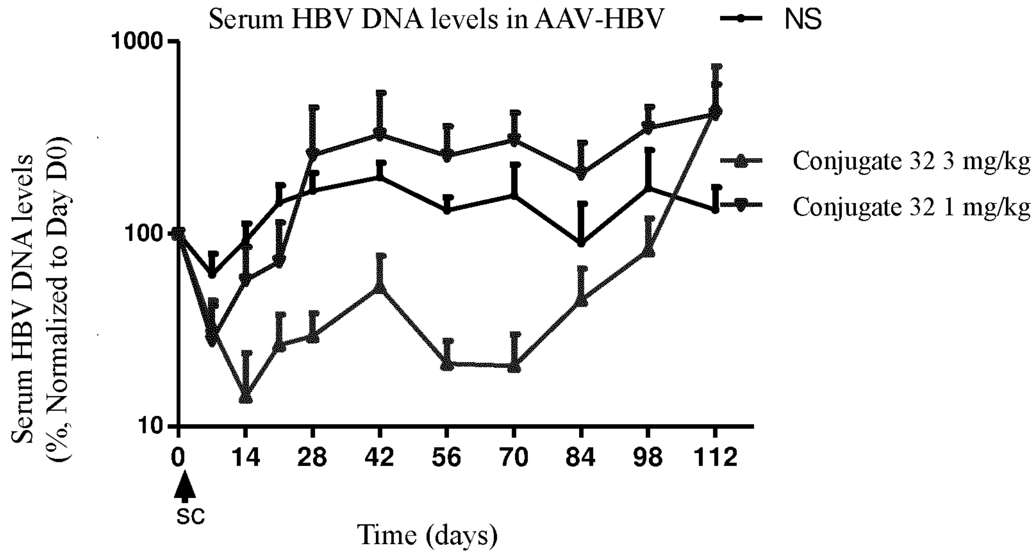


Figure 8B

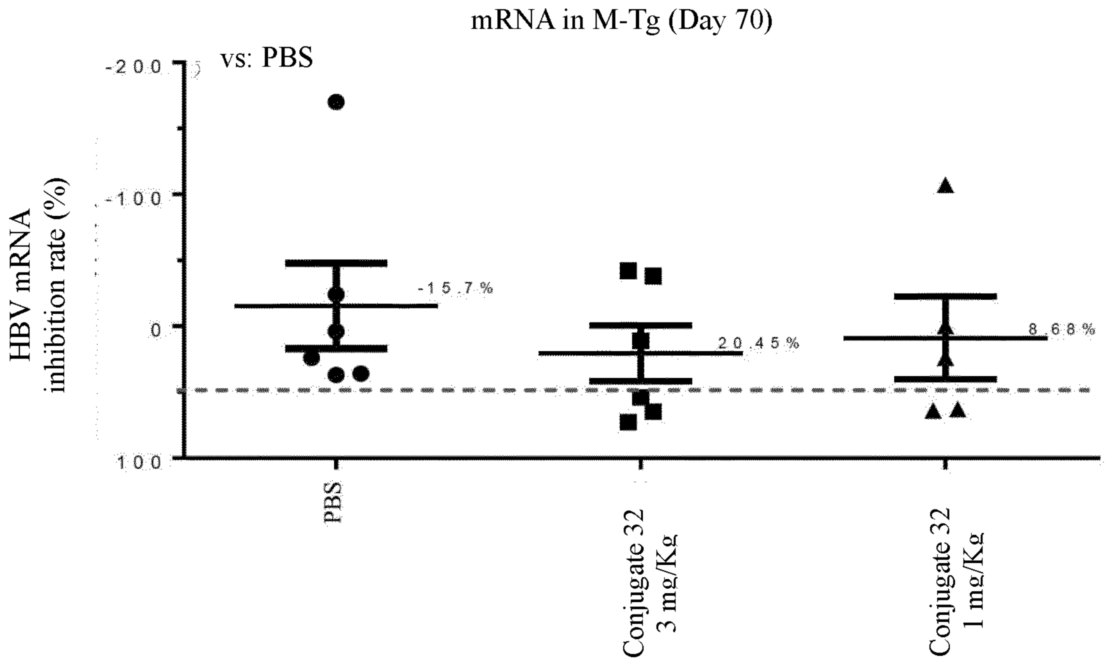


Figure 9

**INTERNATIONAL SEARCH REPORT**

International application No. **PCT/CN2020/112105**

**A. CLASSIFICATION OF SUBJECT MATTER**

C07H 15/04(2006.01)i; C07H 21/02(2006.01)i; C12N 15/113(2010.01)i; C12N 15/00(2006.01)i; A61K 31/7105(2006.01)i; A61K 47/54(2017.01)i; A61P 1/16(2006.01)i

According to International Patent Classification (IPC) or to both national classification and IPC

**B. FIELDS SEARCHED**

Minimum documentation searched (classification system followed by classification symbols)

C07H; C12N; A61K; A61P

Documentation searched other than minimum documentation to the extent that such documents are included in the fields searched

Electronic data base consulted during the international search (name of data base and, where practicable, search terms used)

CNKI; CNABS; CNTXT; CJFD; VEN; USTXT; EPTXT; WOTXT; STN; PATENTICS; 谷歌学术: 瑞博生物, 张鸿雁, 杨志伟, 高山, 曹力强, 刘国成, 缀合物, 递送, 拼接物, 寡核苷酸, conjugate?, diliver+, oligonucleotide, structural formula (I)

**C. DOCUMENTS CONSIDERED TO BE RELEVANT**

| Category* | Citation of document, with indication, where appropriate, of the relevant passages | Relevant to claim No. |
|---|---|---|
| A | WO 2019128611 A1 (SUZHOU RIBO LIFE SCIENCE CO., LTD.) 04 July 2019 (2019-07-04)<br>claims 1-114, description paragraphs [0346]-[0518] | 1-55, 59-61 |
| A | WO 2016206626 A1 (SUZHOU RIBO LIFE SCIENCE CO., LTD.) 29 December 2016 (2016-12-29)<br>entire document | 1-55, 59-61 |
| A | WO 2018223056 A1 (WAVE LIFE SCIENCES LTD. et al.) 06 December 2018 (2018-12-06)<br>entire document | 1-55, 59-61 |
| A | WO 2018223073 A1 (WAVE LIFE SCIENCES LTD. et al.) 06 December 2018 (2018-12-06)<br>entire document | 1-55, 59-61 |
| A | WO 2018223081 A1 (WAVE LIFE SCIENCES LTD. et al.) 06 December 2018 (2018-12-06)<br>entire document | 1-55, 59-61 |
| A | WO 2011154331 A1 (F.HOFFMANN-LA ROCHE AG et al.) 15 December 2011 (2011-12-15)<br>entire document | 1-55, 59-61 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 October 2020** | **27 November 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/112105**

**C. DOCUMENTS CONSIDERED TO BE RELEVANT**

| Category* | Citation of document, with indication, where appropriate, of the relevant passages | Relevant to claim No. |
|---|---|---|
| A | WO 2010012244 A1 (SUZHOU RIBO LIFE SCIENCE CO., LTD. et al.) 04 February 2010 (2010-02-04) entire document | 1-55, 59-61 |
| A | CN 107109405 A (SOLSTICE BIOLOG LTD.) 29 August 2017 (2017-08-29) entire document | 1-55, 59-61 |
| A | CN 105452465 A (QBI ENTERPRISES LTD. et al.) 30 March 2016 (2016-03-30) entire document | 1-55, 59-61 |

Form PCT/ISA/210 (second sheet) (January 2015)

**INTERNATIONAL SEARCH REPORT**

International application No.
**PCT/CN2020/112105**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2020/112105**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **56-60**
because they relate to subject matter not required to be searched by this Authority, namely:

[1] Claims 56-58 set forth a method for treating pathological conditions caused by gene expression in cells, and claims 59 and 60 set forth a method for regulating gene expression in a cell, said subject matter falling within the scope of methods for treatment of the living human or animal body, and thus belonging to subject matter for which an international search is not required under PCT Rule 39.1(iv). Nevertheless, a search has been carried out on the basis of claims 59 and 60 setting forth the subject matter of a use of the drug conjugate in any one of claims 31-52 in preparing a drug for regulating gene expression.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/112105**

| Patent document cited in search report | Publication date (day/month/year) | Patent family member(s) | Publication date (day/month/year) |
|---|---|---|---|
| WO 2019128611 A1 | 04 July 2019 | AU 2018394875 A1 | 09 April 2020 |
| | | IN 202047017398 A | 12 June 2020 |
| | | CN 110959011 A | 03 April 2020 |
| | | CA 3087106 A1 | 04 July 2019 |
| | | KR 2020105812 A | 09 September 2020 |
| | | TW 201929905 A | 01 August 2019 |
| WO 2016206626 A1 | 29 December 2016 | US 2019062749 A1 | 28 February 2019 |
| | | EP 3315608 B1 | 16 September 2020 |
| | | CN 107849567 A | 27 March 2018 |
| | | EP 3315608 A4 | 12 December 2018 |
| | | EP 3315608 A1 | 02 May 2018 |
| | | JP 6715325 B2 | 01 July 2020 |
| | | JP 2018518201 A | 12 July 2018 |
| WO 2018223056 A1 | 06 December 2018 | EP 3630199 A1 | 08 April 2020 |
| | | CN 111050806 A | 21 April 2020 |
| | | JP 2020522510 W | 30 July 2020 |
| | | US 2020157545 A1 | 21 May 2020 |
| WO 2018223073 A1 | 06 December 2018 | JP 2020522265 W | 30 July 2020 |
| | | EP 3630788 A1 | 08 April 2020 |
| | | CN 111164091 A | 15 May 2020 |
| | | AR 112063 A1 | 18 September 2019 |
| | | TW 201908483 A | 01 March 2019 |
| WO 2018223081 A1 | 06 December 2018 | CA 3065523 A1 | 06 December 2018 |
| | | EP 3630789 A1 | 08 April 2020 |
| | | JP 2020524485 W | 20 August 2020 |
| | | AR 112064 A1 | 18 September 2019 |
| | | US 2020190515 A1 | 18 June 2020 |
| | | CN 110997692 A | 10 April 2020 |
| | | TW 201904587 A | 01 February 2019 |
| WO 2011154331 A1 | 15 December 2011 | None | |
| WO 2010012244 A1 | 04 February 2010 | CN 102083983 A | 01 June 2011 |
| | | CN 102083983 B | 16 April 2014 |
| CN 107109405 A | 29 August 2017 | WO 2015188197 A2 | 10 December 2015 |
| | | AU 2015269053 A1 | 22 December 2016 |
| | | EP 3152308 A4 | 27 December 2017 |
| | | WO 2015188197 A3 | 25 February 2016 |
| | | CA 2950960 A1 | 10 December 2015 |
| | | EP 3152308 A2 | 12 April 2017 |
| | | US 2017114341 A1 | 27 April 2017 |
| | | JP 2017522046 A | 10 August 2017 |
| CN 105452465 A | 30 March 2016 | WO 2015015496 A1 | 05 February 2015 |
| | | EP 3027222 A1 | 08 June 2016 |
| | | US 2016175452 A1 | 23 June 2016 |
| | | US 9889200 B2 | 13 February 2018 |
| | | CN 105452465 B | 21 June 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 105378082 A
- US 8106022 B2
- WO 2009082607 A
- WO 2009082607 A2
- WO 2009073809 A2
- WO 2015006740 A2

### Non-patent literature cited in the description


- **BEAUCAGE et al.** *Tetrahedron,* 1992, vol. 48, 2223-2311
- **GREENE ; WUTS.** Protective Groups in Organic Synthesis. John Wiley & Sons, 1991
- **J.K. WATTS ; G.F. DELEAVEY ; M.J. DAMHA.** Chemically modified siRNA: tools and applications. *Drug Discov Today,* 2008, vol. 13 (19-20), 842-55
- **ANASTASIA KHVOROVA ; JONATHAN K. WATTS.** The chemical evolution of oligonucleotide therapies of clinical utility. *Nature Biotechnology,* 2017, vol. 35 (3), 238-48
- Molecular Cloning. Cold Spring Harbor LBboratory Press, 1989
- **REN J et al.** *J. Medical Virology.,* 2006, vol. 78, 551-560
- **DONG XIAOYAN et al.** *Chin J Biotech,* 25 May 2010, vol. 26 (5), 679-686
- *CHEMICAL ABSTRACTS,* 1772-03-8
- *CHEMICAL ABSTRACTS,* 27607-77-8
- *CHEMICAL ABSTRACTS,* 821-41-0
- *CHEMICAL ABSTRACTS,* 7790-28-5
- *CHEMICAL ABSTRACTS,* 14898-67-0
- *CHEMICAL ABSTRACTS,* 2762-32-5
- *CHEMICAL ABSTRACTS,* 102691-36-1
- *CHEMICAL ABSTRACTS,* 5793-73-8
- *CHEMICAL ABSTRACTS,* 24324-17-2
- *CHEMICAL ABSTRACTS,* 1122-58-3
- *CHEMICAL ABSTRACTS,* 538-75-0
- *CHEMICAL ABSTRACTS,* 252847-30-6
- *CHEMICAL ABSTRACTS,* 25952-53-8
- *CHEMICAL ABSTRACTS,* 2592-95-2
- *CHEMICAL ABSTRACTS,* 3022-15-9
- *CHEMICAL ABSTRACTS,* 24424-99-5
- *CHEMICAL ABSTRACTS,* 121-44-8
- *CHEMICAL ABSTRACTS,* 616-30-8
- *CHEMICAL ABSTRACTS,* 165534-43-0
- *CHEMICAL ABSTRACTS,* 88574-06-5
- *CHEMICAL ABSTRACTS,* 108-30-5
- *CHEMICAL ABSTRACTS,* 94790-37-1
- **REN et al.** *J. Medical Virology,* 2006, vol. 78, 551-560